(19) Europäisches Patentamt
European Patent Office
Office européen des brevets

(11) EP 3 896 056 A1

(12) # EUROPEAN PATENT APPLICATION
published in accordance with Art. 153(4) EPC

(43) Date of publication:
**20.10.2021 Bulletin 2021/42**

(21) Application number: **19896830.7**

(22) Date of filing: **12.12.2019**

(51) Int Cl.:
***C07C 229/22*** *(2006.01)* ***C07C 235/06*** *(2006.01)*
***C07C 271/22*** *(2006.01)* ***C07K 7/00*** *(2006.01)*
***C07K 11/02*** *(2006.01)* ***C07K 14/00*** *(2006.01)*
***C40B 40/08*** *(2006.01)* ***C40B 40/10*** *(2006.01)*

(86) International application number:
**PCT/JP2019/048720**

(87) International publication number:
**WO 2020/122182 (18.06.2020 Gazette 2020/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.12.2018 JP 2018232144**

(71) Applicant: **CHUGAI SEIYAKU KABUSHIKI KAISHA**
**Tokyo 115-8543 (JP)**

(72) Inventors:
- **MURAOKA, Terushige**
**Kamakura-shi, Kanagawa 247-8530 (JP)**
- **TANAKA, Shota**
**Kamakura-shi, Kanagawa 247-8530 (JP)**

(74) Representative: **Vossius & Partner**
**Patentanwälte Rechtsanwälte mbB**
**Siebertstraße 3**
**81675 München (DE)**

(54) **AMINO ACID HAVING FUNCTIONAL GROUP CAPABLE OF INTERMOLECULAR HYDROGEN BONDING, PEPTIDE COMPOUND CONTAINING SAME AND METHOD FOR PRODUCTION THEREOF**

(57) It has been found that the membrane permeability of peptide compounds can be improved by making at least one of amino acids constituting the peptide compound be an amino acid having a side chain capable of forming an intramolecular hydrogen bond.

EP 3 896 056 A1

## Description

[Technical Field]

The present invention relates to amino acids capable of increasing the membrane permeability of peptide compounds, peptide compounds containing the amino acid(s), and production methods therefor.

[Background Art]

Access to a tough target, which is represented by inhibition of a protein-protein interaction, may be done better by middle-molecular weight compounds (molecular weight: 500 to 2000) than low molecular weight compounds. Furthermore, middle-molecular weight compounds may be superior to antibodies in that they can transfer into cells. Among middle-molecular weight compounds that have physiological activity, peptide drugs are valuable molecular species, with 40 or more peptide drugs having already been commercially available (NPL 1).

Representative examples of peptide drugs include cyclosporin A and polymyxin B. Focusing on their structures, they are characterized by being peptides containing some non-natural amino acids represented by *N*-methyl amino acids or being cyclic peptides. Non-natural amino acids refer to amino acids that are not naturally encoded on mRNA, and it is highly interesting that not only are non-natural amino acids contained in naturally-occurring cyclosporin A and polymyxin B, their non-natural structural sites or cyclic structures contribute to an interaction with an action site in a living body and *in vivo* kinetics to express pharmacological activity.

In recent years, as conditions for increasing membrane permeability of middle-molecular weight peptides, thereby potentially contributing to improvement of their *in vivo* kinetics (conditions necessary for satisfying drug-likeness), conditions that have been reported are such as their possession of a cyclic portion, the number of *N*-substituted amino acids, the range of the number of amino acid residues, and lipophilicity (PTL 1). Furthermore, there has been a report that *N*-methyl peptide libraries have been made by translational (ribosomal) synthesis, focusing on *N*-methyl amino acids, which are non-natural amino acids (PTL 2). Moreover, an interaction between a target and a drug is important for formation of a complex of the target and the drug, and important among such interactions is a hydrogen bond (PTL 3). A proton donor is essential to form a hydrogen bond, but it can adversely affect membrane permeability. In fact, *N*-methylation of an amino acid has been known to work as one of methods for improving membrane permeability (PTL 1). Other than the *N*-methylation of an amino acid, an investigation has been made for the effect on membrane permeability by masking a proton utilizing an amide bond contained in the main chain (NPL 2).

On the other hand, there has been no report that non-natural amino acids were designed to have a proton and a functional group capable of masking the proton on a side chain of the amino acids and that the membrane permeability of peptides were improved by the function of masking the proton.

[Citation List]

[Patent Literature]

[PTL 1] WO 2013/100132
[PTL 2] WO 2012/033154

[Non-Patent Literature]

[NPL 1] Future Med. Chem. 2009, 1, 1289-1310.
[NPL 2] Angew. Chem. Int. Ed. 2018, 57, 14414-14438.
[NPL 3] Saisin Soyaku Kagaku (The Practice of Medicinal Chemistry), Volume 1, p87

[Summary of Invention]

[Technical Problem]

It can be said that an increased interaction between a target molecule and a peptide is essential for creation of a medically useful peptide. It can be said to be further important that, to increase an interaction between a target

molecule and a peptide, the structure of a side chain, in addition to the main chain, of an amino acid interacts with the target molecule. For an improved interaction with the target molecule, it is more advantageous that a peptide not only is composed of 20 kinds of natural amino acids but also incorporates a non-natural amino acid designed to further strengthen the interaction with the target molecule. While the methods described in PTL 1 and PTL 2 provide suggestions from the viewpoint of the N-methylation of an amide binding site of an amino acid that is a constituent unit of a peptide, the number of amino acid residues, and lipophilicity, neither PTL 1 nor PTL 2 has discussion of structural characteristics attained by introducing into an amino acid side chain a hetero atom represented by an oxygen atom or a nitrogen atom necessary for forming a hydrogen bond that is considered to be advantageous for binding to the target molecule. Although NPL 1 provides examples of a peptide drug and a cyclization method for a cyclic peptide, it does not suggest a correlation between membrane permeability and the structure of an amino acid. NPL 2 provides examples of peptide drugs, but does not suggest that structural conversion of an amino acid that constitutes a peptide is linked to usefulness as medicine. NPL 2 merely provides discussion of known compounds, and versatility as medicine is limited.

The present invention was achieved in view of the circumstances that designing a peptide compound, in particular research concerning an effect on membrane permeability, had been conducted without focusing on the structural characteristics of each amino acid. An objective of the present invention is to provide amino acids that improve the membrane permeability of peptide compounds, and peptide compounds comprising the amino acid(s).

[Solution to Problem]

The present inventors have found the following as a means for solving the above problem and completed the invention. More specifically, the present inventors have designed an amino acid side chain to have a heteroatom necessary for increasing interaction with a target molecule, and furthermore, to be capable of masking a proton present on the amino acid side chain by an intramolecular hydrogen bond within the same amino acid side chain. After having produced a peptide compound containing the amino acid to assess membrane permeability, the present inventors have surprisingly found that the peptide compound containing the amino acid has better membrane permeability than a peptide compound containing an amino acid that does not form an intramolecular hydrogen bond. The present inventors have also found that, concerning the design of an amino acid having a side chain that forms an intramolecular hydrogen bond, an effective structure is such that a proton donor and a proton acceptor can form a pseudo cyclic structure, preferably a 4-membered ring, a 5-membered ring, a 6-membered ring, or a 7-membered ring. Moreover, the present inventors have found optimum conditions for membrane permeation of such amino acid-containing peptide compounds, including the number of amino acid residues constituting the peptide, the number of *N*-substituted amino acid residues, the proportion of *N*-substituted amino acid residues contained in the peptide, the range of cLogP, and the number of aromatic rings.

In one non-limiting specific embodiment, the present invention encompasses the following:

[1] a peptide compound with two or more amino acids connected, wherein at least one of the amino acids is capable of forming a hydrogen bond in a side chain thereof;
[2] the peptide compound of [1], wherein the amino acid capable of forming a hydrogen bond in a side chain thereof is capable of forming a pseudo 4- to 7-membered ring in the side chain;
[3] the peptide compound of [1] or [2], wherein the amino acid capable of forming a hydrogen bond in a side chain thereof is represented by Formula A below:

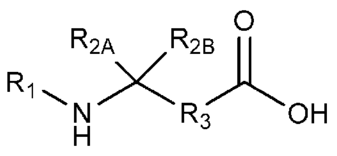
(A)

,

wherein

$R_1$ is hydrogen, $C_1$-$C_6$ alkyl, or a group represented by Formula 1 or Formula 2, and, in this case, as for $R_{2A}$ and $R_{2B}$, $R_{2A}$ is hydrogen, $C_1$-$C_6$ alkyl, or a group represented by Formula 1 or Formula 2, and $R_{2B}$ is hydrogen or $C_1$-$C_6$ alkyl, or $R_{2A}$ and $R_{2B}$ form a 4- to 6-membered ring together with the carbon atom to which they are bonded, or
$R_1$ forms a 4- to 6-membered hetero ring together with the nitrogen atom to which $R_1$ is bonded, $R_{2A}$, and the carbon atom to which $R_{2A}$ is bonded, and the hetero ring optionally has one or more substituents selected from the group consisting of a group represented by Formula 1 or Formula 2, -OH, and an alkoxy group, and, in this

case, $R_{2B}$ is hydrogen or $C_1$-$C_6$ alkyl;
$R_3$ is a single bond or -$CHR_4$-;
$R_4$ is hydrogen, $C_1$-$C_4$ alkyl, or a group represented by Formula 1 or Formula 2;
Formula 1 and Formula 2 are respectively represented by the following formulae:

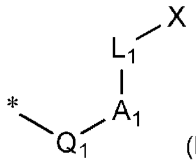 (Formla 1) 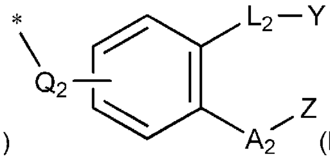 (Formula 2) ,

wherein

* indicates a point of bonding;
$Q_1$ and $Q_2$ are independently a single bond, $C_1$-$C_4$ alkylene, or $C_2$-$C_4$ heteroalkylene containing one oxygen atom;
$A_1$ is -O- or -S-;
$L_1$ is linear $C_1$-$C_3$ alkylene optionally substituted with one or more substituents selected from the group consisting of fluorine, $C_1$-$C_2$ alkyl, $C_1$-$C_2$ fluoroalkyl, and oxo (=O);
$A_2$ is a single bond, -O-, or -S-;
$L_2$ is a single bond or linear $C_1$-$C_3$ alkylene optionally substituted with one or more substituents selected from the group consisting of fluorine, $C_1$-$C_2$ alkyl, $C_1$-$C_2$ fluoroalkyl, and oxo (=O);
X is -OH, -$NR_{Z1}R_{Z2}$, -$CONR_{Z1}R_{Z2}$, or 5- to 6-membered saturated or unsaturated heterocyclyl containing 1 to 3 heteroatoms and optionally substituted with oxo or one or more halogens;
Rzi and $R_{Z2}$ are independently selected from the group consisting of hydrogen, -OH, $C_1$-$C_4$ alkyl, and $C_1$-$C_4$ alkylsulfonyl;
Y is -OH, $C_1$-$C_4$ alkylsulfonylamino, -$NR_{Z3}R_{Z4}$, -$CONR_{Z3}R_{Z4}$, or 5- to 6-membered saturated or unsaturated heterocyclyl containing 1 to 3 heteroatoms and optionally substituted with oxo or halogen;
$R_{Z3}$ and $R_{Z4}$ are independently selected from the group consisting of hydrogen, -OH, $C_1$-$C_4$ alkyl, and $C_1$-$C_4$ alkylsulfonyl; and
Z is hydrogen or $C_1$-$C_4$ alkyl,
provided that when $A_2$ is -O- or -S-, Z is not hydrogen, and
the amino acid represented by Formula A contains at least one group represented by either Formula 1 or Formula 2;

[4] the peptide compound of [3], wherein

$R_1$ is a group represented by Formula 1 or Formula 2;
$R_{2A}$ and $R_{2B}$ are independently hydrogen or $C_1$-$C_6$ alkyl, or $R_{2A}$ and $R_{2B}$ form a 4- to 6-membered ring together with the carbon atom to which they are bonded;
$R_3$ is a single bond or -$CHR_4$-; and
$R_4$ is hydrogen or $C_1$-$C_4$ alkyl;

[5] the peptide compound of [3], wherein

$R_1$ forms a 4- to 6-membered hetero ring together with the nitrogen atom to which $R_1$ is bonded, $R_{2A}$, and the carbon atom to which $R_{2A}$ is bonded, and the hetero ring has a group represented by Formula 1 or Formula 2;
$R_{2B}$ is hydrogen or $C_1$-$C_6$ alkyl;
$R_3$ is a single bond or -$CHR_4$-; and
$R_4$ is hydrogen or $C_1$-$C_4$ alkyl;

[6] the peptide compound of [3], wherein

$R_1$ is hydrogen or $C_1$-$C_6$ alkyl;
$R_{2A}$ is a group represented by Formula 1 or Formula 2;
$R_{2B}$ is hydrogen or $C_1$-$C_6$ alkyl;
$R_3$ is a single bond or -$CHR_4$-; and
$R_4$ is hydrogen or $C_1$-$C_4$ alkyl;

[7] the peptide compound of [3], wherein

$R_1$ is hydrogen or $C_1$-$C_6$ alkyl;
$R_{2A}$ and $R_{2B}$ are independently hydrogen or $C_1$-$C_6$ alkyl, or $R_{2A}$ and $R_{2B}$ form a 4- to 6-membered ring together with the carbon atom to which they are bonded;
$R_3$ is -$CHR_4$-; and
$R_4$ is a group represented by Formula 1 or Formula 2;

[8] the peptide compound of any one of [3] to [7], wherein

$Q_1$ is $C_1$-$C_4$ alkylene;
$A_1$ is -O- or -S-;
$L_1$ is linear $C_1$-$C_3$ alkylene optionally substituted with one or more substituents selected from the group consisting of a $C_1$-$C_2$ alkyl group, $C_1$-$C_2$ fluoroalkyl, and oxo (=O); and
X is -OH;

[9] the peptide compound of any one of [3] to [7], wherein

$Q_1$ is $C_1$-$C_4$ alkylene;
$A_1$ is -O- or -S-;
$L_1$ is linear $C_1$-$C_3$ alkylene optionally substituted with one or more substituents selected from the group consisting of a $C_1$-$C_2$ alkyl group, $C_1$-$C_2$ fluoroalkyl, and oxo (=O);
X is -$CONR_{Z1}R_{Z2}$;
$R_{Z1}$ is $C_1$-$C_4$ alkyl; and
$R_{Z2}$ is hydrogen;

[10] the peptide compound of any one of [3] to [7], wherein

$Q_1$ is $C_1$-$C_4$ alkylene;
$A_1$ is -O- or -S-;
$L_1$ is linear $C_1$-$C_3$ alkylene optionally substituted with one or more substituents selected from the group consisting of a $C_1$-$C_2$ alkyl group, $C_1$-$C_2$ fluoroalkyl, and oxo (=O); and
X is 5- to 6-membered saturated or unsaturated heterocyclyl containing 1 to 3 heteroatoms and optionally substituted with oxo or one or more halogens;

[11] the peptide compound of any one of [3] to [7], wherein

$Q_1$ is a single bond;
$A_1$ is -O- or -S-;
$L_1$ is linear $C_1$-$C_3$ alkylene optionally substituted with one or more substituents selected from the group consisting of a $C_1$-$C_2$ alkyl group, $C_1$-$C_2$ fluoroalkyl, and oxo (=O); and X is -OH;

[12] the peptide compound of any one of [3] to [11], wherein $Q_1$ is $CH_2$-;
[13] the peptide compound of any one of [3] to [12], wherein $A_1$ is -O-;
[14] the peptide compound of any one of [3] to [13], wherein $L_1$ is selected from the group consisting of -$CH_2$-, -$(CH_2)_2$-, -$(CH_2)_3$-, -$CH_2CH(CH_3)$-, -$CH_2CH(CF_3)$-, -$CH_2C(CH_3)_2$-,-$(CH_2)_2CH(CH_3)$-, -$CH_2C(CH_3)_2CH_2$-, -$(CH_2)_2C(CH_3)_2$-, and -$CH_2CO$-;
[15] the peptide compound of any one of [3] to [7], wherein

$Q_2$ is $C_1$-$C_4$ alkylene;
$L_2$ is a single bond;
Y is -$CONR_{Z3}R_{Z4}$;
$A_2$ is -O- or -S-; and
Z is $C_1$-$C_4$ alkyl;

[16] the peptide compound of any one of [3] to [7], wherein

$Q_2$ is $C_1$-$C_4$ alkylene;

$L_2$ is a single bond;
Y is $-CONR_{Z3}R_{Z4}$;
$A_2$ is a single bond; and
Z is hydrogen;

[17] the peptide compound of any one of [3] to [7], wherein

$Q_2$ is $C_1$-$C_4$ alkylene;
$L_2$ is a single bond;
Y is $C_1$-$C_4$ alkylsulfonylaminocarbonyl;
$A_2$ is -O- or -S-; and
Z is $C_1$-$C_4$ alkyl;

[18] the peptide compound of any one of [3] to [7], wherein

$Q_2$ is $C_1$-$C_4$ alkylene;
$L_2$ is a single bond;
Y is -OH;
$A_2$ is -O- or -S-; and
Z is $C_1$-$C_4$ alkyl;

[19] the peptide compound of any one of [3] to [7] and [16] to [18], wherein $Q_2$ is $-CH_2-$;
[20] the peptide compound of any one of [3] to [7], [15], and [19], wherein Y is methylaminocarbonyl, -CON(OH)Me, or -CONH(OH);
[21] the peptide compound of any one of [3] to [7], [16], and [19], wherein Y is -CON(OH)Me;
[22] the peptide compound of any one of [3] to [7], [15], and [17] to [21], wherein $A_2$ is -O-;
[23] the peptide compound of any one of [3] to [7], [15], and [17] to [22], wherein Z is methyl;
[24] the peptide compound of [3], wherein the amino acid represented by Formula A is selected from the group consisting of:

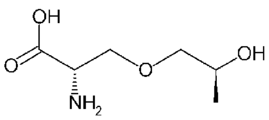 、 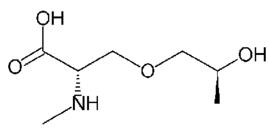 、 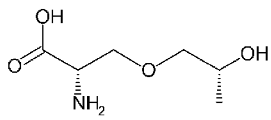 、

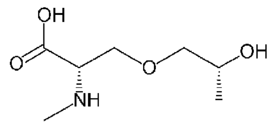 、 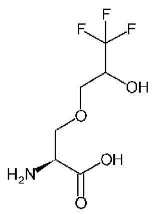 、 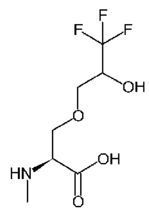 、 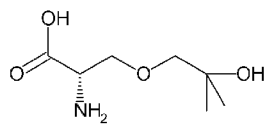 、

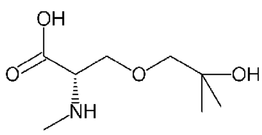 、 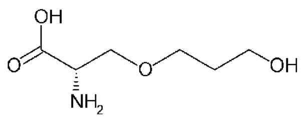 、 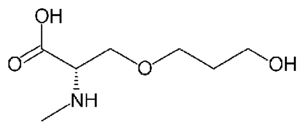 、

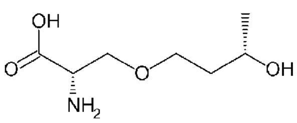 、 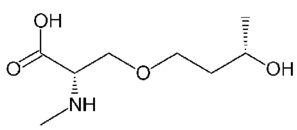 、 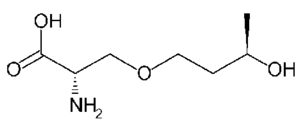 、

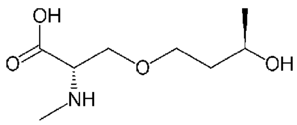
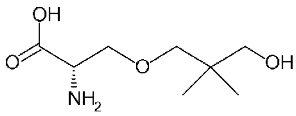
,
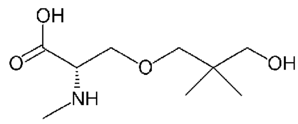
,

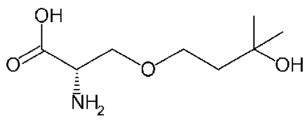
,
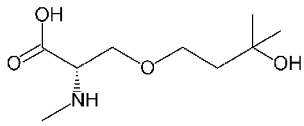
,
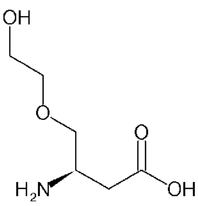
,

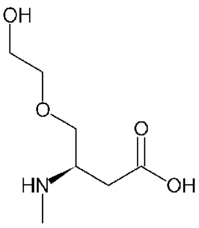
,
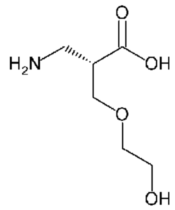
,
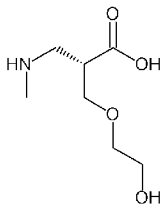
,
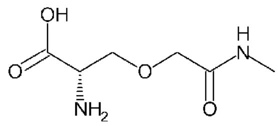
,

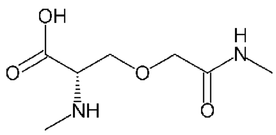
,
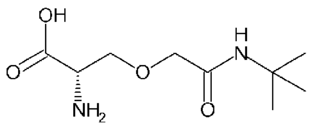
,
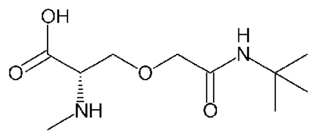
,

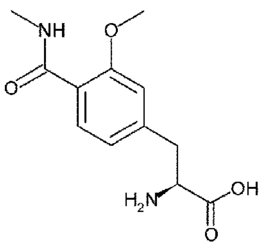
,
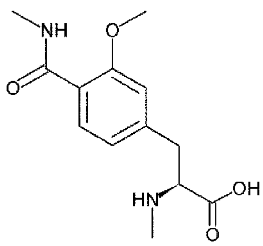
,
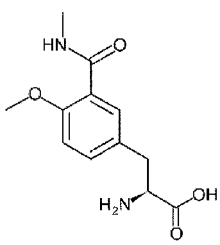
,
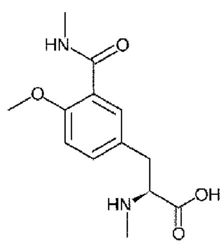
,

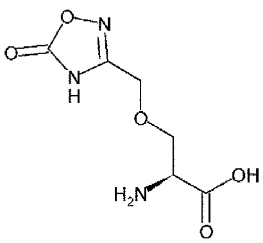
,
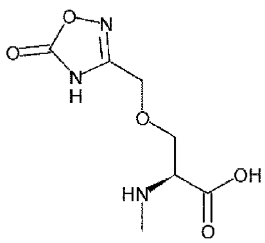
,
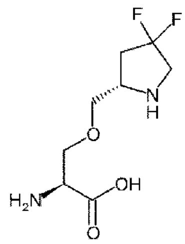
,
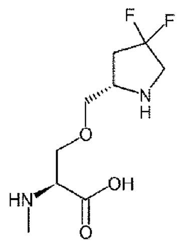
,

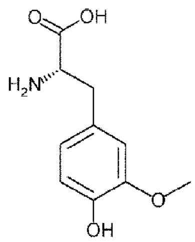、
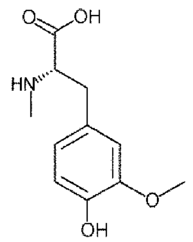、
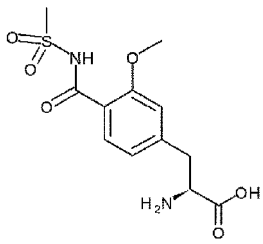、
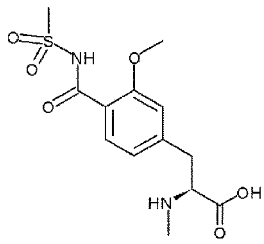

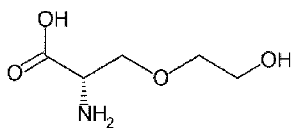、
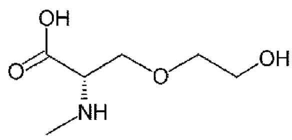、

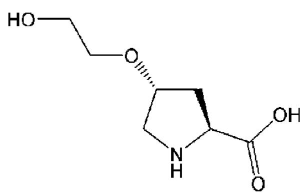、
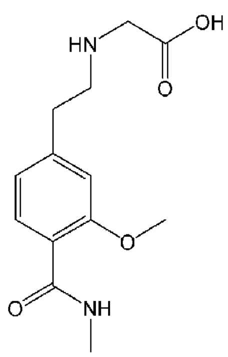、
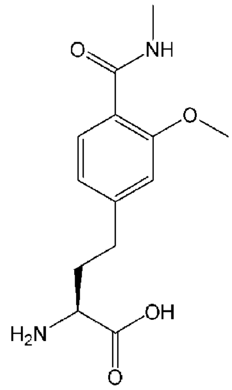、
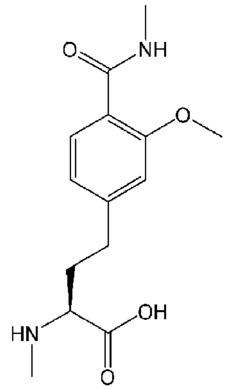、

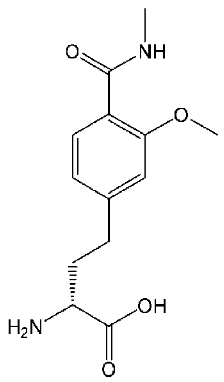、
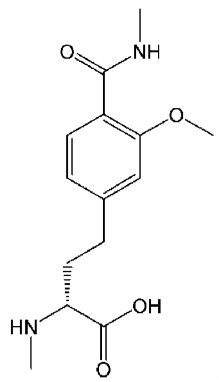、
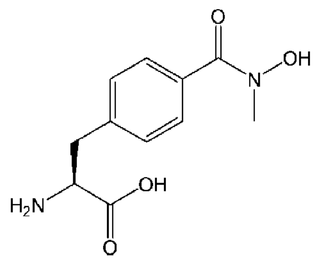、
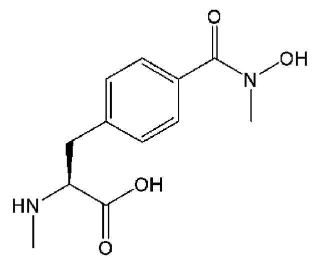、

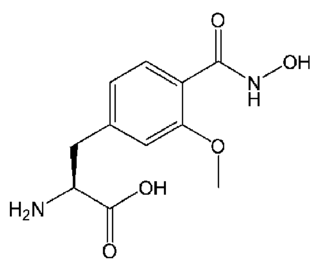、
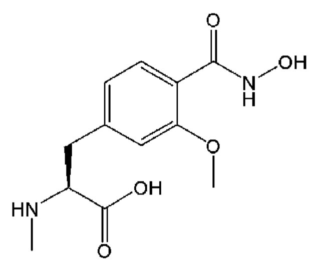、
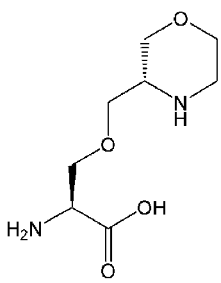、

and

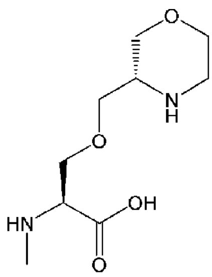

;

[25] the peptide compound of any one of [1] to [24], which is composed of 5 to 30 amino acids;
[26] the peptide compound of any one of [1] to [25], which is cyclic;
[27] the peptide compound of [26], comprising a cyclic portion composed of 2 to 15 amino acids;
[28] the peptide compound of any one of [1] to [27], comprising 2 to 30 *N*-substituted amino acids;
[29] the peptide compound of any one of [1] to [28], wherein a proportion of the number of *N*-substituted amino acids to the total number of amino acids is 30% or higher;
[30] the peptide compound of any one of [1] to [29], which has a ClogP of 4.0 to 18;
[31] the peptide compound of any one of [1] to [30], which has a $P_{app}$ of 1.0 x $10^{-7}$ cm/sec or higher;
[32] a library comprising the peptide of any one of [1] to [31] and/or a nucleic acid encoding the peptide;
[33] an amino acid represented by the Formula A of any one of [3] to [23];
[34] the amino acid of [33], which is selected from the group consisting of:

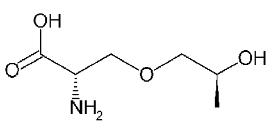 、 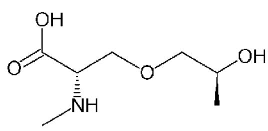 、 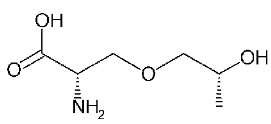 、

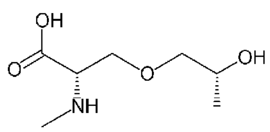 、 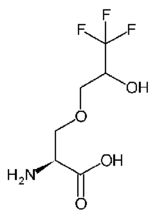 、 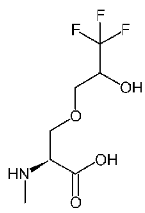 、 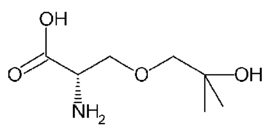 、

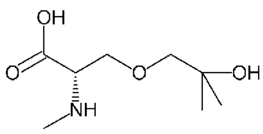 、 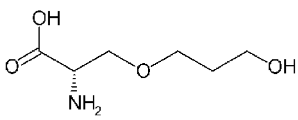 、 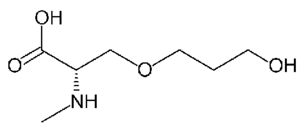 、

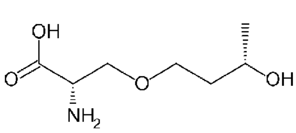 、 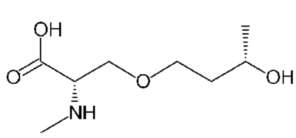 、 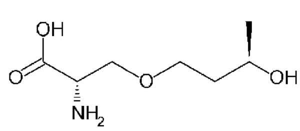 、

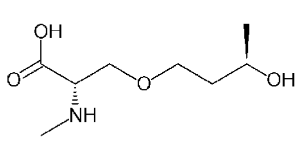 、 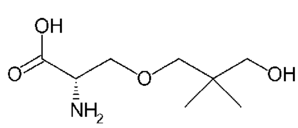 、 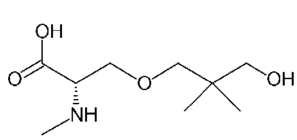 、

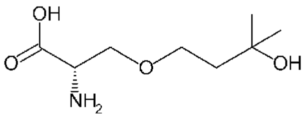,
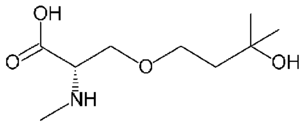,
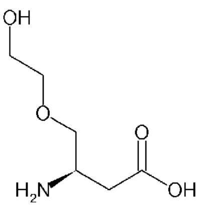,
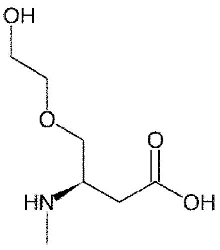,
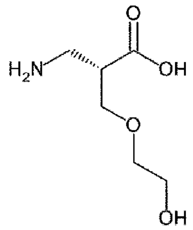,
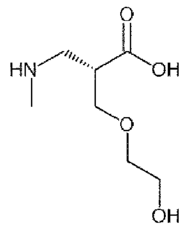,
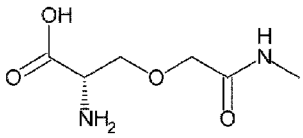,
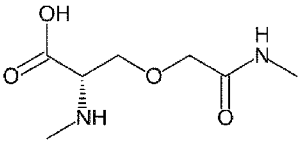,
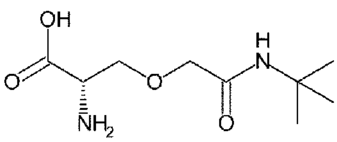,
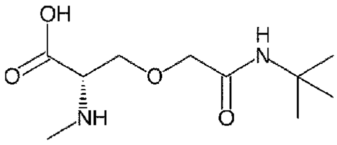,
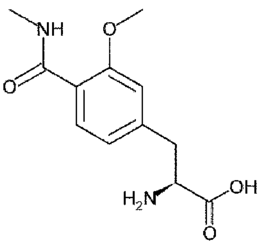,
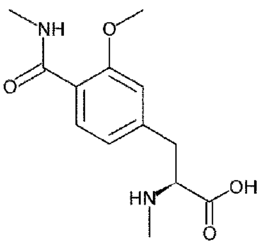,
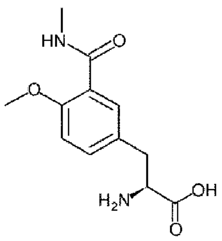,
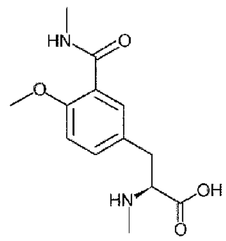,
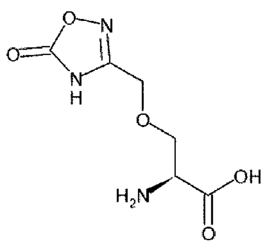,
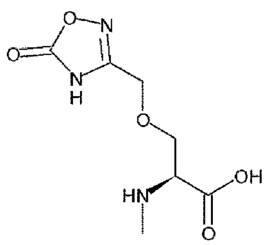,
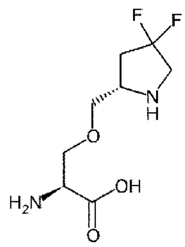,
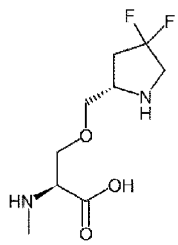,
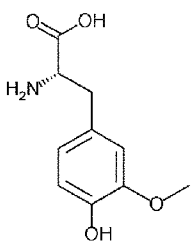,
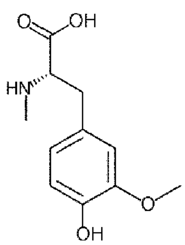,
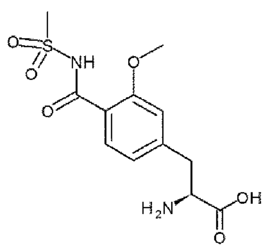,
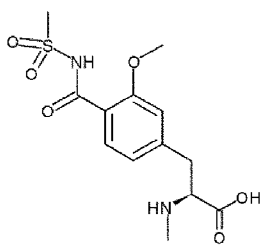

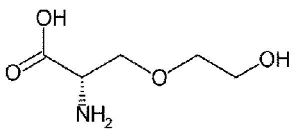 、 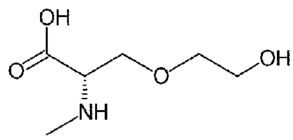 、

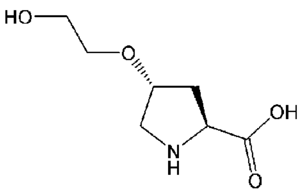 、 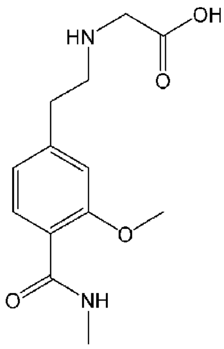 、 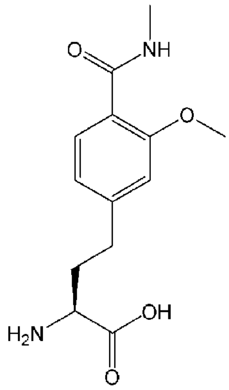 、 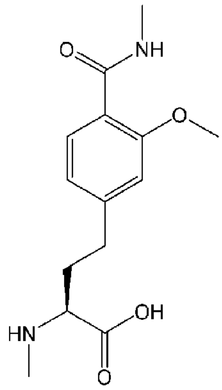 、

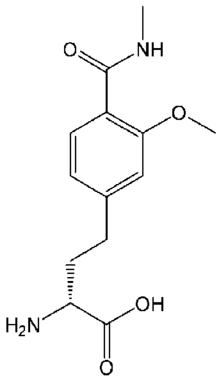 、 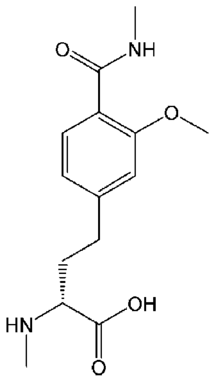 、 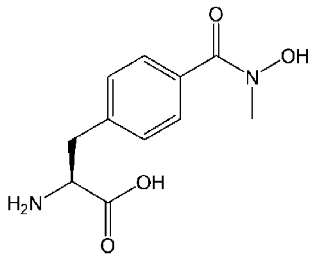 、 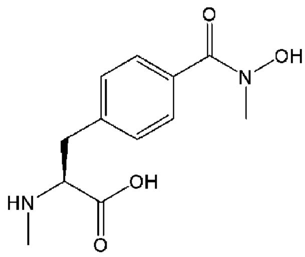 、

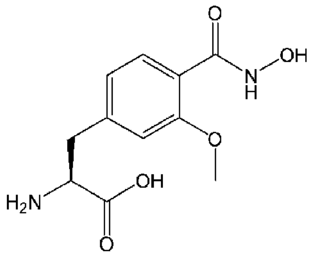 、 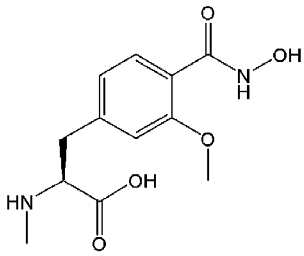 、 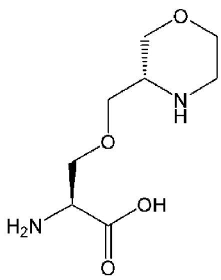 、

and

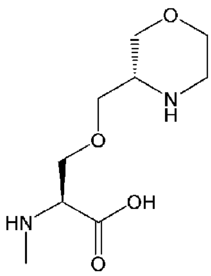 ;

[35] a protected amino acid, wherein an amino group and/or a carboxyl group contained in the amino acid of [33]

or [34] is protected by a protecting group; and
[36] the protected amino acid of [35], wherein

the protecting group for the amino group is selected from the group consisting of an Fmoc group, a Boc group, a Cbz group, an Alloc group, a nosyl group, a dinitronosyl group, a t-Bu group, a trityl group, and a cumyl group, and/or
the protecting group for the carboxyl group is selected from the group consisting of a methyl group, an allyl group, a t-Bu group, a trityl group, a cumyl group, a methoxytrityl group, and a benzyl group.

[Advantageous Effects of Invention]

The present invention makes it possible to search for peptide drugs, specifically amino acids that have excellent membrane permeability and specifically bind to a target molecule and peptides containing the amino acid(s), and to provide the amino acids and peptides.

[Brief Description of Drawings]

Fig. 1-1 is a graph showing the correlation between ClogP/total AA and $P_{app}$ using a population of cyclic peptide compounds not having a Trp side chain.
Fig. 1-2 is a graph showing the correlation between ClogP/total AA and $P_{app}$ using a population of cyclic peptide compounds having a Trp side chain.
Fig. 2 is a graph showing the correlation between ARC and cell membrane permeability of cyclic peptide compounds.
Fig. 3-1 is a drawing showing the potential energy surface in, for example, the structure of the side-chain moiety of Ser(NMe-Aca).
Fig. 3-2 is a drawing showing the conformational distribution of crystal structures in, for example, the structure of the side-chain moiety of Ser(NMe-Aca) using the result of X-ray structural analysis registered in the CSD.
Fig. 4-1 is a drawing showing the potential energy surface in, for example, the structure of the side chain moiety of Ser(EtOH), bAla(3R-MeOEtOH), and bAla(2S-MeOEtOH).
Fig. 4-2 is a drawing showing the conformational distribution of crystal structures in, for example, the structures of the side-chain moieties of Ser(EtOH), bAla(3R-MeOEtOH) and bAla(2S-MeOEtOH) using the result of X-ray structural analysis registered in the CSD.

[Description of Embodiments]

Preferred non-limiting embodiments herein are described below.

It is intended that all elements described in the Examples set forth later will naturally be deemed as also being equally described in this "Description of Embodiments" without being bound by any limitation of patent practice, custom, law, and the like by which one could attempt to interpret what is described in the Examples in a limited manner in countries where patent protection is sought by the present patent application.
It is intended, and is to be naturally understood by persons with ordinary skill in the art, that any combinations of some or all of one or more elements described anywhere herein are included in the present specification as long as they are not technically contradictory based on the common technical knowledge of the skilled persons.
The following abbreviations are used herein: Ala (alanine), Arg (arginine), Asn (asparagine), Asp (aspartic acid), Cys (cysteine), Glu (glutamic acid), Gin (glutamine), Gly (glycine), His (histidine), Ile (isoleucine), Leu (leucine), Lys (lysine), Met (methionine), Phe (phenylalanine), Pro (proline), Ser (serine), Thr (threonine), Trp (tryptophan), Tyr (tyrosine), and Val (valine). In addition to these, the abbreviations set forth in the later-described abbreviation tables and Table 1 are used.

(Definitions of functional groups and such)

The term "alkyl" as used herein refers to a monovalent group derived by removing any one hydrogen atom from an aliphatic hydrocarbon, and covers a subset of hydrocarbyl or hydrocarbon group structures that contain hydrogen and carbon atoms, but do not contain a heteroatom (which refers to an atom other than carbon and hydrogen atoms) or an unsaturated carbon-carbon bond in the skeleton. The alkyl groups include linear or branched groups. The alkyl group is an alkyl group having 1 to 20 carbon atoms ($C_1$-$C_{20}$; hereinafter, "$C_p$-$C_q$" means that it has p to q carbon atoms),

preferred examples of which include a $C_1$-$C_6$ alkyl group, a $C_1$-$C_5$ alkyl group, a $C_1$-$C_4$ alkyl group, and a $C_1$-$C_3$ alkyl group. Specific examples of the alkyl include methyl, ethyl, propyl, butyl, pentyl, hexyl, isopropyl, tert-butyl, sec-butyl, 1-methylpropyl, 1,1-dimethylpropyl, 2,2-dimethylpropyl, 1,2-dimethylpropyl, 1,1,2-trimethylpropyl, 1,2,2-trimethylpropyl, 1,1,2,2-tetramethylpropyl, 1-methylbutyl, 2-methylbutyl, 3-methylbutyl, 1,1-dimethylbutyl, 1,2-dimethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, isopentyl, and neopentyl.

The term "heteroalkyl" as used herein means a group containing preferably 1 to 5 heteroatoms in the backbone of the "alkyl", and preferably include $C_2$-$C_{10}$ heteroalkyl and $C_2$-$C_6$ heteroalkyl. Specific examples of heteroalkyl include $-CH_2OCH_3$, $-CH_2OCH_2CH_3$,$-CH(CH_3)OCH_3$, and $-CH_2CH_2N(CH_3)_2$.

The term "halogen atom" as used herein include F, Cl, Br, and I, and preferred examples include F and Cl.

The term "fluoroalkyl" as used herein means a group obtained by substituting one or more hydrogen atoms of the "alkyl" with fluorine atoms, and preferably includes $C_1$-$C_6$ fluoroalkyl, $C_1$-$C_4$ fluoroalkyl, and $C_1$-$C_2$ fluoroalkyl. Specific examples of fluoroalkyl include a trifluoromethyl group, a 2,2,2-trifluoroethyl group, a pentafluoroethyl group, a 2,2,3,3-tetrafluoropropyl group, a heptafluoropropyl group, a trifluoromethoxy group, a 2,2,2-trifluoroethoxy group, a pentafluoroethoxy group, a 2,2,3,3-tetrafluoropropoxy group, and a heptafluoropropoxy group.

The term "alkylsulfonyl" as used herein means a sulfonyl group to which the "alkyl" is bonded (*i.e.,* $-SO_2$-alkyl). Preferred examples of alkylsulfonyl include $C_1$-$C_6$ alkylsulfonyl and $C_1$-$C_4$ alkylsulfonyl, and specific examples include methylsulfonyl, ethylsulfonyl, n-propylsulfonyl, and i-propylsulfonyl.

The term "alkylsulfonylamino" as used herein means a group obtained by substituting one hydrogen atom of an amino group ($-NH_2$) with the "alkylsulfonyl". Preferred examples of alkylsulfonylamino include $C_1$-$C_6$ alkylsulfonylamino and $C_1$-$C_4$ alkylsulfonylamino, and specific examples include methylsulfonylamino, ethylsulfonylamino, n-propylsulfonylamino, and i-propylsulfonylamino.

The term "alkenyl group" as used herein refers to a monovalent group having at least one double bond (two adjacent $SP_2$ carbon atoms). Depending on the configuration of a double bond and a substituent (if present), the geometry of the double bond can be an entgegen (E) or zuzammen (Z) configuration or a cis or trans configuration. Examples of the alkenyl group include linear or branched ones, including linear ones including internal olefins. Preferred examples include $C_2$-$C_{10}$ alkenyl group, with $C_2$-$C_6$ alkenyl group being more preferred. Specific examples of such alkenyl include vinyl group, allyl group, 1-propenyl group, 2-propenyl group, 1-butenyl group, 2-butenyl group (including cis and trans), 3-butenyl group, pentenyl group, and hexenyl group.

The term "alkynyl" as used herein refers to a monovalent group having at least one triple bond (two adjacent SP carbon atoms). Examples include linear or branched alkynyl groups, including internal alkylenes. Preferred examples include $C_2$-$C_{10}$ alkynyl group, with $C_2$-$C_6$ alkynyl group being more preferred. Specific examples of the alkynyl include ethynyl group, 1-propynyl group, propargyl group, 3-butynyl group, pentynyl group, and hexynyl group.

The term "cycloalkyl" as used herein refers to a saturated or partially saturated cyclic monovalent aliphatic hydrocarbon group, including single rings, bicyclo rings, and spiro rings. Preferred examples include $C_3$-$C_{10}$ cycloalkyl. The cycloalkyl group may be partially unsaturated. Specific examples of the cycloalkyl include cyclopropyl, cyclobutyl, cyclopentyl, cyclohexyl, cycloheptyl, cyclooctyl, and bicyclo[2.2.1]heptyl.

The term "aryl" as used herein refers to a monovalent aromatic hydrocarbon ring, preferred examples of which include $C_6$-$C_{10}$ aryl. Specific examples of the aryl include phenyl and naphthyl.

The term "heteroaryl" as used herein refers to a monovalent aromatic ring group containing preferably 1 to 5 heteroatoms in the ring-forming atoms (herein also called "in the ring"), which may be partially saturated. The ring may be a single ring or two fused rings (such as bicyclic heteroaryl in which heteroaryl is fused with benzene or monocyclic heteroaryl). The number of the ring-forming atoms is preferably 5 to 10 (5- to 10-membered heteroaryl). Specific examples of the heteroaryl include imidazolyl, thiazolyl, oxadiazolyl, thiadiazolyl, oxadiazolonyl, thiadiazolonyl, tetrazolyl, pyridyl, and indolyl.

The term "having a heteroatom in the ring" as used herein means that the atoms constituting the ring include a heteroatom(s), and examples of such rings include aromatic hetero rings such as pyridine and non-aromatic hetero rings such as piperidine, morpholine, pyrrolidine, and azetidine. When the heteroatom is an oxygen atom, it is expressed as "having an oxygen atom in the ring" or the like.

The term "heterocyclic group" as used herein means a group having at least one heteroatom (such as N, O, or S) in the ring, and is also referred to as a saturated heterocyclyl group or an unsaturated heterocyclyl group. The heteroatom is preferably N or O, the number of heteroatoms is preferably 1 or 2, and a 4- to 6-membered ring is preferable. The heterocyclic group may be substituted with an alkyl group, a fluoroalkyl group, oxo, or a halogen atom. Preferred examples of the heterocyclic group include a pyridyl group, a piperidino group, a morpholino group, a pyrrolidino group, an oxadiazolonyl group, and an azetidinyl group.

The term "arylalkyl (aralkyl)" as used herein refers to a group containing both aryl and alkyl, for example, a group in which at least one hydrogen atom of the alkyl is replaced with aryl, preferred examples of which include "$C_5$-$C_{10}$ aryl-$C_1$-$C_6$ alkyl." Examples include benzyl.

The term "alkylene" as used herein refers to a divalent group derived by removing any one hydrogen atom from the "alkyl." Preferred examples of the alkylene include $C_1$-$C_2$ alkylene, $C_1$-$C_3$ alkylene, $C_1$-$C_4$ alkylene, $C_1$-$C_5$ alkylene, and $C_1$-$C_6$ alkylene. Specific examples of the alkylene include $-CH_2-$, $-(CH_2)_2-$, $-(CH_2)_3-$, $CH(CH_3)CH_2-$, $-C(CH_3)_2-$, $-(CH_2)_4-$, $CH(CH_3)CH_2CH_2-$, $-C(CH_3)_2CH_2-$, $-CH_2CH(CH_3)CH_2-$, $-CH_2C(CH_3)_2-$, $-CH_2CH_2CH(CH_3)-$,$-(CH_2)_5-$, and $-(CH_2)_6-$.

The term "heteroalkylene" as used herein means a divalent group derived from further removing any one hydrogen atom from the "heteroalkyl", and examples include $C_2$-$C_6$ heteroalkylene, $C_2$-$C_5$ heteroalkylene, $C_2$-$C_4$ heteroalkylene, $C_2$-$C_3$ heteroalkylene, and $C_2$ heteroalkylene. For example, for heteroalkylene containing an oxygen atom as a heteroatom in the group, specific examples include $-CH_2O-$, $-OCH_2-$, $-CH_2OCH_2-$, $-OCH_2CH_2-$, and-$CH_2CH_2O-$.

The term "arylene" as used herein refers to a divalent group derived by further removing any one hydrogen atom from the aryl. The arylene may be a single ring or fused rings. The number of the ring-forming atoms is not particularly limited, but is preferably 6 to 10 ($C_6$-$C_{10}$ arylene). Specific examples of the arylene include phenylene.

The term "heteroarylene" as used herein refers to a divalent group derived by further removing any one hydrogen atom from the heteroaryl. The heteroarylene may be a single ring or fused rings. The number of the ring-forming atoms is not particularly limited, but is preferably 5 to 10 (5- to 10-membered heteroarylene). Specific examples of the heteroarylene include imidazolediyl, pyridinediyl, oxadiazolediyl, thiazolediyl and thiadiazolediyl.

The term "fused (condensed) ring structure" as used herein refers to a cyclic structure in which in a cyclic compound having two or more rings, a plurality of rings share two or more atoms. A "fused ring structure composed of two or more aromatic rings" refers to a cyclic structure in which in a cyclic compound having two or more aromatic rings, a plurality of aromatic rings share two or more atoms. Examples of the fused ring structure include, but are not limited to, an indole skeleton, a benzofuran skeleton, a benzimidazole skeleton, a quinoline skeleton, and bicyclo[4.4.0]decane.

Herein, when the modifying phrase "optionally substituted" is added, examples of the substituent include an alkyl group, a fluoroalkyl group, an alkoxy group, a fluoroalkoxy group, an alkenyl group, an alkenyloxy group, an alkynyl group, an alkynyloxy group, a cycloalkyl group, an aryl group, a heteroaryl group, a heterocyclyl group, an arylalkyl group, a heteroarylalkyl group, a halogen atom, a nitro group, an amino group, a monoalkylamino group, a dialkylamino group, a cyano group, a carboxyl group, an alkoxycarbonyl group, and a formyl group.

The term "hetero ring" as used herein means a non-aromatic monovalent or divalent hetero ring in which atoms constituting the ring include preferably 1 to 5 heteroatoms. The hetero ring may have double and/or triple bonds in the ring, carbon atoms in the ring may be oxidized to form carbonyl, and the hetero ring may be a monocyclic ring, a fused ring, or a spiro ring. The number of atoms constituting the ring is preferably 3 to 12 (a 3- to 12-membered hetero ring), more preferably 4 to 7 (a 4- to 7-membered hetero ring), and even more preferably 5 to 6 (a 5- to 6-membered hetero ring). Specific examples of the hetero ring include piperazine, pyrrolidine, piperidine, morpholine, homomorpholine, (R)-hexahydropyrrolo[1,2-a]pyrazine, (S)-hexahydropyrrolo[1,2-a]pyrazine, 3-oxopiperazine, 2-oxopyrrolidine, azetidine, 2-oxoimidazolidine, oxetane, dihydrofuran, tetrahydrofuran, dihydropyran, tetrahydropyran, tetrahydropyridine, thiomorpholine, pyrazolidine, imidazoline, oxazolidine, isooxazolidine, thiazolidine, imidazolidine, isothiazolidine, thiadiazolidin, oxazolidone, benzodioxane, benzoxazoline, dioxolane, dioxane, and tetrahydrothiopyran.

(Peptide compounds)

In one aspect, the present invention relates to a peptide compound in which two or more amino acids are connected, wherein at least one (specifically, for example, 1, 2, 3, 4, or more) of the amino acids is capable of forming a hydrogen bond in a side chain thereof.

Structures of peptide compounds

The "peptide compound" in the present invention includes a linear or cyclic peptide compound with two or more amino acids connected. The cyclic peptide compound has the same meaning as a "peptide compound having a cyclic portion".

The term "amino acid" as used herein includes natural and unnatural amino acids. The term "natural amino acid" as used herein refers to Gly, Ala, Ser, Thr, Val, Leu, Ile, Phe, Tyr, Trp, His, Glu, Asp, Gin, Asn, Cys, Met, Lys, Arg, or Pro. Examples of the unnatural amino acid include, but are not particularly limited to, β-amino acids, D-amino acids, *N*-substituted amino acids, α,α-disubstituted amino acids, amino acids having side chains that are different from those of natural amino acids, and hydroxycarboxylic acids. Amino acids herein may have any conformation. There is no particular limitation on the selection of amino acid side chain, but in addition to a hydrogen atom, it can be freely selected from, for example, an alkyl group, an alkenyl group, an alkynyl group, an aryl group, a heteroaryl group, an aralkyl group, a cycloalkyl group, and a spiro-bonded cycloalkyl group. Each group may have a substituent, and there are no limitations on the substituent. For example, one, or two or more substituents may be freely and independently selected from any substituents including a halogen atom, an O atom, an S atom, an N atom, a B atom, an Si atom, or a P atom. Examples

include an optionally substituted alkyl group, alkenyl group, alkynyl group, aryl group, heteroaryl group, aralkyl group, and cycloalkyl group. Amino acids herein may have a side chain "that can form an intramolecular hydrogen bond" described below. In a non-limiting embodiment, amino acids herein may be compounds having a carboxy group and an amino group in the same molecule (even in this case, imino acids such as proline and hydroxyproline are also included in amino acids).

Substituents derived from halogen include fluoro (-F), chloro (-Cl), bromo (-Br), and iodo (-I).

Substituents derived from an O atom include hydroxyl (-OH), oxy (-OR), carbonyl (-C=O-R), carboxyl ($-CO_2H$), oxycarbonyl (-C=O-OR), carbonyloxy (-O-C=O-R), thiocarbonyl (-C=O-SR), carbonylthio group (-S-C=O-R), aminocarbonyl (-C=O-NHR), carbonylamino (-NH-C=O-R), oxycarbonylamino (-NH-C=O-OR), sulfonylamino ($-NH-SO_2-R$), aminosulfonyl ($-SO_2-NHR$), sulfamoylamino ($-NH-SO_2-NHR$), thiocarboxyl (-C(=O)-SH), and carboxylcarbonyl ($-C(=O)-CO_2H$).

Examples of oxy (-OR) include alkoxy, cycloalkoxy, alkenyloxy, alkynyloxy, aryloxy, heteroaryloxy, and aralkyloxy.

Examples of carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.
(-C=O-OR)

Examples of carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C=O-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. Additional examples include compounds in which the H atom bonded to the N atom in -C=O-NHR is further replaced with alkyl, cycloalkyl, alkenyl, alkynyl, aryl, heteroaryl, or aralkyl.

Examples of carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Additional examples include compounds in which the H atom bonded to the N atom in -NH-C=O-R is further replaced with alkyl, cycloalkyl, alkenyl, alkynyl, aryl, heteroaryl, or aralkyl.

Examples of oxycarbonylamino (-NH-C=O-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Additional examples include compounds in which the H atom bonded to the N atom in -NH-C=O-OR is further replaced with alkyl, cycloalkyl, alkenyl, alkynyl, aryl, heteroaryl, or aralkyl.

Examples of sulfonylamino ($-NH-SO_2-R$) include alkylsulfonylamino, cycloalkylsulfonylamino, alkenylsulfonylamino, alkynylsulfonylamino, arylsulfonylamino, heteroarylsulfonylamino, and aralkylsulfonylamino. Additional examples include compounds in which the H atom attached to the N atom in $-NH-SO_2-R$ is further replaced with alkyl, cycloalkyl, alkenyl, alkynyl, aryl, heteroaryl, or aralkyl.

Examples of aminosulfonyl ($-SO_2-NHR$) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Additional examples include compounds in which the H atom attached to the N atom in $-SO_2-NHR$ is further replaced with alkyl, cycloalkyl, alkenyl, alkynyl, aryl, heteroaryl, or aralkyl.

Examples of sulfamoylamino ($-NH-SO_2-NHR$) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. The two H atoms bonded to the N atoms in $-NH-SO_2-NHR$ may be further replaced with substituents independently selected from the group consisting of alkyl, cycloalkyl, alkenyl, alkynyl, aryl, heteroaryl, and aralkyl, and these two substituents may form a ring.

Substituents derived from an S atom include thiol (-SH), thio (-S-R), sulfinyl (-S=O-R), sulfonyl ($-S(O)_2-R$), and sulfo ($-SO_3H$).

Examples of thio (-S-R) are selected from alkylthio, cycloalkylthio, alkenylthio, alkynylthio, arylthio, heteroarylthio, aralkylthio, and such.

Examples of sulfinyl (-S=O-R) include alkylfulfinyl, cycloalkylsulfinyl, alkenylsulfinyl, alkynylsulfinyl, arylsulfinyl, heteroarylsulfinyl, and aralkylsulfinyl.

Examples of sulfonyl ($-S(O)_2-R$) include alkylsulfonyl, cycloalkylsulfonyl, alkenylsulfonyl, alkynylsulfonyl, arylsulfonyl, heteroarylsulfonyl, and aralkylsulfonyl.

Substituents derived from an N atom include azido ($-N_3$, also called "azido group"), cyano (-CN), primary amino

(-$NH_2$), secondary amino (-NH-R), tertiary amino (-NR(R')), amidino (-C(=NH)-$NH_2$), substituted amidino (-C(=NR)-NR'R"), guanidino (-NH-C(=NH)-$NH_2$), substituted guanidino (-NR-C(=NR'")-NR'R"), and aminocarbonylamino (-NR-CO-NR'R").

Examples of secondary amino (-NH-R) include alkylamino, cycloalkylamino, alkenylamino, alkynylamino, arylamino, heteroarylamino, and aralkylamino.

Examples of tertiary amino (-NR(R')) include amino groups having any two substituents each independently selected from alkyl, cycloalkyl, alkenyl, alkynyl, aryl, heteroaryl, aralkyl and such, such as alkyl(aralkyl)amino, where any two such substituents may form a ring.

Examples of substituted amidino (-C(=NR)-NR'R") include groups in which three substituents R, R', and R" on the N atom are each independently selected from alkyl, cycloalkyl, alkenyl, alkynyl, aryl, heteroaryl, and aralkyl, such as alkyl(aralkyl)(aryl)amidino.

Examples of substituted guanidino (-NR-C(=NR'")-NR'R") include groups in which R, R', R", and R'" are each independently selected from alkyl, cycloalkyl, alkenyl, alkynyl, aryl, heteroaryl, and aralkyl, or groups in which these substituents form a ring.

Examples of aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, or groups in which these substituents form a ring.

Examples of B atom-derived substituents include boryl (-BR(R')) and dioxyboryl (-B(OR)(OR')). These two substituents, R and R', are each independently selected from alkyl, cycloalkyl, alkenyl, alkynyl, aryl, heteroaryl, and aralkyl; or they may form a ring.

In general, an amino acid means a molecule having one or more amino groups and one or more carboxyl groups in the molecule, and herein, hydroxycarboxylic acid having a hydroxyl group and a carboxyl group in the molecule can also be included in the amino acid of the present invention. Herein, hydroxycarboxylic acid may also be referred to as hydroxyamino acid.

$\alpha$-Amino acid means an amino acid in which the amino group and the carboxyl group in the amino acid molecule are attached to the same carbon, and a substituent on the carbon is referred to as a side chain of the amino acid. A series of moieties including the amino group, the carboxyl group, and the carbon to which they are attached in the amino acid molecule is referred to as a main chain of the amino acid.

The main-chain amino group of an amino acid may be unsubstituted (an $NH_2$ group) or substituted (*i.e.,* an -NHR group: R represents an optionally substituted alkyl, alkenyl, alkynyl, aryl, heteroaryl, aralkyl, or cycloalkyl, and a carbon chain bonded to the N atom and the carbon atom at $\alpha$-position may form a ring, such as proline). Such an amino acid in which main-chain amino group is substituted is referred to as "*N*-substituted amino acid" herein. Preferred examples of the "*N*-substituted amino acid" herein include, but are not limited to, *N*-alkyl amino acid, *N*-$C_1$-$C_6$ alkyl amino acid, *N*-$C_1$-$C_4$ alkyl amino acid, *N*-methyl amino acid, and *N*-substituted amino acid having a side chain that is "capable of forming an intramolecular hydrogen bond".

The "amino acid" constituting the peptide compound herein includes corresponding all isotopes. In an isotope of an "amino acid", at least one atom is substituted with an atom of the same atomic number (number of protons) and different mass number (total number of protons and neutrons). Examples of the isotope contained in the "amino acid" constituting the peptide compound of the present invention include a hydrogen atom, a carbon atom, a nitrogen atom, an oxygen atom, a phosphorus atom, a sulfur atom, a fluorine atom, and a chlorine atom, including $^{2}H$, $^{3}H$, $^{13}C$, $^{14}C$, $^{15}N$, $^{17}O$, $^{18}O$, $^{32}P$, $^{31}S$, $^{18}F$, and $^{36}Cl$, respectively.

The "linear peptide compound" in the present invention is a compound formed by connecting natural amino acids and/or non-natural amino acids by way of an amide bond or an ester bond, and is not particularly limited as long as it is a compound not having a cyclic portion. The total number of natural amino acids or non-natural amino acids constituting the linear peptide compound may be 1, 2, 3, 4, 5, 6, 7, 8, 9, 10, 11, 12, 13, 14, 15, 20, 25, or 30, and a preferred range is 6 to 20, 7 to 19, 7 to 18, 7 to 17, 7 to 16, 7 to 15, 8 to 14, and 9 to 13.

The "cyclic peptide compound" in the present invention is a compound formed by connecting natural amino acids and/or non-natural amino acids by way of an amide bond or an ester bond, and is not particularly limited as long as it is a compound having a cyclic portion. The total number of natural amino acids or non-natural amino acids constituting the cyclic peptide compound may be 1, 2, 3, 4, 5, 6, 7, 8, 9, 10, 11, 12, 13, 14, 15, 20, 25, or 30, and a preferred range is 6 to 20, 7 to 19, 7 to 18, 7 to 17, 7 to 16, 7 to 15, 8 to 14, and 9 to 13.

Herein, the "cyclic portion" of a peptide compound refers to a cyclic portion formed by linking two or more amino acid residues to each other. Furthermore, herein, the "linear portion" used to refer to a partial structure of a cyclic peptide compound refers to a portion which is not contained in the main chain structure of a cyclic portion and which has at least one amide bond and/or at least one ester bond on the chain of the portion.

Examples of the number of amino acids constituting the cyclic portion of a cyclic peptide compound herein include, but are not limited to, 2 or more, 3 or more, 4 or more, 6 or more, 7 or more, 8 or more, 9 or more, 10 or more, 11 or more, 12 or more, 30 or less, 20 or less, 18 or less, 16 or less, 15 or less, 14 or less, 13 or less, 12 or less, 11 or

less, 7, 8, 9, 10, 11, 12, 13, 14, 15, and 16. In order to achieve both membrane permeability and metabolic stability, the number of amino acids constituting the cyclic portion is preferably 2 to 30, 2 to 15 or 5 to 15, more preferably 5 to 14, 7 to 14, or 8 to 14, still more preferably 8 to 13, 9 to 13, 8 to 12, 8 to 11, or 9 to 12, and particularly preferably 9 to 11.

In a non-limiting embodiment, the number of amino acids (the number of units) in the linear portion is preferably 0 to 8, more preferably 0 to 5, and still more preferably 0 to 3. In a non-limiting embodiment, "linear portions" as used herein may include natural amino acids and unnatural amino acids (including chemically modified or skeletally transformed amino acids).

In one non-limiting embodiment, the peptide compound or the cyclic peptide compound herein is preferably a compound that does not have an ester bond in the cyclic portion or the linear portion.

In one non-limiting embodiment, the cyclic peptide compound herein may be a cyclic peptide compound in which the side chain of the peptide moiety of the cyclic portion or the linear portion does not have at least one selected from the group consisting of (A) to (C) below:

(A) an indole skeleton;
(B) a fused ring structure formed of two or more aromatic rings; and
(C) an unsubstituted hydroxyphenyl group.

In particular, a cyclic peptide compound having neither (A) nor (C) mentioned above in the side chain of the cyclic portion is preferable, and a cyclic peptide compound having neither (B) nor (C) mentioned above in the side chain of the cyclic portion is more preferable. The above-mentioned "fused ring structure formed of two or more aromatic rings" may be a "fused ring structure".

In one non-limiting embodiment, the molecular weight of the cyclic peptide compound herein may be 500 to 2000.

In one non-limiting embodiment, the cyclic peptide compound herein can be a cyclic peptide compound that does not have at least one selected from the group consisting of (A) a methylthio group and (B) a thiol group in the side chain of the peptide moiety of the cyclic portion or the linear portion, or that has neither (A) nor (B).

In the present invention, when a peptide compound is formed by amide bonding of two amino acids, the peptide compound is formed by replacing the OH group of the carboxyl group of the main chain in a first amino acid with the nitrogen atom moiety of the amino group of the main chain in a second amino acid.

In the present invention, when a peptide compound is formed by ester bonding of two amino acids, the peptide compound is formed by replacing the OH group of the carboxyl group of the main chain in a first amino acid with the oxygen atom moiety of the hydroxyl group of the main chain in a second hydroxy amino acid.

In the present invention, when a peptide compound is formed by connecting natural amino acids and/or non-natural amino acids by amide bonding or ester bonding, two or more amino acids are continuously connected by amide bonding and/or ester bonding to form the peptide compound.

The site where connected by amide bonding and/or ester bonding of the peptide compound herein may be referred to as a "peptide site" herein. However, when the peptide compound is cyclic, the mode of bonding of the cyclized portion is not limited to amide bonding or ester bonding. Examples of the mode of bonding of the cyclized portion include covalent bonding such as amide bonding, carbon-carbon bonding, disulfide bonding, ester bonding, thioester bonding, thioether bonding, lactam bonding, bonding through a triazole structure, and bonding through a fluorophore structure, and, among these, amide bonding is preferable because of its high metabolic stability. More specifically, in one embodiment, the cyclic peptide compound herein preferably has amide bonding in the cyclized portion. The "mode of bonding of the cyclized portion" refers to the mode of bonding of the site where a ring is formed by a cyclization reaction.

The "peptide compound" in the present invention can be a linear or cyclic peptide that contains at least two (preferably 2, 3, 4, 5, 6, 7, 8, 9, 10, 11, 12, 13, 14, 15, 20, 25, or 30, particularly preferably 5, 6, or 7, and preferred range includes 2 to 30, 3 to 30, 6 to 20, 7 to 19, 7 to 18, 7 to 17, 7 to 16, 7 to 15, 8 to 14, or 9 to 13) *N*-substituted amino acids and contains at least one amino acid that is not *N*-substituted, in addition to or regardless of satisfying the above-described conditions concerning the total number of natural amino acids and non-natural amino acids. Examples of "*N*-substitution" include, but are not limited to, substitution of a hydrogen atom attached to an N atom with a methyl group, an ethyl group, a propyl group, a butyl group, or a hexyl group. Examples of the *N*-substituted amino acids preferably include amino acids obtained by *N*-methylation, *N*-ethylation, *N*-propylation, *N*-butylation, or *N*-pentylation of an amino group contained in natural amino acids, and such *N*-substituted amino acids are respectively referred to as *N*-methyl amino acid, *N*-ethyl amino acid, *N*-propyl amino acid, *N*-butyl amino acid, and *N*-pentyl amino acid. Conversion of an *N*-unsubstituted amino acid to an *N*-substituted amino acid is referred to as *N*-substitution, and may be referred to as *N*-alkylation, *N*-methylation, or *N*-ethylation. The proportion of the number of *N*-substituted amino acids contained in the peptide compound in the present invention includes, for example, 30% or higher, 40% or higher, 50% or higher, 60% or higher, 70% or higher, and 80% or higher of the total number of amino acids constituting the peptide compound.

In a non-limiting embodiment, examples of the number of *N*-substituted amino acids contained in the peptide moiety of a cyclic peptide compound herein preferably include 2 or more or 3 or more, more preferably 4 or more, 5 or

more, or 6 or more, still more preferably 7 or more, and particularly preferably 8 or more, and also preferably include 20 or less, 15 or less, 14 or less, 13 or less, 12 or less, 10 or less, or 9 or less. Examples of the number of *N*-substituted amino acids contained in a cyclic peptide compound herein include 30% or higher, 40% or higher, 50% or higher, 60% or higher, 70% or higher, or 80% or higher relative to the number of amino acids constituting the cyclic portion. *N*-substituted amino acids as used herein may be preferably *N*-alkylamino acids, and more preferably *N*-methylamino acids. Specifically, in a non-limiting embodiment, the number of *N*-alkylamino or *N*-methylamino acids is provided as an example of the number of the *N*-substituted amino acids. When the number of amino acids constituting the cyclic portion is 8, the number of *N*-substituted amino acids is preferably 3 to 7. When the number of amino acids constituting the cyclic portion is 9, the number of *N*-substituted amino acids is preferably 3 to 8. When the number of amino acids constituting the cyclic portion is 10, the number of *N*-substituted amino acids is preferably 3 to 8. When the number of amino acids constituting the cyclic portion is 11, the number of *N*-substituted amino acids is preferably 4 to 9. When the number of amino acids constituting the cyclic portion is 12, the number of *N*-substituted amino acids is preferably 4 to 10. When the number of amino acids constituting the cyclic portion is 13, the number of *N*-substituted amino acids is preferably 4 to 11. When the number of amino acids constituting the cyclic portion is 14, the number of *N*-substituted amino acids is preferably 5 to 12.

Herein, an "amino acid" constituting the peptide compound may be referred to as an "amino acid residue".

The "peptide compound" herein may include a pharmaceutically acceptable salts thereof, or solvates of the compound or the salts.

Herein, the term "side chain" is used in the context of side chains of amino acids, side chains of cyclic portions of cyclic peptide compounds, or such, and refers to a moiety not contained in each main chain structure.

The term "number of amino acids" as used herein refers to the number of amino acid residues (amino acid units) constituting the peptide compound, and means the number of amino acid units formed when the amide bonds, the ester bonds, and the bonds of cyclized portion connecting amino acids are cut.

<u>Membrane permeability of peptide compounds</u>

In a non-limiting embodiment, peptide compounds or cyclic peptide compounds herein may be cyclic peptide compounds that do not have a functional group that is extremely ionized at a neutral pH (for example, pH = 7.0), in order to have high membrane permeability. The term pKa as used herein refers to an observed pKa unless otherwise indicated. pKa values determined by ADMET Predictor described below are called calculated pKas. The term basic pKa as used herein refers to an observed basic pKa unless otherwise indicated. Basic pKa values determined by ADMET Predictor described below are called calculated basic pKas.

pKas and basic pKas can be determined by a conventional method. For example, they can be measured by a method such as that described in Experimental Chemistry Lecture 5, "Thermal Measurements and Equilibrium", p. 460 (edited by The Chemical Society of Japan, published by Maruzen Co., Ltd.). More specifically, they can be measured by a method described in Reference Example 3-1. When it is difficult to determine the pKa and basic pKa values of the side chain to be measured of an amino acid due to influence of other functional groups, the other functional groups can be appropriately protected by protecting groups or the like so that only the pKa and basic pKa of the functional group of interest can be measured.

Herein, the "acidic side chain" refers to a side chain having a pKa of 10 or less, and the "basic side chain" refers to a side chain having a basic pKa of 4 or higher. Herein, side chains having a pKa of more than 10 and side chains having a basic pKa of less than 4 are defined as neutral side chains.

In one non-limiting embodiment, the peptide compound or the cyclic peptide compound herein can contain amino acids having an acidic side chain that does not form an intramolecular hydrogen bond in addition to an amino acid having a side chain capable of forming an intramolecular hydrogen bond. In this case, the pKa of the acidic side chain may be 3.5 to 10. The pKa of the acidic side chain is preferably 3.5 or higher, more preferably 3.9 or higher, more preferably 4.5 or higher, more preferably 5.0 or higher, more preferably 5.5 or higher, and more preferably 5.7 or higher. The basic pKa is preferably 10 or less. Examples of the range of pKa preferably include 3.5 to 10, 3.9 to 10, 4.5 to 10, 5.0 to 10, 5.5 to 10, and 5.7 to 10. When such pKa is expressed by a calculated value, the calculated pKa is preferably 3.5 or higher, and more preferably 4.5 or higher, 5.0 or higher, 5.4 or higher, 8.0 or higher, and 8.3 or higher. The calculated pKa is preferably 10 or less. Examples of the range of the calculated pKa preferably include 3.5 to 10, 4.5 to 10, 5.0 to 10, 5.4 to 10, 8.0 to 10, and 8.3 to 10.

In one non-limiting embodiment, the peptide compound or the cyclic peptide compound herein can contain amino acids having a basic side chain that does not form an intramolecular hydrogen bond in addition to an amino acid having a side chain capable of forming an intramolecular hydrogen bond. In this case, the basic pKa of the basic side chain may be 4.0 to 10. The basic pKa of the basic side chain is preferably 10 or less, more preferably 9.5 or less, 9.0 or less, 8.5 or less, 7.5 or less, or 7.2 or less, and particularly preferably 6.5 or less. The basic pKa is preferably 4.0 or higher. Examples of the range of the basic pKa preferably include 4.0 to 10, 4.0 to 9.5, 4.0 to 9.0, 4.0 to 8.5, 4.0 to 7.5,

4.0 to 7.2, and 4.0 to 6.5. When such basic pKa is expressed by a calculated value, the basic calculated pKa is preferably 10 or less, and more preferably 9.5 or less, 9.0 or less, 8.8 or less, 8.6 or less, 8.5 or less, 7.5 or less, or 6.5 or less. The basic calculated pKa is preferably 4.0 or higher. Examples of the range of the basic calculated pKa preferably include 4.0 to 10, 4.0 to 9.5, 4.0 to 9.0, 4.0 to 8.8, 4.0 to 8.6, 4.0 to 8.5, 4.0 to 7.5, and 4.0 to 6.5.

While it is not intended to be a limitation, the number or the proportion of aromatic rings contained in the side chains of the peptide moiety may affect the membrane permeability of the cyclic peptide compound as demonstrated in the Reference Examples later. When the number or the proportion of aromatic rings contained in the side chains of the peptide moiety exceeds a certain level, the probability that a compound having high membrane permeability is obtained may decrease. More specifically, the number of aromatic rings can have a negative effect on membrane permeability.

Herein, the term "negative effect" as used in the context of the effect of the invention is the effect that cancels the effect of the invention. For example, when the effect of the invention that should be exhibited is defined as 100% and in the case where the effect is reduced to 30%, 20%, 10%, 5% or less, it can be referred to that there is a "negative effect".

In one non-limiting embodiment, for high membrane permeability, the number of aromatic rings contained in the side chains of the peptide moiety of the cyclic peptide compound herein is preferably 5 or less, with preferable examples being 0, 1, 2, or 3, and preferred examples of ranges being 0 to 3 and 1 to 3. The proportion of the number of aromatic rings contained in the side chains of the peptide moiety to the number of amino acids constituting the peptide moiety is preferably 40% or less, with preferred examples being 35% or less, 30% or less, 27% or less, 25% or less, and 20% or less.

The "number of aromatic rings" (also referred to as an "Aromatic Ring Count" (ARC)) herein refers to the number of aromatic rings contained in the side chains of the peptide moiety of the cyclic peptide compound, and, for example, a phenol group is counted as 1, a bicyclic fused ring such as an indole skeleton is counted as 2, and a tricyclic fused ring such as anthracene is counted as 3.

In one non-limiting embodiment, for high membrane permeability, the number of aromatic rings that may be contained in the cyclic peptide compound herein has a limitation, whereas it is preferred that an amino acid that contains a side chain capable of forming an intramolecular hydrogen bond is present in the cyclic portion that is a site capable of contributing to binding to the target molecule. More specifically, in one embodiment, when the peptide compound herein is a cyclic peptide compound, and a cyclic portion of the cyclic peptide compound contains an amino acid containing an aromatic ring in the side chain capable of forming an intramolecular hydrogen bond, the number of aromatic rings contained in the cyclic portion of the peptide compound is, for example, 1, 2, or 3, and the range of the number of aromatic rings is, for example, 1 to 3 or 2 to 3. In addition, when the peptide compound herein is a cyclic peptide compound and a cyclic portion of the cyclic peptide compound contains an amino acid containing an aromatic ring in the side chain capable of forming an intramolecular hydrogen bond, the proportion of the number of aromatic rings contained in the cyclic portion to the total number of aromatic rings contained in the peptide compound is, for example, 30% or higher, 40% or higher, 60% or higher, 80% or higher, or 100%.

The membrane permeability of peptide compounds in the present invention can be measured by the methods for measuring membrane permeability described in WO 2018/124162.

In a non-limiting embodiment, $P_{app}$ of the peptide compound in the present invention is preferably $1.0 \times 10^{-7}$ cm/sec or higher, $5.0 \times 10^{-7}$ cm/sec or greater or $8.0 \times 10^{-7}$ cm/sec or higher, more preferably $9.0 \times 10^{-7}$ cm/sec or higher, even more preferably $1.0 \times 10^{-6}$ cm/sec or higher, and particularly preferably $3.0 \times 10^{-6}$ cm/sec or higher.

Herein, unless otherwise stated particularly, "membrane permeability coefficient ($P_{app}$)" means a value measured using the measurement method (improved method) described in WO 2018/124162.

In one non-limiting embodiment, examples of ClogP of the cyclic peptide compound herein include preferably 4 or higher, more preferably 5 or higher, even more preferably 6 or higher, and particularly preferably 8 or higher, and preferably 18 or less, 17 or less, and 16 or less, and, for example, 4 to 18, 5 to 17, and 6 to 16. ClogP herein is a computer-calculated distribution coefficient and can be calculated using Daylight Version 4.9 of Daylight Chemical Information Systems, Inc.

In one non-limiting embodiment, the lower limit of ClogP/total aa of the cyclic peptide compound herein is preferably 1.0 or higher, more preferably 1.1 or higher, and even more preferably 1.2 or higher. The upper limit of ClogP/total aa is preferably 1.8 or less, 1.7 or less, 1.6 or less, and 1.5 or less. Examples of ranges of ClogP/total aa include from 1.0 to 1.8, from 1.0 to 1.7, from 1.1 to 1.6, and from 1.1 to 1.5. The term "total aa" as used herein (also expressed as "total AA") refers to the number of amino acids constituting the peptide moiety of a peptide compound. For example, the total aa is 11 for a cyclic peptide compound in which the cyclic portion is composed of 10 amino acids and the linear portion is composed of one amino acid. The ClogP/total aa as used herein is calculated by dividing ClogP by total aa.

Metabolic stability of the peptide compounds

In a non-limiting embodiment, the peptide compounds herein preferably have good metabolic stability. For good metabolic stability, the number of amino acids included in the peptide compound is preferably 8 or more, more preferably 9 or more, and even more preferably 11 or more. In one embodiment, the peptide compounds preferably do not carry a thioether bond, which may be readily oxidized. Furthermore, in one embodiment, the peptide compounds preferably do not carry a methylthio group, since this is easily oxidized and may interfere with metabolic stability.

(Amino acids capable of forming an intramolecular hydrogen bond)

In one non-limiting embodiment, the peptide compound of the present invention contains at least one, and specifically, for example, 1, 2, 3, 4, or more amino acids capable of forming a hydrogen bond in the side chain thereof (*i.e.,* an intramolecular hydrogen bond).

The side chain of an amino acid herein includes a chain bonded to a carbon atom (such as α-, β-, or γ-carbon atom) and a chain bonded to a nitrogen atom contained in the amino acid. Herein, the length of the side chain of the amino acid can be determined by the method described in Reference Example 1. More specifically, the length can be determined by capping the N-terminus of an amino acid unit with an acetyl group and the C-terminus with a methylamino group, generating a conformation by LowModeMD of molecular modeling software MOE (Chemical Computing Group), and measuring the distance from the atom to which the side-chain moiety is attached (α-carbon atom (Cα carbon) in the case of a natural amino acid) to the most distal atom of the same side chain (excluding a hydrogen atom).

The term "long side chain" as used herein refers to a side chain with 5.4 angstroms or more in length. For intramolecular hydrogen bonding, the side chain of the amino acid is preferably a long side chain. The length of the long side chain is preferably 5.4 angstroms or more, more preferably 5.6 angstroms or more, even more preferably 5.8 angstroms or more, and particularly preferably 6.0 angstroms or more. The upper limit of the length of the side chain is not particularly limited, and examples include 20 angstroms or less, 15 angstroms or less, 13 angstroms or less, 12 angstroms or less, 11 angstroms or less, 10 angstroms or less, 9.0 angstroms or less, 8.8 angstroms or less, 8.5 angstroms or less, and 8.0 angstroms or less. Examples of the range of the length of the long side chain include 5.4 to 20 angstroms, 6.0 to 20 angstroms, 6.0 to 15 angstroms, 6.0 to 13 angstroms, and 6.0 to 10 angstroms.

In one non-limiting embodiment, the "long side chain" is preferably (i) a side chain containing no amide bond or one amide bond on the side chain thereof, and may be (ii) a side chain containing no amide bond on the side chain thereof. More specifically, in one non-limiting embodiment, preferred examples include a cyclic peptide compound having a long side chain in the cyclic portion, which side chain does not have two or more amide bonds in the side chain thereof and is 6.0 to 11 angstroms in length. The long side chain herein may or may not have an aromatic ring.

In one non-limiting embodiment, the amino acid capable of forming an intramolecular hydrogen bond is preferably an amino acid having a long side chain.

Whether an intramolecular hydrogen bond is present or not can be judged by, for example, an X-ray structural analysis or a chemical shift by $^1H$ NMR. It is generally judged that when the amount of temperature-dependent chemical shift change is <2 ppb/K, there is a clear hydrogen bond, and when it is >4 ppb/K, there is no hydrogen bond. Moreover, a conformational analysis by computational chemistry as exemplified in Example 3 is also a method used to determine whether there is a possibility of forming an intramolecular hydrogen bond.

In an aspect, the peptide compound of the present invention contains at least one, and, specifically, for example, 1, 2, 3, 4, or more amino acids capable of forming a pseudo W-membered ring in the side chain thereof.

The term "pseudo W-membered ring" as used herein means that the compound has a cyclic structure resulting from covalent bonding, but it forms a pseudo cyclic structure by the conformation fixed by intramolecular hydrogen bonding. "W" represents the ring size selected from a natural number of 3 or more, and the atoms constituting the pseudo cyclic portion invariably include three atoms: a hydrogen atom which is a proton donor; an atom directly bonded to the hydrogen atom; and a proton acceptor capable of forming a hydrogen bond with the proton donor. W representing the ring size of the cyclic structure of the pseudo cyclic structure is 3 or more, preferably 4 to 7 or 5 to 7, and more preferably 5 to 6. In the case of the structure of the side-chain moiety of Ser(nPrOH) exemplified below, the hydrogen atom of a proton-donor hydroxyl group, the oxygen atom of the hydroxyl group to which the hydrogen atom is directly attached and the oxygen atom of a proton-acceptor ether group correspond to the above three atoms, and a pseudo 6-membered ring is formed. An intramolecular hydrogen bond is characterized by being capable of structural conversion to a cyclized form and to a non-cyclized form according to the ambient environment, and an increased membrane permeability of a compound having a functional group that forms an intramolecular hydrogen bond can be expected when adopting a cyclized form structure so as to mask the proton donor under a hydrophobic environment as in a biological membrane. On the other hand, when a case where a compound that binds to a target molecule as an inhibitor of the target molecule is exemplified, when the compound having a functional group that forms an intramolecular hydrogen bond adopts a non-cyclized structure, either one or both of the proton donor and the proton acceptor is expected to bind to the target

molecule, and an intermolecular hydrogen bond can be more efficiently formed between the compound and the target molecule. As an example thereof, a schematic diagram is depicted below in which a part of a structure capable of forming an intramolecular hydrogen bond forms an intermolecular mutual bond with the amide moiety of the target molecule (* indicates the point of bonding with $\alpha$ carbon of Ser(nPrOH), and ** and *** each represent the point of bonding with the protein of the target molecule).

## Side chain moiety structure of Ser(nPrOH)

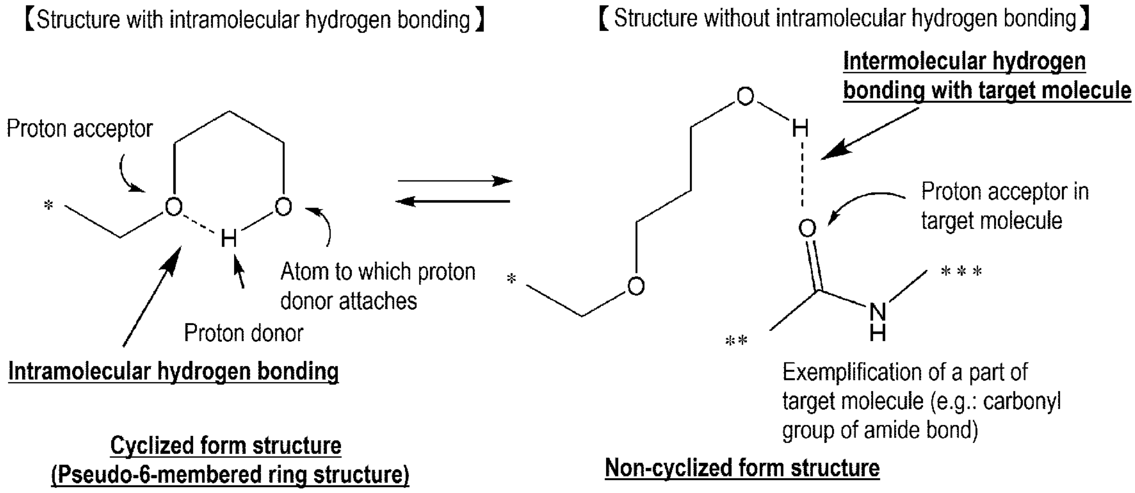


In one non-limiting embodiment, the amino acid having a side chain capable of forming an intramolecular hydrogen bond may form a pseudo 4- to 7-membered ring, preferably forms a pseudo 5- to 7-membered ring, and particularly preferably forms a pseudo 5- to 6-membered ring in the structure of a moiety where an intramolecular hydrogen bond is formed.

In one non-limiting embodiment, examples of the structure capable of forming an intramolecular hydrogen bond herein include the following (* indicates the point of bonding with $Q_1$ of Formula 1 or $Q_2$ of Formula 2).

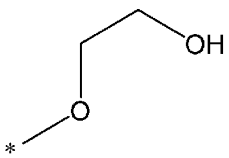

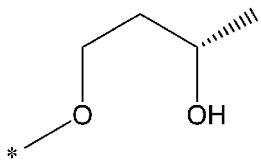

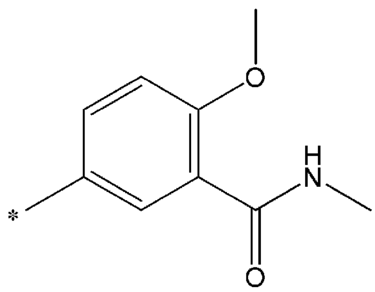

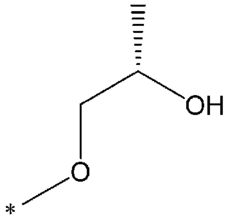

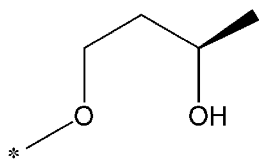

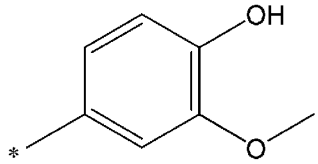

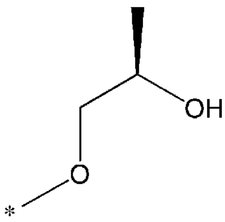
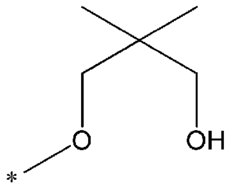
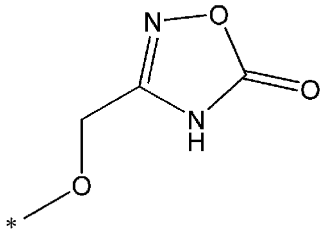
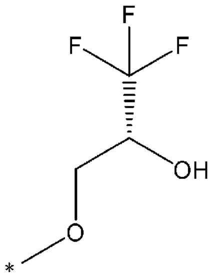
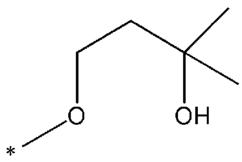
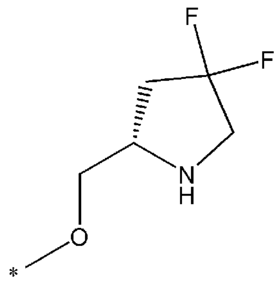
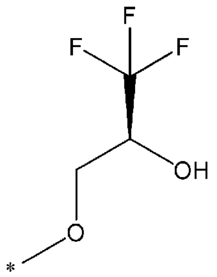
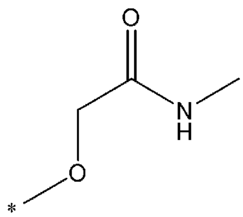
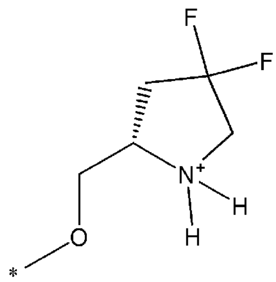
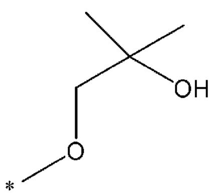
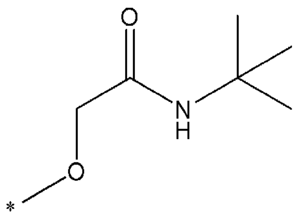
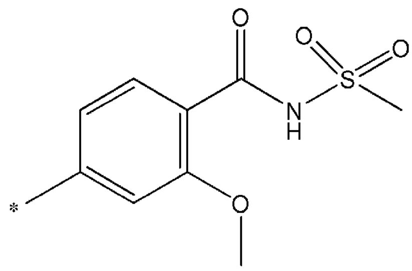
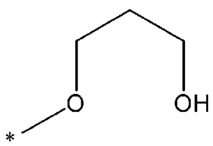
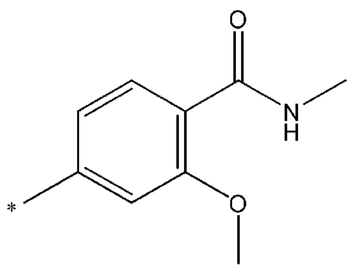
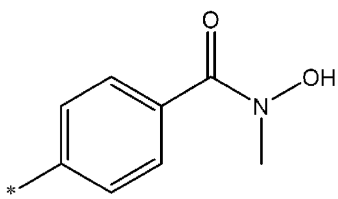
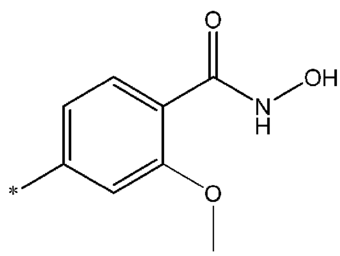
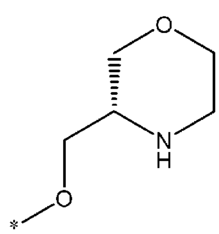

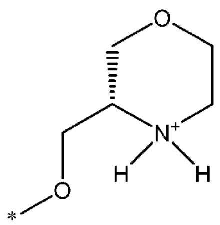

In one non-limiting embodiment, the amino acid capable of forming a hydrogen bond in the side chain can be represented by Formula A below.

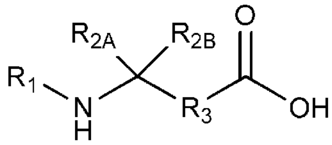

(A)

In an aspect, in Formula (A), $R_1$ is hydrogen, $C_1$-$C_6$ alkyl, or a group represented by Formula 1 or Formula 2, and in this case, (a) as for $R_{2A}$ and $R_{2B}$, $R_{2A}$ is hydrogen, $C_1$-$C_6$ alkyl, or a group represented by Formula 1 or Formula 2, and $R_{2B}$ is hydrogen or $C_1$-$C_6$ alkyl; or (b) $R_{2A}$ and $R_{2B}$ form a 4- to 6-membered ring together with the carbon atom to which they are bonded. When $R_1$ is $C_1$-$C_6$ alkyl, $R_1$ is preferably methyl. When $R_{2B}$ is $C_1$-$C_6$ alkyl, $R_{2B}$ is preferably methyl. When $R_{2B}$ is $C_1$-$C_6$ alkyl, $R_{2A}$ is preferably a group represented by Formula 1 or Formula 2, or $C_1$-$C_6$ alkyl. When $R_{2A}$ and $R_{2B}$ form a 4- to 6-membered ring together with the carbon atom to which they are bonded, the 4- to 6-membered ring is preferably cyclopropyl, cyclobutyl, cyclopentyl, or cyclohexyl.

In another aspect, in Formula (A), $R_1$ forms a 4- to 6-membered hetero ring together with the nitrogen atom to which $R_1$ is bonded, $R_{2A}$ and the carbon atom to which $R_{2A}$ is bonded, and in this case, $R_{2B}$ is hydrogen or $C_1$-$C_6$ alkyl. When $R_{2B}$ is $C_1$-$C_6$ alkyl, $R_{2B}$ is preferably methyl. The 4- to 6-membered hetero ring may have one or more substituents selected from the group consisting of a group represented by Formula 1 or Formula 2, -OH, and an alkoxy group. The 4- to 6-membered hetero ring is preferably pyrrolidine (*i.e.,* a proline-like structure).

As for the combination of $R_1$, $R_{2A}$ and $R_{2B}$, when $R_{2A}$ is a group represented by Formula 1 or Formula 2, it is preferred that $R_1$ is hydrogen or $C_1$-$C_6$ alkyl and $R_{2B}$ is hydrogen or $C_1$-$C_6$ alkyl, and the $C_1$-$C_6$ alkyl is preferably methyl. When $R_1$ is a group represented by Formula 1 or Formula 2, it is preferred that $R_{2A}$ is hydrogen or $C_1$-$C_6$ alkyl and $R_{2B}$ is hydrogen or $C_1$-$C_6$ alkyl, or, alternatively, $R_{2A}$ and $R_{2B}$ together form a 4- to 6-membered ring, and the $C_1$-$C_6$ alkyl is preferably methyl.

In Formula (A), $R_3$ is a single bond or -$CHR_4$-, where $R_4$ is hydrogen, $C_1$-$C_4$ alkyl, or a group represented by Formula 1 or Formula 2. When $R_3$ is -$CHR_4$-, $R_4$ is preferably hydrogen or a group represented by Formula 1 or Formula 2.

In Formula (A), two or more groups represented by Formula 1 and/or Formula 2 may be contained, but it is preferred that only one group represented by either Formula 1 or Formula 2 is contained. Specific examples of embodiments in which one group represented by either Formula 1 or Formula 2 is contained include the following:

(i) $R_1$ is a group represented by Formula 1 or Formula 2, $R_{2A}$ is hydrogen or $C_1$-$C_6$ alkyl and is preferably hydrogen, $R_{2B}$ is hydrogen or $C_1$-$C_6$ alkyl and is preferably hydrogen, $R_3$ is a single bond or -$CHR_4$-, and $R_4$ is hydrogen or $C_1$-$C_4$ alkyl and is preferably hydrogen;
(ii) $R_1$ is hydrogen or $C_1$-$C_6$ alkyl, $R_{2A}$ is a group represented by Formula 1 or Formula 2, $R_{2B}$ is hydrogen or $C_1$-$C_6$ alkyl and is preferably hydrogen, $R_3$ is a single bond or -$CHR_4$- and is preferably a single bond, and $R_4$ is hydrogen or $C_1$-$C_4$ alkyl and is preferably hydrogen;
(iii) $R_1$ is hydrogen or $C_1$-$C_6$ alkyl, $R_{2A}$ is hydrogen or $C_1$-$C_6$ alkyl and is preferably hydrogen, $R_{2B}$ is hydrogen or $C_1$-$C_6$ alkyl and is preferably hydrogen, $R_3$ is -$CHR_4$-, and $R_4$ is a group represented by Formula 1 or Formula 2; and
(iv) $R_1$, the nitrogen atom to which $R_1$ is bonded, $R_{2A}$ and the carbon atom to which $R_{2A}$ is bonded together form a 4- to 6-membered hetero ring (preferably a pyrrolidine ring), the hetero ring has a group represented by Formula 1 or Formula 2, $R_{2B}$ is hydrogen or $C_1$-$C_6$ alkyl and is preferably hydrogen, $R_3$ is a single bond or -$CHR_4$- and is preferably a single bond, and $R_4$ is hydrogen or $C_1$-$C_4$ alkyl and is preferably hydrogen.

Formula 1 and Formula 2 are respectively represented by the following formulae:

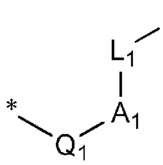 (Formula 1) 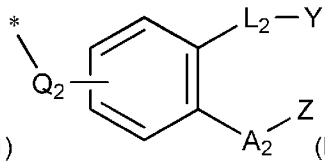 (Formula 2) ,

wherein * indicates the point of bonding. The amino acid represented by Formula A contains at least one group represented by either Formula 1 or Formula 2. For example, the amino acid represented by Formula A can contain one group represented by either Formula 1 or Formula 2, and, in this case, any one of $R_1$, $R_2$, and $R_4$ in Formula A is the group represented by Formula 1 or Formula 2.

In Formula 1, $Q_1$ is a single bond, $C_1$-$C_4$ alkylene, or $C_2$-$C_4$ heteroalkylene containing one oxygen atom. When $Q_1$ is $C_1$-$C_4$ alkylene, $Q_1$ is preferably $-CH_2-$ or $-(CH_2)_2-$.

In Formula 1, $A_1$ is -O- or -S-, and is preferably -O-.

In Formula 1, $L_1$ is linear $C_1$-$C_3$ alkylene optionally substituted with one or more substituents selected from the group consisting of fluorine, $C_1$-$C_2$ alkyl, $C_1$-$C_2$ fluoroalkyl, and oxo (=O). Specific examples of $L_1$ include $-CH_2-$, $-(CH_2)_2-$, $-(CH_2)_3-$, $-CH_2CH(CH_3)-$,$-CH_2CH(CF_3)-$, $-CH_2C(CH_3)_2-$, $-(CH_2)_2CH(CH_3)-$, $-CH_2C(CH_3)_2CH_2-$, $-(CH_2)_2C(CH_3)_2-$, and-$CH_2CO-$. In one aspect, $L_1$ is preferably not unsubstituted $-(CH_2)_2-$.

In Formula 1, X is -OH, $-NR_{Z1}R_{Z2}$, $-CONR_{Z1}R_{Z2}$, or 5- to 6-membered saturated or unsaturated heterocyclyl containing 1 to 3 heteroatoms and optionally substituted with oxo or one or more halogens, wherein Rzi and $R_{Z2}$ are independently selected from the group consisting of hydrogen, -OH, $C_1$-$C_4$ alkyl, and $C_1$-$C_4$ alkylsulfonyl. When X is $-NR_{Z1}R_{Z2}$, X is preferably-$NH_2$, -NHMe, -NHEt, -NH(nPr), -NH(iPr), or -NH(tBu). When X is $-CONR_{Z1}R_{Z2}$, X is preferably $-CONH_2$, -CONHMe, -CONHEt, -CONH(nPr), -CONH(iPr), or -CONH(tBu). When X is 5- to 6-membered saturated or unsaturated heterocyclyl optionally substituted with oxo or one or more halogens, the saturated or unsaturated heterocyclyl is preferably 4,5-dihydro-1,2,4-oxadiazolyl, 4,5-dihydro-1,2,4-thiadiazolyl, or pyrrolidyl, and X is preferably the following groups:

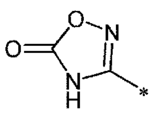 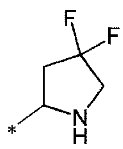 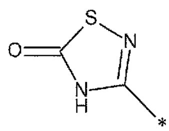 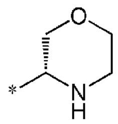

Specific examples of such preferred combinations of $-Q_1$-$A_1$-$L_1$-X include the following:

(i) $Q_1$ is $C_1$-$C_4$ alkylene and is preferably $-CH_2-$, $A_1$ is -O- or -S- and is preferably -O-, $L_1$ is linear $C_1$-$C_3$ alkylene optionally substituted with one or more substituents selected from the group consisting of $C_1$-$C_2$ alkyl, $C_1$-$C_2$ fluoroalkyl, and oxo (=O), and X is -OH;

(ii) $Q_1$ is $C_1$-$C_4$ alkylene and is preferably $-CH_2-$, $A_1$ is -O- or -S- and is preferably -O-, $L_1$ is linear $C_1$-$C_3$ alkylene optionally substituted with one or more substituents selected from the group consisting of $C_1$-$C_2$ alkyl, $C_1$-$C_2$ fluoroalkyl, and oxo (=O), and is preferably $-CH_2-$, X is-$NR_{Z1}R_{Z2}$, where Rzi is $C_1$-$C_4$ alkyl, and $R_{Z2}$ is hydrogen;

(iii) $Q_1$ is $C_1$-$C_4$ alkylene and is preferably $-CH_2-$, $A_1$ is -O- or -S- and is preferably -O-, $L_1$ is linear $C_1$-$C_3$ alkylene optionally substituted with one or more substituents selected from the group consisting of $C_1$-$C_2$ alkyl, $C_1$-$C_2$ fluoroalkyl, and oxo (=O), and is preferably $-CH_2-$, X is-$CONR_{Z1}R_{Z2}$, where Rzi is $C_1$-$C_4$ alkyl and $R_{Z2}$ is hydrogen;

(iv) $Q_1$ is $C_1$-$C_4$ alkylene and is preferably $-CH_2-$, $A_1$ is -O- or -S- and is preferably -O-, $L_1$ is linear $C_1$-$C_3$ alkylene optionally substituted with one or more substituents selected from the group consisting of $C_1$-$C_2$ alkyl, $C_1$-$C_2$ fluoroalkyl, and oxo (=O), and is preferably $-CH_2-$, and X is 5- to 6-membered saturated or unsaturated heterocyclyl containing 1 to 3 heteroatoms and optionally substituted with oxo or one or more halogens (preferably fluorine); and

(v) $Q_1$ is a single bond, $A_1$ is -O- or -S- and is preferably -O-, $L_1$ is linear $C_1$-$C_3$ alkylene optionally substituted with one or more substituents selected from the group consisting of $C_1$-$C_2$ alkyl, $C_1$-$C_2$ fluoroalkyl, and oxo (=O), and X is -OH.

In Formula 2, $Q_2$ is a single bond, $C_1$-$C_4$ alkylene, or $C_2$-$C_4$ heteroalkylene containing one oxygen atom. When $Q_2$ is $C_1$-$C_4$ alkylene, $Q_2$ is preferably $-CH_2-$ or $-(CH_2)_2-$.

In Formula 2, $L_2$ is a single bond or linear $C_1$-$C_3$ alkylene optionally substituted with one or more substituents selected from the group consisting of fluorine, $C_1$-$C_2$ alkyl, $C_1$-$C_2$ fluoroalkyl, and oxo (=O). Specific examples of $L_2$ include a single bond, $-CH_2-$, -CO-, $-(CH_2)_2-$, $-(CH_2)_3-$, $-CH_2CH(CH_3)-$, $-CH_2CH(CF_3)-$, $-CH_2C(CH_3)_2-$, $-(CH_2)_2CH(CH_3)-$,$-CH_2C(CH_3)_2CH_2-$, $-(CH_2)_2C(CH_3)_2-$, and $-CH_2CO-$.

In Formula 2, Y is -OH, $C_1$-$C_4$ alkylsulfonylamino, -$NR_{Z3}R_{Z4}$, -$CONR_{Z3}R_{Z4}$, or 5- to 6-membered saturated or unsaturated heterocyclyl containing 1 to 3 heteroatoms and optionally substituted with oxo or halogen, wherein $R_{Z3}$ and $R_{Z4}$ are independently selected from the group consisting of hydrogen, -OH, $C_1$-$C_4$ alkyl, and $C_1$-$C_4$ alkylsulfonyl. When Y is -$NR_{Z3}R_{Z4}$, Y is preferably -$NH_2$, NHMe, NHEt, NH(nPr), NH(iPr), or NH(tBu). When Y is $C_1$-$C_4$ alkylsulfonylamino, Y is preferably methylsulfonylamino. Moreover, when Y is $C_1$-$C_4$ alkylsulfonylamino, $L_2$ is preferably -CO-.

In Formula 2, $A_2$ is a single bond, -O-, or -S-, and is preferably -O-.

In Formula 2, Z is hydrogen or $C_1$-$C_4$ alkyl, and when Z is $C_1$-$C_4$ alkyl, the $C_1$-$C_4$ alkyl is preferably methyl.

In Formula 2, when $A_2$ is -O- or -S-, Z is not hydrogen. More specifically, as for specific examples of -$A_2$-Z, $A_2$ is a single bond, and Z is hydrogen; $A_2$ is -O-, and Z is $C_1$-$C_4$ alkyl; or $A_2$ is -S-, and Z is $C_1$-$C_4$ alkyl.

Specific examples of preferred combinations of such -$Q_2$-, -$L_2$-Y, and -$A_2$-Z include the following:

(i) $Q_2$ is $C_1$-$C_4$ alkylene and is preferably -$CH_2$-, $L_2$ is linear $C_1$-$C_3$ alkylene optionally substituted with one or more substituents selected from the group consisting of fluorine, $C_1$-$C_2$ alkyl, $C_1$-$C_2$ fluoroalkyl, and oxo (=O), and is preferably -CO-, Y is -$NR_{Z3}R_{Z4}$ and is preferably methylamino, -N(OH)Me, or -NH(OH), $A_2$ is -O- or -S- and is preferably -O-, and Z is $C_1$-$C_4$ alkyl and is preferably methyl;

(ii) $Q_2$ is $C_1$-$C_4$ alkylene and is preferably -$CH_2$-, $L_2$ is a single bond, Y is -$CONR_{Z3}R_{Z4}$ and is preferably methylaminocarbonyl, -CON(OH)Me, or -CONH(OH), $A_2$ is -O- or -S- and is preferably -O-, Z is $C_1$-$C_4$ alkyl and is preferably methyl;

(iii) $Q_2$ is $C_1$-$C_4$ alkylene and is preferably -$CH_2$-, $L_2$ is linear $C_1$-$C_3$ alkylene optionally substituted with one or more substituents selected from the group consisting of fluorine, $C_1$-$C_2$ alkyl, $C_1$-$C_2$ fluoroalkyl, and oxo (=O), and is preferably -CO-, Y is -$NR_{Z3}R_{Z4}$ and is preferably-N(OH)Me, $A_2$ is a single bond, and Z is hydrogen;

(iv) $Q_2$ is $C_1$-$C_4$ alkylene and is preferably -$CH_2$-, $L_2$ is a single bond, Y is -$CONR_{Z3}R_{Z4}$ and is preferably -CON(OH)Me, $A_2$ is a single bond, and Z is hydrogen;

(v) $Q_2$ is $C_1$-$C_4$ alkylene and is preferably -$CH_2$-, $L_2$ is linear $C_1$-$C_3$ alkylene optionally substituted with one or more substituents selected from the group consisting of fluorine, $C_1$-$C_2$ alkyl, $C_1$-$C_2$ fluoroalkyl, and oxo (=O), and is preferably -CO-, Y is $C_1$-$C_4$ alkylsulfonylamino and is preferably methylsulfonylamino, $A_2$ is -O- or -S- and is preferably -O-, and Z is $C_1$-$C_4$ alkyl and is preferably methyl;

(vi) $Q_2$ is $C_1$-$C_4$ alkylene and is preferably -$CH_2$-, $L_2$ is a single bond, Y is $C_1$-$C_4$ alkylsulfonylaminocarbonyl and is preferably methylsulfonylaminocarbonyl, $A_2$ is -O- or -S- and is preferably -O-, and Z is $C_1$-$C_4$ alkyl and is preferably methyl; and

(vii) $Q_2$ is $C_1$-$C_4$ alkylene and is preferably -$CH_2$-, $L_2$ is a single bond, Y is -OH, $A_2$ is-O- or -S- and is preferably -O-, and Z is $C_1$-$C_4$ alkyl and is preferably methyl.

More specific examples of such amino acids capable of forming a hydrogen bond in the side chain and/or amino acids capable of forming a pseudo 4- to 7-membered ring in the side chain include the following amino acids:

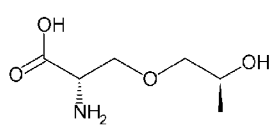
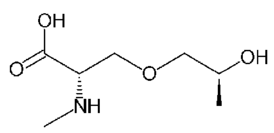
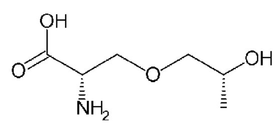

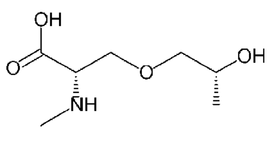
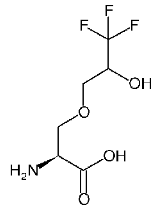
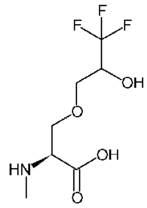
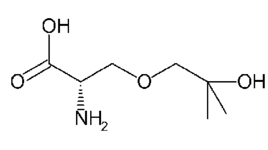

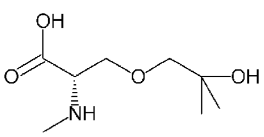
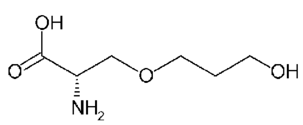
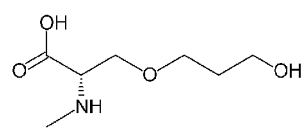

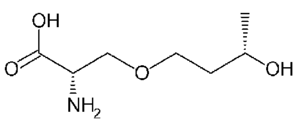
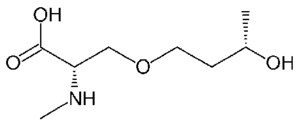
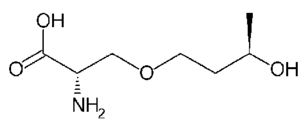

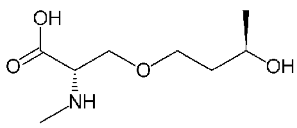
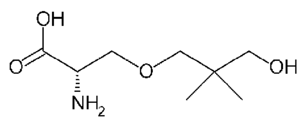
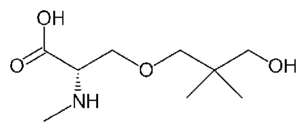

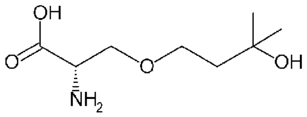
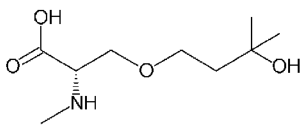
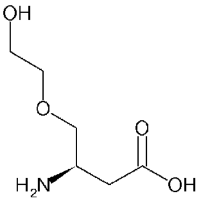

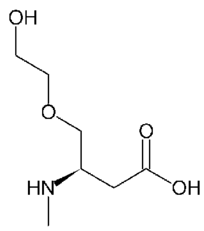
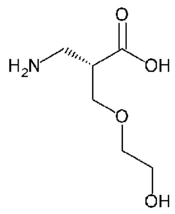
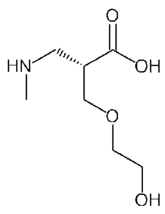
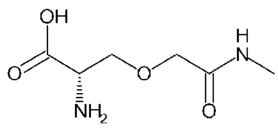

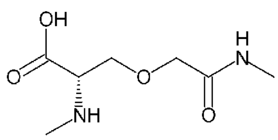
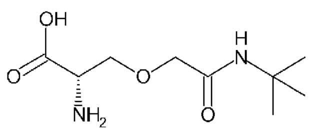
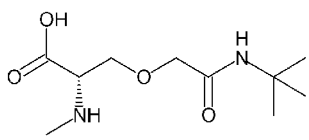

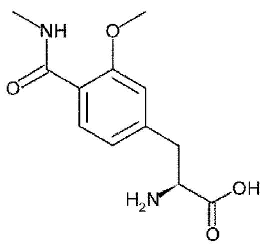 ,
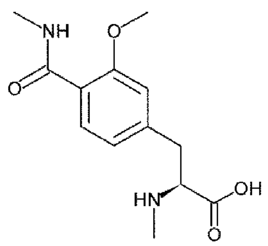 ,
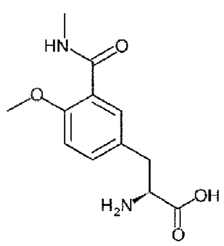 ,
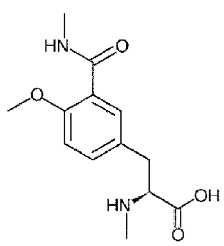 ,

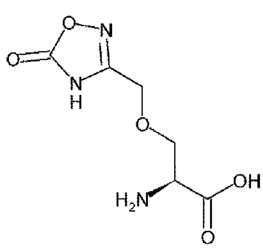 ,
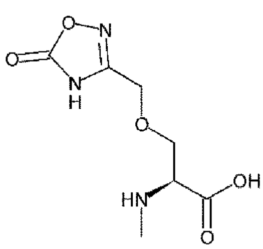 ,
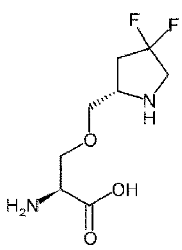 ,
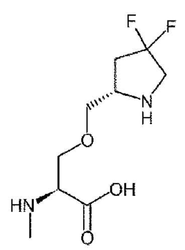 ,

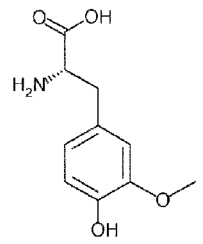, 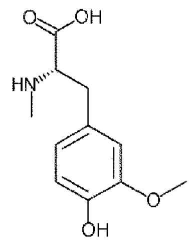, 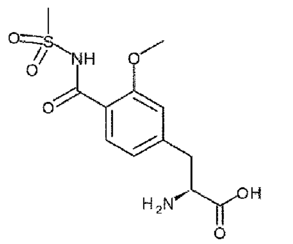, 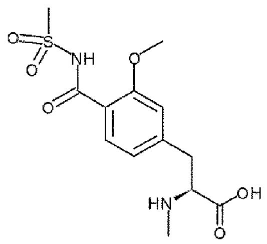

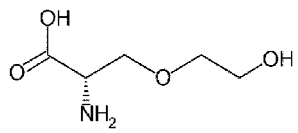, 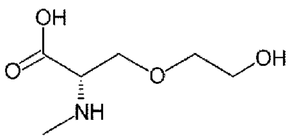,

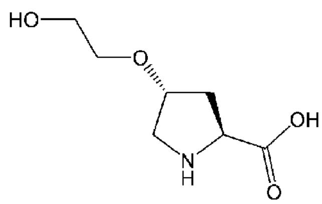 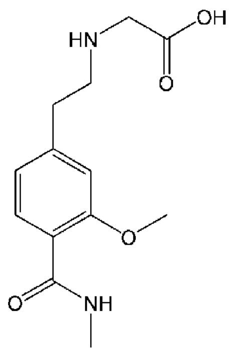 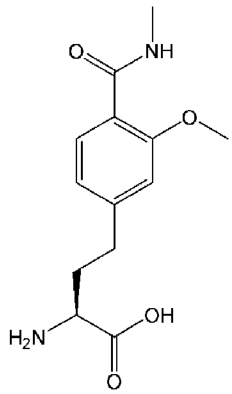 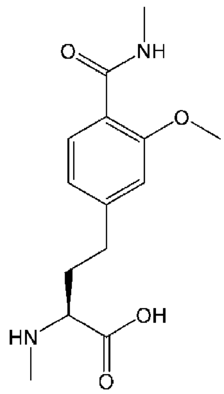

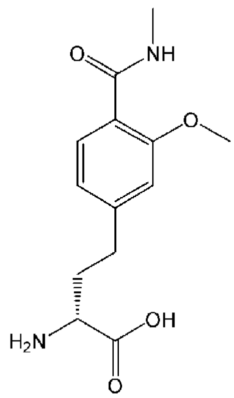 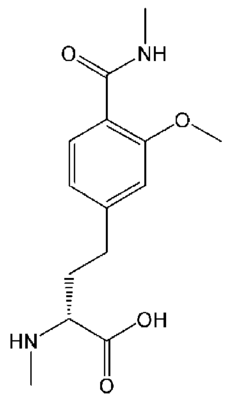 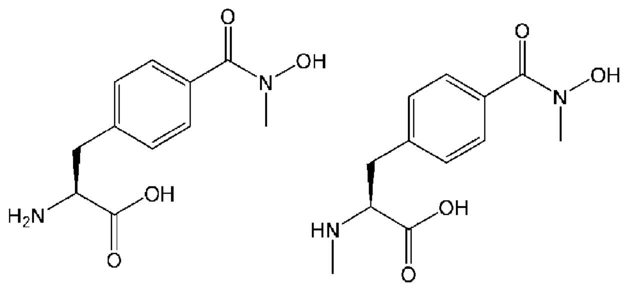

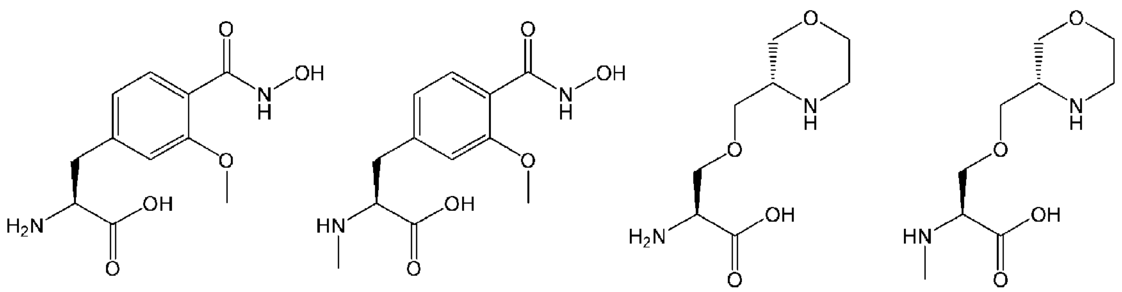.

In one non-limiting embodiment, when the amino acid represented by Formula A of the present invention is contained in a peptide compound, the amino acid is preferably connected to adjacent amino acids *via* the amino group

and the carboxyl group of the main chain of the amino acid. In this case, the amino acid represented by Formula (A) can also be represented as follows (* indicates the point of bonding with adjacent amino acids in the peptide compound):

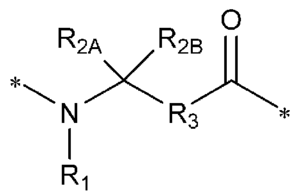

In one non-limiting embodiment, the present invention relates to a protected amino acid wherein the amino group and/or the carboxyl group of the amino acid capable of forming an intramolecular hydrogen bond is protected by a protecting group. Examples of the protecting group for the amino group of the protected amino acid include an Fmoc group, a Boc group, a Cbz group, an Alloc group, a nosyl group, a dinitronosyl group, a t-Bu group, a trityl group, and a cumyl group. Examples of the protecting group for the carboxyl group of the protected amino acid include a methyl group, an allyl group, a t-Bu group, a trityl group, a cumyl group, a methoxytrityl group, and a benzyl group. These protecting groups can be introduced by using, for example, the method described in "Greene's "Protective Groups in Organic Synthesis" (5th Edition, John Wiley & Sons 2014)".

Without wishing to be bound by a particular theory, the present inventors contemplate as follows. When using an amide bond contained in the main chain of a peptide as a means of masking a proton on the side chain of an amino acid contained in the peptide with a hydrogen bond, the formation of a hydrogen bond that masks the proton on the side chain has to rely on chance because each peptide has a different peptide conformation. Accordingly, it is difficult to consistently mask the side-chain proton by formation of a hydrogen bond. Moreover, when forming a hydrogen bond that masks the side-chain proton by using an amide bond contained in the main chain of an adjacent amino acid, the variation of side chains that can be employed is limited. In the case of an amino acid that is expected to form a hydrogen bond with an amide bond contained in the main chain of an adjacent amino acid, the position of a proton donor is limited to a position close to the amide bond of the main chain capable of forming a hydrogen bond. On the other hand, in one non-limiting embodiment, the amino acid herein can form a hydrogen bond "in the side chain" thereof, and thus the side chain can be designed more liberally. For example, it is also possible to lengthen the side chain while maintaining the formation of a hydrogen bond that is capable of masking a proton, and thus there can be an increased possibility that the proton on the side chain is used in interactions with the target.

(General production methods)

Next, general methods of producing an amino acid capable of forming a hydrogen bond in the side chain, and a peptide compound, of the present invention will be described.

<u>(Methods of producing an amino acid capable of forming an intramolecular hydrogen bond)</u>

In the production of an amino acid having an intramolecular hydrogen bond in the side chain, an amino acid having an ether bond in the side chain can be produced by the following two synthesis methods.

Production method 1:

A compound having aziridine and in which an amino group and a carboxylic acid group has been protected is allowed to react with the corresponding alcohol having a suitable protecting group in the presence of a Lewis acid to carry out a ring-opening reaction, and then unnecessary protecting groups are removed. In the following general formulae, a Cbz group, an Fmoc group, an Alloc group, a nosyl group (Ns group), or the like can be used as $P_1$, and a Me group, an allyl group, a benzyl group, or the like can be used as $P_2$. A preferred example of the Lewis acid used in the ring-opening reaction includes a catalytic amount of $BF_3 \cdot OEt_2$. While preferred examples of a solvent include dichloromethane, an alcohol (ROH) used as a reagent is also usable as a solvent. When R contains a functional group that may participate in the reaction, such a functional group is preferably protected with a protecting group that is stable in the presence of a Lewis acid. For example, when R contains a hydroxyl group, the hydroxyl group is preferably protected with a benzyl group or the like, and when R contains a secondary amino group, the amino group is preferably protected with an Alloc group or the like. After constructing the basic skeleton of an amino acid by the ring-opening reaction, the desired amino acid can be formed by suitable conversion of protecting groups (an amino acid in which $P_3$ is protected with a Cbz group, an Fmoc group, a Boc group, an Alloc group, a nosyl group (Ns group), or the like, or an unprotected amino acid in

which $P_3$ is hydrogen).

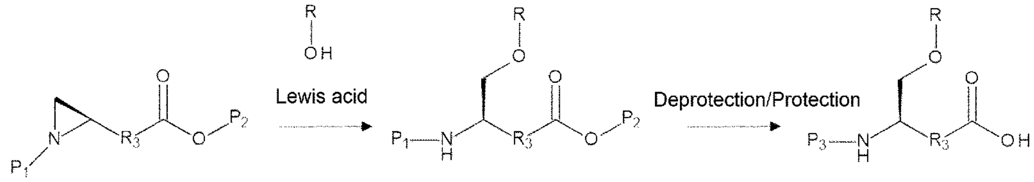


Production method 2:

After carrying out an alkylation reaction between an amino group-protected amino acid having a hydroxyl group in the side chain and an α-halocarbonyl compound such as 2-bromo-N-methylacetoamide or bromoacetonitrile, production as shown in the following scheme can be carried out by suitable conversion of functional groups. In the following general formulae, $P_1$ is preferably a protecting group that is stable under basic conditions, such as a Cbz group, a Boc group, or a Trt group. Preferred examples of the α-halocarbonyl compounds include those in which Ra is NMe, NtBu, or the like. $X_1$ is halogen, and is preferably a bromo group. Examples of the base used in the alkylation reaction include NaH, NaOtBu, and NaOt-Pent, and use of base in an amount of 2 equivalents or more relative to the amino group-protected amino acid having a hydroxyl group in the side chain enables selective alkylation of the hydroxyl group portion of the amino acid. Preferred examples of the solvent used include DMF and DMI. After alkylation with bromoacetonitrile, the basic skeleton of an amino acid can be constructed by constructing a hetero ring based on the resulting nitrile group. Illustrated below is an example of a scheme in which hydroxylamine is allowed to act on the resulting nitrile group, and then the product is cyclized by CDI to construct an oxadiazolone ring. After constructing the basic skeleton of an amino acid, it can be converted to a desired amino acid by suitable conversion of protecting groups (an amino acid in which $P_3$ is protected with a Cbz group, an Fmoc group, a Boc group, an Alloc group, a nosyl group (Ns group), or the like, or an unprotected amino acid in which $P_3$ is hydrogen).

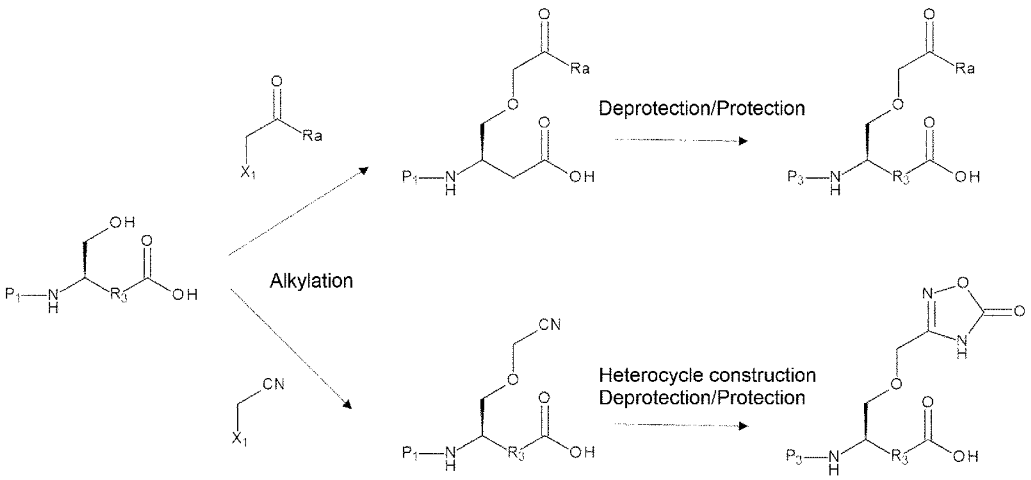


Production method 3:

A phenylalanine derivative can be produced by the method of Jackson-Negishi coupling (Journal of Organic Chemistry, 2010, 75, 245), asymmetric benzylation of benzophenone imine of glycine tert-butyl ester using a phase transfer catalyst (J. Am. Chem. Soc. 1999, 121, 6519), or the like. A phenylalanine derivative can also be produced by carrying out a cross-coupling reaction accompanied by decarboxylation using an N-hydroxyphthalimide ester (NHPI ester) and an aromatic iodine compound or aromatic bromo compound as starting materials as shown in the following general formula. In the following general formula, n represents the number of carbon atoms, and n is preferably, but is not particularly limited to, 1 or 2. A group that is stable under cross-coupling conditions is used as $P_1$, and a preferred example includes an Fmoc group. $R_4$-$X_1$ is an aromatic halogen compound, and $R_4$ represents an aromatic moiety. When $R_4$ contains a functional group that can be converted under cross-coupling reaction conditions, the functional group is preferably protected with a protecting group that is stable under cross-coupling reaction conditions. For example, when $R_4$ contains a hydroxyl group, the hydroxyl group is preferably protected with a tetrahydropyranyl (THP) group or the like. $X_1$ is halogen, and preferred examples include a bromo (Br) group and an iodo (I) group. Nickel and zinc are

preferably used as catalysts for the cross-coupling reaction, and 4,4'-di-tert-butyl-2,2'-bipyridyl (dtbbpy) is preferably used as a catalyst ligand. Dimethylacetamide is preferably used as a solvent. After constructing the basic skeleton of an amino acid by a cross-coupling reaction that is accompanied by decarboxylation, it can be converted to a desired amino acid by suitable conversion of protecting groups (an amino acid in which $P_3$ is protected with a Cbz group, an Fmoc group, a Boc group, an Alloc group, a nosyl group (Ns group), or the like, or an unprotected amino acid in which $P_3$ is hydrogen).

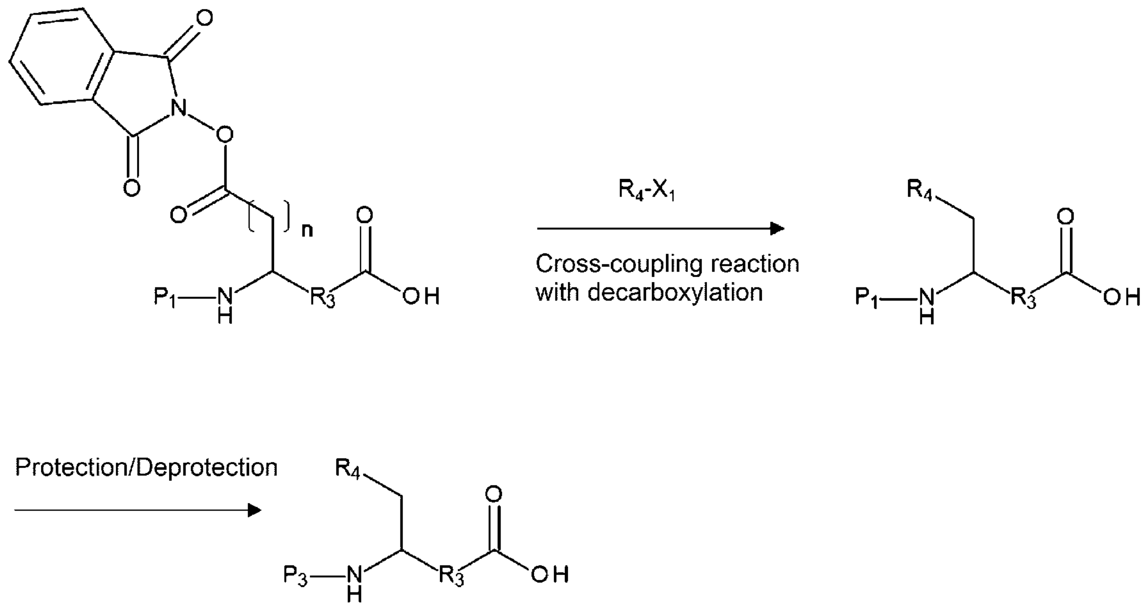


Amino acids having a side-chain moiety on the N atom of the amino acids can also be produced by the same reaction as shown below.

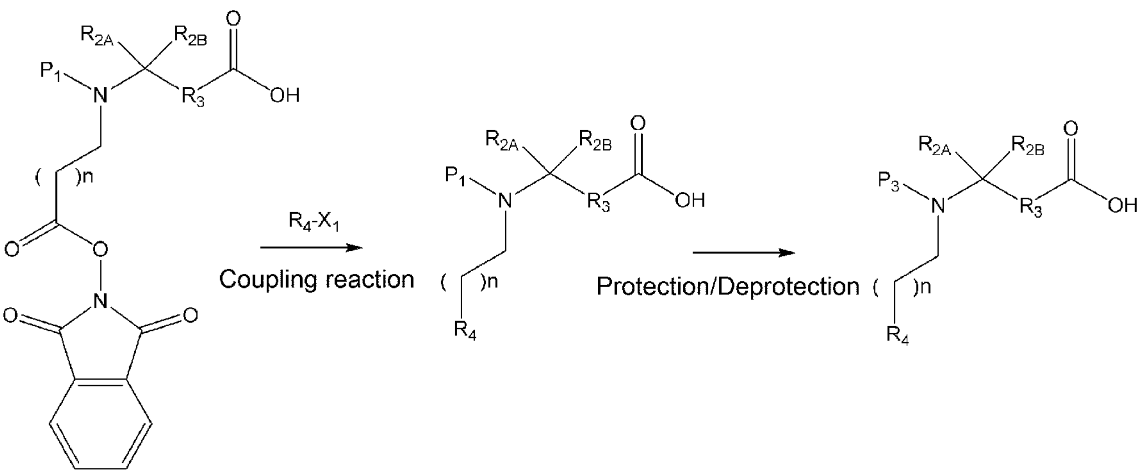


Production method 4:

Corresponding β-amino acids can be produced using Arndt-Eistert synthesis (Strategic Applications of Named Reactions in Organic Synthesis, p18).

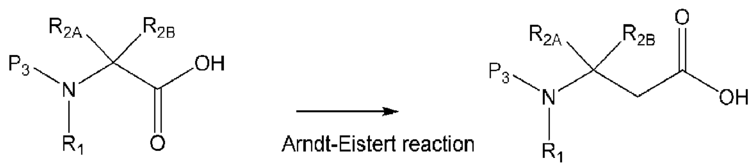

Production method 5:

β-Amino acid can be produced by carrying out a ring-opening reaction using ammonia on the corresponding epoxide having a functional group, introducing a protecting group into the amine moiety of the resulting compound, converting the resulting secondary alcohol to a leaving group, and carrying out conversion to a nitrile group accompanied by steric inversion by a nucleophilic substitution reaction, followed by conversion to carboxylic acid. In the following general formula, when R contains a functional group that participates in the reaction, the functional group is preferably protected with a suitable protecting group, e.g., when R contains a hydroxyl group, the hydroxyl group is preferably protected with a Trt group. Preferred examples of $P_1$ that is a protecting group for the amino group obtained after the ring-opening reaction include a Cbz group, a Boc group, and an Alloc group. The resulting secondary alcohol is converted to a leaving group $X_2$ (a preferred example of $X_2$ includes a mesyl group (OMs group) or the like). The resulting leaving group is converted to a nitrile group by a nucleophilic substitution reaction by cyanohydrin ($CN^-$) while being accompanied by steric inversion, the nitrile group is converted to carboxylic acid, and thus the basic skeleton of an amino acid can be constructed. An example of conversion from the nitrile group to the carboxylic acid includes hydrolysis under acidic conditions. After constructing the basic skeleton of an amino acid, it can be converted to a desired amino acid by suitable swapping of protecting groups (an amino acid in which $P_3$ is protected with a Cbz group, an Fmoc group, a Boc group, an Alloc group, a nosyl group (Ns group), or the like, or an unprotected amino acid in which $P_3$ is hydrogen).

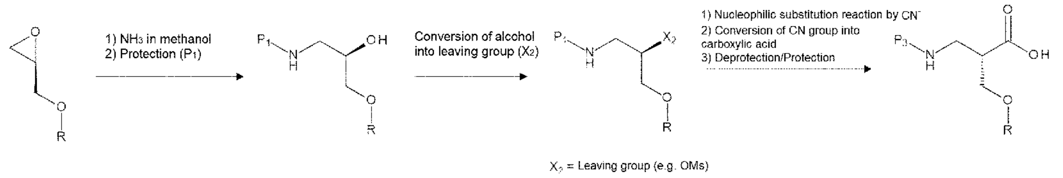


Production method 6

After a halogen compound and an amino group-protected cyclic amino acid having a hydroxyl group are subjected to an alkylation reaction using a base, a cyclic amino acid can be produced by performing suitable conversion of functional groups as shown in the following scheme. The starting-material cyclic amino acid used is preferably an amino acid having a 4- to 8-membered ring, and an amino acid having a 5-membered ring is exemplified in the following scheme. The position of the hydroxyl group present in the cyclic portion is not particularly limited and, as in the general formula, a position two or more atoms apart from the N atom of the amino acid is preferred. In the following general formula, a Cbz group, a Boc group, an Alloc group, a nosyl group (Ns group), or the like can be used as $P_1$. A preferred example of the base used in the alkylation reaction includes sodium hydride (NaH), and a preferred example of the solvent includes dimethylformamide. In the alkylation reaction, when the halogen compound (RX) has a functional group on R that may participate in the reaction, the functional group is preferably protected with a protecting group that is stable under basic conditions. For example, when R contains a hydroxyl group, the hydroxyl group is preferably protected with a tetrahydropyranyl (THP) group, a benzyl (Bn) group, or silyl ether such as a t-butyldimethylsilyl (TBDMS) group. $X_1$ is halogen, and examples include a chloro (Cl) group, a bromo (Br) group, and an iodine (I) group. After constructing the basic skeleton of an amino acid by the alkylation reaction, it can be converted to a desired amino acid by suitable conversion of protecting groups (an amino acid in which $P_3$ is protected with a Cbz group, an Fmoc group, a Boc group, an Alloc group, a nosyl group (Ns group), or the like, or an unprotected amino acid in which $P_3$ is hydrogen).

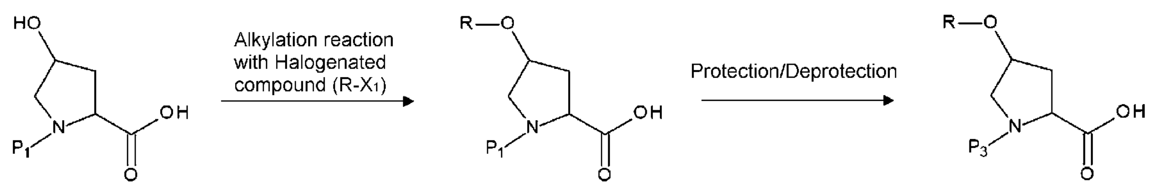


Production method 7:

The above amino acid can be N-methylated according to the following scheme by carrying out a cyclization reaction using paraformaldehyde under acidic conditions, and then ring-opening the resulting cyclized product under acidic conditions while reducing the produced imine.

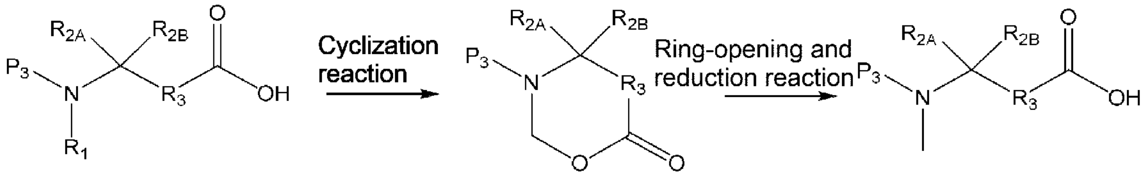


(Production methods for peptide compounds)

Chemical synthesis methods for peptide compounds

Chemical synthesis methods for peptide compounds or cyclic peptide compounds herein include, for example, liquid phase synthesis methods, solid phase synthesis methods using Fmoc synthesis, Boc synthesis, or such, and combinations thereof. In Fmoc synthesis, an amino acid in which the main chain amino group is protected with an Fmoc group, the side-chain functional groups are protected when necessary with protecting groups that are not cleaved by a base such as piperidine, a t-Bu group, a THP group, or a Trt group, and the main chain carboxylic acid group is not protected, is used as a basic unit. The basic unit is not particularly limited and may be any other combination as long as it has an Fmoc-protected amino group and a carboxyl group. For example, a dipeptide may be used as a basic unit. The basic unit to be positioned at the N terminus may be one that is not an Fmoc amino acid. For example, it may be a Boc amino acid, or a carboxylic acid analog that does not have an amino group. The main chain carboxyl group or a side chain carboxyl group of an amino acid having a carboxyl group in its side chain and of which main chain carboxyl group is protected by an appropriate protecting group is immobilized onto a solid phase by a chemical reaction with a functional group on a solid-phase carrier. Next, the Fmoc group is deprotected by a base such as piperidine or DBU, and the newly generated amino group and a subsequently added basic unit, *i.e.* a protected amino acid carrying a carboxyl group, are subjected to a condensation reaction to generate a peptide bond. In the condensation reaction, various combinations such as DIC and HOBt, DIC and HOAt, and HATU and DIPEA are possible as an activator for a carboxyl group. Repeating the Fmoc group deprotection and the subsequent peptide bond-forming reaction enables generation of the desired peptide sequence. After the desired sequence is obtained, this is cleaved from the solid phase, and the protecting groups introduced as necessary to the side-chain functional groups are deprotected. Furthermore, before cleaving from the solid phase, conformational conversion and cyclization of the peptide can be carried out. Cleavage from the solid phase and deprotection may be performed under the same conditions, for example, 90:10 TFA/$H_2O$, or deprotection may be performed under different conditions as necessary. Cleavage from the solid phase may be possible by using weak acids such as 1% TFA in some cases, or by using Pd or such as a protecting group and thus utilizing the orthogonality of these chemical reactions. Steps such as cyclization can be carried out during or after these steps. For example, a side-chain carboxylic acid and an N-terminal main chain amino group can be condensed, or a side-chain amino group and a C-terminal main chain carboxylic acid can be condensed. In this case, reaction orthogonality is necessary between the C-terminal carboxylic acid and the side-chain carboxylic acid to be cyclized, or between the N-terminal main chain amino group or hydroxy group and the side chain amino group to be cyclized, and protecting groups are selected by considering their orthogonality as described above. Reaction products thus obtained can be purified using a reverse-phase column, molecular sieve column, and such. Details of such methods are described, for example, in the Solid-phase Synthesis Handbook, published on May 1, 2002 by Merck Co. Commercially available resins for solid phase synthesis can be used, and examples include CTC resin, Wang resin, and SASRIN resin.

In the production of the compound described herein, when the defined groups undergo undesired chemical conversion under the conditions of the performed method, the compound can be produced by means of, for example, protection and deprotection of the functional groups. Here, the selection of protecting groups and operations of attaching/detaching the protecting groups can include, for example, the method described in "Greene's, "Protective Groups in Organic Synthesis" (5th edition, John Wiley & Sons 2014)", and these are suitably used according to the reaction conditions. In addition, the order of reaction steps such as introduction of a substituent can be changed as necessary. Examples of the protecting group for the amino group include Fmoc, Boc, Cbz, and Alloc groups. Such a carbamate group can be introduced by reacting the amino group with a carbamating agent in the presence of a base catalyst. Examples of the carbamating agent include $Boc_2O$, BocOPh, FmocOSu, FmocCl, CbzCl, and AllocCl. Examples of the base catalyst include lithium carbonate, sodium carbonate, sodium hydrogen carbonate, potassium carbonate, potassium hydrogen carbonate, cesium carbonate, cesium hydrogen carbonate, lithium hydroxide, sodium hydroxide, potassium hydroxide, cesium hydroxide, sodium phosphate, potassium phosphate, N-methylmorpholine, triethylamine, diisopropylethylamine, and N,N-dimethylaminopyridine. The carbamate group that is a protecting group for an amino group can be removed under basic conditions, acidic conditions, hydrolysis reaction conditions, or the like.

Ribosomal synthesis methods for peptide compounds

Examples of translational (ribosomal) synthesis methods for peptide compounds herein include methods of synthesizing compounds using cell-free translation systems, and the methods include synthesis methods that use reconstituted cell-free translation systems. Use of reconstituted cell-free translation systems is preferred from the view point that factors which one would like to remove can be eliminated.

In one aspect, the present specification provides ribosomal synthesis methods for peptide compounds herein containing at least one, two or more, or three or more amino acid types herein. Without limitation, such ribosomal synthesis methods may comprise the steps of:

(i) preparing tRNAs to which at least one, two or more, or three or more amino acid types herein are linked; and
(ii) obtaining the aforementioned peptide compound by translating a nucleic acid comprising at least one of the codons corresponding to the anticodons of the tRNAs in a cell-free translation system.

In a non-limiting embodiment, the ribosomal synthesis methods herein may be ribosomal synthesis methods for cyclic peptide compounds.

Cell-free translation systems

Herein, the "cell-free translation system" refers to a system in which ribosomes extracted from cells are combined with a group of protein factors involved in translation, tRNAs, amino acids, energy sources such as ATP and regenerating systems thereof, and is not limited as long as it can translate mRNAs into proteins. Furthermore, systems in which the ribosomal synthesis reactions are progressing may also be included in the "cell-free translation system" herein. The cell-free translation systems herein can contain nucleic acids that will serve as templates during peptide translation, and additionally contain initiation factors, elongation factors, release factors, aminoacyl-tRNA synthetases, and such. These factors can be obtained by purification from various cell extracts. Examples of the cells for purifying the factors therefrom may include prokaryotic cells and eukaryotic cells. Examples of the prokaryotic cells may include *E. coli* cells, extreme thermophile cells, and *Bacillus subtilis* cells. Known eukaryotic cells include those prepared using as materials yeast cells, wheat germs, rabbit reticulocytes, plant cells, insect cells, or animal cells. In addition to naturally-occurring tRNAs and aminoacyl tRNA synthetases (ARSs), artificial tRNAs and artificial aminoacyl tRNA synthetases that recognize unnatural amino acids can also be used. Peptides in which unnatural amino acids are introduced in a site-specific manner can be synthesized by using artificial tRNAs and artificial aminoacyl tRNA synthetases. Furthermore, when necessary, transcription can be performed from template DNAs by adding RNA polymerases such as T7 RNA polymerase to the cell-free translation systems.

Herein, "a cell-free translation system comprises a certain substance" includes embodiments where even if the substance is not included at the start of ribosomal synthesis, the substance is synthesized within the system in the process of ribosomal synthesis and becomes comprised in the system. For example, when a tRNA acylated with an amino acid is synthesized in the process of ribosomal synthesis, the cell-free translation system is understood to comprise the aminoacyl-tRNA.

PURESYSTEM (registered trademark) (BioComber, Japan) is a reconstituted cell-free translation system in which protein factors, energy-regenerating enzymes, and ribosomes necessary for translation in *E. coli* are respectively extracted and purified and then mixed with tRNAs, amino acids, ATP, GTP, and such. Since this system not only has a low content of impurities, but is also a reconstituted system, it is possible to easily prepare a system free from protein factors and amino acids desired to be excluded ((i) Nat. Biotechnol. 2001; 19: 751-5. Cell-free translation reconstituted with purified components. Shimizu, Y, Inoue, A., Tomari, Y, Suzuki, T., Yokogawa, T., Nishikawa, K., Ueda, T.; (ii) Methods Mol. Biol. 2010; 607: 11-21. PURE technology. Shimizu, Y, Ueda, T.).

For example, there have been many reports of methods using a stop codon as a codon for introducing an unnatural amino acid. By using the PURESYSTEM mentioned above, synthesis systems can be constructed excluding natural amino acids and ARSs. This allows assignment of the codons encoding the excluded natural amino acids to unnatural amino acids (J. Am. Chem. Soc. 2005; 127: 11727-35. Ribosomal synthesis of unnatural peptides. Josephson, K., Hartman, MC., Szostak, JW.). Furthermore, unnatural amino acids can be added without the exclusion of natural amino acids by breaking codon degeneracy (Kwon, I., et al., Breaking the degeneracy of the genetic code. J. Am. Chem. Soc. 2003, 125, 7512-3.). Peptides containing *N*-methylamino acids can be ribosomally synthesized by utilizing the cell-free translation systems such as PURESYSTEM.

More specifically, ribosomal synthesis can be carried out, for example, by the addition of mRNA to a known cell-free translation system such as the PURESYSTEM in which protein factors necessary for translation in *E. coli* (methionyl-tRNA transformylase, EF-G, RF1, RF2, RF3, RRF, IF1, IF2, IF3, EF-Tu, EF-Ts and ARS (necessary ones are selected from AlaRS, ArgRS, AsnRS, AspRS, CysRS, GlnRS, GluRS, GlyRS, HisRS, IleRS, LeuRS, LysRS, MetRS,

PheRS, ProRS, SerRS, ThrRS, TrpRS, TyrRS and ValRS)), ribosome, amino acids, creatine kinase, myokinase, inorganic pyrophosphatase, nucleoside diphosphate kinase, E. coli-derived tRNAs, creatine phosphate, potassium glutamate, HEPES-KOH (pH 7.6), magnesium acetate, spermidine, dithiothreitol, GTP, ATP, CTP, UTP and the like are appropriately selected and mixed. Also, addition of T7 RNA polymerase enables coupled transcription/translation from template DNAs containing T7 promoter. In addition, a group of desired aminoacyl tRNAs and a group of unnatural amino acids (for example, F-Tyr) acceptable by aminoacyl tRNA synthetases (ARSs) can be added to a system to ribosomally synthesize peptide compounds containing the unnatural amino acids (Kawakami, T., et al. Ribosomal synthesis of polypeptoids and peptoid-peptide hybrids. J. Am. Chem. Soc. 2008, 130, 16861-3; Kawakami, T., et al. Diverse backbone-cyclized peptides via codon reprogramming. Nat. Chem. Biol. 2009, 5, 888-90). Furthermore, peptide compounds containing unnatural amino acids can also be ribosomally synthesized by adding variants of ARSs instead of or in addition to natural ARSs, and also adding a group of unnatural amino acids in the system. Alternatively, the translational incorporation efficiency of unnatural amino acids may be increased by using variants of ribosome, EF-Tu, and the like (Dedkova LM, et al. Construction of modified ribosomes for incorporation of D-amino acids into proteins. Biochemistry. 2006, 45, 15541-51; Doi Y, et al. Elongation factor Tu mutants expand amino acid tolerance of protein biosynthesis system. J Am Chem Soc. 2007, 129, 14458-62; Park HS, et al. Expanding the genetic code of Escherichia coli with phosphoserine. Science. 2011, 333, 1151-4).

In a non-limiting embodiment, the cell-free translation systems herein (also referred to as "translation system(s) herein") may be translation systems for producing peptide compounds, and are preferably translation systems for producing cyclic peptide compounds.

In a non-limiting embodiment, the translation systems herein may contain 5 to 32, 5 to 28, or 5 to 20 unnatural amino acids, and preferred examples include 8 to 20, 10 to 20, and 13 to 20 unnatural amino acids. In one embodiment, 50% or more, 60% or more, 70% or more, or 80% or more of the amino acid species included in the translation systems herein may be unnatural amino acids.

In a non-limiting embodiment, the translation systems herein may contain 5 to 28, or 5 to 20 *N*-substituted amino acids, and preferred examples include 5 to 18 and 5 to 15 *N*-substituted amino acids. In one embodiment, 40% or more, 50% or more, 60% or more, or 70% or more of the amino acid species included in the translation systems herein may be *N*-substituted amino acids. Here, the *N*-substituted amino acids may mean *N*-alkylamino acids or *N*-methylamino acids. However, even if this is the case, it does not exclude cases where the translation systems herein contain other *N*-substituted amino acids.

In a non-limiting embodiment, the translation systems herein may be adjusted such that the average of the number of aromatic rings included in the peptide compounds produced using the translation systems will be within a given range. For example, the translation systems herein may be adjusted such that in the ribosomally-synthesized peptide compounds, the average of the percentage of the number of amino acids having an aromatic ring to the number of amino acids constituting the cyclic portion becomes 40% or less, 35% or less, 30% or less, 27% or less, 25% or less, or 20% or less; or the average of the number of aromatic rings included in the side chains of the cyclic portion of each peptide compound having a cyclic portion composed of 8 to 11 amino acids will be 0 to 3. While the adjusting methods are not particularly limited, examples include adjusting the number of aromatic ring-containing amino acid species accounting for the number of amino acid species included in the translation system.

In a non-limiting embodiment, the translation systems herein may include aromatic ring-containing amino acids, and the percentage of the number of aromatic ring-containing amino acid species to the number of amino acid species included in the translation system is preferably 40% or less, and preferred examples include 35% or less, 30% or less, 27% or less, 25% or less, and 20% or less.

In a non-limiting embodiment, the cell-free translation systems herein may be cell-free translation systems for producing a peptide compound herein containing at least one, two or more, or three or more amino acid types herein. While not being limited thereto, such cell-free translation systems may comprise the following:

(i) tRNAs to which at least one, two or more, or three or more amino acid types herein are linked; and
(ii) a nucleic acid encoding the peptide compound;

wherein the nucleic acid may contain at least one of the codons corresponding to the anticodons of the tRNAs.

<u>tRNAs</u>

For translational incorporation of unnatural amino acids into peptides, aminoacylation of tRNAs that are orthogonal and efficiently incorporated into ribosomes is necessary ((i) Biochemistry. 2003; 42: 9598-608. Adaptation of an orthogonal archaeal leucyl-tRNA and synthetase pair for four-base, amber, and opal suppression. Anderson, JC., Schultz, PG.; and (ii) Chem. Biol. 2003; 10: 1077-84. Using a solid-phase ribozyme aminoacylation system to reprogram the genetic code. Murakami, H., Kourouklis, D., Suga, H.). The following five methods can be used as methods for aminoacylating tRNAs.

Within cells, aminoacyl tRNA synthetases (ARS) for respective amino acids are provided as enzymes for aminoacylating tRNAs. Therefore, the first method includes methods of utilizing the fact that certain ARSs accept unnatural amino acids such as *N*-Me His, or methods of preparing and using mutant aminoacyl tRNA synthetases that accept unnatural amino acids ((i) Proc. Natl. Acad. Sci. USA. 2002; 99: 9715-20. An engineered Escherichia coli tyrosyl-tRNA synthetase for site-specific incorporation of an unnatural amino acid into proteins in eukaryotic translation and its application in a wheat germ cell-free system. Kiga, D., Sakamoto, K., Kodama, K., Kigawa, T., Matsuda, T., Yabuki, T., Shirouzu, M., Harada, Y, Nakayama, H., Takio, K., Hasegawa, Y., Endo, Y., Hirao, I., Yokoyama, S.; (ii) Science. 2003; 301: 964-7. An expanded eukaryotic genetic code. Chin, JW., Cropp, TA., Anderson, JC., Mukherji, M., Zhang, Z., Schultz, PG., Chin, JW.; and (iii) Proc. Natl. Acad. Sci. USA. 2006; 103: 4356-61. Enzymatic aminoacylation of tRNA with unnatural amino acids. Hartman, MC., Josephson, K., Szostak, JW.). Second, a method in which tRNAs are aminoacylated *in vitro,* and then the amino acids are chemically modified can also be used (J. Am. Chem. Soc. 2008; 130: 6131-6. Ribosomal synthesis of N-methyl peptides. Subtelny, AO., Hartman, MC., Szostak, JW.). Third, tRNAs in which CA has been removed from the 3'-end CCA sequence can be linked with a separately prepared aminoacylated pdCpA by using RNA ligase to obtain aminoacyl tRNAs (Biochemistry. 1984; 23: 1468-73. T4 RNA ligase mediated preparation of novel "chemically misacylated" tRNAPheS. Heckler, TG., Chang, LH., Zama, Y., Naka, T., Chorghade, MS., Hecht, SM.). There is also aminoacylation by flexizymes, which are ribozymes that allow tRNAs to carry active esters of various unnatural amino acids (J. Am. Chem. Soc. 2002; 124: 6834-5. Aminoacyl-tRNA synthesis by a resin-immobilized ribozyme. Murakami, H., Bonzagni, NJ., Suga, H.). Fourth, a method in which tRNA and the active ester of an amino acid are ultrasonically agitated within cationic micelles can also be used (Chem. Commun. (Camb). 2005; (34): 4321-3. Simple and quick chemical aminoacylation of tRNA in cationic micellar solution under ultrasonic agitation. Hashimoto, N., Ninomiya, K., Endo, T., Sisido, M.). Fifth, aminoacylation is also possible by linking an amino acid active ester to a PNA that is complementary to a sequence close to the 3'-end of a tRNA and adding this to the tRNA (J. Am. Chem. Soc. 2004; 126: 15984-9. In situ chemical aminoacylation with amino acid thioesters linked to a peptide nucleic acid. Ninomiya, K., Minohata, T., Nishimura, M., Sisido, M.).

More specifically, aminoacyl tRNAs can be prepared using methods such as the following. A template DNA encoding a desired tRNA sequence upstream of which a T7, T3 or SP6 promoter is placed is prepared. RNA can be synthesized by transcription of the DNA using an RNA polymerase compatible with the promoter, such as T7 RNA polymerase, or T3 or SP6 RNA polymerase. tRNAs can also be extracted from cells and purified, and a generated tRNA of interest can be extracted therefrom by using a probe having a sequence complementary to the tRNA sequence. In such extraction, cells transformed with an expression vector for the tRNA of interest may be used as a source. RNA with a desired sequence may also be synthesized chemically. For example, the tRNA thus obtained in which CA has been removed from the 3'-end CCA sequence may be linked to a separately prepared aminoacylated pdCpA or pCpA by RNA ligase to obtain an aminoacyl tRNA (pdCpA method, pCpA method). Such tRNAs are useful in the preparation of peptide compounds. Alternatively, aminoacyl tRNAs can also be prepared by preparing full-length tRNAs and aminoacylating them using flexizymes, which are ribozymes that enable tRNAs to carry active esters of various unnatural amino acids. Without any limitation intended, aminoacyl tRNAs can also be prepared using native ARSs or variants thereof. When native ARSs or variants thereof are used, the aminoacyl tRNAs once consumed in the translation system can be regenerated by the native ARSs or variants thereof; therefore, there is no need for aminoacyl tRNAs prepared in advance to exist in a large amount in the translation system. Such ARS variants are described in WO 2016/148044. These methods for preparing aminoacyl tRNAs can also be combined appropriately.

Formation of cyclic portions

In a non-limiting embodiment, the cyclic portion of a peptide compound is formed by subjecting a linear peptide compound to a cyclization reaction.

The term "cyclization reaction" as used herein refers to a reaction that forms a cyclic portion in the peptide moiety of a peptide compound. In one embodiment, peptide compounds herein also include peptide compounds obtained by further chemically modifying or reacting a peptide compound where the cyclic portion is formed by cyclization reaction. Chemical modification or the like may also be performed before cyclization reaction.

Scheme A shows an example of cyclization reaction of the peptide moiety of a peptide compound herein. The black circle (•) units (main chain units), D unit (aspartate unit), and triangle (▲) unit (cyclized N-terminal unit) each represent an amino acid residue constituting the peptide moiety. White circle represents resin for solid synthesis. In scheme A, the cyclic portion refers to a moiety consisting of one triangle unit, nine black circle units and one D unit. The respective units may be the same or different amino acids. The cyclic peptides of the present invention can be produced, for example, by the methods described in WO 2013/100132.

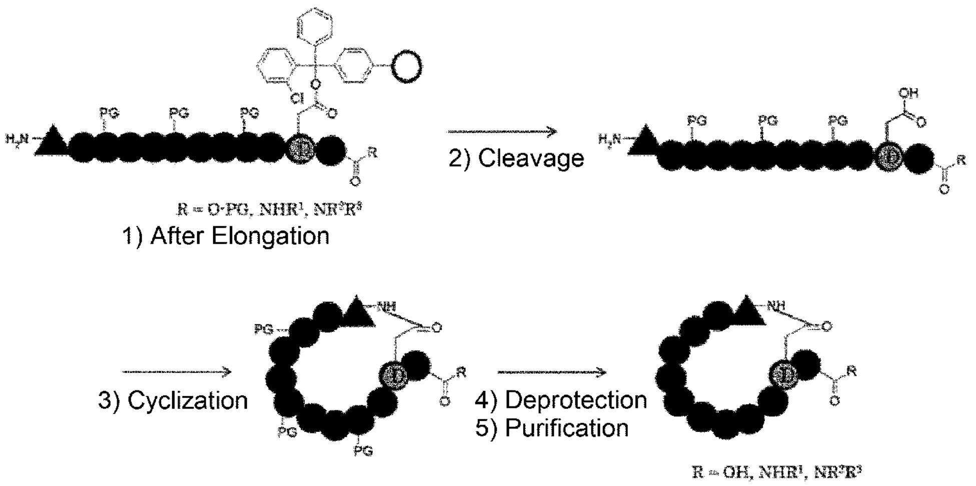


Scheme A

The term "unit" as used herein refers to an amino acid after synthesis of a linear peptide compound herein and before cyclization, after cyclization, or at the time of completion of chemical modification after cyclization. Examples of amino acid residues at the time of completion of chemical modification after cyclization include amino acid residues in which amino acid residues are chemically or skeletally transformed by chemical modification after peptide compound production.

Libraries

In a non-limiting embodiment, the libraries of peptide compounds in the present disclosure or nucleic acids encoding them respectively may be libraries substantially consisting of peptide compounds herein or nucleic acids encoding them.

The libraries herein include libraries comprising the peptide compounds herein, and libraries comprising nucleic acids encoding the peptide compounds herein. The libraries herein include libraries of the peptide compounds and libraries of peptide compound-nucleic acid complexes herein. Among them, libraries of cyclic peptide compounds or libraries of cyclic peptide-nucleic acid complexes are preferred, and libraries of cyclic peptide-mRNA complexes are particularly preferred. Preferred libraries are display libraries. Examples of display libraries include display-utilizing libraries, and among them, mRNA display libraries, DNA display libraries, and ribosome display libraries are preferred, and mRNA display libraries are more preferred.

Without wishing to be bound by a particular theory, the present inventors contemplate as follows. In conventional peptide libraries, masking a proton donor contained in the side chain of an amino acid that constitutes a peptide by forming an intramolecular hydrogen bond relies on chance. In particular, in peptide libraries that confer diversity by randomly placing constituting amino acids, it was difficult to allow formation of a hydrogen bond between amino acids as intended. In peptide compounds containing the amino acids herein, an intramolecular hydrogen bond can be intentionally formed in the side-chain moiety of the peptide compounds since amino acids in which a hydrogen bond is already formed in a proton donor site contained in the side chains of the amino acids are used, and thus in libraries of peptide compounds obtained using the amino acids herein, peptide compounds capable of masking a side-chain proton donor by formation of an intramolecular hydrogen bond can emerge highly frequently, or more specifically, the probability of containing peptide compounds having high membrane permeability can be increased.

Herein, a peptide compound may be "a peptide compound encoded by a nucleic acid". Herein "a peptide compound encoded by a nucleic acid" is not limited to a peptide compound directly synthesized by translating the nucleic acid as a template, and may include a peptide compound that can be synthesized as a result of posttranslational modification. For example, when a linear peptide compound is synthesized by translating a nucleic acid and this is followed by a cyclization step to synthesize a cyclic peptide compound, this cyclic peptide compound may be called a cyclic peptide compound encoded by the aforementioned nucleic acid. Furthermore, in one embodiment, complexes between a peptide compound ribosomally synthesized using a nucleic acid as a template or a peptide compound that has undergone subsequent posttranslational modification and the nucleic acid (peptide compound-nucleic acid complexes) are also included in the (cyclic) peptide compound encoded by a nucleic acid.

In a non-limiting embodiment, the average of the number of unnatural amino acids, *N*-substituted amino acids, or aromatic ring-containing amino acids included in each peptide compound included in a library or the peptide compound encoded by each nucleic acid included in a library herein can be adjusted by means of the frequency of appearance of

a codon assigned to each amino acid in the synthesis of a DNA library. For example, for a library of peptides containing 10 variable amino acid residue sites, when it is supposed that there are 20 amino acid species to choose from, 10 out of the 20 amino acid species can be *N*-substituted amino acids to synthesize the library such that codon units for the *N*-substituted amino acids account for 10/20 (50%) per variable site. In this way, the average of the percentage of the number of unnatural amino acids, the number of *N*-substituted amino acids, the number of aromatic rings, or the number of aromatic ring-containing amino acids can be calculated.

Regarding the libraries of peptide compounds and/or the libraries of nucleic acids encoding such compounds herein, "substantially consist of' means that the percentage of the theoretical number of variations of the mentioned peptide compounds and/or nucleic acids encoding these compounds to the theoretical total number of variations of the peptide compounds and/or the nucleic acids encoding these compounds included in the library may be 90% or higher, 93% or higher, 95% or higher, 97% or higher, 98% or higher, or 99% or higher, but is not limited thereto.

In the libraries herein, when determining the "theoretical (total) number of variations" of peptide compounds, compounds that cannot actually be produced as peptide compounds are not included in the calculation of the theoretical (total) number. For example, in the following cases (1) and (2), since the corresponding amino acids are not ribosomally synthesized, peptide compounds containing such amino acids are not included in the calculation of the theoretical (total) number: (1) a case where amino acids are added to the translation solution as ingredients for a peptide compound, but nucleic acids encoding those amino acids are not included as templates; and (2) a case where nucleotide sequences are included in nucleic acid templates for a peptide compound, but a translation solution not containing the corresponding amino acids is used. Furthermore, when byproducts or unreacted substances are present in the process of producing cyclic peptide compounds, those compounds are not included in the calculation of the aforementioned theoretical (total) number.

For example, when 11-residue peptides are ribosomally synthesized using 20 natural amino acid species in total from nucleotide sequences with randomly-arranged codon units according to a codon table in which each of the codon units corresponds to one amino acid species, the theoretical number of variations becomes $20^{10}$ if the translation initiation amino acid is fixed to methionine.

In a non-limiting embodiment, the libraries herein may comprise, in addition to peptide compounds and/or nucleic acids encoding these compounds, other components necessary for screening for peptide compounds that can specifically bind to target molecules. Furthermore, it may comprise other components to the extent that they do not give negative influence on the effects of the libraries herein.

Libraries herein comprise a plurality of peptide compounds or nucleic acids encoding these compounds. Herein, the number of variations (types) of peptide compounds included in a library is referred to as "diversity". While the diversity of the peptide compounds or nucleic acids encoding these compounds included in a library herein is not particularly limited, examples include $10^3$ or more, $10^4$ or more, $10^5$ or more, $10^6$ or more, $10^7$ or more, $10^8$ or more, $10^9$ or more, $10^{10}$ or more, $10^{11}$ or more, and $10^{12}$ or more. The diversity is not limited to measured values and may be theoretical values.

In a non-limiting embodiment, the average of the molecular weight of the peptide moiety excluding the nucleic acid-linked portion of each peptide compound, or the average of the molecular weight of the cyclic portion of each cyclic peptide compound included in a library or the peptide compound encoded by each nucleic acid included in a library herein, may be 500 to 2000.

## Display libraries

The display library refers to a library in which peptides as phenotypes are associated with their peptide-encoding RNAs or DNAs as genotypes. By using the library, peptide compounds that can specifically bind to a target molecule can be identified. For example, the library is contacted with desired immobilized targets, and peptides binding to the targets can be enriched by washing away molecules unbound with the targets (panning). The gene information associated with the peptides selected through such a process can be analyzed to determine the sequences of the peptides bound to the targets. For example, a method using the nonspecific conjugation of an antibiotic puromycin, an aminoacyl-tRNA analog, to proteins during their mRNA translation elongation in the ribosome has been reported as mRNA display (Proc Natl Acad Sci USA. 1997; 94: 12297-302. RNA-peptide fusions for the in vitro selection of peptides and proteins. Roberts RW, Szostak JW.) or *in vitro* virus (FEBS Lett. 1997; 414: 405-8. In vitro virus: bonding of mRNA bearing puromycin at the 3'-terminal end to the C-terminal end of its encoded protein on the ribosome in vitro. Nemoto N, Miyamoto-Sato E, Husimi Y, Yanagawa H.).

By conjugating spacers such as puromycin to the 3'-ends of an mRNA library obtained by transcription from a DNA library containing a promoter such as T7 promoter, when these mRNAs are translated into proteins in cell-free translation systems, puromycin is mistakenly recognized as amino acid by the ribosome, and is incorporated into proteins. This causes the mRNA to be linked to the proteins encoded thereby, thus a library in which mRNAs are associated with their products can be obtained. This process, which does not involve the transformation of *E. coli* or the like, attains high efficiency and can construct a large-scale display library. cDNA is synthesized from the mRNA serving as a tag containing

genetic information bound to the molecule enriched and selected by panning, and then amplified by PCR. The amplified products can be sequenced to determine the sequence of the protein linked to the desired target substance.

In addition to the mRNA display, the following libraries are known as display libraries using cell-free translation systems:

cDNA display in which peptide-encoding cDNAs are linked to peptide-puromycin complexes (Nucleic Acids Res. 2009; 37(16): e108. cDNA display: a novel screening method for functional disulfide-rich peptides by solid-phase synthesis and stabilization of mRNA-protein fusions.Yamaguchi J, Naimuddin M, Biyani M, Sasaki T, Machida M, Kubo T, Funatsu T, Husimi Y, Nemoto N.);

ribosome display which utilizes characteristics that ribosome-translation product complexes during mRNA translation is relatively stable (Proc Natl Acad Sci USA. 1994; 91: 9022-6. An in vitro polysome display system for identifying ligands from very large peptide libraries. Mattheakis LC, Bhatt RR, Dower WJ.);

covalent display which utilizes characteristics that bacteriophage endonuclease P2A forms covalent bond with DNAs (Nucleic AcidsRes. 2005; 33: e10. Covalent antibody display--an in vitro antibody-DNA library selection system. Reiersen H, Lobersli I, Loset GA, Hvattum E, Simonsen B, Stacy JE, McGregor D, Fitzgerald K, Welschof M, Brekke OH, Marvik OJ.); and CIS display which utilizes characteristics that a microbial plasmid replication initiator protein RepA binds to a replication origin ori (Proc Natl Acad Sci U S A. 2004; 101: 2806-10. CIS display: In vitro selection of peptides from librariesof protein-DNA complexes. Odegrip R, Coomber D, Eldridge B, HedererR, Kuhlman PA, Ullman C, FitzGerald K, McGregor D.). Also, *in vitro* compartmentalization is known in which a transcription-translation system is encapsulated into a water-in-oil emulsion or liposome per DNA molecule constituting a DNA library, and subjected to translation reaction (Nat Biotechnol. 1998; 16: 652-6. Man-made cell-like compartments for molecular evolution. Tawfik DS, Griffiths AD.). The methods described above can be employed appropriately using known methods.

Nucleic acid libraries

The "nucleic acid" in the present invention can also include deoxyribonucleic acids (DNA), ribonucleic acids (RNA), and nucleotide derivatives having an artificial base. Peptide nucleic acids (PNA) can also be included. The nucleic acid of the present invention can be any of or a hybrid of these nucleic acids as long as the desired genetic information is retained. More specifically, a DNA-RNA hybrid nucleotides and chimeric nucleic acids in which different nucleic acids such as DNA and RNA are connected in a single strand are also included in the nucleic acid in the present invention.

Examples of the library of nucleic acids that serve as templates for peptide compounds contained in the peptide compound library include a mRNA library and a DNA library. Nucleic acid libraries can be obtained by mixing bases to sites where amino acid residues are not fixed on a peptide sequence and synthesizing . For example, they can be synthesized as repetition of triplets for mixture (N) of 4 types of bases, *i.e.*, A, T, G, and C as a DNA library and A, U, G, and C, as an RNA library, or as those in which the first and second letters in each codon is N and the third letter is mixture of two types of bases such as W, M, K, or S. There is another method in which the third base may be set to one type if types of amino acids introduced are reduced to 16 or fewer. The frequency of emergence of amino acid residues can be freely regulated by providing codon units corresponding to the three letters of codons and mixing the units in desired proportions for use in synthesis.

These nucleic acid libraries can be translated by using cell-free translation systems. When using the cell-free translation systems, a spacer-encoding sequence is preferably included downstream of the nucleic acid of interest. The spacer sequences include, but are not limited to, sequences containing glycine or serine. In addition, it is preferred that a linker formed by RNA, DNA, hexaethylene glycol (spc18) polymers (*e.g.*, 5 polymers) or the like is contained between the nucleic acid library and a compound which is incorporated into a peptide during ribosomal translation such as puromycin or derivative thereof.

Methods of preparing libraries

In a non-limiting embodiment, the production of the libraries in the present disclosure can be carried out according to the above-described methods of producing peptide compounds in the present disclosure, and can appropriately be combined with known methods. In one embodiment, the peptide compound libraries in the present disclosure can be produced using the above-described cell-free translation systems in the present disclosure. More specifically, the library production methods in the present disclosure may comprise the step of synthesizing peptide compounds using the cell-free translation systems in the present disclosure. In one embodiment, the examples, preferred ranges, and embodiments described for the cell-free translation systems in the present disclosure can also be applied, as they are, to the library production methods in the present disclosure.

In a non-limiting embodiment, mRNA display libraries can be prepared as follows. First, a library of DNAs in

which desired sequences are positioned downstream of a promoter such as T7 promoter is chemically synthesized, and this is used as a template to produce double-stranded DNAs by primer extension reaction. The double-stranded DNAs are used as templates and transcribed into mRNAs by using RNA polymerase such as T7 RNA polymerase. The 3'-end of these RNAs is conjugated to a linker (spacer) to which the aminoacyl-tRNA analog antibiotic puromycin or such is attached. The resulting conjugates are added to a known cell-free translation system such as the above-mentioned PURESYSTEM, and incubated so that the mRNAs are translated, and as a result each mRNA is linked to the peptide encoded thereby through the linker containing puromycin or such. In this way, a display library composed of mRNA-product complexes in which the mRNAs are associated with their products can be constructed. The linker can contain additional spacers well-known to those skilled in the art. Furthermore, when necessary, posttranslational modifications such as cyclization can be performed by the above-described methods or known methods.

In a non-limiting embodiment, the libraries in the present disclosure may be peptide compound libraries comprising peptide compounds each of which contains at least one, two or more, or three or more amino acid types selected from the group consisting of the amino acids described herein. Methods of producing such libraries may include the step of ribosomally synthesizing peptide compounds using a cell-free translation system comprising the following:

(i) tRNAs to which at least one, two or more, or three or more amino acid types selected from the group consisting of the amino acids described herein are linked; and
(ii) a nucleic acid library encoding the peptide compound library;

wherein the nucleic acid library may contain nucleic acids carrying at least one codon corresponding to the anticodon of the tRNA.

All prior art documents cited in the present specification are incorporated herein by reference.

Examples

The present invention is further illustrated by the following Examples, but is not limited thereto.

The abbreviations recited in the following abbreviation tables were used in Examples.

[Table 1]

| Abbreviation | Name |
|---|---|
| 2-MeTHF | 2-Methyltetrahydrofuran |
| AA or $AcONH_4$ | Ammonium Acetate |
| Alloc | Allyloxycarbonyl |
| | 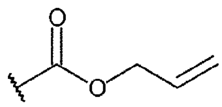 |
| Alloc—OSu | N-(Allyloxycarbonyloxy)succinimide |
| $BF_3 \cdot OEt_2$ | Boron trifluoride - diethyl ether complex |
| $Boc_2O$ | Di-tert-butyl dicarbonate |
| CDI | Carbonyldiimidazole |
| $CH_2CN$ | Cyanomethyl group |
| Clt | 2-Chlorotrityl group |

(continued)

| | |
|---|---|
| | 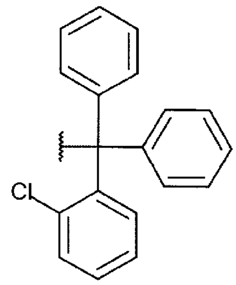 |
| DBU | 1,8-Diazabicyclo[5.4.0]-7-undecene |
| DCE | Dichloroethane |
| DCM | Dichloromethane |
| DEAD | Diethyl azocarboxylate |
| DIBAL | Diisobutylaluminum hydride |
| DIC | Diisopropylcarbodiimide |
| DIPEA | N-Ethyl-isopropylpropan-2-amine or N,N-Diisopropylethylamine |
| DMF | N,N-Dimehylformamide |
| DMSO | Dimethylsulfoxide |
| DNs or Dinitronosyl group | 2,4-Dinitrobenzenesulfonyl group |
| | 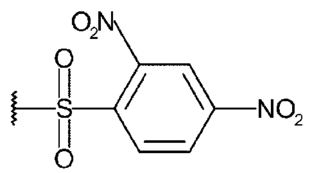 |
| $Et_3SiH$ | Triethylsilane |
| EtOH | Ethanol |
| FA | Formic acid |
| Abbreviation | Name |
| Fmoc — Cl | (9H-Fluoren-9-yl)methyl carbonochloridate |
| Fmoc-OSu | (9H-Fluoren-9-yl)methyl (2,5-dioxopyrrolidin-1-yl) carbonate or N-(9-Fluorenylmethoxycarbonyloxy) succinimide or 9-Fluorenylmethyl N-succinimidyl carbonate |
| HATU | O-(7-Aza-1H-benzotriazol-1-yl)-N,N,N,N-tetramethyluronium hexafluorophosphate |
| HFIP | 1,1,1,3,3,3-Hexafluoroisopropylalcohol |
| HOAt | 1-Hydroxy-7-azabenzotriazole |
| HOBt | 1-Hydroxybenzotriazole |
| $LiAlH_4$ | Lithium aluminium hydride |
| MeCN | Acetonitrile |
| MeOH | Methanol |
| NaHMDS | Sodium bis(trimethylsilyl)amide |

(continued)

| Abbreviation | Name |
|---|---|
| NaOtPen | Sodium t-pentoxide |
| NMP | N-Methyl-2-pyrrolidone |
| Ns or Nosyl group | 2-Nitrobenzenesulfonyl group |
| | 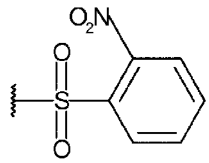 |
| PMHS | Poly(methylhydrosiloxane) |
| $PPh_3$ | Triphenylphosphine |
| PPTS | Pyridinium p-toluenesulfonate |
| T3P | Propylphosphonic acid anhydride |
| TBAF | Tetrabutylammonium fluoride |
| TBME | t-Butyl methyl ether |
| TFA | Trifluoroacetic acid |
| TFE | 2,2,2-Trifluoroethanol |
| THF | Tetrahydrofuran |
| TIPS | Triisopropylsilane |
| TMDS | Tetramethyldisiloxane |
| TsOH | p-Toluenesulfonic acid or Tosyl acid |
| WSC·HCl or EDCI·HCl | 1-(3-Dimethylaminopropyl)-3-ethylcarbodiimide hydrochloride or 1-Ethyl-3-(3-dimethylaminopropyl) carbodiimide hydrochloride |

Peptide synthesis grade solvents (purchased from Watanabe Chemical Industries and Wako Pure Chemical Industries) were used for peptide synthesis and solid-phase synthesis. Examples include DCM, DMF, NMP, 2% DBU in DMF, and 20% piperidine in DMF. Dehydrated solvents, ultradehydrated solvents, and anhydrous solvents (purchased from Kanto Chemical, Wako Pure Chemical Industries, and others) were used for reactions in which water was not added as a solvent.

The LC/MS analytical conditions are as follows.

[Table 2]

| Analytical Condition | Device | Column (I.D.xLength) (mm) | Mobile Phase | Gradient (A/B) | Flow Rate (mL/min) | Column Temp. (°C) | Wavelength |
|---|---|---|---|---|---|---|---|
| SQDAA05 | Acquity UPLC/SQD | Ascentis Express C18 (2.1×50) | A)10mM $AcONH_4$, $H_2O$<br>B)MeOH | 95/5=> 0/100(1.0 min)<br>=> 0/100(0.4 min) | 1.0 | 35 | 210-400nm PDA total |
| SQDAA50 | Acquity UPLC/SQD | Ascentis Express C18 (2.1×50) | A)10mM $AcONH_4$, $H_2O$<br>B)MeOH | 50/50=> 0/100(0.7 min)<br>=> 0/100(0.7 min) | 1.0 | 35 | 210-400nm PDA total |
| SQDFA05 | Acquity UPLC/SQD | Ascentis Express C18 (2.1×50) | A)0.1% FA,$H_2O$<br>B)0.1% FA,MeCN | 95/5=> 0/100(1.0 min)<br>=> 0/100(0.4 min) | 1.0 | 35 | 210-400nm PDA total |
| SQDFA40 | Acquity UPLC/SQD | Ascentis Express C18 (2.1×50) | A)0.1% FA,$H_2O$<br>B)0.1% FA,MeCN | 40/60=> 0/100(1.0 min)<br>=> 0/100(0.4 min) | 1.0 | 35 | 254nm |
| SQDFA50 | Acquity UPLC/SQD | Ascentis Express C18 (2.1×50) | A)0.1% FA,$H_2O$<br>B)0.1% FA,MeCN | 50/50=> 0/100(0.7 min)<br>=> 0/100(0.7 min) | 1.0 | 35 | 210-400nm PDA total |
| SQDAA05-2 | Acquity UPLC I-Class /SQD | Ascentis Express C18 (2.1×50) | A)10mM $AcONH_4$, $H_2O$<br>B)MeOH | 95/5=> 0/100(1.0 min)<br>=> 0/100(0.4 min) | 0.9 | 35 | 210-400nm PDA total |
| SQD2AA05 | Acquity UPLC/SQD2 | Ascentis Express C18 (2.1×50) | A)10mM $AcONH_4$, $H_2O$<br>B)MeOH | 95/5=> 0/100(1.0 min)<br>=> 0/100(0.4 min) | 1.0 | 35 | 210-400nm PDA total |
| SQD2AA50 | Acquity UPLC/SQD2 | Ascentis Express C18 (2.1×50) | A)10mM $AcONH_4$, $H_2O$<br>B)MeOH | 50/50=> 0/100(0.7 min)<br>=> 0/100(0.7 min) | 1.0 | 35 | 210-400nm PDA total |
| SQD2FA05 | Acquity UPLC/SQD2 | Ascentis Express C18 (2.1×50) | A)0.1% FA,$H_2O$<br>B)0.1% FA,MeCN | 95/5=> 0/100(1.0 min)<br>=> 0/100(0.4 min) | 1.0 | 35 | 210-400nm PDA total |
| SQD2FA50 | Acquity UPLC/SQD2 | Ascentis Express C18 (2.1×50) | A)0.1% FA,$H_2O$<br>B)0.1% FA,MeCN | 50/50=> 0/100(0.7 min)<br>=> 0/100(0.7 min) | 1.0 | 35 | 210-400nm PDA total |
| SQD2FA50L | Acquity UPLC/SQD2 | Ascentis Express C18 (2.1×50) | A)0.1% FA,$H_2O$<br>B)0.1% FA,MeCN | 50/50=> 0/100(4.5 min)<br>=> 0/100 (0.5 min) | 1.0 | 35 | 210-400nm PDA total |

(continued)

| Analytical Condition | Device | Column (I.D. × Length) (mm) | Mobile Phase | Gradient (A/B) | Flow Rate (mL/min) | Column Temp. (°C) | Wavelength |
|---|---|---|---|---|---|---|---|
| SMD method5 | Shimadzu LCMS-2020 LC-20AD | Shim-Pack XR-ODS (3.0x50) | A)0.05% TFA,$H_2O$ B)0.05% TFA, MeCN | 95/5=> 0/100(2.2 min)=> 0/100(1.0 min) =>95/5(0.1 min)=>95/5(0.3 min) | 1.0 | 40 | 190-800nm PDA total |
| SMD method6 | Nexera/2020 | Speed Core C18 (2.1×50) | A)0.1% FA,$H_2O$ B)0.1% FA,MeCN | 95/5=> 0/100(1.5 min) => 0/100(0.5 min) | 1.0 | 35 | 210-400nm PDA total |
| SMD method7 | Shimadzu LCMS-2020 LC-20AD | Shim-Pack XR-ODS (3.0x50) | A)0.05% TFA,$H_2O$ B)0.05% TFA, MeCN | 95/5=> 0/100(1.2 min)=> 0/100(1.0 min) =>95/5(0.1 min) | 1.0 | 40 | 190-800nm PDA total |
| SMD method9 | Shimadzu LCMS-2020 LC-20ADXR | phenomenex kinetex (3.0x50) | A)0.1% FA,$H_2O$ B)0.1% FA,MeCN | 90/10=> 0/100(1.2 min)=> 0/100(0.5 min) => 90/10(0.1 min) | 1.5 | 40 | 190-400nm! PDA total |
| SMD method10 | Shimadzu LCMS-2020 LC-30AD | Poroshell HPH-C18 (3.0x50) | A)6.5mM NH4HCO3 pH10 B)MeCN | 90/10=> 5/95(1.1 min)=> 5/95(0.5 min) =>90/10(0.1 min) | 1.2 | 40 | 190-400nm; PDA total |
| SMD method11 | Shimadzu LCMS-2020 LC-30AD | Kinetex EVO C18 (2.1×50) | A)6.5mM NH4HCO3 pH10 B)MeCN | 90/10=> 5/95(1.1 min)=> 5/95(0.5 min) =>90/10(0.1 min) | 1.0 | 40 | 190-400nm PDA total |
| SMD method12 | Shimadzu LCMS-2020 LC-30AD | Poroshell HPH-C18 (3.0×50) | A)6.5mM NH4HCO3 pH10 B)MeCN | 90/10=> 5/95(1.1 min)=> 5/95(0.5 min) =>90/10(0.1 min) | 1.5 | 40 | 190-400nm; PDA total |
| SMD method13 | Shimadzu LCMS-2020 LC-20AD | Shim-Pack XR-ODS (3.0x50) | A)0.05% TFA,$H_2O$ B)0.05% TFA, MeCN | 95/5=> 0/100(1.1 min)=> 0/100(0.6 min) =>95/5 (0.05 min) | 1.2 | 40 | 190-400nm PDA total |

(continued)

| Analytical Condition | Device | Column (I.D. × Length) (mm) | Mobile Phase | Gradient (A/B) | Flow Rate (mL/min) | Column Temp. (°C) | Wavelength |
|---|---|---|---|---|---|---|---|
| SMD method14 | Shimadzu LCMS-2020 LC-20AD | Shim-Pack XR-ODS (3.0x50) | A)0.05% TFA,$H_2O$ B)0.05% TFA, MeCN | 95/5=> 5/95(3.0 min)=> 5/95(0.7 min) =>95/5 (0.05 min) | 1.2 | 40 | 190-400nm PDA total |
| Analytical Condition | Device | Column(I.D. × Length) (mm) | Mobile Phase | Gradient (A/B) | FlowRate (mL/min) | Column Temp. (°C) | Wavelength |
| SMD method15 | Shimadzu LCMS-2020 LC-20AD | Shim-Pack XR-ODS (3.0x50) | A)0.1% FA,$H_2O$ B)0.1% FA,MeCN | 90/10=> 0/100(3.0 min)=> 0/100(0.7 min) =>95/5(0.05 min) | 1.2 | 40 | 190-400nm PDA total |
| SMD method16 | Shimadzu LCMS-2020 LC-20ADXR | CORTECS C18 (2.1×50) | A)0.1% FA,$H_2O$ B)0.1% FA,MeCN | 90/10=> 0/100(1.2 min)=> 0/100(0.5 min)=>90/10(01 min) | 1.0 | 40 | 190-400nm PDA total |
| SMD method18 | Shimadzu LCMS-2020 LC-20AD | Shim-Pack XR-ODS (3.0x50) | A)0.05% TFA,$H_2O$ B)0.05% TFA, MeCN | 95/5=> 0/100(2.2 min)=> 0/100(1.0 min) =>95/5(0.1 min) | 1.0 | 40 | 190-400nm PDA total |
| SMD method20 | Shimadzu LCMS-2020 LC-30AD | Kinetex EVO C18 (2.1×50) | A)6.5mM $NH_4HCO_3$ pH10 B)MeCN | 90/10=> 5/95(2.0 min)=> 5/95(0.7 min) =>90/10(0.1 min) | 1.0 | 40 | 190-400nm PDA total |
| SMD method23 | Shimadzu LCMS-2020 LC-20ADXR | phenomenex kinetex (3.0×50) | A)0.1% FA,$H_2O$ B)0.1% FA,MeCN | 90/10=> 0/100(1.1 min)=> 0/100(0.7 min) => 90/10(0.1 min) | 1.5 | 40 | 190-400nm PDA total |
| SMD method28 | Shimadzu LCMS-2020 LC-20AD | Shim-Pack XR-ODS (3.0x50) | A)0.05% TFA,$H_2O$ B)0.05% TFA, MeCN | 95/5=> 30/70(3.8 min)=> 0/100(0.3 min) =>0/100(0.5 min)=>95/5(0.1 min) | 1.2 | 40 | 190-400 PDA total |
| SMD method31 | Shimadzu LCMS-2020 LC-20AD | Shim-Pack XR-ODS (3.0x50) | A)0.05% TFA,$H_2O$ B)0.05% TFA, MeCN | 95/5=> 0/100(1.2 min)=> 0/100(1.0 min) =>95/5(0.1 min) | 1.0 | 40 | 190-800 PDA total |

(continued)

| Analytical Condition | Device | Column(I.D. × Length) (mm) | Mobile Phase | Gradient (A/B) | FlowRate (mL/min) | Column Temp. (°C) | Wavelength |
|---|---|---|---|---|---|---|---|
| SMD method32 | Shimadzu LCMS-2020 LC-20AD | Shim-Pack XR-ODS (3.0x50) | A)0.05% TFA,$H_2O$ B)0.05% TFA, MeCN | 95/5=> 0/100(1.6 min)=> 0/100(0.6 min) =>95/5(0.1 min) | 1.0 | 40 | 190-800 PDA total |
| SMD method33 | Shimadzu LCMS-2020 LC-20AD | Shim-Pack XR-ODS (3.0x50) | A)0.05% TFA,$H_2O$ B)0.05% TFA, MeCN | 95/5=> 0/100(1.2 min)=> 0/100(1.0 min) =>95/5(0.1 min) | 1.0 | 40 | 190-400 PDA total |
| SMD method34 | Shimadzu LCMS-2020 LC-20AD | Shim-Pack XR-ODS (3.0x50) | A)0.05% TFA,$H_2O$ B)0.05% TFA, MeCN | 95/5=> 5/95(2.0 min)=> 5/95(0.7 min) =>95/5(0.05 min) | 1.2 | 40 | 190-400 PDA total |
| SMD method40 | Shimadzu LCMS-2020 LC-20ADXR | CORTECS C18 (2.1×50) | A)0.1% FA,$H_2O$ B)0.1% FA,MeCN | 90/10=> 5/95(3.0 min)=> 5/95(0.7 min) =>90/10(0.1 min)) | 1.0 | 40 | 190-400nm PDA total |
| SMD method41 | Shimadzu LCMS-2020 LC-30AD | Kinetex EVO C18 (2.1×50) | A)6.5mM NH4HCO3 pH10 B)MeCN | 90/10=> 5/95(3.0 min)=> 5/95(0.7 min) => 90/10(0.1 min) | 1.0 | 35 | 190-400nm PDA total |
| SMD method42 | Shimadzu LCMS-2020 LC-30AD | CORTECS C18 (2.1×50) | A)0.05% TFA,$H_2O$ B)0.05% TFA, MeCN | 95/5=> 5/95(3.0 min) => 5/95(0.7 min) =>95/5(0.1 min) | 1.0 | 45 | 190-400nm PDA total |
| SMD method45 | Shimadzu Nexera/ 2020 | Ascentis Express C18 (2.1×50) | A)0.1% FA,$H_2O$ B)0.1% FA,MeCN | 95/5=> 0/100(1.5 min) => 0/100(0.5 min) | 1.0 | 35 | 210-400nm PDA total |
| SMD method46 | Shimadzu Nexera/ 2020 | Ascentis Express C18 (2.1×50) | A)0.05% TFA,$H_2O$ B)0.05% TFA, MeCN | 95/5=> 0/100(4.5 min)=> 0/100(0.5 min) | 1.0 | 35 | 210-400nm PDA total |
| SMD method47 | Shimadzu Nexera/ 2020 | Ascentis Express C18 (2.1×50) | A)0.1% FA,$H_2O$ B)0.1% FA,MeCN | 95/5=> 0/100(4.5 min)=> 0/100(0.5 min) | 1.0 | 35 | 210-400nm PDA total |

(continued)

| Analytical Condition | Device | Column(I.D. × Length) (mm) | Mobile Phase | Gradient (A/B) | FlowRate (mL/min) | Column Temp. (°C) | Wavelength |
|---|---|---|---|---|---|---|---|
| SMD method49 | Shimadzu LCMS-2010EV | Shim-PackXR-ODS (3.0×50) | A)0.05% TFA,$H_2O$ B)0.05% TFA, MeCN | 95/5=> 5/95(3.0 min) => 5/95(0.7 min) =>95/5(0.05 min) | 1.2 | 40 | 190-400nm PDA total |
| SMD method50 | Shimadzu LCMS-2020 LC-30AD | Kinetex EVO C18 (2.1×50) | A)6.5mM NH4HCO3 pH10 B)MeCN | 90/10=> 5/95(1.1 min)=> 5/95(0.5 min)=> 90/10(0.1 min) | 1.0 | 35 | 190-400nm PDA total |
| SMD method51 | Shimadzu LCMS-2020 LC-20AD | Shim-PackXR-ODS (3.0×50) | A)0.05% TFA,$H_2O$ B)0.05% TFA, MeCN | 95/5=> 5/95(1.0 min)=> 5/95(0.4 min) =>95/5(0.1 min) | 1.2 | 40 | 190-400nm PDA total |
| SMD method52 | Shimadzu LCMS-2020 LC-20ADXR | Ascentis Express C18 (3.0x50) | A)0.05% TFA,$H_2O$ B)0.05% TFA, MeCN | 95/5=> 5/95(1.1 min) => 5/95(0.5 min) =>95/5(0.1 min) | 1.0 | 40 | 190-400nm PDA total |
| SQDAAs | Shimadzu Nexera/ 2020 | Ascentis Express C18 (2.1×50) | A)10mM $AcONH_4$, $H_2O$ B)MeOH | 70/30=> 0/100(8.75 min)=> 0/100 (1.25 min) | 0.5 | 50 | 210-400nm PDA total |
| SQDFAs | Shimadzu Nexera/ 2020 | XSlelectCSH C18 (2.1×50) | A)0.1% FA,$H_2O$ B)0.1% FA,MeCN | 70/30=> 10/90(7.5 min)=> 0/100(2.5 min) | 0.5 | 50 | 210-400nm PDA total |
| GC01 | Agilent GCMS 7890A-5975 | Agilent, DB-5ms (0.2×12) | MeCN | 50°C(1min)=> 40°C~300°C(6.25 min) => 300°C (1.75 min) | 1.0 | 50 | |

[Example 1] Chemical synthesis of peptide compounds

Peptides were elongated by the following basic route according to the peptide synthesis method by the Fmoc chemistry described in WO 2013/100132, specifically, by the following five steps: 1) peptide elongation reaction by the Fmoc chemistry from the N-terminus of Asp in which the Asp side chain carboxylic acid is loaded onto a 2-chlorotrityl resin, 2) a process of cleavage of a peptide from the 2-chlorotrityl resin, 3) amide cyclization by condensation between the Asp side chain carboxylic acid resulting from removal from the 2-chlorotrityl resin by the cleavage process and the N-terminal (triangle unit) amino group of the peptide chain, 4) deprotection of the protecting group for the side chain functional group contained in the peptide chain, and 5) purification of the compound by preparative HPLC. In the peptide compound containing an amino acid having an acidic functional group in the side chain, a 0.08 M solution of triethylamine hydrochloride in dichloromethane can also be used in resin washing during a peptide elongation reaction to suppress excessive peptide elongation resulting from the residual deprotecting agent. In the present Examples, unless otherwise stated particularly, peptide compounds were synthesized based on this basic route.

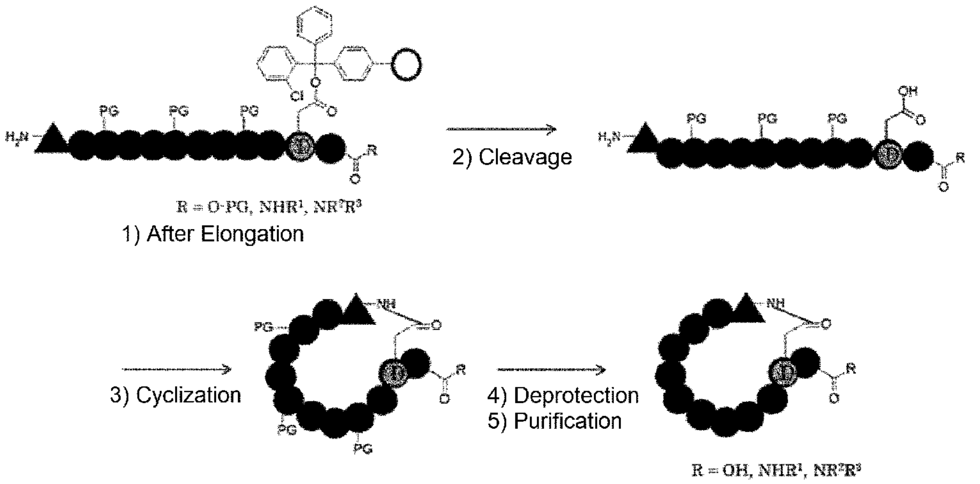


1-1. Fmoc amino acids used in peptide synthesis by a peptide synthesizer

The following Fmoc amino acids were used in peptide synthesis described herein using a peptide synthesizer. When amino acids are those having a reactive functional groups(s) in its side chain(s), those protected by an appropriate protective group(s) were used. The abbreviations for the amino acids are set forth in Table 3 below.

Fmoc-D-Val-OH, Fmoc-MeLeu-OH, Fmoc-MePhe-OH, Fmoc-MeGly-OH, Fmoc-gMeAbu-OH (may be described as Fmoc-g-MeAbu-OH), Fmoc-Leu-OH, Fmoc-Ile-OH, Fmoc-MeAla-OH, Fmoc-MeVal-OH, Fmoc-D-Abu-OH, Fmoc-Val-OH, Fmoc-MeAbu-OH, Fmoc-MeIle-OH, Fmoc-D-Leu-OH, Fmoc-Ser(tBu)-OH, Fmoc-D-Ala-OH, Fmoc-Tyr(Clt)-OH, Fmoc-Phe-OH, Fmoc-Phe(4-$CF_3$)-OH, Fmoc-Ala-OH, Fmoc-Hph-OH, Fmoc-Gly-OH, Fmoc-b-MeAla-OH, Fmoc-Ala(3-Pyr)-OH, Fmoc-D-MeAla-OH, Fmoc-Phe{#$(CH_2)_2$}-OH, Fmoc-Ala(Thz)-OH, Fmoc-D-Pro-OH, Fmoc-Pro-OH, Fmoc-Ser(Bn)-OH, and others were purchased from Watanabe Chemical Industries, Chempep, Chem-Impex, or Bachem. Fmoc-nPrGly-OH, Fmoc-MePhe(3-Cl)-OH, Fmoc-MeAla(4-Thz)-OH, and others were synthesized by the method described in WO 2013/100132. Fmoc-Thr(THP)-OH (Compound aa01), Fmoc-nBuGly-OH (Compound aa02), Fmoc-Ser(iPen)-OH (Compound aa04), Fmoc-MeHph-OH (Compound aa05), Fmoc-Ser(3-F-5-Me-Pyr)-OH (Compound aa12), Fmoc-Ser(Ph-3-Cl)-OH (Compound aa16), Fmoc-Nle(6-OTHP)-OH (Compound aa17), Fmoc-Ser(EtOTHP)-OH (Compound aa22), Fmoc-MeSer(EtOTHP)-OH (Compound aa27), Fmoc-Ser(S-2-PrOTHP)-OH (Compound aa34), Fmoc-MeSer(S-2-PrOTHP)-OH (Compound aa39), Fmoc-Ser(R-2-PrOTHP)-OH (Compound aa46), Fmoc-MeSer(R-2-PrOTHP)-OH (Compound aa51), Fmoc-Ser(tBuOTHP)-OH (Compound aa54), Fmoc-MeSer(tBuOTHP)-OH (Compound aa55), Fmoc-Ser(2-Me-2-BuOTHP)-OH (Compound aa62), Fmoc-MeSer(2-Me-2-BuOTHP)-OH (Compound aa64), Fmoc-Hnl(7-F3-6-OH)-OH (Compound aa68), Fmoc-Ser(1-CF3-EtOH)-OH (Compound aa70), Fmoc-MeSer(1-CF3-EtOH)-OH (Compound aa71), Fmoc-Ser(1-CF3-EtOTHP)-OH (Compound aa72), Fmoc-Gln(Me)-OH (Compound aa75), Fmoc-Ser(NtBu-Aca)-OH (Compound aa78), Fmoc-MeSer(NtBu-Aca)-OH (Compound aa79), Fmoc-Ser(NMe-Aca)-OH (Compound aa81), Fmoc-Ser(nPrOTHP)-OH (Compound aa85), Fmoc-Ser(2-Me2-PrOTHP)-OH (Compound aa89), Fmoc-Ser(S-2-BuOTHP)-OH (Compound aa95), Fmoc-Ser(R-2-BuOTHP)-OH (Compound aa101), Fmoc-Phe(4-OClt-3-OMe)-OH (Compound aa104, a compound in which a side chain phenol moiety of Tyr(3-OMe) is protected with a chlorotrityl group), Fmoc-Ser(3-Me-5-Oxo-Odz)-OH (Compound aa109), Fmoc-bAla(3R-MeOEtOTHP)-OH (Compound aa111), Fmoc-bAla(2S-MeOEtOTHP)-OH (Compound aa117), Fmoc-Nva(2R-4-F2-Pyrro(N-Alloc))-OH (Compound

aa121), Fmoc-Ser(S-4-F2-Pyrro(N-Alloc)-Me)-OH (Compound aa126), Fmoc-Phe(3-OMe-4-CONMe)-OH (Compound aa127), Fmoc-Phe(4-OMe-3-CONMe)-OH (Compound aa128), Fmoc-Phe(3-OMe-4-CONHMs)-OH (Compound aa129), Fmoc-Hyp(2-EtOTHP)-OH (Compound aa133), Fmoc-(Ph(3-OMe-4-CONMe)Et)Gly-OH (Compound 134), Fmoc-Hph(3-OMe-4-CONMe)-OH (Compound aa135), Fmoc-D-Hph(3-OMe-4-CONMe)-OH (Compound aa136), Fmoc-MeHph(3-OMe-4-CONMe)-OH (Compound aa137), Fmoc-Phe(4-CONMeOTHP)-OH (Compound aa139), Fmoc-Phe(3-OMe-4-CONHOTHP)-OH (Compound aa140), Fmoc-Ser(S-(Alloc)Mor-3-Me)-OH (Compound aa141), and others were synthesized as described below.

Concerning the sequences containing an amino acid having a tertiary alcohol in the side chain (*e.g.* Ser(tBuOH), MeSer(tBuOH), Ser(2-Me-2-BuOH), and MeSer(2-Me-2-BuOH)), peptides were elongated without a problem even when an Fmoc amino acid was used in which the tertiary alcohol site of the side chain was not protected with THP (for example, Fmoc-Ser(tBuOH)-OH (Compound aa53), Fmoc-Ser(2-Me-2-BuOH)-OH (Compound aa61) and such were used).

Concerning the sequences containing an amino acid having a structure in which the side chain has a secondary alcohol and a CF3 group is attached to the carbon to which the alcohol is attached (*e.g.* Hnl(7-F3-6-OH) and Ser(1-CF3-EtOH)), peptides were elongated without a problem even when an Fmoc amino acid was used in which the secondary alcohol site of the side chain was not protected with THP (for example, Fmoc-Hnl(7-F3-6-OH)-OH (Compound aa68), Fmoc-Ser(1-CF3-EtOH)-OH (Compound aa70) and such were used).

[Table 3]

## Abbreviations of Amino Acids

| Abbreviation | Structural Formula | Abbreviation | Structural Formula |
|---|---|---|---|
| **D-Val** | 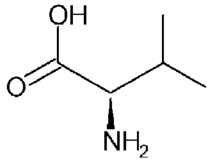 | **Thr** | 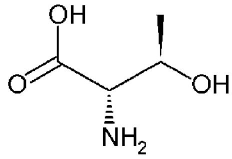 |
| **MeHph** | 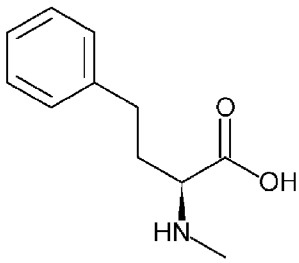 | **nPrGly** | 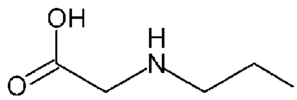 |
| **Ser (EtOH)** | 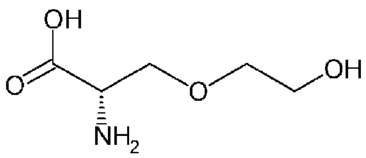 | **Ser (iPen)** | 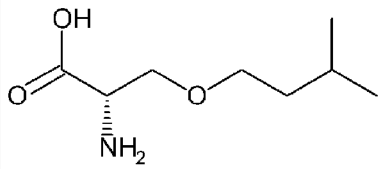 |
| **MeSer (EtOH)** | 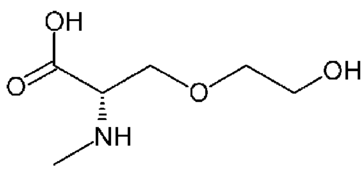 | **MePhe** | 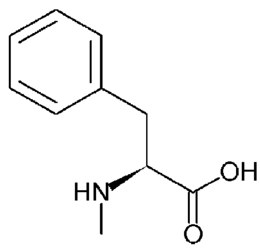 |
| **MeLeu** | 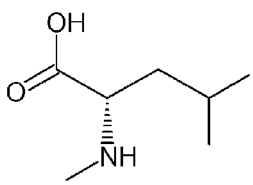 | **MeGly** | 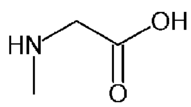 |

| Abbreviation | Structural Formula |
|---|---|
| MePhe (3-Cl) | 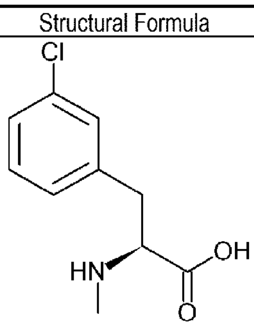 |
| gMeAbu | 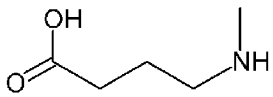 |
| Leu | 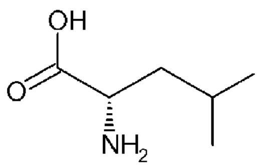 |
| Ile | 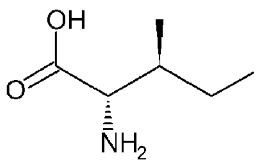 |
| MeAla | 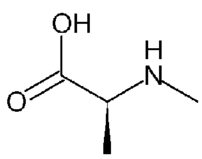 |

| Abbreviation | Structural Formula |
|---|---|
| MeVal | 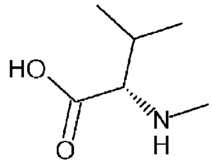 |
| D-Abu | 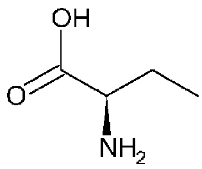 |
| Nle (6-OH) | 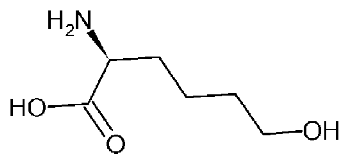 |
| Val | 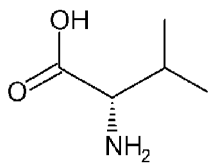 |
| MeAbu | 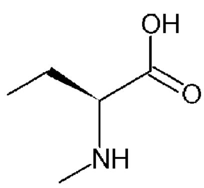 |

| Abbreviation | Structural Formula |
|---|---|
| Hnl (7-F3-6-OH) | 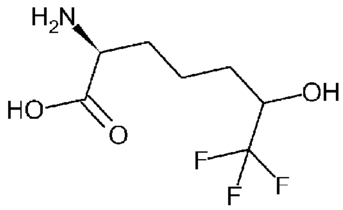 |
| MeIle | 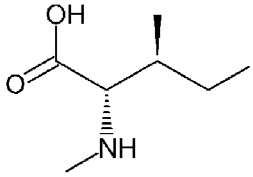 |
| D-Leu | 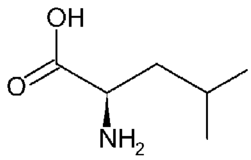 |
| nBuGly | 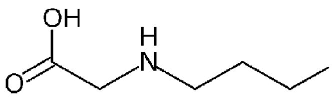 |
| Ser (1-CF3-EtOH) | 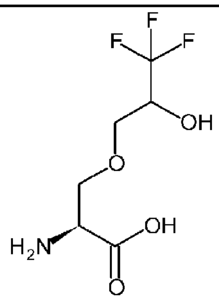 |

| Abbreviation | Structural Formula |
|---|---|
| MeSer (1-CF3-EtOH) | 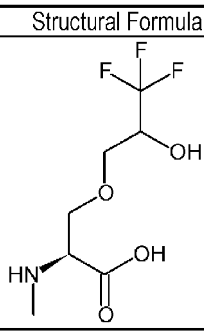 |
| Ser (tBu) | 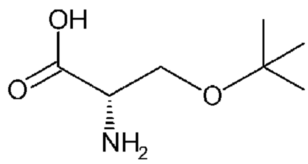 |
| Ser (R-2-PrOH) | 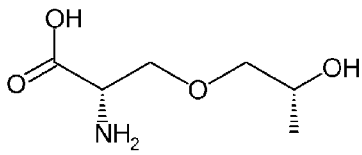 |
| MeSer (R-2-PrOH) | 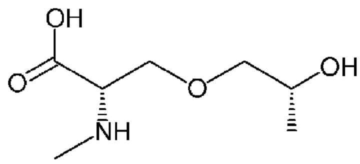 |
| Ser (S-2-PrOH) | 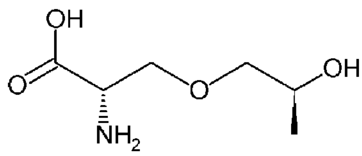 |

| Abbreviation | Structural Formula | Abbreviation | Structural Formula |
|---|---|---|---|
| MeSer (S-2-PrOH) | 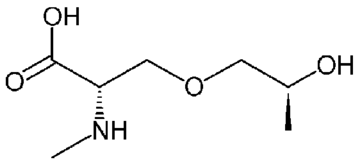 | Phe | 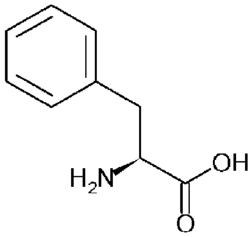 |
| Tyr (3-OMe) | 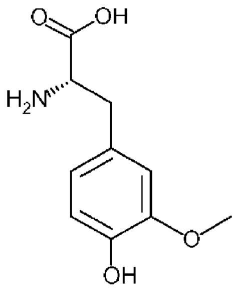 | Gln (Me) | 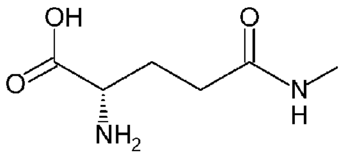 |
| D-Ala | 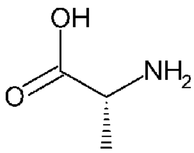 | Phe (4-CF3) | 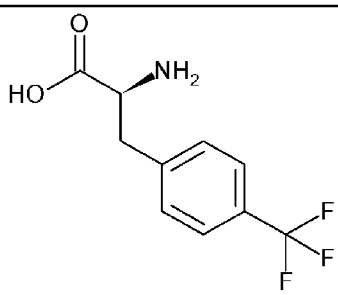 |
| Tyr | 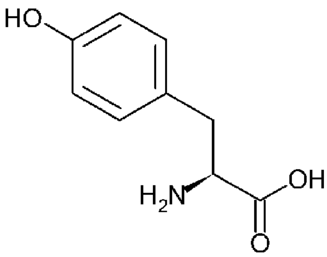 | Ser (3-Me-5-Oxo-Odz) | 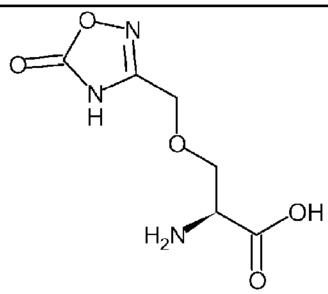 |
| Ser (NMe-Aca) | 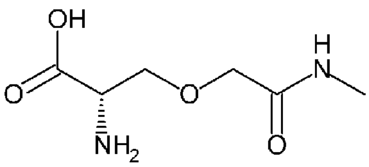 | Ser (tBuOH) | 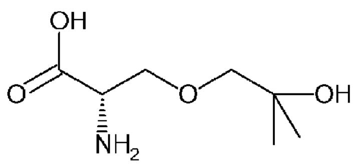 |

| Abbreviation | Structural Formula |
|---|---|
| MeSer (tBuOH) | 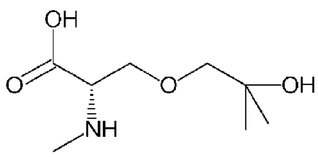 |
| Ser (2-Me-2-BuOH) | 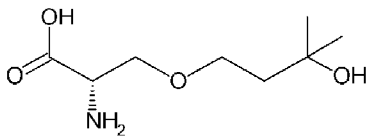 |
| MeSer (2-Me-2-BuOH) | 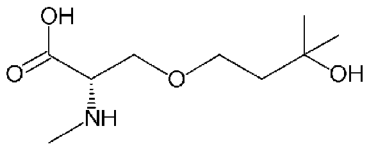 |
| Ser (NtBu-Aca) | 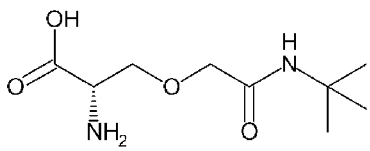 |
| MeSer (NtBu-Aca) | 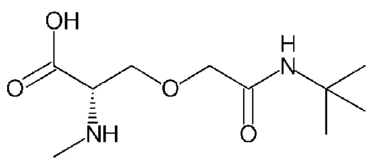 |

| Abbreviation | Structural Formula |
|---|---|
| bAla (3R-MeOEtOH ) | 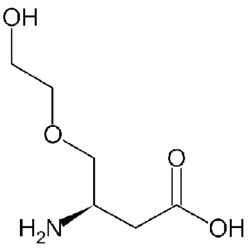 |
| Ser (nPrOH) | 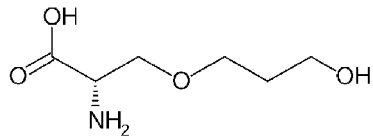 |
| Ser (S-2-BuOH) | 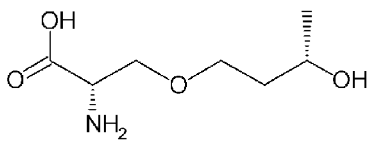 |
| Ser (R-2-BuOH) | 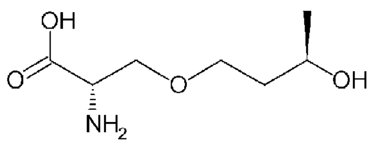 |
| Ser (2-Me2-PrOH) | 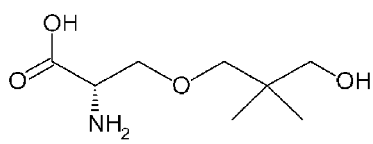 |

| Abbreviation | Structural Formula |
|---|---|
| Phe (3-OMe-4-CONMe) | 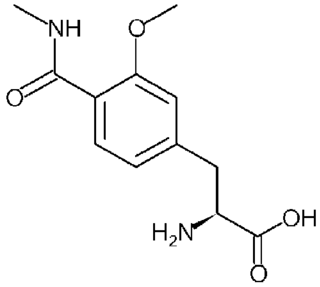 |
| Asp | 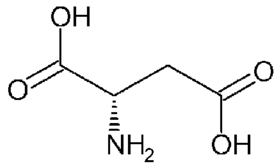 |
| MeAsp | 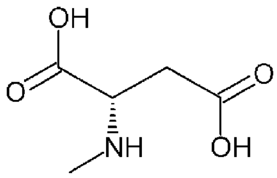 |
| Phe (4-OMe-3-CONMe) | 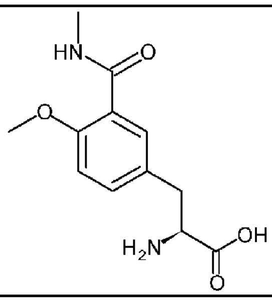 |
| Ala | 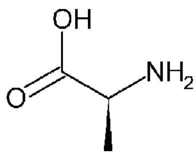 |

| Abbreviation | Structural Formula |
|---|---|
| Hph | 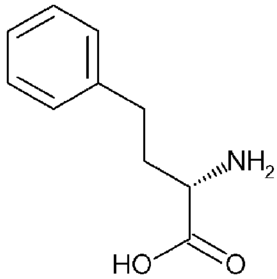 |
| Gly | 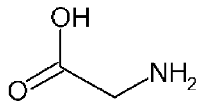 |
| b-MeAla | 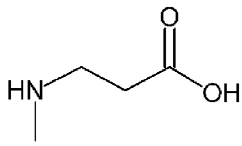 |
| Ala (3-Pyr) | 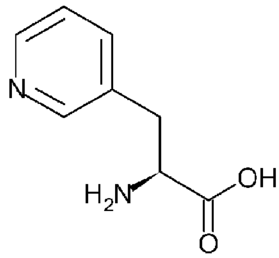 |
| D-MeAla | 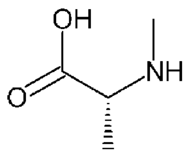 |

| Abbreviation | Structural Formula |
|---|---|
| MeAla (4-Thz) | 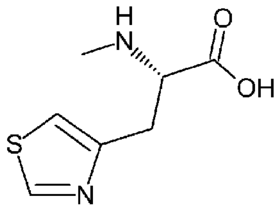 |
| Phe {#(CH2)2} | 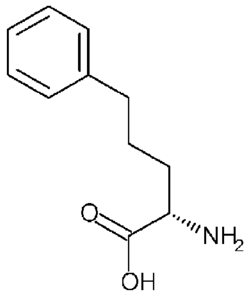 |
| Ser (3-F-5-Me-Pyr) | 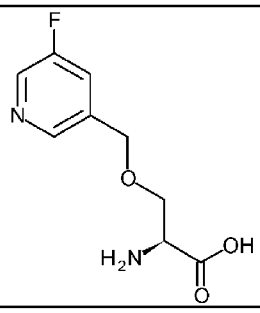 |
| Ala (4-Thz) | 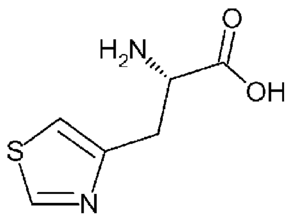 |
| D-Pro | 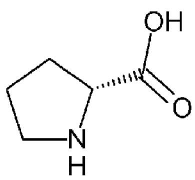 |

| Abbreviation | Structural Formula |
|---|---|
| Ser (Ph-3-Cl) | 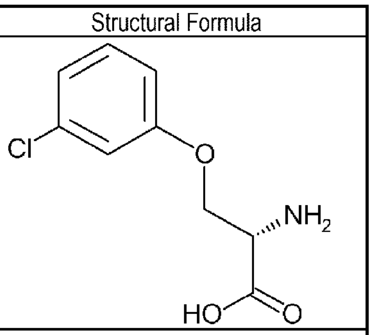 |
| Pro | 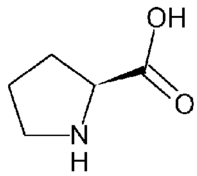 |
| Ser (Bn) | 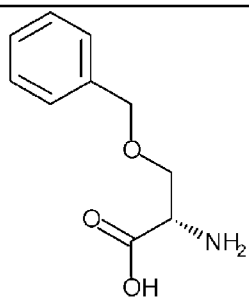 |
| bAla (2S-MeOEtOH) | 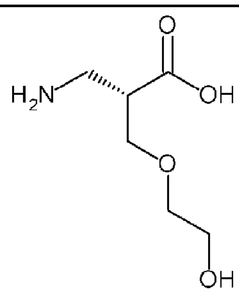 |
| Ser (S-4-F2-Pyrro-Me) | 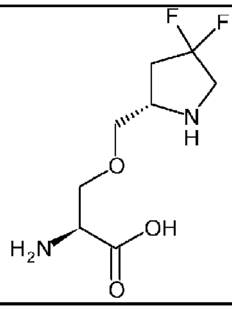 |

| Abbreviation | Structural Formula |
|---|---|
| Phe (3-OMe-4-CONHMs) | 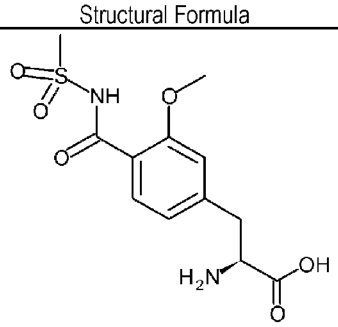 |
| Hyp (2-EtOH) | 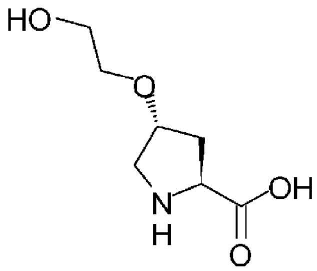 |
| (Ph(3-OMe-4-CONMe)Et)Gly | 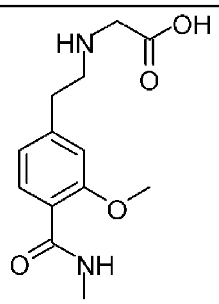 |
| Hph (3-OMe-4-CONMe) | 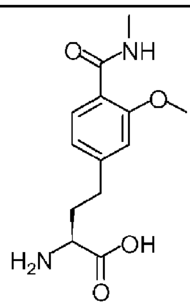 |
| D-Hph (3-OMe-4-CONMe) | 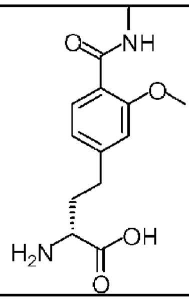 |

| Abbreviation | Structural Formula |
|---|---|
| Nva (2-R-4-F2-Pyrro) | 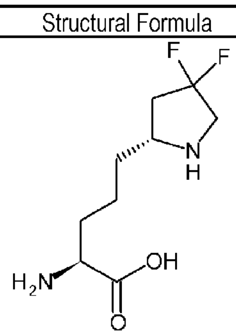 |
| MeHph (3-OMe-4-CONMe) | 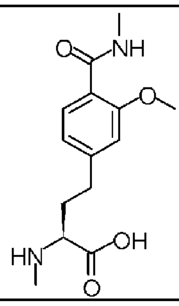 |
| Phe (4-CONMeOH) | 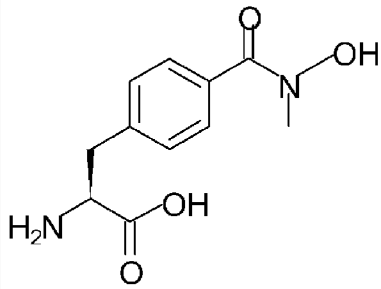 |
| Phe (3-OMe-4-CONHOH) | 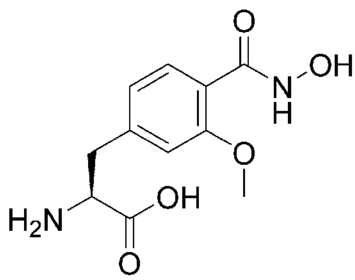 |
| Ser (S-Mor-3-Me) | 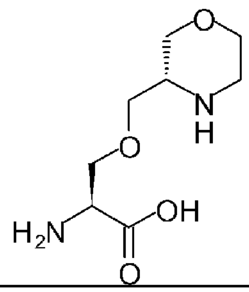 |

| Abbreviation | Structural Formula |
|---|---|
| Fmoc-D-Val-OH | 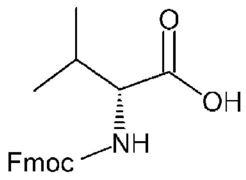 |
| Fmoc-MeHph-OH | 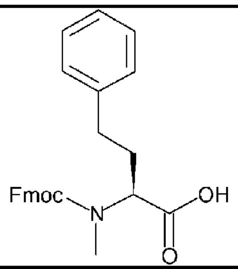 |
| Fmoc-Ser(EtOTHP)-OH | 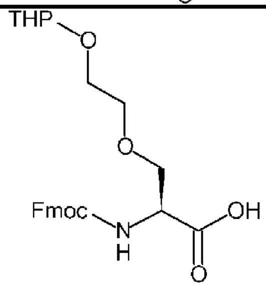 |
| Fmoc-MeSer(EtOTHP)-OH | 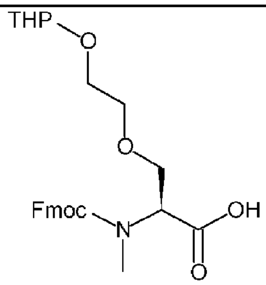 |

| Abbreviation | Structural Formula |
|---|---|
| Fmoc-MePhe-OH | 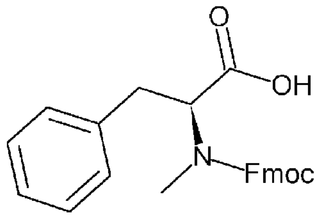 |
| Fmoc-MeGly-OH | 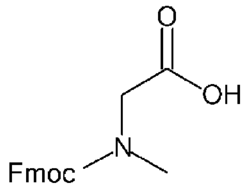 |
| Fmoc-MePhe(3-Cl)-OH | 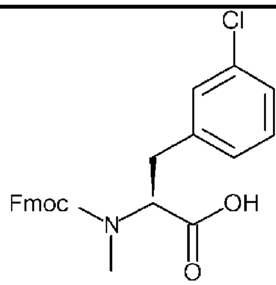 |
| Fmoc-gMeAbu-OH | 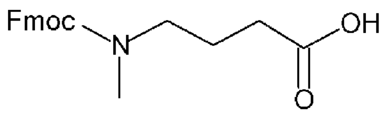 |

| Abbreviation | Structural Formula |
|---|---|
| Fmoc-MeLeu-OH | 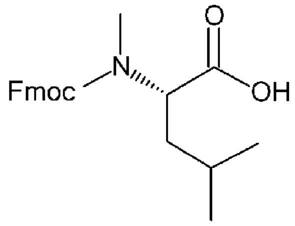 |
| Fmoc-Thr(THP)-OH | 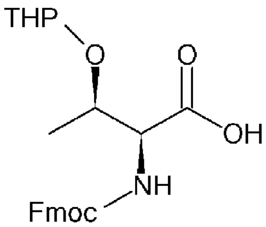 |
| Fmoc-nPrGly-OH | 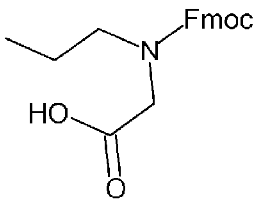 |
| Fmoc-Ser(iPen)-OH | 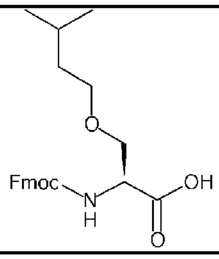 |

| Abbreviation | Structural Formula |
|---|---|
| Fmoc-Leu-OH | 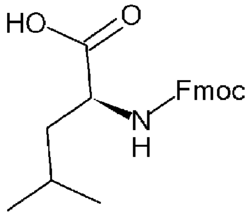 |
| Fmoc-Ile-OH | 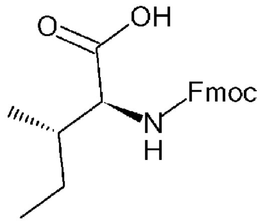 |
| Fmoc-MeAla-OH | 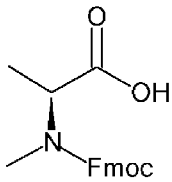 |
| Fmoc-MeVal-OH | 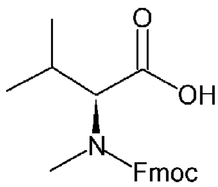 |

| Abbreviation | Structural Formula |
|---|---|
| **Fmoc-D-Abu-OH** | 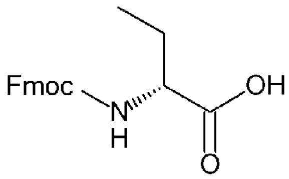 |
| **Fmoc-Nle(6-OTHP)-OH** | 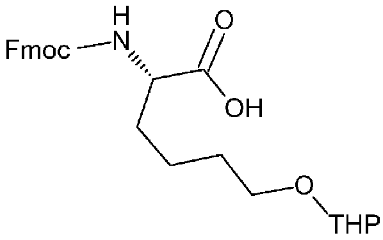 |
| **Fmoc-Val-OH** | 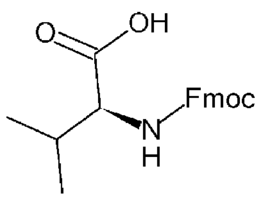 |
| **Fmoc-MeAbu-OH** | 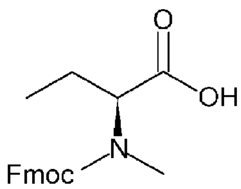 |

| Abbreviation | Structural Formula |
|---|---|
| **Fmoc-nBuGly-OH** | 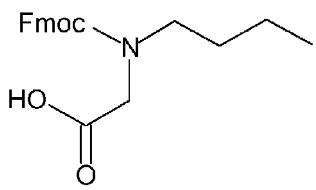 |
| **Fmoc-Ser(1-CF3-EtOH)-OH** | 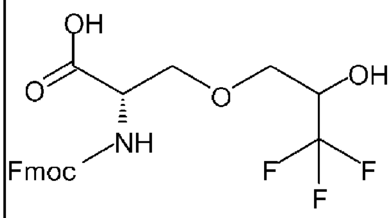 |
| **Fmoc-Ser(1-CF3-EtOTHP)-OH** | 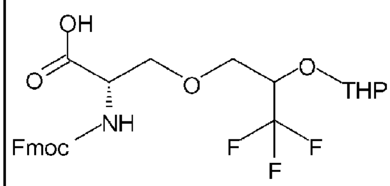 |
| **Fmoc-MeSer(1-CF3-EtOH)-OH** | 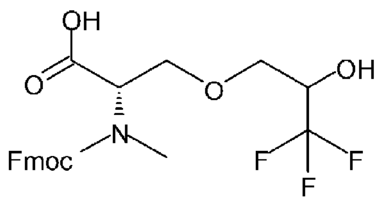 |

| Abbreviation | Structural Formula |
|---|---|
| Fmoc-Hnl(7-F3-6-OH)-OH | 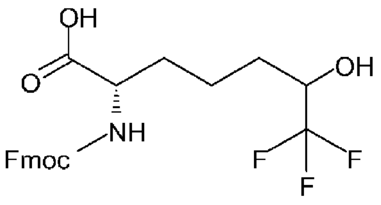 |
| Fmoc-Hnl(7-F3-6-OTHP)-OH | 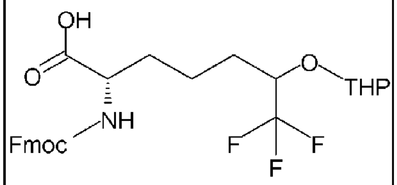 |
| Fmoc-MeIle-OH | 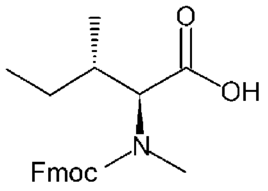 |
| Fmoc-D-Leu-OH | 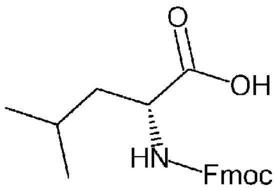 |

| Abbreviation | Structural Formula |
|---|---|
| Fmoc-MeSer(1-CF3-EtOTHP)-OH | 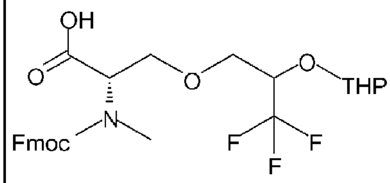 |
| Fmoc-Ser(tBu)-OH | 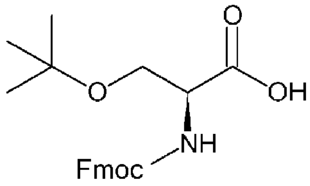 |
| Fmoc-Ser(R-2-PrOTHP)-OH | 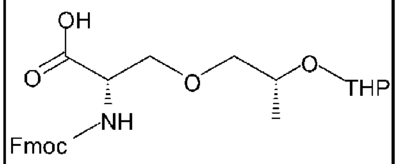 |
| Fmoc-MeSer(R-2-PrOTHP)-OH | 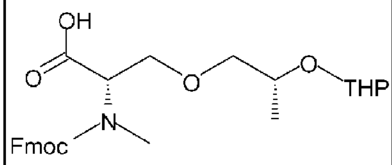 |

| Abbreviation | Structural Formula |
|---|---|
| Fmoc-Ser(S-2-PrOTHP)-OH | 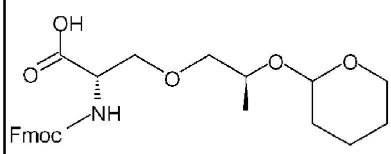 |
| Fmoc-MeSer(S-2-PrOTHP)-OH | 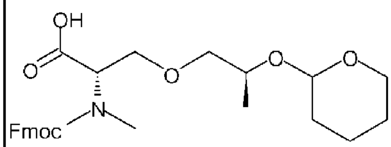 |
| Fmoc-Phe(4-OClt-3-OMe)-OH | 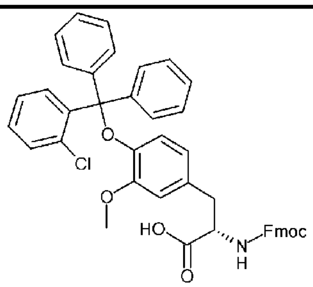 |
| Fmoc-D-Ala-OH | 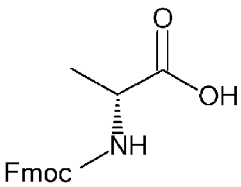 |

| Abbreviation | Structural Formula |
|---|---|
| Fmoc-Phe(4-CF3)-OH | 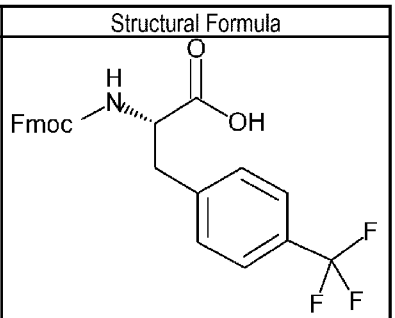 |
| Fmoc-Ser(3-Me-5-Oxo-Odz)-OH | 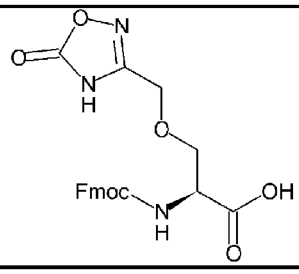 |
| Fmoc-Ser(tBuOH)-OH | 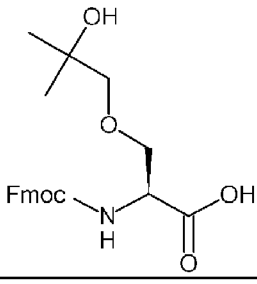 |
| Fmoc-Ser(tBuOTHP)-OH | 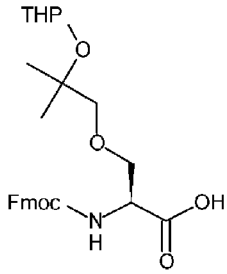 |

| Abbreviation | Structural Formula |
|---|---|
| Fmoc-Tyr(Clt)-OH | 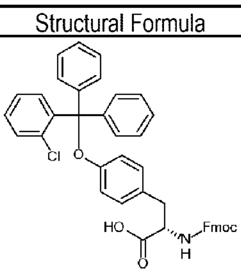 |
| Fmoc-Ser(NMe-Aca)-OH | 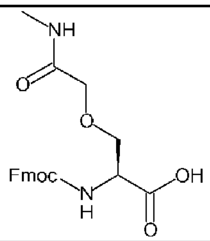 |
| Fmoc-Phe-OH | 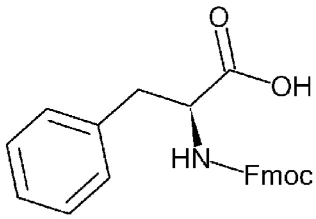 |
| Fmoc-Gln(Me)-OH | 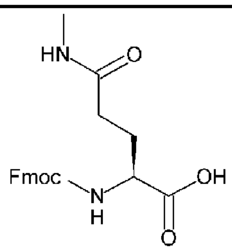 |
| Fmoc-MeSer(tBuOH)-OH | 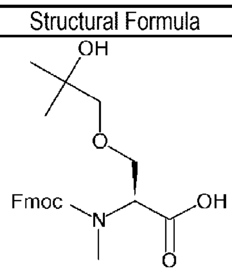 |
| Fmoc-MeSer(tBuOTHP)-OH | 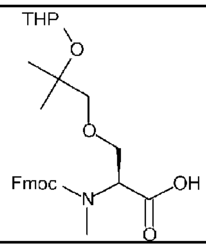 |
| Fmoc-Ser(2-Me-2-BuOH)-OH | 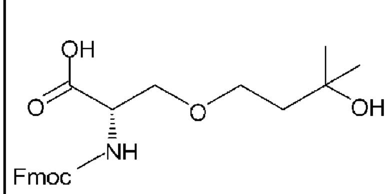 |
| Fmoc-Ser(2-Me-2-BuOTHP)-OH | 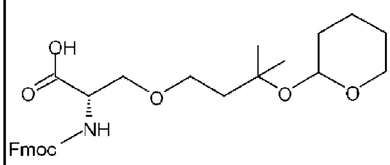 |

| Abbreviation | Structural Formula |
|---|---|
| Fmoc-MeSer(2-Me-2-BuOH)-OH | 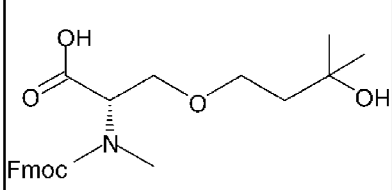 |
| Fmoc-MeSer(2-Me-2-BuOTHP)-OH | 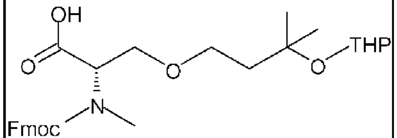 |
| Fmoc-Ser(NtBu-Aca)-OH | 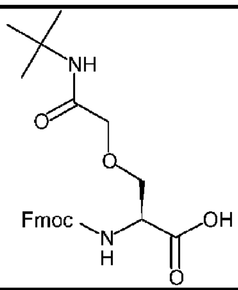 |
| Fmoc-MeSer (NtBu-Aca)-OH | 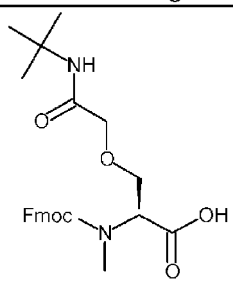 |

| Abbreviation | Structural Formula |
|---|---|
| Fmoc-Ser(2-Me2-PrOTHP)-OH | 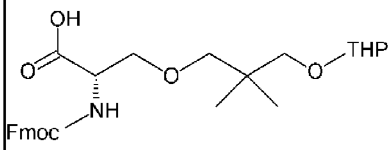 |
| Fmoc-Phe(3-OMe-4-CONMe)-OH | 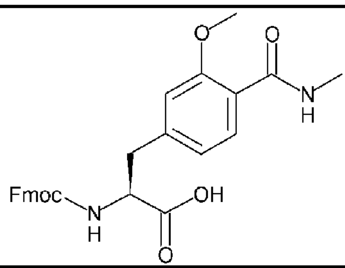 |
| Fmoc-Phe(4-OMe-3-CONMe)-OH | 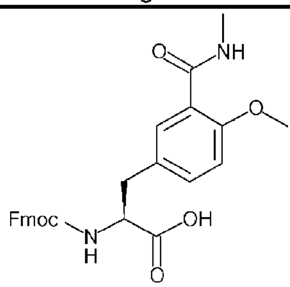 |
| Fmoc-Ala-OH | 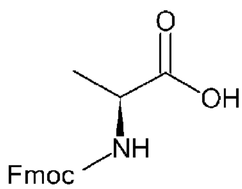 |

| Abbreviation | Structural Formula | Abbreviation | Structural Formula |
|---|---|---|---|
| Fmoc-bAla (3R-MeOEtOTHP)-OH | 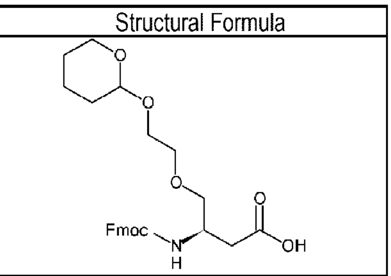 | Fmoc-Hph-OH | 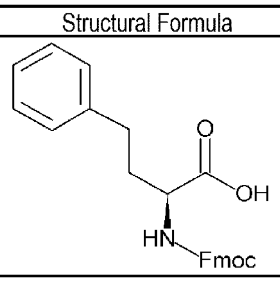 |
| Fmoc-Ser(nPrOTHP)-OH | 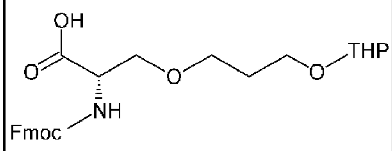 | Fmoc-Gly-OH | 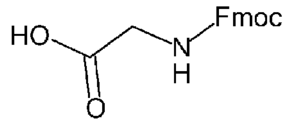 |
| Fmoc-Ser (S-2-BuOTHP)-OH | 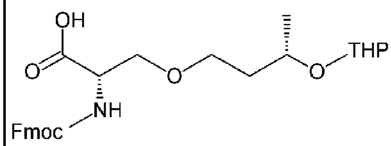 | Fmoc-b-MeAla-OH | 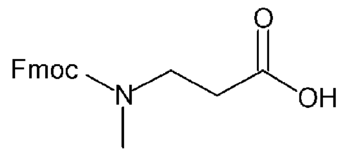 |
| Fmoc-Ser (R-2-BuOTHP)-OH | 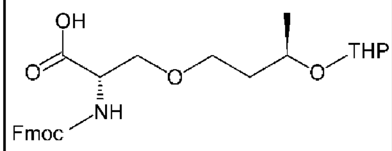 | Fmoc-Ala(3-Pyr)-OH | 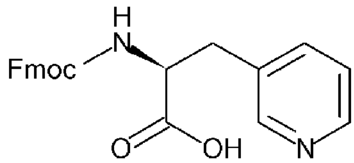 |

| Abbreviation | Structural Formula |
|---|---|
| Fmoc-D-MeAla-OH | 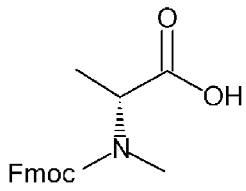 |
| Fmoc-MeAla(4-Thz)-OH | 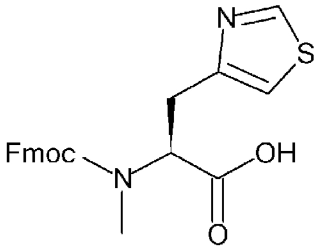 |
| Fmoc-Phe{#(CH2)2}-OH | 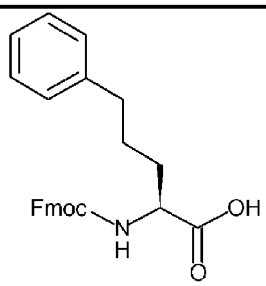 |
| Fmoc-Ser(3-F-5-Me-Pyr)-OH | 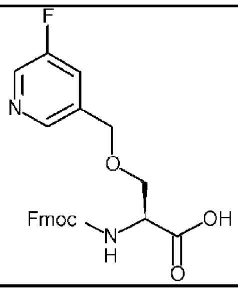 |

| Abbreviation | Structural Formula |
|---|---|
| Fmoc-Ser(Bn)-OH | 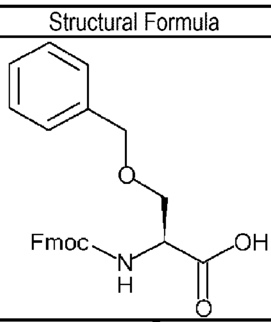 |
| Fmoc-bAla(2S-MeOEtOTHP)-OH | 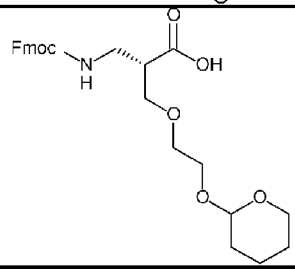 |
| Fmoc-Ser(S-4-F2-Pyrro(N-Alloc)-Me)-OH | 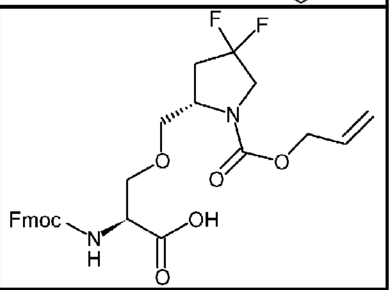 |
| Fmoc-Nva(2-R-4-F2-Pyrro(N-Alloc))-OH | 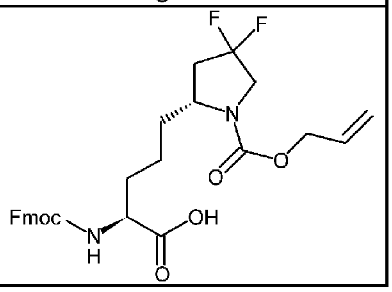 |

| Abbreviation | Structural Formula |
|---|---|
| Fmoc-Ala(4-Thz)-OH | 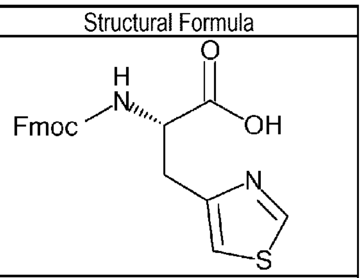 |
| Fmoc-D-Pro-OH | 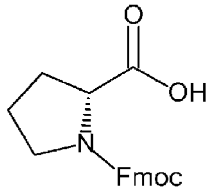 |
| Fmoc-Ser(Ph-3-Cl)-OH | 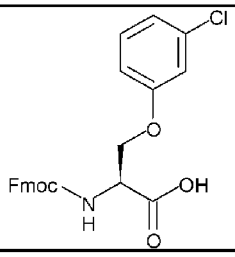 |
| Fmoc-Pro-OH | 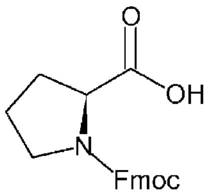 |

| Abbreviation | Structural Formula |
|---|---|
| Fmoc-Phe(3-OMe-4-CONHMs)-OH | 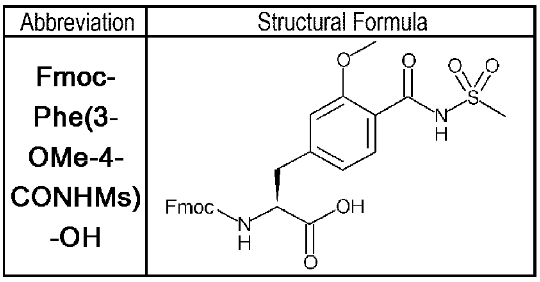 |

| Abbreviation | Structural Formula | Abbreviation | Structural Formula |
|---|---|---|---|
| Fmoc-Hyp(2-EtOTHP)-OH | 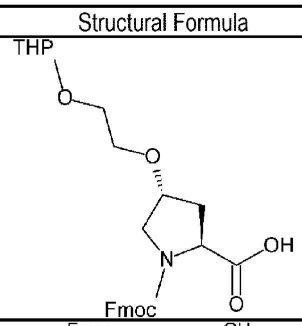 | Fmoc-MeHph(3-OMe-4-CONMe)-OH | 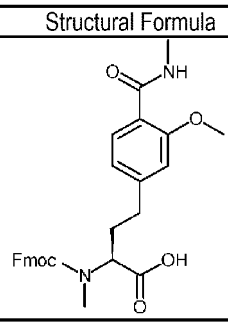 |
| Fmoc-(Ph(3-OMe-4-CONMe)Et)Gly-OH | 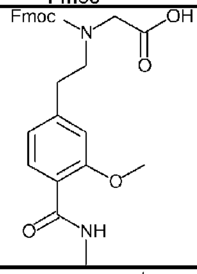 | Fmoc-Phe(4-CONMeOTHP)-OH | 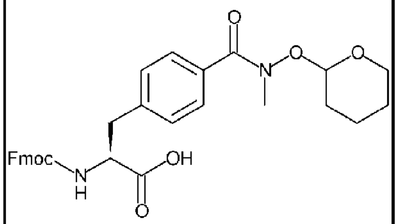 |
| Fmoc-Hph(3-OMe-4-CONMe)-OH | 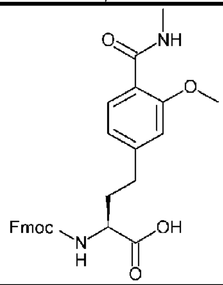 | Fmoc-Phe(3-OMe-4-CONHOTHP)-OH | 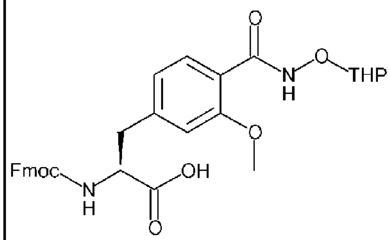 |
| Fmoc-D-Hph(3-OMe-4-CONMe)-OH | 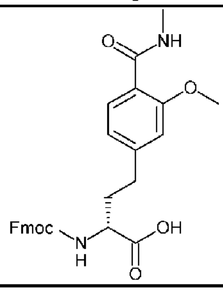 | Fmoc-Ser(S-(Alloc)Mor-3-Me)-OH | 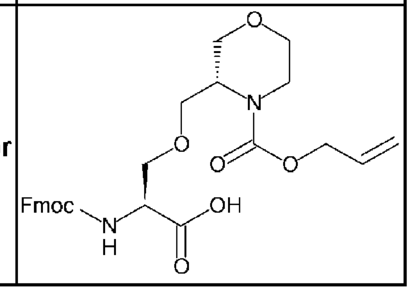 |

1-2. Synthesis of amino acids used for peptide synthesis by a peptide synthesizer

Synthesis of (2S,3R)-2-((((9H-fluoren-9-yl)methoxy)carbonyl)amino)-3-((tetrahydro-2H-pyran-2-yl)oxy)butanoic acid (Compound aa01, Fmoc-Thr(THP)-OID

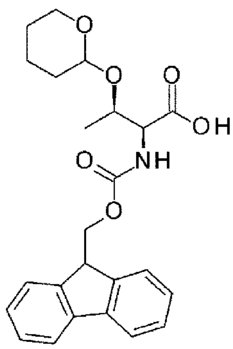

To a mixture of (2S,3R)-2-((((9H-fluoren-9-yl)methoxy)carbonyl)amino)-3-hydroxybutanoic acid monohydrate (Fmoc-Thr-OH monohydrate, purchased from Tokyo Chemical Industry, 5.0 g, 13.9 mmol) and pyridinium p-toluenesulfonate (PPTS) (0.175 g, 0.70 mmol) was added toluene (50 mL), and the toluene was evaporated under reduced pressure to azeotropically remove water. To the resulting residue were added ultradehydrated tetrahydrofuran (THF) (28 mL) and 3,4-dihydro-2H-pyran (8.8 mL, 97 mmol), and the mixture was stirred at 50°C for 4 h under a nitrogen atmosphere. After the disappearance of the raw materials was confirmed by LCMS (SQDFA05), the mixture was cooled to 25°C and ethyl acetate (30 mL) was added. The organic layer was then washed by adding a saturated aqueous sodium chloride solution (30 mL), and the aqueous layer was extracted with ethyl acetate (30 mL). All the resulting organic layers were combined and further washed with a saturated aqueous sodium chloride solution (30 mL) twice. The organic layers were dried over sodium sulfate and the solvent was evaporated under reduced pressure to afford a crude product (9.3 g). 4.65 g of the resulting crude product was dissolved in tetrahydrofuran (THF) (30 mL), followed by addition of 1.0 M phosphate buffer adjusted to pH 8.0 (30 mL). This mixture was stirred at 50°C for 4 h. After cooling to 25°C, ethyl acetate (30 mL) was added and the organic layer and the aqueous layer were separated. The aqueous layer was extracted by adding ethyl acetate (30 mL), and all the resulting organic layers were then combined and washed with a saturated aqueous sodium chloride solution (30 mL) twice. The organic layers were dried over sodium sulfate, the solvent was evaporated under reduced pressure, and the residue was further dried under reduced pressure using a pump at 25°C for 30 min.

The resulting residue was dissolved in diethyl ether (50 mL) and heptane (50 mL) was then added. Only the diethyl ether was evaporated under controlled reduced pressure (~100 hPa) and the resulting mixture was filtered to afford a solid. This washing operation with heptane was repeated twice. The resulting solid was dried under reduced pressure using a pump at 25°C for 2 h to afford Fmoc-Thr(THP)-OH sodium salt (2.80 g, 6.26 mmol).

Ethyl acetate (50 mL) and 0.05 M aqueous phosphoric acid solution, pH 2.1 (140 mL) were added to the total amount of the resulting Fmoc-Thr(THP)-OH sodium salt. After stirring at 25°C for 5 min, the organic layer and the aqueous layer were separated. The aqueous layer was extracted by adding ethyl acetate (50 mL), and all the resulting organic layers were then combined and washed with a saturated aqueous sodium chloride solution (50 mL) twice. The organic layers were dried over sodium sulfate and the solvent was evaporated under reduced pressure. The residue was dried under reduced pressure using a pump at 25°C for 2 h, after which the resulting solid was dissolved in t-butyl methyl ether (TBME) (50 mL) and the solvent was evaporated under reduced pressure. The residue was further dried under reduced pressure using a pump at 25°C for 1 h to afford (2S,3R)-2-((((9H-fluoren-9-yl)methoxy)carbonyl)amino)-3-((tetrahydro-2H-pyran-2-yl)oxy)butanoic acid (Compound aa01, Fmoc-Thr(THP)-OH, 2.70 g, 30 mol% of t-butyl methyl ether (TBME) remaining) as a THP-protected diastereomer derived from asymmetric carbon. The obtained Fmoc-Thr(THP)-OH was stored in a freezer at -25°C. LCMS (ESI) m/z = 424.2 (M-H)-

Retention time: 0.84 min, 0.85 min (analytical condition SQDFA05)

Synthesis of N-(((9H-fluoren-9-yl)methoxy)carbonyl)-N-butylglycine (Compound aa02, Fmoc-nBuGly-OH)

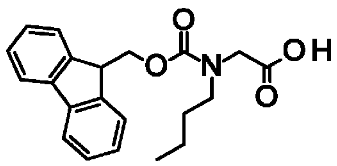

To a solution of sodium hydride (26 g, 1.08 mol) in tetrahydrofuran (THF) (500 mL) was added tert-butyl (tert-butoxycarbonyl)glycinate (Boc-Gly-OtBu) (100 g, 432.36 mmol) in small portions at room temperature. After stirring for 30 min, a solution of 1-iodobutane (239 g, 1.30 mol) in dimethylformamide (DMF) (50 mL) was added dropwise. The reaction solution was stirred at room temperature for 16 h, followed by addition of water. After removing the tetrahydrofuran (THF) under reduced pressure, the reaction solution was extracted with ethyl acetate three times. The resulting organic solvent was dried over anhydrous sodium sulfate, and the solvent was evaporated under reduced pressure. The resulting residue was purified by flash column chromatography (petroleum ether/ethyl acetate) to afford tert-butyl N-(tert-butoxycarbonyl)-N-butylglycinate (Boc-nBuGly-OtBu) (130 g) quantitatively.

To a solution of tert-butyl N-(tert-butoxycarbonyl)-N-butylglycinate (Boc-nBuGly-OtBu) obtained by the above method (260 g, 904.68 mmol) in 1,4-dioxane (1000 mL) was added concentrated hydrochloric acid (1000 mL) dropwise at 0°C. The reaction solution was warmed to room temperature and then stirred at room temperature for 16 h. The reaction solution was concentrated to afford butylglycine (H-nBuGly-OH) hydrochloride (200 g) as a crude product.

A mixture of the crude product butylglycine (H-nBuGly-OH) (60 g), potassium carbonate (188.9 g, 1.37 mol),

and water/1,4-dioxane (1:1) (3000 mL) was stirred at room temperature for 30 min, and N-(9-fluorenylmethoxycarbonyloxy)succinimide (Fmoc-OSu) (183.2 g, 543.09 mmol) was then added in small portions at room temperature. The reaction solution was stirred at room temperature for 16 h and then washed with ether three times. A 5 M aqueous hydrochloric acid solution was added to the resulting aqueous layer until pH = 3, and the aqueous layer was extracted with ethyl acetate three times. The organic solvent was dried over anhydrous sodium sulfate and the solvent was evaporated under reduced pressure. The resulting crude product was washed with ether to afford N-(((9H-fluoren-9-yl)methoxy)carbonyl)-N-butylglycine (Compound aa02, Fmoc-nBuGly-OH) (121 g).

LCMS (ESI) m/z = 354 (M+H)+

Retention time: 0.87 min (analytical condition SQDFA05)

Fmoc-Ser(iPen)-OH (Compound aa04) was synthesized according to the following scheme.

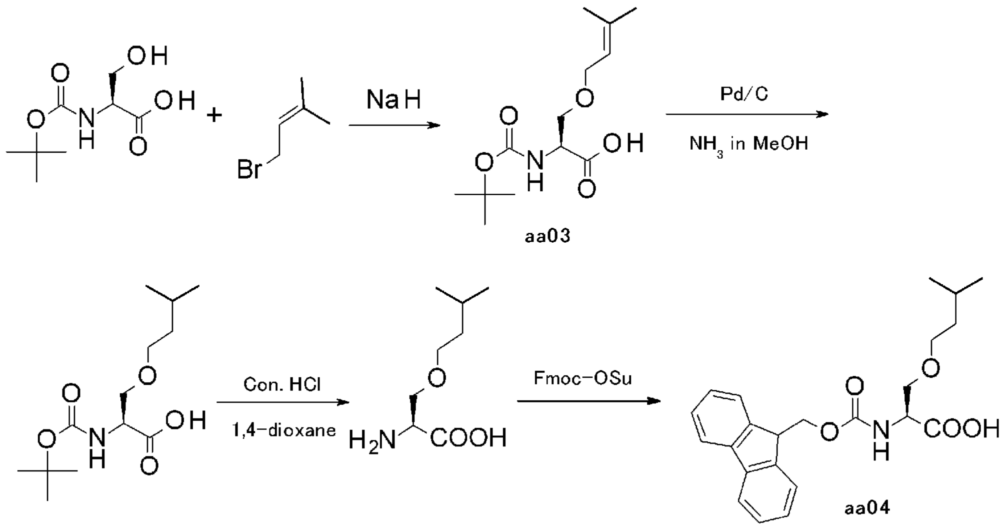


Synthesis of N-(tert-butoxycarbonyl)-O-(3-methylbut-2-en-1-yl)-L-serine (Compound aa03)

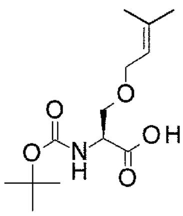

(tert-Butoxycarbonyl)-L-serine (Boc-Ser-OH) (50 g, 234.65 mmol) was dissolved in dimethylformamide (DMF) (250 mL), and sodium hydride (22 g, 916.67 mmol, 60% oil dispersion) was added under ice-cooling (0°C). After stirring under ice-cooling for 30 min, 1-bromo-3-methylbut-2-ene (44 g, 295.24 mmol) was added dropwise. After the addition, the reaction solution was stirred at 25°C for 16 h, the reaction was then quenched by adding ice water, and the pH was adjusted to 2-3 by adding an aqueous hydrochloric acid solution (5 M). The reaction solution was then extracted with ethyl acetate twice, and the organic layers were washed with brine and dried over anhydrous sodium sulfate. After filtration, the ethyl acetate was removed by concentration under reduced pressure to afford a crude product containing the target compound. This crude product was purified by normal phase column chromatography (petroleum ether/ethyl acetate = 100/0 -> 80/20) to afford N-(tert-butoxycarbonyl)-O-(3-methylbut-2-en-1-yl)-L-serine (Compound aa03) (32 g, 48%).
LCMS (ESI) m/z = 296 (M+Na)+

Retention time: 1.39 min (analytical condition SMD method 7)

Synthesis of N-(((9H-fluoren-9-yl)methoxy)carbonyl)-O-isopentyl-L-serine (Compound aa04, Fmoc-Ser(iPen)-OH)

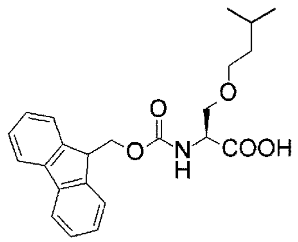

N-(tert-Butoxycarbonyl)-O-(3-methylbut-2-en-1-yl)-L-serine (Compound aa03) (185 g, 676.85 mmol), a solution of ammonia in methanol (2 M, 555 mL), Pd/C (18.5 g), and methanol (1500 mL) were mixed and stirred under a hydrogen atmosphere (5 atm) at room temperature for 16 h. The solid was removed by filtration and the solvent was then removed by concentration under reduced pressure to afford N-(tert-butoxycarbonyl)-O-isopentyl-L-serine (Boc-Ser(iPen)-OH) (183 g).

The obtained N-(tert-butoxycarbonyl)-O-isopentyl-L-serine (Boc-Ser(iPen)-OH) (175 g) was dissolved in 1,4-dioxane (500 mL), concentrated hydrochloric acid (300 mL) was added, and the mixture was then stirred at room temperature for 16 h. The reaction solution was concentrated under reduced pressure, the resulting crude product (122 g) was dissolved in 1,4-dioxane/water (1000 mL/1000 mL), potassium carbonate (239 g, 1.73 mol) and N-(9-fluorenylmethoxycarbonyloxy)succinimide (Fmoc-OSu) (234 g, 694.36 mmol) were added, and the mixture was stirred at room temperature for 16 h. The reaction solution was then washed with hexane three times. After the washing, the aqueous layer was adjusted to pH 2-3 by adding an aqueous hydrochloric acid solution (6 M) and extracted with ethyl acetate twice. The organic layers were dried over anhydrous sodium sulfate and filtered, after which the solvent was removed by concentration under reduced pressure. The resulting crude product was washed with ether, hexane, and ethyl acetate and further purified by reverse phase column chromatography (water/acetonitrile = 100/0 -> 30/70) to afford N-(((9H-fluoren-9-yl)methoxy)carbonyl)-O-isopentyl-L-serine (Compound aa04, Fmoc-Ser(iPen)-OH) (95 g, 41%) as a white solid.

LCMS (ESI) m/z = 398 (M+H)+

Retention time: 2.29 min (analytical condition SMD method 18)

Synthesis of (S)-2-((((9H-fluoren-9-yl)methoxy)carbonyl)(methyl)amino)-4-phenylbutanoic acid (Compound aa05, Fmoc-MeHph-OH)

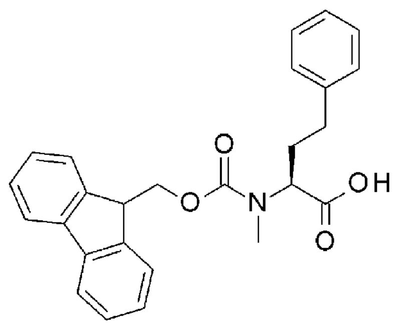

To a solution of commercially available (S)-2-((((9H-fluoren-9-yl)methoxy)carbonyl)amino)-4-phenylbutanoic acid (100 g, 244.11 mmol) in toluene (1.5 L) were added tosyl acid (TsOH) (2.575 g, 14.95 mmol) and paraformaldehyde (15.96 g, 488.77 mmol) at room temperature under a nitrogen atmosphere, and the mixture was stirred at 110°C for 16 h. After adding water to the organic layer and washing it with water three times, the resulting organic layers and the organic layer obtained by extracting the resulting aqueous layer with ethyl acetate were combined, dried over anhydrous

sodium sulfate, filtered, and concentrated under reduced pressure to afford 100 g of (9H-fluoren-9-yl)methyl (S)-5-oxo-4-phenethyloxazolidine-3-carboxylate as a crude product.

To a solution of (9H-fluoren-9-yl) (S)-5-oxo-4-phenethyloxazolidine-3-carboxylate obtained as described above (50 g, 118.51 mmol) in dichloromethane (700 mL) were added triethylsilane ($Et_3SiH$) and trifluoroacetic acid (TFA) (700 mL), and the mixture was stirred at 25°C for 16 h. The reaction solution was concentrated under reduced pressure, an aqueous potassium carbonate solution was added to the resulting residue, and the mixture was washed with petroleum ether. The resulting aqueous layer was adjusted to pH = 3 with concentrated hydrochloric acid and extracted with ethyl acetate. The organic layer was dried over anhydrous sodium sulfate, filtered, and concentrated under reduced pressure. To the resulting residue were added methanol and hexane, and the mixture was again concentrated under reduced pressure to afford (S)-2-((((9H-fluoren-9-yl)methoxy)carbonyl)(methyl)amino)-4-phenylbutanoic acid (Compound aa05, Fmoc-MeHph-OH) (29.5 g, 58%, over two steps).
LCMS (ESI) m/z = 416 $(M+H)^+$

Retention time: 0.95 min (analytical condition SQDFA05)

Synthesis of N-(((9H-fluoren-9-yl)methoxy)carbonyl)-O-((5-fluoropyridin-3-yl)methyl)-L-serine (Compound aa12, Fmoc-Ser(3-F-5-Me-Pyr)-OH)

N-(((9H-Fluoren-9-yl)methoxy)carbonyl)-O-((5-fluoropyridin-3-yl)methyl)-L-serine (Compound aa12, Fmoc-Ser(3-F-5-Me-Pyr)-OH) was synthesized by the following route.

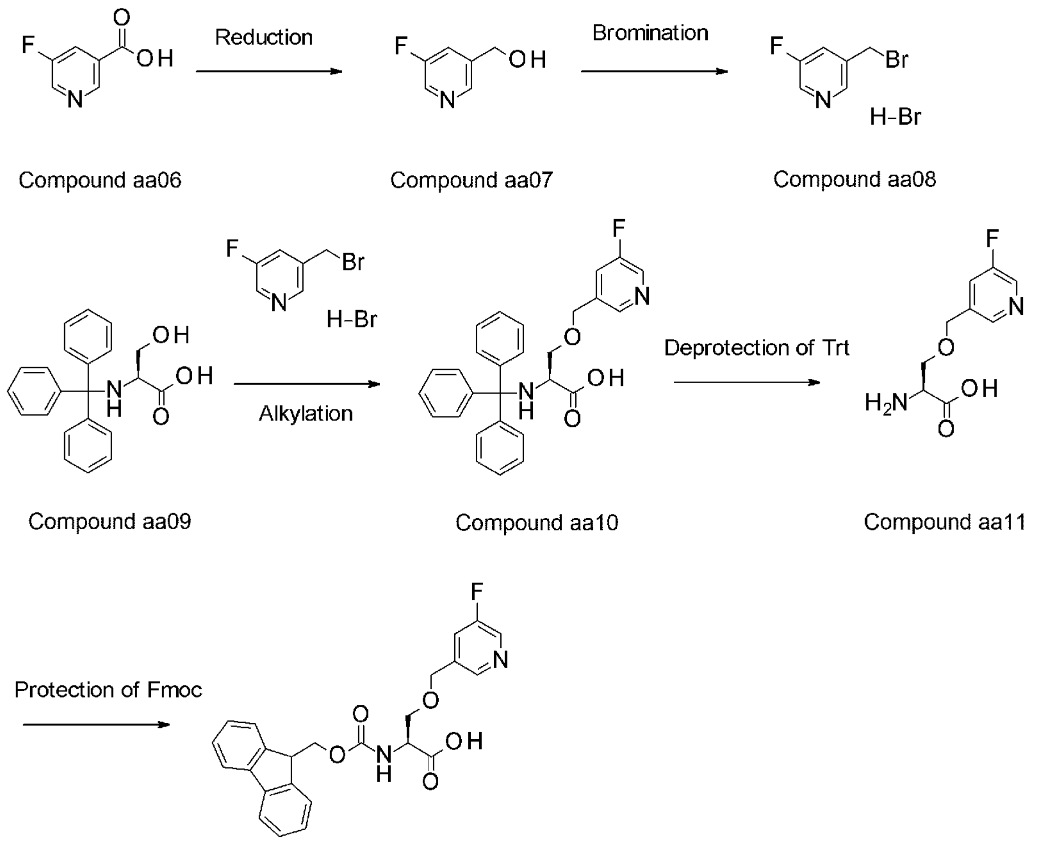


Compound aa06 Compound aa07 Compound aa08

Compound aa09 Compound aa10 Compound aa11

Compound aa12

Synthesis of (5-fluoropyridin-3-yl)methanol (Compound aa07)

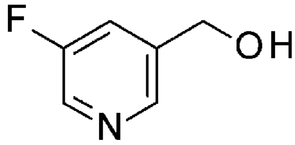

THF (200 mL) was added to commercially available 5-fluoronicotinic acid (Compound aa06) (14.1 g, 100 mmol) under a nitrogen atmosphere, and the mixture was stirred using a mechanical stirrer. Triethylamine (19.51 mL, 140 mmol) was then added at room temperature, and the mixture was stirred until the solid was completely dissolved, after which the reaction solution was cooled using an ice bath. Ethyl chloroformate (11.5 mL, 120 mmol) was then added dropwise and the mixture was stirred in an ice bath for 30 min. The reaction solution was then cooled to -60°C or lower using a dry ice bath, and a solution of $LiAlH_4$ (lithium aluminum hydride) in THF (2.5 M, 40 mL, 100 mmol) was added dropwise over 5 min or more so that the temperature of the reaction solution did not exceed -20°C. After stirring the reaction solution at - 78°C for 3 h, ethyl acetate (69 mL) was added so that the temperature of the reaction solution did not exceed -20°C. The reaction solution was then further stirred using an ice bath for 1 h, water (18 mL) was added, and the mixture was stirred at room temperature for 15 min. The reaction solution was filtered using an NH silica gel and concentrated under reduced pressure to afford (5-fluoropyridin-3-yl)methanol (Compound aa07) (9.28 g, 73%) as a yellow oily component.
LCMS (ESI) m/z = 128 $(M+H)^+$

Retention time: 0.28 min (analytical condition SQDFA05)

Synthesis of 3-(bromomethyl)-5-fluoropyridine hydrobromide (Compound aa08)

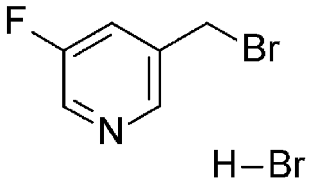

A condenser cooled with acetone/dry ice was connected to a round-bottom flask, and (5-fluoropyridin-3-yl)methanol (Compound aa07) (8.21 g, 64.6 mmol) and a 25% HBr-acetic acid solution (96 mL, 388 mmol) were added under a nitrogen atmosphere. The reaction was carried out in an open system, and a 1 M aqueous sodium hydroxide solution was used to trap the generated HBr. The reaction solution was gradually warmed from room temperature to 100°C with stirring and was further stirred for 3 h. The reaction solution was then cooled to room temperature, slow addition of diisopropyl ether (48 mL) was repeated three times, and the resulting solid was filtered to afford 3-(bromomethyl)-5-fluoropyridine hydrobromide (Compound aa08) (12.43 g, 71%) as a gray solid.
$^1H$ NMR (Varian 400-MR, 400 MHz, d-DMSO) δ 4.77 (2H, s), 7.85-7.88 (1H, m), 8.55-8.56 (2H, m)

Synthesis of O-((5-fluoropyridin-3-yl)methyl)-N-trityl-L-serine (Compound aa10, Trt-Ser(3-F-5-Me-Pyr)-OH

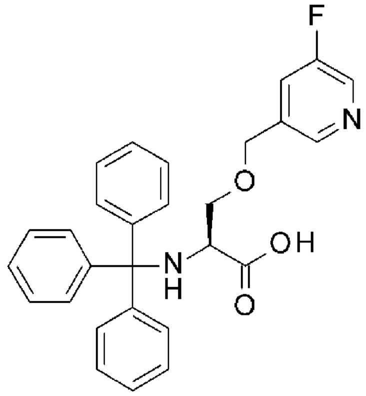

THF (45.8 mL) was added to sodium t-pentoxide (NaOtPen) (13.7 g, 125 mmol) under a nitrogen atmosphere. After stirring, commercially available tritylserine triethylamine salt (Trt-Ser-OH triethylamine salt) (Compound aa09) (11.24 g, 25 mmol) was added in three portions and the mixture was stirred at room temperature for 30 min. The reaction solution was stirred in an ice bath for 15 min and cooled, after which a solution of 3-(bromomethyl)-5-fluoropyridine hydrobromide (Compound aa08) (8.13 g, 30 mmol) in DMF (16 mL) was added dropwise and DMF (14 mL) was further added. After stirring the reaction solution in an ice bath for 45 min, a solution of 3-(bromomethyl)-5-fluoropyridine hydrobromide (Compound aa08) (2.03 g, 7.5 mmol) in DMF (4.0 mL) was added dropwise and DMF (4.0 mL) was further added. The reaction solution was then further stirred at room temperature for 1 h, and water (125 mL) was added. The resulting mixture was washed with t-butyl methyl ether (TBME), and the organic layer was extracted with water. The resulting aqueous layers were combined, adjusted to pH = 7 with a saturated aqueous sodium dihydrogenphosphate solution (15 mL), and then extracted with ethyl acetate twice. The resulting organic layers were combined, washed with 2-fold diluted brine three times, and then washed with brine twice. The resulting organic layers were dried over anhydrous sodium sulfate, filtered, and concentrated under reduced pressure to afford O-((5-fluoropyridin-3-yl)methyl)-N-trityl-L-serine (Compound aa10, Trt-Ser(3-F-5-Me-Pyr)-OH) (11.07 g, 97%) as a yellow amorphous.
LCMS (ESI) m/z = 455 $(M-H)^-$

Retention time: 0.86 min (analytical condition SQDFA05)

Synthesis of O-((5-fluoropyridin-3-yl)methyl)-L-serine (Compound aa11, H-Ser(3-F-5-Me-Pyr)-OH)

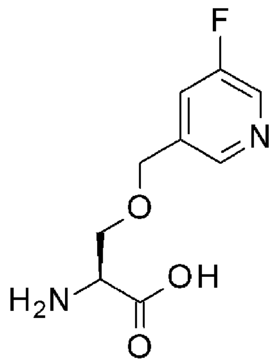

To a solution of O-((5-fluoropyridin-3-yl)methyl)-N-trityl-L-serine (Compound aa10, Trt-Ser(3-F-5-Me-Pyr)-OH) (10.76 g, 23.57 mmol) in 1,4-dioxane (21.5 mL) was added a 5-10% hydrochloric acid/methanol solution (64.2 mL) at room temperature. The reaction solution was stirred at room temperature for 10 min to 20 min, followed by addition of 1,4-dioxane (135 mL). After further adding additional 1,4-dioxane (90 mL), seed crystals previously prepared in a small amount as described below (5 mg) were added and the mixture was further stirred at room temperature for 30 min. The resulting solid was filtered, washed with diisopropyl ether (50 mL) four times, and dried under reduced pressure to afford O-((5-fluoropyridin-3-yl)methyl)-L-serine (Compound aa11, H-Ser(3-F-5-Me-Pyr)-OH) (6.58 g, 95%) as a hydrochloride.
LCMS (ESI) m/z = 213 $(M-H)^-$

Retention time: 0.24 min (analytical condition SQDAA05)

Preparation of seed crystals of O-((5-fluoropyridin-3-yl)methyl)-L-serine (Compound aa11, H-Ser(3-F-5-Me-Pyr)-OH

To a solution of O-((5-fluoropyridin-3-yl)methyl)-N-trityl-L-serine (Compound aa10, Trt-Ser(3-F-5-Me-Pyr)-OH) (98 mg, 2.69 mmol) in 1,4-dioxane (819 μL) was added a 5-10% hydrochloric acid/methanol solution (2.45 mL) at room temperature, and the mixture was stirred for 5 h. To the reaction solution was added 1,4-dioxane (6.0 mL), and the resulting solid was filtered, washed with diisopropyl ether, and dried under reduced pressure to afford O-((5-fluoropyridin-3-yl)methyl)-L-serine (Compound aa11, H-Ser(3-F-5-Me-Pyr)-OH) (222.5 mg, 86%) as a hydrochloride.
LCMS (ESI) m/z = 213 $(M-H)^-$

Retention time: 0.24 min (analytical condition SQDAA05)

Synthesis of N-(((9H-fluoren-9-yl)methoxy)carbonyl)-O-((5-fluoropyridin-3-yl)methyl)-L-serine (Compound aa12, Fmoc-Ser(3-F-5-Me-Pyr)-OH)

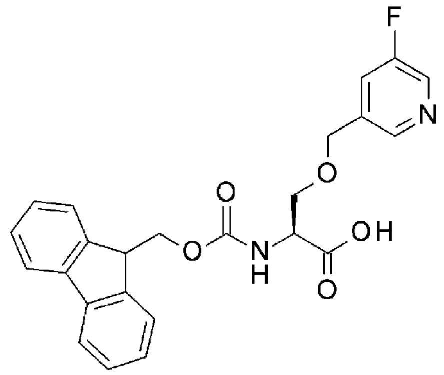

To O-((5-fluoropyridin-3-yl)methyl)-L-serine (Compound aa11, H-Ser(3-F-5-Me-Pyr)-OH) hydrochloride (6.37 g, 22.19 mmol) were added water (42 mL), 1,4-dioxane (115 mL), and diisopropylethylamine (DIPEA) (13.53 mL, 78 mmol) at room temperature, followed by stirring. To the reaction solution was then added 9-fluorenylmethyl N-succinimidyl carbonate (Fmoc-OSu) (7.86 g, 23.3 mmol) at room temperature, followed by stirring at room temperature. After the disappearance of the raw materials was confirmed by LC-MS, water (56.2 mL) was added to the reaction solution at room temperature and the mixture was washed with a 25% t-butyl methyl ether (MTBE)/hexane solution twice. The resulting aqueous layer was adjusted to pH = 6.1 with a saturated aqueous sodium dihydrogenphosphate solution ($NaH_2PO_4$). The aqueous layer was then extracted with ethyl acetate twice, and the resulting organic layers were combined and washed with 2-fold diluted brine and with brine. The resulting organic layers were dried over anhydrous sodium sulfate, filtered, and concentrated under reduced pressure to afford N-(((9H-fluoren-9-yl)methoxy)carbonyl)-O-((5-fluoropyridin-3-yl)methyl)-L-serine (Compound aa12, Fmoc-Ser(3-F-5-Me-Pyr)-OH) (9.47 g, 98%) as a yellow solid.
LCMS (ESI) m/z = 437 (M+H)+

Retention time: 0.86 min (analytical condition SQDAA05)

Synthesis of (S)-2-((((9H-fluoren-9-yl)methoxy)carbonyl)amino)-3-(3-chlorophenoxy)propanoic acid (Compound aa16, Fmoc-Ser(Ph-3-Cl)-OH)

(S)-2-((((9H-Fluoren-9-yl)methoxy)carbonyl)amino)-3-(3-chlorophenoxy)propanoic acid (Compound aa16, Fmoc-Ser(Ph-3-Cl)-OH) was synthesized by the following route.

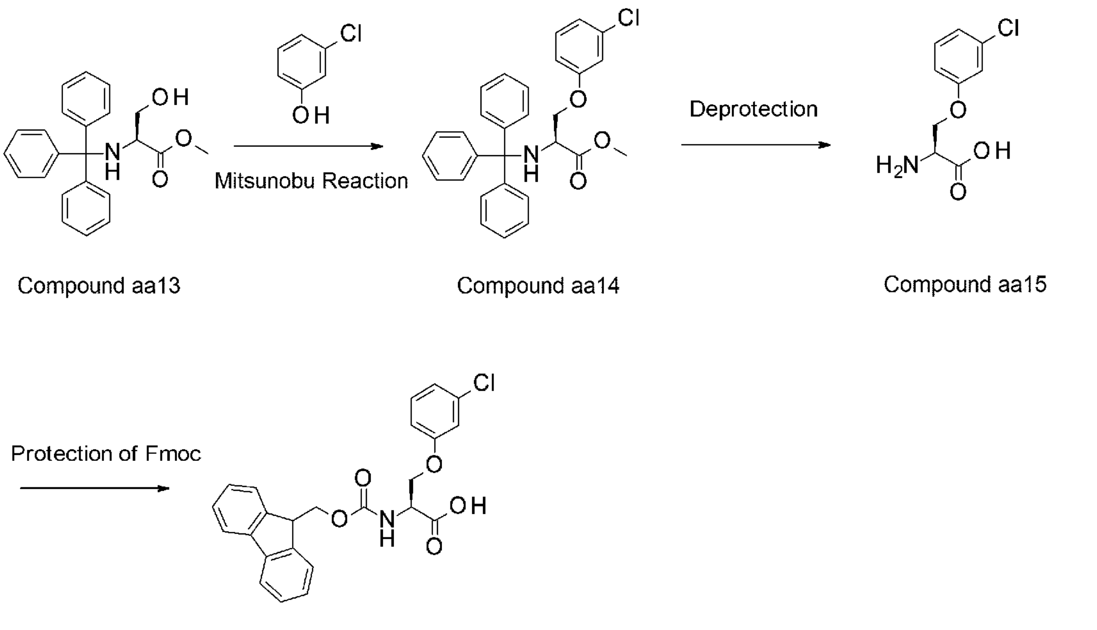


Compound aa13 Compound aa14 Compound aa15

Compound aa16

Synthesis of (S)-methyl 3-(3-chlorophenoxy)-2-(tritylamino)propanoate (Trt-Ser(Ph-3-Cl)-OMe) (Compound aa14)

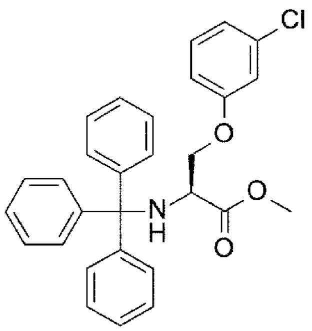

To a solution of commercially available (S)-methyl 3-hydroxy-2-(tritylamino)propanoate (Compound aa13, Trt-Ser-OMe) (6.0 g, 16.6 mmol) and triphenylphosphine ($PPh_3$) (8.71 g, 33.2 mmol) in toluene (35 mL) was added a 2.2 M solution of diethyl azocarboxylate (DEAD) in toluene (15.06 mL, 33.2 mmol) at room temperature under a nitrogen atmosphere, and the reaction solution was stirred at 50°C for 30 min. The reaction solution was concentrated under reduced pressure and the resulting residue was then purified by reverse phase silica gel column chromatography (10 mM aqueous ammonium acetate/methanol) to afford (S)-methyl 3-(3-chlorophenoxy)-2-(tritylamino)propanoate (Trt-Ser(Ph-3-Cl)-OMe)
(Compound aa14) (4.43 g, 57%).

$^1H$ NMR (Varian400-MR, 400 MHz, d-DMSO) δ 7.42-7.44 (6H, m), 7.28-7.30 (7H, m), 7.20-7.21 (3H, m), 6.99-7.01 (2H, m), 6.83-6.85 (1H, m), 4.19-4.22 (1H, m), 4.06-4.08 (1H, m), 3.49-3.51 (1H, m), 3.17 (3H, s)

Synthesis of (S)-2-amino-3-(3-chlorophenoxy)propanoic acid (Compound aa15, H-Ser(Ph-3-Cl)-OH)

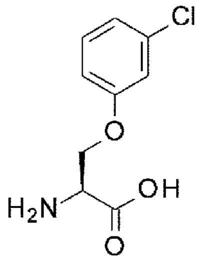

To a solution of (S)-methyl 3-(3-chlorophenoxy)-2-(tritylamino)propanoate (Trt-Ser(Ph-3-Cl)-OMe) (Compound aa14) (3.9 g, 8.26 mmol) in 1,4-dioxane (80 mL) was added a 1.0 M lithium hydroxide/methanol solution (83 mL) at room temperature, and the reaction solution was stirred at 50°C for 3 h. After concentrating the reaction solution under reduced pressure, trifluoroacetic acid (TFA) (30 mL) was added to the resulting residue and the mixture was stirred at 50°C for 10 min. The reaction solution was then concentrated under reduced pressure and the resulting residue was purified by reverse phase silica gel column chromatography (0.1% aqueous formic acid solution/0.1% formic acid-acetonitrile) to afford (S)-2-amino-3-(3-chlorophenoxy)propanoic acid (Compound aa15, H-Ser(Ph-3-Cl)-OH) (850 mg, 48%, over two steps).
LCMS (ESI) m/z = 216 $(M+H)^+$

Retention time: 0.37 min (analytical condition SQDFA05)

Synthesis of (S)-2-((((9H-fluoren-9-yl)methon)carbonyl)amino)-3-(3-chlorophenoxy)-propanoic acid (Compound aa16, Fmoc-Ser(Ph-3-Cl)-OH)

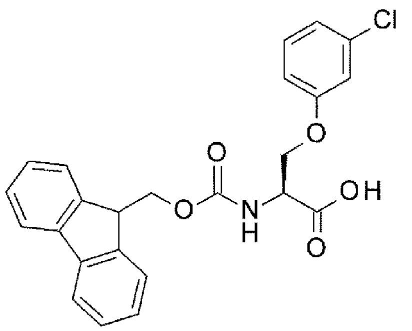

To (S)-2-amino-3-(3-chlorophenoxy)propanoic acid (Compound aa15, H-Ser(Ph-3-Cl)-OH) (850 mg, 3.94 mmol) and 9-fluorenylmethyl N-succinimidyl carbonate (Fmoc-OSu) (1.33 g, 3.94 mmol) were added 1,4-dioxane (20 mL) and water (20 mL) at room temperature, cesium carbonate (2.569 g, 7.88 mmol) was added, and the mixture was stirred at room temperature. After the disappearance of the raw materials was confirmed by LC-MS, water (20 mL) was added and the mixture was washed with diethyl ether (40 mL) twice. The resulting aqueous layer was adjusted to pH = 2 with a 5N aqueous hydrochloric acid solution and extracted with ethyl acetate twice. The organic layers were dried over anhydrous sodium sulfate, filtered, and concentrated under reduced pressure. The resulting residue was purified by reverse phase silica gel column chromatography (0.1% aqueous formic acid solution/0.1 % formic acid-acetonitrile) to afford (S)-2-((((9H-fluoren-9-yl)methoxy)carbonyl)amino)-3 -(3 -chlorophenoxy)propanoic acid (Compound aa16, Fmoc-Ser(Ph-3-Cl)-OH) (1.42 g, 82%).
LCMS (ESI) m/z = 438 $(M+H)^+$

Retention time: 0.91 min (analytical condition SQDFA05)

Synthesis of (2S)-2-((((9H-fluoren-9-yl)methoxy)carbonyl)amino)-6-((tetrahydro-2H-pyran-2-yl)oxy)hexanoic acid (Compound aa17, Fmoc-Nle(6-OTHP)-OH)

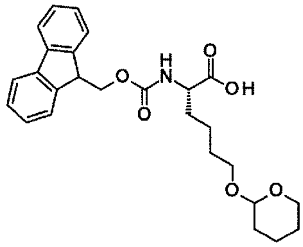

A solution of commercially purchased (S)-2-((((9H-fluoren-9-yl)methoxy)carbonyl)amino)-6-hydroxyhexanoic acid (Fmoc-Nle(6-OH)-OH) (3 g, 8.12 mmol) and pyridinium p-toluenesulfonate (PPTS) (408 mg, 1.62 mmol) in toluene (10 mL) was concentrated under reduced pressure and azeotropically dehydrated. The resulting residue was dissolved in tetrahydrofuran (15 mL), dihydropyran (5.41 mL, 56.8 mmol) was added, and the mixture was stirred at 50°C for 1 h. Ethyl acetate was added to the reaction solution at room temperature, and the organic layer was washed with brine twice, after which the resulting organic layer was dried over anhydrous sodium sulfate, filtered, and concentrated under reduced pressure. The resulting residue was dissolved in tetrahydrofuran (30 mL), phosphate buffer adjusted to pH 8 (30 mL) was added, and the mixture was stirred at 50°C for 3 h. The reaction solution was extracted with ethyl acetate twice at room temperature, and the resulting organic layers were washed with brine, dried over anhydrous sodium sulfate, filtered, and concentrated under reduced pressure. The resulting residue was dissolved in diethyl ether (50 mL), heptane (50 mL) was added thereto to allow the crude product to precipitate, the diethyl ether was removed by concentration under reduced pressure, and the remaining supernatant solution was removed by decantation. This operation was repeated twice to afford a crude product. The resulting crude product was dissolved in tert-butyl methyl ether (150 mL), a 0.05 M aqueous phosphoric acid solution (150 mL) was added, and the mixture was stirred at room temperature for 1 h. The organic layer was recovered and the aqueous layer was extracted with ethyl acetate. The resulting organic layers were combined and then washed with brine twice. The resulting organic layers were dried over anhydrous sodium sulfate, filtered, and concentrated under reduced pressure to afford (2S)-2-((((9H-fluoren-9-yl)methoxy)carbonyl)amino)-6-((tetrahydro-2H-pyran-2-yl)oxy)hexanoic acid (Compound aa17, Fmoc-Nle(6-OTHP)-OH) (2.925 g, 79%).

LCMS (ESI) m/z = 454 (M+H)+

Retention time: 0.86 min (analytical condition SQDFA05)

Synthesis of methyl N-((benzyloxy)carbonyl)-0-(2-(benzyloxy)ethyl)-L-serinate (Compound aa18, Cbz-Ser(EtO-Bn)-OMe)

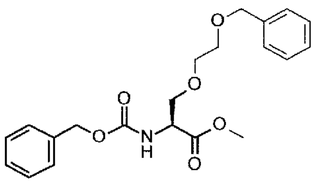

1-Benzyl 2-methyl (S)-aziridine-1,2-dicarboxylate (Cbz-Azy-OMe) (25.0 g, 106 mmol) and 2-(benzyloxy)ethan-1-ol (23.8 g, 156 mmol) were dissolved in dichloromethane (100 mL) under a nitrogen atmosphere. After cooling to 0°C, boron trifluoride-diethyl ether complex (2.00 mL, 15.9 mmol) was added and the mixture was stirred at 0°C for 1 h. Water was added to the reaction solution, the mixture was extracted with dichloromethane twice, and the organic layers were then washed with an aqueous sodium carbonate solution and with brine. The organic layers were dried over anhydrous sodium sulfate, the solvent was evaporated under reduced pressure, and the residue was further dried under reduced pressure using a vacuum pump. The resulting residue was purified by reverse phase column chromatography (water/acetonitrile) to afford methyl N-((benzyloxy)carbonyl)-O-(2-(benzyloxy)ethyl)-L-serinate (Compound aa18, Cbz-Ser(EtOBn)-OMe) (30.0 g, 73%).
LCMS (ESI) m/z = 388 (M+H)+

Retention time: 1.13 min (analytical condition SMD method 9)

Synthesis of N-((benzylon)carbonyl)-O-(2-(benzyloxy)ethyl)-L-serine (Compound aa19, Cbz-Ser(EtOBn)-OH)

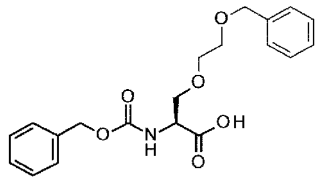

Lithium hydroxide monohydrate (13.9 g, 331 mmol) and calcium chloride (129 g, 1.24 mol) were dissolved in water (321 mL) under a nitrogen atmosphere. A solution of methyl N-((benzyloxy)carbonyl)-O-(2-(benzyloxy)ethyl)-L-serinate (Compound aa18, Cbz-Ser(EtOBn)-OMe) (30.0 g, 77.4 mmol) in 2-propanol/tetrahydrofuran (1.28 L/321 mL) was added thereto at room temperature, and the mixture was stirred for 3 h. A 2 M aqueous hydrochloric acid solution was added until pH 2, the organic layer was removed, and the aqueous layer was extracted with ethyl acetate three times. The resulting organic layers were combined and dried over anhydrous sodium sulfate, the solvent was evaporated under reduced pressure, and the residue was further dried using a vacuum pump under reduced pressure to afford N-((benzyloxy)carbonyl)-O-(2-(benzyloxy)ethyl)-L-serine (Compound aa19, Cbz-Ser(EtOBn)-OH) (30.0 g) as a crude product. This was used in the next step without purification.

Synthesis of O-(2-hydroxyethyl)-L-serine (Compound aa20, H-Ser(EtOH)-OH)

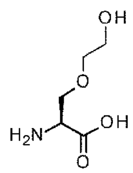

N-((Benzyloxy)carbonyl)-O-(2-(benzyloxy)ethyl)-L-serine (Compound aa19, Cbz-Ser(EtOBn)-OH) (34.0 g, 91.1 mmol) and palladium on carbon (6.80 g, 20% w/w) were dissolved in methanol (500 mL) under a hydrogen atmosphere, and the reaction solution was stirred at room temperature for 16 h. The reaction solution was filtered, the solvent was then evaporated under reduced pressure, and the residue was further dried using a vacuum pump under reduced pressure to afford O-(2-hydroxyethyl)-L-serine (Compound aa20, H-Ser(EtOH)-OH) (10.7 g) as a crude product. This was used in the next step without purification.

Synthesis of N-(((9H-fluoren-9-yl)methoxy)carbonyl)-O-(2-hydroxyethyl)-L-serine (Compound aa21, Fmoc-Ser(EtOH)-OH)

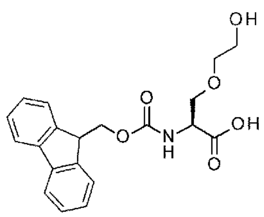

O-(2-Hydroxyethyl)-L-serine (Compound aa20, H-Ser(EtOH)-OH) (5.00 g, 33.5 mmol), N-(9-fluorenylmethoxycarbonyloxy)succinimide (Fmoc-OSu) (12.4 g, 36.9 mmol), and sodium carbonate (10.6 g, 100 mmol) were dissolved in water/1,4-dioxane (136 mL/56.0 mL) under a nitrogen atmosphere, and the reaction solution was stirred at room temperature for 3 h. After washing the reaction solution with t-butyl methyl ether three times, a 2 M aqueous hydrochloric

acid solution was added to the aqueous layer until pH 2, and the aqueous layer was extracted with ethyl acetate three times. The resulting organic layers were combined, washed with brine, and then dried over anhydrous sodium sulfate, the solvent was evaporated under reduced pressure, and the residue was further dried using a vacuum pump under reduced pressure. The resulting residue was purified by reverse phase column chromatography (0.1% aqueous formic acid solution/0.1 % formic acid-acetonitrile solution) to afford N-(((9H-fluoren-9-yl)methoxy)carbonyl)-O-(2-hydroxyethyl)-L-serine (Compound aa21, Fmoc-Ser(EtOH)-OH) (12.0 g, 96%).
LCMS (ESI) m/z = 372 (M+H)+

Retention time: 1.31 min (analytical condition SMD method 33)

Synthesis of N-(((9H-fluoren-9-yl)methoxy)carbonyl)-O-(2-((tetrahydro-2H-pyran-2-yl)oxy)ethyl)-L-serine (Compound aa22, Fmoc-Ser(EtOTHP)-OH)

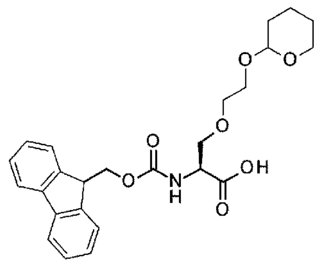

To a solution of N-(((9H-fluoren-9-yl)methoxy)carbonyl)-O-(2-hydroxyethyl)-L-serine (Compound aa21, Fmoc-Ser(EtOH)-OH) (1.6 g, 4.31 mmol) and 3,4-dihydro-2H-pyran (2.728 mL, 30.3 mmol) in tetrahydrofuran (8.616 mL) was added pyridinium p-toluenesulfonate (PPTS) (54.1 mg, 0.215 mmol) under a nitrogen atmosphere, and the mixture was stirred at 50°C for 2 h. The mixture was cooled, and ethyl acetate was then added. The organic layer was then washed with brine three times and dried over anhydrous sodium sulfate, and the solvent was evaporated under reduced pressure. The resulting residue was dissolved in tetrahydrofuran (10 mL), followed by addition of 0.05 M phosphate buffer (100 mL) (prepared by mixing a 1 M aqueous sodium dihydrogenphosphate ($NaH_2PO_4$) solution (94.3 mL) with a 1 M aqueous disodium hydrogenphosphate ($Na_2HPO_4$) solution (5.7 mL)). This mixture was stirred at room temperature for 10 min and then extracted with t-butyl methyl ether, the organic layer was washed with brine and dried over anhydrous sodium sulfate, and the solvent was evaporated under reduced pressure. The resulting residue was purified by reverse phase column chromatography (water/acetonitrile) to afford N-(((9H-fluoren-9-yl)methoxy)carbonyl)-O-(2-((tetrahydro-2H-pyran-2-yl)oxy)ethyl)-L-serine (Compound aa22, Fmoc-Ser(EtOTHP)-OH) (1.75 g, 96%).
LCMS (ESI) m/z = 456 (M+H)+

Retention time: 0.81 min (analytical condition SQDFA05)

Synthesis of benzyl (S)-4-((2-(benzyloxy)ethoxy)methyl)-5-oxooxazolidine-3-carboxylate (Compound aa23)

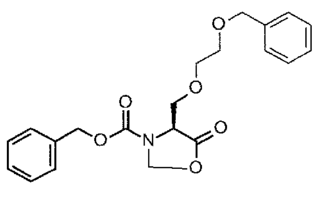

N-((Benzyloxy)carbonyl)-O-(2-(benzyloxy)ethyl)-L-serine (Compound aa19, Cbz-Ser(EtOBn)-OH) (30.0 g, 80.3 mmol), paraformaldehyde (7.20 g), and p-toluenesulfonic acid (0.83 g, 4.82 mmol) were dissolved in toluene (300 mL), and the reaction solution was stirred at 110°C for 16 h. After cooling to room temperature, the reaction solution was washed with an aqueous sodium bicarbonate solution, the organic layers were combined and dried over anhydrous sodium sulfate, the solvent was evaporated under reduced pressure, and the residue was further dried using a vacuum pump under reduced pressure to afford benzyl (S)-4-((2-(benzyloxy)ethoxy)methyl)-5-oxooxazolidine-3-carboxylate

(Compound aa23) (27.0 g) as a crude product. This was used in the next step without purification.

Synthesis of N-((benzyloxy)carbonyl)-O-(2-(benzyloxy)ethyl)-N-methyl-L-serine (Compound aa24, Cbz-MeSer(EtO-Bn)-OH)

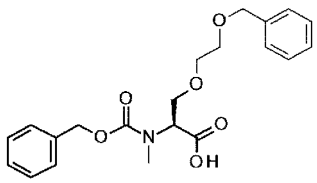

To a solution of benzyl (S)-4-((2-(benzyloxy)ethoxy)methyl)-5-oxooxazolidine-3-carboxylate (Compound aa23) (27.0 g, 70.1 mmol) in dichloromethane (450 mL) were added triethylsilane (24.4 g, 210 mmol) and trifluoroacetic acid (450 mL) under a nitrogen atmosphere, and the mixture was stirred at room temperature for 48 h. The solvent was evaporated from the reaction solution under reduced pressure, and the residue was further dried using a vacuum pump under reduced pressure and then re-dissolved in an aqueous potassium carbonate solution. The reaction solution was washed with diethyl ether, concentrated hydrochloric acid was added until pH 2, and the mixture was then extracted with ethyl acetate twice. The resulting organic layers were combined and dried over anhydrous sodium sulfate, the solvent was evaporated under reduced pressure, and the residue was further dried using a vacuum pump under reduced pressure to afford N-((benzyloxy)carbonyl)-O-(2-(benzyloxy)ethyl)-N-methyl-L-serine (Compound aa24, Cbz-MeSer(EtOBn)-OH) (24.0 g) as a crude product. This was used in the next step without purification.

Synthesis of O-(2-hydroxyethyl)-N-methyl-L-serine (Compound aa25, H-MeSer(EtOH)-OH)

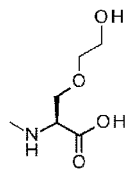

N-((Benzyloxy)carbonyl)-O-(2-(benzyloxy)ethyl)-N-methyl-L-serine (Compound aa24, Cbz-MeSer(EtOBn)-OH) (24.0 g, 62.0 mmol) and palladium on carbon (2.40 g, 10% w/w) were dissolved in methanol (400 mL) under a hydrogen atmosphere, and the reaction solution was stirred at room temperature for 16 h. The reaction solution was filtered, the solvent was then evaporated under reduced pressure, and the residue was further dried using a vacuum pump under reduced pressure to afford O-(2-hydroxyethyl)-N-methyl-L-serine (Compound aa25, H-MeSer(EtOH)-OH) (12.0 g) as a crude product. This was used in the next step without purification.

Synthesis of N-(((9H-fluoren-9-yl)methoxy)carbonyl)-O-(2-hydroxyethyl)-N-methyl-L-serine (Compound aa26, Fmoc-MeSer(EtOH)-OH)

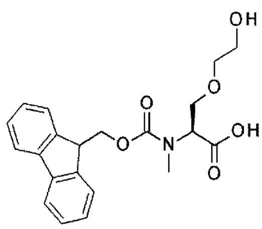

O-(2-Hydroxyethyl)-N-methyl-L-serine (Compound aa25, H-MeSer(EtOH)-OH) (12 g, 73.54 mmol), N-(9-fluorenylmethoxycarbonyloxy)succinimide (Fmoc-OSu) (27.3 g, 81.0 mmol), and sodium carbonate (27.3 g, 258 mmol) were dissolved in water/1,4-dioxane (320 mL/160 mL) under a nitrogen atmosphere, and the reaction solution was then stirred at room temperature for 3 h. After washing the reaction solution with t-butyl methyl ether three times, a 2 M aqueous hydrochloric acid solution was added to the aqueous layer until pH 2, and the aqueous layer was extracted with ethyl acetate three times. The resulting organic layers were combined, washed with brine, and then dried over anhydrous sodium sulfate, the solvent was evaporated under reduced pressure, and the residue was further dried using a vacuum pump under reduced pressure to afford N-(((9H-fluoren-9-yl)methoxy)carbonyl)-O-(2-hydroxyethyl)-N-methyl-L-serine (Compound aa26, Fmoc-MeSer(EtOH)-OH) (20.0 g) as a crude product. This was used in the next step without purification.

Synthesis of N-(((9H-fluoren-9-yl)methoxy)carbonyl)-N-methyl-O-(2-((tetrahydro-2H-pyran-2-yl)oxy)ethyl)-L-serine (Compound aa27, Fmoc-MeSer(EtOTHP)-OH)

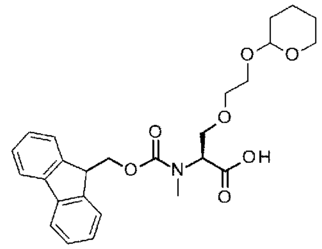

To a solution of N-(((9H-fluoren-9-yl)methoxy)carbonyl)-O-(2-hydroxyethyl)-N-methyl-L-serine (Compound aa26, Fmoc-MeSer(EtOH)-OH) (18.0 g, 46.7 mmol) in tetrahydrofuran (100 mL) were added pyridinium p-toluenesulfonate (0.59 g, 2.34 mmol) and 3,4-dihydro-2H-pyran (27.5 g, 327 mmol) under a nitrogen atmosphere, and the mixture was stirred at 50°C for 3 h. The mixture was cooled to 25°C and ethyl acetate was added. The organic layer was then washed with brine and dried over anhydrous sodium sulfate, and the solvent was evaporated under reduced pressure. 25.0 g of the resulting residue (31 g) was dissolved in tetrahydrofuran (200 mL), followed by addition of 1.0 M phosphate buffer adjusted to pH 6.8 (200 mL). This mixture was stirred at 50°C for 3 h. After cooling to 25°C, ethyl acetate was added, and the organic layer and the aqueous layer were separated. The aqueous layer was extracted with ethyl acetate, after which all the resulting organic layers were combined, washed with brine, and then dried over anhydrous sodium sulfate. The solvent was evaporated under reduced pressure, and the residue was further dried using a vacuum pump under reduced pressure to afford N-(((9H-fluoren-9-yl)methoxy)carbonyl)-N-methyl-O-(2-((tetrahydro-2H-pyran-2-yl)oxy)ethyl)-L-serine (Compound aa27, Fmoc-MeSer(EtOTHP)-OH) (20.0 g).
LCMS (ESI) m/z = 487 $(M+NH_4)+$

Retention time: 0.68 min (analytical condition SMD method 11)

Synthesis of (4S)-4-methyl-2-phenyl-L3-dioxolane (Compound aa28)

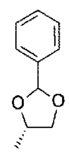

To a solution of (S)-propane-1,2-diol (20.0 g, 263 mmol) in toluene (200 mL) were added benzaldehyde (29.3 g, 276 mmol) and p-toluenesulfonic acid (0.45 g, 2.61 mmol) under a nitrogen atmosphere, and the mixture was stirred at 130°C for 16 h. The reaction solution was concentrated and the residue was purified by normal phase column chromatography (hexane/ethyl acetate) to afford (4S)-4-methyl-2-phenyl-1,3-dioxolane (Compound aa28) (31.5 g, 73%).
LCMS (ESI) m/z = 165 (M+H)+

Retention time: 0.89 min (analytical condition SMD method 12)

Synthesis of (2S)-2-(benzyloxy)propan-1-ol (Compound aa29)

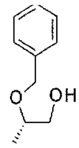

To a solution of (4S)-4-methyl-2-phenyl-1,3-dioxolane (Compound aa28) (32 g, 194.88 mmol) in dichloromethane (1.00 L) was added a 1 M solution of diisobutylaluminum hydride in hexane (390 mL, 390 mmol) at -50°C under a nitrogen atmosphere, and the mixture was stirred at the same temperature for 15 min and then stirred at room temperature for 2 h. The reaction was quenched by adding a saturated aqueous ammonium chloride solution, and the mixture was then extracted with dichloromethane twice. The combined organic layers were washed with brine and dried over anhydrous sodium sulfate, the solvent was evaporated under reduced pressure, and the residue was further dried using a vacuum pump under reduced pressure to afford (2S)-2-(benzyloxy)propan-1-ol (Compound aa29) (35.5 g) as a crude product. This was used in the next step without purification.

Synthesis of methyl N-((benzyloxy)carbonyl)-O-((S)-2-(tenzyloxy)propyl)-L-serinate (Compound aa30, Cbz-Ser(S-2-PrOBn)-OMe)

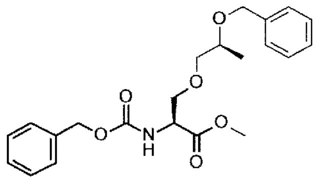

1-Benzyl 2-methyl (S)-aziridine-1,2-dicarboxylate (Cbz-Azy-OMe) (25.0 g, 106 mmol) and (2S)-2-(benzyloxy)propan-1-ol (Compound aa29) (26.5 g, 159 mmol) were dissolved in dichloromethane (188 mL) under a nitrogen atmosphere. After cooling to 0°C, boron trifluoride-diethyl ether complex (2.75 mL) was added and the mixture was stirred at 0°C for 1 hour and 30 minutes. Water was added to the reaction solution, the mixture was extracted with dichloromethane, and the organic layer was then washed with a saturated aqueous sodium carbonate solution and with brine. The organic layers were dried over anhydrous sodium sulfate, the solvent was evaporated under reduced pressure, and the residue was further dried using a vacuum pump under reduced pressure to afford methyl N-((benzyloxy)carbonyl)-O-((S)-2-(benzyloxy)propyl)-L-serinate (Compound aa30, Cbz-Ser(S-2-PrOBn)-OMe) (30.0 g) as a crude product. This was used in the next step without purification.

Synthesis of N-((benzylon)carbonyl)-O-((S)-2-(benzyloxy)propyl)-L-serine (Compound aa31, Cbz-Ser(S-2-PrOBn)-OH

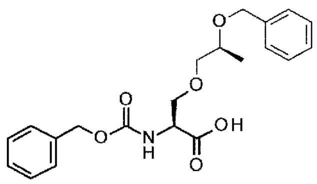

Lithium hydroxide monohydrate (10.5 g) and calcium chloride (103 g) were dissolved in water (300 mL) under a nitrogen atmosphere. A solution of methyl N-((benzyloxy)carbonyl)-O-((S)-2-(benzyloxy)propyl)-L-serinate (Compound aa30, Cbz-Ser(S-2-PrOBn)-OMe) (25 g, 62.3 mmol) in 2-propanol/tetrahydrofuran (1.20 L/300 mL) was added thereto at room temperature, and the mixture was stirred for 5 h. A 2 M aqueous hydrochloric acid solution was added until pH

2, the organic layer was removed, and the aqueous layer was extracted with ethyl acetate three times. The resulting organic layers were combined and dried over anhydrous sodium sulfate, the solvent was evaporated under reduced pressure, and the residue was further dried using a vacuum pump under reduced pressure to afford N-((benzyloxy)carbonyl)-O-((S)-2-(benzyloxy)propyl)-L-serine (Compound aa31, Cbz-Ser(S-2-PrOBn)-OH) (25.0 g) as a crude product. This was used in the next step without purification.

Synthesis of O-((S)-2-hydroxypropyl)-L-serine (Compound aa32, H-Ser(S-2-PrOH)-OH)

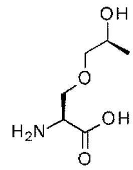

N-((Benzyloxy)carbonyl)-O-((S)-2-(benzyloxy)propyl)-L-serine (Compound aa31, Cbz-Ser(S-2-PrOBn)-OH) (3.00 g, 7.74 mmol) and palladium on carbon (0.30 g, 10% w/w) were dissolved in methanol (50.0 mL) under a hydrogen atmosphere, and the mixture was stirred at room temperature for 16 h. After filtering the reaction solution, the solvent was evaporated under reduced pressure and the residue was further dried using a vacuum pump under reduced pressure to afford O-((S)-2-hydroxypropyl)-L-serine (Compound aa32, H-Ser(S-2-PrOH)-OH) (0.78 g) as a crude product. This was used in the next step without purification.

Synthesis of N-(((9H-fluoren-9-yl)methoxy)carbonyl)-O-((S)-2-hydroxyropyl)-L-serine (Compound aa33, Fmoc-Ser(S-2-PrOH)-OH)

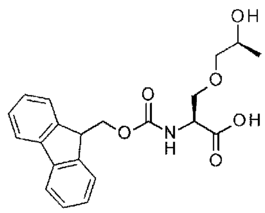

O-((S)-2-Hydroxypropyl)-L-serine (Compound aa32, H-Ser(S-2-PrOH)-OH) (0.78 g, 4.78 mmol), N-(9-fluorenylmethoxycarbonyloxy)succinimide (Fmoc-OSu) (1.61 g, 4.78 mmol), and N-ethyl-isopropylpropan-2-amine (DIPEA) (0.93 mg, 7.17 mmol) were dissolved in water/1,4-dioxane (8.00 mL/22.0 mL) under a nitrogen atmosphere, and the mixture was stirred at room temperature for 30 min. The reaction solution was washed with hexane (18.0 mL)/t-butyl methyl ether (6.00 mL) twice, a 2 M aqueous hydrochloric acid solution was then added to the aqueous layer until pH 2, and the aqueous layer was extracted with ethyl acetate (20.0 mL) twice. The resulting organic layers were combined, washed with brine, and then dried over anhydrous sodium sulfate, the solvent was evaporated under reduced pressure, and the residue was further dried using a vacuum pump under reduced pressure. The resulting residue was purified by reverse phase column chromatography (0.1% aqueous formic acid solution/0.1 % formic acid-acetonitrile solution) to afford N-(((9H-fluoren-9-yl)methoxy)carbonyl)-O-((S)-2-hydroxypropyl)-L-serine (Compound aa33, Fmoc-Ser(S-2-PrOH)-OH) (1.62 g, 88%).
LCMS (ESI) m/z = 386 (M+H)+

Retention time: 0.68 min (analytical condition SQDFA05)

Synthesis of N-(((9H-fluoren-9-yl)methoxy)carbonyl)-O-((2S)-2-((tetrahydro-2H-pyran-2-yl)oxy)propyl)-L-serine (Compound aa34, Fmoc-Ser(S-2-PrOTHP)-OH)

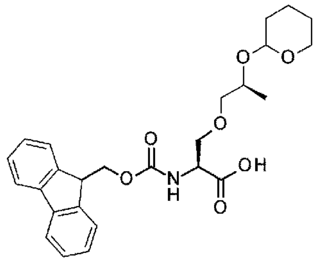

To a solution of N-(((9H-fluoren-9-yl)methoxy)carbonyl)-O-((S)-2-hydroxypropyl)-L-serine (Compound aa33, Fmoc-Ser(S-2-PrOH)-OH) (83 mg, 0.22 mmol) in tetrahydrofuran (1.00 mL) were added pyridinium p-toluenesulfonate (2.71 mg, 0.01 mmol) and 3,4-dihydro-2H-pyran (0.13 g, 1.51 mmol) under a nitrogen atmosphere, and the mixture was stirred at 50°C for 2 h. The mixture was cooled to 25°C and ethyl acetate was added. The organic layer was then washed with brine and dried over anhydrous sodium sulfate, and the solvent was evaporated under reduced pressure. The resulting residue was dissolved in tetrahydrofuran (1.00 mL), followed by addition of 1.0 M phosphate buffer adjusted to pH 6.8 (1.00 mL). This mixture was stirred at 50°C for 3 h. After cooling to 25°C, ethyl acetate (175 mL) was added and the organic layer and the aqueous layer were separated. The aqueous layer was extracted with ethyl acetate (175 mL), after which all the resulting organic layers were combined, washed with brine, and then dried over anhydrous sodium sulfate. The solvent was evaporated under reduced pressure, and the residue was further dried using a vacuum pump under reduced pressure to afford N-(((9H-fluoren-9-yl)methoxy)carbonyl)-O-((2S)-2-((tetrahydro-2H-pyran-2-yl)oxy)propyl)-L-serine (Compound aa34, Fmoc-Ser(S-2-PrOTHP)-OH) (88.0 mg, 87%).
LCMS (ESI) m/z = 468 (M-H)-

Retention time: 0.86 min, 0.87 min (analytical condition SQDFA05)

Synthesis of benzyl (S)-4-(((S)-2-(benzyloxy)propoxy)methyl)-5-oxooxazolidine-3-carboxylate (Compound aa35)

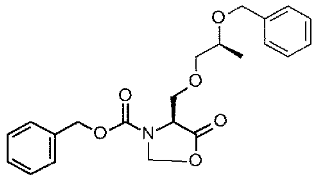

N-((Benzyloxy)carbonyl)-O-((S)-2-(benzyloxy)propyl)-L-serine (Compound aa31, Cbz-Ser(S-2-PrOBn)-OH) (25.0 g, 64.53 mmol), paraformaldehyde (5.80 g), and p-toluenesulfonic acid (0.67 g, 3.89 mmol) were dissolved in toluene (250 mL), and the reaction solution was stirred at 110°C for 16 h. After cooling to room temperature, the reaction solution was washed with an aqueous sodium bicarbonate solution, the organic layers were combined and dried over anhydrous sodium sulfate, the solvent was evaporated under reduced pressure, and the residue was further dried using a vacuum pump under reduced pressure to afford benzyl (S)-4-(((S)-2-(benzyloxy)propoxy)methyl)-5-oxooxazolidine-3-carboxylate (Compound aa35) (18.6 g) as a crude product. This was used in the next step without purification.

Synthesis of N-((benzyloxy)carbonyl)-O-((S)-2-(benzyloxy)propyl)-N-methyl-L-serine (Compound aa36, Cbz-MeSer(S-2-PrOBn)-OH)

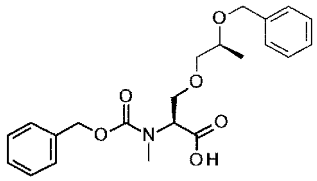

To a solution of benzyl (S)-4-(((S)-2-(benzyloxy)propoxy)methyl)-5-oxooxazolidine-3-carboxylate (Compound

aa35) (18.6 g, 46.6 mmol) in dichloromethane (296 mL) were added triethylsilane (16.2 g, 139 mmol) and trifluoroacetic acid (296 mL) under a nitrogen atmosphere, and the mixture was stirred at room temperature for 48 h. The solvent was evaporated from the reaction solution under reduced pressure, and the residue was further dried using a vacuum pump under reduced pressure and then re-dissolved in an aqueous potassium carbonate solution. The reaction solution was washed with diethyl ether, concentrated hydrochloric acid was added until pH 2, and the mixture was then extracted with ethyl acetate three times. The resulting organic layers were combined and dried over anhydrous sodium sulfate, the solvent was evaporated under reduced pressure, and the residue was further dried using a vacuum pump under reduced pressure to afford N-((benzyloxy)carbonyl)-O-((S)-2-(benzyloxy)propyl)-N-methyl-L-serine (Compound aa36, Cbz-MeSer(S-2-PrOBn)-OH) (9.4 g) as a crude product. This was used in the next step without purification.

Synthesis of O-((S)-2-hydroxypropyl)-N-methyl-L-serine (Compound aa37, H-MeSer(S-2-PrOH)-OH)

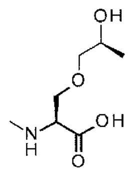

N-((Benzyloxy)carbonyl)-O-((S)-2-(benzyloxy)propyl)-N-methyl-L-serine (Compound aa36, Cbz-MeSer(S-2-PrOBn)-OH) (9.40 g, 23.4 mmol) and palladium on carbon (1.80 g, 10% w/w) were dissolved in methanol (185 mL) under a hydrogen atmosphere, and the reaction solution was stirred at room temperature for 16 h. The reaction solution was filtered, the solvent was then evaporated under reduced pressure, and the residue was further dried using a vacuum pump under reduced pressure to afford O-((S)-2-hydroxypropyl)-N-methyl-L-serine (Compound aa37, H-MeSer(S-2-PrOH)-OH) (3.40 g) as a crude product. This was used in the next step without purification.

Synthesis of N-(((9H-fluoren-9-yl)methoxy)carbonyl)-O-((S)-2-hydroxypropyl)-N-methyl-L-serine (Compound aa38, Fmoc-MeSer(S-2-PrOH)-OH)

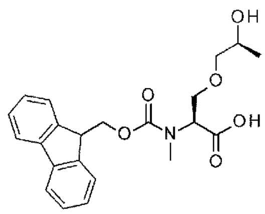

O-((S)-2-Hydroxypropyl)-N-methyl-L-serine (Compound aa37, H-MeSer(S-2-PrOH)-OH) (3.40 g, 19.2 mmol), N-(9-fluorenylmethoxycarbonyloxy)succinimide (Fmoc-OSu) (7.12 g, 22.1 mmol), and sodium carbonate (7.13 g, 67.3 mmol) were dissolved in water/1,4-dioxane (80.0 mL/40.0 mL) under a nitrogen atmosphere, and the reaction solution was stirred at room temperature for 3 h. After washing the reaction solution with t-butyl methyl ether three times, a 2 M aqueous hydrochloric acid solution was added to the aqueous layer until pH 2, and the aqueous layer was extracted with ethyl acetate three times. The resulting organic layers were combined, washed with brine, and then dried over anhydrous sodium sulfate, the solvent was evaporated under reduced pressure, and the residue was further dried using a vacuum pump under reduced pressure to afford N-(((9H-fluoren-9-yl)methoxy)carbonyl)-O-((S)-2-hydroxypropyl)-N-methyl-L-serine (Compound aa38, Fmoc-MeSer(S-2-PrOH)-OH) (8.00 g) as a crude product. This was used in the next step without purification.

Synthesis of N-(((9H-fluoren-9-yl)methoxy)carbonyl)-N-methyl-O-((2S)-2-((tetrahydro-2H-pyran-2-yl)oxy)propyl)-L-serine (Compound aa39, Fmoc-MeSer(S-2-PrOTHP)-OH)

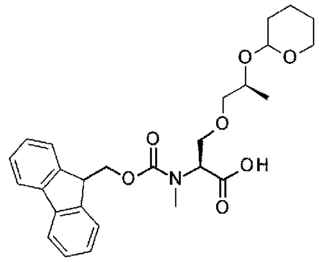

To a solution of N-(((9H-fluoren-9-yl)methoxy)carbonyl)-O-((S)-2-hydroxypropyl)-N-methyl-L-serine (Compound aa38, Fmoc-MeSer(S-2-PrOH)-OH) (80.0 g, 20.0 mmol) in tetrahydrofuran (40.0 mL) were added pyridinium p-toluenesulfonate (0.26 g, 1.02 mmol) and 3,4-dihydro-2H-pyran (11.8 g, 140 mmol) under a nitrogen atmosphere, and the mixture was stirred at 50°C for 2 h. The mixture was cooled to 25°C and ethyl acetate was added. The organic layer was then washed with brine and dried over anhydrous sodium sulfate, and the solvent was evaporated under reduced pressure. The resulting residue (9.30 g) was dissolved in tetrahydrofuran (50.0 mL), followed by addition of 1.0 M phosphate buffer adjusted to pH 6.8 (50.0 mL). This mixture was stirred at 50°C for 3 h. After cooling to 25°C, ethyl acetate was added and the organic layer and the aqueous layer were separated. The aqueous layer was extracted with ethyl acetate, after which all the resulting organic layers were combined, washed with brine, and then dried over anhydrous sodium sulfate. The solvent was evaporated under reduced pressure, and the residue was further dried using a vacuum pump under reduced pressure. The resulting residue was purified by reverse phase column chromatography (0.5% aqueous ammonium bicarbonate solution/acetonitrile) to afford N-(((9H-fluoren-9-yl)methoxy)carbonyl)-N-methyl-O-((2S)-2-((tetrahydro-2H-pyran-2-yl)oxy)propyl)-L-serine (Compound aa39, Fmoc-MeSer(S-2-PrOTHP)-OH) (7.00 g, 72%).
LCMS (ESI) m/z = 501 $(M+NH_4)^+$

Retention time: 0.82 min (analytical condition SMD method 10)

Synthesis of (4R)-4-methyl-2-phenyl-1,3-dioxolane (Compound aa40)

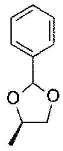

To a solution of (R)-propane-1,2-diol (20 g, 262.83 mmol) in toluene (200 mL) were added benzaldehyde (29.3 g, 276 mmol) and p-toluenesulfonic acid (0.45 g, 2.61 mmol) under a nitrogen atmosphere, and the mixture was stirred at 130°C for 16 h. The reaction solution was concentrated and the residue was purified by normal phase column chromatography (hexane/ethyl acetate) to afford (4R)-4-methyl-2-phenyl-1,3-dioxolane (Compound aa40) (31.5 g, 73%).
LCMS (ESI) m/z = 163 (M-H)-

Retention time: 7.21 min (analytical condition GC01)

Synthesis of (2R)-2-(benzyloxy)propan-1-ol (Compound aa41)

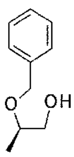

To a solution of (4R)-4-methyl-2-phenyl-1,3-dioxolane (Compound aa40) (32.0 g, 195 mmol) in dichloromethane (1.00 L) was added a 1 M solution of diisobutylaluminum hydride in hexane (390 mL, 390 mmol) at -50°C under a nitrogen

atmosphere, and the mixture was stirred at the same temperature for 15 min and then stirred at room temperature for 2 h. The reaction was quenched by adding a saturated aqueous ammonium chloride solution, and the mixture was then extracted with dichloromethane twice. The combined organic layers were washed with brine and dried over anhydrous sodium sulfate, the solvent was evaporated under reduced pressure, and the residue was further dried using a vacuum pump under reduced pressure to afford (2R)-2-(benzyloxy)propan-1-ol (Compound aa41) (35.5 g) as a crude product. This was used in the next step without purification.

Synthesis of methyl N-((benzyloxy)carbonyl)-O-((R)-2-(benzyloxy)-propyl)-L-serinate (Compound aa42, Cbz-Ser(R-2-PrOBn)-OMe)

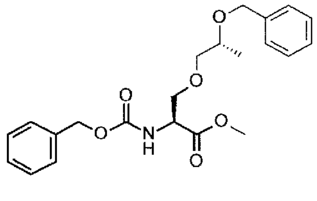

1-Benzyl 2-methyl (S)-aziridine-1,2-dicarboxylate (Cbz-Azy-OMe) (25 g, 106.28 mmol) and (2R)-2-(benzyloxy)propan-1-ol (Compound aa41) (26.5 g, 159 mmol) were dissolved in dichloromethane (188 mL) under a nitrogen atmosphere. After cooling to 0°C, boron trifluoride-diethyl ether complex (2.75 mL) was added and the mixture was stirred at 0°C for 1 hour and 30 minutes. Water was added to the reaction solution, the mixture was extracted with dichloromethane, and the organic layer was then washed with a saturated aqueous sodium carbonate solution and with brine. The organic layers were dried over anhydrous sodium sulfate, the solvent was evaporated under reduced pressure, and the residue was further dried using a vacuum pump under reduced pressure to afford methyl N-((benzyloxy)carbonyl)-O-((R)-2-(benzyloxy)propyl)-L-serinate (Compound aa42, Cbz-Ser(R-2-PrOBn)-OMe) (50.0 g) as a crude product. This was used in the next step without purification.

Synthesis of N-((benzyloxy)carbonyl)-O-((R)-2-(benzyloxy)propyl)-L-serine (Compound aa43,

Cbz-Ser(R-2-PrOBn)-OH)

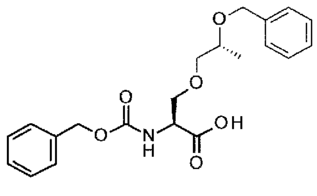

Lithium hydroxide monohydrate (10.5 g) and calcium chloride (103 g) were dissolved in water (300 mL) under a nitrogen atmosphere. A solution of methyl N-((benzyloxy)carbonyl)-O-((R)-2-(benzyloxy)propyl)-L-serinate (Compound aa42, Cbz-Ser(R-2-PrOBn)-OMe) (25.0 g, 62.3 mmol) in 2-propanol/tetrahydrofuran (1.50 L/150 mL) was added thereto at room temperature, and the mixture was stirred for 5 h. A 2 M aqueous hydrochloric acid solution was added until pH 2, the organic layer was removed, and the aqueous layer was extracted with ethyl acetate three times. The resulting organic layers were combined and dried over anhydrous sodium sulfate, the solvent was evaporated under reduced pressure, and the residue was further dried using a vacuum pump under reduced pressure to afford N-((benzyloxy)carbonyl)-O-((R)-2-(benzyloxy)propyl)-L-serine (Compound aa43, Cbz-Ser(R-2-PrOBn)-OH) (25.0 g) as a crude product. This was used in the next step without purification.

Synthesis of O-((R)-2-hydroxypropyl)-L-serine (Compound aa44, H-Ser(R-2-PrOH)-OH)

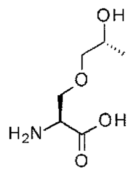

N-((Benzyloxy)carbonyl)-O-((R)-2-(benzyloxy)propyl)-L-serine (Compound aa43, Cbz-Ser(R-2-PrOBn)-OH) (2.87 g, 7.41 mmol) and palladium on carbon (570 mg, 20% w/w) were dissolved in methanol (50 mL) under a hydrogen atmosphere, and the mixture was stirred at room temperature for 16 h. The reaction solution was filtered, the solvent was then evaporated under reduced pressure, and the resulting residue was recrystallized with ethyl acetate at 60°C to afford O-((R)-2-hydroxypropyl)-L-serine (Compound aa44, H-Ser(R-2-PrOH)-OH) (900 mg, 74%).

<u>Synthesis of N-(((9H-fluoren-9-yl)methoxy)carbonyl)-O-((R)-2-hydroxypropyl)-L-serine (Compound aa45, Fmoc-Ser(R-2-PrOH)-OH)</u>

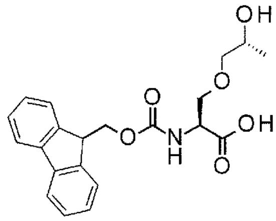

O-((R)-2-Hydroxypropyl)-L-serine (Compound aa44, H-Ser(R-2-PrOH)-OH) synthesized by the method described above (5.0 g, 30.64 mmol) and sodium bicarbonate (7.71 g, 91.8 mmol) were dissolved in water/1,4-dioxane (127 mL/52.9 mL) under a nitrogen atmosphere, N-(9-fluorenylmethoxycarbonyloxy)succinimide (Fmoc-OSu) (10.85 g, 32.2 mmol) was added at room temperature, and the mixture was stirred for 4 h. Water was added to the reaction solution, and the mixture was washed with t-butyl methyl ether. Concentrated hydrochloric acid was then added to the aqueous layer until pH 3, and the aqueous layer was extracted with t-butyl methyl ether twice. The resulting organic layers were washed with brine and then dried over anhydrous sodium sulfate, and the solvent was evaporated under reduced pressure. The resulting residue was purified by reverse phase column chromatography (water/acetonitrile) to afford N-(((9H-fluoren-9-yl)methoxy)carbonyl)-O-((R)-2-hydroxypropyl)-L-serine (Compound aa45, Fmoc-Ser(R-2-PrOH)-OH) (8 g, 68%).
LCMS (ESI) m/z = 386 (M+H)+

Retention time: 2.08 min (analytical condition SMD method 49)

<u>Synthesis of N-(((9H-fluoren-9-yl)methoxy)carbonyl)-O-((2R)-2-((tetrahydro-2H-pyran-2-yl)oxy)propyl)-L-serine (Compound aa46, Fmoc-Ser(R-2-PrOTHP)-OH)</u>

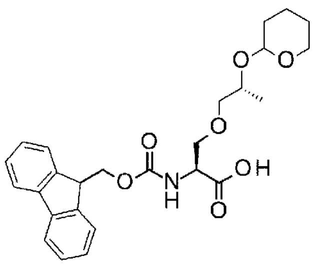

To a solution of N-(((9H-fluoren-9-yl)methoxy)carbonyl)-O-((R)-2-hydroxypropyl)-L-serine (Compound aa45, Fmoc-Ser(R-2-PrOH)-OH) (4.4 g, 11.4 mmol) in tetrahydrofuran (50 mL) were added pyridinium p-toluenesulfonate (143

mg, 5.7 mmol) and 3,4-dihydro-2H-pyran (6.7 g, 79.8 mmol) under a nitrogen atmosphere, and the mixture was stirred at 50°C for 3 h. The mixture was cooled at room temperature, t-butyl methyl ether was added, and the mixture was then washed with brine. The organic layer was dried over anhydrous sodium sulfate and filtered, and the resulting solvent was evaporated under reduced pressure to afford a crude product (8 g). To the resulting crude product (800 mg) was added a mixture of tetrahydrofuran (10 mL) and 1.0 M phosphate buffer adjusted to pH 6.8 (10 mL), followed by stirring at 50°C for 3 h. After cooling the reaction solution at room temperature, t-butyl methyl ether was added and the reaction solution was washed with brine and filtered. The resulting solution was dried over anhydrous sodium sulfate, and the solvent was evaporated under reduced pressure. The resulting residue was purified by reverse phase column chromatography (0.5% aqueous ammonium bicarbonate solution/acetonitrile), the resulting fractions were combined, and the acetonitrile was evaporated under reduced pressure. To the resulting aqueous layer was added a 1.0 M phosphate buffer adjusted to pH 6.8. The aqueous layer was extracted with t-butyl methyl ether three times and the solvent was evaporated under reduced pressure to afford N-(((9H-fluoren-9-yl)methoxy)carbonyl)-O-((2R)-2-((tetrahydro-2H-pyran-2-yl)oxy)propyl)-L-serine (Compound aa46, Fmoc-Ser(R-2-PrOTHP)-OH) (400 mg, 65% over two steps).
LCMS (ESI) m/z = 468 (M-H)-

Retention time: 0.85 min, 0.87 min (analytical condition SQDFA05)

Synthesis of benzyl (S)-4-(((R)-2-(benzyloxy)propoxy)methyl)-5-oxooxazolidine-3 -carboxylate (Compound aa47)

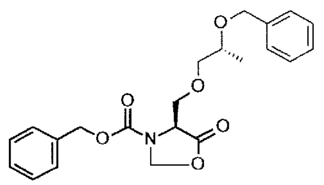

N-((Benzyloxy)carbonyl)-O-((R)-2-(benzyloxy)propyl)-L-serine (Compound aa43, Cbz-Ser(R-2-PrOBn)-OH) (25.0 g, 64.5 mmol), paraformaldehyde (5.80 g), and p-toluenesulfonic acid (0.67 g, 3.89 mmol) were dissolved in toluene (250 mL), and the reaction solution was stirred at 110°C for 16 h. After cooling to room temperature, the reaction solution was washed with an aqueous sodium bicarbonate solution, the organic layers were combined and dried over anhydrous sodium sulfate, the solvent was evaporated under reduced pressure, and the residue was further dried using a vacuum pump under reduced pressure to afford benzyl (S)-4-(((R)-2-(benzyloxy)propoxy)methyl)-5-oxooxazolidine-3-carboxylate (Compound aa47) (18.6 g) as a crude product. This was used in the next step without purification.

Synthesis of N-((benzyloxy)carbonyl)-O-((R)-2-(benzyloxy)propyl)-N-methyl-L-serine (Compound aa48, Cbz-MeSer(R-2-PrOBn)-OH)

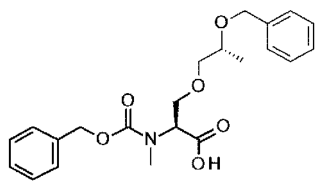

To a solution of benzyl (S)-4-(((R)-2-(benzyloxy)propoxy)methyl)-5-oxooxazolidine-3-carboxylate (Compound aa47) (18.6 g, 46.6 mmol) in dichloromethane (296 mL) were added triethylsilane (16.2 g, 139 mmol) and trifluoroacetic acid (296 mL) under a nitrogen atmosphere, and the mixture was stirred at room temperature for 48 h. The solvent was evaporated from the reaction solution under reduced pressure, and the residue was further dried using a vacuum pump under reduced pressure and then re-dissolved in an aqueous potassium carbonate solution. The reaction solution was washed with diethyl ether, concentrated hydrochloric acid was added until pH 2, and the mixture was then extracted with ethyl acetate three times. The resulting organic layers were combined and dried over anhydrous sodium sulfate, the solvent was evaporated under reduced pressure, and the residue was further dried using a vacuum pump under reduced pressure to afford N-((benzyloxy)carbonyl)-O-((R)-2-(benzyloxy)propyl)-N-methyl-L-serine (Compound aa48, Cbz-MeSer(R-2-PrOBn)-OH) (9.40 g) as a crude product. This was used in the next step without purification.

Synthesis of O-((R)-2-hydroxypropyl)-N-methyl-L-serine (Compound aa49, H-MeSer(R-2-PrOH)-OH)

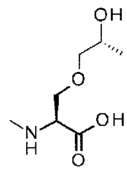

N-((Benzyloxy)carbonyl)-O-((R)-2-(benzyloxy)propyl)-N-methyl-L-serine (Compound aa48, Cbz-MeSer(R-2-PrOBn)-OH) (9.40 g, 23.4 mmol) and palladium on carbon (1.80 g, 10% w/w) were dissolved in methanol (185 mL) under a hydrogen atmosphere, and the reaction solution was stirred at room temperature for 16 h. The reaction solution was filtered, the solvent was then evaporated under reduced pressure, and the residue was further dried using a vacuum pump under reduced pressure to afford O-((R)-2-hydroxypropyl)-N-methyl-L-serine (Compound aa49, H-MeSer(R-2-PrOH)-OH) (3.40 g) as a crude product. This was used in the next step without purification.

Synthesis of N-(((9H-fluoren-9-yl)methoxy)carbonyl)-O-((R)-2-hydroxyropyl)-N-methyl-L-serine (Compound aa50, Fmoc-MeSer(R-2-PrOH)-OH)

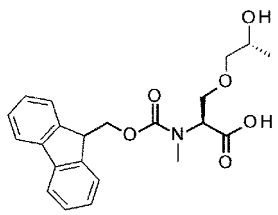

O-((R)-2-Hydroxypropyl)-N-methyl-L-serine (Compound aa49, H-MeSer(R-2-PrOH)-OH) (3.40 g, 19.2 mmol), N-(9-fluorenylmethoxycarbonyloxy)succinimide (Fmoc-OSu) (7.12 g, 22.1 mmol), and sodium carbonate (7.13 g, 67.3 mmol) were dissolved in water/1,4-dioxane (80.0 mL/40.0 mL) under a nitrogen atmosphere, and the reaction solution was stirred at room temperature for 3 h. After washing the reaction solution with t-butyl methyl ether three times, a 2 M aqueous hydrochloric acid solution was added to the aqueous layer until pH 2, and the aqueous layer was extracted with ethyl acetate three times. The resulting organic layers were combined, washed with brine, and then dried over anhydrous sodium sulfate, the solvent was evaporated under reduced pressure, and the residue was further dried using a vacuum pump under reduced pressure to afford N-(((9H-fluoren-9-yl)methoxy)carbonyl)-O-((R)-2-hydroxypropyl)-N-methyl-L-serine (Compound aa50, Fmoc-MeSer(R-2-PrOH)-OH) (8.00 g) as a crude product. This was used in the next step without purification.

Synthesis of N-(((9H-fluoren-9-yl)methoxy)carbonyl)-N-methyl-O-((2R)-2-((tetrahydro-2H-pyran-2-yl)oxy)propyl)-L-serine (Compound aa51, Fmoc-MeSer(R-2-PrOTHP)-OH)

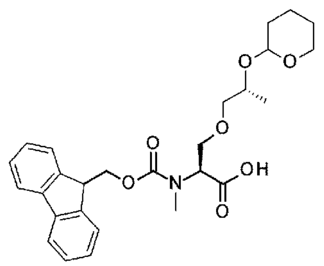

To a solution of N-(((9H-fluoren-9-yl)methoxy)carbonyl)-O-((R)-2-hydroxypropyl)-N-methyl-L-serine (Compound aa50, Fmoc-MeSer(R-2-PrOH)-OH) (8.00 g, 20.0 mmol) in tetrahydrofuran (40.0 mL) were added pyridinium p-toluenesulfonate (0.26 g, 1.02 mmol) and 3,4-dihydro-2H-pyran (11.8 g, 139 mmol) under a nitrogen atmosphere, and the mixture was stirred at 50°C for 2 h. The mixture was cooled to 25°C and ethyl acetate was added. The organic layer was then washed with brine and dried over anhydrous sodium sulfate, and the solvent was evaporated under reduced pressure. The resulting residue (9.3 g) was dissolved in tetrahydrofuran (100 mL), followed by addition of 1.0 M phosphate buffer adjusted to pH 6.8 (100 mL). This mixture was stirred at 50°C for 3 h. After cooling to 25°C, ethyl acetate was added and the organic layer and the aqueous layer were separated. The aqueous layer was extracted with ethyl acetate, after which all the resulting organic layers were combined, washed with brine, and then dried over anhydrous sodium sulfate. The solvent was evaporated under reduced pressure, and the residue was further dried using a vacuum pump under reduced pressure to afford N-(((9H-fluoren-9-yl)methoxy)carbonyl)-N-methyl-O-((2R)-2-((tetrahydro-2H-pyran-2-yl)oxy)propyl)-L-serine (Compound aa51, Fmoc-MeSer(R-2-PrOTHP)-OH) (8.50 g).
LCMS (ESI) m/z = 501 $(M+NH_4)+$

Retention time: 0.82 min (analytical condition SMD method 10)

N-(((9H-Fluoren-9-yl)methoxy)carbonyl)-O-(2-methyl-2-((tetrahydro-2H-pyran-2-yl)oxy)propyl)-L-serine (Compound aa54, Fmoc-Ser(tBuOTHP)-OH) was synthesized according to the following scheme.

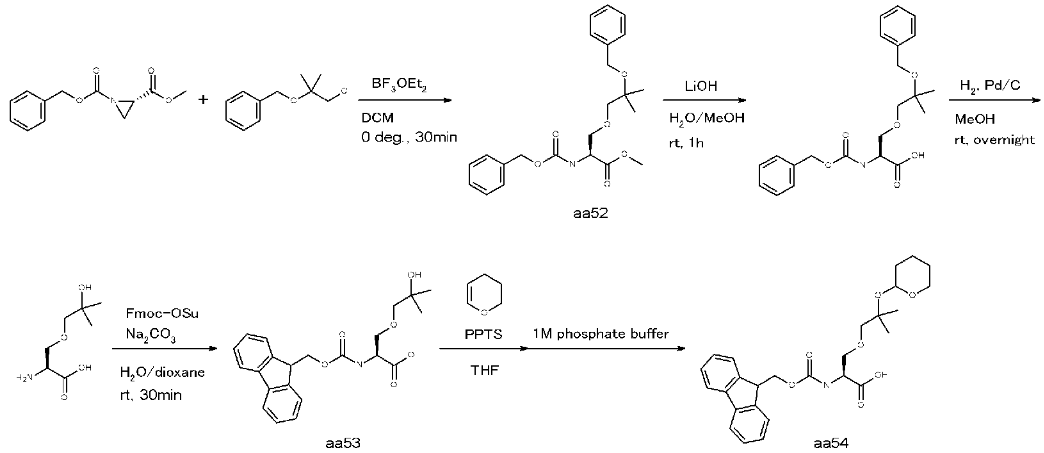

aa52
aa53
aa54

<u>Synthesis of methyl O-(2-(benzyloxy)-2-methylpropyl)-N-((benzyloxy)carbonyl)-L-serinate (Compound aa52, Cbz-Ser(tBuOBn)-OMe)</u>

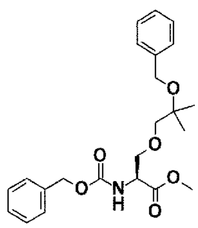

1-Benzyl 2-methyl (S)-aziridine-1,2-dicarboxylate (Cbz-Azy-OMe) (2.0 g, 8.50 mmol) and 2-benzyloxy-2-methylpropan-1-ol (2.30 g, 12.75 mmol) were dissolved in dichloromethane (7.5 mL), and boron trifluoride-diethyl ether complex ($BF_3 \cdot OEt_2$) (0.160 mL, 1.28 mmol) was added dropwise over 5 min under ice-cooling. After stirring for 30 min under ice-cooling, water (2.0 mL) was added, the reaction was quenched by stirring for 10 min, and a saturated aqueous sodium bicarbonate solution was added. The aqueous layer was extracted with dichloromethane, and the organic layer was dried over anhydrous magnesium sulfate. After filtration, the organic solvent was removed by concentration under reduced pressure, and the resulting residue was purified by normal phase column chromatography (hexane/ethyl acetate = 100/0 -> 75/25) to afford methyl O-(2-(benzyloxy)-2-methylpropyl)-N-((benzyloxy)carbonyl)-L-serinate (Compound aa52, Cbz-Ser(tBuOBn)-OMe) (2.36 g, 67%).
LCMS (ESI) m/z = 416 (M+H)+

Retention time: 0.93 min (analytical condition SQDFA05)

Synthesis of N-(((9H-fluoren-9-yl)methoxy)carbonyl)-O-(2-hydroxy-2-methylpropyl)-L-serine (Compound aa53, Fmoc-Ser(tBuOH)-OH)

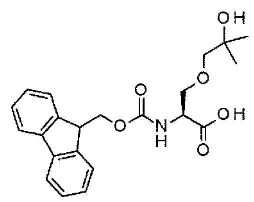

Methyl O-(2-(benzyloxy)-2-methylpropyl)-N-((benzyloxy)carbonyl)-L-serinate (Compound aa52, Cbz-Ser(tBuOBn)-OMe) (2.36 g, 5.68 mmol) was dissolved in methanol (8.0 mL). An aqueous solution of lithium hydroxide monohydrate (0.477 g, 11.36 mmol) dissolved in water (8.0 mL) was added thereto and the mixture was stirred at room temperature for 1 h. Thereafter, a 2 M aqueous hydrochloric acid solution (8.52 mL, 17.04 mmol) was added. The aqueous layer was extracted with ethyl acetate three times, and the organic layers were dried over anhydrous magnesium sulfate. After filtration, the ethyl acetate was removed by concentration under reduced pressure to afford O-(2-(benzyloxy)-2-methylpropyl)-N-((benzyloxy)carbonyl)-L-serine (Cbz-Ser(tBuOBn)-OH).

O-(2-(benzyloxy)-2-methylpropyl)-N-((benzyloxy)carbonyl)-L-serine (Cbz-Ser(tBuOBn)-OH) obtained above was dissolved in methanol (45 mL), Pd/C (456 mg) was added, and the mixture was stirred at room temperature overnight under a hydrogen atmosphere. The Pd/C was removed by filtration through celite and the methanol was then removed by concentration under reduced pressure to afford O-(2-hydroxy-2-methylpropyl)-L-serine (H-Ser(tBuOH)-OH) (1.05 g) quantitatively.

The resulting O-(2-hydroxy-2-methylpropyl)-L-serine (H-Ser(tBuOH)-OH) (1.59 g, 8.97 mL) and sodium carbonate (2.85 g, 26.9 mmol) were dissolved in water (36 mL) and 1,4-dioxane (15 mL), and N-(9-fluorenylmethoxycarbonyloxy)succinimide (Fmoc-OSu) (3.18 g, 9.42 mmol) was slowly added. After stirring at room temperature for 30 min, water (40 mL) was added to the reaction solution, and the mixture was washed with t-butyl methyl ether (60 mL) three times. Thereafter, the aqueous layer was adjusted to pH 1 by adding a 2 M aqueous hydrochloric acid solution (26.9 mL, 53.8 mmol) to the aqueous layer, and was then extracted with t-butyl methyl ether (60 mL) three times. The organic layers were dried over anhydrous magnesium sulfate and filtered, after which the t-butyl methyl ether was removed by concentration under reduced pressure. The resulting residue was purified by reverse phase column chromatography (0.1% aqueous formic acid solution/0.1 formic acid-acetonitrile solution) to afford N-(((9H-fluoren-9-yl)methoxy)carbonyl)-O-(2-hydroxy-2-methylpropyl)-L-serine (Compound aa53, Fmoc-Ser(tBuOH)-OH) (2.62 g, 73%).
LCMS (ESI) m/z = 400 (M+H)+

Retention time: 0.71 min (analytical condition SQDFA05)

Synthesis of N-(((9H-fluoren-9-yl)methoxy)carbonyl)-O-(2-methyl-2-((tetrahydro-2H-pyran-2-yl)oxy)propyl)-L-serine (Compound aa54, Fmoc-Ser(tBuOTHP)-OH)

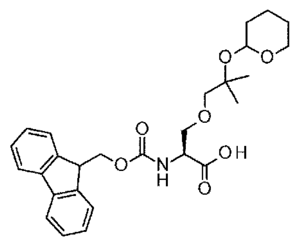

N-(((9H-Fluoren-9-yl)methoxy)carbonyl)-O-(2-hydroxy-2-methylpropyl)-L-serine (Compound aa53, Fmoc-Ser(tBuOH)-OH) (1.2 g, 3.00 mmol) and 3,4-dihydro-2H-pyran (1.90 mL, 21.03 mmol) were dissolved in tetrahydrofuran (6.0 mL), pyridinium p-toluenesulfonate (PPTS) (0.038 g, 0.150 mmol) was added, and the mixture was stirred at 50°C

for 2 h. The reaction solution was then diluted with ethyl acetate (15 mL) and washed with brine (15 mL) three times. The organic layers were dried over anhydrous sodium sulfate and then filtered, and the solvent was removed by concentration under reduced pressure. The resulting residue was dissolved in tetrahydrofuran (24.0 mL), 1.0 M phosphate buffer adjusted to pH 6.8 (24.0 mL) was added, and the mixture was stirred at 50°C for 3 h. The reaction solution was diluted with ethyl acetate (45 mL) and then washed with brine (45 mL) three times, and the organic layers were dried over anhydrous sodium sulfate. After filtration, concentration under reduced pressure gave a residue, which was then purified by reverse phase column chromatography (10 mM aqueous ammonium acetate solution/methanol) to afford N-(((9H-fluoren-9-yl)methoxy)carbonyl)-O-(2-methyl-2-((tetrahydro-2H-pyran-2-yl)oxy)propyl)-L-serine (Compound aa54, Fmoc-Ser(tBuOTHP)-OH) (1.05 g, 72%).
LCMS (ESI) m/z = 484 (M+H)+

Retention time: 0.91 min (analytical condition SQDFA05)

N-(((9H-Fluoren-9-yl)methoxy)carbonyl)-N-methyl-O-(2-methyl-2-((tetrahydro-2H-pyran-2-yl)oxy)propyl)-L-serine (Compound aa55, Fmoc-MeSer(tBuOTHP)-OH) was synthesized according to the following scheme.

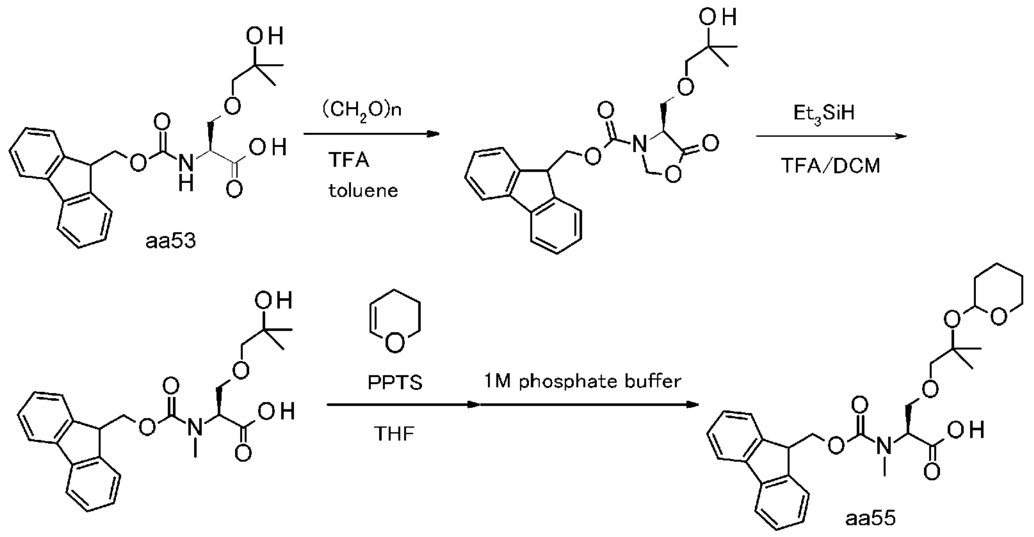


Synthesis of N-(((9H-fluoren-9-yl)methoxy)carbonyl)-N-methyl-O-(2-methyl-2-((tetrahydro-2H-pyran-2-yl)oxy)propyl)-L-serine (Compound aa55, Fmoc-MeSer(tBuOTHP)-OH)

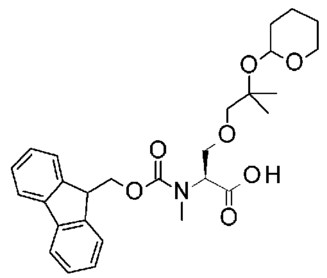

N-(((9H-Fluoren-9-yl)methoxy)carbonyl)-O-(2-hydroxy-2-methylpropyl)-L-serine (Compound aa53, Fmoc-Ser(tBuOH)-OH) (12 g, 30.04 mmol) and paraformaldehyde (2.75 g, 91.67 mmol) were dissolved in trifluoroacetic acid (31.4 g, 277.78 mmol) and toluene (60 mL), and the reaction solution was stirred at room temperature for 4 h. The reaction solution was concentrated under reduced pressure, after which the resulting residue was dissolved in dichloromethane, washed with a saturated aqueous sodium bicarbonate solution and with brine, and then dried over anhydrous sodium sulfate. After filtration, the dichloromethane was removed by concentration under reduced pressure to afford (9H-fluoren-9-yl)methyl (S)-4-((2-hydroxy-2-methylpropoxy)methyl)-5-oxooxazolidine-3-carboxylate (12 g, 97%).

The obtained (9H-fluoren-9-yl)methyl (S)-4-((2-hydroxy-2-methylpropoxy)methyl)-5-oxooxazolidine-3-carboxylate (1 g, 2.43 mmol) was dissolved in trifluoroacetic acid/dichloromethane (13 mL/13 mL), and triethylsilane ($Et_3SiH$) (832 mg, 7.16 mmol) was added dropwise. After stirring at room temperature for 16 h, the reaction solution was concentrated under reduced pressure. The resulting residue was dissolved in an aqueous potassium carbonate solution and washed with t-butyl methyl ether, after which the aqueous layer was adjusted to pH 2 with an aqueous hydrochloric acid solution (1 M). The aqueous layer was extracted with t-butyl methyl ether twice, and the organic layers were washed with water twice and with brine twice and dried over anhydrous sodium sulfate. After filtration, concentration under reduced pressure gave a residue, which was then purified by reverse phase column chromatography (water/acetonitrile = 100/0 -> 50/50) to afford N-(((9H-fluoren-9-yl)methoxy)carbonyl)-O-(2-hydroxy-2-methylpropyl)-N-methyl-L-serine (Fmoc-MeSer(tBuOH)-OH) (700 mg, 70%).

The obtained N-(((9H-fluoren-9-yl)methoxy)carbonyl)-O-(2-hydroxy-2-methylpropyl)-N-methyl-L-serine (Fmoc-MeSer(tBuOH)-OH) (6 g, 14.51 mmol) and pyridinium p-toluenesulfonate (PPTS) (182 mg, 0.73 mmol) were dissolved in tetrahydrofuran (48 mL), and 3,4-dihydro-2H-pyran (8.5 g, 101.05 mmol) was added dropwise at room temperature under a nitrogen atmosphere. The reaction solution was stirred at 50°C for 5 h, and the reaction solution was then extracted by adding ethyl acetate. The organic layers were dried over anhydrous sodium sulfate, filtered, and then concentrated under reduced pressure to afford N-(((9H-fluoren-9-yl)methoxy)carbonyl)-N-methyl-O-(2-methyl-2-((tetrahydro-2H-pyran-2-yl)oxy)propyl)-L-serine and tetrahydro-2H-pyran-2-yl N-(((9H-fluoren-9-yl)methoxy)carbonyl)-N-methyl-O-(2-methyl-2-((tetrahydro-2H-pyran-2-yl)oxy)propyl)-L-serinate as a mixture.

The obtained mixture (50 g) was dissolved in phosphate buffer (1 M, pH = 6.8, 1000 mL) and tetrahydrofuran (1000 mL), and the reaction solution was stirred at 50°C for 4 h. The reaction solution was then extracted with ethyl acetate. The organic layers were washed with brine, then dried over anhydrous sodium sulfate, filtered, and then concentrated under reduced pressure. The resulting residue was purified by reverse phase column chromatography (0 to 40% 0.5% aqueous ammonium bicarbonate solution/acetonitrile) to afford N-(((9H-fluoren-9-yl)methoxy)carbonyl)-N-methyl-O-(2-methyl-2-((tetrahydro-2H-pyran-2-yl)oxy)propyl)-L-serine (Compound aa55, Fmoc-MeSer(tBuOTHP)-OH) (20 g).

LCMS (ESI) m/z = 498 $(M+H)^+$

Retention time: 0.94 min (analytical condition SMD method 20)

Synthesis of 4,4-dimethyl-2-phenyl-1,3-dioxane (Compound aa56)

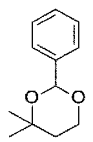

To a solution of 3-methylbutane-1,3-diol (40.0 g, 384 mmol) in chloroform (400 mL) were added dimethoxymethylbenzene (87.6 g, 576 mmol) and p-toluenesulfonic acid (3.59 g, 18.9 mmol) under a nitrogen atmosphere, and the mixture was stirred at 0°C for 1.5 h. The reaction solution was concentrated and the residue was purified by column chromatography (hexane/ethyl acetate) to afford 4,4-dimethyl-2-phenyl-1,3-dioxane (Compound aa56) (70.0 g, 95%).

Synthesis of 3-(benzyloxy)-3-methylbutan-1-ol (Compound aa57)

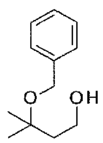

To a solution of 4,4-dimethyl-2-phenyl-1,3-dioxane (Compound aa56) obtained by the above method (5.00 g, 26.0 mmol) in dichloromethane (130 mL) was added a 1 M solution of diisobutylaluminum hydride in hexane (156 mL, 156 mmol) at -50°C under a nitrogen atmosphere, and the mixture was stirred at the same temperature for 15 min and then stirred at room temperature for 1 h. The reaction was quenched by adding methanol, and the mixture was then extracted with dichloromethane twice. The combined organic layers were washed with brine and dried over anhydrous

sodium sulfate, the solvent was evaporated under reduced pressure, and the residue was purified by column chromatography (hexane/ethyl acetate) to afford 3-(benzyloxy)-3-methylbutan-1-ol (Compound aa57) (2.00 g, 40%).

Synthesis of methyl O-(3-(benzyloxy)-3-methylbutyl)-N-((benzyloxy)carbonyl)-L-serinate (Compound aa58, Cbz-Ser(2-Me-BuOBn)-OMe)

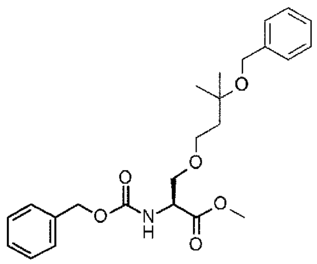

1-Benzyl 2-methyl (S)-aziridine-1,2-dicarboxylate (25 g, 106 mmol) and 3-(benzyloxy)-3-methylbutan-1-ol (Compound aa57) (31.0 g, 159 mmol) were dissolved in dichloromethane (113 mL) under a nitrogen atmosphere and the reaction solution was cooled to 0°C, after which boron trifluoride-diethyl ether complex (2.26 g, 15.9 mmol) was added and the mixture was stirred at 0°C for 3 h. Water was added to the reaction solution, the mixture was extracted with dichloromethane, and the organic layer was then washed with a saturated aqueous sodium carbonate solution and brine. The organic layer was dried over anhydrous sodium sulfate, the solvent was evaporated under reduced pressure, and the residue was purified by column chromatography (hexane/ethyl acetate) to afford methyl O-(3-(benzyloxy)-3-methylbutyl)-N-((benzyloxy)carbonyl)-L-serinate (Compound aa58, Cbz-Ser(2-Me-BuOBn)-OMe) (28.0 g, 55%).
LCMS (ESI) m/z = 452 (M+Na)+

Retention time: 1.46 min (analytical condition SMD method 13)

Synthesis of O-(3-(benzyloxy)-3-methylbutyl)-N-((benzyloxy)carbonyl)-L-serine (Compound aa59, Cbz-Ser(2-Me-BuOBn)-OH)

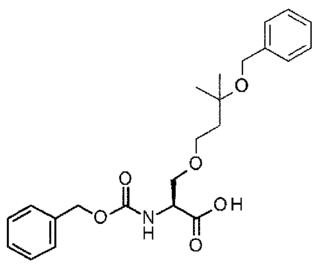

Lithium hydroxide monohydrate (11.2 g) and calcium chloride (110 g) were dissolved in water (278 mL) under a nitrogen atmosphere. A solution of methyl O-(3-(benzyloxy)-3-methylbutyl)-N-((benzyloxy)carbonyl)-L-serinate (Compound aa58, Cbz-Ser(2-Me-BuOBn)-OMe) (28.7 g, 68.4 mmol) in 2-propanol/tetrahydrofuran (278 mL/1115 mL) was added thereto at room temperature, and the mixture was stirred for 5 h. A 2 M aqueous hydrochloric acid solution was added until pH 2, the organic layer was removed, and the aqueous layer was extracted with ethyl acetate three times. The resulting organic layers were combined and dried over anhydrous sodium sulfate, the solvent was evaporated under reduced pressure, and the residue was further dried using a vacuum pump under reduced pressure to afford O-(3-(benzyloxy)-3-methylbutyl)-N-((benzyloxy)carbonyl)-L-serine (Compound aa59, Cbz-Ser(2-Me-BuOBn)-OH) (28.0 g) as a crude product. This was used in the next step without purification.

Synthesis of O-(3-hydroxy-3-methylbutyl)-L-serine (Compound aa60, H-Ser(2-Me-BuOH)-OH)

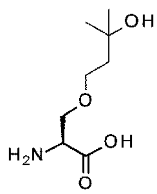

O-(3-(Benzyloxy)-3-methylbutyl)-N-((benzyloxy)carbonyl)-L-serine (Compound aa59, Cbz-Ser(2-Me-BuOBn)-OH) (28.0 g, 67.4 mmol) and palladium on carbon (6.00 g, 20% w/w) were dissolved in methanol (500 mL) under a hydrogen atmosphere, and the reaction solution was stirred at room temperature for 16 h. The reaction solution was filtered, the solvent was then evaporated under reduced pressure, and the residue was further dried using a vacuum pump under reduced pressure, followed by recrystallization from methanol/dichloromethane (1/5) to afford O-(3-hydroxy-3-methylbutyl)-L-serine (Compound aa60, H-Ser(2-Me-BuOH)-OH) (7.00 g, 54%).

Synthesis of N-(((9H-fluoren-9-yl)methoxy)carbonyl)-O-(3-hydroxy-3-methylbutyl)-L-serine (Compound aa61, Fmoc-Ser(2-Me-BuOH)-OH)

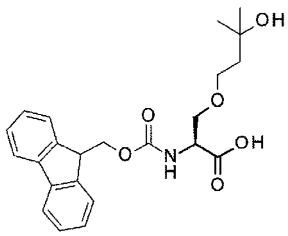

O-(3-Hydroxy-3-methylbutyl)-L-serine (Compound aa60, H-Ser(2-Me-BuOH)-OH) (2.80 g, 14.6 mmol) and sodium carbonate (4.66 g, 44.0 mmol) were dissolved in 1,4-dioxane (24.5 mL)/water (58.8 mL) under a nitrogen atmosphere, after which N-(9-fluorenylmethoxycarbonyloxy)succinimide (Fmoc-OSu) (5.18 g, 15.4 mmol) was added and the mixture was stirred at room temperature for 3 h. After washing the reaction solution with t-butyl methyl ether three times, a 2 M aqueous hydrochloric acid solution was added to the aqueous layer until pH 2, and the aqueous layer was extracted with ethyl acetate three times. The resulting organic layers were combined, washed with brine, and then dried over anhydrous sodium sulfate, the solvent was evaporated under reduced pressure, and the residue was further dried using a vacuum pump under reduced pressure to afford N-(((9H-fluoren-9-yl)methoxy)carbonyl)-O-(3-hydroxy-3-methylbutyl)-L-serine (Compound aa61, Fmoc-Ser(2-Me-BuOH)-OH) (5.80 g) as a crude product. This was used in the next step without purification.

Synthesis of N-(((9H-fluoren-9-yl)methoxy)carbonyl)-O-(3-methyl-3-(tetrahydro-2H-pyran-2-yl)oxy)butyl)-L-serine (Compound aa62, Fmoc-Ser(2-Me-BuOTHP)-OH)

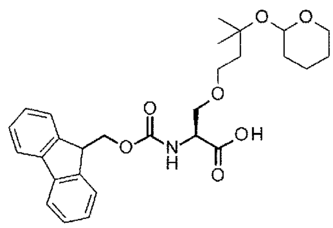

To a solution of N-(((9H-fluoren-9-yl)methoxy)carbonyl)-O-(3-hydroxy-3-methylbutyl)-L-serine (Compound aa61, Fmoc-Ser(2-Me-BuOH)-OH) (6.80 g, 16.5 mmol) in tetrahydrofuran (35.0 mL) were added pyridinium p-toluenesulfonate (0.20 g, 0.82 mmol) and 3,4-dihydro-2H-pyran (10.3 mL) under a nitrogen atmosphere, and the mixture was stirred at 50°C for 5 h. The mixture was cooled to 25°C and ethyl acetate was added. The organic layer was then washed

with brine and dried over anhydrous sodium sulfate, and the solvent was evaporated under reduced pressure. 0.75 g of the resulting residue (11.0 g) was dissolved in tetrahydrofuran (20.0 mL), followed by addition of 1.0 M phosphate buffer adjusted to pH 6.8 (20.0 mL). This mixture was stirred at 50°C for 4 h. After cooling to 25°C, ethyl acetate was added and the organic layer and the aqueous layer were separated. The aqueous layer was extracted with ethyl acetate, after which all the resulting organic layers were combined, washed with brine, and then dried over anhydrous sodium sulfate. The solvent was evaporated under reduced pressure, and the resulting residue was purified by reverse phase column chromatography (0.5% aqueous ammonium bicarbonate solution/acetonitrile) to afford N-(((9H-fluoren-9-yl)methoxy)carbonyl)-O-(3-methyl-3-((tetrahydro-2H-pyran-2-yl)oxy)butyl)-L-serine (Compound aa62, Fmoc-Ser(2-Me-BuOTHP)-OH) (0.60 g, 80%).
LCMS (ESI) m/z = 498 (M+H)+

Retention time: 0.72 min (analytical condition SMD method 11)

Synthesis of (9H-fluoren-9-yl)methyl (S)-4-((3-hydroxy-3-methylbutoxy)methyl)-5-oxooxazolidine-3-carboxylate (Compound aa63)

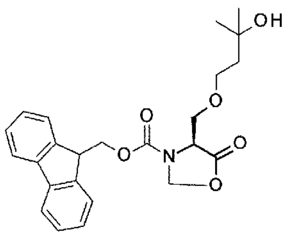

N-(((9H-Fluoren-9-yl)methoxy)carbonyl)-O-(3-hydroxy-3-methylbutyl)-L-serine (Compound aa61, Fmoc-Ser(2-Me-BuOH)-OH) (0.20 g, 0.48 mmol), paraformaldehyde (0.06 g), and trifluoroacetic acid (0.64 g, 5.62 mmol) were dissolved in toluene (10.0 mL), and the reaction solution was stirred at room temperature for 16 h. The solvent was evaporated from the reaction solution under reduced pressure, and the resulting residue was purified by reverse phase column chromatography (water/acetonitrile) to afford (9H-fluoren-9-yl)methyl (S)-4-((3-hydroxy-3-methylbutoxy)methyl)-5-oxooxazolidine-3-carboxylate (Compound aa63) (0.16 g, 80%).
LCMS (ESI) m/z = 448 (M+Na)+

Retention time: 2.47 min (analytical condition SMD method 14)

Synthesis of N-(((9H-fluoren-9-yl)methoxy)carbonyl)-O-(3-hydroxy-3-methylbutyl)-N-methyl-L-serine (Compound aa64, Fmoc-MeSer(2-Me-BuOH)-OH)

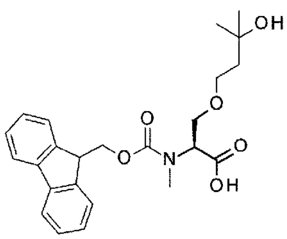

To a solution of (9H-fluoren-9-yl)methyl (S)-4-((3-hydroxy-3-methylbutoxy)methyl)-5-oxooxazolidine-3-carboxylate (Compound aa63) (1.00 g, 2.35 mmol) and aluminum chloride (0.63 g, 4.70 mmol) in dichloromethane (50.0 mL) was slowly added triethylsilane (0.75 mL) dropwise at 0°C under a nitrogen atmosphere, and the mixture was stirred at room temperature for 2 h. The reaction solution was diluted with dichloromethane, washed with a 2 M aqueous hydrochloric acid solution and brine, and the organic layer was then dried over anhydrous sodium sulfate, the solvent was evaporated under reduced pressure, and the resulting residue was purified by reverse phase column chromatography (water/acetonitrile) to afford N-(((9H-fluoren-9-yl)methoxy)carbonyl)-O-(3-hydroxy-3-methylbutyl)-N-methyl-L-serine (Compound

aa64, Fmoc-MeSer(2-Me-BuOH)-OH) (0.30 g, 30%).
LCMS (ESI) m/z = 450 (M+Na)+

Retention time: 2.12 min (analytical condition SMD method 15)

Synthesis of methyl (S)-2-(1,3-dioxoisoindolin-2-yl)-6-oxohexanoate (Compound aa65)

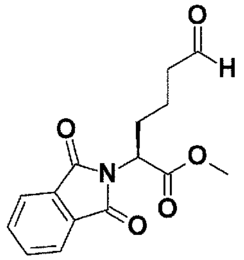

To a solution of commercially available (S)-2-((tert-butoxycarbonyl)amino)-6-hydroxyhexanoic acid (Boc-Nle(6-OH)-OH) (10 g, 40.4 mmol) in toluene/methanol (90 mL/60 mL) was added a 2 M (trimethylsilyl)diazomethane/hexane solution (24.26 mL, 48.5 mmol) dropwise at room temperature under a nitrogen atmosphere, and the mixture was stirred overnight. The reaction solution was concentrated under reduced pressure using a rotary evaporator and the resulting residue was dissolved by adding a toluene/methanol solution (90 mL/60 mL), after which a 2 M (trimethylsilyl)diazomethane/hexane solution (24.26 mL, 48.5 mmol) was added dropwise at room temperature and the mixture was stirred for 6 h. The reaction solution was concentrated under reduced pressure using a rotary evaporator to afford methyl (S)-2-((tert-butoxycarbonyl)amino)-6-hydroxyhexanoate (Boc-Nle(6-OH)-OMe) (13 g) as a crude product.

To the resulting crude product methyl (S)-2-((tert-butoxycarbonyl)amino)-6-hydroxyhexanoate (Boc-Nle(6-OH)-OMe) (13 g) was added a 4N hydrochloric acid/1,4-dioxane solution (50 mL, 200 mmol) at room temperature, and the mixture was stirred for 6 h. The reaction solution was concentrated under reduced pressure using a rotary evaporator to afford hydrochloride of methyl (S)-2-amino-6-hydroxyhexanoate (H-Nle(6-OH)-OMe) (9.2 g) as a crude product.

To a solution of the resulting crude product hydrochloride of methyl (S)-2-amino-6-hydroxyhexanoate (H-Nle(6-OH)-OMe) (9.2 g) in acetonitrile (100 mL) were added ethyl 1,3-dioxoisoindoline-2-carboxylate (9.74 g, 44.4 mmol) and N,N-diisopropylethylamine (DIPEA) (15.52 mL, 89 mmol) at room temperature, and the mixture was stirred overnight. The reaction solution was concentrated under reduced pressure using a rotary evaporator, and the resulting residue was purified by normal phase column chromatography (dichloromethane/methanol) to afford methyl (S)-2-(1,3-dioxoisoindolin-2-yl)-6-hydroxyhexanoate (14.6 g).

To a solution of the above methyl (S)-2-(1,3-dioxoisoindolin-2-yl)-6-hydroxyhexanoate (7.08 g, 24.3 mmol) in dichloromethane (100 mL) was added Dess-Martin periodinane (CAS #87413-09-0, 11.34 g, 26.7 mmol) at 0°C, and the mixture was stirred at room temperature for 3 h. The reaction solution was diluted with dichloromethane and washed with a solution of saturated aqueous sodium bicarbonate solution/water (1/1), an aqueous saturated sodium thiosulfate solution, and brine. The organic layer was dried over anhydrous sodium sulfate, filtered, and then concentrated under reduced pressure. The resulting residue was purified by normal phase column chromatography (hexane/ethyl acetate) to afford methyl (S)-2-(1,3-dioxoisoindolin-2-yl)-6-oxohexanoate (Compound aa65) (3.6 g, 51% over four steps).
LCMS (ESI) m/z = 290 (M+H)+

Retention time: 0.70 min (analytical condition SQDAA05)

Synthesis of methyl (2S)-2-(1,3-dioxoisoindolin-2-yl)-7,7,7-trifluoro-6-hydroxyheptanoate (Compound aa66)

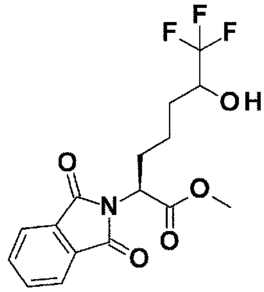

To a solution of methyl (S)-2-(1,3-dioxoisoindolin-2-yl)-6-oxohexanoate (Compound aa65) (3.62 g, 12.51 mmol) in tetrahydrofuran (50 mL) were added (trifluoromethyl)trimethylsilane (1.390 mL, 9.39 mmol) and a 1 M tetrabutylammonium fluoride (TBAF)/tetrahydrofuran (THF) solution (0.626 mL, 0.626 mmol) at 0°C under a nitrogen atmosphere, and the mixture was stirred at 0°C for 10 min. To the reaction solution were added (trifluoromethyl)trimethylsilane (1.390 mL, 9.39 mmol) and a 1 M tetrabutylammonium fluoride (TBAF)/tetrahydrofuran (THF) solution (0.626 mL, 0.626 mmol) at 0°C, and the mixture was stirred for 30 min. To the reaction solution were further added (trifluoromethyl)trimethylsilane (1.390 mL, 9.39 mmol) and a 1 M tetrabutylammonium fluoride (TBAF)/tetrahydrofuran (THF) solution (0.626 mL, 0.626 mmol) at 0°C, and the mixture was stirred for 2 hours and 30 minutes. To the reaction solution was added a IN aqueous hydrochloric acid solution (37.5 mL) at 0°C, and the mixture was stirred at room temperature for 20 min and then extracted with ethyl acetate. The organic layer was washed with brine, dried over anhydrous sodium sulfate, filtered, and concentrated under reduced pressure. The resulting residue was purified by reverse phase column chromatography (10 mM aqueous ammonium acetate solution/methanol) to afford methyl (2S)-2-(1,3-dioxoisoindolin-2-yl)-7,7,7-trifluoro-6-hydroxyheptanoate (Compound aa66) (1.8 g, 40%).
LCMS (ESI) m/z = 358 (M-H)-

Retention time: 0.81 min (analytical condition SQDAA05)

Synthesis of methyl (2S)-2-((((9H-fluoren-9-yl)methoxy)carbonyl)amino)-7,7,7-trifluoro-6-hydroxyheptanoate (Compound aa67, Fmoc-Hnl(7-F3-6-OH)-OMe)

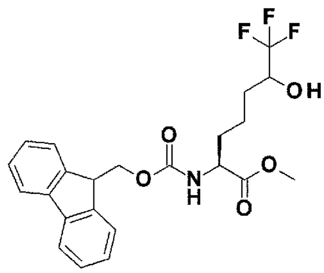

To a solution of methyl (2S)-2-(1,3-dioxoisoindolin-2-yl)-7,7,7-trifluoro-6-hydroxyheptanoate (Compound aa66) obtained by the method described above (3.1 g, 8.63 mmol) in methanol (30 mL) were added hydrazine monohydrate (1.258 mL, 25.9 mmol) and acetic acid (1.482 mL, 25.9 mmol) at room temperature, and the mixture was stirred overnight. The reaction solution was concentrated under reduced pressure using a rotary evaporator to remove the methanol, and the resulting solution was diluted with dimethyl sulfoxide and then purified by reverse phase column chromatography (10 mM aqueous ammonium acetate solution/methanol) to afford methyl (2S)-2-amino-7,7,7-trifluoro-6-hydroxyheptanoate (H-Hnl(7-F3-6-OH)-OMe) (2 g).

To the above methyl (2S)-2-amino-7,7,7-trifluoro-6-hydroxyheptanoate (H-Hnl(7-F3-6-OH)-OMe) (2 g, 8.73 mmol) were added water (25 mL), sodium carbonate (2.93 g, 34.9 mmol), tetrahydrofuran (50 mL), and 9-fluorenylmethyl N-succinimidyl carbonate (Fmoc-OSu) (3.53 g, 10.47 mmol) at room temperature, and the reaction solution was stirred for 2 h. The reaction solution was concentrated under reduced pressure using a rotary evaporator to remove the tetrahydrofuran, ethyl acetate and IN aqueous hydrochloric acid solution were added, and the mixture was extracted with ethyl acetate twice. The organic layers were washed with brine, dried over anhydrous magnesium sulfate, then filtered, and concentrated under reduced pressure. The resulting residue was purified by reverse phase column chromatography (0.1% aqueous formic acid solution/0.1 % formic acid-acetonitrile solution) and normal phase column chromatography

(dichloromethane/methanol) and then further purified by reverse phase column chromatography (10 mM aqueous ammonium acetate solution/methanol). The resulting fractions were collected, the methanol was evaporated under reduced pressure, and the fractions were extracted with ethyl acetate twice and washed with a saturated potassium bisulfate solution and brine. The resulting organic solvent was evaporated under reduced pressure to afford methyl (2S)-2-((((9H-fluoren-9-yl)methoxy)carbonyl)amino)-7,7,7 -trifluoro-6-hydroxyheptanoate (Compound aa67, Fmoc-Hnl(7-F3-6-OH)-OMe) (1.4 g, 36% over two steps).
LCMS (ESI) m/z = 452 (M+H)+

Retention time: 0.86 min (analytical condition SQDFA05)

Synthesis of (2S)-2-((((9H-fluoren-9-yl)methoxy)carbonyl)amino)-7,7,7-trifluoro-6-hydroxyheptanoic acid (Compound aa68, Fmoc-Hnl(7-F3-6-OH)-OH)

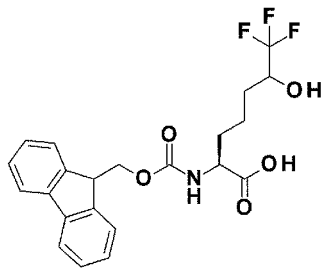

To a solution of calcium chloride (5.16 g, 46.5 mmol) in water (7.00 mL) was added lithium hydroxide monohydrate (0.521 g, 12.40 mmol) at room temperature, and the mixture was stirred for 5 min. A solution of methyl (2S)-2-((((9H-fluoren-9-yl)methoxy)carbonyl)amino)-7,7,7-trifluoro-6-hydroxyheptanoate (Compound aa67, Fmoc-Hnl(7-F3-6-OH)-OMe) (1.4 g, 3.10 mmol) in isopropanol/tetrahydrofuran (28 mL/7 mL) was added dropwise at room temperature, and the reaction solution was stirred overnight. To the reaction solution was added a 1N aqueous hydrochloric acid solution, and the reaction solution was extracted with t-butyl methyl ether twice, washed with brine, dried over anhydrous magnesium sulfate, then filtered, and concentrated under reduced pressure. The resulting residue was purified by reverse phase column chromatography (0.1% aqueous formic acid solution/0.1 % formic acid-acetonitrile solution) to afford (2S)-2-((((9H-fluoren-9-yl)methoxy)carbonyl)amino)-7,7,7-trifluoro-6-hydroxyheptanoic acid (Compound aa68, Fmoc-Hnl(7-F3-6-OH)-OH) (950 mg, 70%).
LCMS (ESI) m/z = 438.6 (M+H)+

Retention time: 0.76 min (analytical condition SQDFA05)

Synthesis of methyl N-(((9H-fluoren-9-yl)methoxy)carbonyl)-O-(3,3,3-trifluoro-2-hydroxypropyl)-L-serinate (Compound aa69, Fmoc-Ser(1-CF3-EtOH)-OMe)

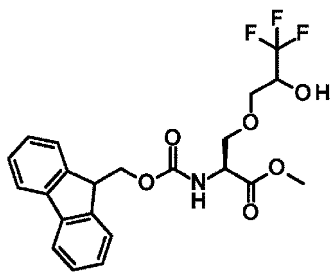

To a solution of separately synthesized 1-((9H-fluoren-9-yl)methyl) 2-methyl (S)-aziridine-1,2-dicarboxylate (Compound aa73, Fmoc-Azy-OMe) (0.20 g, 0.62 mmol) and 3,3,3-trifluoropropane-1,2-diol (0.24 g, 1.9 mmol) in dichloromethane (3.0 mL) was added boron trifluoride-diethyl ether complex ($BF_3 \cdot OEt_2$) (7.8 μL, 0.062 mmol) at 0°C under a nitrogen atmosphere, and the mixture was stirred for 30 min. Moreover, to a solution of 1-((9H-fluoren-9-yl)methyl) 2-methyl (S)-aziridine-1,2-dicarboxylate (Compound aa73, Fmoc-Azy-OMe) (5.0 g, 15.46 mmol) and 3,3,3-trifluoropropane-1,2-diol (6.03 g, 46.4 mmol) in dichloromethane (77.0 mL) was added boron trifluoride-diethyl ether complex ($BF_3{\cdot}OEt_2$) (0.19 mL, 1.55 mmol) at 0°C under a nitrogen atmosphere, and the mixture was stirred for 30 min. Furthermore, to a solution of 1-((9H-fluoren-9-yl)methyl) 2-methyl (S)-aziridine-1,2-dicarboxylate (Compound aa73, Fmoc-Azy-OMe) (3.6 g, 11.13 mmol) and 3,3,3-trifluoropropane-1,2-diol (4.34 g, 33.4 mmol) in dichloromethane (55.7 mL) was added boron trifluoride-diethyl ether complex ($BF_3{\cdot}OEt_2$) (0.14 mL, 1.11 mmol) at 0°C under a nitrogen atmosphere, and the mixture was stirred for 30 min.

A saturated aqueous sodium bicarbonate solution was added to each of the above three reaction solutions, after which all the reaction solutions were combined, most of the organic solvent was evaporated under reduced pressure, and the remaining solution was extracted by adding ethyl acetate (300 mL). The organic layer was washed with a brine solution, and the organic solvent was then evaporated under reduced pressure. The resulting residue was purified by normal phase silica gel column chromatography (hexane/ethyl acetate) to afford methyl N-(((9H-fluoren-9-yl)methoxy)carbonyl)-O-(3,3,3-trifluoro-2-hydroxypropyl)-L-serinate (Compound aa69, Fmoc-Ser(1-CF3-EtOH)-OMe) (6.57 g, 53%, purity: 95%) and the same compound (3.1 g, 25%, purity: 87%).
LCMS (ESI) m/z = 454 (M+H)+

Retention time: 1.18 min (analytical condition SMD method 45)

Synthesis of N-(((9H-fluoren-9-yl)methoxy)carbonyl)-O-(3,3,3-trifluoro-2-hydroxypropyl)-L-serine (Compound aa70, Fmoc-Ser(1-CF3-EtOH)-OH)

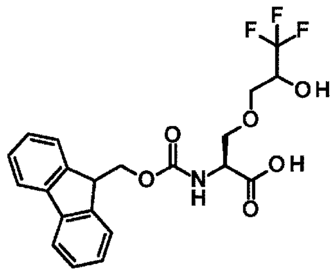

Calcium chloride (2.06 g, 18.5 mmol) was dissolved in water (5.2 mL), lithium hydroxide monohydrate (207 mg, 4.94 mmol) was added, and the mixture was stirred at room temperature for 10 min. To the solution was added a solution of methyl N-(((9H-fluoren-9-yl)methoxy)carbonyl)-O-(3,3,3-trifluoro-2-hydroxypropyl)-L-serinate (Compound aa69, Fmoc-Ser(1-CF3-EtOH)-OMe) (560 mg, 1.24 mmol) in tetrahydrofuran (THF) (5.15 mL) and isopropanol (20.6 mL), and the mixture was stirred at room temperature for 2 h. The reaction was completed by adding a IN aqueous hydrochloric acid solution. Further, the same reaction was performed using 0.15 g, 1.11 g, 0.48 g, and 0.96 g of Fmoc-Ser(1-CF3-EtOH)-OMe, respectively. All the reaction solutions were collected and most of the solvent was evaporated under reduced pressure. The resulting solution was extracted by adding ethyl acetate and water. The solvent was evaporated from the organic layer under reduced pressure, and the resulting residue was then purified by reverse phase column chromatography (0.1% aqueous formic acid solution/0.1% formic acid-acetonitrile solution) to afford N-(((9H-fluoren-9-yl)methoxy)carbonyl)-O-(3,3,3-trifluoro-2-hydroxypropyl)-L-serine (Compound aa70, Fmoc-Ser(1-CF3-EtOH)-OH) (3.10 g, 98%).
LCMS (ESI) m/z = 462 (M+Na)+

Retention time: 2.24 min (analytical condition SMD method 46)

Synthesis of N-(((9H-fluoren-9-yl)methoxy)carbonyl)-N-methyl-O-(3,3,3-trifluoro-2-hydroxypropyl)-L-serine (Compound aa71, Fmoc-MeSer(1-CF3-EtOH)-OH)

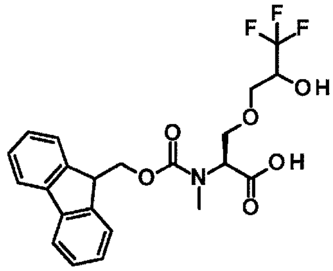

To a solution of N-(((9H-fluoren-9-yl)methoxy)carbonyl)-O-(3,3,3-trifluoro-2-hydroxypropyl)-L-serine (Compound aa70, Fmoc-Ser(1-CF3-EtOH)-OH) (1.84 g, 4.19 mmol) in dichloromethane (20.9 mL) were added paraformaldehyde (151 mg, 5.03 mmol), anhydrous magnesium sulfate (1.26 g, 10.5 mmol), and boron trifluoride-diethyl ether complex ($BF_3 \cdot OEt_2$) (526 μL, 4.19 mmol) under a nitrogen atmosphere, and the mixture was stirred at room temperature for 3 h. The reaction solution was filtered to remove the insoluble matter, and was then washed with dichloromethane (10 mL).

To the filtered reaction solution were added triethylsilane (2.0 mL, 12.6 mmol) and boron trifluoride-diethyl ether complex ($BF_3 \cdot OEt_2$) (1.58 mL, 12.6 mmol), and the mixture was stirred at room temperature for 30 min. The reaction solution was diluted with brine and extracted with dichloromethane. The resulting organic layer was concentrated under reduced pressure, and the resulting residue was then purified by reverse phase column chromatography (0.1% aqueous formic acid solution/0.1 % formic acid-acetonitrile solution) to afford N-(((9H-fluoren-9-yl)methoxy)carbonyl)-N-methyl-O-(3,3,3-trifluoro-2-hydroxypropyl)-L-serine (Compound aa71, Fmoc-MeSer(1-CF3-EtOH)-OH) (640 mg, 34%).
LCMS (ESI) m/z = 452 (M-H)-

Retention time: 2.33 min (analytical condition SMD method 47)

Synthesis of N-(((9H-fluoren-9-yl)methoxy)carbonyl)-O-(3,3,3-trifluoro-2-((tetrahydro-2H-pyran-2-yl)oxy)propyl)-L-serine (Compound aa72, Fmoc-Ser(1-CF3-EtOTHP)-OH)

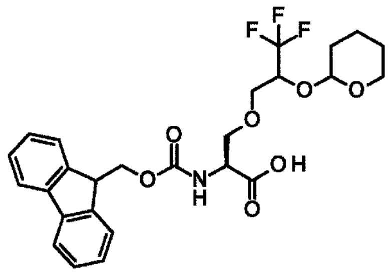

To a solution of N-(((9H-fluoren-9-yl)methoxy)carbonyl)-O-(3,3,3-trifluoro-2-hydroxypropyl)-L-serine (Compound aa70, Fmoc-Ser(1-CF3-EtOH)-OH) (2.52 g, 5.74 mmol) in dichloromethane (19.12 mL) were added 3,4-dihydro-2H-pyran (3.92 mL, 43.0 mmol) and pyridinium p-toluenesulfonate (0.144 g, 0.574 mmol), and the mixture was stirred at 40°C overnight. Water was then added to the reaction solution, the mixture was extracted with dichloromethane, and the organic layer was washed with brine. The organic layer was dried over anhydrous sodium sulfate and then filtered, and the solvent was removed by concentration under reduced pressure. The resulting residue was dissolved in tetrahydrofuran (29 mL), a 1 M aqueous phosphoric acid solution (pH = 8, 29 mL) was added, and the mixture was stirred at 50°C for 3 h. The reaction solution was diluted with water and extracted with ethyl acetate twice. The organic layers were combined, washed with brine, dried over anhydrous sodium sulfate, and then filtered, and the solvent was removed by concentration under reduced pressure. The resulting residue was dissolved in dichloromethane (60 mL) and heptane (60 mL), and the dichloromethane was removed by concentration under reduced pressure to precipitate an oily crude product. The heptane solution was removed by decantation. This operation was repeated twice. The resulting crude product was dissolved in ethyl acetate and washed with a 0.05 M aqueous phosphoric acid solution twice and with brine once. The organic layer was dried over anhydrous sodium sulfate, filtered, and then concentrated under reduced pressure to afford N-(((9H-fluoren-9-yl)methoxy)carbonyl)-O-(3,3,3-trifluoro-2-((tetrahydro-2H-pyran-2-yl)oxy)propyl)-L-serine (Compound aa72, Fmoc-Ser(1-CF3-EtOTHP)-OH) (2.89 g, 96%).
LCMS (ESI) m/z = 522 (M-H)-

Retention time: 1.00 min (analytical condition SQDAA05-2)

Synthesis of 1-((9H-fluoren-9-yl)methyl) 2-methyl (S)-aziridine-1,2-dicarboxylate (Compound aa73, Fmoc-Azy-OMe)

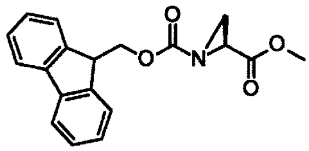

To a solution of commercially available methyl (S)-1-tritylaziridine-2-carboxylate (Trt-Azy-OMe) (50 g, 145.60 mmol) in chloroform/methanol (145 mL/145 mL) was added trifluoroacetic acid (33 mL) dropwise at 0°C under a nitrogen atmosphere, and the reaction solution was stirred for 7 h. To the reaction solution was added a solution of N,N-diisopropylethylamine (DIPEA) (127 mL) and (9H-fluoren-9-yl)methyl carbonochloridate (Fmoc-Cl) (36 g, 139.16 mmol) in 1,4-dioxane (145 mL) dropwise at 0°C, and the reaction solution was stirred at 0°C for 1 hour and 30 minutes. The solvent was evaporated from the reaction solution under reduced pressure, and the reaction solution was diluted with ethyl acetate and then washed with water, with a saturated aqueous ammonium chloride solution twice, and with brine twice. The resulting organic layer was dried over anhydrous sodium sulfate and filtered, and then the solvent was evaporated under reduced pressure. The resulting residue was purified by normal phase column chromatography (petroleum ether/ethyl acetate) to afford 1-((9H-fluoren-9-yl)methyl) 2-methyl (S)-aziridine-1,2-dicarboxylate (Compound aa73, Fmoc-Azy-OMe) (40 g, 85% over two steps).
LCMS (ESI) m/z = 324 (M+H)+

Retention time: 0.86 min (analytical condition SQDFA05)

Synthesis of tert-butyl N2-(((9H-fluoren-9-yl)methoxy)carbonyl)-N5-methyl-L-glutaminate (Compound aa74, Fmoc-Gln(Me)-OtBu)

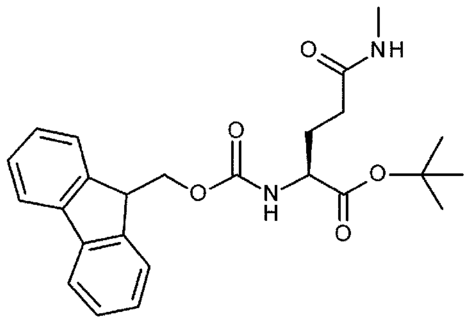

To a solution of (S)-4-((((9H-fluoren-9-yl)methoxy)carbonyl)amino)-5-(tert-butoxy)-5-oxopentanoic acid (Fmoc-Glu-OtBu) (20.0 g, 47.0 mmol) and 1-hydroxy-7-azabenzotriazole (HOAt) (8.8 g) in dimethylformamide (DMF) (300 mL) was added 1-(3-dimethylaminopropyl)-3-ethylcarbodiimide hydrochloride (WSC·HCl) (13.5 g) under ice-cooling, and the mixture was stirred for 10 min. A 2 mol/L solution of methylamine in tetrahydrofuran (29.5 mL) was added while maintaining the temperature of the reaction solution at 5°C or lower, and the mixture was stirred at room temperature for 16 h. The reaction solution was diluted with hexane/ethyl acetate (1/1, 1400 mL) and an aqueous ammonium chloride solution (500 mL), and the organic layer was separated. The organic layer was washed with an aqueous sodium bicarbonate solution and brine and dried over anhydrous sodium sulfate, and the solvent was then evaporated under reduced pressure to afford tert-butyl N2-(((9H-fluoren-9-yl)methoxy)carbonyl)-N5-methyl-L-glutaminate (Compound aa74, Fmoc-Gln(Me)-OtBu) (36.2 g) as a crude product.
LCMS (ESI) m/z = 439 (M+H)+

Retention time: 1.05 min (analytical condition SMD method 23)

Synthesis of N2-(((9H-fluoren-9-yl)methoxy)carbonyl)-N5-methyl-L-glutamine (Compound aa75, Fmoc-Gln(Me)-OH)

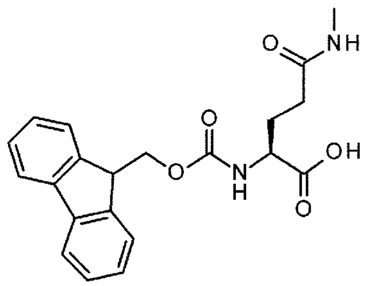

To a solution of tert-butyl N2-(((9H-fluoren-9-yl)methoxy)carbonyl)-N5-methyl-L-glutaminate (Compound aa74, Fmoc-Gln(Me)-OtBu) (43.6 g) in dichloromethane (DCM) (300 mL) was added trifluoroacetic acid (TFA) (300 mL) dropwise under ice-cooling, and the mixture was stirred at room temperature for 16 h. The solvent was evaporated under reduced pressure, diethyl ether and aqueous sodium bicarbonate solution were added to the resulting residue, and the organic phase was separated. The resulting aqueous layer was adjusted to pH 1-2 with a 5 mol/L aqueous hydrochloric acid solution, and the precipitated solid was collected by filtration to afford N2-(((9H-fluoren-9-yl)methoxy)carbonyl)-N5-methyl-L-glutamine (Compound aa75, Fmoc-Gln(Me)-OH) (26.7 g).
LCMS (ESI) m/z = 383 (M+H)+

Retention time: 1.75 min (analytical condition SMD method 5)

Synthesis of 2-bromo-N-tert-butylacetamide (Compound aa76)

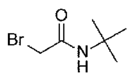

To a solution of 2-bromoacetic acid (50.0 g, 360 mmol) in N,N-dimethylformamide (80.0 mL) were added 2-methylpropan-2-amine (26.7 g, 365 mmol), N-ethyl-isopropylpropan-2-amine (DIPEA) (139 g, 1.08 mol), and propylphosphonic acid anhydride (T3P) (229 g, 720 mmol) under a nitrogen atmosphere, and the mixture was stirred at room temperature for 2 h. Water was added to the reaction solution, and the mixture was extracted with ethyl acetate twice. The resulting organic layers were combined, washed with brine, and then dried over anhydrous sodium sulfate. The solvent was evaporated under reduced pressure, and the resulting residue was washed with hexane to afford 2-bromo-N-tert-butylacetamide (Compound aa76, 36.5 g) as a crude product. This was used in the next step without purification.

Synthesis of O-(2-(tert-butylamino)-2-oxoethyl)-N-trityl-L-serine (Compound aa77, Trt-Ser(NtBu-Aca)-OH)

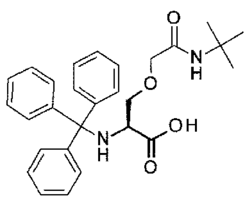

To a solution of trityl-L-serine (Trt-Ser-OH) triethylamine salt (1.50 g, 4.32 mmol) in dimethylformamide (DMF) (2.00 mL) was added sodium hydride (0.52 g, 13.0 mmol, 60% oil dispersion) under a nitrogen atmosphere, and the

mixture was stirred at room temperature for 2 h. A solution of 2-bromo-N-tert-butylacetamide (Compound aa76) (0.96 g) in DMF (1.00 mL) was added thereto, and the mixture was stirred at room temperature for 1 h. Water was added to the reaction solution, and the mixture was extracted with ethyl acetate twice. The resulting organic layers were combined, washed with brine, and then dried over anhydrous sodium sulfate. The solvent was evaporated under reduced pressure, and the residue was further dried using a vacuum pump under reduced pressure to afford O-(2-(tert-butylamino)-2-oxoethyl)-N-trityl-L-serine (Compound aa77, Trt-Ser(NtBu-Aca)-OH) (1.60 g) as a crude product. This was used in the next step without purification.

Synthesis of N-(((9H-fluoren-9-yl)methoxy)carbonyl)-O-(2-(tert-butylamino)-2-oxoethyl)-L-serine (Compound aa78, Fmoc-Ser(NtBu-Aca)-OH)

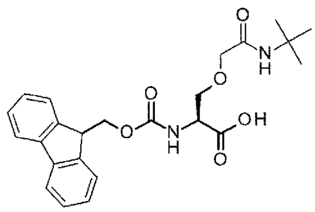

To a solution of O-(2-(tert-butylamino)-2-oxoethyl)-N-trityl-L-serine (Compound aa77, Trt-Ser(NtBu-Aca)-OH) (15.3 g, 33.2 mmol) in dichloromethane (150 mL)/water (150 mL) was added trifluoroacetic acid (11.2 g, 99.1 mmol) at 4°C under a nitrogen atmosphere, and the mixture was stirred at the same temperature for 2 h. The aqueous layer was separated and N-ethyl-isopropylpropan-2-amine (DIPEA) was added until pH 7. After adding 1,4-dioxane (150 mL) thereto, N-ethyl-isopropylpropan-2-amine (DIPEA) (14.9 g, 115 mmol) and N-(9-fluorenylmethoxycarbonyloxy)succinimide (Fmoc-OSu) (22.3 g, 231 mmol) were added and the mixture was stirred at 25°C for 16 h. Water was added to the reaction solution, and the mixture was washed with hexane twice. Concentrated hydrochloric acid was then added to the aqueous layer until pH 2, and the aqueous layer was extracted with ethyl acetate twice. The resulting organic layers were combined, washed with brine, and then dried over anhydrous sodium sulfate. The solvent was evaporated under reduced pressure, and the resulting residue was purified by reverse phase column chromatography (0.1% aqueous formic acid solution/0.1% formic acid-acetonitrile solution) to afford N-(((9H-fluoren-9-yl)methoxy)carbonyl)-O-(2-(tert-butylamino)-2-oxoethyl)-L-serine (Compound aa78, Fmoc-Ser(NtBu-Aca)-OH) (14.2 g, 98%).
LCMS (ESI) m/z = 441 (M+H)+

Retention time: 1.08 min (analytical condition SMD method 9)

Synthesis of N-(((9H-fluoren-9-yl)methoxy)carbonyl)-O-(2-(tert-butylamino)-2-oxoethyl)-N-methyl-L-serine (Compound aa79, Fmoc-MeSer(NtBu-Aca)-OH)

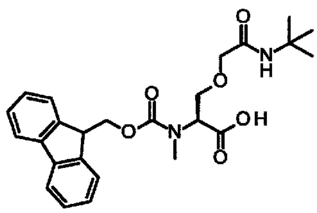

A solution of N-(((9H-fluoren-9-yl)methoxy)carbonyl)-O-(2-(tert-butylamino)-2-oxoethyl)-L-serine (Compound aa78, Fmoc-Ser(NtBu-Aca)-OH) (1.0 g, 2.270 mmol), paraformaldehyde (203 mg, 6.81 mmol), and ((1S,4R)-7,7-dimethyl-2-oxobicyclo[2.2.1]heptan-1-yl)methanesulfonic acid (26 mg, 0.114 mmol) in toluene (1.2 mL) was stirred at 80°C for 1 h under a nitrogen atmosphere. A saturated aqueous sodium bicarbonate solution was added to the reaction solution, and the mixture was extracted with ethyl acetate three times and washed with brine. The resulting organic layers were dried over anhydrous sodium sulfate, then filtered, and concentrated under reduced pressure to afford (9H-fluoren-9-yl)methyl (S)-4-((2-(tert-butylamino)-2-oxoethoxy)methyl)-5-oxooxazolidine-3-carboxylate (1.035 g) as a crude product.

To a solution of the above crude product (9H-fluoren-9-yl)methyl (S)-4-((2-(tert-butylamino)-2-oxoethoxy)methyl)-5-oxooxazolidine-3-carboxylate (1.035 g) in dichloromethane (DCM) (2.86 mL) were added triethylsilane ($Et_3SiH$) (1.096 mL, 6.86 mmol) and water (40 $\mu$L) under a nitrogen atmosphere, and the mixture was stirred at room temperature for 2 min, after which boron trifluoride-diethyl ether complex ($BF_3 \cdot OEt_2$) (0.580 mL, 4.57 mmol) was added and the mixture was stirred at room temperature for 24 h. A saturated aqueous ammonium chloride solution was added to the reaction solution, and the mixture was stirred for 40 min at room temperature and then extracted with dichloromethane (DCM) twice. The resulting organic layers were dried over anhydrous sodium sulfate and concentrated under reduced pressure, acetonitrile was added to the resulting residue, and the mixture was washed with hexane. The acetonitrile was evaporated under reduced pressure to afford N-(((9H-fluoren-9-yl)methoxy)carbonyl)-O-(2-(tert-butylamino)-2-oxoethyl)-N-methyl-L-serine (Compound aa79, Fmoc-MeSer(NtBu-Aca)-OH) (0.9449 g, 92% over two steps).
LCMS (ESI) m/z = 455 (M+H)+

Retention time: 0.79 min (analytical condition SQDFA05)

Synthesis of 2-bromo-N-methylacetamide (Compound aa80)

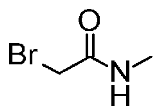

Under a nitrogen atmosphere, dichloromethane (100 mL) was added to potassium carbonate (11.61 g, 84 mmol), and then methylamine hydrochloride (2.84 g, 42.0 mmol) was added at room temperature. 2-Bromoacetyl bromide (3.47 mL, 40 mmol) was added dropwise to the reaction solution at 0°C, and the mixture was stirred at room temperature for 1 h. Water (20 mL) was added to the reaction solution, the mixture was extracted with dichloromethane, then concentrated under reduced pressure, and dried with an oil pump to afford 2-bromo-N-methylacetamide (Compound aa80) (5.2 g) as a crude product. This was used in the next step without purification.

Synthesis of N-(((9H-fluoren-9-yl)methoxy)carbonyl)-O-(2-(methylamino)-2-oxoethyl)-L-serine (Compound aa81, Fmoc-Ser(NMe-Aca)-OH)

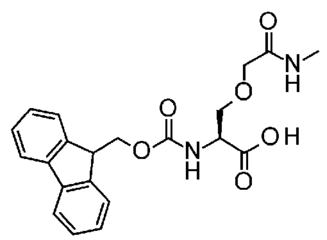

Under a nitrogen atmosphere, a 1.9 M solution of sodium bis(trimethylsilyl)amide (NaHMDS) in tetrahydrofuran (THF) (4.86 mL, 9.23 mmol) was added at 0°C to a solution of a triethylamine salt of trityl-L-serine (Trt-Ser-OH) (1.80 g) in THF (2.00 mL), and the mixture was stirred for 15 min. A solution of 2-bromo-N-methylacetamide (Compound aa80) (1.22 g) in THF (2.00 mL) was added at 0°C to the reaction solution, and the mixture was stirred at room temperature for 15 min. The reaction solution was added to a mixed solution of ethyl acetate (300 mL), water (60 mL), and a IN aqueous hydrochloric acid solution (4.8 mL), and the resulting organic layer was evaporated under reduced pressure to afford a crude product. Furthermore, under a nitrogen atmosphere, a 1.9 M solution of sodium bis(trimethylsilyl)amide (NaHMDS) in tetrahydrofuran (THF) (4.65 mL, 8.83 mmol) was added at 0°C to a solution of a triethylamine salt of trityl-L-serine (Trt-Ser-OH) (1.80 g) in THF (2.00 mL), and the mixture was stirred for 15 min. A solution of 2-bromo-N-methylacetamide (Compound aa80) (1.22 g) in THF (2.00 mL) was added at 0°C to the reaction solution, and the mixture was stirred at room temperature for 15 min. The reaction solution was added to a mixed solution of ethyl acetate (300 mL), water (60 mL), and a 1N aqueous hydrochloric acid solution (4.8 mL), the resulting organic layer was evaporated under reduced pressure to afford a crude product. The above two crude products were combined and purified by reverse phase column chromatography (0.1% aqueous formic acid solution/0.1% formic acid acetonitrile solution) to afford

O-(2-(methylamino)-2-oxoethyl)-N-trityl-L-serine (Trt-Ser(NMe-Aca)-OH) (2.5 g).

Trifluoroacetic acid (TFA) (1.325 mL, 17.20 mmol) was added at 0°C to a solution of the resulting O-(2-(methylamino)-2-oxoethyl)-N-trityl-L-serine (Trt-Ser(NMe-Aca)-OH) (2.4 g, 5.73 mmol) in dichloromethane (DCM) (2.87 mL), and the mixture was stirred for 30 min. Water (2.87 mL) was added to the reaction solution, and the resulting aqueous layer was purified by reverse phase column chromatography (water/acetonitrile) to afford O-(2-(methylamino)-2-oxoethyl)-L-serine (H-Ser(NtBu-Aca)-OH) (900 mg) as a crude product. This was used in the next step without purification.

Water (4.2 mL) and a solution of N-ethyl-N-isopropylpropan-2-amine (DIPEA) (2.231 mL, 12.77 mmol) in 1,4-dioxane (14.0 mL) were added at room temperature to the resulting crude product O-(2-(methylamino)-2-oxoethyl)-L-serine (H-Ser(NtBu-Aca)-OH) (900 mg), N-(9-fluorenylmethoxycarbonyloxy)succinimide (Fmoc-OSu) (1.723 g, 5.11 mmol) was then added at room temperature, and the mixture was stirred for 30 min. The reaction solution was added to a mixed solution of ethyl acetate (150 mL), water (30 mL), and a 1N aqueous hydrochloric acid solution (3.0 mL), and the resulting organic layer was evaporated under reduced pressure. The resulting residue was purified by reverse phase column chromatography (water/acetonitrile) to afford N-(((9H-fluoren-9-yl)methoxy)carbonyl)-O-(2-(methylamino)-2-oxoethyl)-L-serine (Compound aa81, Fmoc-Ser(NMe-Aca)-OH) (1.8 g).
LCMS(ESI) m/z = 399 (M+H)+

Retention time: 0.63 min (Analytical condition SQDFA05)

Synthesis of methyl N-((benzyloxy)carbonyl-O-(3-hydroxypropyl)-L-serinate (Compound aa82, Cbz-Ser(nPrOH)-OMe)

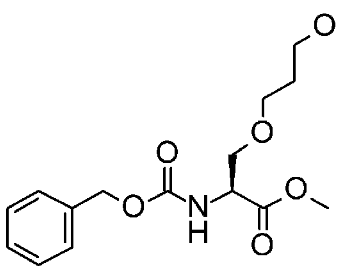

Under a nitrogen atmosphere, commercially available 1-benzyl 2-methyl (S)-aziridine-1,2-dicarboxylate (Cbz-Azy-OMe) (500 mg, 2.125 mmol) and propan-1,3-diol (810 mg, 10.645 mmol) were dissolved in dichloromethane (10 mL), the mixture was cooled to 0°C, a boron trifluoride-diethyl ether complex ($BF_3 \cdot Et_2O$) (46 mg, 0.324 mmol) was then added, and the mixture was stirred at 0°C for 4 h. Dichloromethane was added to the reaction solution, and the mixture was washed with a saturated aqueous sodium hydrogen carbonate solution and with brine. The organic layer was dried over anhydrous sodium sulfate, the solvent was evaporated under reduced pressure, and the resulting residue was purified by reverse phase column chromatography (water/acetonitrile) to afford methyl N-((benzyloxy)carbonyl)-O-(3-hydroxypropyl)-L-serinate (Compound aa82, Cbz-Ser(nPrOH)-OMe) (390 mg, 59%).
LCMS (ESI) m/z = 334 (M+Na)+

Retention time: 1.09 min (Analytical condition SMD method 13)

Synthesis of N-((benzyloxy)carbonyl)-O-(3-hydroxypropyl)-L-serine (Compound aa83, Cbz-Ser(nPrOH)-OH)

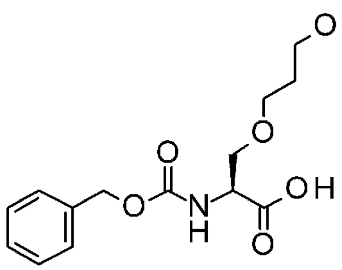

Under a nitrogen atmosphere, water (134 mL) and 2-propanol (538 mL) were added at room temperature to lithium hydroxide (3.14 g, 131.049 mmol) and calcium chloride (54.54 g, 491.434 mmol), the mixture was stirred, a solution of methyl N-((benzyloxy)carbonyl)-O-(3-hydroxypropyl)-L-serinate (Compound aa82, Cbz-Ser(nPrOH)-OMe) (10.2 g, 32.762 mmol) in tetrahydrofuran (134 mL) was then added at room temperature, and the mixture was stirred for 5 h. A 6N aqueous hydrochloric acid solution was added to the reaction solution until pH 3, the organic solvent was removed under reduced pressure, and then the aqueous layer was extracted with ethyl acetate. The resulting organic layer was washed with brine and dried over anhydrous sodium sulfate, the solvent was evaporated under reduced pressure, and the residue was further dried under reduced pressure using a vacuum pump to afford N-((benzyloxy)carbonyl)-O-(3-hydroxypropyl)-L-serine (Compound aa83, Cbz-Ser(nPrOH)-OH) (10.6 g) as a crude product. This was used in the next step without purification.

Synthesis of N-(((9H-fluoren-9-yl)methoxy)carbonyl)-O-(3-hydroxypropyl)-L-serine (Compound aa84, Fmoc-Ser(nPrOH)-OH)

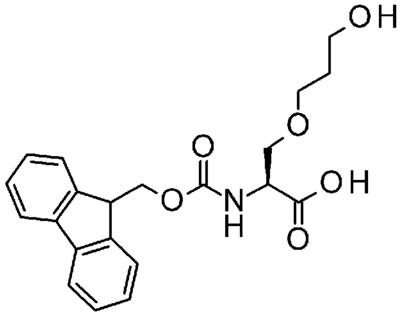

Under a hydrogen atmosphere (3 atm or less), a mixture of the crude product N-((benzyloxy)carbonyl)-O-(3-hydroxypropyl)-L-serine (Compound aa83, Cbz-Ser(nPrOH)-OH) (13 g) obtained as described above, 10% palladium on carbon (4.65 g), and methanol (130 mL) was stirred at room temperature for 16 h. After the reaction solution was filtered, the solvent was evaporated under reduced pressure, and the resulting residue was recrystallized from ethyl acetate to afford O-(3-hydroxypropyl)-L-serine (H-Ser(nPrOH)-OH) (5.5 g, 78% over two steps).

Under a nitrogen atmosphere, N-(9-fluorenylmethoxycarbonyloxy)succinimide (Fmoc-OSu) (11.94 g, 35.396 mmol) was added at room temperature to a solution of O-(3-hydroxypropyl)-L-serine (H-Ser(nPrOH)-OH) (5.5 g, 33.707 mmol) and sodium hydrogen carbonate (8.6 g, 102 mmol) in water/1,4-dioxane (50 mL/130 mL), and the mixture was stirred for 4 h. Water was added to the reaction solution, the mixture was washed with t-butyl methyl ether twice, a 6N aqueous hydrochloric acid solution was then added until the pH of the aqueous layer was 3, and the mixture was extracted with t-butyl methyl ether. After the resulting organic layer was dried over anhydrous sodium sulfate and filtered, the solvent was evaporated under reduced pressure, and the organic layer was further dried under reduced pressure using a vacuum pump. The resulting residue was purified by reverse phase column chromatography (water/acetonitrile) to afford N-(((9H-fluoren-9-yl)methoxy)carbonyl)-O-(3-hydroxypropyl)-L-serine (Compound aa84, Fmoc-Ser(nPrOH)-OH) (8 g, 67%).
LCMS (ESI) m/z = 386 (M+H)+

Retention time: 0.61 min (Analytical condition SMD method 50)

Synthesis of N-(((9H-fluoren-9-yl)methoxy)carbonyl)-O-(3-((tetrahydro-2H-pyran-2-yl)oxy)propyl)-L-serine (Compound aa85, Fmoc-Ser(nPrOTHP)-OH)

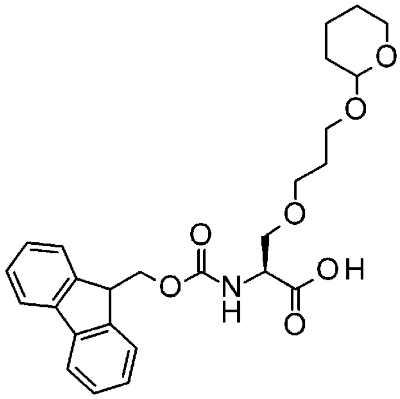

Under a nitrogen atmosphere, pyridinium p-toluenesulfonate (PPTS) (32.6 mg, 0.130 mmol) was added to a solution of N-(((9H-fluoren-9-yl)methoxy)carbonyl)-O-(3-hydroxypropyl)-L-serine (Compound aa84, Fmoc-Ser(nPrOH)-OH) (1 g, 2.595 mmol) and 3,4-dihydro-2H-pyran (1.5 g, 17.83 mmol) in tetrahydrofuran (5 mL), and the mixture was stirred at 50°C for 4 h. The reaction solution was cooled, t-butyl methyl ether was added, the mixture was washed with brine, and the organic layer was then dried over anhydrous sodium sulfate and filtered. The solvent was evaporated under reduced pressure to afford N-(((9H-fluoren-9-yl)methoxy)carbonyl)-O-(3-((tetrahydro-2H-pyran-2-yl)oxy)propyl)-L-serine (Compound aa85, Fmoc-Ser(nPrOTHP)-OH) and tetrahydro-2H-pyran-2-yl N-(((9H-fluoren-9-yl)methoxy)carbonyl)-O-(3-((tetrahydro-2H-pyran-2-yl)oxy)propyl)-L-serinate (Fmoc-Ser(nPrOTHP)-OTHP) (1.5 g) as a mixture.

Tetrahydrofuran (8 mL) and a 1 M phosphate buffer (8 mL, pH 8.0) were added to the mixture of N-(((9H-fluoren-9-yl)methoxy)carbonyl)-O-(3-((tetrahydro-2H-pyran-2-yl)oxy)propyl)-L-serine (Compound aa85, Fmoc-Ser(nPrOTHP)-OH) and tetrahydro-2H-pyran-2-yl N-(((9H-fluoren-9-yl)methoxy)carbonyl)-O-(3-((tetrahydro-2H-pyran-2-yl)oxy)propyl)-L-serinate (Fmoc-Ser(nPrOTHP)-OTHP) (1.5 g) obtained as described above, and the mixture was stirred at 50°C for 3 h. The reaction solution was cooled, t-butyl methyl ether was added, the mixture was washed with brine, dried over anhydrous sodium sulfate, and filtered, and then the solvent was evaporated under reduced pressure. The resulting residue was recrystallized from diethyl ether/heptane to afford N-(((9H-fluoren-9-yl)methoxy)carbonyl)-O-(3-((tetrahydro-2H-pyran-2-yl)oxy)propyl)-L-serine (Compound aa85, Fmoc-Ser(nPrOTHP)-OH) as a sodium salt (1 g).

t-Butyl methyl ether (100 mL) and a 0.05 M aqueous phosphoric acid solution (250 mL) were added to the sodium salt of N-(((9H-fluoren-9-yl)methoxy)carbonyl)-O-(3-((tetrahydro-2H-pyran-2-yl)oxy)propyl)-L-serine (Compound aa85, Fmoc-Ser(nPrOTHP)-OH) (5 g) obtained as described above, and the mixture was stirred at room temperature for 5 min. The reaction solution was extracted with t-butyl methyl ether twice, washed with brine, dried over anhydrous sodium sulfate, and filtered. The solvent was evaporated under reduced pressure to afford N-(((9H-fluoren-9-yl)methoxy)carbonyl)-O-(3-((tetrahydro-2H-pyran-2-yl)oxy)propyl)-L-serine (Compound aa85, Fmoc-Ser(nPrOTHP)-OH) (4.9 g) as a crude product. This was used in peptide synthesis without further purification.
LCMS (ESI) m/z = 468 (M-H)-

Retention time: 0.84 min (Analytical condition SQDFA05)

Synthesis of methyl N-((benzyloxy)carbonyl)-O-(3-hydroxy-2,2-dimethylpropyl)-L-serinate (Compound aa86, Cbz-Ser(2-Me2-PrOH)-OMe)

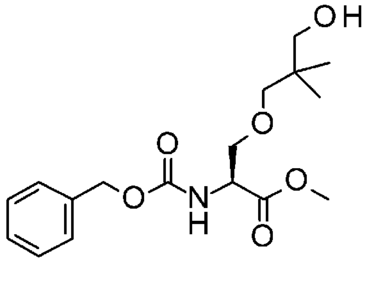

Under a nitrogen atmosphere, commercially available 1-benzyl 2-methyl (S)-aziridine-1,2-dicarboxylate (Cbz-Azy-OMe) (30 g, 127.53 mmol) and 2,2-dimethylpropan-1,3-diol (26.56 g, 255.06 mmol) were dissolved in dichloromethane (400 mL), the mixture was cooled to 0°C, a boron trifluoride-diethyl ether complex ($BF_3 \cdot Et_2O$) (2.72 g, 19.129 mmol) was then added dropwise, and the mixture was stirred for 1 h. After the reaction solution was washed with a saturated aqueous sodium hydrogen carbonate solution and with brine, the resulting organic layer was dried over anhydrous sodium sulfate and filtered, and the solvent was evaporated under reduced pressure. The resulting residue was purified by reverse phase column chromatography (water/acetonitrile) to afford methyl N-((benzyloxy)carbonyl)-O-(3-hydroxy-2,2-dimethylpropyl)-L-serinate (Compound aa86, Cbz-Ser(2-Me2-PrOH)-OMe) (26.7 g, 62%).
LCMS (ESI) m/z = 326 (M+H)+

Retention time: 1.12 min (Analytical condition SMD method 13)

Synthesis of N-((benzyloxy)carbonyl)-O-(3-hydroxy-2.2-dimethylpropyl)-L-serine (Compound aa87, Cbz-Ser(2-Me2-PrOH)-OH)

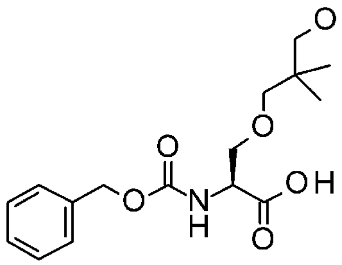

Under a nitrogen atmosphere, water (260 mL) and 2-propanol (1050 mL) were added at room temperature to lithium hydroxide monohydrate (13.16 g, 313.505 mmol) and calcium chloride (130.48 g, 1175.646 mmol), the mixture was stirred, a solution of methyl N-((benzyloxy)carbonyl)-O-(3-hydroxy-2,2-dimethylpropyl)-L-serinate (Compound aa86, Cbz-Ser(2-Me2-PrOH)-OMe) (26.6 g, 78.376 mmol) in tetrahydrofuran (260 mL) was then added at room temperature, and the mixture was stirred for 2 h. A 6N aqueous hydrochloric acid solution was added to the reaction solution until pH 3, the organic solvent was removed under reduced pressure, and then the aqueous layer was extracted with ethyl acetate. The resulting organic layer was dried over anhydrous sodium sulfate and filtered, and the resulting solvent was evaporated under reduced pressure to afford N-((benzyloxy)carbonyl)-O-(3-hydroxy-2,2-dimethylpropyl)-L-serine (Compound aa87, Cbz-Ser(2-Me2-PrOH)-OH) (25.0 g) as a crude product. This was used in the next step without purification.

Synthesis of N-(((9H-fluoren-9-yl)methoxy)carbonyl)-O-(3-hydroxy-2,2-dimethylpropyl)-L-serine (Compound aa88, Fmoc-Ser(2-Me2-PrOH)-OH)

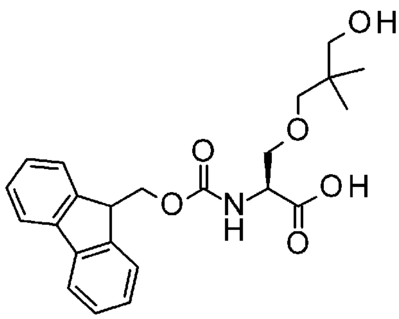

Under a hydrogen atmosphere (3 atm or less), a mixture of the crude product N-((benzyloxy)carbonyl)-O-(3-hydroxy-2,2-dimethylpropyl)-L-serine (Compound aa87, Cbz-Ser(2-Me2-PrOH)-OH) (25.0 g) obtained as described above, 10% palladium on carbon (5 g, 20% w/w), and methanol (250 mL) was stirred at room temperature for 16 h. The reaction solution was filtered, the solvent was then evaporated under reduced pressure, and the resulting solids were

washed with ethyl acetate three times to afford O-(3-hydroxy-2,2-dimethylpropyl)-L-serine (H-Ser(2-Me2-PrOH)-OH) (13.1 g, 87% over two steps).

Under a nitrogen atmosphere, N-(9-fluorenylmethoxycarbonyloxy)succinimide (Fmoc-OSu) (24.08 g, 71.381 mmol) was added at room temperature to a solution of O-(3-hydroxy-2,2-dimethylpropyl)-L-serine (H-Ser(2-Me2-PrOH)-OH) (13 g, 67.982 mmol) and sodium hydrogen carbonate (17.13 g, 203.946 mmol) in water/1,4-dioxane (260 mL/104 mL), and the mixture was stirred for 2 h. Water was added to the reaction solution, the mixture was washed with t-butyl methyl ether twice, a 6N aqueous hydrochloric acid solution was added to the aqueous layer until pH 3, and the mixture was extracted with t-butyl methyl ether. After the resulting organic layer was dried over anhydrous sodium sulfate and filtered, the solvent was evaporated under reduced pressure, and the organic layer was further dried under reduced pressure using a vacuum pump. The resulting residue was purified by reverse phase column chromatography (water/acetonitrile) to afford N-(((9H-fluoren-9-yl)methoxy)carbonyl)-O-(3-hydroxy-2,2-dimethylpropyl)-L-serine (Compound aa88, Fmoc-Ser(2-Me2-PrOH)-OH) (26.5 g, 94%).
LCMS (ESI) m/z = 414 (M+H)+

Retention time: 1.44 min (Analytical condition SMD method 51)

Synthesis of N-(((9H-fluoren-9-yl)methoxy)carbonyl)-O-(2,2-dimethyl-3-((tetrahydro-2H-pyran-2-yl)oxy)propyl)-L-serine (Compound aa89, Fmoc-Ser(2-Me2-PrOTHP)-OH)

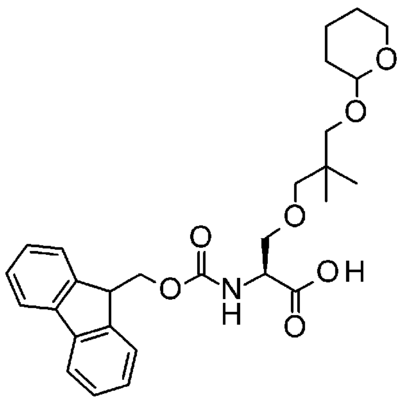

Under a nitrogen atmosphere, pyridinium p-toluenesulfonate (PPTS) (607.79 mg, 2.419 mmol) was added to a solution of N-(((9H-fluoren-9-yl)methoxy)carbonyl)-O-(3-hydroxy-2,2-dimethylpropyl)-L-serine (Compound aa88, Fmoc-Ser(2-Me2-PrOH)-OH) (20 g, 48.371 mmol) and 3,4-dihydro-2H-pyran (28.48 g, 338.598 mmol) in tetrahydrofuran (161 mL), and the mixture was stirred at 50°C for 3 h. The reaction solution was cooled, t-butyl methyl ether was added, the mixture was washed with brine, and then the organic layer was dried over anhydrous sodium sulfate and filtered. The solvent was evaporated under reduced pressure to afford N-(((9H-fluoren-9-yl)methoxy)carbonyl)-O-(2,2-dimethyl-3-((tetrahydro-2H-pyran-2-yl)oxy)propyl)-L-serine (Compound aa89, Fmoc-Ser(2-Me2-PrOTHP)-OH) and tetrahydro-2H-pyran-2-yl N-(((9H-fluoren-9-yl)methoxy)carbonyl)-O-(2,2-dimethyl-3((tetrahydro-2H-pyran-2-yl)oxy)propyl)-L-serinate (Fmoc-Ser(2-Me2-PrOTHP)-OTHP) as a mixture.

Tetrahydrofuran (161 mL) and a 1 M phosphate buffer (161 mL, pH 8.0) were added to the mixture of N-(((9H-fluoren-9-yl)methoxy)carbonyl)-O-(2,2-dimethyl-3-((tetrahydro-2H-pyran-2-yl)oxy)propyl)-L-serine (Compound aa89, Fmoc-Ser(2-Me2-PrOTHP)-OH) and tetrahydro-2H-pyran-2-yl N-(((9H-fluoren-9-yl)methoxy)carbonyl)-O-(2,2-dimethyl-3((tetrahydro-2H-pyran-2-yl)oxy)propyl)-L-serinate (Fmoc-Ser(2-Me2-PrOTHP)-OTHP) obtained as described above, and the mixture was stirred at 50°C for 3 h. The reaction solution was cooled, t-butyl methyl ether was added, the mixture was washed with brine, dried over anhydrous sodium sulfate, and filtered, and then the solvent was evaporated under reduced pressure. The resulting residue was recrystallized from diethyl ether/heptane to afford N-(((9H-fluoren-9-yl)methoxy)carbonyl)-O-(2,2-dimethyl-3-((tetrahydro-2H-pyran-2-yl)oxy)propyl)-L-serine (Compound aa89, Fmoc-Ser(2-Me2-PrOTHP)-OH) as a sodium salt (17.1 g).

t-Butyl methyl ether (120 mL) and a 0.05 M aqueous phosphoric acid solution (350 mL) were added to the sodium salt of N-(((9H-fluoren-9-yl)methoxy)carbonyl)-O-(2,2-dimethyl-3-((tetrahydro-2H-pyran-2-yl)oxy)propyl)-L-serine (Compound aa89, Fmoc-Ser(2-Me2-PrOTHP)-OH) (8 g) obtained as described above, and the mixture was stirred at room temperature for 5 min. The reaction solution was extracted with t-butyl methyl ether, washed with brine twice, then dried over anhydrous sodium sulfate, and filtered. The solvent was evaporated under reduced pressure to afford

N-(((9H-fluoren-9-yl)methoxy)carbonyl)-O-(2,2-dimethyl-3-((tetrahydro-2H-pyran-2-yl)oxy)propyl)-L-serine (Compound aa89, Fmoc-Ser(2-Me2-PrOTHP)-OH) (7 g) as a crude product. This was used in peptide synthesis without further purification.
LCMS (ESI) m/z = 496 (M-H)-

Retention time: 0.97 min (Analytical condition SQDFA05)

Synthesis of (4S)-4-methyl-2-phenyl-1,3-dioxane (Compound aa90)

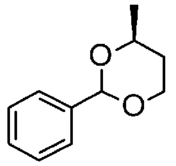

Under a nitrogen atmosphere, p-toluenesulfonic acid (TsOH) (2.64 g, 13.880 mmol) was added at 0°C to a solution of (S)-butane-1,3-diol (25 g, 277.407 mmol, CAS # 24621-61-2) and (dimethoxymethyl)benzene (63.37 g, 416.688 mmol) in chloroform ($CHCl_3$) (210 mL), and the mixture was stirred at room temperature for 16 h. Dichloromethane was added to the reaction solution, the mixture was washed with an aqueous sodium carbonate solution and with water, then dried over anhydrous sodium sulfate, and filtered, and the solvent was evaporated under reduced pressure. The resulting residue was dissolved in ethanol (EtOH) (135 mL), sodium borohydride ($NaBH_4$) (5.25 g, 138.778 mmol) was added, and the mixture was stirred at room temperature for 30 min. A saturated aqueous sodium hydrogen carbonate solution (210 mL) was added to the reaction solution, the mixture was stirred at room temperature for 2 h, and then ethyl acetate was added for extraction. The organic layer was washed with brine, dried over anhydrous sodium sulfate, and then filtered, and the solvent was evaporated under reduced pressure. The resulting residue was purified by normal phase column chromatography (petroleum ether/ethyl acetate) to afford (4S)-4-methyl-2-phenyl-1,3-dioxane (Compound aa90) (42.7 g, 86%).
LCMS (ESI) m/z = 179 (M+H)+

Retention time: 1.26 min (Analytical condition SMD method 13)

Synthesis of (S)-3-(benzyloxy)butan-1-ol (Compound aa91)

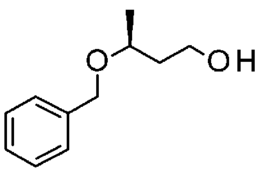

Under a nitrogen atmosphere, a 1 M solution of diisobutylaluminum hydride (DIBAL) in hexane (390 mL, 390 mmol) was added dropwise at -50°C to a solution of (4S)-4-methyl-2-phenyl-1,3-dioxane (Compound aa90) (35 g, 196.629 mmol) obtained as described above in dichloromethane (960 mL). The reaction solution was stirred at -50°C for 30 min, and then further stirred at room temperature for 2 h. Methanol (MeOH) was added to the reaction solution at -30°C to quench the reaction, and then water was added. A 6N aqueous hydrochloric acid solution was added until the reaction solution was pH = 2, the mixture was extracted with dichloromethane, and the resulting organic layer was washed with brine, dried over anhydrous sodium sulfate, and filtered. The solvent was evaporated under reduced pressure, and the resulting residue was purified by normal phase column chromatography (petroleum ether/ethyl acetate) to afford (S)-3-(benzyloxy)butan-1-ol (Compound aa91) (21.6 g, 61%).
LCMS (ESI) m/z = 181 (M+H)+

Retention time: 1.16 min (Analytical condition SMD method 13)

Synthesis of methyl O-((S)-3-(benzyloxy)butyl)-N-((benzyloxy)carbonyl)-L-serinate (Compound aa92, Cbz-Ser(S-2-BuOBn)-OMe)

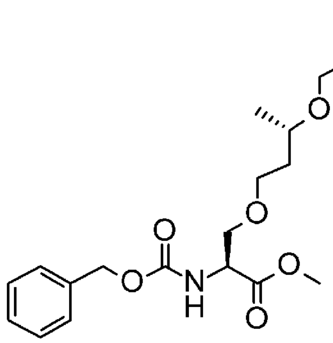

Under a nitrogen atmosphere, 1-benzyl 2-methyl (S)-aziridine-1,2-dicarboxylate (Cbz-Azy-OMe) (18.8 g, 80.0 mmol) and (S)-3-(benzyloxy)butan-1-ol (Compound aa91) (21.6 g, 120 mmol) obtained as described above were dissolved in dichloromethane (190 mL), a boron trifluoride-diethyl ether complex ($BF_3 \cdot Et_2O$) (1.51 mL, 11.976 mmol) was added at 0°C, and the mixture was stirred at room temperature for 2 h. Dichloromethane was added to the reaction solution, and the mixture was washed with a saturated aqueous sodium hydrogen carbonate solution and with brine. After the organic layer was dried over anhydrous sodium sulfate and filtered, the solvent was evaporated under reduced pressure, and the resulting residue was purified by reverse phase column chromatography (water/acetonitrile) to afford methyl O-((S)-3-(benzyloxy)butyl)-N-((benzyloxy)carbonyl)-L-serinate (Compound aa92, Cbz-Ser(S-2-BuOBn)-OMe) (15 g, 30%).
LCMS (ESI) m/z = 416 (M+H)+

Retention time: 1.39 min (Analytical condition SMD method 13)

Synthesis of O-((S)-3-(benzyloxy)butyl)-N-((benzyloxy)carbonyl)-L-serine (Compound aa93, Cbz-Ser(S-2-BuOBn)-OH)

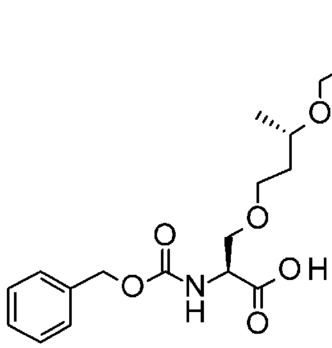

Under a nitrogen atmosphere, water (155 mL) and 2-propanol (615 mL) were added at room temperature to lithium hydroxide monohydrate (6.1 g, 145.238 mmol) and calcium chloride (60.2 g, 542.342 mmol), the mixture was stirred, and then a solution of methyl O-((S)-3-(benzyloxy)butyl)-N-((benzyloxy)carbonyl)-L-serinate (Compound aa92, Cbz-Ser(S-2-BuOBn)-OMe) (15 g, 36.144 mmol) obtained as described above in tetrahydrofuran (155 mL) was added at room temperature. After the reaction solution was stirred at room temperature for 5 h, a 6N aqueous hydrochloric acid solution was added until the pH of the reaction solution was 3. After the organic solvent was removed under reduced pressure, the aqueous layer was extracted with ethyl acetate. After the resulting organic layer was washed with brine, dried over anhydrous sodium sulfate, and filtered, the solvent was evaporated under reduced pressure, and the residue was further dried under reduced pressure using a vacuum pump to afford O-((S)-3-(benzyloxy)butyl)-N-((benzyloxy)carbonyl)-L-serine (Compound aa93, Cbz-Ser(S-2-BuOBn)-OH) (16.5 g) as a crude product. This was used in the next step without purification.

Synthesis of N-(((9H-fluoren-9-yl)methoxy)carbonyl)-O-((S)-3-hydroxybutyl)-L-serine (Compound aa94, Fmoc-Ser(S-2-BuOH)-OH)

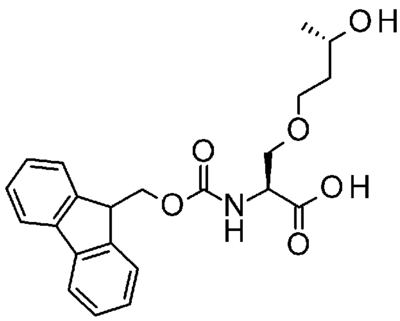

Under a hydrogen atmosphere (3 atm or less), a mixture of the crude product O-((S)-3-(benzyloxy)butyl)-N-((benzyloxy)carbonyl)-L-serine (Compound aa93, Cbz-Ser(S-2-BuOBn)-OH) (16.5 g) obtained as described above, 10% palladium on carbon (8.25 g, 50% w/w), and methanol (280 mL) was stirred at room temperature for 16 h. After the reaction solution was filtered, the solvent was evaporated under reduced pressure, and the resulting solids were washed with ethyl acetate three times to afford O-((S)-3-hydroxybutyl)-L-serine (H-Ser(S-2-BuOH)-OH) (5.85 g).

Under a nitrogen atmosphere, N-(9-fluorenylmethoxycarbonyloxy)succinimide (Fmoc-OSu) (11.7 g, 34.6815 mmol) was added at room temperature to a solution of O-((S)-3-hydroxybutyl)-L-serine (H-Ser(S-2-BuOH)-OH) (5.85 g 33.03 mmol) obtained as described above and sodium hydrogen carbonate (8.30 g, 98.81 mmol) in water/1,4-dioxane (135 mL/55 mL), and the mixture was stirred for 2 h. Water was added to the reaction solution, the mixture was washed with t-butyl methyl ether twice, a 6N aqueous hydrochloric acid solution was added until the pH of the aqueous layer was 3, and the mixture was extracted with t-butyl methyl ether. After the resulting organic layer was dried over anhydrous sodium sulfate and filtered, the solvent was evaporated under reduced pressure, and the organic layer was further dried under reduced pressure using a vacuum pump. The resulting residue was purified by reverse phase column chromatography (water/acetonitrile) to afford N-(((9H-fluoren-9-yl)methoxy)carbonyl)-O-((S)-3-hydroxybutyl)-L-serine (Compound aa94, Fmoc-Ser(S-2-BuOH)-OH) (8.1 g, 61%).
LCMS (ESI) m/z = 400 (M+H)+

Retention time: 1.21 min (Analytical condition SMD method 13)

Synthesis of N-(((9H-fluoren-9-yl)methoxy)carbonyl)-O-((3S)-3-((tetrahydro-2H-pyran-2-yl)oxy)butyl)-L-serine (Compound aa95, Fmoc-Ser(S-2-BuOTHP)-OH)

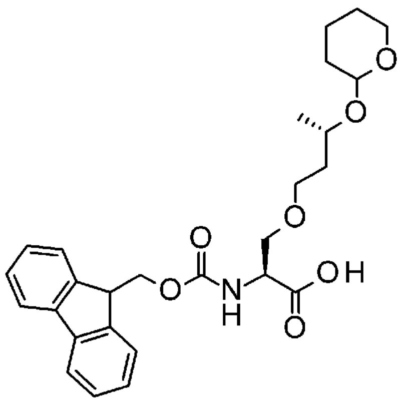

Under a nitrogen atmosphere, pyridinium p-toluenesulfonate (PPTS) (255 mg, 1.014 mmol) was added to a solution of N-(((9H-fluoren-9-yl)methoxy)carbonyl)-O-((S)-3-hydroxybutyl)-L-serine (Compound aa94, Fmoc-Ser(S-2-BuOH)-OH) (8.10 g, 20.292 mmol) obtained as described above and 3,4-dihydro-2H-pyran (11.94 g, 141.94 mmol) in tetrahydrofuran (90 mL), and the mixture was stirred at 50°C for 3 h. The reaction solution was cooled, t-butyl methyl ether was added, the mixture was washed with brine, and then the organic layer was dried over anhydrous sodium sulfate and filtered. The solvent was evaporated under reduced pressure to afford N-(((9H-fluoren-9-yl)methoxy)carbonyl)-O-((3S)-3-((tetrahydro-2H-pyran-2-yl)oxy)butyl)-L-serine (Compound aa95, Fmoc-Ser(S-2-BuOTHP)-OH) and tetrahydro-2H-pyran-2-yl N-(((9H-fluoren-9-yl)methoxy)carbonyl)-O-((3S)-3-((tetrahydro-2H-pyran-2-yl)oxy)butyl)-L-serinate (Fmoc-Ser(S-2-BuOTHP)-OTHP) as a mixture.

Tetrahydrofuran (90 mL) and a 1 M phosphate buffer (90 mL, pH 8.0) were added to the mixture of N-(((9H-fluoren-9-yl)methoxy)carbonyl)-O-((3S)-3-((tetrahydro-2H-pyran-2-yl)oxy)butyl)-L-serine (Compound aa95, Fmoc-Ser(S-2-BuOTHP)-OH) and tetrahydro-2H-pyran-2-yl N-(((9H-fluoren-9-yl)methoxy)carbonyl)-O-((3S)-3-((tetrahydro-2H-pyran-2-yl)oxy)butyl)-L-serinate (Fmoc-Ser(S-2-BuOTHP)-OTHP) obtained as described above, and the mixture was stirred at 50°C for 3 h. The reaction solution was cooled, t-butyl methyl ether was added, the mixture was washed with brine, dried over anhydrous sodium sulfate, and filtered, and then the solvent was evaporated under reduced pressure. The resulting residue was recrystallized from diethyl ether/heptane to afford N-(((9H-fluoren-9-yl)methoxy)carbonyl)-O-((3S)-3-((tetrahydro-2H-pyran-2-yl)oxy)butyl)-L-serine (Compound aa95, Fmoc-Ser(S-2-BuOTHP)-OH) as a sodium salt (7.4 g).

t-Butyl methyl ether (130 mL) and a 0.05 M aqueous phosphoric acid solution (360 mL) were added to the sodium salt of N-(((9H-fluoren-9-yl)methoxy)carbonyl)-O-((3S)-3-((tetrahydro-2H-pyran-2-yl)oxy)butyl)-L-serine (Compound aa95, Fmoc-Ser(S-2-BuOTHP)-OH) (7.4 g) obtained as described above, and the mixture was stirred at room temperature for 5 min. The reaction solution was extracted with t-butyl methyl ether, washed with brine twice, then dried over anhydrous sodium sulfate, and filtered. The solvent was evaporated under reduced pressure to afford N-(((9H-fluoren-9-yl)methoxy)carbonyl)-O-((3S)-3-((tetrahydro-2H-pyran-2-yl)oxy)butyl)-L-serine (Compound aa95, Fmoc-Ser(S-2-BuOTHP)-OH) (6.9 g) as a crude product. This was used in peptide synthesis without further purification.
LCMS (ESI) m/z = 482 (M-H)-

Retention time: 0.87 min, 0.88 min (Analytical condition SQDFA05)

Synthesis of (4R)-4-methyl-2-phenyl-1,3-dioxane (Compound aa96)

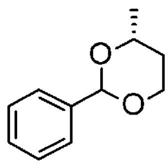

Under a nitrogen atmosphere, p-toluenesulfonic acid (TsOH) (1.91 g, 11.096 mmol) was added at 0°C to a solution of (R)-butane-1,3-diol (20 g, 221.921 mmol, CAS # 6290-03-5) and (dimethoxymethyl)benzene (50.66 g, 332.867 mmol) in chloroform ($CHCl_3$) (200 mL), and the mixture was stirred at room temperature for 16 h. Dichloromethane was added to the reaction solution, the mixture was washed with an aqueous sodium carbonate solution and with water, then dried over anhydrous sodium sulfate, and filtered, and the solvent was evaporated under reduced pressure. The resulting residue was dissolved in ethanol (EtOH) (13 mL), sodium borohydride ($NaBH_4$) (4.20 g, 110.961 mmol) was added, and the mixture was stirred at room temperature for 30 min. A saturated aqueous sodium hydrogen carbonate solution (13 mL) was added to the reaction solution, the mixture was stirred at room temperature for 2 h, and then ethyl acetate was added for extraction. The organic layer was washed with brine, dried over anhydrous sodium sulfate, and then filtered, and the solvent was evaporated under reduced pressure. The resulting residue was purified by normal phase column chromatography (petroleum ether/ethyl acetate) to afford (4R)-4-methyl-2-phenyl-1,3-dioxane (Compound aa96) (25 g, 63%).
LCMS (ESI) m/z = 179 (M+H)+

Retention time: 1.29 min (Analytical condition SMD method 13)

Synthesis of (R)-3-(benzyloxy)butan-1-ol (Compound aa97)

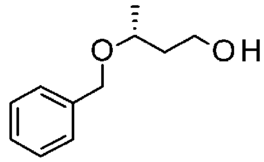

Under a nitrogen atmosphere, a 1 M solution of diisobutylaluminum hydride (DIBAL) in hexane (225 mL, 225 mmol) was added dropwise at -50°C to a solution of (4R)-4-methyl-2-phenyl-1,3-dioxane (Compound aa96) (20 g, 112.36 mmol) obtained as described above in dichloromethane (561 mL). The reaction solution was stirred at -50°C for 30 min, and then further stirred at room temperature for 2 h. Methanol (MeOH) was added to the reaction solution at -30°C to quench the reaction, and then water was added. A 6N aqueous hydrochloric acid solution was added until the reaction solution was pH = 2, the mixture was extracted with dichloromethane, and the resulting organic layer was washed with brine, dried over anhydrous sodium sulfate, and filtered. The solvent was evaporated under reduced pressure, and the resulting residue was purified by normal phase column chromatography (petroleum ether/ethyl acetate) to afford (R)-3-(benzyloxy)butan-1-ol (Compound aa97) (16.6 g, 82%).
LCMS (ESI) m/z = 181 (M+H)+

Retention time: 1.11 min (Analytical condition SMD method 13)

Synthesis of methyl O-((R)-3-(benzyloxy)butyl)-N-((benzyloxy)carbonyl)-L-serinate (Compound aa98, Cbz-Ser(R-2-BuOBn)-OMe)

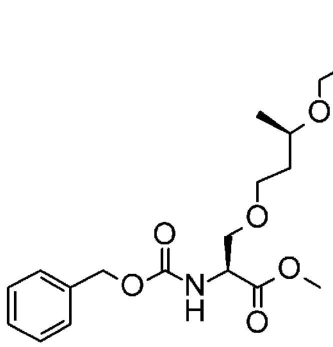

Under a nitrogen atmosphere, 1-benzyl 2-methyl (S)-aziridine-1,2-dicarboxylate (Cbz-Azy-OMe) (14.4 g, 61.481 mmol) and (R)-3-(benzyloxy)butan-1-ol (Compound aa97) (16.6 g, 92.2 mmol) obtained as described above were dissolved in dichloromethane (145 mL), a boron trifluoride-diethyl ether complex ($BF_3 \cdot Et_2O$) (1.31 g, 9.222 mmol) was added dropwise at 0°C, and the mixture was stirred at room temperature for 2 h. Dichloromethane was added to the reaction solution, and the mixture was washed with a saturated aqueous sodium hydrogen carbonate solution and with brine. After the organic layer was dried over anhydrous sodium sulfate and filtered, the solvent was evaporated under reduced pressure, and the resulting residue was purified by reverse phase column chromatography (water/acetonitrile) to afford methyl O-((R)-3-(benzyloxy)butyl)-N-((benzyloxy)carbonyl)-L-serinate (Compound aa98, Cbz-Ser(R-2-BuOBn)-OMe) (13.4 g, 52%).
LCMS (ESI) m/z = 416 (M+H)+

Retention time: 1.42 min (Analytical condition SMD method 13)

Synthesis of O-((R)-3-(benzyloxy)butyl)-N-((benzyloxy)carbonyl)-L-serine (Compound aa99, Cbz-Ser(R-2-BuOBn)-OH)

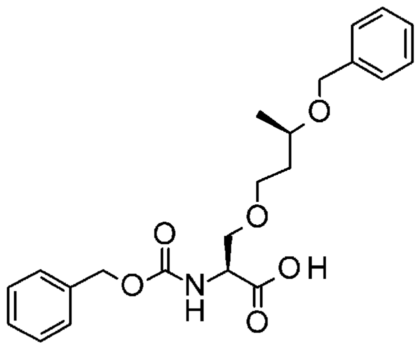

Under a nitrogen atmosphere, water (134 mL) and 2-propanol (538 mL) were added at room temperature to lithium hydroxide monohydrate (5.41 g, 128.92 mmol) and calcium chloride (53.69 g, 483.771 mmol), the mixture was stirred, and then a solution of methyl O-((R)-3-(benzyloxy)butyl)-N-((benzyloxy)carbonyl)-L-serinate (Compound aa98, Cbz-Ser(R-2-BuOBn)-OMe) (13.4 g, 32.251 mmol) obtained as described above in tetrahydrofuran (134 mL) was added at room temperature. After the reaction solution was stirred at room temperature for 5 h, a 6N aqueous hydrochloric acid solution was added until the pH of the reaction solution was 3. After the organic solvent was removed under reduced pressure, the aqueous layer was extracted with ethyl acetate. After the resulting organic layer was washed with brine, dried over anhydrous sodium sulfate, and filtered, the solvent was evaporated under reduced pressure. The residue was further dried under reduced pressure using a vacuum pump to afford O-((R)-3-(benzyloxy)butyl)-N-((benzyloxy)carbonyl)-L-serine (Compound aa99, Cbz-Ser(R-2-BuOBn)-OH) (13.6 g) as a crude product. This was used in the next step without purification.

Synthesis of N-(((9H-fluoren-9-yl)methoxy)carbonyl)-O-((R)-3-hydroxybutyl)-L-serine (Compound aa100, Fmoc-Ser(R-2-BuOH)-OH)

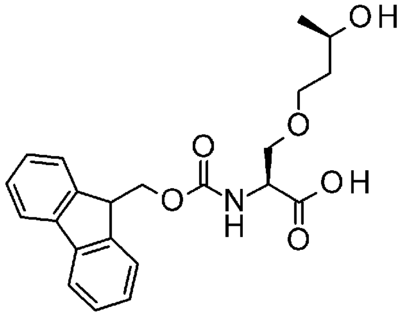

Under a hydrogen atmosphere (3 atm or less), a mixture of the crude product O-((R)-3-(benzyloxy)butyl)-N-((benzyloxy)carbonyl)-L-serine (Compound aa99, Cbz-Ser(R-2-BuOBn)-OH) (13.6 g) obtained as described above, 10% palladium on carbon (5.44 g), and methanol (270 mL) was stirred at room temperature for 16 h. After the reaction solution was filtered, the solvent was evaporated under reduced pressure, and the resulting solids were washed with ethyl acetate three times to afford O-((R)-3-hydroxybutyl)-L-serine (H-Ser(R-2-BuOH)-OH) (5.3 g, 93% over two steps).

Under a nitrogen atmosphere, N-(9-fluorenylmethoxycarbonyloxy)succinimide (Fmoc-OSu) (10.58 g, 31.405 mmol) was added at room temperature to a solution of O-((R)-3-hydroxybutyl)-L-serine (H-Ser(R-2-BuOH)-OH) (5.3 g, 29.91 mmol) obtained as described above and sodium hydrogen carbonate (7.54 g, 89.73 mmol) in water/1,4-dioxane (134 mL/57 mL), and the mixture was stirred for 2 h. Water was added to the reaction solution, the mixture was washed

with t-butyl methyl ether twice, a 6N aqueous hydrochloric acid solution was added until the pH of the aqueous layer was 3, and the mixture was extracted with t-butyl methyl ether. After the resulting organic layer was dried over anhydrous sodium sulfate and filtered, the solvent was evaporated under reduced pressure, and the organic layer was further dried under reduced pressure using a vacuum pump. The resulting residue was purified by reverse phase column chromatography (water/acetonitrile) to afford N-(((9H-fluoren-9-yl)methoxy)carbonyl)-O-((R)-3-hydroxybutyl)-L-serine (Compound aa100, Fmoc-Ser(R-2-BuOH)-OH) (7.4 g, 62%).
LCMS (ESI) m/z = 400 (M+H)+

Retention time: 1.20 min (Analytical condition SMD method 13)

Synthesis of N-(((9H-fluoren-9-yl)methoxy)carbonyl)-O-((3R)-3-((tetrahydro-2H-pyran-2-yl)oxy)butyl)-L-serine (Compound aa101, Fmoc-Ser(R-2-BuOTHP)-OH)

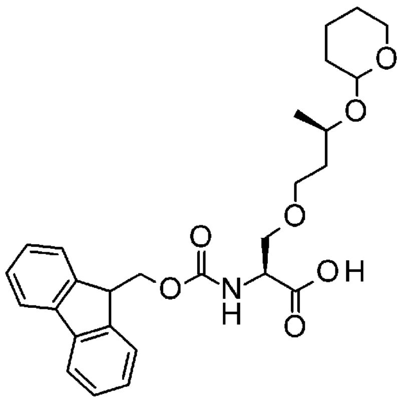

Under a nitrogen atmosphere, pyridinium p-toluenesulfonate (PPTS) (280 mg, 1.114 mmol) was added to a solution of N-(((9H-fluoren-9-yl)methoxy)carbonyl)-O-((R)-3-hydroxybutyl)-L-serine (Compound aa100, Fmoc-Ser(R-2-BuOH)-OH) (8.90 g, 22.281 mmol) obtained as described above and 3,4-dihydro-2H-pyran (13.12 g, 155.971 mmol) in tetrahydrofuran (74 mL), and the mixture was stirred at 50°C for 3 h. The reaction solution was cooled, t-butyl methyl ether was added, the mixture was washed with brine, and then the organic layer was dried over anhydrous sodium sulfate and filtered. The solvent was evaporated under reduced pressure to afford N-(((9H-fluoren-9-yl)methoxy)carbonyl)-O-((3R)-3-((tetrahydro-2H-pyran-2-yl)oxy)butyl)-L-serine (Compound aa101, Fmoc-Ser(R-2-BuOTHP)-OH) and tetrahydro-2H-pyran-2-yl N-(((9H-fluoren-9-yl)methoxy)carbonyl)-O-((3R)-3-((tetrahydro-2H-pyran-2-yl)oxy)butyl)-L-serinate (Fmoc-Ser(R-2-BuOTHP)-OTHP) as a mixture.

Tetrahydrofuran (74 mL) and a 1 M phosphate buffer (74 mL, pH 8.0) were added to the mixture of N-(((9H-fluoren-9-yl)methoxy)carbonyl)-O-((3R)-3-((tetrahydro-2H-pyran-2-yl)oxy)butyl)-L-serine (Compound aa101, Fmoc-Ser(R-2-BuOTHP)-OH) and tetrahydro-2H-pyran-2-yl N-(((9H-fluoren-9-yl)methoxy)carbonyl)-O-((3R)-3-((tetrahydro-2H-pyran-2-yl)oxy)butyl)-L-serinate (Fmoc-Ser(R-2-BuOTHP)-OTHP) obtained as described above, and the mixture was stirred at 50°C for 3 h. The reaction solution was cooled, t-butyl methyl ether was added, the mixture was washed with brine, dried over anhydrous sodium sulfate, and filtered, and then the solvent was evaporated under reduced pressure. The resulting residue was recrystallized from diethyl ether/heptane to afford N-(((9H-fluoren-9-yl)methoxy)carbonyl)-O-((3R)-3-((tetrahydro-2H-pyran-2-yl)oxy)butyl)-L-serine (Compound aa101, Fmoc-Ser(R-2-BuOTHP)-OH) as a sodium salt (10.2 g).

t-Butyl methyl ether (160 mL) and a 0.05 M aqueous phosphoric acid solution (440 mL) were added to the sodium salt of N-(((9H-fluoren-9-yl)methoxy)carbonyl)-O-((3R)-3-((tetrahydro-2H-pyran-2-yl)oxy)butyl)-L-serine (Compound aa101, Fmoc-Ser(R-2-BuOTHP)-OH) (10 g) obtained as described above, and the mixture was stirred at room temperature for 5 min. The reaction solution was extracted with t-butyl methyl ether, washed with brine twice, then dried over anhydrous sodium sulfate, and filtered. The solvent was evaporated under reduced pressure to afford N-(((9H-fluoren-9-yl)methoxy)carbonyl)-O-((3R)-3-((tetrahydro-2H-pyran-2-yl)oxy)butyl)-L-serine (Compound aa101, Fmoc-Ser(R-2-BuOTHP)-OH) (8.8 g) as a crude product. This was used in peptide synthesis without further purification.
LCMS (ESI) m/z = 482 (M-H)-

Retention time: 0.86 min, 0.88 min (Analytical condition SQDFA05)

Synthesis of (S)-2-((((9H-fluoren-9-yl)methoxy)carbonyl)amino)-3-(4-hydroxy-3-methoxyphenyl)propanoic acid (Compound aa102, Fmoc-Tyr(3-OMe)-OH)

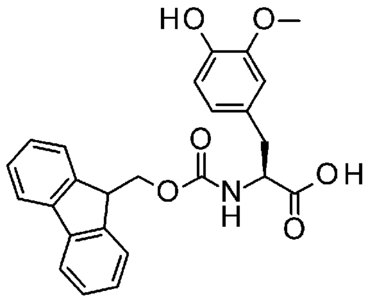

Water (49.9 mL), sodium carbonate (3.65 g, 34.4 mmol), and 1,4-dioxane (14.9 mL) were added at room temperature to commercially available (S)-2-amino-3-(4-hydroxy-3-methoxyphenyl)propanoic acid (H-Tyr(3-OMe)-OH) (2.5 g, 11.48 mmol). A solution of N-(9-fluorenylmethoxycarbonyloxy)succinimide (Fmoc-OSu) (3.10 g, 9.18 mmol) in 1,4-dioxane (50 mL) was added dropwise to the reaction solution at 0°C, the mixture was stirred at 0°C for 20 min, then N-(9-fluorenylmethoxycarbonyloxy)succinimide (Fmoc-OSu) (388 mg, 1.15 mmol) was further added to the reaction solution, and the mixture was stirred for 20 min. N-(9-fluorenylmethoxycarbonyloxy)succinimide (Fmoc-OSu) (388 mg, 1.15 mmol) was added again to the reaction solution at 0°C, and the mixture was stirred for 40 min. Water and t-butyl methyl ether (TBME) were added to the reaction solution, and the resulting aqueous layer was acidified with a 1N aqueous hydrochloric acid solution and then extracted with ethyl acetate three times. The resulting organic layers were combined, washed with brine, dried over anhydrous magnesium sulfate, and filtered, and then the solvent was evaporated under reduced pressure. The resulting residue was purified by reverse phase column chromatography (0.1% aqueous formic acid solution/0.1% formic acid acetonitrile solution) to afford (S)-2-((((9H-fluoren-9-yl)methoxy)carbonyl)amino)-3-(4-hydroxy-3-methoxyphenyl)propanoic acid (Compound aa102, Fmoc-Tyr(3-OMe)-OH) (3.53 g, 93%).
LCMS (ESI) m/z = 434 (M+H)+

Retention time: 0.73 min (Analytical condition SQD2FA05)

Synthesis of methyl (S)-2-((((9H-fluoren-9-yl)methoxy)carbonyl)amino)-3-(4-hydroxy-3-methoxyphenyl)propanoate (Compound aa103, Fmoc-Tyr(3-OMe)-OMe)

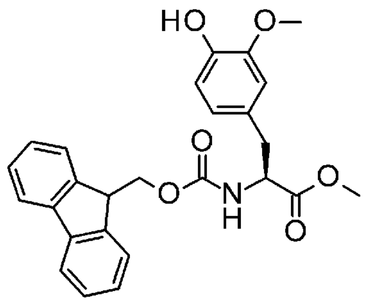

Under a nitrogen atmosphere, methanol (20.75 mL) and acetonitrile (5.40 mL) were added at room temperature to (S)-2-((((9H-fluoren-9-yl)methoxy)carbonyl)amino)-3-(4-hydroxy-3-methoxyphenyl)propanoic acid (Compound aa102, Fmoc-Tyr(3-OMe)-OH) (2.29 g, 5.23 mmol), the mixture was stirred at 0°C for 10 min, and then a 2.0 M solution of trimethylsilyldiazomethane (TMS diazomethane) in hexane (2.62 mL, 5.23 mmol) was added dropwise at 0°C. After a 2.0 M solution of trimethylsilyldiazomethane (TMS diazomethane) in hexane (2.62 mL, 5.23 mmol) was again added dropwise to the reaction solution at 0°C, a 2.0 M solution of trimethylsilyldiazomethane (TMS diazomethane) in hexane (3.93 mL, 7.85 mmol) was further added dropwise at 0°C. After quenching the reaction by adding acetic acid (2.0 mL),

a 50% saturated aqueous sodium hydrogen carbonate solution and ethyl acetate were added, and the mixture was washed with ethyl acetate. The organic layer was extracted with a 50% saturated aqueous sodium hydrogen carbonate solution, and the resulting aqueous layers were combined and washed with ethyl acetate three times. The organic layer was washed with brine, dried over anhydrous magnesium sulfate, and then filtered. The solvent was evaporated under reduced pressure to afford methyl (S)-2-((((9H-fluoren-9-yl)methoxy)carbonyl)amino)-3-(4-hydroxy-3-methoxyphenyl)propanoate (Compound aa103, Fmoc-Tyr(3-OMe)-OMe) (2.39 g) as a crude product. This was used in the next step without further purification.

Synthesis of (S)-2-((((9H-fluoren-9-yl)methoxy)carbonyl)amino)-3-(4-((2-chlorophenyl)diphenylmethoxy)-3-methoxyphenyl)propanoic acid (Compound aa104, Fmoc-Phe(4-OClt-3-OMe)-OH)

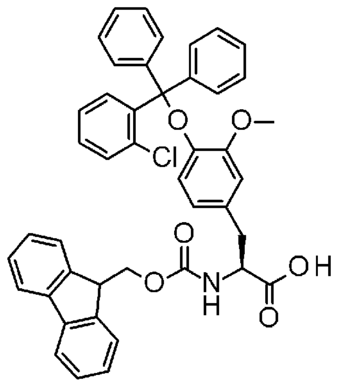

Under a nitrogen atmosphere, acetonitrile (10.58 mL), 2-chlorotrityl chloride (1.656 g, 5.29 mmol), and N,N-diisopropylethylamine (0.906 mL, 5.29 mmol) were added to the crude product methyl (S)-2-((((9H-fluoren-9-yl)methoxy)carbonyl)amino)-3-(4-hydroxy-3-methoxyphenyl)propanoate (Compound aa103, Fmoc-Tyr(3-OMe)-OMe) (2.39 g), and the mixture was stirred at room temperature for 1 h. Water and t-butyl methyl ether were added to the reaction solution, the organic layer was concentrated under reduced pressure, and the resulting residue was purified by reverse phase column chromatography (10 mM aqueous ammonium acetate solution/methanol) to afford methyl (S)-2-((((9H-fluoren-9-yl)methoxy)carbonyl)amino)-3-(4-((2-chlorophenyl)diphenylmethoxy)-3-methoxyphenyl)propanoate (Fmoc-Phe(4-OClt-3-OMe)-OMe) (2.6 g, 68%).

Water (14.96 mL) and lithium hydroxide monohydrate (0.603 g, 14.36 mmol) were added to calcium chloride (5.98 g, 53.8 mmol), and the mixture was stirred at room temperature for 5 min. Isopropanol (59.8 mL) and a solution of methyl (S)-2-((((9H-fluoren-9-yl)methoxy)carbonyl)amino)-3-(4-((2-chlorophenyl)diphenylmethoxy)-3-methoxyphenyl)propanoate (Fmoc-Phe(4-OClt-3-OMe)-OMe) (2.6 g, 3.59 mmol) in tetrahydrofuran (THF) (14.96 mL) were added to the reaction solution, the mixture was stirred for 4 h, and then a 0.05 M aqueous phosphoric acid solution and t-butyl methyl ether were added. The aqueous layer was extracted with t-butyl methyl ether three times, the resulting organic layer was washed with brine, dried over anhydrous sodium sulfate, and filtered. The solvent was then evaporated under reduced pressure to afford (S)-2-((((9H-fluoren-9-yl)methoxy)carbonyl)amino)-3-(4-((2-chlorophenyl)diphenylmethoxy)-3-methoxyphenyl)propanoic acid (Compound aa104, Fmoc-Phe(4-OClt-3-OMe)-OH) (2.8 g) as a crude product. This was used in peptide synthesis without further purification.
LCMS (ESI) m/z = 708 (M-H)-

Retention time: 0.74 min (Analytical condition SQD2AA50)

Synthesis of N-(((9H-fluoren-9-yl)methoxy)carbonyl)-O-((5-oxo-4,5-dihydro-1,2,4-oxadiazol-3-yl)methyl)-L-serine (Compound aa109, Fmoc-Ser(3-Me-5-Oxo-Odz)-OH)

N-(((9H-Fluoren-9-yl)methoxy)carbonyl)-O-((5-oxo-4,5-dihydro-1,2,4-oxadiazol-3-yl)methyl)-L-serine (Compound aa109, Fmoc-Ser(3-Me-5-Oxo-Odz)-OH) was synthesized according to the following scheme.

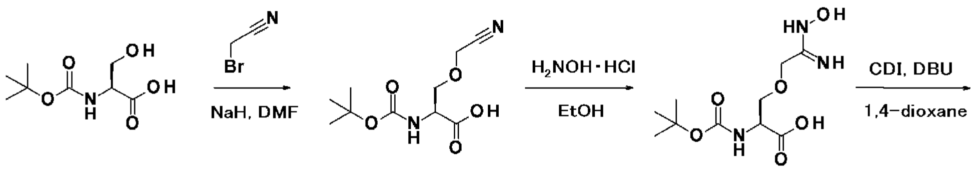


Boc-Ser-OH aa105 aa106

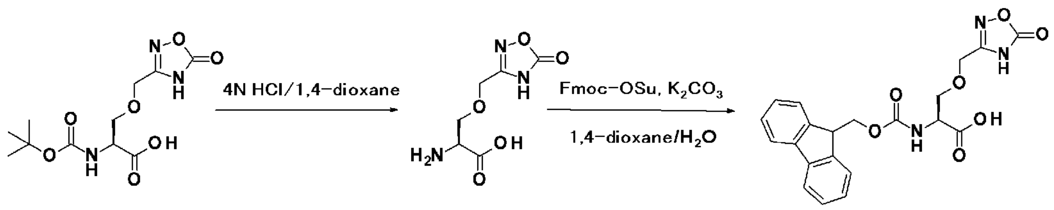


aa107 aa108 aa109

Under a nitrogen atmosphere, sodium hydride (34 g, 1420 mmol, 60% oil dispersion) was added at 0°C to a solution of commercially available (tert-butoxycarbonyl)-L-serine (Boc-Ser-OH) (50 g, 243.65 mmol) in dimethylformamide (DMF) (1000 mL), and the mixture was stirred for 1 h. A solution of 2-bromoacetonitrile (37.2 mL, 536.03 mmol) in dimethylformamide (DMF) (50 mL) was added dropwise to the reaction solution at 0°C, and the mixture was further stirred for 1 h. After water was added to the reaction solution, the mixture was extracted with ethyl acetate three times, and the resulting organic layer was dried over anhydrous sodium sulfate and filtered. The filtrate was concentrated under reduced pressure, and the resulting residue was purified by normal phase column chromatography (petroleum ether/ethyl acetate) to afford N-(tert-butoxycarbonyl)-O-(cyanomethyl)-L-serine (Compound aa105, Boc-Ser($CH_2CN$)-OH) (29 g, 49%).

Hydroxylamine hydrochloride (22.4 g, 315.26 mmol) and triethylamine (58.8 mL, 425.60 mmol) were added at room temperature to a solution of N-(tert-butoxycarbonyl)-O-(cyanomethyl)-L-serine (Compound aa105, Boc-Ser($CH_2CN$)-OH) (38.5 g, 157.63 mmol) obtained as described above in ethanol (EtOH) (400 mL), and the mixture was stirred at 85°C for 2 h. The solvent was evaporated under reduced pressure to afford N-(tert-butoxycarbonyl)-O-(2-(hydroxyamino)-2-iminoethyl)-L-serine (Compound aa106) (107 g) as a crude product.

Under a nitrogen atmosphere, carbonyldiimidazole (CDI) (3.96 g, 24.42 mmol) and 1,8-diazabicyclo[5.4.0]-7-undecene (DBU) (4.92 g, 32.32 mmol) were added at room temperature to a solution of the resulting crude product N-(tert-butoxycarbonyl)-O-(2-(hydroxyamino)-2-iminoethyl)-L-serine (Compound aa106) (12 g) in 1,4-dioxane (120 mL), and the mixture was stirred at 110°C for 2 h. The reaction solution was adjusted to pH = 2 with concentrated hydrochloric acid, and then extracted with dichloromethane (DCM) twice. After the resulting organic layer was dried over anhydrous sodium sulfate and filtered, the filtrate was concentrated under reduced pressure to afford N-(tert-butoxycarbonyl)-O-((5-oxo-4,5-dihydro-1,2,4-oxadiazol-3-yl)methyl)-L-serine (Compound aa107, Boc-Ser(3-Me-5-Oxo-Odz)-OH) (1.2 g) as a crude product.

Under a nitrogen atmosphere, a 4N hydrochloric acid/1,4-dioxane solution (15 mL) was added at room temperature to a solution of the resulting crude product N-(tert-butoxycarbonyl)-O-((5-oxo-4,5-dihydro-1,2,4-oxadiazol-3-yl)methyl)-L-serine (Compound aa107, Boc-Ser(3-Me-5-Oxo-Odz)-OH) (258 mg) in 1,4-dioxane (10 mL), and the mixture was stirred for 16 h. The solvent was evaporated under reduced pressure to afford O-((5-oxo-4,5-dihydro-1,2,4-oxadiazol-3-yl)methyl)-L-serine (Compound aa108, H-Ser(3-Me-5-Oxo-Odz)-OH) (160 mg) as a crude product.

Under a nitrogen atmosphere, N-(9-fluorenylmethoxycarbonyloxy)succinimide (Fmoc-OSu) (23 g, 68.2 mmol) was added at room temperature to a solution of the crude product O-((5-oxo-4,5-dihydro-1,2,4-oxadiazol-3-yl)methyl)-L-serine (Compound aa108, H-Ser(3-Me-5-Oxo-Odz)-OH) (22.4 g) obtained as described above and potassium carbonate (18.8 g, 135.04 mmol) in 1.4-dioxane/water (165 mL/110 mL), and the mixture was stirred for 3 h. The reaction solution was washed with t-butyl methyl ether/hexane (1/3), and the resulting aqueous layer was adjusted to pH = 2 with concentrated hydrochloric acid and then extracted with dichloromethane (DCM) twice. The resulting organic layer was dried over anhydrous sodium sulfate and filtered. The filtrate was concentrated under reduced pressure, and the resulting residue was purified by reverse phase column chromatography (water/acetonitrile) to afford N-(((9H-fluoren-9-yl)methoxy)carbonyl)-O-((5-oxo-4,5-dihydro-1,2,4-oxadiazol-3-yl)methyl)-L-serine (Compound aa109, Fmoc-Ser(3-Me-5-Oxo-Odz)-OH) (5.69 g).

LCMS (ESI) m/z = 426 (M+H)+

Retention time: 0.72 min (Analytical condition SQD2FA05)

Synthesis of (9H-fluoren-9-yl)methyl((2S)-4-diazo-3-oxo-1-(2-((tetrahydro-2H-pyran-2-yl)oxy)ethoxy)butan-2-yl)carbamate (Compound aa110)

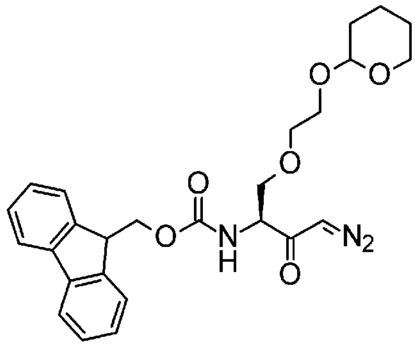

Under a nitrogen atmosphere, 4-methylmorpholine (0.355 mL, 3.23 mmol) and ethyl chloroformate (0.310 mL, 3.23 mmol) were added to a solution of N-(((9H-fluoren-9-yl)methoxy)carbonyl)-O-(2-((tetrahydro-2H-pyran-2-yl)oxy)ethyl)-L-serine (Compound aa22, Fmoc-Ser(EtOTHP)-OH) (1.4 g, 3.07 mmol) in tetrahydrofuran (5.0 mL) while cooling the tetrahydrofuran solution using an ice bath prepared with ice and common salt, and the mixture was stirred for 1 hour and 10 minutes. Subsequently, a solution of diazomethane in diethyl ether prepared separately was added dropwise to the reaction solution while cooling the reaction solution using an ice bath prepared with ice and common salt, and the mixture was stirred for 30 min. Acetic acid (0.88 mL, 15.37 mmol) was added to the reaction solution, the mixture was stirred for 10 min. Ethyl acetate and water were then added, the mixture was extracted with ethyl acetate, and the resulting organic layer was washed with brine, dried over anhydrous sodium sulfate, and filtered. The residue obtained by evaporating the solvent from the filtrate under reduced pressure was purified by normal phase column chromatography (hexane/ethyl acetate) to afford (9H-fluoren-9-yl)methyl ((2S)-4-diazo-3-oxo-1-(2-((tetrahydro-2H-pyran-2-yl)oxy)ethoxy)butan-2-yl)carbamate (Compound aa110) (1.13 g, 77%).
LCMS (ESI) m/z = 502 (M+Na)+

Retention time: 0.87 min (Analytical condition SQDFA05)

Preparation of diethyl ether solution of diazomethane

Diethyl ether (17.0 mL) was added to 1-methyl-3-nitro-1-nitrosoguanidine containing about 50% water (5.0 g, purchased from Tokyo Chemical Industry Co., Ltd., or FUJIFILM Wako Pure Chemical Corporation), and a 50% aqueous potassium hydroxide solution (9.5 mL) was added dropwise at 0°C. After the reaction solution was stirred for 1 h, the yellow diethyl ether layer was separated using a Pasteur pipette having a smoothed tip, and the resulting solution was used directly in the above-described reaction.

Synthesis of (3R)-3-((((9H-fluoren-9-yl)methoxy)carbonyl)amino)-4-(2-((tetrahydro-2H-pyran-2-yl)oxy)ethoxy)butanoic acid (Compound aa111, Fmoc-bAla(3R-MeOEtOTHP)-OH)

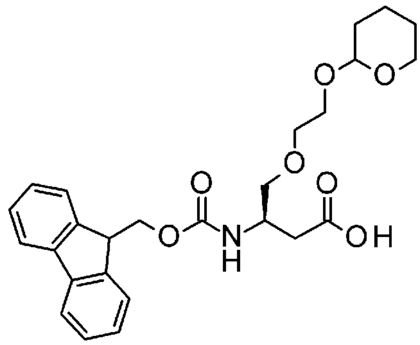

Under a nitrogen atmosphere, 4-methylmorpholine (0.974 mL, 8.86 mmol) and silver trifluoroacetate ($AgOCF_3$) (0.078 mL, 0.355 mmol) were added at 0°C to a solution of (9H-fluoren-9-yl)methyl ((2S)-4-diazo-3-oxo-1-(2-((tetrahydro-2H-pyran-2-yl)oxy)ethoxy)butan-2-yl)carbamate (Compound aa110) (1.7 g, 3.55 mmol) obtained as described above in tetrahydrofuran/water (18 mL/1.8 mL), and the mixture was stirred at room temperature for 2 hours and 30 minutes. Dimethyl sulfoxide (DMSO) was added to the reaction solution, and the mixture was purified by reverse phase column chromatography (water/acetonitrile) to afford (3R)-3-((((9H-fluoren-9-yl)methoxy)carbonyl)amino)-4-(2-((tetrahydro-2H-pyran-2-yl)oxy)ethoxy)butanoic acid (Compound aa111, Fmoc-bAla(3R-MeOEtOTHP)-OH) (1.4 g, 84%).
LCMS (ESI) m/z = 468 (M-H)-

Retention time: 0.78 min (Analytical condition SQDFA05)

(2S)-3-((((9H-Fluoren-9-yl)methoxy)carbonyl)amino)-2-((2-((tetrahydro-2H-pyran-2-yl)oxy)ethoxy)methyl)propanoic acid (Compound aa117) was synthesized according to the following scheme.

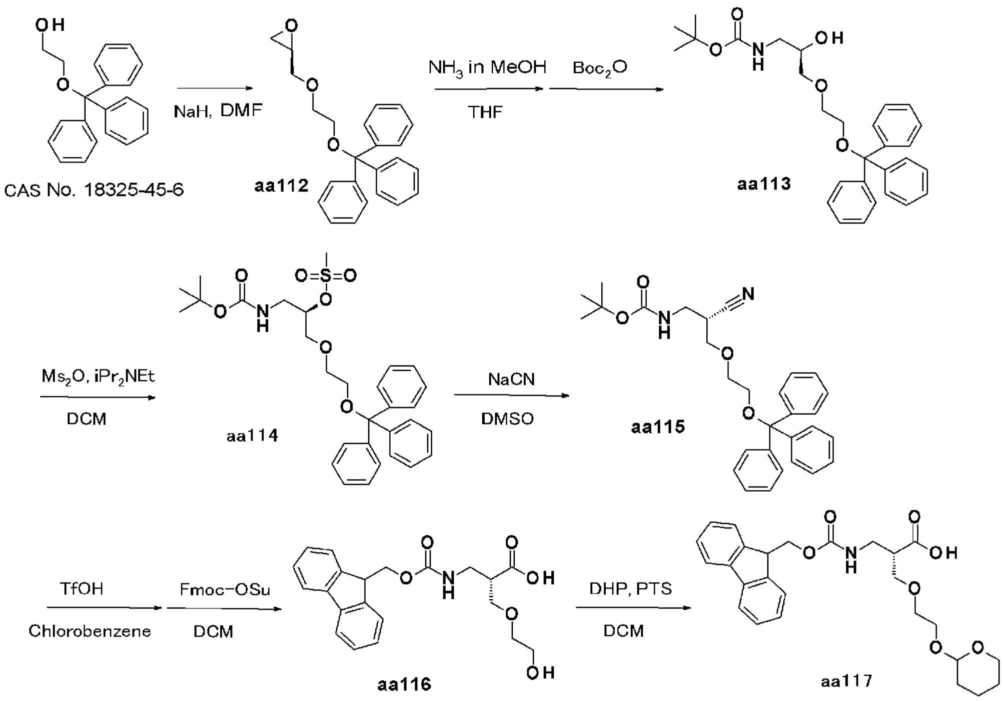


<u>Synthesis of (2S)-3-((((9H-fluoren-9-yl)methoxy)carbonyl)amino-2-((2-((tetrahydro-2H-pyran-2-yl)oxy)ethoxy)methyl)propanoic acid (Compound aa117, Fmoc-bAla(2S-MeOEtOTHP)-OH)</u>

Under a nitrogen atmosphere, sodium hydride (1.16 g, 28.9 mmol, 60% oil dispersion) was added at 0°C to a solution of 2-(trityloxy)ethan-1-ol (CAS. No. 18325-45-6) (8.8 g, 28.9 mmol) synthesized by the method described in a literature (Beilstein Journal of Organic Chemistry, 2017, 13, 2428-2441) and commercially available (R)-oxylan-2-ylmethyl 4-methylbenzenesulfonate (6.0 g, 26.3 mmol) in dimethylformamide (DMF) (100 mL), and the mixture was stirred at

room temperature for 12 h. After water was added to the reaction solution, the mixture was extracted with ethyl acetate twice. The resulting organic layer was concentrated under reduced pressure, and the resulting residue was purified by normal phase column chromatography (hexane/ethyl acetate = 5/1) to afford (R)-2-((2-(trityloxy)ethoxy)methyl)oxirane (Compound aa112) (7.32 g, 77%).
LCMS (ESI) m/z = 383 (M+Na)+

Retention time: 1.40 min (Analytical condition SMD method 45)

A 7 M solution of ammonia in methanol (196 mL) was added at room temperature to (R)-2-((2-(trityloxy)ethoxy)methyl)oxirane (Compound aa112) (38.5 g, 157.63 mmol) obtained as described above, and the mixture was stirred at room temperature for 15 h. The solvent was evaporated from the reaction solution under reduced pressure, water was added to the resulting residue, and the mixture was extracted with ethyl acetate. The resulting organic layer was concentrated under reduced pressure, and the resulting residue was purified by reverse phase column chromatography (water/acetonitrile). Moreover, the resulting fractions were combined, and a saturated sodium hydrogen carbonate solution (21.4 mL) and di-tert-butyl dicarbonate ($BOC_2O$) (5.55 g) were added to the solution thereof, and the mixture was stirred at room temperature for 10 min. The solvent was evaporated from the reaction solution under reduced pressure, water was added to the resulting residue, and the mixture was extracted with ethyl acetate. The resulting organic layer was concentrated under reduced pressure, the resulting residue was purified by normal phase column chromatography (hexane/ethyl acetate = 4/1) to afford tert-butyl (R)-(2-hydroxy-3-(2-(trityloxy)ethoxy)propyl)carbamate (Compound aa113) (4.25 g, 70%).
LCMS (ESI) m/z = 500 (M+Na)+

Retention time: 1.40 min (Analytical condition SMD method 45)

Under a nitrogen atmosphere, triethylamine (3.97 mL, 28.5 mmol) and methanesulfonic acid anhydride (2.48 g, 14.2 mmol) were added at room temperature to a solution of the resulting tert-butyl (R)-(2-hydroxy-3-(2-(trityloxy)ethoxy)propyl)carbamate (Compound aa113) (4.53 g, 9.48 mmol) in dichloromethane (47. 4 mL), and the mixture was stirred at room temperature for 1 h. The reaction solution was concentrated under reduced pressure, and the resulting residue was purified by reverse phase column chromatography (water/acetonitrile) to afford (R)-12,12-dimethyl-10-oxo-1,1,1-triphenyl-2,5,11-trioxa-9-azatridecan-7-yl methanesulfonate (Compound aa114) (4.62 g), 88%).
LCMS (ESI) m/z = 578 (M+Na)+

Retention time: 1.47 min (Analytical condition SMD method 45)

Under a nitrogen atmosphere, sodium cyanide (1.95 g, 39.7 mmol) was added at room temperature to a solution of the resulting (R)-12,12-dimethyl-10-oxo-1,1,1-triphenyl-2,5,11-trioxa-9-azatridecan-7-yl methanesulfonate (Compound aa114) (4.41 g, 7.93 mmol) in dimethyl sulfoxide (DMSO) (39.6 mL), and the mixture was stirred at 80°C for 10 h. Acetonitrile and water were added to the reaction solution, and the mixture was purified by reverse phase column chromatography (water/acetonitrile) to afford tert-butyl (R)-(2-cyano-3-(2-(trityloxy)ethoxy)propyl)carbamate (Compound aa115) (2.16 g, 53%).
LCMS (ESI) m/z = 509 (M+Na)+

Retention time: 1.49 min (Analytical condition SMD method 45)

Under a nitrogen atmosphere, trifluoromethanesulfonic acid (3.5 mL, 39.5 mmol) was added at 0°C to a solution of the resulting tert-butyl (R)-(2-cyano-3-(2-(trityloxy)ethoxy)propyl)carbamate (Compound aa115) (3.63 g, 7.46 mmol) in chlorobenzene (9.3 mL), and the mixture was stirred at room temperature for 1 h. Water (3.6 mL, 201 mmol) was then added to the reaction solution at room temperature, and the mixture was stirred at 110°C for 3 h. Water (20 mL), hexane (7 mL), and toluene (20 mL) were added to the reaction solution, and the aqueous layer was removed. Water (12 mL) was added to the resulting organic layer for washing. The resulting aqueous layers were combined, acetonitrile (37.3 mL) and sodium hydrogen carbonate (6.27 g, 74.6 mmol) were added, (1-fluorenylmethoxycarbonyloxy)succinimide (Fmoc-OSu) (3.77 g, 11.2 mmol) was added at room temperature, and the mixture was stirred for 30 min. Moreover, Fmoc-OSu (1.26 g, 3.73 mmol) was added at room temperature, and the mixture was stirred overnight. After being filtered, the reaction solution was extracted with t-butyl methyl ether. After 6N concentrated hydrochloric acid was added to the resulting aqueous layer until pH = 1, the mixture was purified by reverse phase column chromatography (water/acetonitrile) to afford (S)-3-((((9H-fluoren-9-yl)methoxy)carbonyl)amino)-2-((2-hydroxyethoxy)methyl)propanoic acid (Compound aa116) (1.31 g, 46%).
LCMS (ESI) m/z = 386 (M+H)+

Retention time: 1.78 min (Analytical condition SMD method 47)

Under a nitrogen atmosphere, pyridinium paratoluenesulfonate (PPTS) (67 mg, 0.27 mmol) was added at room temperature to a solution of (S)-3-((((9H-fluoren-9-yl)methoxy)carbonyl)amino)-2-((2-hydroxyethoxy)methyl)propanoic acid (Compound aa116) (1.03 g, 2.67 mmol) obtained as described above and 3,4-dihydro-2H-pyran (0.73 mL, 8.02 mmol) in dichloromethane (13.4 mL), and the mixture was stirred at 40°C for 10 h. Water was added to the reaction solution, and the mixture was extracted with dichloromethane. The resulting organic layer was washed with brine, then dried over anhydrous sodium sulfate, and filtered. A separately prepared phosphate buffer (pH = 8, 13.4 mL) was added to a solution of the resulting crude product in tetrahydrofuran (13.4 mL), and the mixture was stirred at 50°C for 20 h. Water (50 mL) was added to the reaction solution, and the mixture was extracted with t-butyl methyl ether/hexane (40 mL/10 mL). The resulting organic layer was washed with brine, then dried over anhydrous sodium sulfate, and filtered. The resulting organic layer was concentrated under reduced pressure, and the resulting residue was purified by reverse phase column chromatography (water/acetonitrile) to afford (2S)-3-((((9H-fluoren-9-yl)methoxy)carbonyl)amino)-2-((2-((tetrahydro-2H-pyran-2-yl)oxy)ethoxy)methyl)propanic acid (Compound aa117) (1.04 g, 83%).
LCMS (ESI) m/z = 492 (M+Na)+

Retention time: 0.95 min (Analytical condition SQDAA05-2)

(S)-2-((((9H-Fluoren-9-yl)methoxy)carbonyl)amino)-5-((R)-1-((allyloxy)carbonyl)-4,4-difluoropyrrolidin-2-yl)pentanoic acid (Compound aa122, Fmoc-Nva(2-R-4-F2-Pyrro(N-Alloc))-OH) was synthesized according to the following scheme.

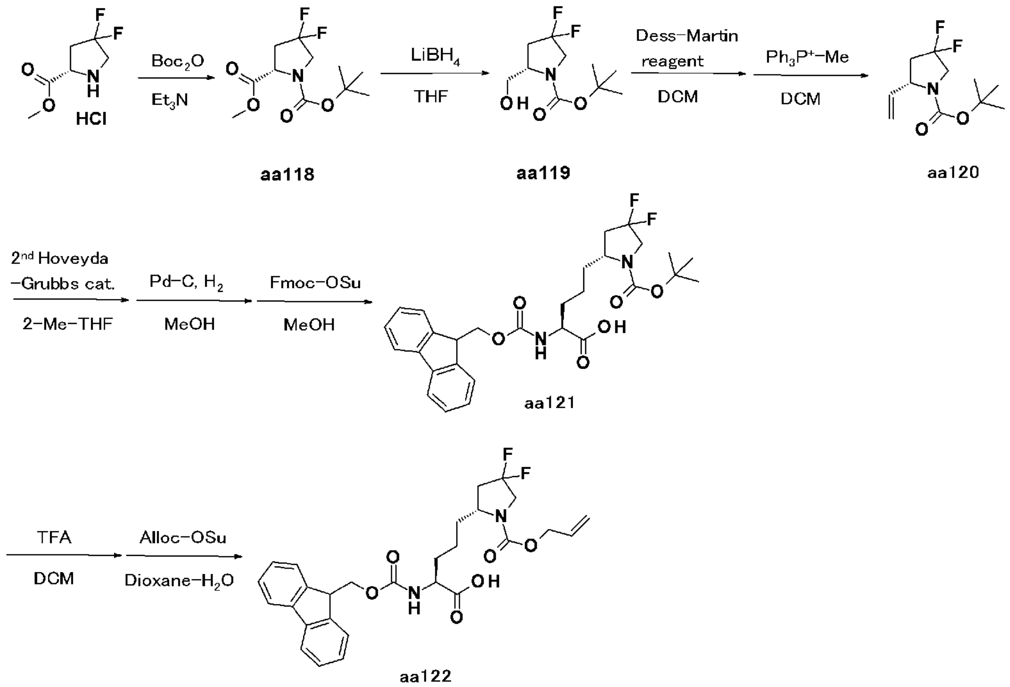

aa118 aa119 aa120 aa121 aa122

Synthesis of (S)-2-((((9H-fluoren-9-yl)methoxy)carbonyl)amino)-5-((R)-1-((allyloxy)carbonyl)-4,4-difluoropyrrolidin-2-yl)pentanoic acid (Compound aa122, Fmoc-Nva(2-R-4-F2-Pyrro(N-Alloc))-OH)

Under a nitrogen atmosphere, triethylamine (11.9 mL, 86 mmol) was added at room temperature to a solution of commercially available methyl (S)-4,4-difluoropyrrolidin-2-carboxylate hydrochloride (CAS. No. 156046-05-8) (6.9 g, 34.2 mmol) in dichloromethane (68.5 mL). Di-tert-butyl dicarbonate (Boc$_2$O) (11.2 g, 51.3 mmol) was added to the reaction solution at room temperature, and the mixture was stirred for 1 h. After water was added to the reaction solution, the mixture was extracted with ethyl acetate. The resulting organic layer was concentrated under reduced pressure, and the resulting residue was purified by normal phase column chromatography (n-hexane/ethyl acetate) to afford 1-(tert-butyl) 2-methyl (S)-4,4-difluoropyrrolidine-1,2-dicarboxylate (Compound aa118) (6.85 g, 75%).

A lithium borohydride/tetrahydrofuran (THF) solution (6.67 mL, 26.7 mmol) was added at 0°C to a solution of 1-(tert-butyl) 2-methyl (S)-4,4-difluoropyrrolidine-1,2-dicarboxylate (Compound aa118) (6.43 g, 24.2 mmol) obtained as described above in tetrahydrofuran (THF) (121 mL), and the mixture was stirred at room temperature for 1.5 h. A lithium

borohydride/tetrahydrofuran (THF) solution (1.5 mL, 6.0 mmol) was further added at 0°C, and the mixture was stirred at room temperature for 1 h. A 10% aqueous sodium chloride solution was added to the reaction solution at 0°C, and the mixture was extracted with ethyl acetate. The resulting organic layer was concentrated under reduced pressure, and the resulting residue was purified by normal phase column chromatography (n-hexane/ethyl acetate) to afford tert-butyl (S)-4,4-difluoro-2-(hydroxymethyl)pyrrolidine-1-carboxylate (Compound aa119) (5.70 g, 99%).

Under a nitrogen atmosphere, commercially available 1,1,1-triacetoxy-1,1-dihydro-1,2-benzoiodoxol-3(1H)-one (Dess-Martin reagent) (719 mg, 1.69 mmol) was added at 0°C to a solution of the resulting tert-butyl (S)-4,4-difluoro-2-(hydroxymethyl)pyrrolidine-1-carboxylate (335 mg, 1.41 mmol) in dichloromethane (7.0 mL), and the mixture was stirred at room temperature for 30 min. After a saturated aqueous sodium hydrogen carbonate solution was added to the reaction solution, the mixture was extracted with ethyl acetate, and the resulting organic layer was washed with water. The resulting organic layer was concentrated under reduced pressure, and the resulting residue was purified by normal phase column chromatography (n-hexane/ethyl acetate) to afford an aldehyde intermediate.

Under a nitrogen atmosphere, potassium 2-methylpropan-2-olate (tBuOK) (177 mg, 1.57 mmol) was added at room temperature to a solution of methyltriphenylphosphonium bromide (562 mg, 1.57 mmol) in diethyl ether (3.1 mL), and the mixture was stirred at room temperature for 2 h. A solution of the separately prepared aldehyde intermediate (185 mg, 0.786 mmol) in diethyl ether (0.79 mL) was added to the reaction solution, and the mixture was stirred at room temperature for 1 h. After a 10% aqueous ammonium chloride solution was added to the reaction solution, the mixture was extracted with ethyl acetate, and the resulting organic layer was washed with water. The resulting organic layer was concentrated under reduced pressure, and the resulting residue was purified by normal phase column chromatography (n-hexane/ethyl acetate) to afford tert-butyl (S)-4,4-difluoro-2-vinylpyrrolidine-1-carboxylate (Compound aa120) (135 mg, 74%).

Under a nitrogen atmosphere, the resulting tert-butyl (S)-4,4-difluoro-2-vinylpyrrolidine-1-carboxylate (Compound aa120) (3.50 g, 15.0 mmol) and (S)-2-((((9H-fluoren-9-yl)methoxy)carbonyl)amino)penta-4-enoic acid (CAS #: 146549-21-5, Fmoc-Algly-OH) (9.52 g, 28.2 mmol) were dissolved in 2-methyltetrahydrofuran (2-MeTHF) (60 mL). The second-generation Hoveyda-Grubbs catalyst (0.94 g, 1.50 mmol) was added to the solution, and the mixture was stirred at 60°C overnight. After being cooled to room temperature, the mixture was filtered through Celite, and the filtrate was concentrated under reduced pressure. The resulting residue was purified by reverse phase silica gel column chromatography (0.1% aqueous formic acid solution/0.1% formic acid-acetonitrile solution) to afford a crude product (7.58 g).

The resulting crude product (7.58 g) was dissolved in methanol (186 mL), 10% palladium on carbon (1.6 g) was added, and the mixture was stirred at room temperature for 2 days under a hydrogen atmosphere. The reaction solution was filtered through Celite, and the filtrate was concentrated under reduced pressure. The resulting residue was dissolved in methanol (34.9 mL), a saturated aqueous sodium hydrogen carbonate solution (34.9 mL) and N-(9-fluorenylmethoxycarbonyloxy)succinimide (Fmoc-OSu) (7.07 g, 20.95 mmol) were added, and the mixture was stirred at room temperature for 2 h. Insoluble matter was filtered off, and the filtrate was acidified with a 1 M aqueous hydrochloric acid solution and then extracted with ethyl acetate. The organic layer was washed with brine, dried over anhydrous magnesium sulfate, filtered, and concentrated under reduced pressure. The resulting residue was purified by reverse phase silica gel column chromatography (0.1% aqueous formic acid solution/0.1% formic acid-acetonitrile solution) to afford (S)-2-((((9H-fluoren-9-yl)methoxy)carbonyl)amino)-5-((R)-1-(tert-butoxycarbonyl)-4,4-difluoropyrrolidin-2-yl)pentanoic acid (Compound aa121) (2.54 g, 33%).
LCMS (ESI) m/z = 567 (M+Na)+

Retention time: 1.32 min (Analytical condition SMD method 45)

The resulting (S)-2-((((9H-fluoren-9-yl)methoxy)carbonyl)amino)-5-((R)-1-(tertbutoxycarbonyl)-4,4-difluoropyrrolidin-2-yl)pentanoic acid (Compound aa121) (2.17 g, 3.98 mmol) was dissolved in dichloromethane (DCM) (53.1 mL), trifluoroacetic acid (9.15 mL, 119.8 mmol) was added, and the mixture was stirred at room temperature for 1 hour and 30 minutes. The reaction solution was concentrated under reduced pressure, toluene was added to the residue, and the mixture was concentrated again under reduced pressure to afford a crude product (2.66 g).

The resulting crude product (2.66 g) was dissolved in a 1,4-dioxane/water (12.67 mL/12.67 mL) solution, sodium hydrogen carbonate (1.92 g, 22.80 mmol) and N-(allyloxycarbonyloxy)succinimide (Alloc-OSu) (0.61 mL, 3.91 mmol) were added at room temperature, and the mixture was stirred for 30 min. Formic acid and dimethylsulfoxide were added to the reaction solution, and the mixture was purified by reverse phase silica gel column chromatography (0.1% aqueous formic acid solution/0.1% formic acid-acetonitrile solution) to afford (S)-2-((((9H-fluoren-9-yl)methoxy)carbonyl)amino)-5-((R)-1-((allyloxy)carbonyl)-4,4-difluoropyrrolidin-2-yl)pentanoic acid (Compound aa122, Fmoc-Nva(2-R-4-F2-Pyrro(N-Alloc))-OH) (1.97 g, 94%).
LCMS (ESI) m/z = 529 (M+H)+

Retention time: 2.75 min (Analytical condition SMD method 47)

N-(((9H-Fluoren-9-yl)methoxy)carbonyl)-O-(((S)-1-((allyloxy)carbonyl)-4,4-difluoropyrrolidin-2-yl)methyl)-L-serine (Compound aa126, Fmoc-Ser(S-4-F2-Pyrro(N-Alloc)-Me)-OH) was synthesized according to the following scheme.

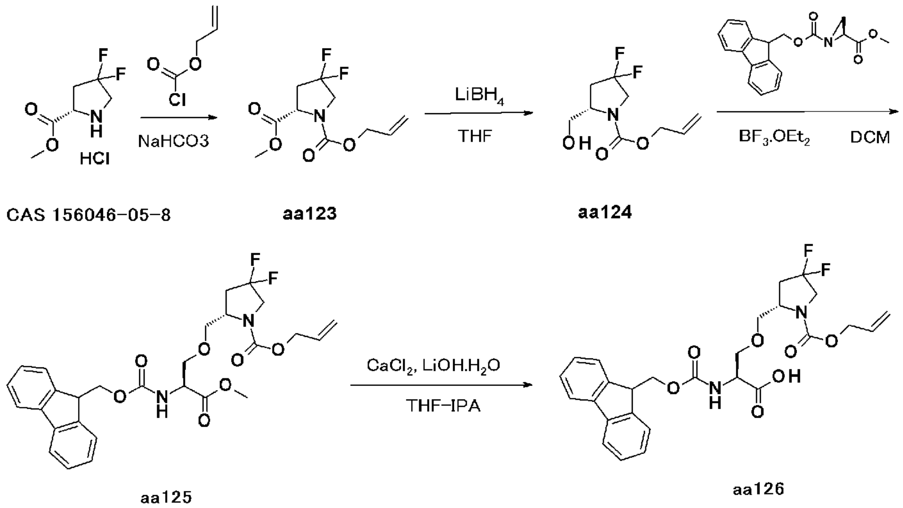

CAS 156046-05-8
aa123
aa124
aa125
aa126

Under a nitrogen atmosphere, sodium hydrogen carbonate (5.29 g, 63.0 mmol) was added at room temperature to an aqueous solution (11.43 mL) of commercially available methyl (S)-4,4-difluoropyrrolidin-2-carboxylate hydrochloride (CAS. No. 156046-05-8) (5.08 g, 25.2 mmol). A solution of allyl chloroformate (4.0 mL, 37.8 mmol) in 1,4-dioxane (22.9 mL) was added dropwise to the reaction solution at room temperature, and the mixture was further stirred for 1.5 h. After water was added to the reaction solution, the mixture was extracted with ethyl acetate twice, and the resulting organic layer was dried over anhydrous sodium sulfate and filtered. The filtrate was concentrated under reduced pressure, and the resulting residue was purified by normal phase column chromatography (n-hexane/ethyl acetate) to afford 1-allyl 2-methyl (S)-4,4-difluoropyrrolidine-1,2-dicarboxylate (Compound aa123) (6.21 g, 99%).
LCMS (ESI) m/z = 250 (M+H)+

Retention time: 0.91 min (Analytical condition SMD method 45)

A lithium borohydride/tetrahydrofuran (THF) solution (10.24 mL, 41.0 mmol) was added dropwise at 0°C to a solution of 1-allyl 2-methyl (S)-4,4-difluoropyrrolidine-1,2-dicarboxylate (Compound aa123) (6.81 g, 27.3 mmol) obtained as described above in tetrahydrofuran (THF) (82.0 mL), and the mixture was stirred at room temperature for 2 h. After water and a 2N aqueous hydrochloric acid solution (20.5 mL, 41.0 mmol) were added to the reaction solution at 0°C, the mixture was extracted with ethyl acetate, and the resulting organic layer was dried over anhydrous sodium sulfate and filtered. The filtrate was concentrated under reduced pressure, and the resulting residue was purified by normal phase column chromatography (n-hexane/ethyl acetate) to afford allyl (S)-4,4-difluoro-2-(hydroxymethyl)pyrrolidine-1-carboxylate (Compound aa124) (5.13 g, 85%).
LCMS (ESI) m/z = 222 (M+H)+

Retention time: 0.76 min (Analytical condition SMD method 45)

Under a nitrogen atmosphere, a solution of a boron trifluoride-ethyl ether complex (0.876 mL, 6.92 mmol) in dichloromethane was added dropwise at 0°C to a solution of the resulting allyl (S)-4,4-difluoro-2-(hydroxymethyl)pyrrolidine-1-carboxylate (Compound aa124) (5.1 g, 23.06 mmol) and 1-((9H-fluoren-9-yl)methyl) 2-methyl (S)-aziridine-1,2-dicarboxylate (Compound aa73, Fmoc-Azy-OMe) (4.97 g, 15.37 mmol) in dichloromethane (36 mL), and the mixture was further stirred for 1.5 h. After water was added to the reaction solution, the mixture was extracted with dichloromethane, and the resulting organic layer was dried over anhydrous sodium sulfate and filtered. The filtrate was concentrated under reduced pressure, and the resulting residue was purified by normal phase column chromatography (n-hexane/ethyl acetate) to afford allyl (S)-2-(((S)-2-((((9H-fluoren-9-yl)methoxy)carbonyl)amino)-3-methoxy-3-oxopropoxy)methyl)-4,4-difluoropyrrolidine-1-carboxylate (Compound 125, Fmoc-Ser(S-4-F2-Pyrro(N-Alloc)-Me)-OMe) (5.68 g, 68%).
LCMS (ESI) m/z = 545 (M+H)+

Retention time: 3.02 min (Analytical condition SMD method 47)

Under a nitrogen atmosphere, lithium hydroxide monohydrate (1.89 g, 44.9 mmol) was added to an aqueous solution (46.8 mL) of calcium chloride (18.7 g, 168 mmol) at room temperature, and the mixture was stirred for 5 min at room temperature. Isopropyl alcohol (187 mL) and a solution of allyl (S)-2-(((S)-2-((((9H-fluoren-9-yl)methoxy)carbonyl)amino)-3-methoxy-3-oxopropoxy)methyl)-4,4-difluoropyrrolidine-1-carboxylate (Compound 125, Fmoc-Ser(S-4-F2-Pyrro(N-Alloc)-Me)-OMe) (6.12 g, 11.23 mmol) in tetrahydrofuran (46.8 mL) were added at room temperature, and the mixture was stirred for 6.5 h. After 2N hydrochloric acid (33.7 mL, 67.4 mmol) was added, the solvent was evaporated under reduced pressure, and the resulting residue was purified by reverse phase column chromatography (water/acetonitrile) to afford N-(((9H-fluoren-9-yl)methoxy)carbonyl)-O-(((S)-1 -((allyloxy)carbonyl)-4,4-difluoropyrrolidin-2-yl)methyl)-L-serine (Compound aa126, Fmoc-Ser(S-4-F2-Pyrro(N-Alloc)-Me)-OH) (3.76 g, 63%).
LCMS (ESI) m/z = 531 (M+H)+

Retention time: 2.69 min (Analytical condition SMD method 47)

(S)-2-((((9H-Fluoren-9-yl)methoxy)carbonyl)amino)-3-(3-methoxy-4-(methylcarbamoyl)phenyl)propanoic acid (Compound aa127, Fmoc-Phe(3-OMe-4-CONMe)-OH), (S)-2-((((9H-fluoren-9-yl)methoxy)carbonyl)amino)-3-(4-methoxy-3-(methylcarbamoyl)phenyl)propanoic acid (Compound aa128, Fmoc-Phe(4-OMe-3-CONMe)-OH), and (S)-2-((((9H-fluoren-9-yl)methoxy)carbonyl)amino)-3-(3-methoxy-4-((methylsulfonyl)carbamoyl)phenyl)propanoic acid (Compound aa129, Fmoc-Phe(3-OMe-4-CONHMs)-OH) were synthesized according to the following scheme.

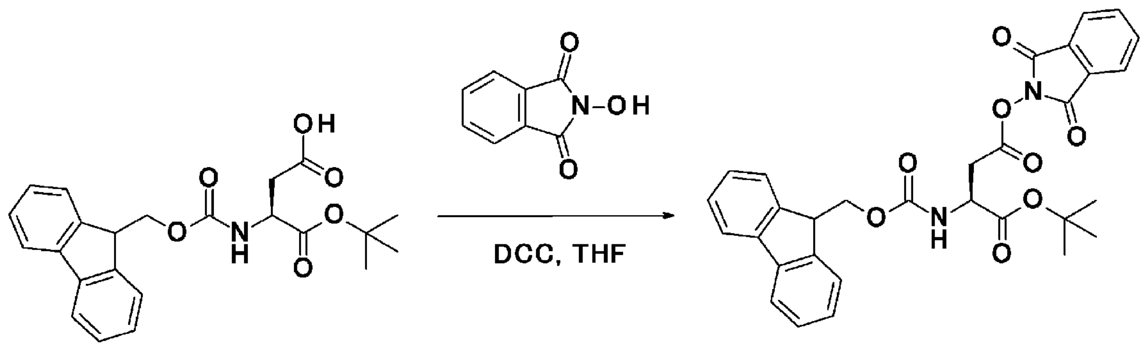


Fmoc-Asp -OtBu

aa130

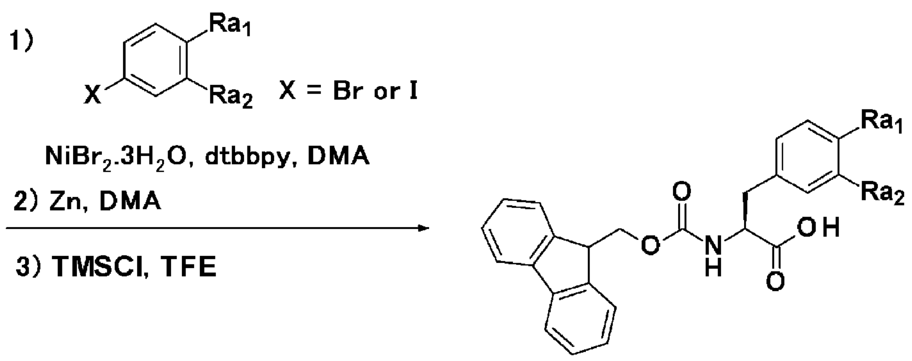


aa127~129

Synthesis of 1-(tert-butyl) 4-(1,3-dioxoisoindolin-2-yl) (((9H-fluoren-9-yl)methoxy)carbonyl)-L-aspartate (Compound aa130)

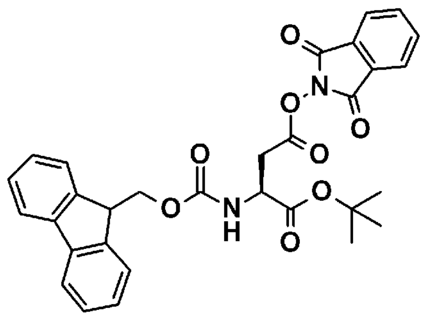

Under a nitrogen atmosphere, N,N'-dicyclohexylcarbodiimide (DCC) (181.8 g, 1.10 mmol) was added dropwise at room temperature to a solution of commercially available (S)-3-((((9H-fluoren-9-yl)methoxy)carbonyl)amino)-4-(tert-butoxy)-4-oxobutanoic acid (Fmoc-Asp-OtBu) (300 g, 729.12 mmol) and N-hydroxyphthalimide (130.8 g, 801.8 mmol) in tetrahydrofuran (3000 mL), and the mixture was stirred for 3 h. Toluene was added to the residue obtained by concentrating the reaction solution under reduced pressure, the mixture was filtered, and then the resulting solids were washed with toluene. The residue obtained by concentrating the filtrate under reduced pressure was purified by normal phase column chromatography (hexane/ethyl acetate) to afford 1-(tert-butyl) 4-(1,3-dioxoisoindolin-2-yl) (((9H-fluoren-9-yl)methoxy)carbonyl)-L-aspartate (Compound aa130) (272 g, 67%).
LCMS (ESI) m/z = 574 (M+$NH_4$)+

Retention time: 1.00 min (Analytical condition SQD2FA05)

Synthesis of 4-iodo-2-methoxy-N-methylbenzamide (Compound aa131)

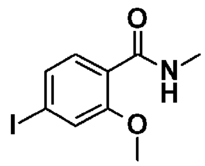

Under a nitrogen atmosphere, a 2 M solution of methylamine in tetrahydrofuran (THF) (22.5 mL, 45.0 mmol) and N,N-diisopropylethylamine (DIPEA) (12.53 mL, 94.942 mmol) were added at room temperature to a solution of commercially available 4-iodo-2-methoxybenzoic acid (5 g, 17.983 mmol), 1-(3-dimethylaminopropyl)-3-ethylcarbodiimide hydrochloride (EDCI·HCl) (4.15 g, 21.148 mmol), and 1-hydroxybenzotriazole (HOBt) (4.15 g, 21.148 mmol) in dimethylformamide (DMF) (50 mL), and the mixture was stirred for 16 h. Water was added to the reaction solution, the mixture was extracted with ethyl acetate three times, and then the resulting organic layer was washed with water, dried over anhydrous sodium sulfate, and filtered. The resulting filtrate was concentrated under reduced pressure, and the residue was purified by normal phase column chromatography (hexane/ethyl acetate) to afford 4-iodo-2-methoxy-N-methylbenzamide (Compound aa131) (3.3 g, 63 %).
LCMS (ESI) m/z = 292 (M+H)+

Retention time: 0.78 min (Analytical condition SMD method 50)

Synthesis of (S)-2-((((9H-fluoren-9-yl)methoxy)carbonyl)amino)-3-(3-methoxy-4-(methylcarbamoyl)phenyl)propanoic acid (Compound aa127, Fmoc-Phr(3-OMe-4-CONMe)-OH)

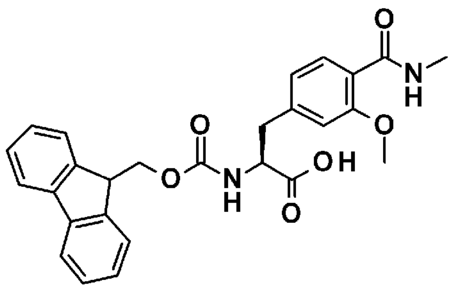

Under a nitrogen atmosphere, a solution of nickel(II) bromide trihydrate ($NiBr_2 \cdot 3H_2O$) (2.15 g, 7.59 mmol) and 4,4'-di-tert-butyl-2,2'-bipyridyl (dtbbpy) (2.15 g, 7.59 mmol) in dimethylacetamide (DMA) (120 mL) was stirred at 50°C for 3 h and then cooled to room temperature to afford a nickel catalyst solution. Under a nitrogen atmosphere, a solution of zinc powder (8.61 g, 134.5 mmol), 1-(tert-butyl) 4-(1,3-dioxoisoindolin-2-yl) (((9H-fluoren-9-yl)methoxy)carbonyl)-L-aspartate (Compound aa130) (14.6 g, 26.3 mmol) and 4-iodo-2-methoxy-N-methylbenzamide (Compound aa131) (11.5 g, 39.5 mmol) in dimethylacetamide (DMA) (120 mL) was stirred at room temperature for 30 min, then the above-described nickel catalyst solution was added dropwise at room temperature, and the mixture was stirred for 16 h. A 10% aqueous EDTA·2Na (disodium salt of ethylenediaminetetraacetic acid) solution was added to the reaction solution, and the mixture was extracted with ethyl acetate three times. The organic layer was washed with brine, and the residue obtained by evaporating the solvent under reduced pressure was purified by normal phase column chromatography (petroleum ether/ethyl acetate) to afford tert-butyl (S)-2-((((9H-fluoren-9-yl)methoxy)carbonyl)amino)-3-(3-methoxy-4-(methylcarbamoyl)phenyl)propanoate (Fmoc-Phe(3-OMe-4-CONMe)-OtBu) (9.2 g, 84%).

Under a nitrogen atmosphere, a solution of tert-butyl (S)-2-((((9H-fluoren-9-yl)methoxy)carbonyl)amino)-3-(3-methoxy-4-(methylcarbamoyl)phenyl)propanoate (Fmoc-Phe(3-OMe-4-CONMe)-OtBu) (11 g, 20.8 mmol) obtained as described above and chlorotrimethylsilane (TMSCl) (7.96 mL, 62.3 mmol) in 2,2,2-trifluoroethanol (TFE) (104 mL) was stirred at room temperature for 1 h. The reaction solution was concentrated under reduced pressure, and the resulting residue was purified by reverse phase column chromatography (water/acetonitrile) to afford (S)-2-((((9H-fluoren-9-yl)methoxy)carbonyl)amino)-3-(3-methoxy-4-(methylcarbamoyl)phenyl)propanoic acid (Compound aa127, Fmoc-Phr(3-OMe-4-CONMe)-OH) (5.0 g, 51%).
LCMS (ESI) m/z = 475 (M+H)+

Retention time: 0.68 min (Analytical condition SQDFA05)

Synthesis of 5-iodo-2-methoxy-N-methylbenzamide (Compound aa132)

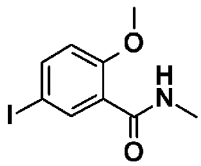

Under a nitrogen atmosphere, a tetrahydrofuran (THF) solution of 2 M methylamine (22.5 mL, 45.0 mmol) and N,N-diisopropylethylamine (DIPEA) (12.5 mL, 94.942 mmol) were added at room temperature to a solution of commercially available 5-iodo-2-methoxybenzoic acid (5 g, 17.983 mmol), 1-(3-dimethylaminopropyl)-3-ethylcarbodiimide hydrochloride (EDCI·HCl) (4.15 g, 21.148 mmol), and 1-hydroxybenzotriazole (HOBt) (4.15 g, 21.148 mmol) in dimethylformamide (DMF) (50 mL), and the mixture was stirred for 16 h. Water was added to the reaction solution, the mixture was extracted with ethyl acetate twice, and then the resulting organic layer was washed with water, dried over anhydrous sodium sulfate, and filtered. The resulting filtrate was concentrated under reduced pressure, and the residue was purified by normal phase column chromatography (petroleum ether/ethyl acetate) to afford 5-iodo-2-methoxy-N-methylbenzamide (Compound aa132) (3.3 g, 63%).
LCMS (ESI) m/z = 292 (M+H)+

Retention time: 0.79 min (Analytical condition SMD method 50)

Synthesis of (S)-2-((((9H-fluoren-9-yl)methoxy)carbonyl)amino)-3-(4-methoxy-3-(methylcarbamoyl)phenyl)propanoic acid (Compound aa128, Fmoc-Phe(4-OMe-3-CONMe)-OH)

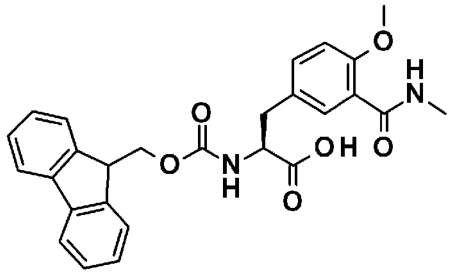

Under a nitrogen atmosphere, a solution of nickel(II) bromide trihydrate ($NiBr_2 \cdot 3H_2O$) (2.86 g, 10.50 mmol) and 4,4'-di-tert-butyl-2,2'-bipyridyl (dtbbpy) (2.86 g, 10.66 mmol) in dimethylacetamide (DMA) (161 mL) was stirred at 50°C for 3 h and then cooled to room temperature to afford a nickel catalyst solution. Under a nitrogen atmosphere, a solution of zinc powder (11.4 g, 174 mmol), 1-(tert-butyl) 4-(1,3-dioxoisoindolin-2-yl) (((9H-fluoren-9-yl)methoxy)carbonyl)-L-aspartate (Compound aa130) (19.74 g, 35.47 mmol) and 5-iodo-2-methoxy-N-methylbenzamide (Compound aa132) (15.5 g, 53.2 mmol) in dimethylacetamide (DMA) (161 mL) was stirred at room temperature for 30 min, then the above-described nickel catalyst solution was added dropwise at room temperature, and the mixture was stirred for 16 h. A 10% aqueous EDTA·2Na (disodium salt of ethylenediaminetetraacetic acid) solution was added to the reaction solution, the mixture was filtered through Celite, and then the organic layer of the resulting filtrate was separated, washed with brine, and dried over anhydrous sodium sulfate. After the mixture was filtered, the residue obtained by evaporating the solvent from the filtrate under reduced pressure was purified by normal phase column chromatography (petroleum ether/ethyl acetate) to afford tert-butyl (S)-2-((((9H-fluoren-9-yl)methoxy)carbonyl)amino)-3-(4-methoxy-3-(methylcarbamoyl)phenyl)propanoate (Fmoc-Phe(4-OMe-3-CONMe)-OtBu) (10.2 g, 36%).

Under a nitrogen atmosphere, a solution of tert-butyl (S)-2-((((9H-fluoren-9-yl)methoxy)carbonyl)amino)-3-(4-methoxy-3-(methylcarbamoyl)phenyl)propanoate (Fmoc-Phe(4-OMe-3-CONMe)-OtBu) (10.2 g, 19.22 mmol) obtained as described above and chlorotrimethylsilane (TMSCl) (7.26 mL, 56.8 mmol) in 2,2,2-trifluoroethanol (TFE) (96 mL) was stirred at room temperature for 1 h. The reaction solution was concentrated under reduced pressure, and the resulting residue was purified by reverse phase column chromatography (water/acetonitrile) to afford (S)-2-((((9H-fluoren-9-yl)methoxy)carbonyl)amino)-3-(4-methoxy-3-(methylcarbamoyl)phenyl)propanoic acid (Compound aa128, Fmoc-Phe(4-OMe-3-CONMe)-OH) (6.12 g, 36%).
LCMS (ESI) m/z = 475 (M+H)+

Retention time: 0.71 min (Analytical condition SQDFA05)

Synthesis of (S)-2-((((9H-fluoren-9-yl)methoxy)carbonyl)amino)-3-(3-methoxy-4-((methylsulfonyl)carbamoyl)phenyl)propanoic acid (Compound aa129, Fmoc-Phe(3-OMe-4-CONHMs)-OH)

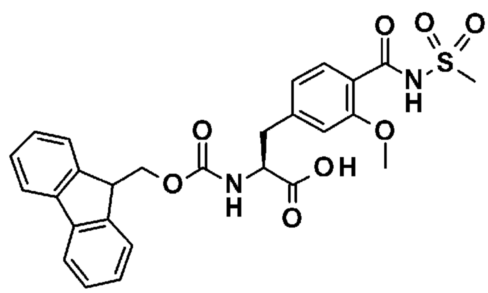

Under a nitrogen atmosphere, carbonyldiimidazole (CDI) (47.37 g, 292.151 mmol) was added at room temperature to a solution of commercially available 4-bromo-2-methoxybenzoic acid (45 g, 194.767 mmol) in dimethylformamide (DMF) (220 mL), and the mixture was stirred for 2 h. Methanesulfonamide (55.58 g, 584.315 mmol) and 1,8-diazabicyclo[5.4.0]-7-undecene (DBU) (88.95 g, 584.302 mmol) were added to the reaction solution, the mixture was stirred for 16 h, and then ethyl acetate was added. After a 2N aqueous hydrochloric acid solution was added to the mixture until pH 5, the resulting organic layer was washed with water and with brine, dried over anhydrous sodium sulfate, and filtered. The resulting filtrate was concentrated under reduced pressure, and the residue was purified by normal phase column chromatography (petroleum ether/ethyl acetate) to afford 4-bromo-2-methoxy-N-(methylsulfonyl)benzamide (57.5 g, 95%).

Under a nitrogen atmosphere, a solution of nickel(II) bromide trihydrate ($NiBr_2 \cdot 3H_2O$) (2.93 g, 10.78 mmol) and 4,4'-di-tert-butyl-2,2'-bipyridyl (dtbbpy) (2.89 g, 10.78 mmol) in dimethylacetamide (DMA) (100 mL) was stirred at 50°C for 3 h and then cooled to room temperature to afford a nickel catalyst solution. Under a nitrogen atmosphere, a solution of zinc powder (11.75 g, 179.672 mmol), 1-(tert-butyl) 4-(1,3-dioxoisoindolin-2-yl) (((9H-fluoren-9-yl)methoxy)carbonyl)-L-aspartate (Compound aa130) (20.0 g, 35.934 mmol) and 4-bromo-2-methoxy-N-(methylsulfonyl)benzamide (33.22 g, 107.803 mmol) in dimethylacetamide (DMA) (100 mL) was stirred at room temperature, then the above-described nickel catalyst solution was added dropwise at room temperature, and the mixture was stirred for 16 h. A 10% aqueous EDTA·2Na (disodium salt of ethylenediaminetetraacetic acid) solution was added to the reaction solution, and the mixture was extracted with ethyl acetate three times. The resulting organic layer was washed with brine, dried over anhydrous sodium sulfate, then filtered, and concentrated under reduced pressure, and the residue thus obtained was purified by normal phase column chromatography (petroleum ether/ethyl acetate) to afford tert-butyl (S)-2-((((9H-fluoren-9-yl)methoxy)carbonyl)amino)-3-(3-methoxy-4-((methylsulfonyl)carbamoyl)phenyl)propanoate (Fmoc-Phe(3-OMe-4-CONHMs)-OtBu) (5.05 g, 19%).

Under a nitrogen atmosphere, a solution of tert-butyl (S)-2-((((9H-fluoren-9-yl)methoxy)carbonyl)amino)-3-(3-methoxy-4-((methylsulfonyl)carbamoyl)phenyl)propanoate (Fmoc-Phe(3-OMe-4-CONHMs)-OtBu) (10.1 g, 16.984 mmol) obtained as described above and chlorotrimethylsilane (TMSCl) (5.54 g, 50.952 mmol) in 2,2,2-trifluoroethanol (TFE) (100 mL) was stirred at room temperature for 1 h. The reaction solution was concentrated under reduced pressure, and the resulting residue was purified by reverse phase column chromatography (water/acetonitrile) to afford (S)-2-((((9H-fluoren-9-yl)methoxy)carbonyl)amino)-3-(3-methoxy-4-((methylsulfonyl)carbamoyl)phenyl)propanoic acid (Compound aa129, Fmoc-Phe(3-OMe-4-CONHMs)-OH) (5.06 g, 55%).
LCMS (ESI) m/z = 539 (M+H)+

Retention time: 0.72 min (Analytical condition SQDFA05)

Synthesis of (2S,4R)-1-(((9H-fluoren-9-yl)methoxy)carbonyl)-4-(2-((tetrahydro-2H-pyran-2-yl)oxy)ethoxy)pyrrolidine-2-carboxylic acid (Compound aa133, Fmoc-Hyp(2-EtOTHP)-OH)

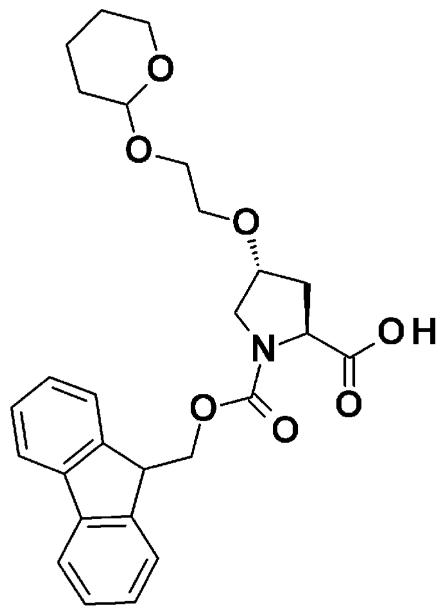

Under a nitrogen atmosphere, sodium hydride (NaH) (18.1 g, 451.9 mmol, 60 wt%) was added in small portions to a solution of commercially available (2S,4R)-1-((benzyloxy)carbonyl)-4-hydroxypyrrolidine-2-carboxylic acid (Cbz-Hyp-OH) (12.0 g, 45.2 mmol) in dimethylformamide (120 mL) at -10°C, and the mixture was stirred at -10°C for 1 h. 2-(2-Bromoethoxy)tetrahydro-2H-pyran (28.3 g, 135.7 mmol) was added dropwise to the reaction solution at -10°C, and the reaction solution was stirred at 30°C for 3 h. The reaction was quenched with an aqueous sodium hydrogen carbonate solution (120 mL), and ethyl acetate (120 mL) was added for washing. After a 1N aqueous hydrochloric acid solution was added to the resulting aqueous layer until pH = 6, the mixture was extracted with ethyl acetate (120 mL) twice. The resulting organic layer was washed with brine and with a 5% aqueous sodium thiosulfate solution, dried over anhydrous sodium sulfate, and filtered. The resulting filtrate was concentrated under reduced pressure to afford (2S,4R)-1-((benzyloxy)carbonyl)-4-(2-((tetrahydro-2H-pyran-2-yl)oxy)ethoxy)pyrrolidine-2-carboxylic acid (Cbz-Hyp(2-EtOTHP)-OH) (10.4 g) as a crude product.

Under a hydrogen atmosphere, a solution of the resulting crude product (2S,4R)-1-((benzyloxy)carbonyl)-4-(2-((tetrahydro-2H-pyran-2-yl)oxy)ethoxy)pyrrolidine-2-carboxylic acid (Cbz-Hyp(2-EtOTHP)-OH) (10.1 g) and palladium on carbon (Pd/C) (3.0 g) in methanol (100 mL) was stirred at room temperature for 2 h. After the reaction solution was filtered, the resulting solids were washed with methanol (100 mL). The residue obtained by concentrating the filtrate under reduced pressure was washed with ether to afford (2S,4R)-4-(2-((tetrahydro-2H-pyran-2-yl)oxy)ethoxy)pyrrolidine-2-carboxylic acid (H-Hyp(2-EtOTHP)-OH) (6.1 g) as a crude product.

N-(9-Fluorenylmethoxycarbonyloxy)succinimide (Fmoc-OSu) (7.9 g, 23.5 mmol) was added to a solution of the resulting crude product (2S,4R)-4-(2-((tetrahydro-2H-pyran-2-yl)oxy)ethoxy)pyrrolidine-2-carboxylic acid (H-Hyp(2-EtOTHP)-OH) (6.1 g) and sodium hydrogen carbonate (5.9 g, 70.5 mmol) in 1,4-dioxane/water = 1/1 (130 mL), and the mixture was stirred at room temperature for 2 h. After the operation of adding a t-butyl methyl ether (TBME)/hexane = 1/3 solution (100 mL) to the reaction solution to wash the reaction solution therewith was performed three times, a 1N aqueous hydrochloric acid solution was added to the resulting aqueous layer until pH = 6, and the mixture was extracted with t-butyl methyl ether (TBME) (100 mL). The resulting organic layer was washed with brine, then dried over anhydrous sodium sulfate, and filtered. The resulting filtrate was concentrated under reduced pressure, and the residue was purified by reverse phase column chromatography (water/acetonitrile) to afford (2S,4R)-1-(((9H-fluoren-9-yl)methoxy)carbonyl)-4-(2-((tetrahydro-2H-pyran-2-yl)oxy)ethoxy)pyrrolidine-2-carboxylic acid (Compound aa133, Fmoc-Hyp(2-EtOTHP)-OH) (1.88 g, 30% over three steps).
LCMS (ESI) m/z = 482 (M+H)+

Retention time: 0.80 min (Analytical condition SQDFA05)

Synthesis of N-(((9H-fluoren-9-yl)methoxy)carbonyl)-N-(3-methoxy-4-(methylcarbamoyl)phenethyl)glycine (Compound aa134, Fmoc-(Ph(3-OMe-4-CONMe)Et)Gly-OH)

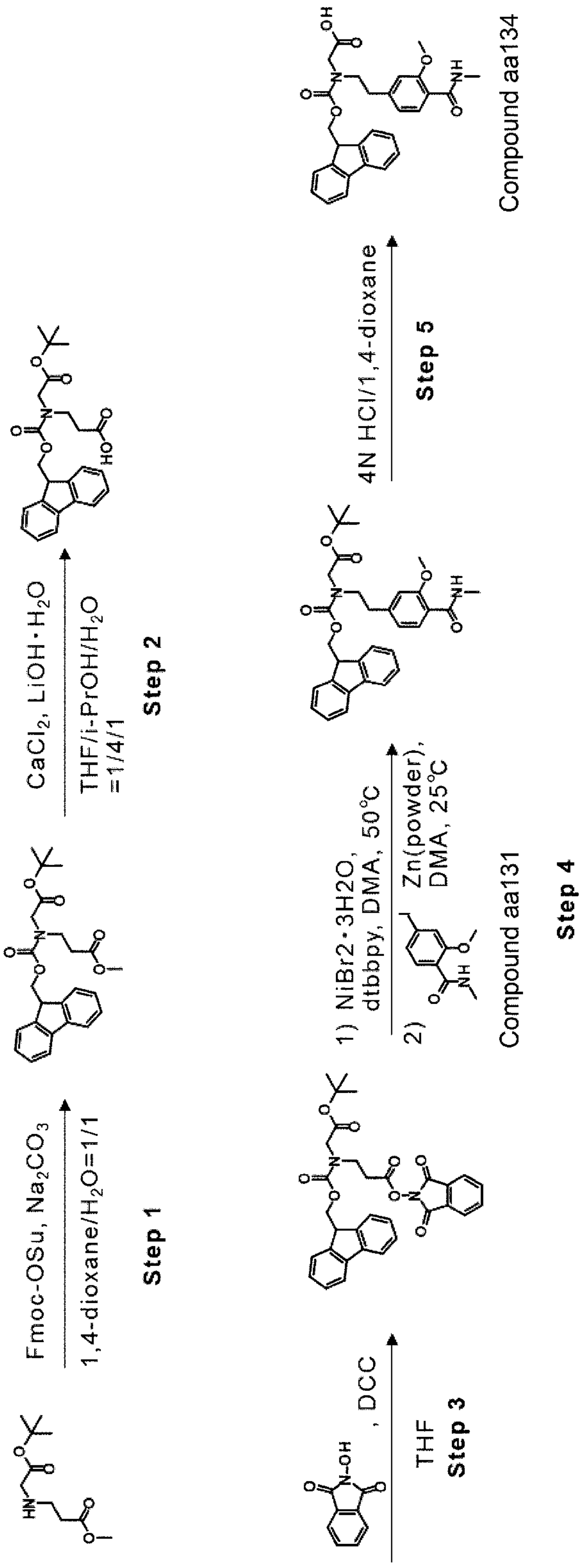


Step 1

An aqueous solution (200 mL) of sodium carbonate (30.25 g, 285.366 mmol) was added at room temperature to a solution of methyl 3-((2-(tert-butoxy)-2-oxoethyl)amino)propanoate (31.0 g, 142.683 mmol) prepared by the method described in a literature (Journal of Medicinal Chemistry (1991), 34(4), 1297-1301) and N-(9-fluorenylmethoxycarbonyloxy)succinimide (Fmoc-OSu) (48.13 g, 142.683 mmol) in 1,4-dioxane (200 mL), and the mixture was stirred for 16 h. Acetonitrile was added to the reaction solution, the mixture was filtered, and then the filtrate was concentrated under

reduced pressure. Ethyl acetate was added to the resulting residue, and the mixture was washed with brine, then dried over anhydrous sodium sulfate, and filtered. The resulting filtrate was concentrated under reduced pressure to afford methyl 3-((((9H-fluoren-9-yl)methoxy)carbonyl)(2-(tert-butoxy)-2-oxoethyl)amino)propanoate (62 g, 36%) as a crude product.

Step 2

After water (250 mL) and isopropanol (1000 mL) were added to calcium chloride (84.54 g, 761.761 mmol) and lithium hydroxide monohydrate (8.52 g, 203.136 mmol), a solution of the above-described crude product methyl 3-((((9H-fluoren-9-yl)methoxy)carbonyl)(2-(tert-butoxy)-2-oxoethyl)amino)propanoate (62 g, 50.784 mmol) in tetrahydrofuran (250 mL) was added at 0°C, and the mixture was stirred for 16 h. After the reaction solution was concentrated under reduced pressure, water was added to the resulting residue, and the mixture was washed with t-butyl methyl ether (TBME). After a 1N aqueous hydrochloric acid solution was added to the resulting aqueous layer until the pH of the solution was acidified, the mixture was extracted with ethyl acetate. The resulting organic layer was washed with water and with brine, dried over anhydrous sodium sulfate, and filtered. The resulting filtrate was concentrated under reduced pressure to afford 3-((((9H-fluoren-9-yl)methoxy)carbonyl)(2-(tert-butoxy)-2-oxoethyl)amino)propanoic acid (14 g, 58%) as a crude product.

Step 3

Under a nitrogen atmosphere, a solution of the above-described crude product 3-((((9H-fluoren-9-yl)methoxy)carbonyl) (2-(tert-butoxy)-2-oxoethyl)amino)propanoic acid (14 g, 32.904 mmol), N-hydroxyphthalimide (5.90 g, 36.194 mmol) and N,N'-dicyclohexylcarbodiimide (DCC) (7.47 g, 36.194 mmol) in tetrahydrofuran (140 mL) was stirred at room temperature for 16 h. After the reaction solution was filtered, ethyl acetate was added to the residue obtained by concentrating the filtrate under reduced pressure, and the mixture was washed with brine, then dried over anhydrous sodium sulfate, and filtered. The resulting filtrate was concentrated under reduced pressure to afford 1,3-dioxoisoindolin-2-yl 3-((((9H-fluoren-9-yl)methoxy)carbonyl)(2-(tert-butoxy)-2-oxoethyl)amino)propanoate (14 g, 75%) as a crude product.

Step 4

Under a nitrogen atmosphere, a solution of nickel(II) bromide trihydrate ($NiBr_2 \cdot 3H_2O$) (5.73 g, 21.031 mmol) and 4,4'-di-tert-butyl-2,2'-bipyridyl (dtbbpy) (5.64 g, 21.031 mmol) in dimethylacetamide (DMA) (200 mL) was stirred at 50°C for 3 h and then cooled to room temperature to afford a nickel catalyst solution. Under a nitrogen atmosphere, a solution of zinc powder (22.93 g, 350.509 mmol), the crude product 1,3-dioxoisoindolin-2-yl 3-((((9H-fluoren-9-yl)methoxy)carbonyl) (2-(tert-butoxy)-2-oxoethyl)amino)propanoate (40.0 g, 70.102 mmol) obtained as described above and 4-iodo-2-methoxy-N-methylbenzamide (Compound aa131) (40.81 g, 140.204 mmol) synthesized as described in the Examples in dimethylacetamide (DMA) (200 mL) was stirred at room temperature for 10 min, then the above-described nickel catalyst solution was added dropwise at room temperature, and the mixture was stirred for 16 h. A 10% aqueous EDTA·2Na (disodium salt of ethylenediaminetetraacetic acid) solution was added to the reaction solution, and the mixture was filtered and then extracted with ethyl acetate three times. After the organic layer was washed with brine, dried over anhydrous sodium sulfate, and filtered, the resulting filtrate was concentrated under reduced pressure. The residue was purified by normal phase column chromatography to afford tert-butyl N-(((9H-fluoren-9-yl)methoxy)carbonyl)-N-(3-methoxy-4-(methylcarbamoyl)phenethyl)glycinate (14 g, 37%).

Step 5

A solution of the above-described tert-butyl N-(((9H-fluoren-9-yl)methoxy)carbonyl)-N-(3-methoxy-4-(methylcarbamoyl)phenethyl)glycinate (14.0 g, 25.705 mmol) in 4N hydrochloric acid/1,4-dioxane (65 mL) was stirred at room temperature for 48 h. After the reaction solution was concentrated under reduced pressure, the resulting residue was purified by reverse phase column chromatography (water/acetonitrile) to afford N-(((9H-fluoren-9-yl)methoxy)carbonyl)-N-(3-methoxy-4-(methylcarbamoyl)phenethyl)glycine (Compound aa134, Fmoc-(Ph(3-OMe-4-CONMe)Et)Gly-OH)(6 g, 47%).
LCMS (ESI) m/z = 489 (M+H)+

Retention time: 0.70 min (Analytical condition SQDFA05)

Synthesis of (S)-2-((((9H-fluoren-9-yl)methoxy)carbonyl)amino)-4-(3-methoxy-4-(methylcarbamoyl)phenyl)butanoic acid (Compound aa135, Fmoc-Hph(3-OMe-4-CONMe)-OH)

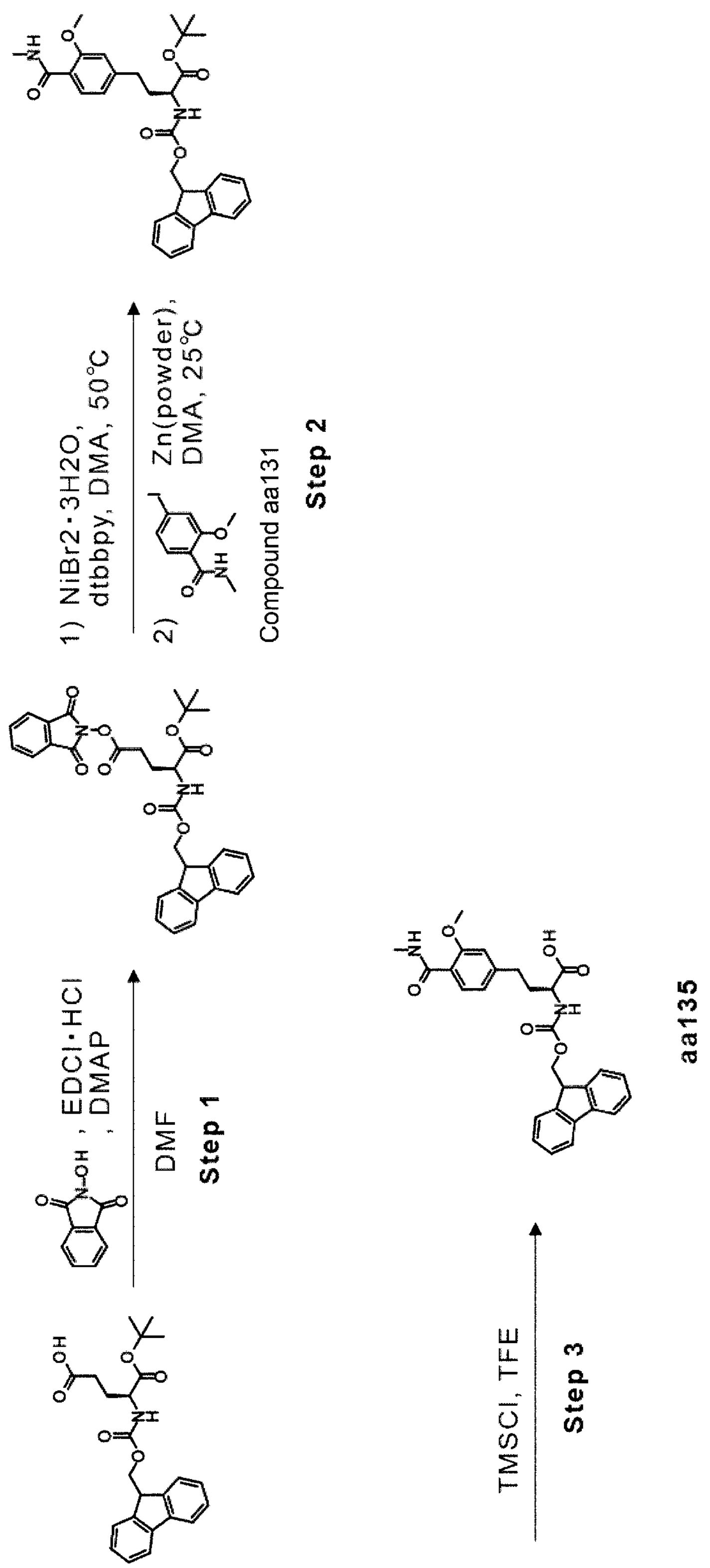

Step 1

Under a nitrogen atmosphere, a solution of commercially available (S)-4-((((9H-fluoren-9-yl)methoxy)carbonyl)amino)-5-(tert-butoxy)-5-oxopentanoic acid (Fmoc-Glu-OtBu) (175 g, 411.299 mmol), N-hydroxyphthalimide (67.1 g, 411.299 mmol), 1-ethyl-3-(3-dimethylaminopropyl)carbodiimide hydrochloride (EDCI·HCl) (78.85 g, 411.299 mmol), and N,N-dimethyl-4-aminopyridine (DMAP) (2.51 g, 20.565 mmol) in dimethylformamide (2000 mL) was stirred at room temperature for 16 h. After a 1 M aqueous hydrochloric acid solution was added to the reaction solution, the mixture was extracted with t-butyl methyl ether (TBME). After the resulting organic layer was washed with water, with a 50% saturated aqueous sodium hydrogen carbonate solution, and with brine, dried over anhydrous sodium sulfate, and filtered, the resulting filtrate was concentrated under reduced pressure. The residue was purified by normal phase column chromatography (petroleum ether/ethyl acetate) to afford 1-(tert-butyl) 5-(1,3-dioxoisoindolin-2-yl) (((9H-fluoren-9-yl)methoxy)carbonyl)-L-glutamate (105 g, 45%).

Step 2

Under a nitrogen atmosphere, a solution of nickel(II) bromide trihydrate ($NiBr_2 \cdot 3H_2O$) (141 mg, 0.526 mmol) and 4,4'-di-tert-butyl-2,2'-bipyridyl (dtbbpy) (140 mg, 0.526 mmol) in dimethylacetamide (DMA) (5 mL) was stirred at 50°C for 3 h and then cooled to room temperature to afford a nickel catalyst solution. Under a nitrogen atmosphere, a solution of zinc powder (617 mg, 8.75 mmol), 1-(tert-butyl) 5-(1,3-dioxoisoindolin-2-yl) (((9H-fluoren-9-yl)methoxy)carbonyl)-L-glutamate (1.0 g, 1.75 mmol) obtained as described above and 4-iodo-2-methoxy-N-methylbenzamide (Compound aa131) (1.19 g, 3.50 mmol) synthesized as described in the Examples in dimethylacetamide (DMA) (5 mL) was stirred at room temperature for 1 h, then the above-described nickel catalyst solution was added dropwise at room temperature, and the mixture was stirred for 16 h. A 10% aqueous EDTA·2Na (disodium salt of ethylenediaminetetraacetic acid) solution was added to the reaction solution, and the mixture was filtered and then extracted with ethyl acetate three times. After the organic layer was washed with brine, dried over anhydrous sodium sulfate, and filtered, the resulting filtrate was concentrated under reduced pressure. The residue was purified by normal phase column chromatography (petroleum ether/ethyl acetate) to afford tert-butyl (S)-2-((((9H-fluoren-9-yl)methoxy)carbonyl)amino)-4-(3 -methoxy-4-(methylcarbamoyl)phenyl)butanoate (550 mg, 57%).

Step 3

Chlorotrimethylsilane (TMS-Cl) (1.80 g, 16.568 mmol) was added to a solution of tert-butyl (S)-2-((((9H-fluoren-9-yl)methoxy)carbonyl)amino)-4-(3-methoxy-4-(methylcarbamoyl)phenyl)butanoate (3.00 g, 5.508 mmol) obtained as described above in trifluoroethanol (TFE) (30 mL) at room temperature, and the mixture was stirred for 3 h. After the reaction solution was concentrated under reduced pressure, the resulting residue was purified by ethyl acetate to afford (S)-2-((((9H-fluoren-9-yl)methoxy)carbonyl)amino)-4-(3-methoxy-4-(methylcarbamoyl)phenyl)butanoic acid (Compound aa135, Fmoc-Hph(3-OMe-4-CONMe)-OH) (1.9 g, 70%).
LCMS (ESI) m/z = 489 (M+H)+

Retention time: 0.71 min (Analytical condition SQDFA05)

Synthesis of (R)-2-((((9H-fluoren-9-yl)methoxy)carbonyl)amino)-4-(3-methoxy-4-(methylcarbamoyl)phenyl)butanoic acid (Compound aa136, Fmoc-D-Hph(3-OMe-4-CONMe)-OH)

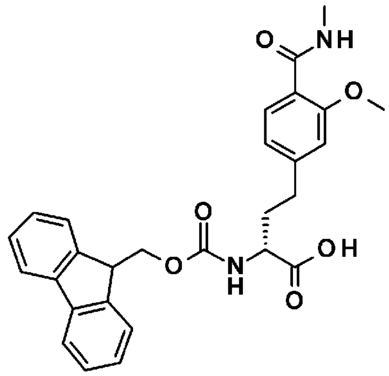

Using commercially available (R)-4-((((9H-fluoren-9-yl)methoxy)carbonyl)amino)-5-(tert-butoxy)-5-oxopentanoic acid (Fmoc-D-Glu-OtBu), (R)-2-((((9H-fluoren-9-yl)methoxy)carbonyl)amino)-4-(3-methoxy-4-(methylcarbamoyl)phenyl)butanoic acid (Compound aa136, Fmoc-D-Hph(3-OMe-4-CONMe)-OH) (5 g, 20% over three steps) was synthesized by the similar method as the synthesis of Compound aa135.
LCMS (ESI) m/z = 487 (M-H)-

Retention time: 0.83 min (Analytical condition SQDFA05)

Synthesis of (S)-2-((((9H-fluoren-9-yl)methoxy)carbonyl)(methyl)amino)-4-(3-methoxy-4-(methylcarbamoyl)-phenyl)butanoic acid (Compound aa137, Fmoc-MeHph(3-OMe-4-CONMe)-OH)

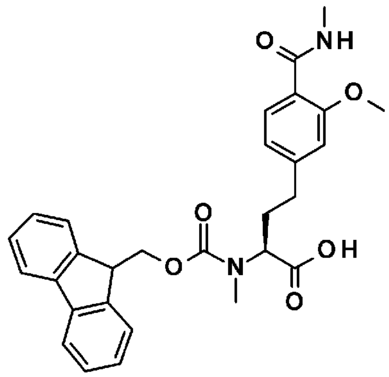

Using separately synthesized (S)-4-((((9H-fluoren-9-yl)methoxy)carbonyl)(methyl)amino)-5-(tert-butoxy)-5-oxopentanoic acid (Compound aa138, Fmoc-MeGlu-OtBu), (S)-2-((((9H-fluoren-9-yl)methoxy)carbonyl)(methyl)amino)-4-(3-methoxy-4-(methylcarbamoyl)phenyl)butanoic acid (Compound aa137, Fmoc-MeHph(3-OMe-4-CONMe)-OH) (2.8 g, 20% over three steps) was synthesized by the similar method as the synthesis of Compound aa135.
LCMS (ESI) m/z = 503 (M+H)+

Retention time: 0.73 min (Analytical condition SQDFA05)

Synthesis of (S)-4-((((9H-fluoren-9-yl)methoxy)carbonyl)(methyl)amino)-5-(tert-butoxy)-5-oxopentanoic acid (Compound aa138, Fmoc-MeGlu-OtBu)

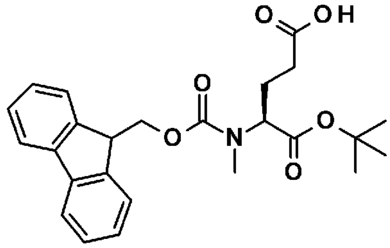

Under a nitrogen atmosphere, a solution of commercially available (S)-2-((((9H-fluoren-9-yl)methoxy)carbonyl)amino)-5-(allyloxy)-5-oxopentanoic acid (Fmoc-Glu(OAll)-OH) (100 g, 244.237 mmol), paraformaldehyde (22 g, 733.333 mmol), and p-toluenesulfonic acid (TsOH) (0.42 g, 2.438 mmol) in toluene (1000 mL) was stirred at 110°C for 1.5 h. The reaction solution was cooled at room temperature and concentrated under reduced pressure, ethyl acetate was added to the residue thus obtained, and the mixture was washed with an aqueous sodium hydrogen carbonate solution and with brine. The organic layer was dried over anhydrous sodium sulfate, filtered, and then concentrated under reduced pressure to afford (9H-fluoren-9-yl)methyl (S)-4-(3-(allyloxy)-3-oxopropyl)-5-oxooxazolidine-3-carboxylate (110 g) as a crude product.

Under a nitrogen atmosphere, a boron trifluoride-diethyl ether complex ($BF_3 \cdot Et_2O$) (74.2 g, 522.565 mmol) was added at 0°C to a solution of (9H-fluoren-9-yl)methyl (S)-4-(3-(allyloxy)-3-oxopropyl)-5-oxooxazolidine-3-carboxylate (110 g) obtained as described above as a crude product, triethylsilane ($Et_3SiH$) (60.6 g, 522.565 mmol), and water (4.7 mL, 264.282 mmol) in dichloromethane (1100 mL), and the mixture was stirred for 16 h. An aqueous ammonium chloride solution was added to the reaction solution, the mixture was extracted with ethyl acetate twice, and then the resulting organic layer was concentrated under reduced pressure. After an aqueous sodium hydrogen carbonate solution was added to dissolve the residue, the solution was washed with hexane/ethyl acetate (5/1) three times. A 6N aqueous hydrochloric acid solution was added to the aqueous layer until pH = 2, and the mixture was extracted with ethyl acetate twice. After the organic layer was washed with water and with brine, dried over anhydrous sodium sulfate and filtered, the filtrate was concentrated under reduced pressure. The resulting residue was recrystallized from a solution of 1% trifluoroacetic acid (TFA) in acetonitrile/water (1/5) to afford (S)-2-((((9H-fluoren-9-yl)methoxy)carbonyl)(methyl)amino)-5-(allyloxy)-5-oxopentanoic acid (Fmoc-MeGlu(OAll)-OH) (95 g, 86% over two steps).

Under a nitrogen atmosphere, a solution of tert-butyl 2,2,2-trichloroacetimidate (51 g, 472.813 mmol) in cyclohexane was added at 0°C to a solution of (S)-2-((((9H-fluoren-9-yl)methoxy)carbonyl)(methyl)amino)-5-(allyloxy)-5-oxopentanoic acid (Fmoc-MeGlu(OAll)-OH) (100 g, 236.406 mmol) obtained as described above and a boron trifluoride-diethyl ether complex ($BF_3 \cdot OEt_2$) (0.16 g, 2.364 mmol) in dichloromethane (260 mL), and then the mixture was stirred for 1 h. Pyridine was added to the reaction solution, the mixture was filtered, and then the filtrate was washed with an aqueous sodium hydrogen carbonate solution and with brine, dried over anhydrous sodium sulfate, and filtered. The residue obtained by concentrating the filtrate under reduced pressure was recrystallized from an acetonitrile/water (1/5) solution to afford 5-allyl 1-(tert-butyl) N-(((9H-fluoren-9-yl)methoxy)carbonyl)-N-methyl-L-glutamate (102 g) quantitatively.

Under a nitrogen atmosphere, phenylsilane ($PhSiH_3$) (45.77 g, 423.799 mmol) was added dropwise at room temperature to a solution of 5-allyl 1-(tert-butyl) N-(((9H-fluoren-9-yl)methoxy)carbonyl)-N-methyl-L-glutamate (290 g, 605.427 mmol) obtained as described above and tetrakis(triphenylphosphine)palladium(0) ($Pd(PPh_3)_4$) (7.0 g, 6.054 mmol) in dichloromethane (1450 mL), and the mixture was stirred. After the disappearance of raw materials was confirmed by LC-MS, the reaction solution was filtered, and the resulting filtrate was concentrated under reduced pressure. After the residue was dissolved in an aqueous sodium carbonate solution, the solution was washed with a hexane/t-butyl methyl ether (1/3) solution three times. After phosphoric acid was added to the aqueous layer until pH = 2, the mixture was extracted twice with t-butyl methyl ether. After the organic layer was washed with brine, dried over anhydrous sodium sulfate, and filtered, the resulting filtrate was concentrated under reduced pressure to afford (S)-4-((((9H-fluoren-9-yl)methoxy)carbonyl)(methyl)amino)-5-(tert-butoxy)-5-oxopentanoic acid (Compound aa138, Fmoc-MeGlu-OtBu) (247 g, 93%).

LCMS (ESI) m/z = 462 (M+Na)+

Retention time: 1.380 min (Analytical condition SMD method 52)

(2S)-2-((((9H-Fluoren-9-yl)methoxy)carbonyl)amino)-3-(4-(methyl((tetrahydro-2H-pyran-2-yl)oxy)carbamoyl)phenyl)propanoic acid (Compound aa139, Fmoc-Phe(4-CONMeOTHP)-OH) and (2S)-2-((((9H-fluoren-9-yl)methoxy)carbonyl)amino)-3-(3-methoxy-4-(((tetrahydro-2H-pyran-2-yl)oxy)carbamoyl)phenyl)propanoic acid (Compound aa140, Fmoc-Phe(3-OMe-4-CONHOTHP)-OH) were synthesized according to the following scheme.

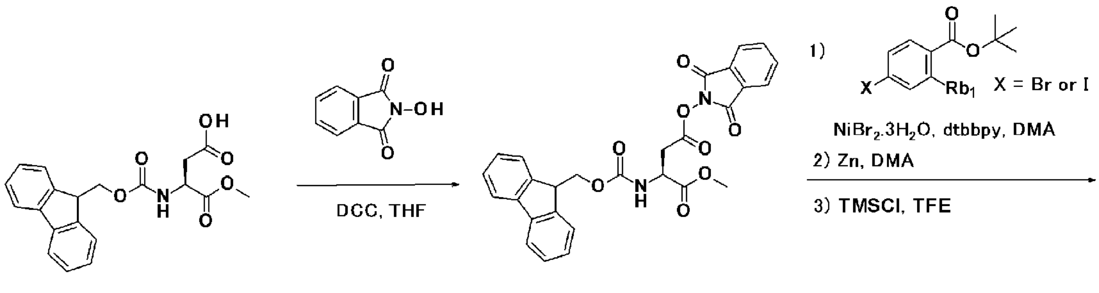


Fmoc-Asp -OMe

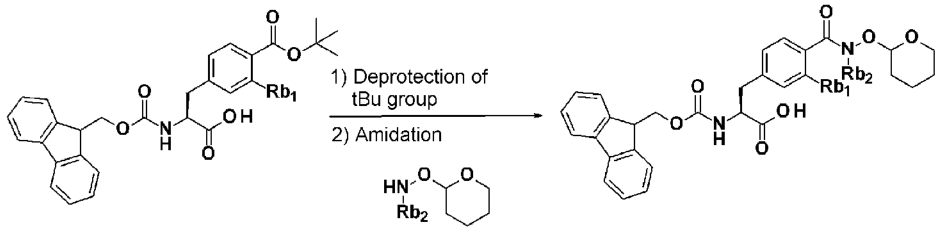


Synthesis of 1-methyl 4-(1,3-dioxoisoindolin-2-yl) (((9H-fluoren-9-yl)methoxy)carbonyl)-L-aspartate

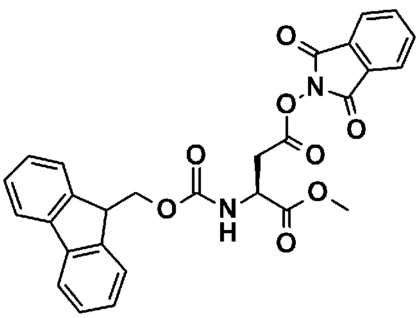

Under a nitrogen atmosphere, a solution of commercially available (S)-3-((((9H-fluoren-9-yl)methoxy)carbonyl)amino)-4-methoxy-4-oxobutanoic acid (Fmoc-Asp-OMe) (15.0 g, 40.6 mmol), N-hydroxyphthalimide (7.3 g, 44.7 mmol) and N,N'-dicyclohexylcarbodiimide (DCC) (8.4 g, 40.6 mmol) in tetrahydrofuran (225 mL) was stirred at room temperature at 25°C for 2 h. Ethyl acetate was added to the residue obtained by concentrating the reaction solution under reduced pressure, the mixture was filtered, and then the resulting solids were washed with ethyl acetate. Ethyl acetate (1.5 L) was added to the residue obtained by concentrating the filtrate under reduced pressure, and the mixture was stirred at 50°C for 16 h. After being cooled to room temperature, the mixture was filtered, solids were washed with ethyl acetate, and then the filtrate was concentrated under reduced pressure to afford 1-methyl 4-(1,3-dioxoisoindolin-2-yl) (((9H-fluoren-9-yl)methoxy)carbonyl)-L-aspartate (10.4 g) as a crude product.

Synthesis of tert-butyl (S)-4-(2-((((9H-fluoren-9-yl)methoxy)carbonyl)amino)-3-methoxy-3-oxopropyl)benzoate (Fmoc-Phe(4-COtBu)-OMe)

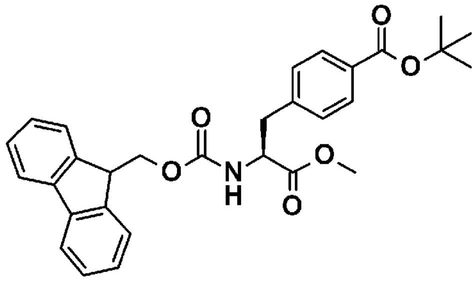

Under a nitrogen atmosphere, a solution of nickel(II) bromide trihydrate ($NiBr_2 \cdot 3H_2O$) (1.649 g, 6.064 mmol) and 4,4'-di-tert-butyl-2,2'-bipyridyl (dtbbpy) (1.627 g, 6.064 mmol) in dimethylacetamide (DMA) (50 mL) was stirred at 50°C for 3 h and then cooled to room temperature to afford a nickel catalyst solution. Under a nitrogen atmosphere, tert-butyl 4-bromobenzoate (10.4 g, 40.447 mmol) was added dropwise at 0°C to a solution of zinc powder (6.61 g, 101.071 mmol) and 1-methyl 4-(1,3-dioxoisoindolin-2-yl) (((9H-fluoren-9-yl)methoxy)carbonyl)-L-aspartate (10.4 g, 20.214 mmol) in dimethylacetamide (DMA) (50 mL), and the mixture was stirred at room temperature for 16 h. A 10% aqueous EDTA·2Na (disodium salt of ethylenediaminetetraacetic acid) solution was added to the reaction solution at 0°C, the mixture was filtered, solids were washed with ethyl acetate, and then the filtrate was extracted with ethyl acetate. The organic layer was washed with brine, dried over anhydrous sodium sulfate, and filtered. The residue obtained by concentrating the filtrate under reduced pressure was purified by normal phase column chromatography (petroleum ether/ethyl acetate) to afford tert-butyl (S)-4-(2-((((9H-fluoren-9-yl)methoxy)carbonyl)amino)-3-methoxy-3-oxopropyl)benzoate (Fmoc-Phe(4-COtBu)-OMe) (11.9 g, 76%).

Synthesis of (S)-4-(2-((((9H-fluoren-9-yl)methoxy)carbonyl)amino)-3-methoxy-3-oxopropyl)benzoic acid (Fmoc-Phe(4-COOH)-OMe)

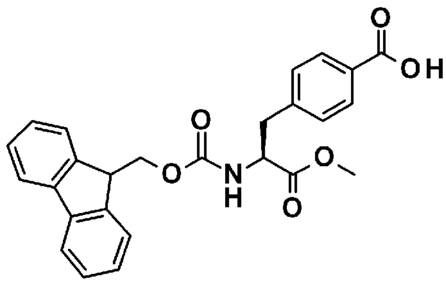

Under a nitrogen atmosphere, trifluoroacetic acid (TFA) (71.4 mL) was added to a solution of the above-described tert-butyl (S)-4-(2-((((9H-fluoren-9-yl)methoxy)carbonyl)amino)-3-methoxy-3-oxopropyl)benzoate (11.9 g, 23.7 mmol) in dichloromethane (238 mL), and the mixture was stirred at room temperature for 3 h. After water (250 mL) was added to the reaction solution, the mixture was extracted with dichloromethane (250 mL). After the organic layer was washed with water, dried over anhydrous sodium sulfate, and filtered, the filtrate was concentrated under reduced pressure to afford (S)-4-(2-((((9H-fluoren-9-yl)methoxy)carbonyl)amino)-3-methoxy-3-oxopropyl)benzoic acid (Fmoc-Phe(4-COOH)-OMe) (10.1 g) as a crude product.

Synthesis of methyl (2S)-2-((((9H-fluoren-9-yl)methoxy)carbonyl)amino)-3-(4-(methyl((tetrahydro-2H-pyran-2-yl)oxy)carbamoyl)phenyl)propanoate(Fmoc-Phe(4-CONMeOTHP)-OMe)

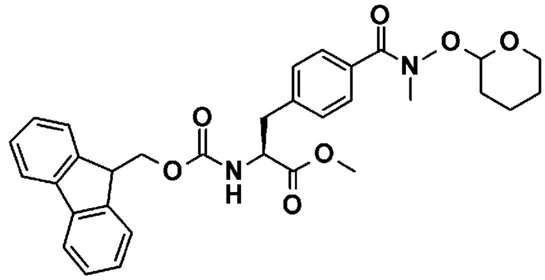

Under a nitrogen atmosphere, O-(7-aza-1H-benzotriazol-1-yl)-N,N,N',N'-tetramethyluronium hexafluorophosphate (HATU) (12.9 g, 34.0 mmol) and N,N-diisopropylethylamine (DIPEA) (4.4 g, 34.0 mmol) were added at room temperature to a solution of the above-described crude product (S)-4-(2-((((9H-fluoren-9-yl)methoxy)carbonyl)amino)-3-methoxy-3-oxopropyl)benzoic acid (10.1 g, 22.6 mmol) and N-methyl-O-(tetrahydro-2H-pyran-2-yl)hydroxylamine (14.9 g, 113.5 mmol) separately synthesized by the method described in a literature (Journal of Organic Chemistry, 2014, 79(20), 9699-9703) in dichloromethane (100 mL), and the mixture was stirred for 16 h. Water (100 mL) was added to the reaction solution, and the mixture was extracted with ethyl acetate (200 mL) and then washed with water (100 mL) and with brine (100 mL). After the organic layer was dried over anhydrous sodium sulfate and filtered, the residue obtained by concentrating the filtrate under reduced pressure was purified by normal phase column chromatography (petroleum ether/ethyl acetate) to afford methyl (2S)-2-((((9H-fluoren-9-yl)methoxy)carbonyl)amino)-3-(4-(methyl((tetrahydro-2H-pyran-2-yl)oxy)carbamoyl)phenyl)propanoate (Fmoc-Phe(4-CONMeOTHP)-OMe) (7.2 g, 55%).

Synthesis of (2S)-2-((((9H-fluoren-9-yl)methoxy)carbonyl)amino)-3-(4-(methyl(tetrahydro-2H-pyran-2-yl)oxy)carbamoyl)phenyl)propanic acid (Compound aa139. Fmoc-Phe(4-CONMeOTHP)-OH)

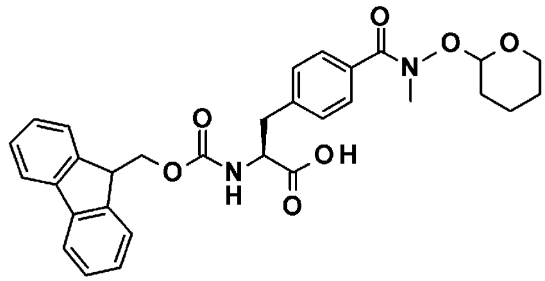

Water (72 mL) was added to calcium chloride (21.46 g, 193.33 mmol) and lithium hydroxide monohydrate (2.16 g, 51.47 mmol), the mixture was stirred at room temperature for 15 min, then a solution of the above-described methyl (2S)-2-((((9H-fluoren-9-yl)methoxy)carbonyl)amino)-3-(4-(methyl((tetrahydro-2H-pyran-2-yl)oxy)carbamoyl)phenyl)propanoate (Fmoc-Phe(4-CONMeOTHP)-OMe) (7.20 g, 12.89 mmol) in tetrahydrofuran/t-butanol (72 mL/288 mL) was added at room temperature, and the mixture was stirred for 16 h. The reaction solution was filtered, solids were washed with tetrahydrofuran (200 mL), then the filtrate was concentrated under reduced pressure, water (50 mL) was added, and the mixture was extracted with ethyl acetate (200 mL) twice. After the organic layer was washed with a 0.05N aqueous phosphoric acid solution (350 mL), the resulting aqueous layer was extracted again with ethyl acetate (200 mL). The organic layers were combined and concentrated under reduced pressure, and the resulting residue was purified by reverse phase column chromatography (water/acetonitrile) to afford (2S)-2-((((9H-fluoren-9-yl)methoxy)carbonyl)amino)-3-(4-(methyl((tetrahydro-2H-pyran-2-yl)oxy)carbamoyl)phenyl)propanoic acid (Compound aa139, Fmoc-Phe(4-CONMeOTHP)-OH) (5.1 g, 71%).
LCMS (ESI) m/z = 545 (M+H)+

Retention time: 0.79 min (Analytical condition SQDFA05)

Synthesis of tert-butyl 4-iodo-2-methoxybenzoate

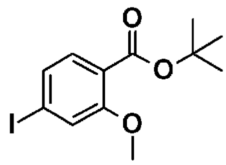

Under a nitrogen atmosphere, a solution of tert-butyl 2,2,2-trichloroacetimidate (23.58 g, 107.896 mmol) in cyclohexane (120 mL) was added dropwise at room temperature to a solution of commercially available 4-iodo-2-methoxybenzoic acid (15.0 g, 53.948 mmol) in dichloromethane (60.0 mL), a boron trifluoride-diethyl ether complex ($BF_3 \cdot OEt_2$) (76.57 mg, 0.539 mmol) was then added dropwise, and the mixture was stirred at room temperature for 2 h. The reaction solution was concentrated under reduced pressure, hexane was added to the resulting residue, the mixture was stirred for 1 h and filtered. The filtrate was then concentrated under reduced pressure to afford tert-butyl 4-iodo-2-methoxybenzate (21 g) as a crude product.

Synthesis of tert-butyl (S)-4-(2-((((9H-fluoren-9-yl)methoxy)carbonyl)amino)-3-methoxy-3-oxopropyl)-2-methoxybenzoate (Fmoc-Phe(3-OMe-4-COtBu)-OMe)

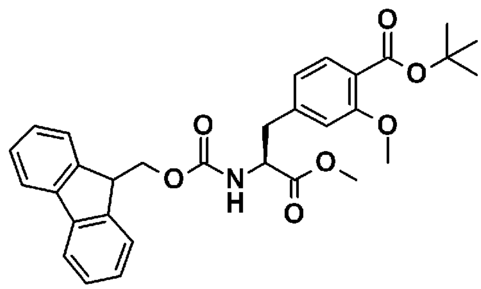

Under a nitrogen atmosphere, a solution of nickel(II) bromide trihydrate ($NiBr_2 \cdot 3H_2O$) (4.28 g, 15.711 mmol) and 4,4'-di-tert-butyl-2,2'-bipyridyl (dtbbpy) (4.22 g, 15.711 mmol) in dimethylacetamide (DMA) (150 mL) was stirred at 50°C for 3 h and then cooled to room temperature to afford a nickel catalyst solution. Under a nitrogen atmosphere, a solution of zinc powder (17.13 g, 261.856 mmol), 1-methyl 4-(1,3-dioxoisoindolin-2-yl) (((9H-fluoren-9-yl)methoxy)carbonyl)-L-aspartate (26.94 g, 52.371 mmol) synthesized as described above and the crude product tert-butyl 4-iodo-2-methoxybenzoate (35.0 g, 104.742 mmol) synthesized as described above in dimethylacetamide (DMA) (150 mL) was stirred at room temperature for 10 min. The above-described nickel catalyst solution was then added dropwise at room temperature, and the mixture was stirred for 16 h. A 10% aqueous EDTA·2Na (disodium salt of ethylenediaminetetraacetic acid) solution was added to the reaction solution, the mixture was filtered, and then the resulting filtrate was extracted with ethyl acetate twice. The organic layer was washed with brine, dried over anhydrous sodium sulfate, and filtered. The residue obtained by concentrating the filtrate under reduced pressure was purified by normal phase column chromatography (petroleum ether/ethyl acetate) to afford tert-butyl (S)-4-(2-((((9H-fluoren-9-yl)methoxy)carbonyl)amino)-3-methoxy-3-oxopropyl)-2-methoxybenzoate (Fmoc-Phe(3-OMe-4-COtBu)-OMe) (8.4 g, 13%).

Synthesis of (S)-4-(2-((((9H-fluoren-9-yl)methoxy)carbonyl)amino)-3-methoxy-3-oxopropyl)-2-methoxybenzoic acid (Fmoc-Phe(3-OMe-4-COOH)-OMe)

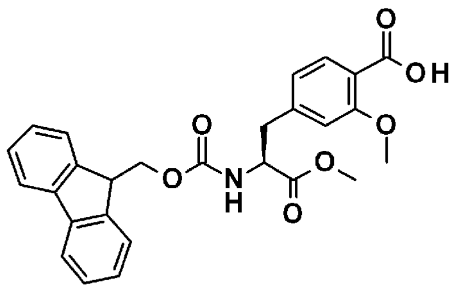

Under a nitrogen atmosphere, a 4N hydrochloric acid/1,4-dioxane solution (268 mL) was added at room temperature to a solution of tert-butyl (S)-4-(2-((((9H-fluoren-9-yl)methoxy)carbonyl)amino)-3-methoxy-3-oxopropyl)-2-methoxybenzoate (Fmoc-Phe(3-OMe-4-COtBu)-OMe) (26.8 g, 50.4 mmol) obtained as described above in 1,4-dioxane (30.0 mL), and the mixture was stirred for 1 h. The residue obtained by concentrating the reaction solution under reduced pressure was washed with hexane/ethyl acetate (5/1) to afford (S)-4-(2-((((9H-fluoren-9-yl)methoxy)carbonyl)amino)-3-methoxy-3-oxopropyl)-2-methoxybenzoic acid (Fmoc-Phe(3-OMe-4-COOH)-OMe) (22 g) as a crude product.

Synthesis of methyl (2S)-2-((((9H-fluoren-9-yl)methoxy)carbonyl)amino)-3-(3-methoxy-4-(((tetrahydro-2H-pyran-2-yl)oxy)carbamoyl)phenyl)propanate (Fmoc-Phe(3-OMe-4-CONHOTBP)-OMe)

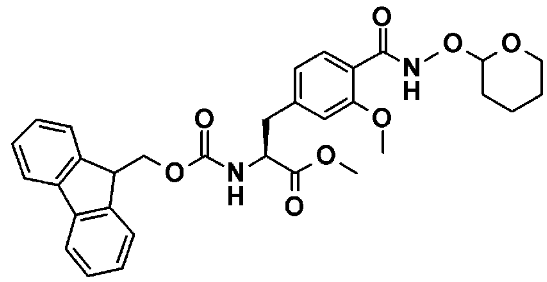

Under a nitrogen atmosphere, O-(7-aza-1H-benzotriazol-1-yl)-N,N,N',N'-tetramethyluronium hexafluorophosphate (HATU) (21.11 g, 55.521 mmol) and N,N-diisopropylethylamine (DIPEA) (7.18 g, 55.521 mmol) were added at room temperature to a solution of the above-described crude product (S)-4-(2-((((9H-fluoren-9-yl)methoxy)carbonyl)amino)-3-methoxy-3-oxopropyl)-2-methoxybenzoic acid (Fmoc-Phe(3-OMe-4-COOH)-OMe) (22.0 g, 46.267 mmol) and commercially available O-(tetrahydro-2H-pyran-2-yl)hydroxylamine (27.1 g, 231.337 mmol) in dichloromethane (400 mL), and the mixture was stirred for 16 h. Water was added to the reaction solution, and the mixture was extracted with ethyl acetate twice and then washed with brine. After the organic layer was dried over anhydrous sodium sulfate and filtered, the filtrate was concentrated under reduced pressure to afford methyl (2S)-2-((((9H-fluoren-9-yl)methoxy)carbonyl)amino)-3-(3-methoxy-4-(((tetrahydro-2H-pyran-2-yl)oxy)carbamoyl)phenyl)propanate (Fmoc-Phe(3-OMe-4-CONHOTHP)-OMe) (35.0 g) as a crude product.

Synthesis of (2S)-2-((((9H-fluoren-9-yl)methoxy)carbonyl)amino)-3-(3-methoxy-4-(((tetrahydro-2H-pyran-2-yl)oxy)carbamoyl)phenyl)propanoic acid (Compound aa140, Fmoc-Phe(3-OMe-4-CONHOTHP)-OH)

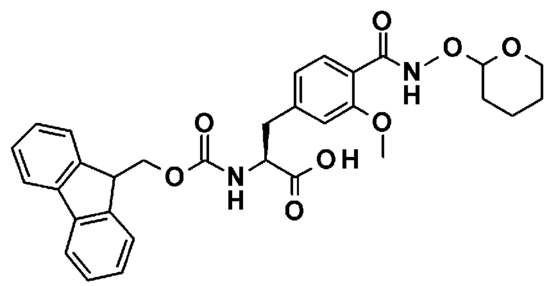

Water (360 mL) and isopropanol (1440 mL) were added at 0°C to calcium chloride (101.40 g, 913.631 mmol) and lithium hydroxide monohydrate (5.83 g, 243.635 mmol), a solution of the above-described crude product methyl (2S)-2-((((9H-fluoren-9-yl)methoxy)carbonyl)amino)-3-(3-methoxy-4-(((tetrahydro-2H-pyran-2-yl)oxy)carbamoyl)phenyl)propanate (Fmoc-Phe(3-OMe-4-CONHOTHP)-OMe) (35.0 g, 60.909 mmol) in tetrahydrofuran (360 mL) was added dropwise, and then the mixture was stirred at room temperature for 16 h. After the reaction solution was concentrated under reduced pressure to remove the organic solvent, a 2 M aqueous hydrochloric acid solution was added to the resulting aqueous layer until pH = 7, and the mixture was extracted with ethyl acetate twice. After the organic layer was washed with brine, dried over anhydrous sodium sulfate, and filtered, the resulting filtrate was concentrated under reduced pressure. The residue was purified by reverse phase column chromatography (water/acetonitrile) to afford (2S)-2-((((9H-fluoren-9-yl)methoxy)carbonyl)amino)-3-(3-methoxy-4-(((tetrahydro-2H-pyran-2-yl)oxy)carbamoyl)phenyl)propanoic acid (Compound aa140, Fmoc-Phe(3-OMe-4-CONHOTHP)-OH) (7.3 g, 40%).
LCMS (ESI) m/z = 561 (M+H)+

Retention time: 0.77 min (Analytical condition SQDFA05)

N-(((9H-Fluoren-9-yl)methoxy)carbonyl)-O-(((S)-4-((allyloxy)carbonyl)morpholin-3-yl)methyl)-L-serine (Compound aa141, Fmoc-Ser(S-(Alloc)Mor-3-Me)-OH) was synthesized according to the following scheme.

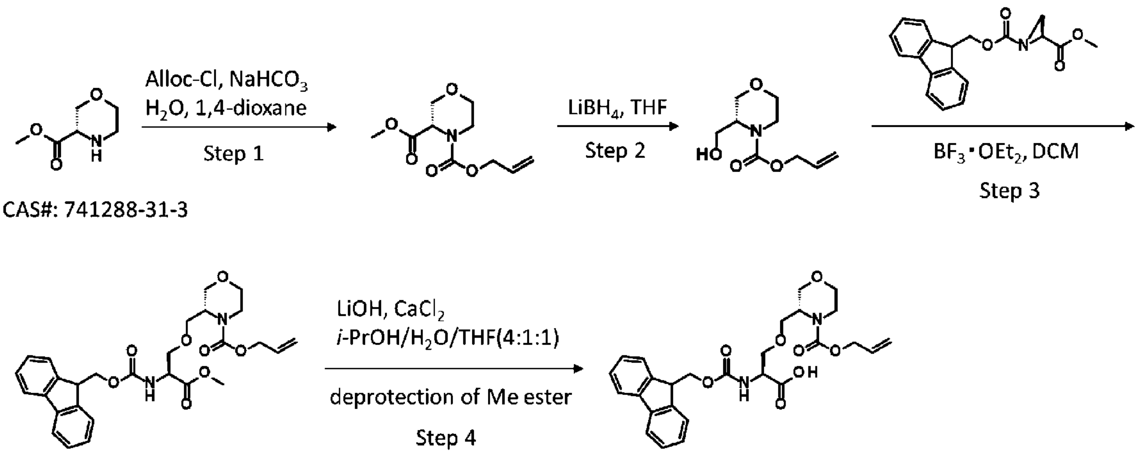


CAS#: 741288-31-3

Compound aa141

Step 1

Under a nitrogen atmosphere, sodium hydrogen carbonate (20.26 g, 241.172 mmol) was added to an aqueous solution (100 mL) of commercially available (S)-morpholine-3-carboxylic acid (CAS. No. 741288-31-3) (14.0 g, 107 mmol) at room temperature. A solution of allyl chloroformate (Alloc-Cl) (17.44 g, 120.53 mmol) in 1,4-dioxane (100 mL) was added dropwise to the reaction solution at room temperature, and the mixture was stirred for 2 h. After water was added to the reaction solution, the mixture was extracted with ethyl acetate, and the resulting organic layer was washed with brine, dried over anhydrous sodium sulfate, and filtered. The filtrate was concentrated under reduced pressure, and the resulting residue was purified by normal phase column chromatography (petroleum ether/ethyl acetate) to afford 4-allyl 3-methyl (S)-morpholine-3,4-dicarboxylate (19 g, 86%).

Step 2

A lithium borohydride (5.56 g, 255.233)/tetrahydrofuran (THF) solution was added dropwise at 0°C to a solution of 4-allyl 3-methyl (S)-morpholine-3,4-dicarboxylate (19.5 g, 85.067 mmol) obtained as described above in tetrahydrofuran (THF) (283 mL), and the mixture was stirred at room temperature for 2 h. Water and a 2N aqueous hydrochloric acid solution were added at 0°C to the reaction solution, the mixture was extracted with ethyl acetate, and the resulting organic layer was washed with brine, dried over anhydrous sodium sulfate, and filtered. The filtrate was concentrated under reduced pressure, and the resulting residue was purified by normal phase column chromatography (petroleum ether/ethyl acetate) to afford allyl (R)-3-(hydroxymethyl)morpholine-4-carboxylate (10.1 g, 59%).

Step 3

Under a nitrogen atmosphere, a solution of a boron trifluoride-ethyl ether complex (1.48 g, 10.45 mmol) in dichloromethane was added dropwise at 0°C to a solution of the above-described allyl (R)-3-(hydroxymethyl)morpholine-4-carboxylate (7.0 g, 34.8 mmol) and 2-methyl 1-((9H-fluoren-9-yl)methyl) (S)-aziridine-1,2-dicarboxylate (7.50 g, 23.2 mmol) synthesized by the method described in a patent literature (WO 2015/179823) in dichloromethane (47 mL), and the mixture was stirred for 2 h. After water was added to the reaction solution, the mixture was extracted with dichloromethane, and the resulting organic layer was washed with brine, dried over anhydrous sodium sulfate, and filtered. The filtrate was concentrated under reduced pressure, and the resulting residue was purified by reverse phase column chromatography (water/acetonitrile) to afford allyl (S)-3-(((S)-2-((((9H-fluoren-9-yl)methoxy)carbonyl)amino)-3 -methoxy-3 -oxopropoxy)methyl)morpholine-4-carboxylate (4.5 g, 37%).

Step 4

Under a nitrogen atmosphere, lithium hydroxide monohydrate (0.91 g, 37.999 mmol) was added at room temperature to an aqueous solution (50 mL) of calcium chloride (15.87 g, 142.974 mmol), and the mixture was stirred at room temperature for 10 min. A solution of allyl (S)-3-(((S)-2-((((9H-fluoren-9-yl)methoxy)carbonyl)amino)-3-methoxy-3-oxopropoxy)methyl)morpholine-4-carboxylate (5.0 g, 9.532 mmol) obtained as described above in tetrahydrofuran (50 mL) and isopropanol (200 mL) were added at 0°C to the reaction solution, the mixture was stirred at room temperature for 9 h, a 1 M aqueous hydrochloric acid solution was added, and the solvent was evaporated under reduced pressure. The resulting residue was purified by reverse phase column chromatography (water/acetonitrile) to afford N-(((9H-fluoren-9-yl)methoxy)carbonyl)-O-(((S)-4-((allyloxy)carbonyl)morpholin-3-yl)methyl)-L-serine (Compound aa141, Fmoc-Ser(S-(Alloc)Mor-3-Me)-OH) (2.4 g, 49%).
LCMS (ESI) m/z = 511 (M+H)+

Retention time: 0.77 min (analytical condition SQDFA05)

1-3. Synthesis of resins used for peptide synthesis by a peptide synthesizer

Resins used for peptide synthesis by a peptide synthesizer were synthesized as described below. 2-Chlorotrityl chloride resin (100-200 mesh, 1% DVB) was purchased from Watanabe Chemical Industries and Chem-Impex.

Synthesis of (S)-3-((((9H-fluoren-9-yl)methoxy)carbonyl)amino)-4-oxo-4-(-piperidin-1-yl)butanoic acid-2-chlorotrityl resin (Compound pd01, Fmoc-Asp(O-Trt(2-Cl)-resin)-pip)

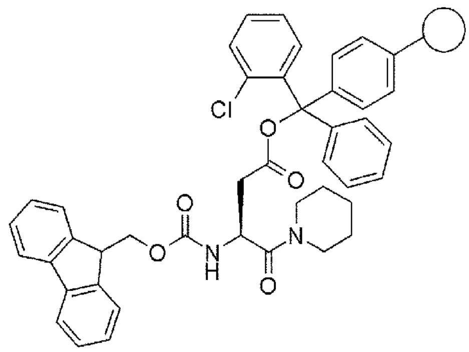

(S)-3-((((9H-Fluoren-9-yl)methoxy)carbonyl)amino)-4-oxo-4-(piperidin-1-yl)butanoic acid-2-chlorotrityl resin (Compound pd01, Fmoc-Asp(O-Trt(2-Cl)-resin)-pip) was synthesized by the method described in WO 2013/100132.

In the present specification, when a polymer or resin is attached to a compound, the polymer or resin site may be represented with " o." In order to specify the point of reaction in the resin site, the chemical structure of the reaction site may be represented as a structure connected to "o." The above structure shows a manner in which the 2-chlorotrityl group on the resin is attached to the side chain carboxylic acid of Asp through an ester bond in Fmoc-Asp(O-Trt(2-Cl)-resin)-pip (Compound pd01).

Synthesis of (S)-3-((((9H-fluoren-9-yl)methoxy)carbonyl)(methyl)amino)-4-oxo-4-(piperidin-1-yl)butanoic acid-2-chlorotrityl resin (Compound pd07, Fmoc-MeAsp(O-Trt(2-Cl)-Resin)-pip)

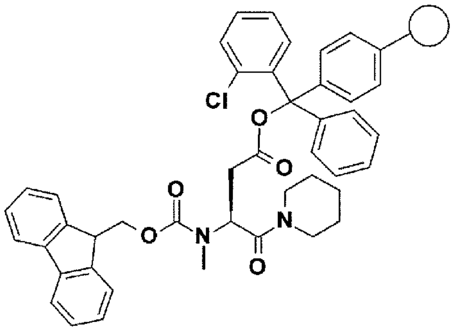

(S)-3-((((9H-Fluoren-9-yl)methoxy)carbonyl)(methyl)amino)-4-oxo-4-(piperidin-1-yl)butanoic acid-2-chlorotrityl resin (Compound pd07, Fmoc-MeAsp(O-Trt(2-Cl)-Resin)-pip) was synthesized by the following route.

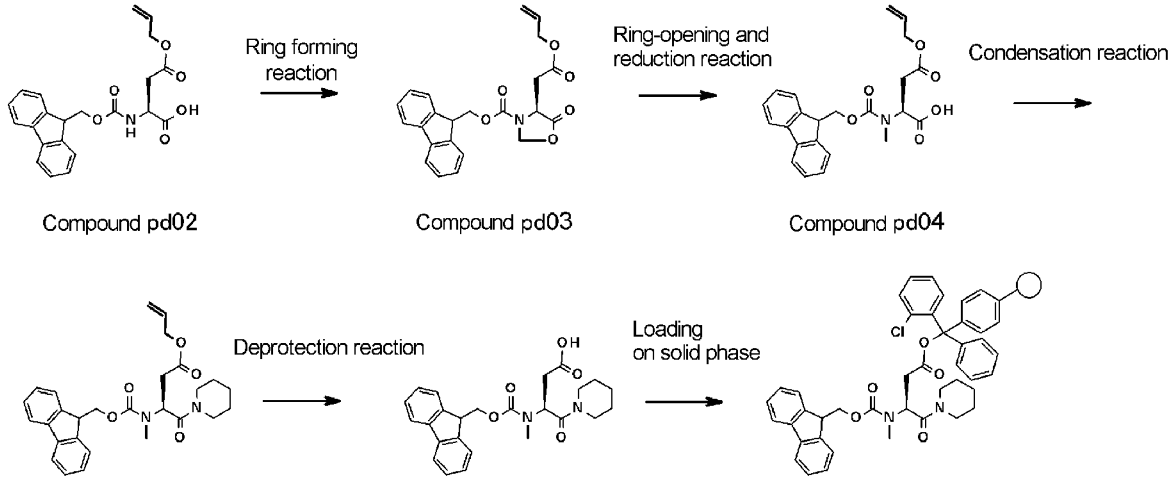


Compound pd02 Compound pd03 Compound pd04

Compound pd05 Compound pd06 Compound pd07

Synthesis of (S)-2-((((9H-fluoren-9-yl)methoxy)carbonyl)(methyl)amino)-4-(allyloxy)-4-oxobutanoic acid (Compound pd04, Fmoc-MeAsp(OAl)-OH)

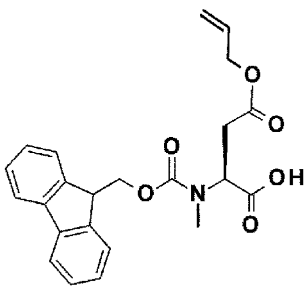

To a solution of (S)-2-((((9H-fluoren-9-yl)methoxy)carbonyl)amino)-4-(allyloxy)-4-oxobutanoic acid (Compound pd02, Fmoc-Asp(OAl)-OH) (10.0 g, 25.3 mmol) in toluene (100 mL) were added paraformaldehyde (1.52 g) and tosyl acid (TsOH) (260 mg, 1.51 mmol) at room temperature under a nitrogen atmosphere, and the mixture was stirred at 110°C for 16 h. The reaction solution was then cooled to room temperature and washed with a saturated aqueous sodium

bicarbonate solution twice. The resulting organic layers were dried over anhydrous sodium sulfate, filtered, and concentrated under reduced pressure to afford (9H-fluoren-9-yl)methyl (S)-4-(2-(allyloxy)-2-oxoethyl)-5-oxooxazolidine-3-carboxylate (Compound pd03) (8.0 g) as a crude product.

A solution of (9H-fluoren-9-yl)methyl (S)-4-(2-(allyloxy)-2-oxoethyl)-5-oxooxazolidine-3-carboxylate obtained in the previous step (Compound pd03) (5.0 g, 12.3 mmol) and triethylsilane ($Et_3SiH$, 4.3 g) (37.0 mmol) in dichloromethane/trifluoroacetic acid (TFA) = 1/1 (80 mL/80 mL) was stirred at room temperature for two days under a nitrogen atmosphere, after which the reaction solution was concentrated under reduced pressure. To the resulting residue was added an aqueous potassium carbonate ($K_2CO_3$) solution. After washing with petroleum ether three times, the reaction solution was adjusted to pH 3 with hydrochloric acid and extracted with ethyl acetate three times. The organic layers were combined, dried over anhydrous sodium sulfate, filtered, and concentrated under reduced pressure. The resulting residue was purified by reverse phase silica gel column chromatography (0.1% aqueous formic acid solution/0.1% formic acid-acetonitrile) to afford (S)-2-((((9H-fluoren-9-yl)methoxy)carbonyl)(methyl)amino)-4-(allyloxy)-4-oxobutanoic acid (Compound pd04, Fmoc-MeAsp(OAl)-OH) (3.0 g, 46%, over two steps).
LCMS (ESI) m/z = 410 (M+H)+

Retention time: 0.84 min (analytical condition SQDFA05)

Synthesis of allyl (S)-3-((((9H-fluoren-9-yl)methoxy)carbonyl)(methyl)amino)-4-oxo-4-(piperidin-1-yl)butanoate (Compound pd05, Fmoc-MeAsp(OAl)-pip)

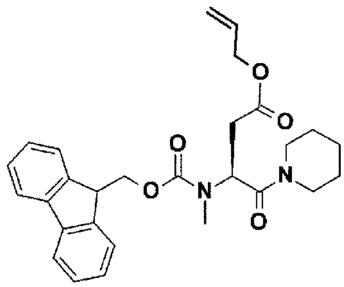

To a solution of 1-ethyl-3-(3-dimethylaminopropyl)carbodiimide hydrochloride (WSCI·HCl) (16.5 g, 86 mmol) in DMF (143 mL) was added 1-hydroxybenzotriazole (HOBt) (10.6 g, 79 mmol) at 0°C under a nitrogen atmosphere. Subsequently, a mixed solution of (S)-2-((((9H-fluoren-9-yl)methoxy)carbonyl)(methyl)amino)-4-(allyloxy)-4-oxobutanoic acid (Compound pd04, Fmoc-MeAsp(OAl)-OH) (29.3 g, 71.6 mmol) in DMF/DCM = 1/1 (117 mL) was added dropwise at 0°C, and the mixture was stirred for 30 min. Piperidine (8.49 mL, 86 mmol) was then added dropwise at 0°C and the mixture was stirred for 30 min. After the progress of the reaction was confirmed by LC-MS, ethyl acetate was added to the reaction solution, and the solution was warmed to room temperature. The resulting organic layer was washed with a 2 M aqueous hydrochloric acid solution twice, with a 5% aqueous sodium bicarbonate solution twice, and with brine twice, and then dried over magnesium sulfate. The resulting mixture was filtered and concentrated under reduced pressure to afford allyl (S)-3-((((9H-fluoren-9-yl)methoxy)carbonyl)(methyl)amino)-4-oxo-4-(piperidin-1-yl)butanoate (Compound pd05, Fmoc-MeAsp(OAl)-pip) (33.7 g, 99%).
LCMS (ESI) m/z = 477 (M+H)+

Retention time: 1.32 min (analytical condition SMD method 6)

Synthesis of (S)-3-((((9H-fluoren-9-yl)methoxy)carbonyl)(methyl)amino)-4-oxo-4-(piperidin-1-yl)butanoic acid (Compound pd06, Fmoc-MeAsp-pip)

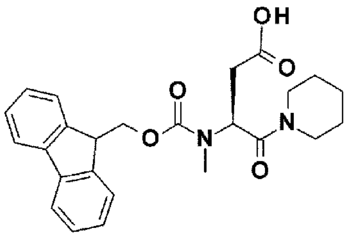

Dichloromethane (132 mL) was added to allyl (S)-3-((((9H-fluoren-9-yl)methoxy)carbonyl)(methyl)amino)-4-oxo-4-(piperidin-1 -yl)butanoate (Compound pd05, Fmoc-MeAsp(OAl)-pip) (31.4 g, 65.9 mmol), sodium 4-methylbenzenesulfinate (11.2 g, 62.6 mmol), and tetrakistriphenylphosphine palladium ($Pd(PPh_3)_4$) (761 mg, 0.659 mmol) under a nitrogen atmosphere, and the mixture was stirred at room temperature for 1.5 h. To the reaction solution was then added a 5% aqueous sodium bicarbonate solution, and the reaction solution was washed with t-butyl methyl ether (TBME) twice. The resulting aqueous layer was adjusted to an acidic pH with a 6 M aqueous hydrochloric acid solution and then extracted with ethyl acetate. The resulting organic layer was washed with 50% saline twice and dried over anhydrous magnesium sulfate, after which the resulting mixture was filtered and concentrated under reduced pressure. The resulting residue was purified by reverse phase silica gel column chromatography (0.1% aqueous formic acid solution/0.1 % formic acid-acetonitrile) and further purified by normal phase silica gel column chromatography ($CO_2H$ silica gel, hexane/ethyl acetate) to afford (S)-3-((((9H-fluoren-9-yl)methoxy)carbonyl)(methyl)amino)-4-oxo-4-(piperidin-1-yl)butanoic acid (Compound pd06, Fmoc-MeAsp-pip) (17.1 g, 60%).
LCMS (ESI) m/z = 437 (M+H)+

Retention time: 1.10 min (analytical condition SMD method 6)

Synthesis of (S)-3-((((9H-fluoren-9-yl)methoxy)carbonyl)(methyl)amino)-4-oxo-4-(piperidin-1 - yl)butanoic acid-2-chlorotrityl resin (Compound pd07, Fmoc-MeAsp(O-Trt(2-Cl)-resin)-pip)

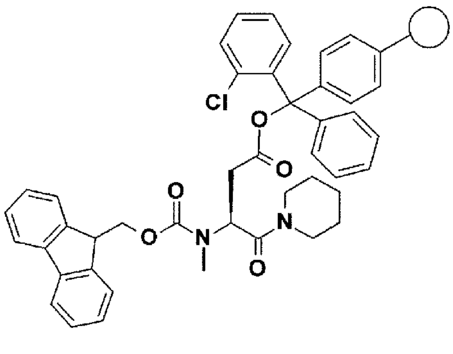

In a reaction vessel with a filter was placed 2-chlorotrityl chloride resin (1.60 mmol/g, 100-200 mesh, 1% DVB, purchased from Watanabe Chemical Industries, 25 g, 40.0 mmol) and dehydrated dichloromethane (400 mL), and the vessel was shaken at room temperature for 10 min. The dichloromethane was removed by applying nitrogen pressure, after which dehydrated methanol (6.48 mL) and diisopropylethylamine (DIPEA) (16.7 mL) were added to (S)-3-((((9H-fluoren-9-yl)methoxy)carbonyl)(methyl)amino)-4-oxo-4-(piperidin-1 -yl)butanoic acid (Compound pd06, Fmoc-MeAsp-pip) (8.37 g, 20.0 mmol) and dehydrated dichloromethane (400 mL), the resulting mixture was added to the reaction vessel, and the vessel was shaken for 30 min. The reaction solution was removed by applying nitrogen pressure, after which dehydrated methanol (50.0 mL) and diisopropylethylamine (DIPEA) (16.7 mL) were added to dehydrated dichloromethane (400 mL), the resulting mixture was added to the reaction vessel, and the vessel was shaken for 1 hour and 30 minutes. The reaction solution was removed by applying nitrogen pressure, after which dichloromethane was placed in the vessel, followed by shaking for 5 min. The reaction solution was removed by applying nitrogen pressure. This washing of the resin with dichloromethane was repeated twice, and the resulting resin was dried under reduced pressure overnight to afford (S)-3-((((9H-fluoren-9-yl)methoxy)carbonyl)(methyl)amino)-4-oxo-4-(piperidin-1-yl)butanoic acid-2-chlorotrityl resin (Compound pd07, Fmoc-MeAsp(O-Trt(2-Cl)-resin)-pip) (29.9 g).

The resulting (S)-3-((((9H-fluoren-9-yl)methoxy)carbonyl)(methyl)amino)-4-oxo-4-(piperidin-1-yl)butanoic acid-2-chlorotrityl resin (Compound pd07, Fmoc-MeAsp(O-Trt(2-Cl)-resin)-pip) (10.7 mg) was placed in a reaction vessel,

DMF (200 μL) and piperidine (200 μL) were added, and the vessel was shaken at room temperature for 1 h. To the reaction mixture was then added DMF (1.6 mL), 400 μL of the mixture was taken out, its absorbance (301.2 nm) was measured (using Shimadzu, UV-1600PC (cell length: 1.0 cm)), and the loading amount of (S)-3-((((9H-fluoren-9-yl)methoxy)carbonyl)(methyl)amino)-4-oxo-4-(piperidin-1 -yl)butanoic acid-2-chlorotrityl resin (Compound pd07, Fmoc-MeAsp(O-Trt(2-Cl)-resin)-pip) was calculated to be 0.416 mmol/g.

Another lot similarly synthesized with a different loading amount was also used for peptide synthesis.

Synthesis of (S)-3-((((9H-fluoren-9-yl)methoxy)carbonyl)amino)-4-(methyl((S)-1-oxo-1-(((S)-1-oxo-1-(piperidin-1 -yl)propan-2-yl)amino)-3-phenylpropan-2-yl)amino)-4-oxobutanoic acid-2-chlorotrityl resin (Compound pd08, Fmoc-Asp(O-Trt(2-Cl)-resin)-MePhe-Ala-pip)

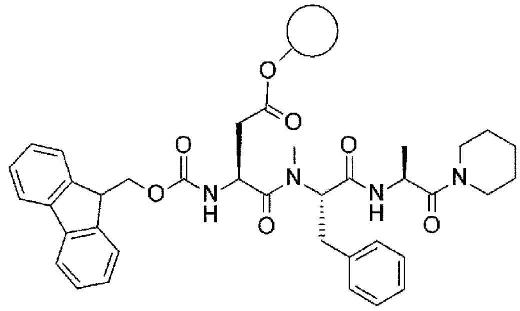

(S)-3-((((9H-Fluoren-9-yl)methoxy)carbonyl)amino)-4-(methyl((S)-1-oxo-1-(((S)-1-oxo-1 -(piperidin-1 -yl)propan-2-yl)amino)-3 -phenylpropan-2-yl)amino)-4-oxobutanoic acid-2-chlorotrityl resin (Compound pd08, Fmoc-Asp(O-Trt(2-Cl)-resin)-MePhe-Ala-pip) was synthesized by the method described in the document (Document: International Publication No. WO 2013/100132A1).

Synthesis of (S)-3-((((9H-fluoren-9-yl)methoxy)carbonyl)amino)-4-(methyl((S)-1-oxo-1-(((S)-1-oxo-3-phenyl-1-(piperidin-1-yl)propan-2-yl)amino)-3-phenylpropan-2-yl)amino)-4-oxobutanoic acid-2-chlorotrityl resin (Compound pd09, Fmoc-Asp(O-Trt(2-Cl)-resin)-MePhe-Phe-pip)

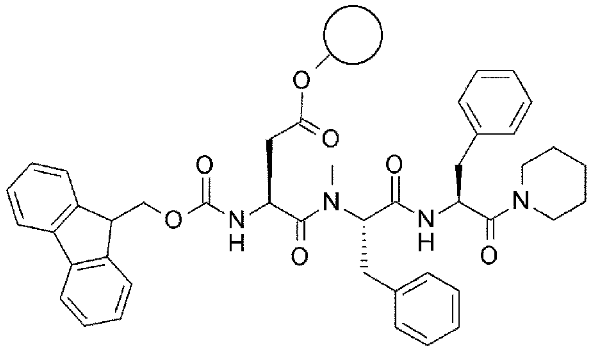

(S)-3-((((9H-Fluoren-9-yl)methoxy)carbonyl)amino)-4-(methyl((S)-1-oxo-1-(((S)-1-oxo-3-phenyl-1-(piperidin-1-yl)propan-2-yl)amino)-3-phenylpropan-2-yl)amino)-4-oxobutanoic acid-2-chlorotrityl resin (Compound pd09, Fmoc-Asp(O-Trt(2-Cl)-resin)-MePhe-Phe-pip) was synthesized by a similar procedure as for Compound pd08 using the method described in the document (Document: International Publication No. WO 2013/100132A1).

Synthesis of (S)-3-((((9H-fluoren-9-yl)methoxy)carbonyl)amino)-4-(methyl((S)-1-oxo-1-(((S)-1-oxo-1-(((S)-1-oxo-1-(piperidin-1-yl)propan-2-yl)amino)-3-phenylpropan-2-yl)amino)-3-phenylpropan-2-yl)amino)-4-oxobutanoic acid-2-chlorotrityl resin (Compound pd10, Fmoc-Asp(O-Trt(2-Cl)-resin)-MePhe-MePhe-Ala-pip)

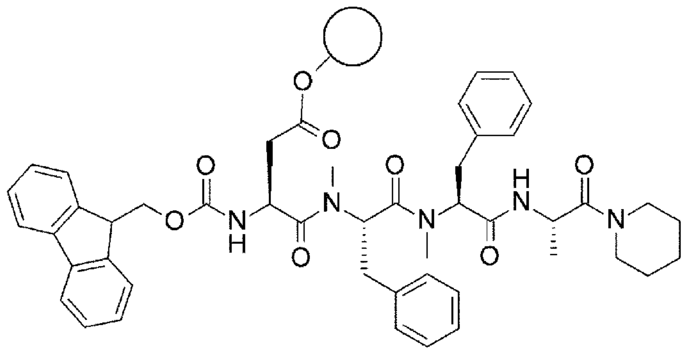

(S)-3-((((9H-Fluoren-9-yl)methoxy)carbonyl)amino)-4-(methyl((S)-1-oxo-1-(((S)-1-oxo-1-(((S)-1-oxo-1-(piperidin-1-yl)propan-2-yl)amino)-3-phenylpropan-2-yl)amino)-3-phenylpropan-2-yl)amino)-4-oxobutanoic acid-2-chlorotrityl resin (Compound pd10, Fmoc-Asp(O-Trt(2-Cl)-resin)-MePhe-MePhe-Ala-pip) was synthesized by the method described in WO 2013/100132.

1-4. Chemical synthesis of peptides

Unless otherwise stated, peptide compounds A01 to A10, B01 to B10, C01 to C17, D01 to D17, E01 to E08, F01 to F08, G01 to G11, H01 to H11, 101 to 106, J01 to J06, AP01 to AP143, pd30 to pd36, pd50 to pd70, pd100 to pd129, pd162, pd163, pd167, pd177 to pd247, pd386 to pd395, pd452 to pd454, and pd482 to pd487 were synthesized by the basic route described at the beginning of Example 1 according to the following method.

1) Solid-phase synthesis of peptides by an automated synthesizer

Peptides were synthesized by the Fmoc method described in WO 2013/100132 using a peptide synthesizer (Multipep RS; manufactured by Intavis). The manual attached to the synthesizer was followed for the detailed operational procedure.

In the synthesizer was placed 2-chlorotrityl resin (e.g. (S)-3-((((9H-fluoren-9-yl)methoxy)carbonyl)amino)-4-oxo-4-(piperidin-1-yl)butanoic acid-2-chlorotrityl resin (Compound pd01, Fmoc-Asp(O-Trt(2-Cl)-resin)-pip)) (100 mg per column) to which was attached the side chain carboxylic acid site of aspartic acid with the N-terminus protected by Fmoc; various Fmoc amino acids (0.6 mol/L); a solution of 1-hydroxy-7-azabenzotriazole (HOAt) or oxyma (0.375 mol/L) in N-methyl-2-pyrrolidone (NMP); and a solution (10% v/v) of diisopropylcarbodiimide (DIC) in N,N-dimethylformamide (DMF). Fmoc-Thr(THP)-OH (Compound aa01) was allowed to coexist with oxyma in the NMP solution, to which molecular sieves 4A1/8 (Wako Pure Chemical Industries) or molecular sieves 4A1/16 (Wako Pure Chemical Industries) were further added and placed in the synthesizer. As with Fmoc-Thr(THP)-OH (Compound aa01), amino acids having a THP group as a protecting group for the amino acid side chain (such as Fmoc-Ser(EtOTHP)-OH (Compound aa22)) was also allowed to coexist with oxyma in the NMP solution, to which molecular sieves 4A1/8 (Wako Pure Chemical Industries) or molecular sieves 4A1/16 (Wako Pure Chemical Industries) were further added and placed in the synthesizer. When using an amino acid salt such as hydrochloride, a solution of DIPEA/DMF = 3/1 was set, and 0.96 equivalents of DIPEA relative to an amino acid used during peptide elongation was separately added as the above DMF solution. Furthermore, in peptide compounds containing an amino acid having an acidic functional group in the side chain, a solution of 0.08 M triethylamine hydrochloride in dichloromethane was used in resin washing during a peptide elongation reaction to suppress excessive peptide elongation due to a residual deprotecting agent.

2) Cleavage of the elongated peptide from the resin

The resin was swollen again according to the method described in WO 2013/100132 by adding DCM to the linear peptide loaded on the solid phase obtained by the above method, and 2,2,2-trifluoroethanol (TFE)/DCM (1/1, v/v, 2 mL) was then added to the resin, followed by shaking at room temperature for 2 h. The solution in the tube was then filtered using a synthesis column to remove the resin, and the remaining resin was further washed with 2,2,2-trifluoroethanol (TFE)/DCM (1/1, v/v, 1 mL) twice. A solution obtained by combining all resulting cleavage solutions or a solution obtained by further diluting the solution with dichloroethane (DCE) (4 mL) was concentrated under reduced pressure. Meanwhile, when cleaving the peptide by 2,2,2-trifluoroethanol (TFE)/DCM (1/1, v/v, 2 mL), DIPEA can also be added in a volume of 1.8 equivalents relative to the number of moles on the resin used (which is obtained by multiplying the loading amount (mmol/g) by the amount of the resin used (usually 0.10 g)).

3) Method for cyclizing the cleaved peptide

After the cleavage, concentration under reduced pressure gave a residue, which was then dissolved in DMF/DCM (1/1, v/v, 8 mL). A 0.5 M O-(7-aza-1H-benzotriazol-1-yl)-N,N,N,N-tetramethyluronium hexafluorophosphate (HATU)/DMF solution (1.5 volume equivalents relative to the number of moles (the loading amount (mmol/g) multiplied by the amount of the resin used (usually 0.10 g)) on the resin used) and DIPEA (1.8 equivalents relative to the number of moles on the resin used) were added, and the tube was stirred at room temperature for 2 h or longer. The solvent was then evaporated under reduced pressure. Generation of the intended cyclic peptide was confirmed by LCMS measurement.

4) 5) Deprotection of the protecting group for the side chain functional group possessed by the cyclic peptide

4 mL of the prepared 0.05 M tetramethylammonium hydrogen sulfate/1,1,1,3,3,3-hexafluoroisopropyl alcohol (HFIP) solution (2% triisopropylsilane (TIPS)) was added to dissolve the residue, and the resulting solution was then stirred at room temperature for 4 h or longer. After allowing to stand for a certain period of time, diisopropylethylamine (DIPEA) (70 μL) was added and the solvent was evaporated under reduced pressure.

A peptide containing an amino acid having an Alloc group in the side chain (such as a peptide containing Nva(2-R-4-F2-Pyrro), Ser(S-4-F2-Pyrro-Me), or the like in its sequence) was stirred at room temperature for 3 hours and 30 minutes or longer using zinc chloride (1.0 equivalent relative to the number of moles on the resin used), tetrakis(triphenylphosphine)palladium(0) ($Pd(PPh_3)_4$) (1.0 equivalent relative to the number of moles on the resin used), poly(methylhydrosiloxane) (PMHS, CAS # 63148-57-2) (0.9 M relative to the number of moles on the resin used), and tetrahydrofuran (0.1 M relative to the number of moles on the resin used) to deprotect the Alloc group, then 4 mL of the prepared 0.05 M tetramethylammonium hydrogensulfate/1,1,1,3,3,3-hexafluoroisopropyl alcohol (HFIP) solution (2% triisopropylsilane (TIPS)) was added to the reaction solution, and the mixture was stirred at room temperature for 4 h or longer. After allowing to stand for a certain period of time, diisopropylethylamine (DIPEA) (70 μL) was added, and the solvent was evaporated under reduced pressure. In the deprotection of the Alloc group, tetramethyldisiloxane (TMDS, CAS # 3277-26-7) (4.0 equivalents relative to the number of moles on the resin used) can be used instead of PMHS above.

In all cases, the solvent was evaporated under reduced pressure, DMF, DMSO, or a 20% aqueous methanol solution was then added, the insoluble matter was removed by filtration through a filter, and the residue was then purified by preparative HPLC.

The 0.05 M tetramethylammonium hydrogen sulfate/HFIP solution (2% TIPS) was prepared by dissolving 34.3 mg of tetrabutylammonium hydrogen sulfate in 4 mL of a solution taken out from a mixed solution of HFIP (11.66 mL), TIPS (0.24 mL), and DCE (0.10 mL). Other fluoroalcohols such as 2,2,2-trifluoroethanol (TFE) may also be used instead of HFIP for this solution (a 0.05 M tetramethylammonium hydrogen sulfate/HFIP solution (2% TIPS)).

[Example 2] Evaluation of membrane permeability of amino acids having a donor in side chain and having intramolecular hydrogen bond in the donor moiety

2-1 Comparison of membrane permeability of amino acids having a donor in side chain and having or not having intramolecular hydrogen bond in donor moiety

To confirm that amino acids having a donor in the side chain and having an intramolecular hydrogen bond in the donor moiety has better membrane permeability than amino acids having a donor in side chain but not having intramolecular hydrogen bond in the donor moiety, two cyclic peptides containing respective amino acids were synthesized, and their membrane permeability was compared by the modified method described in WO 2018/124162. A plurality of combinations of such two cyclic peptides were synthesized and evaluated to verify generality. As a result, in the combinations of (1) Ser(EtOH) and Nle(6-OH), (2) Ser(1-CF3-EtOH) and Hnl(7-F3-6-OH), (3) Tyr(3-OMe) and Tyr, (4) Ser(S-4-F2-Pyrro-Me) and Nva(2-R-4-F2-Pyrro), and (5) Ser(NMe-Aca) and Gln(Me), peptides containing an amino acid having a donor in the side chain and having an intramolecular hydrogen bond in the donor moiety were confirmed as having better membrane permeability than peptides containing an amino acid having no intramolecular hydrogen bond in the donor moiety.

The sequences of two cyclic peptides to be compared were designed such that the influence of factors relating to membrane permeability other than the structure of the donor-containing side chain moiety was minimized.

For example, the amino acids to be compared were each inserted into the same position of the two cyclic peptides, such that the number of amino acids constituting the cyclic portion and the linear portion became the same (as a result, the total AA was the same). The number of donors in the side chain moieties of the whole of the two cyclic peptides became the same (an example of amino acids having a donor in the side chain other than the amino acid to be evaluated includes Thr). Furthermore, when there was an amino acid having a donor in the side chain other than the

amino acid to be compared, the sequence was designed such that the position of such an amino acid became the same. In addition, the peptides were designed so that the number of *N*-alkyl groups in the amide group moiety on the two cyclic peptide main chains became the same. Furthermore, the appearance pattern (*N*-alkyl pattern) of the *N*-alkyl group (such as an NMe group) and the NH group of an amide group, the position of Gly derivatives (including alkyl-Gly such as MeGly and nBuGly), and the position of cyclic amino acids such as Pro on the two cyclic peptide main chains were designed to be the same so that the two cyclic peptides to be compared had the same conformation. As described in Reference Example 4, the two peptides to be compared were regulated so that difference in ClogP/total AA values between them is within ± 0.03 and so as not to contain Trp. As described in Reference Example 6, the peptides were designed so that the number of aromatic rings (Aromatic Ring Count: ARC) became the same.

ClogP as used herein is a computer-calculated distribution coefficient, and was calculated using Daylight Version 4.9 of Daylight Chemical Information Systems, Inc.

(1) Comparison of Ser(EtOH) and Nle(6-OH) (Table 4)

Cyclic peptides containing Ser(EtOH) or Nle(6-OH) designed based on the above concept were synthesized, and their membrane permeability was compared by the modified method described in WO 2018/124162. The compound number of a sequence containing Ser(EtOH) is AXX (XX means a number), and the compound number of a sequence containing Nle(6-OH) is BXX (XX means a number). Compound numbers A01 and B01 denote respectively corresponding compounds, and, likewise, sequences having the same XX number such as AXX and BXX are respectively corresponding combinations below. When 10 combinations were compared, cyclic peptides containing Ser(EtOH) had better membrane permeability than cyclic peptides containing Nle(6-OH) in all combinations.

Hereinafter, an analysis was made in the same manner unless otherwise specifically stated.

Taking A01 as an example, the columns in Table 4, Table 6, Table 8, Table 10, Table 12, and Table 14 will be explained. A01 is a cyclic peptide composed of 11 residues, and forms a ring with 11 amino acid residues. The cyclized portion is defined as a first amino acid, and Asp corresponds to a first amino acid in the case of A01. An amino acid that constitutes the cyclic portion and that is next to Asp is defined as a second amino acid (to which MeLeu corresponds in the case of A01), and the third amino acid, the fourth amino acid, and so on are defined accordingly until the N-terminus (in the case of A01, the third amino acid is MePhe, the fourth amino acid is Ser(iPen), and the N-terminus is the 11th amino acid D-Val because the cyclic portion is composed of 11 residues). The C-term column means a functional group condensed with the carboxylic acid moiety of the C-terminal amino acid, and pip means piperidine. Table 4, Table 6, Table 8, Table 10, Table 12, and Table 14 show that the peptides are cyclized by the amide-bonding of the amino group of the N-terminus and the side-chain carboxylic acid of the amino acid of the cyclized portion. For example, A01 is cyclized by the amide-bonding of the amino group of D-Val at the N-terminus and the side-chain carboxylic acid of Asp of the cyclized portion. Unless otherwise specifically stated, all cyclic peptides set forth herein are described in the same manner.

(2) Comparison of Ser(1-CF3-EtOH) and Hnl(7-F3-6-OH) (Table 6)

The compound number of a sequence containing Ser(1-CF3-EtOH) is CXX, and the compound number of a sequence containing Hnl(7-F3-6-OH) is DXX. When 17 combinations were compared, cyclic peptides containing Ser(1-CF3-EtOH) had better membrane permeability than cyclic peptides containing Hnl(7-F3-6-OH) in 15 combinations.

(3) Comparison of Tyr(3-OMe) and Tyr (Table 8)

The compound number of a sequence containing Tyr(3-OMe) is EXX, and the compound number of a sequence containing Tyr is FXX. When 8 combinations were compared, cyclic peptides containing Tyr(3-OMe) had better membrane permeability than cyclic peptides containing Tyr in all combinations.

(4) Comparison of Ser(S-4-F2-Pyrro-Me) and Nva(2-R-4-F2-Pyrro) (Table 10)

The compound number of a sequence containing Ser(S-4-F2-Pyrro-Me) is GXX, and the compound number of a sequence containing Nva(2-R-4-F2-Pyrro) is HXX. When 11 combinations were compared, cyclic peptides containing Ser(S-4-F2-Pyrro-Me) had better membrane permeability than cyclic peptides containing Nva(2-R-4-F2-Pyrro) in 10 combinations.

(5) Comparison of Ser(NMe-Aca) and Gln(Me) (Table 12)

The compound number of a sequence containing Ser(NMe-Aca) is IXX, and the compound number of a sequence containing Gln(Me) is JXX. When six combinations were compared, cyclic peptides containing Ser(NtBu-Aca) had better membrane permeability than cyclic peptides containing Gln(Me) in all combinations.

[Table 4]

# Comparison of Ser(EtOH) and Nle(6-OH)

**Ser(EtOH)**

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| A01 | D-Val | MeHph | **Ser(EtOH)** | MeLeu | Thr | nPrGly | MeLeu | Ser(iPen) | MePhe | MeLeu | Asp | pip | 1.29 | 6.0E-07 |
| A02 | D-Val | MeHph | Leu | MeLeu | Thr | MeGly | MeLeu | **Ser(EtOH)** | MePhe(3-Cl) | MeLeu | Asp | pip | 1.23 | 4.5E-07 |
| A03 | gMeAbu | MeLeu | **Ser(EtOH)** | MeHph | MeHph | Leu | MeLeu | Thr | MeLeu | MeLeu | Asp | pip | 1.23 | 3.2E-07 |
| A04 | gMeAbu | MeLeu | Ile | MeHph | MeHph | **Ser(EtOH)** | MeLeu | Thr | MeLeu | MeLeu | Asp | pip | 1.23 | 4.1E-07 |
| A05 | MeAla | **Ser(EtOH)** | MeLeu | MePhe(3-Cl) | Leu | MeHph | MeLeu | Thr | nPrGly | MeLeu | Asp | pip | 1.30 | 9.0E-07 |
| A06 | MeVal | Leu | MeLeu | MePhe | **Ser(EtOH)** | MeHph | MeLeu | Thr | MeGly | MeLeu | Asp | pip | 1.22 | 5.3E-07 |
| A07 | MeGly | MePhe(3-Cl) | **Ser(EtOH)** | MeLeu | Thr | nPrGly | MeHph | MeLeu | Ser(iPen) | MeLeu | Asp | pip | 1.27 | 5.3E-07 |
| A08 | MeGly | MeHph | Ile | MeLeu | Thr | nPrGly | MePhe(3-Cl) | MeLeu | **Ser(EtOH)** | MeLeu | Asp | pip | 1.25 | 3.2E-07 |
| A09 | MeVal | **Ser(EtOH)** | MeLeu | MePhe(3-Cl) | Leu | MeHph | MeLeu | Thr | MeGly | MeLeu | Asp | pip | 1.28 | 7.2E-07 |
| A10 | D-Val | MePhe | Leu | MeLeu | Thr | nPrGly | MeLeu | **Ser(EtOH)** | MePhe(3-Cl) | MeLeu | Asp | pip | 1.29 | 8.0E-07 |

**Nle(6-OH)**

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| B01 | D-Abu | MeHph | **Nle(6-OH)** | MeLeu | Thr | nPrGly | MeLeu | Ser(iPen) | MePhe | MeLeu | Asp | pip | 1.30 | < 1.1E-07 |
| B02 | D-Abu | MeHph | Leu | MeLeu | Thr | MeGly | MeLeu | **Nle(6-OH)** | MePhe(3-Cl) | MeLeu | Asp | pip | 1.24 | 1.5E-07 |
| B03 | gMeAbu | MeVal | **Nle(6-OH)** | MeHph | MeHph | Leu | MeLeu | Thr | MeLeu | MeLeu | Asp | pip | 1.23 | 2.7E-07 |
| B04 | gMeAbu | MeLeu | Val | MeHph | MeHph | **Nle(6-OH)** | MeLeu | Thr | MeLeu | MeLeu | Asp | pip | 1.23 | 7.0E-08 |
| B05 | MeAla | **Nle(6-OH)** | MeLeu | MePhe(3-Cl) | Val | MeHph | MeLeu | Thr | nPrGly | MeLeu | Asp | pip | 1.30 | 2.6E-07 |
| B06 | MeAbu | Leu | MeLeu | MePhe | **Nle(6-OH)** | MeHph | MeLeu | Thr | MeGly | MeLeu | Asp | pip | 1.23 | 3.9E-07 |
| B07 | MeGly | MePhe(3-Cl) | **Nle(6-OH)** | MeVal | Thr | nPrGly | MeHph | MeLeu | Ser(iPen) | MeLeu | Asp | pip | 1.27 | 2.1E-07 |
| B08 | MeGly | MeHph | Val | MeLeu | Thr | nPrGly | MePhe(3-Cl) | MeLeu | **Nle(6-OH)** | MeLeu | Asp | pip | 1.25 | 1.5E-07 |
| B09 | MeAbu | **Nle(6-OH)** | MeLeu | MePhe(3-Cl) | Leu | MeHph | MeLeu | Thr | MeGly | MeLeu | Asp | pip | 1.29 | 3.1E-07 |
| B10 | D-Abu | MePhe | Leu | MeLeu | Thr | nPrGly | MeLeu | **Nle(6-OH)** | MePhe(3-Cl) | MeLeu | Asp | pip | 1.29 | 2.5E-07 |

Structures of cyclic peptides A01 to B10 are shown below. (In the structural formulae of the cyclic peptides, hydrogen atoms attached to a heteroatom may be omitted to clarify the structure.)

[Table 5]

| ID | Structural Formula | ID | Structural Formula |
|---|---|---|---|
| A01 | 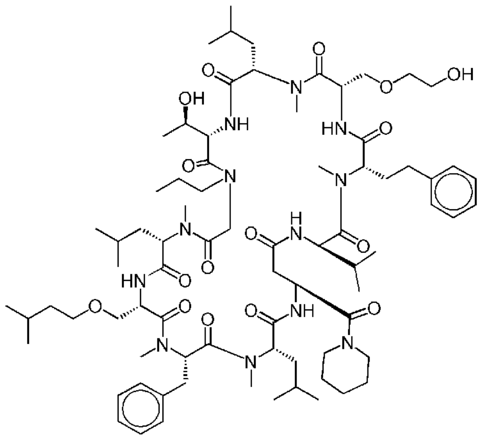 | A02 | 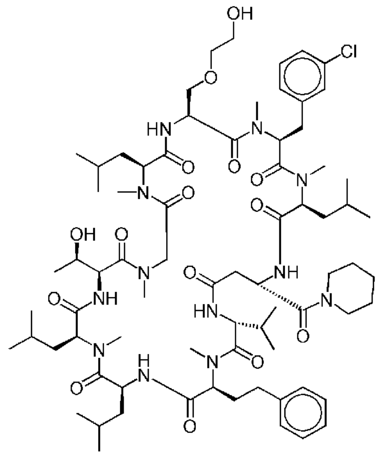 |
| A03 | 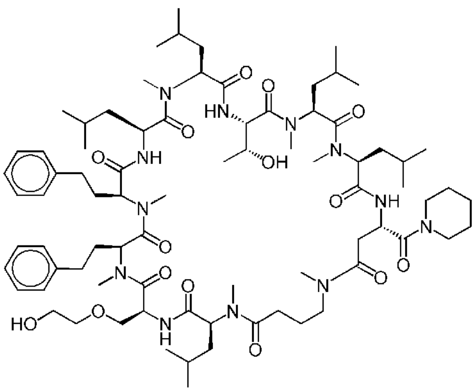 | A04 | 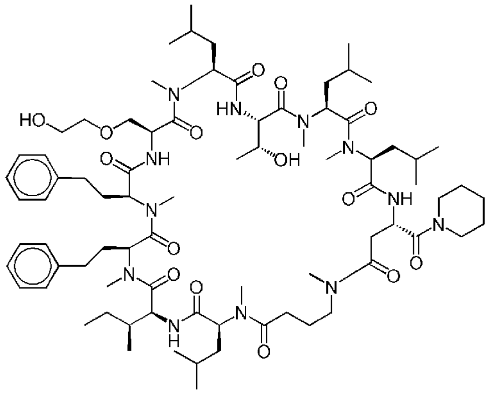 |
| A05 | 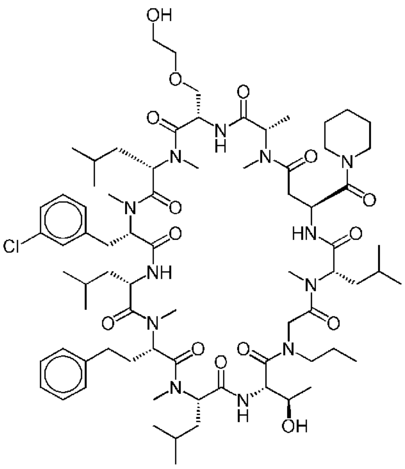 | A06 | 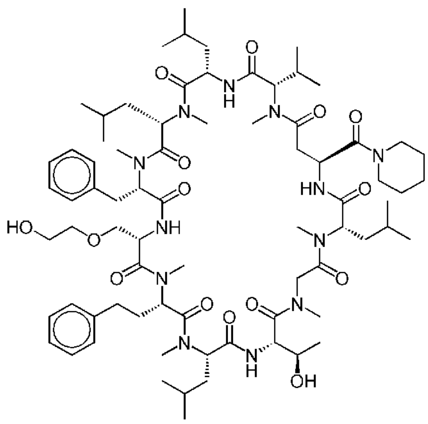 |

(continued)

| ID | Structural Formula | ID | Structural Formula |
|---|---|---|---|
| A07 | 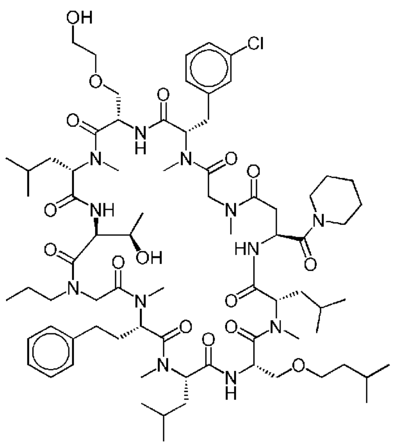 | A08 | 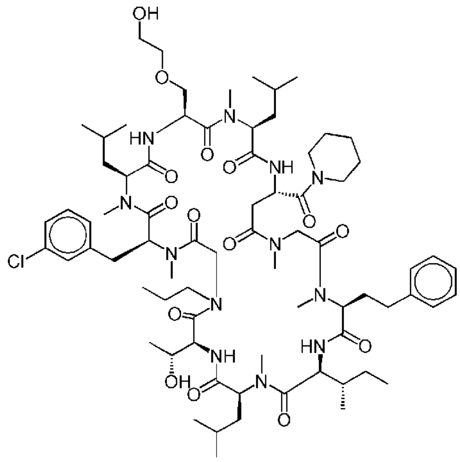 |
| A09 | 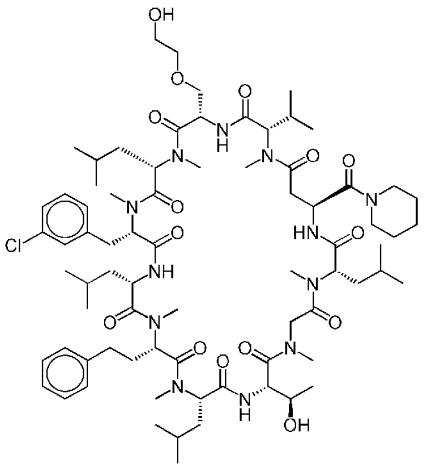 | A10 | 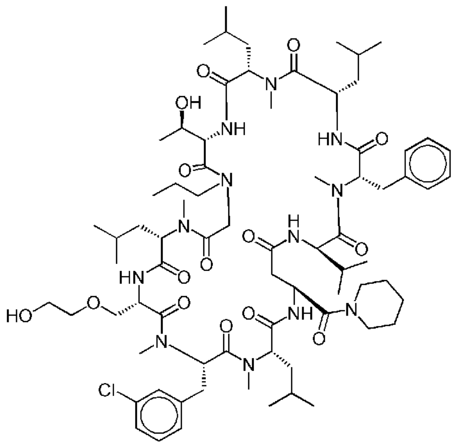 |
| B01 | 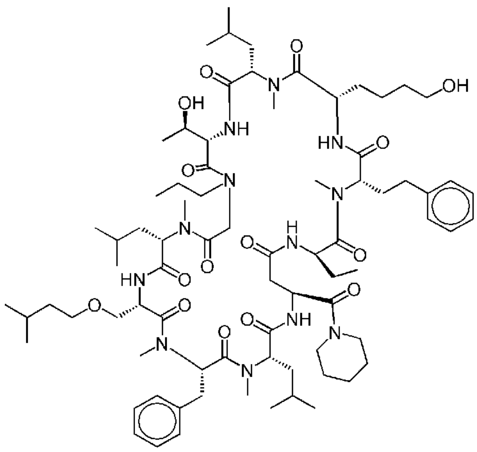 | B02 | 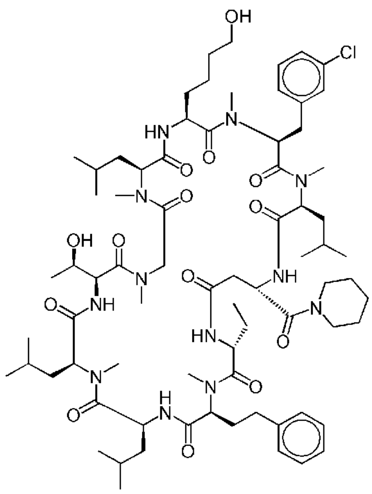 |

(continued)

| ID | Structural Formula | ID | Structural Formula |
|---|---|---|---|
| B03 | 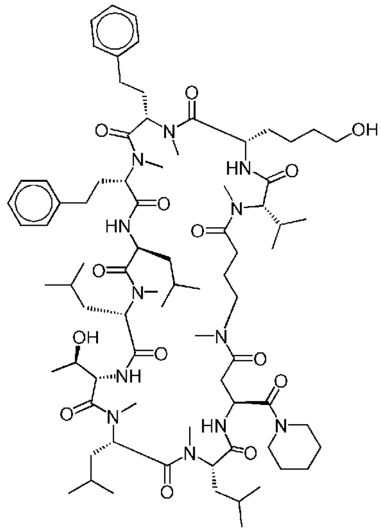 | B04 | 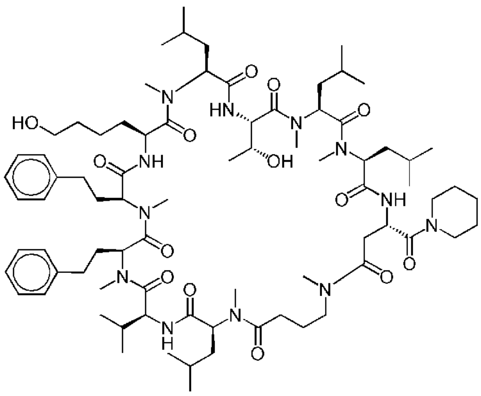 |
| B05 | 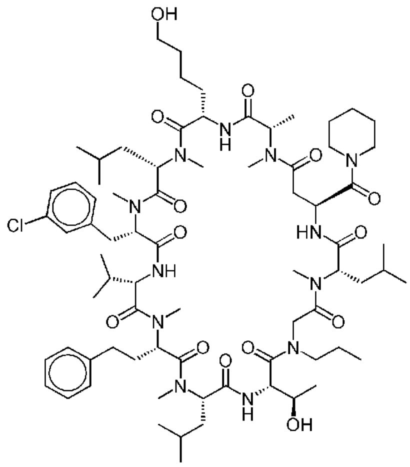 | B06 | 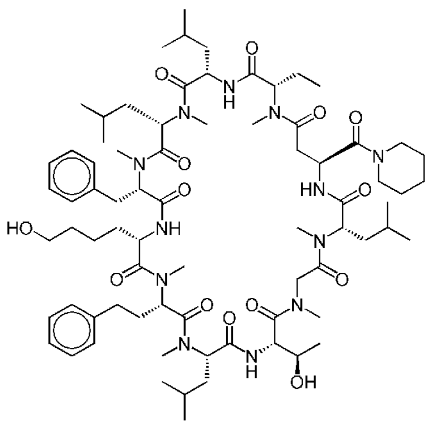 |
| B07 | 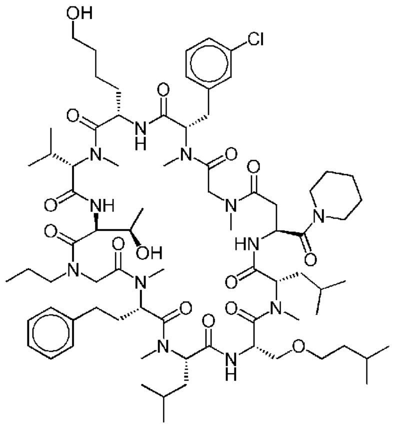 | B08 | 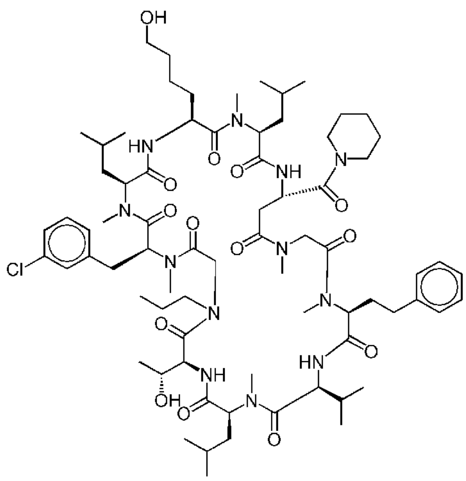 |

(continued)

| ID | Structural Formula | ID | Structural Formula |
|---|---|---|---|
| B09 | 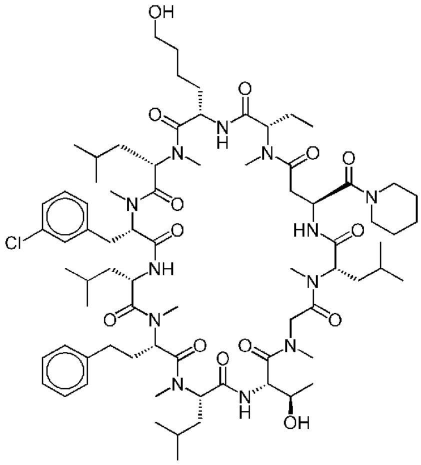 | B10 | 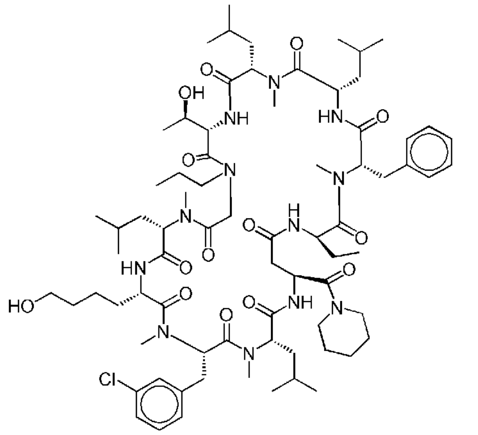 |

[Table 6]

# Comparison of Ser(1-CF3-EtOH) and Hnl(7-F3-6-OH)

Ser(1-CF3-EtOH)

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| C01 | D-Val | MePhe | **Ser(1-CF3-EtOH)** | MeLeu | Thr | nBuGly | MeLeu | Ser(tBu) | MePhe | MeLeu | Asp | pip | 1.30 | 1.5E-07 |
| C02 | gMeAbu | MeLeu | **Ser(1-CF3-EtOH)** | MePhe(3-Cl) | MePhe | Ser(tBu) | MeLeu | Thr | MeLeu | MeLeu | Asp | pip | 1.23 | 1.9E-07 |
| C03 | gMeAbu | MeLeu | Ile | MePhe | MePhe | **Ser(1-CF3-EtOH)** | MeLeu | Thr | MeLeu | MeLeu | Asp | pip | 1.22 | 5.0E-07 |
| C04 | MeAla | **Ser(1-CF3-EtOH)** | MeLeu | MePhe | Leu | MePhe | MeIle | Thr | nBuGly | MeLeu | Asp | pip | 1.32 | 2.9E-07 |
| C05 | MeVal | Leu | MeLeu | MePhe | **Ser(1-CF3-EtOH)** | MePhe | MeIle | Thr | MeGly | MeLeu | Asp | pip | 1.25 | 5.7E-07 |
| C06 | MeGly | MePhe(3-Cl) | **Ser(1-CF3-EtOH)** | MeLeu | Thr | nBuGly | MePhe | MeLeu | Ser(tBu) | MeLeu | Asp | pip | 1.29 | 4.8E-07 |
| C07 | MeGly | MePhe | Ile | MeLeu | Thr | nBuGly | MePhe | MeLeu | **Ser(1-CF3-EtOH)** | MeLeu | Asp | pip | 1.27 | 4.5E-07 |
| C08 | MeVal | **Ser(1-CF3-EtOH)** | MeLeu | MePhe | Leu | MePhe | MeIle | Thr | MeGly | MeLeu | Asp | pip | 1.25 | 6.0E-07 |
| C09 | D-Val | MePhe | Leu | MeLeu | Thr | nBuGly | MeLeu | **Ser(1-CF3-EtOH)** | MePhe | MeVal | Asp | pip | 1.31 | 1.2E-07 |
| C10 | D-Leu | MePhe | Leu | MeAbu | **Ser(1-CF3-EtOH)** | MeGly | MeAbu | Ser(tBu) | MePhe | MeLeu | Asp | pip | 1.22 | 9.5E-07 |
| C11 | D-Val | MePhe | **Ser(1-CF3-EtOH)** | MeLeu | Val | MeGly | MeLeu | Ser(tBu) | MePhe | MeLeu | Asp | pip | 1.29 | 2.3E-07 |
| C12 | gMeAbu | MeLeu | Val | MePhe | MePhe | Ser(tBu) | MeLeu | **Ser(1-CF3-EtOH)** | MeLeu | MeLeu | Asp | pip | 1.31 | 2.3E-07 |
| C13 | gMeAbu | MeAbu | **Ser(1-CF3-EtOH)** | MePhe | MePhe | Ser(tBu) | MeLeu | Ile | MeLeu | MeLeu | Asp | pip | 1.27 | 2.6E-07 |
| C14 | MeAla | Leu | MeAbu | MePhe | Leu | MePhe | MeVal | **Ser(1-CF3-EtOH)** | MeGly | MeLeu | Asp | pip | 1.23 | 2.0E-06 |
| C15 | MeAla | **Ser(1-CF3-EtOH)** | MeLeu | MePhe | Leu | MePhe | MeIle | Val | MeGly | MeLeu | Asp | pip | 1.31 | 8.3E-07 |
| C16 | MeGly | MePhe | Val | MeAbu | **Ser(1-CF3-EtOH)** | nBuGly | MePhe | MeLeu | Ser(tBu) | MeLeu | Asp | pip | 1.28 | 4.1E-07 |
| C17 | MeGly | MePhe | **Ser(1-CF3-EtOH)** | MeLeu | Ile | MeGly | MePhe | MeLeu | Ser(tBu) | MeLeu | Asp | pip | 1.26 | 4.1E-06 |

Hnl(7-F3-6-OH)

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| D01 | D-Val | MePhe | **Hnl(7-F3-6-OH)** | MeLeu | Thr | nBuGly | MeLeu | Ser(tBu) | MePhe | MeLeu | Asp | pip | 1.32 | 7.0E-08 |
| D02 | gMeAbu | MeLeu | **Hnl(7-F3-6-OH)** | MePhe(3-Cl) | MePhe | Ser(tBu) | MeLeu | Thr | MeLeu | MeLeu | Asp | pip | 1.24 | 1.4E-07 |
| D03 | gMeAbu | MeLeu | Ile | MePhe | MePhe | **Hnl(7-F3-6-OH)** | MeLeu | Thr | MeLeu | MeLeu | Asp | pip | 1.23 | 3.3E-07 |
| D04 | MeAla | **Hnl(7-F3-6-OH)** | MeLeu | MePhe | Leu | MePhe | MeIle | Thr | nBuGly | MeLeu | Asp | pip | 1.33 | 2.0E-07 |
| D05 | MeVal | Leu | MeLeu | MePhe | **Hnl(7-F3-6-OH)** | MePhe | MeIle | Thr | MeGly | MeLeu | Asp | pip | 1.27 | 3.6E-07 |
| D06 | MeGly | MePhe(3-Cl) | **Hnl(7-F3-6-OH)** | MeLeu | Thr | nBuGly | MePhe | MeLeu | Ser(tBu) | MeLeu | Asp | pip | 1.30 | 2.3E-07 |
| D07 | MeGly | MePhe | Ile | MeLeu | Thr | nBuGly | MePhe | MeLeu | **Hnl(7-F3-6-OH)** | MeLeu | Asp | pip | 1.29 | 3.7E-07 |
| D08 | MeVal | **Hnl(7-F3-6-OH)** | MeLeu | MePhe | Leu | MePhe | MeIle | Thr | MeGly | MeLeu | Asp | pip | 1.27 | 5.3E-07 |
| D09 | D-Val | MePhe | Leu | MeLeu | Thr | nBuGly | MeLeu | **Hnl(7-F3-6-OH)** | MePhe | MeVal | Asp | pip | 1.32 | 1.3E-07 |
| D10 | D-Leu | MePhe | Leu | MeAbu | **Hnl(7-F3-6-OH)** | MeGly | MeAbu | Ser(tBu) | MePhe | MeLeu | Asp | pip | 1.23 | 8.1E-07 |
| D11 | D-Val | MePhe | **Hnl(7-F3-6-OH)** | MeLeu | Val | MeGly | MeLeu | Ser(tBu) | MePhe | MeLeu | Asp | pip | 1.31 | 2.1E-07 |
| D12 | gMeAbu | MeLeu | Val | MePhe | MePhe | Ser(tBu) | MeLeu | **Hnl(7-F3-6-OH)** | MeLeu | MeLeu | Asp | pip | 1.32 | 1.3E-07 |
| D13 | gMeAbu | MeAbu | **Hnl(7-F3-6-OH)** | MePhe | MePhe | Ser(tBu) | MeLeu | Ile | MeLeu | MeLeu | Asp | pip | 1.29 | 1.4E-07 |
| D14 | MeAla | Leu | MeAbu | MePhe | Leu | MePhe | MeVal | **Hnl(7-F3-6-OH)** | MeGly | MeLeu | Asp | pip | 1.24 | 9.0E-07 |
| D15 | MeAla | **Hnl(7-F3-6-OH)** | MeLeu | MePhe | Leu | MePhe | MeIle | Val | MeGly | MeLeu | Asp | pip | 1.32 | 6.1E-07 |
| D16 | MeGly | MePhe | Val | MeAbu | **Hnl(7-F3-6-OH)** | nBuGly | MePhe | MeLeu | Ser(tBu) | MeLeu | Asp | pip | 1.30 | 5.1E-07 |
| D17 | MeGly | MePhe | **Hnl(7-F3-6-OH)** | MeLeu | Ile | MeGly | MePhe | MeLeu | Ser(tBu) | MeLeu | Asp | pip | 1.27 | 1.0E-06 |

Structures of cyclic peptides C01 to D17 are shown below. (In the structural formulae of the cyclic peptides, hydrogen atoms attached to a heteroatom may be omitted to clarify the structure.)

[Table 7]

| ID | Structural Formula | ID | Structural Formula |
|---|---|---|---|
| C01 | 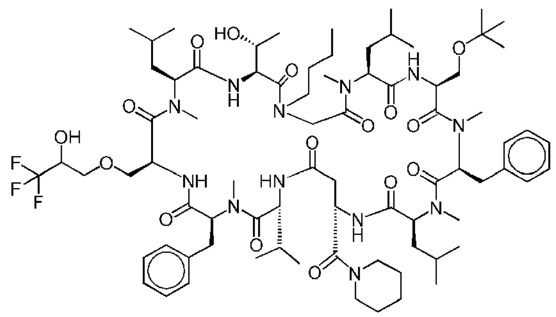 | C02 | 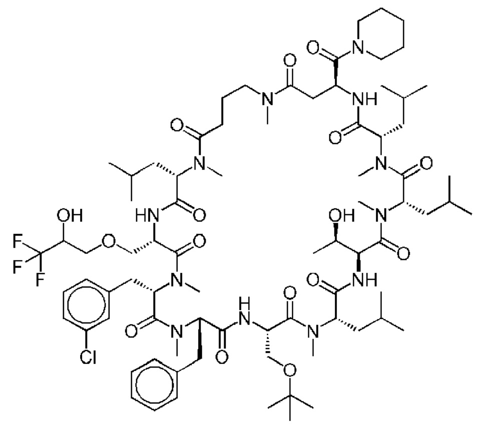 |
| C03 | 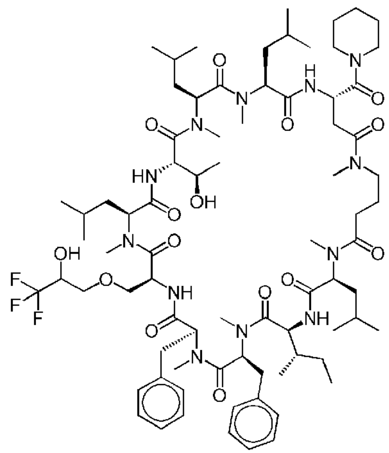 | C04 | 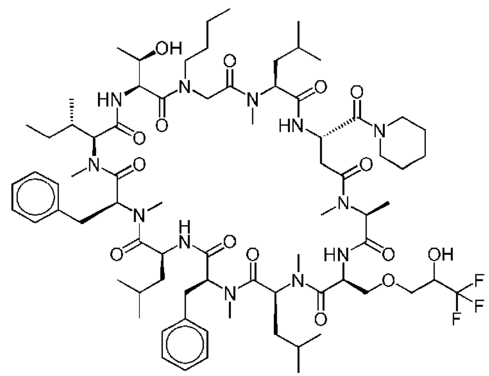 |

(continued)

| ID | Structural Formula | ID | Structural Formula |
|---|---|---|---|
| C05 | 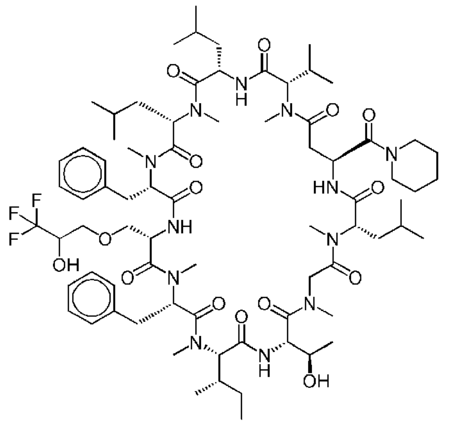 | C06 | 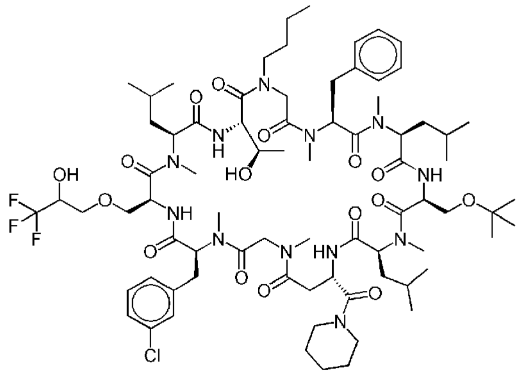 |
| C07 | 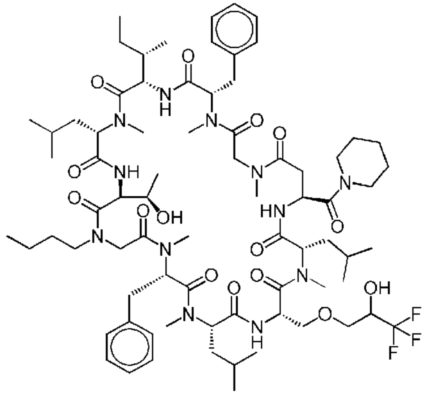 | C08 | 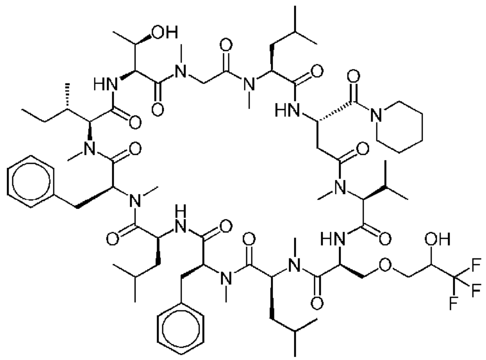 |

(continued)

| ID | Structural Formula | ID | Structural Formula |
|---|---|---|---|
| C09 | 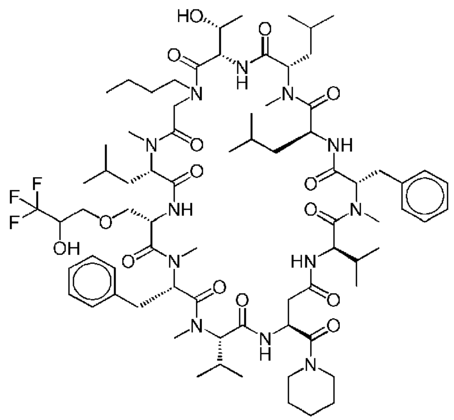 | C10 | 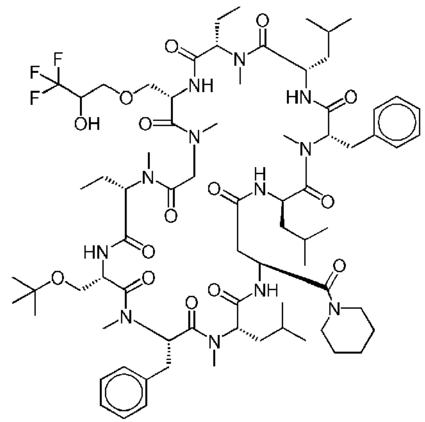 |
| C11 | 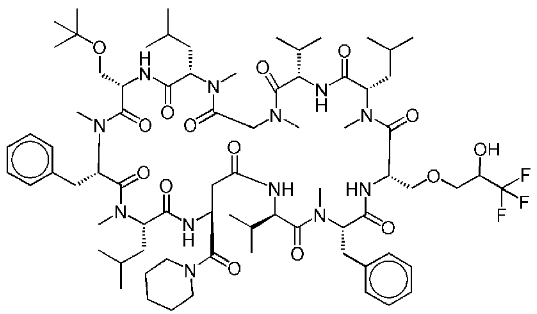 | C12 | 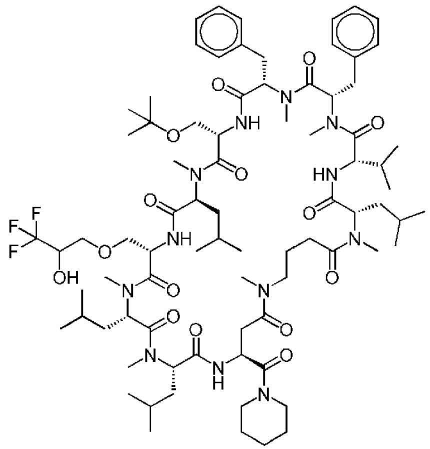 |

(continued)

| ID | Structural Formula | ID | Structural Formula |
|---|---|---|---|
| C13 | 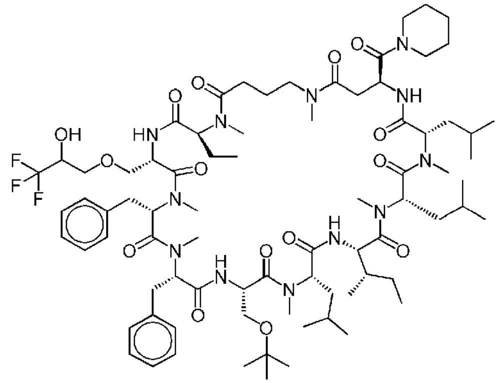 | C14 | 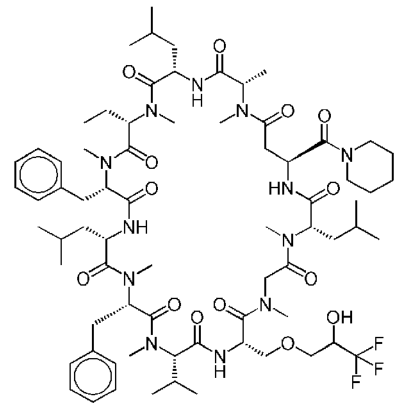 |
| C15 | 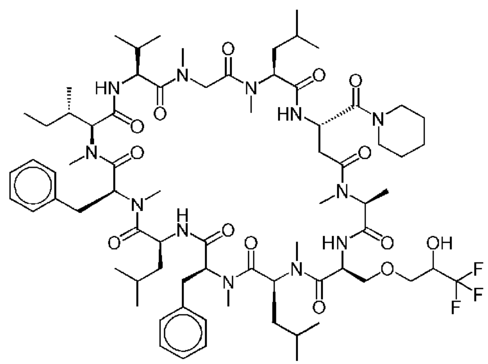 | C16 | 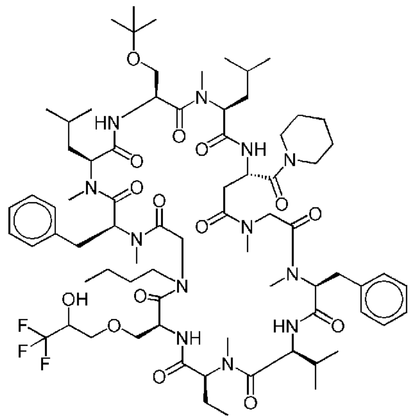 |

(continued)

| ID | Structural Formula | ID | Structural Formula |
| --- | --- | --- | --- |
| C17 | 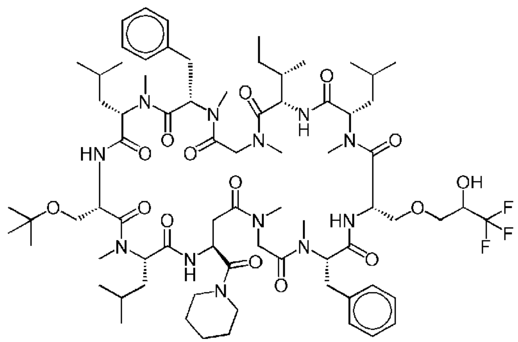 | D01 | 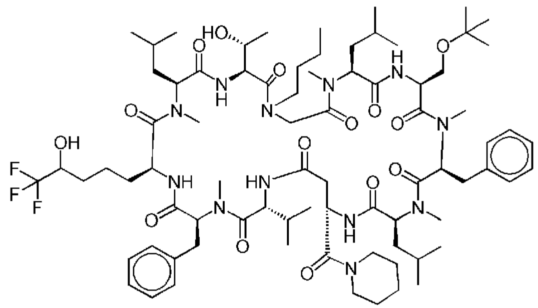 |
| D02 | 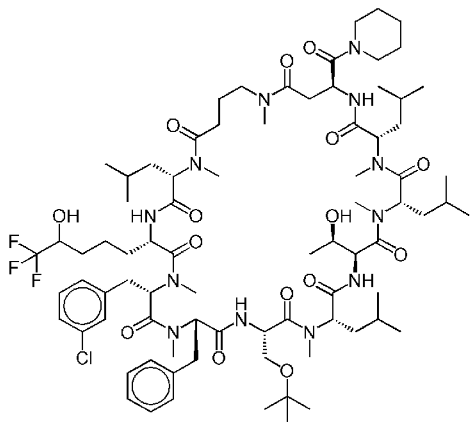 | D03 | 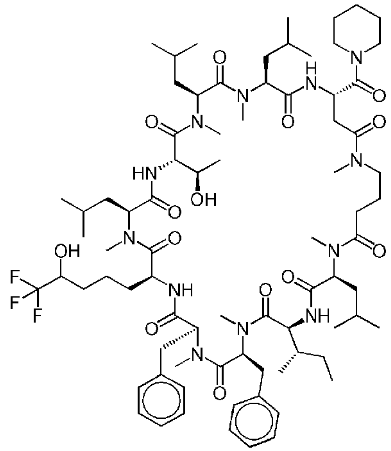 |

(continued)

| ID | Structural Formula | ID | Structural Formula |
|---|---|---|---|
| D04 | 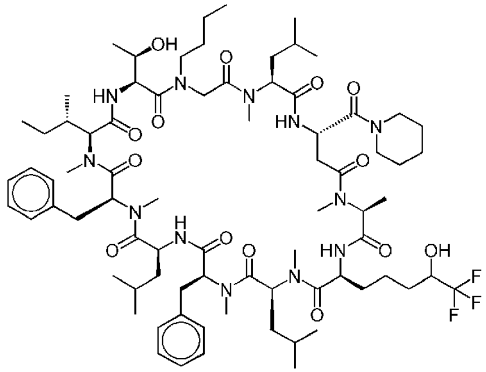 | D05 | 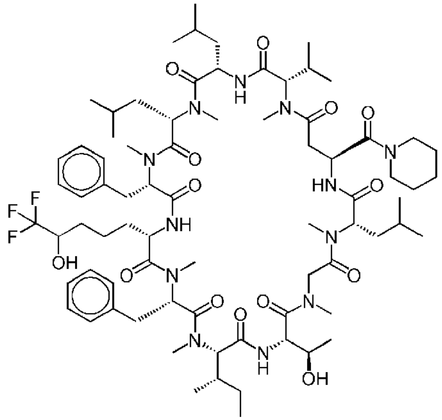 |
| D06 | 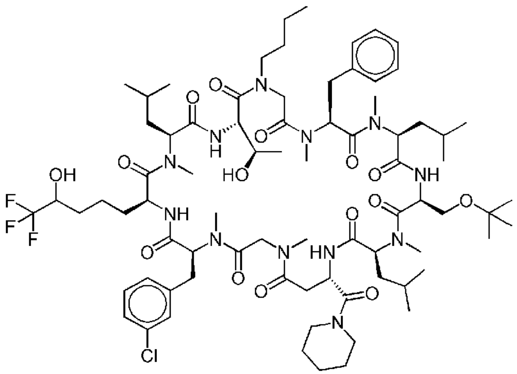 | D07 | 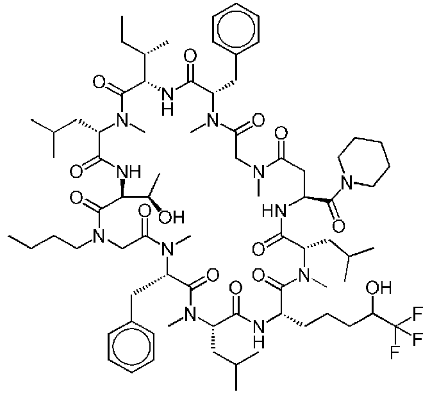 |

(continued)

| ID | Structural Formula | ID | Structural Formula |
|---|---|---|---|
| D08 | 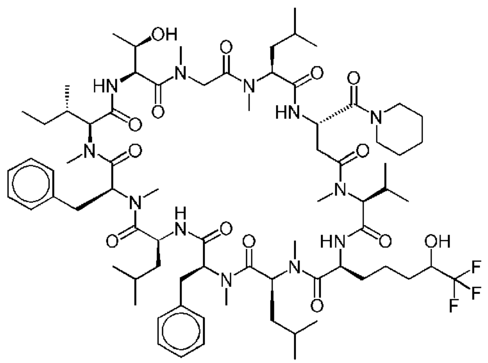 | D09 | 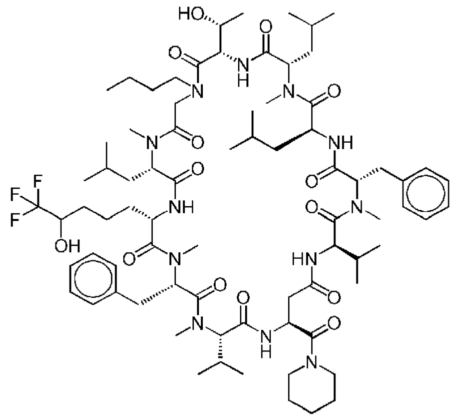 |
| D10 | 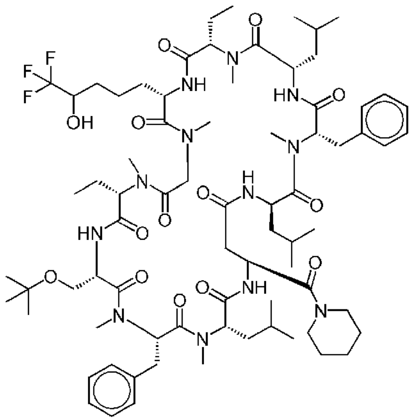 | D11 | 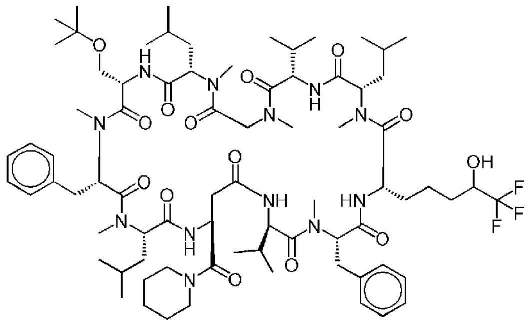 |

(continued)

| ID | Structural Formula | ID | Structural Formula |
|---|---|---|---|
| D12 | 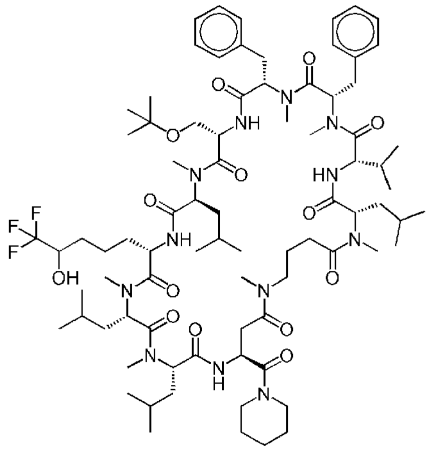 | D13 | 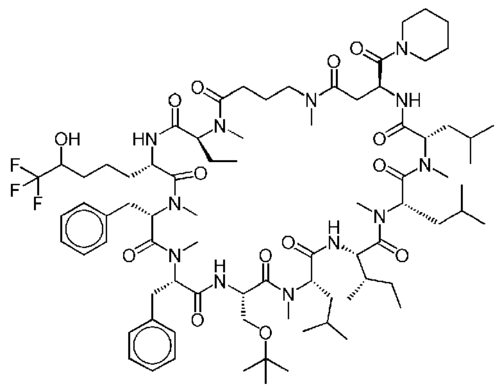 |
| D14 | 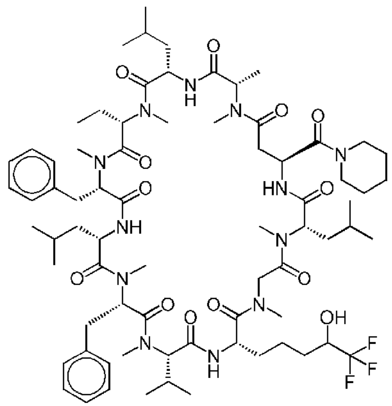 | D15 | 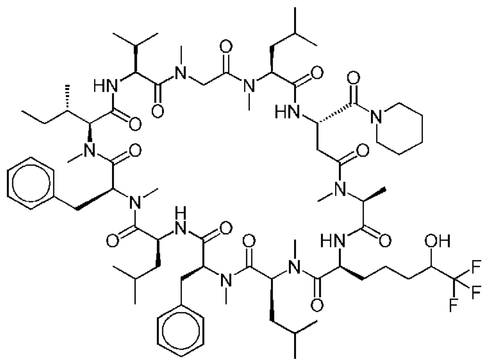 |

(continued)

| ID | Structural Formula | ID | Structural Formula |
|---|---|---|---|
| D16 | 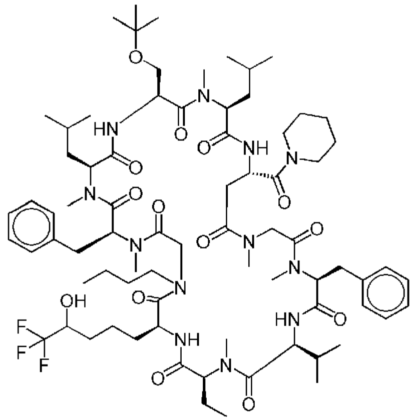 | D17 | 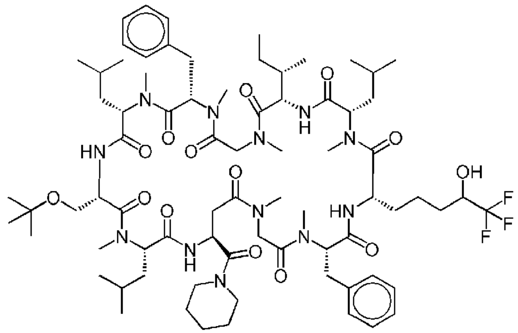 |

[Table 8]

Comparison of Tyr(3-OMe) and Tyr

Tyr(3-OMe)

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| E01 | D-Val | MePhe | Leu | MeLeu | Thr | MeGly | MeLeu | **Tyr(3-OMe)** | MeLeu | MeLeu | Asp | pip | 1.26 | 1.1E-06 |
| E02 | D-Leu | MePhe | **Tyr(3-OMe)** | MeLeu | Thr | nBuGly | MeLeu | Ser(tBu) | MeLeu | MeAbu | Asp | pip | 1.32 | 3.1E-07 |
| E03 | gMeAbu | MeLeu | Ile | MePhe | MeLeu | **Tyr(3-OMe)** | MeLeu | Thr | MeLeu | MeLeu | Asp | pip | 1.27 | 1.8E-07 |
| E04 | gMeAbu | MeLeu | **Tyr(3-OMe)** | MeLeu | MePhe | Ser(tBu) | MeLeu | Thr | MeLeu | MeLeu | Asp | pip | 1.22 | 4.0E-07 |
| E05 | MeAbu | Leu | MeLeu | MePhe | **Tyr(3-OMe)** | MeLeu | MeLeu | Thr | MeGly | MeLeu | Asp | pip | 1.27 | 1.8E-06 |
| E06 | MeAbu | **Tyr(3-OMe)** | MeLeu | MeLeu | Val | MePhe | MeLeu | Thr | MeGly | MeLeu | Asp | pip | 1.22 | 9.7E-07 |
| E07 | MeGly | MeLeu | **Tyr(3-OMe)** | MeAbu | Thr | nBuGly | MePhe | MeLeu | Leu | MeLeu | Asp | pip | 1.24 | 1.1E-06 |
| E08 | MeGly | MeLeu | Ile | MeLeu | Thr | nBuGly | MePhe | MeLeu | **Tyr(3-OMe)** | MeLeu | Asp | pip | 1.33 | 1.2E-07 |

Tyr

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| F01 | D-Val | MePhe | Leu | MeLeu | Thr | MeGly | MeLeu | **Tyr** | MeLeu | MeLeu | Asp | pip | 1.27 | 3.2E-07 |
| F02 | D-Leu | MePhe | **Tyr** | MeLeu | Thr | nBuGly | MeLeu | Ser(tBu) | MeLeu | MeAbu | Asp | pip | 1.34 | 4.0E-08 |
| F03 | gMeAbu | MeLeu | Ile | MePhe | MeLeu | **Tyr** | MeLeu | Thr | MeLeu | MeLeu | Asp | pip | 1.29 | 1.3E-07 |
| F04 | gMeAbu | MeLeu | **Tyr** | MeLeu | MePhe | Ser(tBu) | MeLeu | Thr | MeLeu | MeLeu | Asp | pip | 1.24 | 1.3E-07 |
| F05 | MeAbu | Leu | MeLeu | MePhe | **Tyr** | MeLeu | MeLeu | Thr | MeGly | MeLeu | Asp | pip | 1.29 | 3.8E-07 |
| F06 | MeAbu | **Tyr** | MeLeu | MeLeu | Val | MePhe | MeLeu | Thr | MeGly | MeLeu | Asp | pip | 1.24 | 2.3E-07 |
| F07 | MeGly | MeLeu | **Tyr** | MeAbu | Thr | nBuGly | MePhe | MeLeu | Leu | MeLeu | Asp | pip | 1.26 | 5.4E-07 |
| F08 | MeGly | MeLeu | Ile | MeLeu | Thr | nBuGly | MePhe | MeLeu | **Tyr** | MeLeu | Asp | pip | 1.34 | 5.0E-08 |

Structures of cyclic peptides E01 to F08 are shown below. (In the structural formulae of the cyclic peptides, hydrogen atoms attached to a heteroatom may be omitted to clarify the structure.)

[Table 9]

| ID | Structural Formula | ID | Structural Formula |
|---|---|---|---|
| E01 | 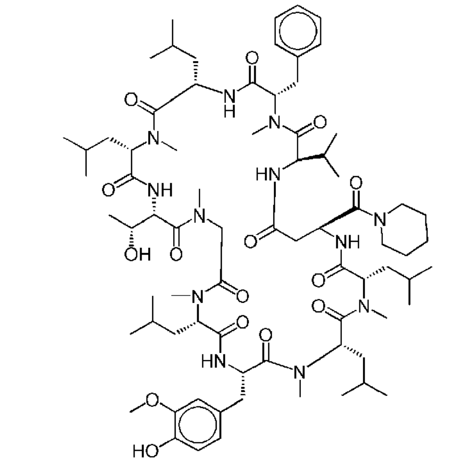 | E02 | 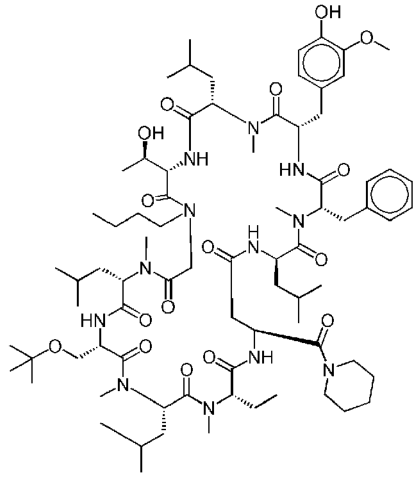 |
| E03 | 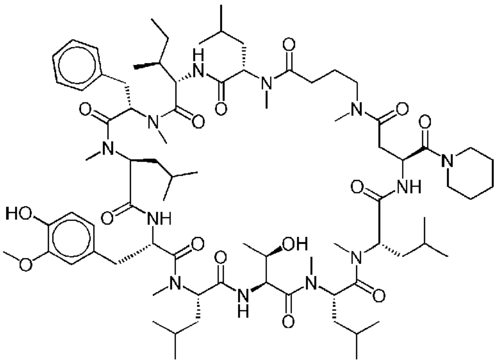 | E04 | 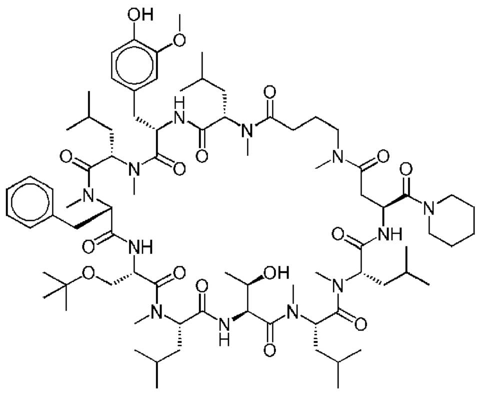 |
| E05 | 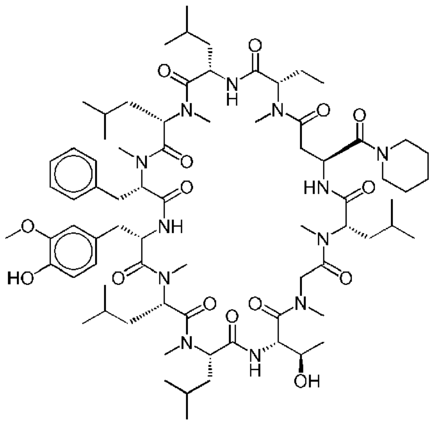 | E06 | 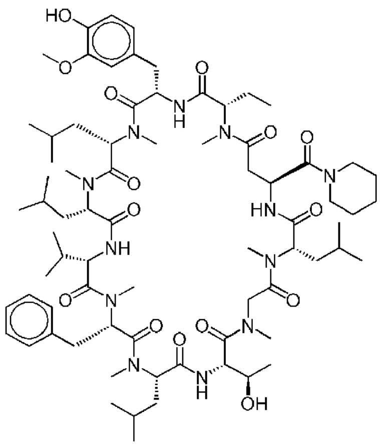 |

(continued)

| ID | Structural Formula | ID | Structural Formula |
|---|---|---|---|
| E07 | 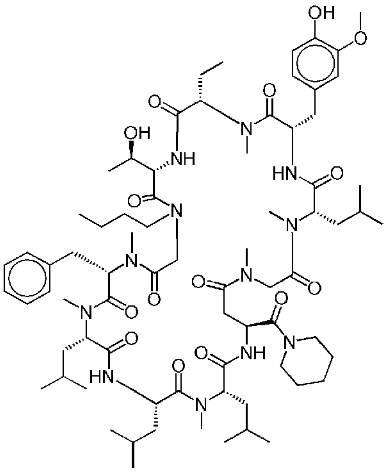 | E08 | 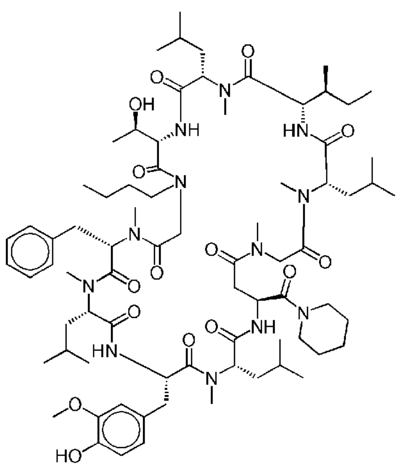 |
| F01 | 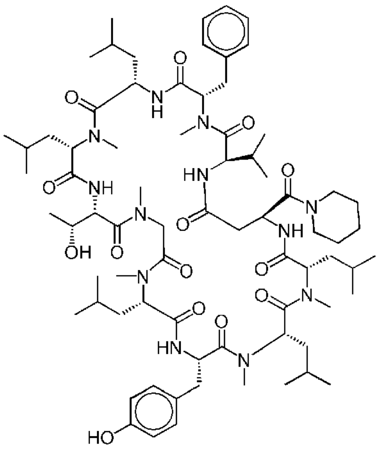 | F02 | 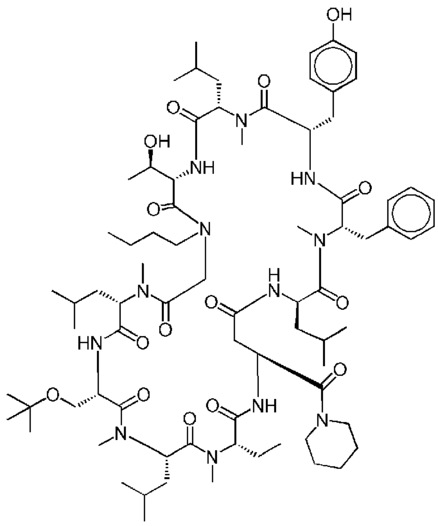 |
| F03 | 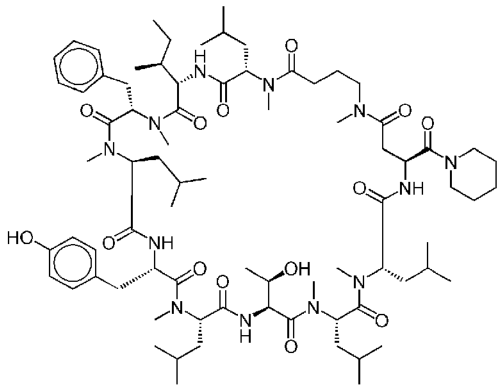 | F04 | 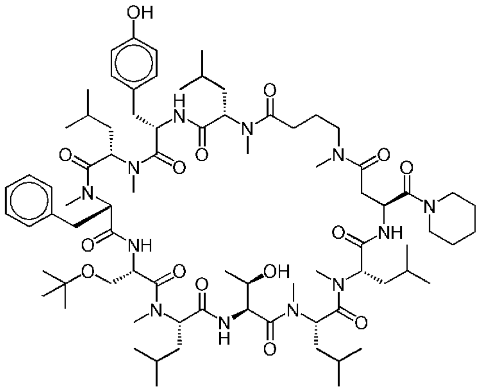 |

(continued)

| ID | Structural Formula | ID | Structural Formula |
| --- | --- | --- | --- |
| F05 | 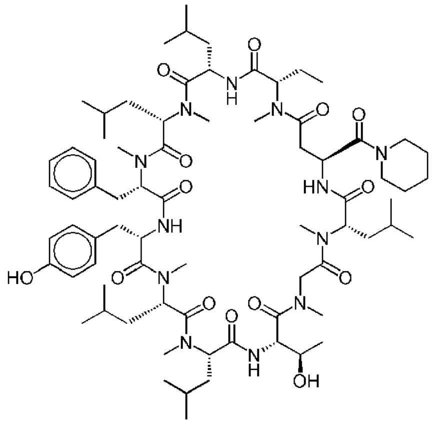 | F06 | 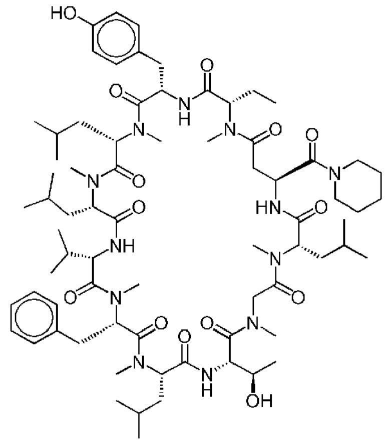 |
| F07 | 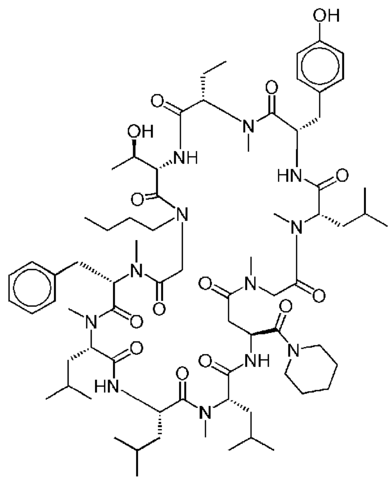 | F08 | 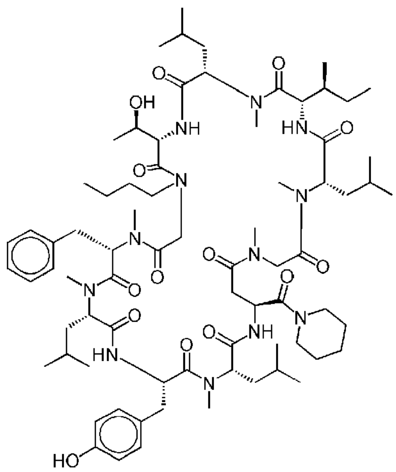 |

[Table 10]

# Comparison of Ser(S-4-F2-Pyrro-Me) and Nva(2-R-4-F2-Pyrro)

**Ser(S-4-F2-Pyrro-Me)**

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| G01 | D-Val | MePhe | Leu | MeAbu | Thr | nBuGly | MeLeu | **Ser(S-4-F2-Pyrro-Me)** | MeLeu | MeLeu | Asp | pip | 1.31 | 5.3E-07 |
| G02 | D-Leu | MeLeu | Val | MeLeu | Thr | nBuGly | MeAbu | **Ser(S-4-F2-Pyrro-Me)** | MePhe | MeAbu | Asp | pip | 1.23 | 4.0E-07 |
| G03 | gMeAbu | MeLeu | Ile | MePhe | MeLeu | **Ser(S-4-F2-Pyrro-Me)** | MeLeu | Thr | MeLeu | MeLeu | Asp | pip | 1.26 | 2.3E-07 |
| G04 | MeAbu | Leu | MeLeu | MePhe | **Ser(S-4-F2-Pyrro-Me)** | MeAbu | MeLeu | Thr | nBuGly | MeLeu | Asp | pip | 1.33 | 1.8E-06 |
| G05 | MeAla | **Ser(S-4-F2-Pyrro-Me)** | MeLeu | MeAbu | Val | MePhe | MeLeu | Thr | nBuGly | MeLeu | Asp | pip | 1.23 | 3.2E-06 |
| G06 | MeGly | MeLeu | **Ser(S-4-F2-Pyrro-Me)** | MeLeu | Thr | nBuGly | MePhe | MeLeu | Val | MeLeu | Asp | pip | 1.27 | 1.8E-06 |
| G07 | MeGly | MeLeu | Ile | MeLeu | Thr | nBuGly | MePhe | MeLeu | **Ser(S-4-F2-Pyrro-Me)** | MeLeu | Asp | pip | 1.31 | 5.7E-07 |
| G08 | D-Ala | MeAbu | Val | MeLeu | **Ser(S-4-F2-Pyrro-Me)** | nBuGly | MeLeu | Ser(tBu) | MePhe | MeAbu | Asp | pip | 1.23 | 1.3E-06 |
| G09 | gMeAbu | MeLeu | Val | MePhe | MeAbu | Ser(tBu) | MeLeu | **Ser(S-4-F2-Pyrro-Me)** | MeLeu | MeLeu | Asp | pip | 1.27 | 1.2E-06 |
| G10 | MeAla | Leu | MeAbu | MePhe | Leu | MeLeu | MeLeu | **Ser(S-4-F2-Pyrro-Me)** | MeGly | MeLeu | Asp | pip | 1.32 | 1.1E-06 |
| G11 | MeGly | MeAbu | Val | MeAbu | **Ser(S-4-F2-Pyrro-Me)** | nBuGly | MePhe | MeLeu | Leu | MeLeu | Asp | pip | 1.29 | 1.5E-06 |

**Nva(2-R-4-F2-Pyrro)**

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| H01 | D-Abu | MePhe | Leu | MeAbu | Thr | nBuGly | MeLeu | **Nva(2-R-4-F2-Pyrro)** | MeLeu | MeLeu | Asp | pip | 1.33 | 2.5E-07 |
| H02 | D-Leu | MeLeu | Val | MeLeu | Thr | nBuGly | MeAla | **Nva(2-R-4-F2-Pyrro)** | MePhe | MeAbu | Asp | pip | 1.23 | 1.4E-07 |
| H03 | gMeAbu | MeLeu | Val | MePhe | MeLeu | **Nva(2-R-4-F2-Pyrro)** | MeLeu | Thr | MeLeu | MeLeu | Asp | pip | 1.26 | < 1.8E-07 |
| H04 | MeAbu | Leu | MeLeu | MePhe | **Nva(2-R-4-F2-Pyrro)** | MeAla | MeLeu | Thr | nBuGly | MeLeu | Asp | pip | 1.33 | 9.3E-07 |
| H05 | MeAla | **Nva(2-R-4-F2-Pyrro)** | MeLeu | MeAla | Val | MePhe | MeLeu | Thr | nBuGly | MeLeu | Asp | pip | 1.23 | 1.2E-06 |
| H06 | MeGly | MeVal | **Nva(2-R-4-F2-Pyrro)** | MeLeu | Thr | nBuGly | MePhe | MeLeu | Val | MeLeu | Asp | pip | 1.27 | 2.7E-07 |
| H07 | MeGly | MeLeu | Val | MeLeu | Thr | nBuGly | MePhe | MeLeu | **Nva(2-R-4-F2-Pyrro)** | MeLeu | Asp | pip | 1.32 | 1.6E-07 |
| H08 | D-Ala | MeAbu | Val | MeLeu | **Nva(2-R-4-F2-Pyrro)** | nBuGly | MeLeu | Ser(tBu) | MePhe | MeAla | Asp | pip | 1.24 | 9.2E-07 |
| H09 | gMeAbu | MeLeu | Val | MePhe | MeAla | Ser(tBu) | MeLeu | **Nva(2-R-4-F2-Pyrro)** | MeLeu | MeLeu | Asp | pip | 1.27 | 5.2E-07 |
| H10 | MeAla | Leu | MeAla | MePhe | Leu | MeLeu | MeLeu | **Nva(2-R-4-F2-Pyrro)** | MeGly | MeLeu | Asp | pip | 1.32 | 4.3E-07 |
| H11 | MeGly | MeAla | Val | MeAbu | **Nva(2-R-4-F2-Pyrro)** | nBuGly | MePhe | MeLeu | Leu | MeLeu | Asp | pip | 1.30 | 2.0E-06 |

Structures of cyclic peptides G01 to H10 are shown below. (In the structural formulae of the cyclic peptides, hydrogen atoms attached to a heteroatom may be omitted to clarify the structure.)

[Table 11]

| ID | Structural Formula | ID | Structural Formula |
|---|---|---|---|
| G01 | 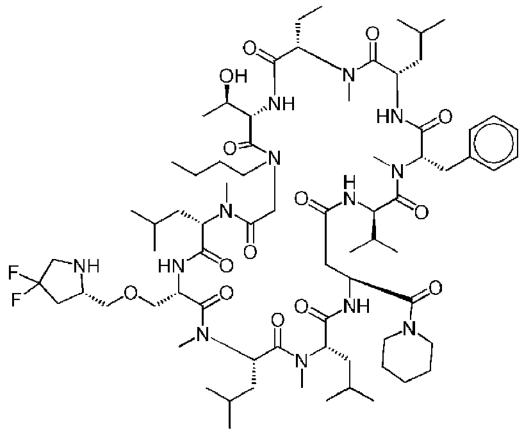 | G02 | 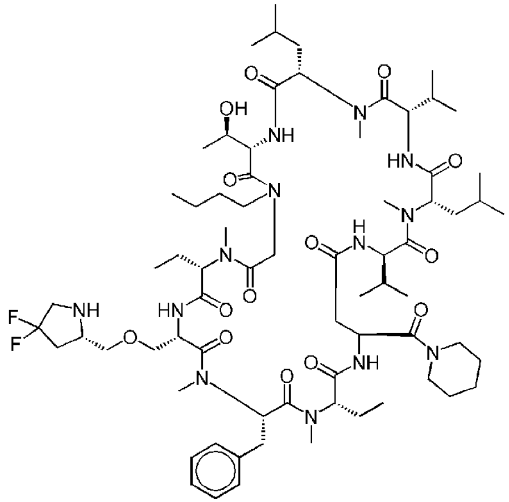 |
| G03 | 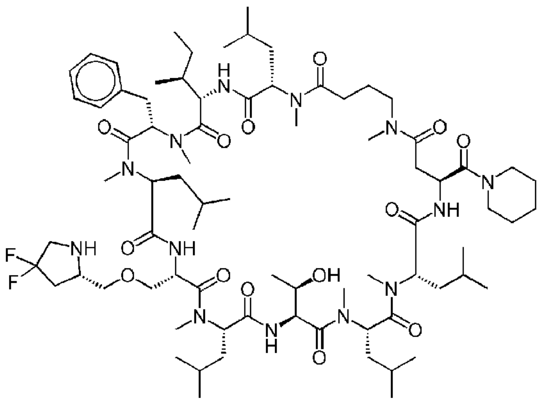 | G04 | 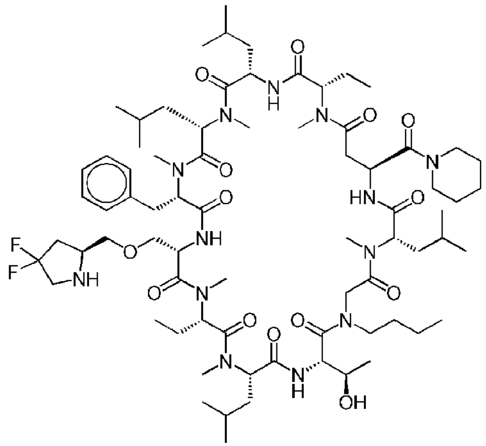 |

(continued)

| ID | Structural Formula | ID | Structural Formula |
|---|---|---|---|
| G05 | 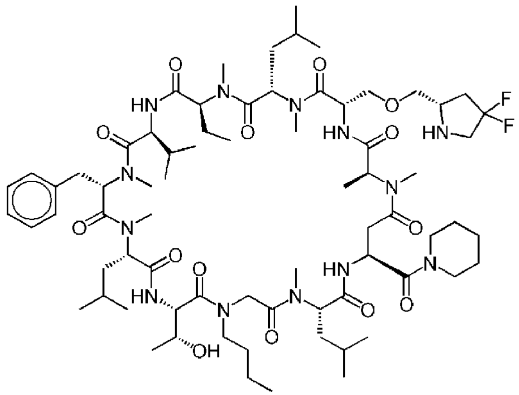 | G06 | 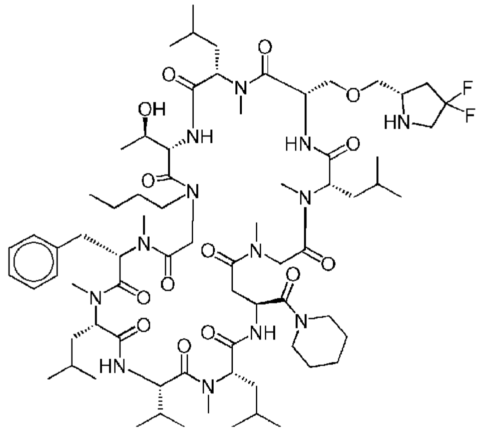 |
| G07 | 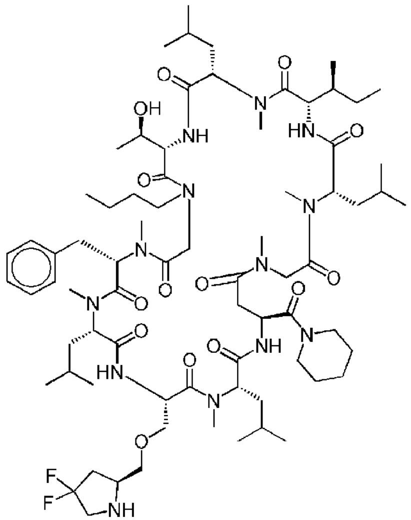 | G08 | 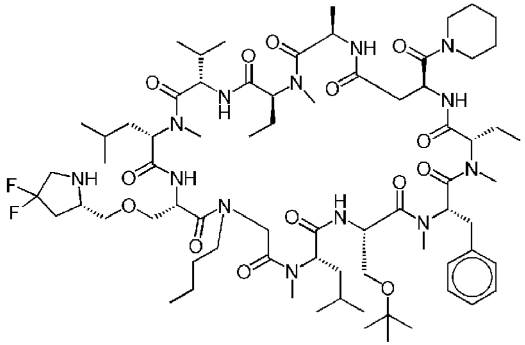 |

(continued)

| ID | Structural Formula | ID | Structural Formula |
|---|---|---|---|
| G09 | 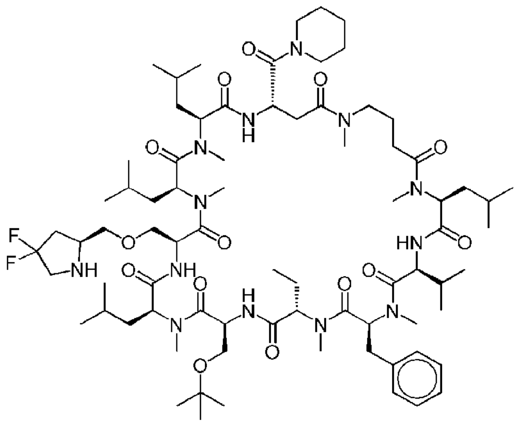 | G10 | 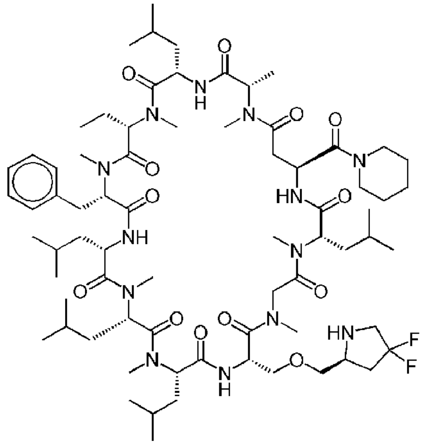 |
| G11 | 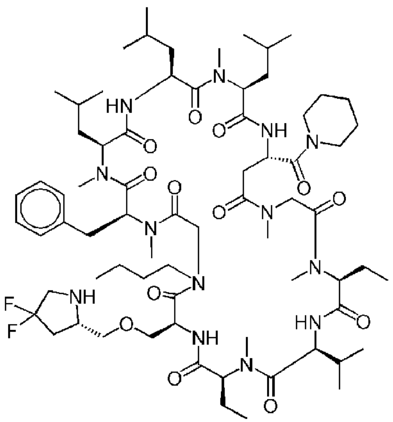 | H01 | 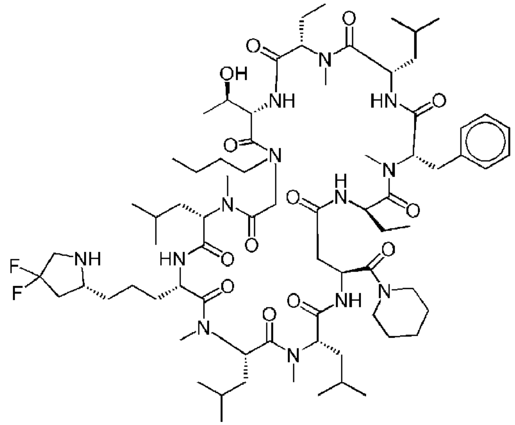 |

(continued)

| ID | Structural Formula | ID | Structural Formula |
|---|---|---|---|
| H02 | 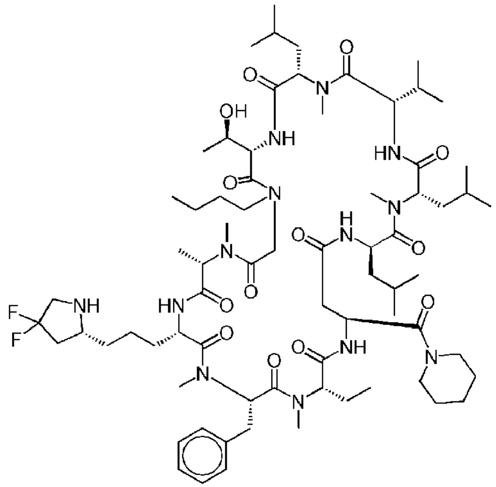 | H03 | 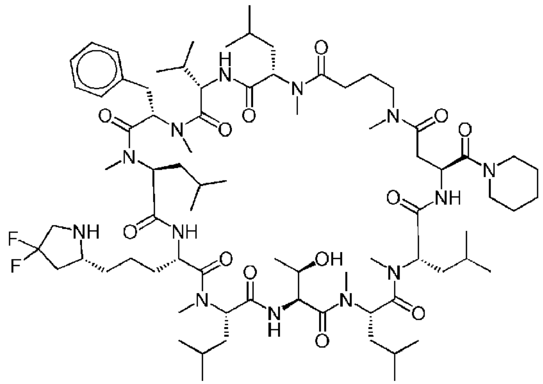 |
| H04 | 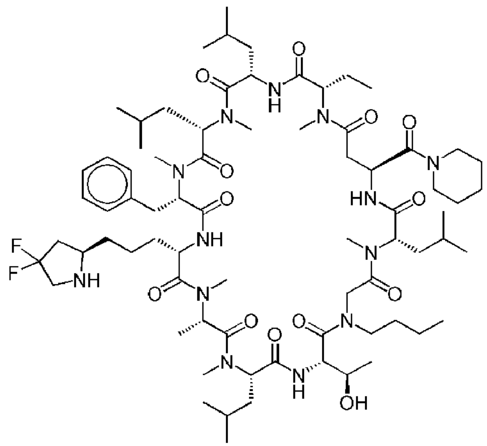 | H05 | 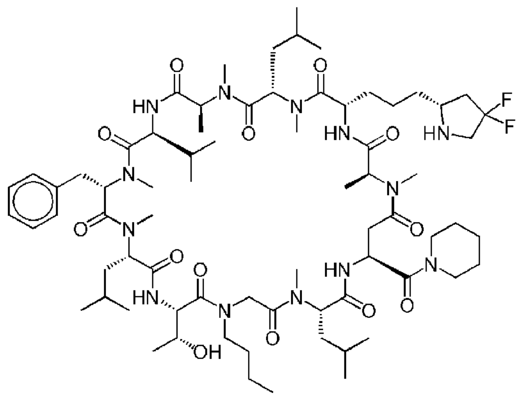 |

(continued)

| ID | Structural Formula | ID | Structural Formula |
|---|---|---|---|
| H06 | 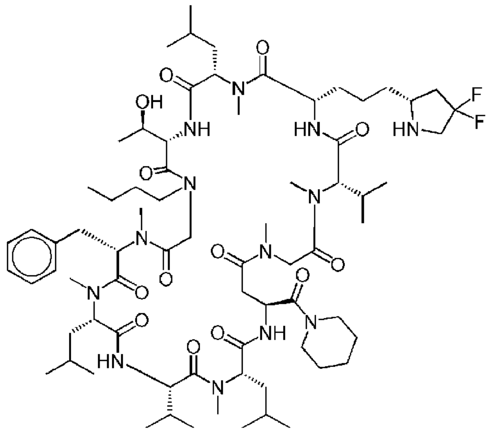 | H07 | 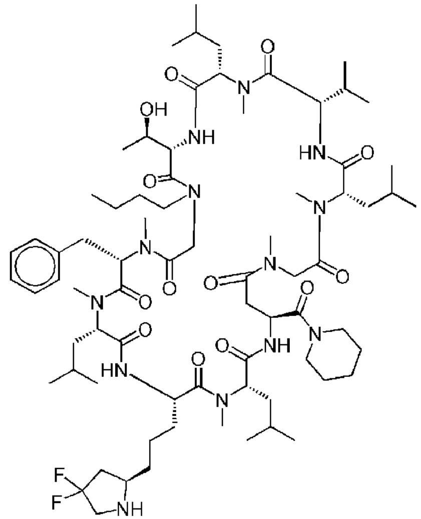 |
| H08 | 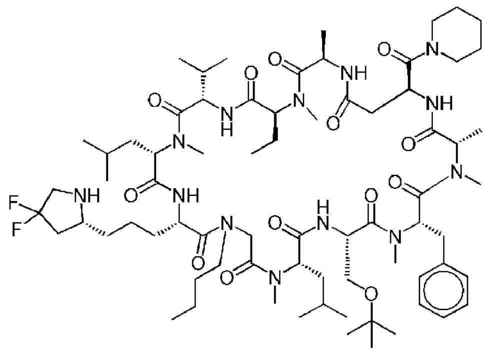 | H09 | 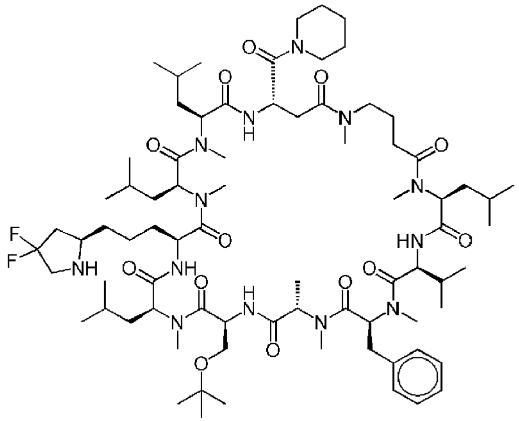 |

(continued)

| ID | Structural Formula | ID | Structural Formula |
|---|---|---|---|
| H10 | 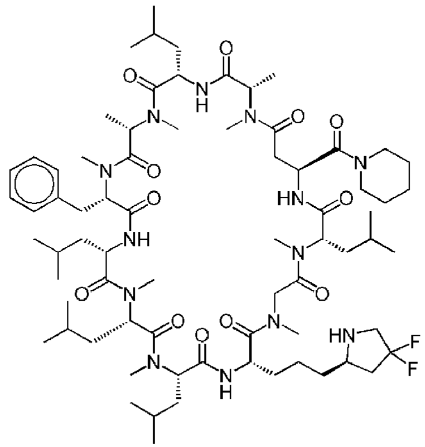 | H11 | 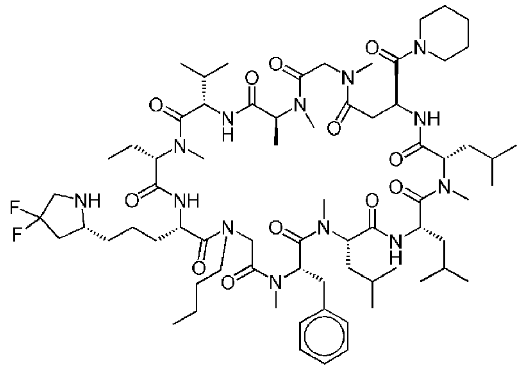 |

[Table 12]

## Comparison of Ser(NMe-Aca) and Gln(Me)

**Ser(NMe-Aca)**

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| I01 | D-Leu | MePhe | **Ser(NMe-Aca)** | MeLeu | Thr | nPrGly | MeLeu | Leu | MePhe | MeLeu | Asp | pip | 1.24 | 2.7E-07 |
| I02 | D-Leu | MeLeu | Phe | MeLeu | Thr | nBuGly | MeLeu | **Ser(NMe-Aca)** | MeHph | MeLeu | Asp | pip | 1.34 | 5.2E-08 |
| I03 | MeLeu | **Ser(NMe-Aca)** | MeLeu | MePhe | Leu | MeHph | MeLeu | Thr | MeGly | MeLeu | Asp | pip | 1.24 | 2.5E-07 |
| I04 | MeLeu | Phe | MeLeu | MeLeu | **Ser(NMe-Aca)** | MeHph | MeLeu | Thr | nPrGly | MeLeu | Asp | pip | 1.34 | 5.5E-07 |
| I05 | nBuGly | MePhe | **Ser(NMe-Aca)** | MeLeu | Thr | nPrGly | MePhe | MeLeu | Leu | MeLeu | Asp | pip | 1.27 | 8.0E-07 |
| I06 | nBuGly | MePhe | Ile | MeLeu | Thr | nPrGly | MePhe | MeLeu | **Ser(NMe-Aca)** | MeLeu | Asp | pip | 1.27 | 2.4E-07 |

**Gln(Me)**

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| J01 | D-Leu | MePhe | **Gln(Me)** | MeLeu | Thr | nBuGly | MeLeu | Leu | MePhe | MeIle | Asp | pip | 1.24 | 6.2E-08 |
| J02 | D-Leu | MeLeu | Phe(4-CF3) | MeLeu | Thr | nBuGly | MeLeu | **Gln(Me)** | MePhe | MeIle | Asp | pip | 1.32 | 4.0E-09 |
| J03 | MeLeu | **Gln(Me)** | MeLeu | MePhe | Leu | MePhe | MeIle | Thr | nPrGly | MeLeu | Asp | pip | 1.24 | 2.1E-07 |
| J04 | MeLeu | Phe(4-CF3) | MeLeu | MeLeu | **Gln(Me)** | MePhe | MeIle | Thr | nPrGly | MeLeu | Asp | pip | 1.32 | 8.7E-08 |
| J05 | nBuGly | MePhe | **Gln(Me)** | MeLeu | Thr | nBuGly | MePhe | MeLeu | Leu | MeIle | Asp | pip | 1.26 | 5.2E-08 |
| J06 | nBuGly | MePhe | Ile | MeLeu | Thr | nBuGly | MePhe | MeLeu | **Gln(Me)** | MeIle | Asp | pip | 1.26 | 8.5E-09 |

Structures of cyclic peptides 101 to J06 are shown below. (In the structural formulae of the cyclic peptides, hydrogen atoms attached to a heteroatom may be omitted to clarify the structure.)

[Table 13]

| ID | Structural Formula | ID | Structural Formula |
|---|---|---|---|
| I01 | 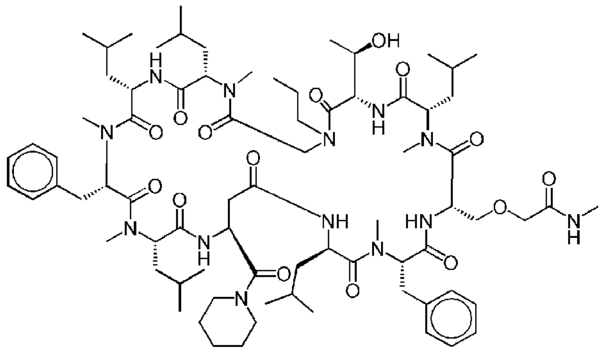 | I02 | 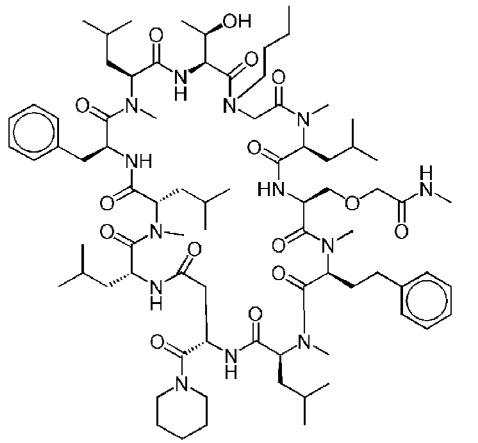 |
| I03 | 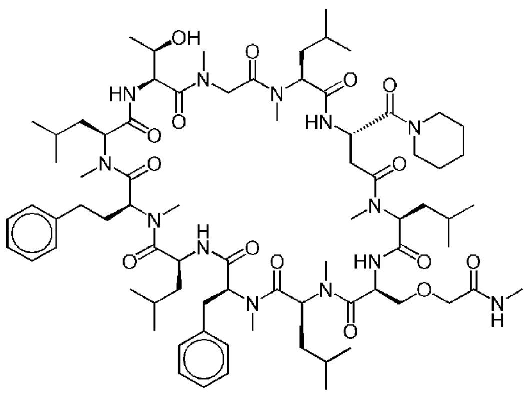 | I04 | 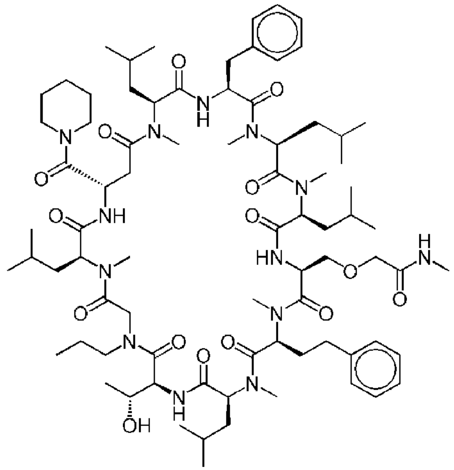 |

(continued)

| ID | Structural Formula | ID | Structural Formula |
|---|---|---|---|
| I05 | 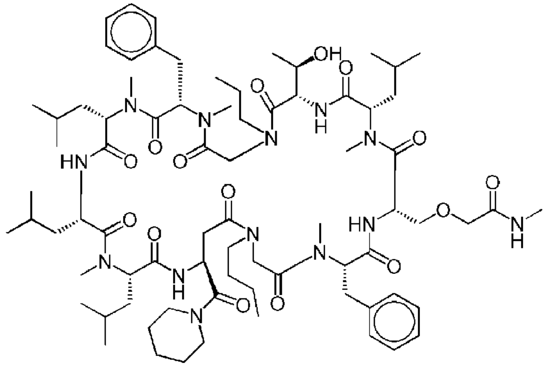 | I06 | 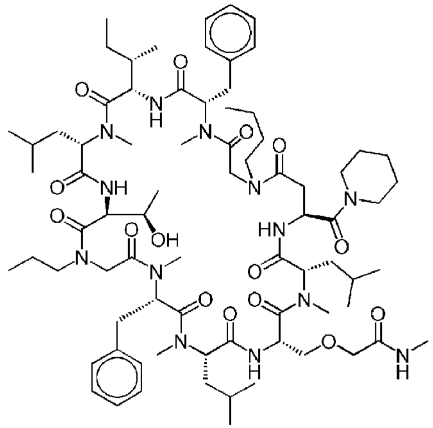 |
| J01 | 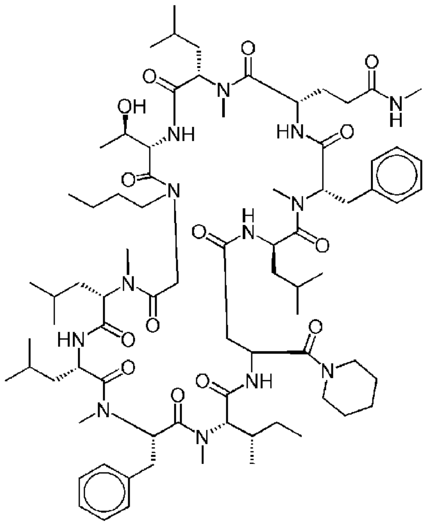 | J02 | 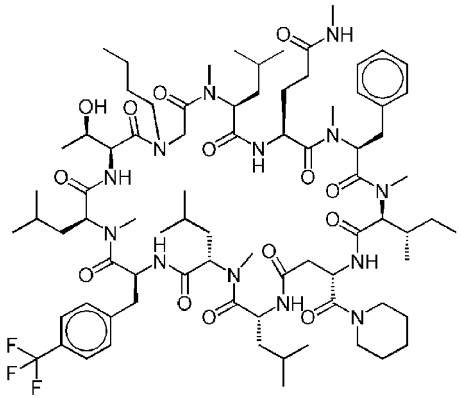 |

(continued)

| ID | Structural Formula | ID | Structural Formula |
|---|---|---|---|
| J03 | 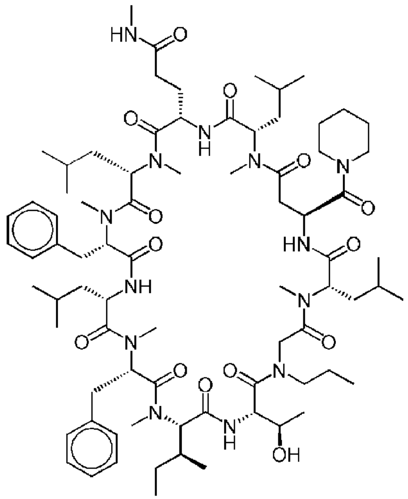 | J04 | 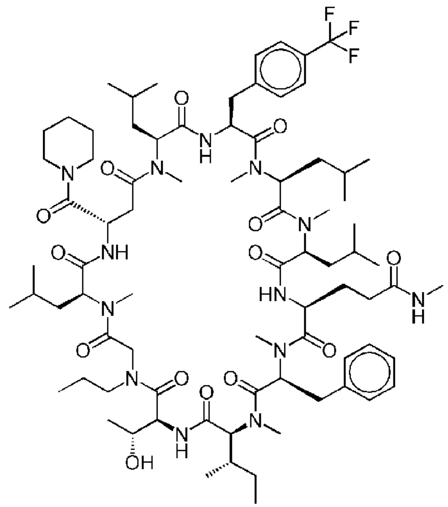 |

(continued)

| ID | Structural Formula | ID | Structural Formula |
|---|---|---|---|
| J05 | 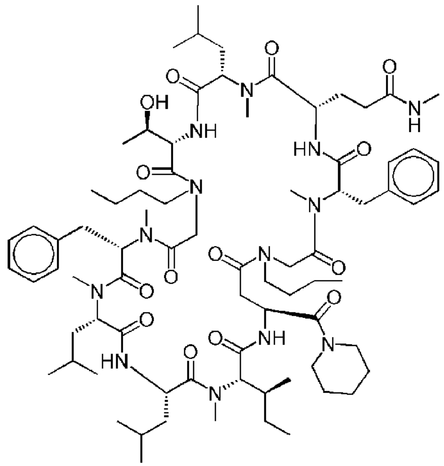 | J06 | 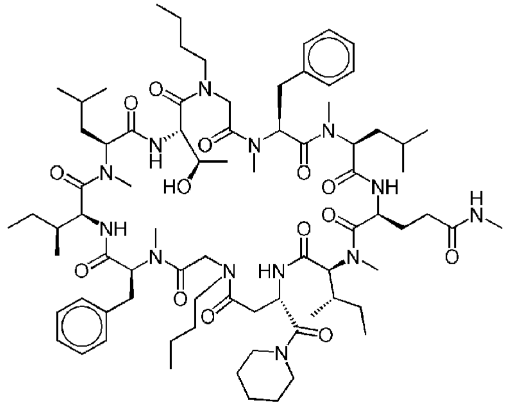 |

When the partial structures of side chains of Ser(EtOH), Ser(1-CF3-EtOH), Tyr(3-OMe), Ser(S-4-F2-Pyrro-Me), and Ser(NMe-Aca) where calculated by the low energy conformation calculation method described in Example 3, their most stable conformations were conformations having an intramolecular hydrogen bond. Accordingly, it was confirmed also from the result of calculation that the improved membrane permeability is due to the masking of a donor present in the side chain with an intramolecular hydrogen bond.

2-2 Result of membrane permeability of peptides containing amino acid having a donor in side chain and having an intramolecular hydrogen bond in the donor moiety

The membrane permeability of Ser(tBuOH), Ser(NtBu-Aca), bAla(3R-MeOEtOH), Phe(3-OMe-4-CONMe), Phe(4-OMe-3-CONMe), Ser(3-Me-5-Oxo-Odz), Ser(2-Me-2-BuOH), Ser(S-2-PrOH), Ser(R-2-PrOH), Ser(nPrOH), Ser(S-2-BuOH), Ser(R-2-BuOH), Ser(2-Me2-PrOH), bAla(2S-MeOEtOH), Phe(3-OMe-4-CONHMs), Hph(3-OMe-4-CONMe), D-Hph(3-OMe-4-CONMe), (Ph(3-OMe-4-CONMe)Et)Gly, MeHph(3-OMe-4-CONMe), Hyp(2-EtOH), Ser(S-Mor-3-Me), Phe(4-CONMeOH), and Phe(3-OMe-4-CONHOH) was evaluated. The results were as provided below. When the membrane permeability of Ser(nPrOH) and Ser(2-Me2-PrOH), both of which form a pseudo 6-membered ring, was compared, Ser(2-Me2-PrOH) having geminal methyl groups tended to have better membrane permeability than Ser(nPrOH) having no geminal methyl groups. Possible reasons therefor include a reduced conformational freedom due to the Thorpe-Ingold effect, a reduced polar surface area (PSA) due to the presence of a sterically large lipophilic substituent such as a dimethyl group in the vicinity of a donor moiety, and the like.

The results of evaluating the membrane permeability of cyclic peptides AP01 to AP143 and AP200 to AP266 are shown below.

[Table 14]

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| AP01 | D-Val | MePhe | Ser(tBuOH) | MeLeu | Thr | nPrGly | MeLeu | Ser(iPen) | MePhe | MeVal | Asp | pip | 1.26 | 1.4E-06 |
| AP02 | D-Val | MePhe | Leu | MeLeu | Thr | MeGly | MeLeu | Ser(tBuOH) | MePhe(3-Cl) | MeLeu | Asp | pip | 1.25 | 1.3E-06 |
| AP03 | gMeAbu | MeLeu | Ser(tBuOH) | MePhe | MePhe | Ser(iPen) | MeLeu | Thr | MeLeu | MeLeu | Asp | pip | 1.22 | 4.6E-07 |
| AP04 | gMeAbu | MeLeu | Ile | MePhe | MePhe(3-Cl) | Ser(tBuOH) | MeLeu | Thr | MeLeu | MeLeu | Asp | pip | 1.26 | 5.3E-07 |
| AP05 | MeAla | Ser(tBuOH) | MeLeu | MePhe | Leu | MePhe | MeIle | Thr | nPrGly | MeLeu | Asp | pip | 1.25 | 2.8E-06 |
| AP06 | MeAla | Leu | MeLeu | MePhe | Ser(tBuOH) | MePhe | MeIle | Thr | nPrGly | MeLeu | Asp | pip | 1.25 | 3.3E-06 |
| AP07 | MeGly | MePhe | Ser(tBuOH) | MeLeu | Thr | nPrGly | MePhe(3-Cl) | MeLeu | Ser(tBu) | MeLeu | Asp | pip | 1.22 | 1.3E-06 |
| AP08 | MeGly | MePhe | Ile | MeLeu | Thr | nPrGly | MePhe | MeLeu | Ser(tBuOH) | MeLeu | Asp | pip | 1.21 | 6.7E-07 |
| AP09 | nPrGly | MePhe | Ile | MeLeu | Thr | nPrGly | MePhe | MeLeu | Ser(tBuOH) | MeVal | Asp | pip | 1.26 | 1.1E-06 |
| AP10 | D-Val | MePhe | Leu | MeLeu | Thr | nPrGly | MeLeu | Ser(tBuOH) | MePhe | MeLeu | Asp | pip | 1.29 | 1.5E-06 |
| AP11 | D-Leu | MePhe | Ser(NtBu-Aca) | MeLeu | Thr | nPrGly | MeLeu | Leu | MePhe | MeAla | Asp | pip | 1.22 | 1.1E-06 |
| AP12 | D-Leu | MeLeu | Phe | MeLeu | Thr | nBuGly | MeLeu | Ser(NtBu-Aca) | MeHph | MeAla | Asp | pip | 1.32 | 1.7E-07 |
| AP13 | D-Leu | MeLeu | Phe | MeHph | Thr | nPrGly | MeLeu | Ser(NtBu-Aca) | MeLeu | MeAla | Asp | pip | 1.27 | 3.2E-07 |
| AP14 | MeLeu | Ser(NtBu-Aca) | MeLeu | MePhe | Leu | MeHph | MeLeu | Thr | MeGly | MeAla | Asp | pip | 1.22 | 3.9E-07 |
| AP15 | MeLeu | Phe | MeLeu | MeLeu | Ser(NtBu-Aca) | MeHph | MeLeu | Thr | nPrGly | MeAla | Asp | pip | 1.32 | 8.6E-07 |
| AP16 | MeLeu | Ser(NtBu Aca) | MeHph | MeLeu | Leu | MeLeu | MeHph | Thr | MeGly | MeAla | Asp | pip | 1.27 | 2.7E 07 |
| AP17 | nBuGly | MePhe | Ser(NtBu-Aca) | MeLeu | Thr | nPrGly | MePhe | MeLeu | Leu | MeAla | Asp | pip | 1.25 | 7.2E-07 |
| AP18 | nBuGly | MePhe | Ile | MeLeu | Thr | nPrGly | MePhe | MeLeu | Ser(NtBu-Aca) | MeAla | Asp | pip | 1.25 | 1.0E-06 |
| AP19 | nBuGly | MeLeu | Ile | MePhe | Thr | nBuGly | MeLeu | MePhe | Ser(NtBu-Aca) | MeAla | Asp | pip | 1.29 | 8.8E-07 |
| AP20 | D-Leu | MePhe | bAla(3R-MeOEtOH) | MeLeu | Thr | nPrGly | MeLeu | Leu | MePhe | MeLeu | Asp | pip | 1.22 | 3.7E-07 |
| AP21 | D-Leu | MeLeu | Phe | MeLeu | Thr | nBuGly | MeLeu | bAla(3R-MeOEtOH) | MePhe | MeLeu | Asp | pip | 1.27 | 2.1E-07 |
| AP22 | gMeAbu | MeLeu | Ile | MePhe(3-Cl) | MeLeu | bAla(3R-MeOEtOH) | MeLeu | Thr | MeLeu | MeLeu | Asp | pip | 1.16 | 2.3E-07 |
| AP23 | MeLeu | bAla(3R-MeOEtOH) | MeLeu | MePhe | Leu | MePhe | MeLeu | Thr | nBuGly | MeLeu | Asp | pip | 1.32 | 4.3E-07 |
| AP24 | MeLeu | Phe | MeLeu | MeLeu | bAla(3R-MeOEtOH) | MePhe | MeLeu | Thr | nPrGly | MeLeu | Asp | pip | 1.28 | 4.2E-07 |
| AP25 | nBuGly | MePhe | bAla(3R-MeOEtOH) | MeLeu | Thr | nPrGly | MePhe | MeLeu | Leu | MeLeu | Asp | pip | 1.25 | 6.2E-07 |
| AP26 | nBuGly | MePhe | Ile | MeLeu | Thr | nPrGly | MePhe | MeLeu | bAla(3R-MeOEtOH) | MeLeu | Asp | pip | 1.25 | 3.7E-07 |
| AP27 | D-Val | MeVal | Phe(3-OMe-4-CONMe) | MeLeu | Thr | nBuGly | MeLeu | Ser(tBu) | MePhe | MeLeu | Asp | pip | 1.28 | 9.1E-07 |
| AP28 | D-Leu | MePhe | Val | MeLeu | Thr | nBuGly | MeLeu | Phe(3-OMe-4-CONMe) | MeLeu | MeLeu | Asp | pip | 1.38 | 3.8E-07 |
| AP29 | D-Ala | MePhe | Phe(3-OMe-4-CONMe) | MeLeu | Thr | nBuGly | MeLeu | Ser(tBu) | MeIle | MeLeu | Asp | pip | 1.24 | 3.6E-07 |
| AP30 | gMeAbu | MeLeu | Ile | MePhe | MeLeu | Phe(3-OMe-4-CONMe) | MeLeu | Thr | MeLeu | MeLeu | Asp | pip | 1.24 | 1.9E-07 |
| AP31 | MeLeu | Phe(3-OMe-4-CONMe) | MeLeu | MePhe | Leu | MeLeu | MeIle | Thr | MeGly | MeAbu | Asp | pip | 1.24 | 5.2E-07 |
| AP32 | MeLeu | Leu | MeLeu | MeLeu | Phe(3-OMe-4-CONMe) | MeLeu | MeVal | Thr | MeGly | MeLeu | Asp | pip | 1.28 | 1.3E-06 |
| AP33 | MeGly | MePhe(3-Cl) | Phe(3-OMe-4-CONMe) | MeLeu | Thr | nBuGly | MeLeu | MeLeu | Ser(tBu) | MeLeu | Asp | pip | 1.31 | 6.1E-07 |
| AP34 | MeGly | MeLeu | Ile | MeLeu | Thr | MeGly | MePhe(3-Cl) | MeLeu | Phe(3-OMe-4-CONMe) | MeLeu | Asp | pip | 1.21 | 2.0E-07 |
| AP35 | D-Val | MeVal | Phe(4-OMe-3-CONMe) | MeLeu | Thr | nBuGly | MeLeu | Ser(tBu) | MePhe | MeLeu | Asp | pip | 1.28 | 2.0E-06 |
| AP36 | D-Leu | MePhe | Val | MeLeu | Thr | nBuGly | MeLeu | Phe(4-OMe-3-CONMe) | MeLeu | MeLeu | Asp | pip | 1.38 | 2.6E-07 |
| AP37 | D-Ala | MePhe | Phe(4-OMe-3-CONMe) | MeLeu | Thr | nBuGly | MeLeu | Ser(tBu) | MeIle | MeLeu | Asp | pip | 1.24 | 4.0E-07 |
| AP38 | gMeAbu | MeLeu | Ile | MePhe | MeLeu | Phe(4-OMe-3-CONMe) | MeLeu | Thr | MeLeu | MeLeu | Asp | pip | 1.24 | 2.0E-07 |
| AP39 | MeLeu | Phe(4-OMe-3-CONMe) | MeLeu | MePhe | Leu | MeLeu | MeIle | Thr | MeGly | MeAbu | Asp | pip | 1.24 | 1.0E-06 |
| AP40 | MeLeu | Leu | MeLeu | MeLeu | Phe(4-OMe-3-CONMe) | MeLeu | MeVal | Thr | MeGly | MeLeu | Asp | pip | 1.28 | 6.3E-07 |
| AP41 | MeGly | MePhe(3-Cl) | Phe(4-OMe-3-CONMe) | MeLeu | Thr | nBuGly | MeLeu | MeLeu | Ser(tBu) | MeLeu | Asp | pip | 1.31 | 4.5E-07 |
| AP42 | MeGly | MeLeu | Ile | MeLeu | Thr | MeGly | MePhe(3-Cl) | MeLeu | Phe(4-OMe-3-CONMe) | MeLeu | Asp | pip | 1.21 | 2.5E-07 |
| AP43 | D-Val | MePhe | Leu | MeLeu | Thr | nBuGly | MeLeu | Ser(3-Me-5-Oxo-Odz) | MeLeu | MeLeu | Asp | pip | 1.24 | 8.2E-07 |
| AP44 | D-Leu | MeLeu | Ile | MeLeu | Thr | nBuGly | MeLeu | Ser(3-Me-5-Oxo-Odz) | MePhe | MeLeu | Asp | pip | 1.28 | 3.0E-07 |
| AP45 | D-Leu | MePhe | Ser(3-Me-5-Oxo-Odz) | MeLeu | Thr | nBuGly | MeLeu | Ser(tBu) | MeLeu | MeLeu | Asp | pip | 1.23 | 1.4E-06 |
| AP46 | gMeAbu | MeLeu | Ile | MePhe(3-Cl) | MeLeu | Ser(3-Me-5-Oxo-Odz) | MeLeu | Thr | MeLeu | MeLeu | Asp | pip | 1.16 | 2.2E-07 |
| AP47 | MeAbu | Leu | MeLeu | MePhe | Ser(3-Me-5-Oxo-Odz) | MeLeu | MeLeu | Thr | nBuGly | MeLeu | Asp | pip | 1.25 | 5.3E-06 |
| AP48 | MeLeu | Ser(3-Me-5-Oxo-Odz) | MeLeu | MeLeu | Val | MePhe | MeLeu | Thr | nBuGly | MeLeu | Asp | pip | 1.29 | 4.0E-07 |
| AP49 | MeGly | MeLeu | Ser(3-Me-5-Oxo-Odz) | MeLeu | Thr | nBuGly | MePhe(3-Cl) | MeLeu | Ile | MeLeu | Asp | pip | 1.22 | 3.3E-07 |
| AP50 | MeGly | MeLeu | Ile | MeLeu | Thr | nBuGly | MePhe(3-Cl) | MeLeu | Ser(3-Me-5-Oxo-Odz) | MeLeu | Asp | pip | 1.22 | 1.1E-07 |

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| AP51 | D-Val | MePhe | Ser(2-Me-2-BuOH) | MeLeu | Thr | MeGly | MeLeu | Ser(iPen) | MePhe | MeLeu | Asp | pip | 1.23 | 1.4E-06 |
| AP52 | D-Val | MePhe | Leu | MeVal | Thr | MeGly | MeLeu | Ser(2-Me-2-BuOH) | MePhe(3-Cl) | MeLeu | Asp | pip | 1.23 | 1.7E-06 |
| AP53 | gMeAbu | MeLeu | Ser(2-Me-2-BuOH) | MePhe | MePhe | Ser(iPen) | MeVal | Thr | MeLeu | MeLeu | Asp | pip | 1.20 | 7.4E-07 |
| AP54 | gMeAbu | MeVal | Ile | MePhe | MePhe(3-Cl) | Ser(2-Me-2-BuOH) | MeLeu | Thr | MeLeu | MeLeu | Asp | pip | 1.24 | 1.4E-06 |
| AP55 | MeAla | Ser(2-Me-2-BuOH) | MeLeu | MePhe | Leu | MeHph | MeLeu | Thr | MeGly | MeLeu | Asp | pip | 1.23 | 1.3E-06 |
| AP56 | MeAla | Leu | MeLeu | MePhe | Ser(2-Me-2-BuOH) | MeHph | MeLeu | Thr | MeGly | MeLeu | Asp | pip | 1.23 | 1.3E-06 |
| AP57 | MeGly | MeHph | Ser(2-Me-2-BuOH) | MeLeu | Thr | MeGly | MePhe(3-Cl) | MeLeu | Ser(tBu) | MeLeu | Asp | pip | 1.19 | 8.3E-07 |
| AP58 | MeGly | MePhe | Ile | MeLeu | Thr | MeGly | MeHph | MeLeu | Ser(2-Me-2-BuOH) | MeLeu | Asp | pip | 1.18 | 4.9E-07 |
| AP59 | nPrGly | MePhe | Ile | MeLeu | Thr | MeGly | MePhe | MeLeu | Ser(2-Me-2-BuOH) | MeLeu | Asp | pip | 1.24 | 7.8E-07 |
| AP60 | D-Val | MeHph | Leu | MeLeu | Thr | MeGly | MeLeu | Ser(2-Me-2-BuOH) | MePhe | MeLeu | Asp | pip | 1.26 | 1.0E-06 |
| AP61 | D-Val | MePhe | Ser(S-2-PrOH) | MeLeu | Thr | nPrGly | MeLeu | Ser(iPen) | MePhe | MeLeu | Asp | pip | 1.27 | 5.1E-07 |
| AP62 | D-Val | MePhe | Leu | MeLeu | Thr | MeGly | MeLeu | Ser(S-2-PrOH) | MePhe(3-Cl) | MeLeu | Asp | pip | 1.21 | 5.5E-07 |
| AP63 | g-MeAbu | MeLeu | Ser(S-2-PrOH) | MePhe | MePhe(3-Cl) | Ser(iPen) | MeLeu | Thr | MeLeu | MeLeu | Asp | pip | 1.24 | 1.4E-07 |
| AP64 | g-MeAbu | MeLeu | Ile | MePhe | MePhe(3-Cl) | Ser(S-2-PrOH) | MeLeu | Thr | MeLeu | MeLeu | Asp | pip | 1.23 | 3.5E-07 |
| AP65 | MeAla | Ser(S-2-PrOH) | MeLeu | MePhe(3-Cl) | Leu | MePhe | MeIle | Thr | nPrGly | MeLeu | Asp | pip | 1.28 | 1.2E-06 |
| AP66 | MeVal | Leu | MeLeu | MePhe | Ser(S-2-PrOH) | MePhe | MeIle | Thr | MeGly | MeLeu | Asp | pip | 1.20 | 6.8E-07 |
| AP67 | MeGly | MePhe(3-Cl) | Ser(S-2-PrOH) | MeLeu | Thr | nPrGly | MePhe | MeLeu | Ser(iPen) | MeLeu | Asp | pip | 1.25 | 7.1E-07 |
| AP68 | MeGly | MePhe | Ile | MeLeu | Thr | nPrGly | MePhe(3-Cl) | MeLeu | Ser(S-2-PrOH) | MeLeu | Asp | pip | 1.23 | 2.4E-07 |
| AP69 | MeVal | Ser(S-2-PrOH) | MeLeu | MePhe(3-Cl) | Leu | MePhe | MeIle | Thr | MeGly | MeLeu | Asp | pip | 1.26 | 7.0E-07 |
| AP70 | D-Val | MePhe | Leu | MeLeu | Thr | nPrGly | MeLeu | Ser(S-2-PrOH) | MePhe(3-Cl) | MeVal | Asp | pip | 1.27 | 7.5E-07 |
| AP71 | D-Val | MePhe | Ser(R-2-PrOH) | MeLeu | Thr | nBuGly | MeLeu | Ser(tBu) | MePhe | MeLeu | Asp | pip | 1.25 | 7.1E-07 |
| AP72 | D-Val | MePhe | Leu | MeLeu | Thr | MeGly | MeLeu | Ser(R-2-PrOH) | MePhe(3-Cl) | MeLeu | Asp | pip | 1.21 | 9.5E-07 |
| AP73 | gMeAbu | MeLeu | Ser(R-2-PrOH) | MePhe | MePhe(3-Cl) | Ser(tBu) | MeLeu | Thr | MeLeu | MeLeu | Asp | pip | 1.18 | 4.4E-07 |
| AP74 | gMeAbu | MeLeu | Ile | MePhe | MePhe(3-Cl) | Ser(R-2-PrOH) | MeLeu | Thr | MeLeu | MeLeu | Asp | pip | 1.23 | 3.1E-07 |
| AP75 | MeAla | Ser(R-2-PrOH) | MeLeu | MePhe(3-Cl) | Leu | MePhe | MeIle | Thr | nBuGly | MeLeu | Asp | pip | 1.33 | 1.1E-06 |
| AP76 | MeVal | Leu | MeLeu | MePhe | Ser(R-2-PrOH) | MePhe | MeIle | Thr | MeGly | MeLeu | Asp | pip | 1.20 | 8.4E-07 |
| AP77 | MeGly | MePhe(3-Cl) | Ser(R-2-PrOH) | MeLeu | Thr | nBuGly | MePhe | MeLeu | Ser(tBu) | MeLeu | Asp | pip | 1.23 | 1.0E-06 |
| AP78 | MeGly | MePhe | Ile | MeLeu | Thr | nBuGly | MePhe(3-Cl) | MeLeu | Ser(R-2-PrOH) | MeLeu | Asp | pip | 1.28 | 2.4E-07 |
| AP79 | MeVal | Ser(R-2-PrOH) | MeLeu | MePhe(3-Cl) | Leu | MePhe | MeIle | Thr | MeGly | MeLeu | Asp | pip | 1.26 | 1.1E-06 |
| AP80 | D-Val | MePhe | Leu | MeLeu | Thr | nBuGly | MeLeu | Ser(R-2-PrOH) | MePhe(3-Cl) | MeVal | Asp | pip | 1.31 | 3.6E-07 |
| AP81 | D-Leu | MePhe | Leu | MeLeu | Ser(R-2-PrOH) | MeGly | MeLeu | Ser(tBu) | MePhe | MeLeu | Asp | pip | 1.33 | 6.9E-07 |
| AP82 | D-Val | MePhe | Ser(R-2-PrOH) | MeLeu | Val | MeGly | MeLeu | Ser(tBu) | MePhe | MeLeu | Asp | pip | 1.24 | 2.5E-06 |
| AP83 | gMeAbu | MeLeu | Val | MePhe | MePhe | Ser(tBu) | MeLeu | Ser(R-2-PrOH) | MeLeu | MeLeu | Asp | pip | 1.25 | 1.3E-06 |
| AP84 | gMeAbu | MeLeu | Ser(R-2-PrOH) | MePhe | MePhe | Ser(tBu) | MeLeu | Ile | MeLeu | MeLeu | Asp | pip | 1.30 | 5.8E-07 |
| AP85 | MeAla | Leu | MeLeu | MePhe | Leu | MePhe | MeIle | Ser(R-2-PrOH) | MeGly | MeLeu | Asp | pip | 1.30 | 5.2E-06 |
| AP86 | MeAla | Ser(R-2-PrOH) | MeLeu | MePhe | Leu | MePhe | MeIle | Val | MeGly | MeLeu | Asp | pip | 1.26 | 3.7E-06 |
| AP87 | MeGly | MePhe | Val | MeLeu | Ser(R-2-PrOH) | nBuGly | MePhe | MeLeu | Ser(tBu) | MeLeu | Asp | pip | 1.31 | 1.0E-06 |
| AP88 | MeGly | MePhe | Ser(R-2-PrOH) | MeLeu | Ile | MeGly | MePhe | MeLeu | Ser(tBu) | MeLeu | Asp | pip | 1.21 | 3.3E-06 |
| AP89 | D-Leu | MePhe | Ser(nPrOH) | MeLeu | Thr | nBuGly | MeLeu | Ser(tBu) | MePhe | MeLeu | Asp | pip | 1.30 | 1.2E-07 |
| AP90 | D-Val | MePhe | Leu | MeLeu | Thr | MeGly | MeLeu | Ser(nPrOH) | MePhe(3-Cl) | MeLeu | Asp | pip | 1.21 | 4.5E-07 |
| AP91 | gMeAbu | MeLeu | Ser(nPrOH) | MePhe | MePhe(3-Cl) | Leu | MeLeu | Thr | MeLeu | MeLeu | Asp | pip | 1.23 | 1.4E-07 |
| AP92 | gMeAbu | MeLeu | Ile | MePhe | MePhe(3-Cl) | Ser(nPrOH) | MeLeu | Thr | MeLeu | MeLeu | Asp | pip | 1.23 | 1.8E-07 |
| AP93 | MeVal | Ser(nPrOH) | MeLeu | MePhe | Val | MePhe | MeLeu | Thr | nBuGly | MeLeu | Asp | pip | 1.30 | 1.1E-06 |
| AP94 | MeLeu | Leu | MeLeu | MePhe | Ser(nPrOH) | MePhe | MeLeu | Thr | MeGly | MeLeu | Asp | pip | 1.25 | 3.5E-07 |
| AP95 | nBuGly | MePhe | Ser(nPrOH) | MeLeu | Thr | nBuGly | MePhe | MeVal | Ser(tBu) | MeLeu | Asp | pip | 1.27 | 6.9E-07 |
| AP96 | MeGly | MePhe | Ile | MeLeu | Thr | nBuGly | MePhe(3-Cl) | MeLeu | Ser(nPrOH) | MeLeu | Asp | pip | 1.28 | 1.7E-07 |
| AP97 | MeLeu | Ser(nPrOH) | MeLeu | MePhe | Leu | MePhe(3-Cl) | MeLeu | Thr | MeGly | MeLeu | Asp | pip | 1.31 | 2.1E-07 |
| AP98 | D-Val | MePhe | Leu | MeLeu | Thr | nBuGly | MeLeu | Ser(nPrOH) | MePhe(3-Cl) | MeLeu | Asp | pip | 1.36 | 1.6E-07 |
| AP99 | D-Val | MePhe | Ser(S-2-BuOH) | MeLeu | Thr | nBuGly | MeVal | Ser(tBu) | MePhe | MeLeu | Asp | pip | 1.23 | 8.6E-07 |
| AP100 | D-Val | MePhe | Leu | MeLeu | Thr | MeGly | MeLeu | Ser(S-2-BuOH) | MePhe(3-Cl) | MeLeu | Asp | pip | 1.24 | 5.5E-07 |

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| AP101 | gMeAbu | MeVal | Ser(S-2-BuOH) | MePhe | MePhe(3-Cl) | Ser(tBu) | MeLeu | Thr | MeLeu | MeLeu | Asp | pip | 1.16 | 5.9E-07 |
| AP102 | gMeAbu | MeLeu | Val | MePhe | MePhe(3-Cl) | Ser(S-2-BuOH) | MeLeu | Thr | MeLeu | MeLeu | Asp | pip | 1.21 | 4.3E-07 |
| AP103 | MeAla | Ser(S-2-BuOH) | MeLeu | MePhe(3-Cl) | Leu | MePhe | MeVal | Thr | nBuGly | MeLeu | Asp | pip | 1.31 | 8.9E-07 |
| AP104 | MeVal | Leu | MeLeu | MePhe | Ser(S-2-BuOH) | MePhe | MeVal | Thr | MeGly | MeLeu | Asp | pip | 1.18 | 2.9E-06 |
| AP105 | MeGly | MePhe(3-Cl) | Ser(S-2-BuOH) | MeLeu | Thr | nBuGly | MePhe | MeVal | Ser(tBu) | MeLeu | Asp | pip | 1.21 | 5.5E-07 |
| AP106 | MeGly | MePhe | Val | MeLeu | Thr | nBuGly | MePhe(3-Cl) | MeLeu | Ser(S-2-BuOH) | MeLeu | Asp | pip | 1.26 | 2.5E-07 |
| AP107 | MeVal | Leu | MeLeu | MePhe | Ser(S-2-BuOH) | MePhe | MeVal | Thr | nBuGly | MeLeu | Asp | pip | 1.33 | 9.3E-07 |
| AP108 | D-Val | MePhe | Ser(R-2-BuOH) | MeLeu | Thr | nBuGly | MeVal | Ser(tBu) | MePhe | MeLeu | Asp | pip | 1.23 | 8.1E-07 |
| AP109 | D-Val | MePhe | Leu | MeLeu | Thr | MeGly | MeLeu | Ser(R-2-BuOH) | MePhe(3-Cl) | MeLeu | Asp | pip | 1.24 | 6.4E-07 |
| AP110 | gMeAbu | MeVal | Ser(R-2-BuOH) | MePhe | MePhe(3-Cl) | Ser(tBu) | MeLeu | Thr | MeLeu | MeLeu | Asp | pip | 1.16 | 2.3E-07 |
| AP111 | gMeAbu | MeLeu | Val | MePhe | MePhe(3-Cl) | Ser(R-2-BuOH) | MeLeu | Thr | MeLeu | MeLeu | Asp | pip | 1.21 | 1.3E-07 |
| AP112 | MeAla | Ser(R-2-BuOH) | MeLeu | MePhe(3-Cl) | Leu | MePhe | MeVal | Thr | nBuGly | MeLeu | Asp | pip | 1.31 | 7.3E-07 |
| AP113 | MeVal | Leu | MeLeu | MePhe | Ser(R-2-BuOH) | MePhe | MeVal | Thr | MeGly | MeLeu | Asp | pip | 1.18 | 1.7E-06 |
| AP114 | MeGly | MePhe | Val | MeLeu | Thr | nBuGly | MePhe(3-Cl) | MeLeu | Ser(R-2-BuOH) | MeLeu | Asp | pip | 1.26 | 2.8E-07 |
| AP115 | gMeAbu | MeLeu | Ser(R-2-BuOH) | MePhe | MePhe(3-Cl) | Ser(tBu) | MeLeu | Thr | MeLeu | MeLeu | Asp | pip | 1.21 | 2.7E-07 |
| AP116 | MeVal | Leu | MeLeu | MePhe | Ser(R-2-BuOH) | MePhe | MeVal | Thr | nBuGly | MeLeu | Asp | pip | 1.33 | 8.9E-07 |
| AP117 | D-Abu | MePhe | Ser(2-Me2-PrOH) | MeLeu | Thr | nBuGly | MeLeu | Ser(tBu) | MePhe | MeLeu | Asp | pip | 1.28 | 3.6E-07 |
| AP118 | D-Val | MePhe | Leu | MeLeu | Thr | MeGly | MeLeu | Ser(2-Me2-PrOH) | MePhe | MeLeu | Asp | pip | 1.22 | 1.8E-06 |
| AP119 | gMeAbu | MeLeu | Ser(2-Me2-PrOH) | MePhe | MePhe | Leu | MeLeu | Thr | MeLeu | MeLeu | Asp | pip | 1.24 | 3.4E-07 |
| AP120 | gMeAbu | MeLeu | Ile | MePhe | MePhe | Ser(2-Me2-PrOH) | MeLeu | Thr | MeLeu | MeLeu | Asp | pip | 1.24 | 1.1E-06 |
| AP121 | MeAla | Ser(2-Me2-PrOH) | MeLeu | MePhe | Val | MePhe | MeLeu | Thr | nBuGly | MeLeu | Asp | pip | 1.29 | 3.1E-06 |
| AP122 | MeAbu | Leu | MeLeu | MePhe | Ser(2-Me2-PrOH) | MePhe | MeLeu | Thr | MeGly | MeLeu | Asp | pip | 1.23 | 2.9E-06 |
| AP123 | MeGly | MePhe(3-Cl) | Ser(2-Me2-PrOH) | MeLeu | Thr | nBuGly | MePhe | MeVal | Ser(tBu) | MeLeu | Asp | pip | 1.26 | 8.0E-07 |
| AP124 | MeGly | MePhe | Val | MeLeu | Thr | nBuGly | MePhe | MeLeu | Ser(2-Me2-PrOH) | MeLeu | Asp | pip | 1.24 | 1.6E-06 |
| AP125 | MeLeu | Ser(2-Me2-PrOH) | MeLeu | MePhe | Leu | MePhe | MeLeu | Thr | MeGly | MeLeu | Asp | pip | 1.32 | 7.4E-07 |
| AP126 | D-Val | MePhe | Leu | MeLeu | Thr | nBuGly | MeLeu | Ser(2-Me2-PrOH) | MePhe | MeLeu | Asp | pip | 1.37 | 1.4E-07 |
| AP127 | D-Leu | MePhe | bAla(2S-MeOEtOH) | MeLeu | Thr | nPrGly | MeLeu | Leu | MePhe | MeLeu | Asp | pip | 1.24 | 3.0E-07 |
| AP128 | D-Leu | MeLeu | Phe | MeLeu | Thr | nBuGly | MeLeu | bAla(2S-MeOEtOH) | MePhe | MeLeu | Asp | pip | 1.29 | 7.8E-07 |
| AP129 | gMeAbu | MeLeu | bAla(2S-MeOEtOH) | MePhe(3-Cl) | MeLeu | Leu | MeLeu | Thr | MeLeu | MeLeu | Asp | pip | 1.17 | 4.6E-07 |
| AP130 | gMeAbu | MeLeu | Ile | MePhe(3-Cl) | MeLeu | bAla(2S-MeOEtOH) | MeLeu | Thr | MeLeu | MeLeu | Asp | pip | 1.17 | 4.6E-07 |
| AP131 | MeLeu | bAla(2S-MeOEtOH) | MeLeu | MePhe | Leu | MePhe | MeLeu | Thr | nBuGly | MeLeu | Asp | pip | 1.34 | 1.1E-06 |
| AP132 | MeLeu | Phe | MeLeu | MeLeu | bAla(2S-MeOEtOH) | MePhe | MeLeu | Thr | nPrGly | MeLeu | Asp | pip | 1.29 | 1.3E-06 |
| AP133 | nBuGly | MePhe | bAla(2S-MeOEtOH) | MeLeu | Thr | nPrGly | MePhe | MeLeu | Leu | MeLeu | Asp | pip | 1.26 | 1.8E-06 |
| AP134 | nBuGly | MePhe | Ile | MeLeu | Thr | nPrGly | MePhe | MeLeu | bAla(2S-MeOEtOH) | MeLeu | Asp | pip | 1.26 | 6.2E-07 |
| AP135 | D-Val | MeVal | Phe(3-OMe-4-CONHMs) | MeLeu | Thr | nBuGly | MeLeu | Ser(tBu) | MePhe | MeLeu | Asp | pip | 1.28 | 8.8E-07 |
| AP136 | D-Leu | MePhe | Val | MeLeu | Thr | nBuGly | MeLeu | Phe(3-OMe-4-CONHMs) | MeLeu | MeLeu | Asp | pip | 1.38 | 4.7E-07 |
| AP137 | D-Ala | MePhe | Phe(3-OMe-4-CONHMs) | MeLeu | Thr | nBuGly | MeLeu | Ser(tBu) | Melle | MeLeu | Asp | pip | 1.24 | < 2.9E-07 |
| AP138 | gMeAbu | MeLeu | Ile | MePhe | MeLeu | Phe(3-OMe-4-CONHMs) | MeLeu | Thr | MeLeu | MeLeu | Asp | pip | 1.24 | 6.0E-08 |
| AP139 | MeLeu | Phe(3-OMe-4-CONHMs) | MeLeu | MePhe | Leu | MeLeu | Melle | Thr | MeGly | MeAbu | Asp | pip | 1.24 | < 2.5E-07 |
| AP140 | MeGly | MePhe(3-Cl) | Phe(3-OMe-4-CONHMs) | MeLeu | Thr | nBuGly | MeLeu | MeLeu | Ser(tBu) | MeLeu | Asp | pip | 1.31 | 4.9E-07 |
| AP141 | gMeAbu | MeLeu | Ala | MeLeu | MePhe | Ser(tBu) | MeLeu | Phe(3-OMe-4-CONHMs) | MeLeu | MeLeu | Asp | pip | 1.25 | < 2.7E-07 |
| AP142 | MeLeu | Ala | MeLeu | MeLeu | Leu | MeLeu | MeVal | Phe(3-OMe-4-CONHMs) | MeGly | MeLeu | Asp | pip | 1.34 | 6.3E-07 |
| AP143 | MeGly | MeLeu | Val | MeLeu | Phe(3-OMe-4-CONHMs) | MeGly | MePhe | MeLeu | Abu | MeLeu | Asp | pip | 1.20 | 1.3E-07 |

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| AP200 | D-Val | MePhe | Leu | MeLeu | Thr | MeGly | MeLeu | Hph(3-OMe-4-CONMe) | MeLeu | MeLeu | Asp | pip | 1.27 | 8.8E-07 |
| AP201 | D-Leu | MePhe | Hph(3-OMe-4-CONMe) | MeLeu | Thr | nBuGly | MeLeu | Ser(tBu) | MeLeu | MeAbu | Asp | pip | 1.34 | 3.5E-07 |
| AP202 | gMeAbu | MeLeu | Ile | MePhe | MeLeu | Hph(3-OMe-4-CONMe) | MeLeu | Thr | MeLeu | MeLeu | Asp | pip | 1.29 | 1.8E-07 |
| AP203 | gMeAbu | MeLeu | Hph(3-OMe-4-CONMe) | MeLeu | MePhe | Ser(tBu) | MeLeu | Thr | MeLeu | MeLeu | Asp | pip | 1.24 | 4.1E-07 |
| AP204 | MeAbu | Leu | MeLeu | MePhe | Hph(3-OMe-4-CONMe) | MeLeu | MeLeu | Thr | MeGly | MeLeu | Asp | pip | 1.29 | 1.6E-06 |
| AP205 | MeAbu | Hph(3-OMe-4-CONMe) | MeLeu | MeLeu | Val | MePhe | MeLeu | Thr | MeGly | MeLeu | Asp | pip | 1.24 | 7.9E-07 |
| AP206 | MeGly | MeLeu | Hph(3-OMe-4-CONMe) | MeAbu | Thr | nBuGly | MePhe | MeLeu | Leu | MeLeu | Asp | pip | 1.26 | 8.6E-07 |
| AP207 | MeGly | MeLeu | Ile | MeLeu | Thr | nBuGly | MePhe | MeLeu | Hph(3-OMe-4-CONMe) | MeLeu | Asp | pip | 1.35 | 5.1E-07 |
| AP208 | D-Val | MePhe | Leu | MeLeu | Thr | MeGly | MeLeu | D-Hph(3-OMe-4-CONMe) | MeLeu | MeLeu | Asp | pip | 1.27 | 6.0E-07 |
| AP209 | D-Leu | MePhe | D-Hph(3-OMe-4-CONMe) | MeLeu | Thr | nBuGly | MeLeu | Ser(tBu) | MeLeu | MeAbu | Asp | pip | 1.34 | 5.6E-07 |
| AP210 | gMeAbu | MeLeu | Ile | MePhe | MeLeu | D-Hph(3-OMe-4-CONMe) | MeLeu | Thr | MeLeu | MeLeu | Asp | pip | 1.29 | 3.4E-07 |
| AP211 | gMeAbu | MeLeu | D-Hph(3-OMe-4-CONMe) | MeLeu | MePhe | Ser(tBu) | MeLeu | Thr | MeLeu | MeLeu | Asp | pip | 1.24 | 3.6E-07 |
| AP212 | MeAbu | Leu | MeLeu | MePhe | D-Hph(3-OMe-4-CONMe) | MeLeu | MeLeu | Thr | MeGly | MeLeu | Asp | pip | 1.29 | 6.4E-07 |
| AP213 | MeAbu | D-Hph(3-OMe-4-CONMe) | MeLeu | MeLeu | Val | MePhe | MeLeu | Thr | MeGly | MeLeu | Asp | pip | 1.24 | 3.4E-07 |
| AP214 | MeGly | MeLeu | D-Hph(3-OMe-4-CONMe) | MeAbu | Thr | nBuGly | MePhe | MeLeu | Leu | MeLeu | Asp | pip | 1.26 | 8.4E-07 |
| AP215 | MeGly | MeLeu | Ile | MeLeu | Thr | nBuGly | MePhe | MeLeu | D-Hph(3-OMe-4-CONMe) | MeLeu | Asp | pip | 1.35 | 4.3E-07 |
| AP216 | D-Ala | MePhe | Leu | MeAbu | Thr | (Ph(3-OMe-4-CONMe)Et)Gly | MeLeu | Ser(tBu) | MeLeu | MeLeu | Asp | pip | 1.20 | 9.9E-07 |
| AP217 | MeVal | Leu | MeAbu | MePhe | Ala | MeLeu | MeLeu | Thr | (Ph(3-OMe-4-CONMe)Et)Gly | MeLeu | Asp | pip | 1.26 | 1.0E-06 |
| AP218 | MeGly | MeLeu | Ile | MeAbu | Thr | (Ph(3-OMe-4-CONMe)Et)Gly | MePhe | MeLeu | Ser(tBu) | MeLeu | Asp | pip | 1.21 | 5.5E-07 |
| AP219 | (Ph(3-OMe-4-CONMe)Et)Gly | MeLeu | Leu | MeAbu | Thr | MeGly | MePhe | MeLeu | Ser(tBu) | MeLeu | Asp | pip | 1.21 | 1.3E-06 |
| AP220 | D-Leu | MePhe | Leu | MeLeu | Thr | nBuGly | MeAla | Ser(tBu) | MeLeu | (Ph(3-OMe-4-CONMe)Et)Gly | Asp | pip | 1.26 | 8.0E-07 |

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| AP221 | D-Leu | MeLeu | Ile | MeLeu | Thr | MeGly | (Ph(3-OMe-4-CONMe)Et)Gly | Ser(tBu) | MePhe | MeLeu | Asp | pip | 1.24 | 2.8E-07 |
| AP222 | D-Ala | MeLeu | Ile | (Ph(3-OMe-4-CONMe)Et)Gly | Thr | MeAbu | MeLeu | Ser(tBu) | MePhe | MeLeu | Asp | pip | 1.20 | 3.1E-07 |
| AP223 | MeAbu | Leu | MeLeu | MePhe | Leu | MeLeu | (Ph(3-OMe-4-CONMe)Et)Gly | Thr | MeGly | MeLeu | Asp | pip | 1.26 | 1.6E-06 |
| AP224 | D-Val | MeHph(3-OMe-4-CONMe) | Leu | MeLeu | Thr | MeGly | MeLeu | Leu | MePhe | MeLeu | Asp | pip | 1.27 | 1.1E-06 |
| AP225 | D-Leu | MePhe | Val | MeLeu | Thr | MeGly | MeLeu | Leu | MeHph(3-OMe-4-CONMe) | MeLeu | Asp | pip | 1.27 | 5.7E-07 |
| AP226 | D-Val | MeLeu | Val | MeHph(3-OMe-4-CONMe) | Thr | MeGly | MeLeu | Leu | MePhe | MeLeu | Asp | pip | 1.23 | 1.0E-06 |
| AP227 | gMeAbu | MeLeu | Ile | MeHph(3-OMe-4-CONMe) | MePhe | Ser(tBu) | MeLeu | Thr | MeLeu | MeLeu | Asp | pip | 1.24 | 4.8E-07 |
| AP228 | gMeAbu | MeLeu | Ile | MePhe | MeLeu | Ser(tBu) | MeLeu | Thr | MeHph(3-OMe-4-CONMe) | MeLeu | Asp | pip | 1.24 | 8.4E-07 |
| AP229 | MeAla | Leu | MeLeu | MeHph(3-OMe-4-CONMe) | Abu | MePhe | MeLeu | Thr | MeGly | MeLeu | Asp | pip | 1.16 | 4.3E-07 |
| AP230 | MeAla | Leu | MeLeu | MePhe | Leu | MeHph(3-OMe-4-CONMe) | MeLeu | Thr | MeGly | MeLeu | Asp | pip | 1.24 | 9.6E-07 |
| AP231 | MeGly | MeHph(3-OMe-4-CONMe) | Val | MeLeu | Thr | nBuGly | MePhe | MeLeu | Ser(tBu) | MeLeu | Asp | pip | 1.25 | 1.0E-06 |
| AP232 | nBuGly | MePhe | Ile | MeLeu | Thr | MeGly | MeHph(3-OMe-4-CONMe) | MeLeu | Ser(tBu) | MeLeu | Asp | pip | 1.30 | 2.0E-06 |
| AP233 | D-MeLeu | MeLeu | Leu | MeLeu | Thr | Hyp(2-EtOH) | MeLeu | Ile | MeLeu | Leu | Asp | pip | 1.33 | 3.0E-06 |
| AP234 | D-Leu | Pro | Ser(tBu) | MeLeu | Hyp(2-EtOH) | MeLeu | Thr | MePhe | Phe | MeLeu | Asp | pip | 1.13 | 1.3E-06 |
| AP235 | Leu | MeLeu | Ser(tBu) | MeLeu | Hyp(2-EtOH) | MeLeu | Thr | MePhe | Phe | MeLeu | Asp | pip | 1.28 | 1.5E-06 |
| AP236 | D-MeLeu | MeLeu | Hyp(2-EtOH) | MeLeu | Thr | MeLeu | MeVal | Phe | Pic(2) | Leu | Asp | pip | 1.24 | 3.8E-06 |
| AP237 | D-Val | MeLeu | Ser(tBu) | MeLeu | MeLeu | Leu | Thr | Hyp(2-EtOH) | Phe | MeLeu | Asp | pip | 1.17 | 1.3E-06 |
| AP238 | D-Leu | MeIle | Pro | MeLeu | Ser(tBu) | Hyp(2-EtOH) | Thr | MeLeu | MeLeu | MeLeu | Asp | pip | 1.20 | 2.0E-06 |
| AP239 | D-Val | MePhe | Leu | MeAbu | Thr | nBuGly | MeLeu | Ser(S-Mor-3-Me) | MeLeu | MeLeu | Asp | pip | 1.22 | 1.8E-07 |
| AP240 | D-Leu | MeLeu | Ile | MeLeu | Thr | nBuGly | MeAbu | Ser(S-Mor-3-Me) | MePhe | MeAbu | Asp | pip | 1.18 | 8.0E-08 |
| AP241 | gMeAbu | MeLeu | Ile | MePhe | MeLeu | Ser(S-Mor-3-Me) | MeLeu | Thr | MeLeu | MeLeu | Asp | pip | 1.18 | 5.0E-08 |
| AP242 | MeAbu | Leu | MeLeu | MePhe | Ser(S-Mor-3-Me) | MeAbu | MeLeu | Thr | nBuGly | MeLeu | Asp | pip | 1.25 | 1.2E-06 |
| AP243 | MeAla | Ser(S-Mor-3-Me) | MeLeu | MeAbu | Val | MePhe | MeLeu | Thr | nBuGly | MeLeu | Asp | pip | 1.15 | 4.6E-07 |
| AP244 | MeGly | MeLeu | Ser(S-Mor-3-Me) | MeLeu | Thr | nBuGly | MePhe | MeLeu | Val | MeLeu | Asp | pip | 1.19 | 4.6E-07 |
| AP245 | MeGly | MeLeu | Ile | MeLeu | Thr | nBuGly | MePhe | MeLeu | Ser(S-Mor-3-Me) | MeLeu | Asp | pip | 1.24 | 1.0E-07 |
| AP246 | MeAla | Leu | MeAbu | MePhe | Leu | MeLeu | MeLeu | Ser(S-Mor-3-Me) | MeGly | MeLeu | Asp | pip | 1.24 | 2.3E-07 |
| AP247 | MeGly | MeAbu | Val | MeAbu | Ser(S-Mor-3-Me) | nBuGly | MePhe | MeLeu | Leu | MeLeu | Asp | pip | 1.21 | 6.9E-07 |

| Compound ID | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|
| AP248 | D-Val | MeVal | Phe(4-CONMeOH) | MeLeu | Thr | nBuGly | MeLeu | Ser(tBu) | MePhe | MeLeu | Asp | pip | 1.34 | 2.4E-07 |
| AP249 | D-Leu | MePhe | Val | MeLeu | Thr | nBuGly | MeLeu | Phe(4-CONMeOH) | MeLeu | MeLeu | Asp | pip | 1.44 | 4.0E-08 |
| AP250 | D-Ala | MePhe | Phe(4-CONMeOH) | MeLeu | Thr | nBuGly | MeLeu | Ser(tBu) | MeIle | MeLeu | Asp | pip | 1.30 | 1.2E-07 |
| AP251 | gMeAbu | MeLeu | Ile | MePhe | MeLeu | Phe(4-CONMeOH) | MeLeu | Thr | MeLeu | MeLeu | Asp | pip | 1.31 | 3.0E-08 |
| AP252 | MeLeu | Phe(4-CONMeOH) | MeLeu | MePhe | Leu | MeLeu | MeIle | Thr | MeGly | MeAbu | Asp | pip | 1.31 | 1.3E-07 |
| AP253 | MeLeu | Leu | MeLeu | MeLeu | Phe(4-CONMeOH) | MeLeu | MeVal | Thr | MeGly | MeLeu | Asp | pip | 1.35 | 1.2E-07 |
| AP254 | MeGly | MePhe(3-Cl) | Phe(4-CONMeOH) | MeLeu | Thr | nBuGly | MeLeu | MeLeu | Ser(tBu) | MeLeu | Asp | pip | 1.38 | 1.5E-07 |
| AP255 | MeGly | MeLeu | Ile | MeLeu | Thr | MeGly | MePhe(3-Cl) | MeLeu | Phe(4-CONMeOH) | MeLeu | Asp | pip | 1.28 | 5.0E-08 |
| AP256 | D-Val | MeVal | Phe(3-OMe-4-CONHOH) | MeLeu | Thr | nBuGly | MeLeu | Ser(tBu) | MePhe | MeLeu | Asp | pip | 1.23 | 8.0E-08 |
| AP257 | D-Leu | MePhe | Val | MeLeu | Thr | nBuGly | MeLeu | Phe(3-OMe-4-CONHOH) | MeLeu | MeLeu | Asp | pip | 1.33 | 3.0E-08 |
| AP258 | D-Ala | MePhe | Phe(3-OMe-4-CONHOH) | MeLeu | Thr | nBuGly | MeLeu | Ser(tBu) | MeIle | MeLeu | Asp | pip | 1.19 | 3.0E-08 |
| AP259 | gMeAbu | MeLeu | Ile | MePhe | MeLeu | Phe(3-OMe-4-CONHOH) | MeLeu | Thr | MeLeu | MeLeu | Asp | pip | 1.21 | 2.0E-08 |
| AP260 | MeGly | MePhe(3-Cl) | Phe(3-OMe-4-CONHOH) | MeLeu | Thr | nBuGly | MeLeu | MeLeu | Ser(tBu) | MeLeu | Asp | pip | 1.28 | 4.0E-08 |
| AP261 | MeGly | MeLeu | Ile | MeLeu | Thr | MeGly | MePhe(3-Cl) | MeLeu | Phe(3-OMe-4-CONHOH) | MeLeu | Asp | pip | 1.17 | 2.0E-08 |

| Compound ID | 13 | 12 | 11 | 10 | 9 | 8 | 7 | 6 | 5 | 4 | 3 | 2 | 1 | C-term | clogP/ total AA | Caco-2 (cm/sec) |
|---|---|---|---|---|---|---|---|---|---|---|---|---|---|---|---|---|
| AP262 | | | | | | D-MeAla | MeLeu | MeLeu | Thr | MeLeu | Hph(3-OMe-4-CONMe) | MeLeu | Asp | pip | 1.23 | 1.7E-07 |
| AP263 | | | | | Leu | MeLeu | Leu | Ile | MeLeu | Hph(3-OMe-4-CONMe) | Thr | MeLeu | Asp | pip | 1.29 | 1.8E-06 |
| AP264 | | | | D-MeLeu | Ser(tBu) | MeLeu | Thr | MePhe | MeLeu | Gly | MeIle | Hph(3-OMe-4-CONMe) | Asp | pip | 1.17 | 1.7E-07 |
| AP265 | | D-Val | Thr | nBuGly | Leu | Pro | MeVal | Hph(3-OMe-4-CONMe) | MePhe | MeLeu | Leu | MeIle | Asp | pip | 1.25 | 4.6E-07 |
| AP266 | MeAbu | Gly | MeLeu | MeLeu | Val | MeLeu | Hph(3-OMe-4-CONMe) | MePhe | Ile | MeLeu | MeLeu | Thr | Asp | pip | 1.28 | < 8.95E-08 |

When the side chain partial structures of Ser(tBuOH), Ser(NtBu-Aca), bAla(3R-MeOEtOH), Phe(3-OMe-4-CONMe), Phe(4-OMe-3-CONMe), Ser(2-Me-2-BuOH), Ser(S-2-PrOH), Ser(R-2-PrOH), Ser(nPrOH), Ser(S-2-BuOH), Ser(R-2-BuOH), Ser(2-Me2-PrOH), bAla(2S-MeOEtOH), Phe(3-OMe-4-CONHMs), Hph(3-OMe-4-CONMe), D-Hph(3-OMe-4-CONMe), (Ph(3-OMe-4-CONMe)Et)Gly, MeHph(3-OMe-4-CONMe), Hyp(2-EtOH), Ser(S-Mor-3-Me), Phe(4-CONMeOH), and Phe(3-OMe-4-CONHOH) were calculated by the low energy conformation calculation method described in Example 3, their most stable conformations were conformations having an intramolecular hydrogen bond. When the side chain partial structure of Ser(3-Me-5-Oxo-Odz) was calculated, the conformation having an intramolecular hydrogen bond was 0.89 kJ/mol different from the most stable conformation. Accordingly, it is considered that these amino acids also form an intramolecular hydrogen bond when permeating a membrane and have better membrane permeability than amino acids having no intramolecular hydrogen bond.

Structures of cyclic peptides AP01 to AP143 and AP200 to AP266, for which membrane permeability was evaluated, are shown below. (In the structural formulae of the cyclic peptides, hydrogen atoms attached to a heteroatom may be omitted to clarify the structure.)

[Table 15]

| ID | Structural Formula | ID | Structural Formula |
|---|---|---|---|
| AP01 | 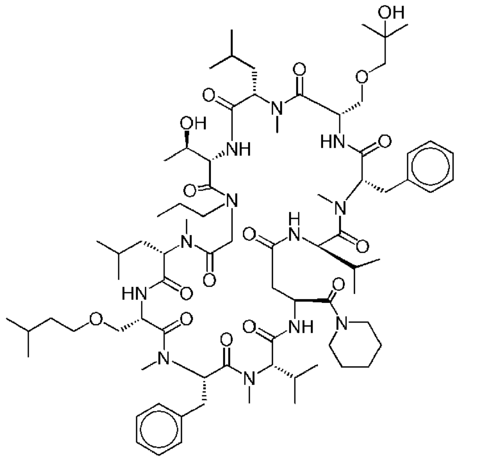 | AP02 | 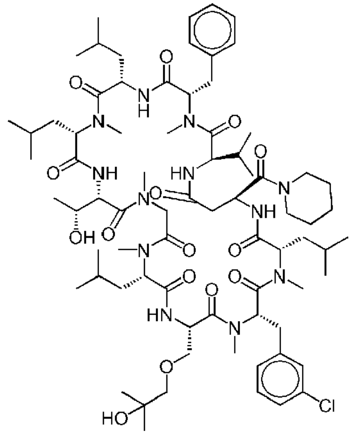 |
| AP03 | 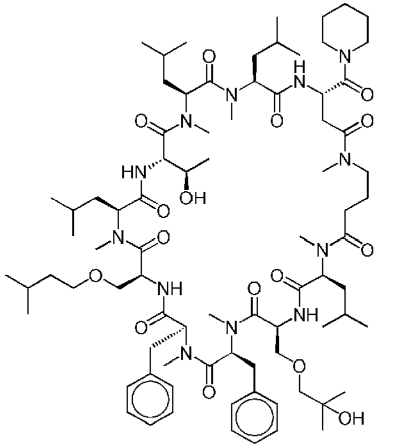 | AP04 | 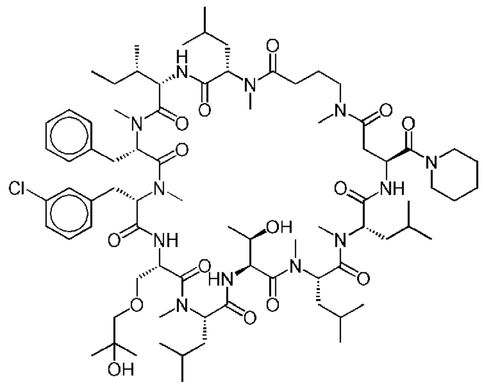 |

(continued)

| ID | Structural Formula | ID | Structural Formula |
|---|---|---|---|
| AP05 | 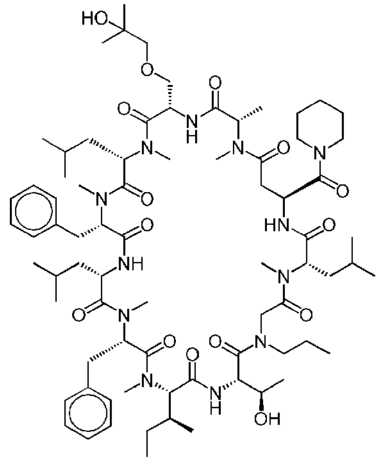 | AP06 | 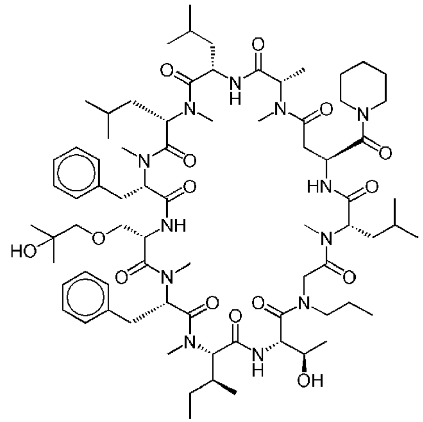 |
| AP07 | 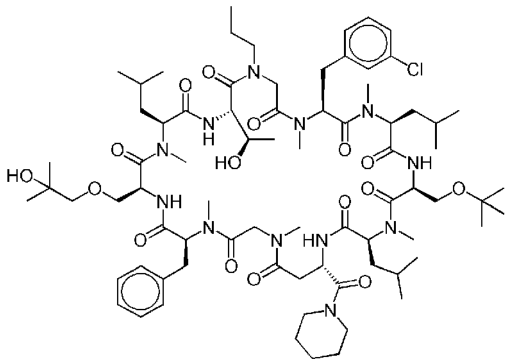 | AP08 | 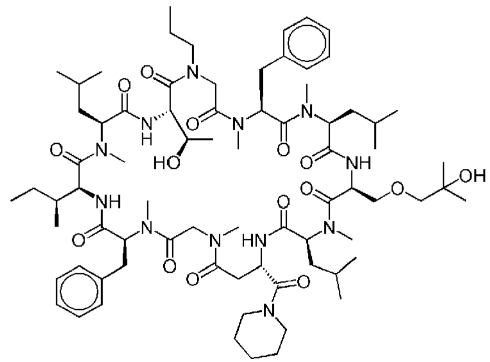 |

(continued)

| ID | Structural Formula | ID | Structural Formula |
|---|---|---|---|
| AP09 | 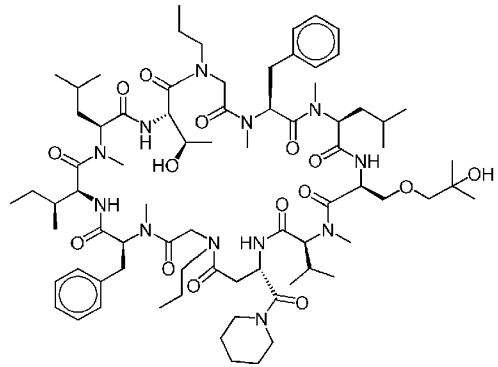 | AP10 | 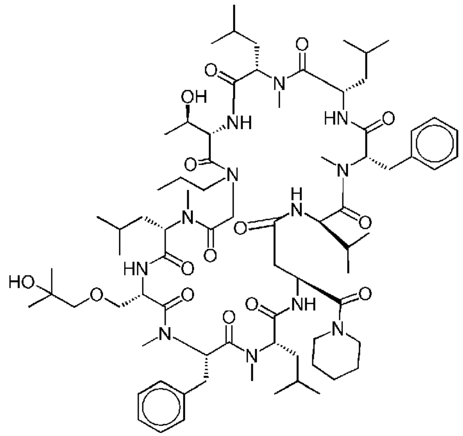 |
| AP11 | 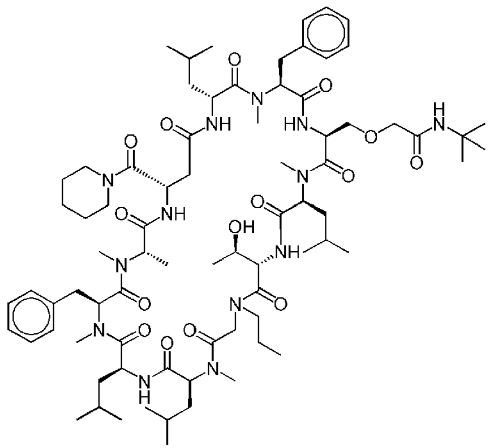 | AP12 | 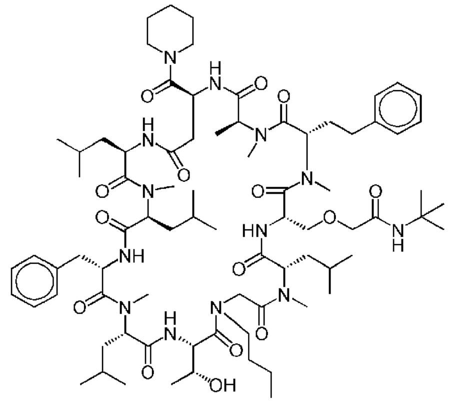 |

(continued)

| ID | Structural Formula | ID | Structural Formula |
|---|---|---|---|
| AP13 | 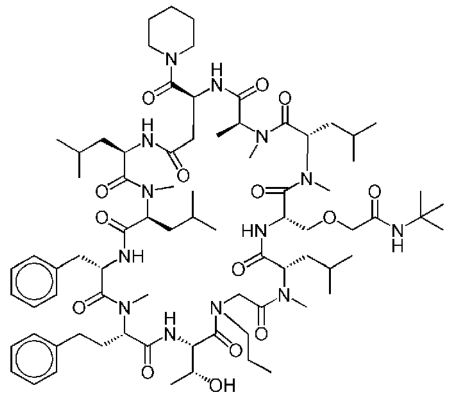 | AP14 | 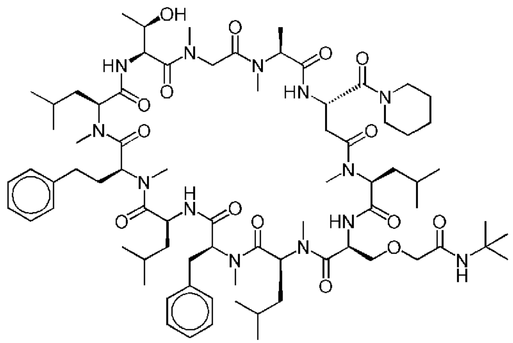 |
| AP15 | 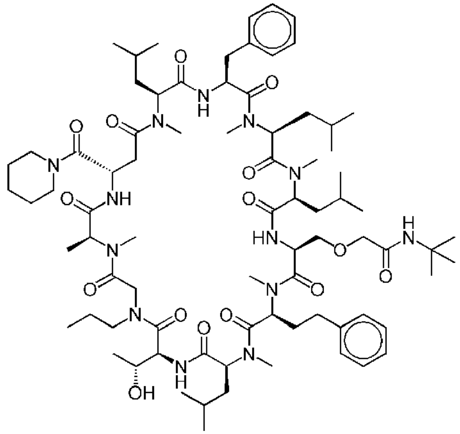 | AP16 | 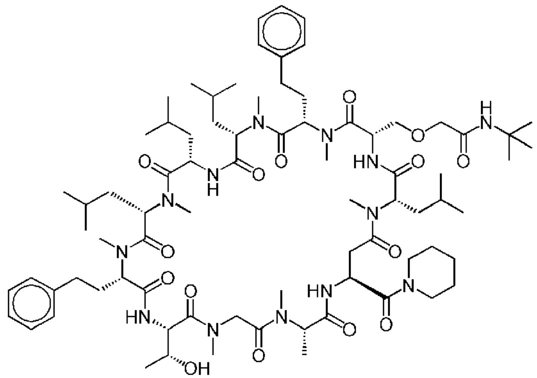 |

(continued)

| ID | Structural Formula | ID | Structural Formula |
| --- | --- | --- | --- |
| AP17 | 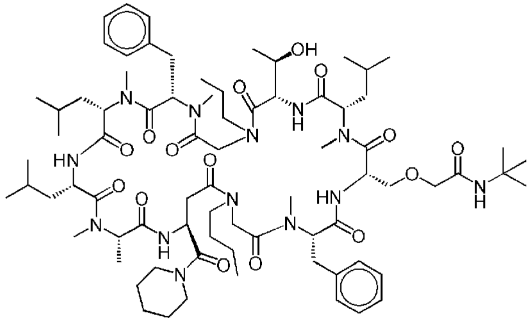 | AP18 | 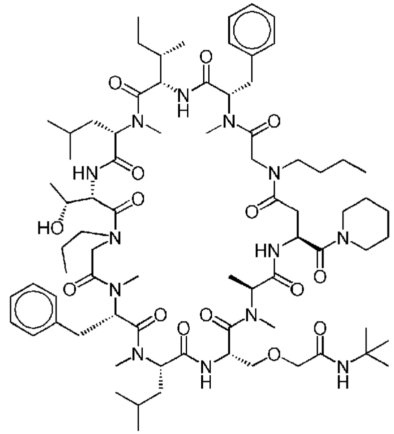 |
| AP19 | 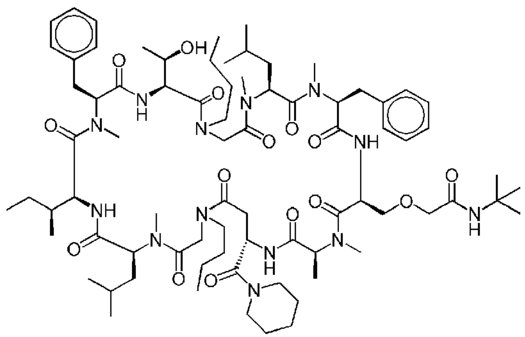 | AP20 | 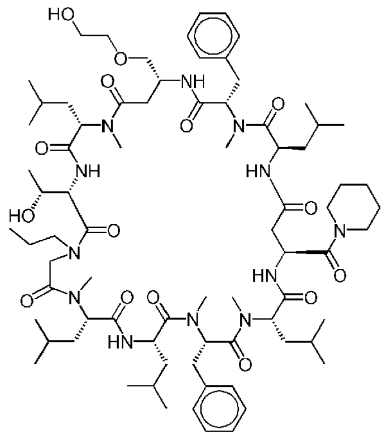 |

(continued)

| ID | Structural Formula | ID | Structural Formula |
|---|---|---|---|
| AP21 | 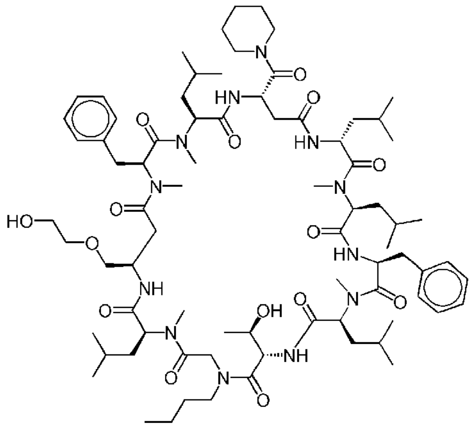 | AP22 | 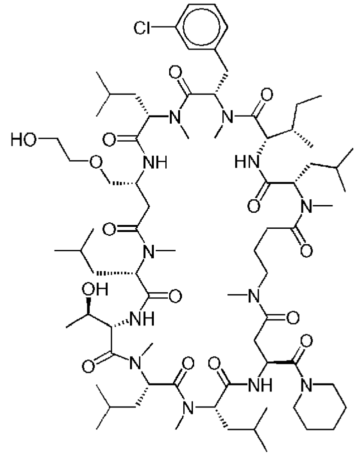 |
| AP23 | 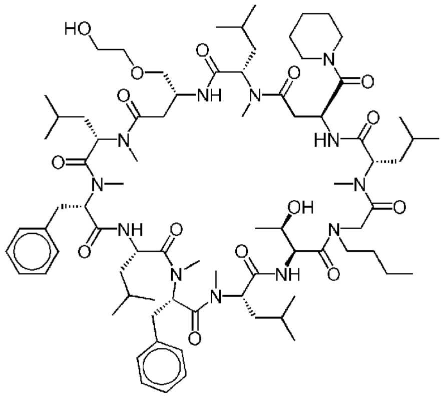 | AP24 | 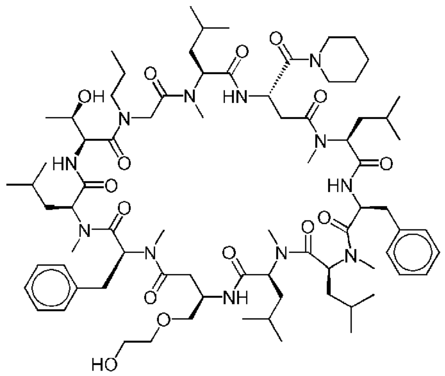 |

(continued)

| ID | Structural Formula | ID | Structural Formula |
|---|---|---|---|
| AP25 | 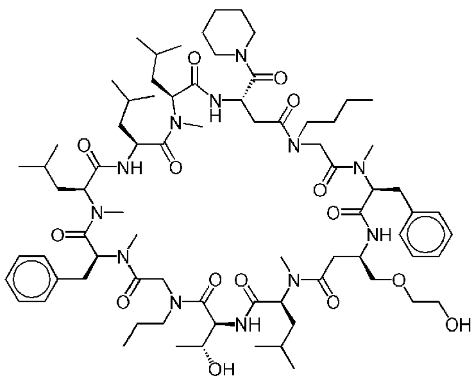 | AP26 | 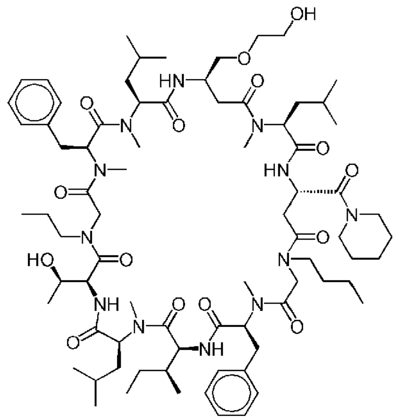 |
| AP27 | 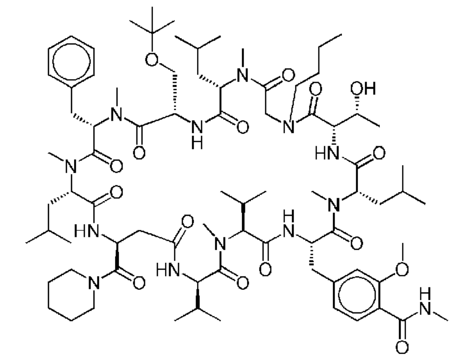 | AP28 | 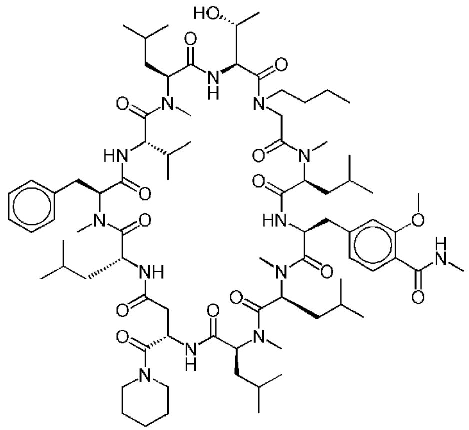 |

(continued)

| ID | Structural Formula | ID | Structural Formula |
|---|---|---|---|
| AP29 | 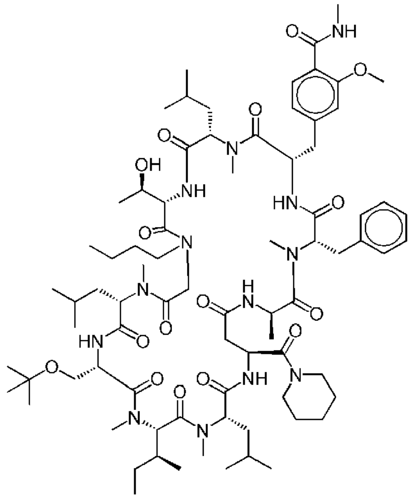 | AP30 | 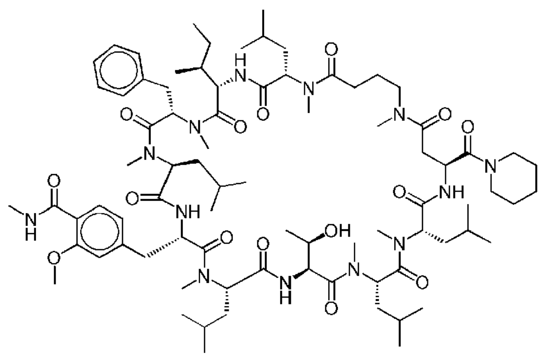 |
| AP31 | 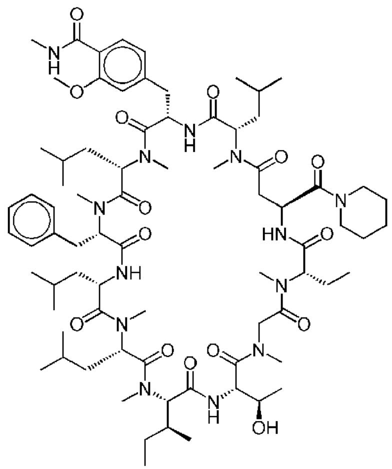 | AP32 | 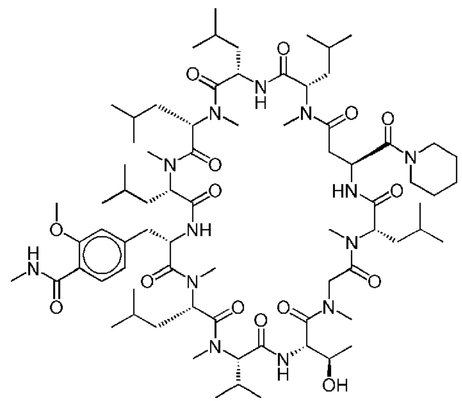 |

(continued)

| ID | Structural Formula | ID | Structural Formula |
|---|---|---|---|
| AP33 | 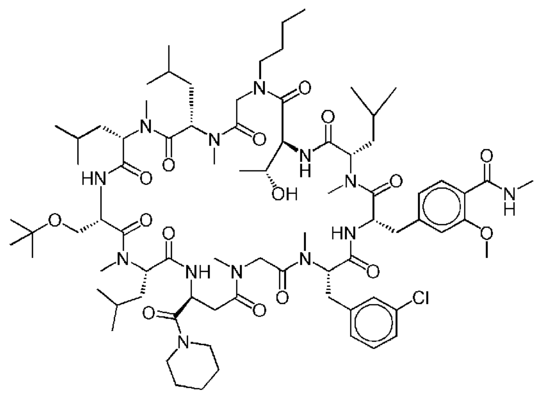 | AP34 | 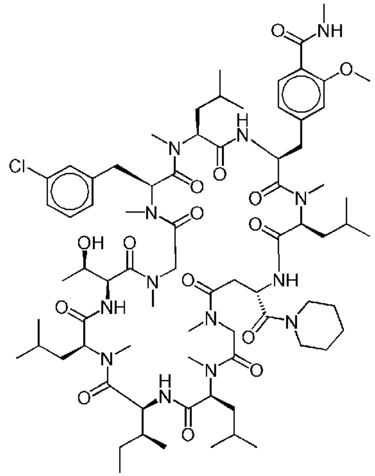 |
| AP35 | 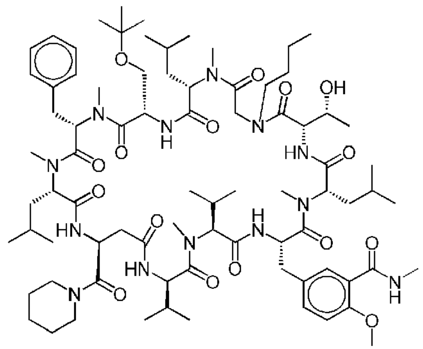 | AP36 | 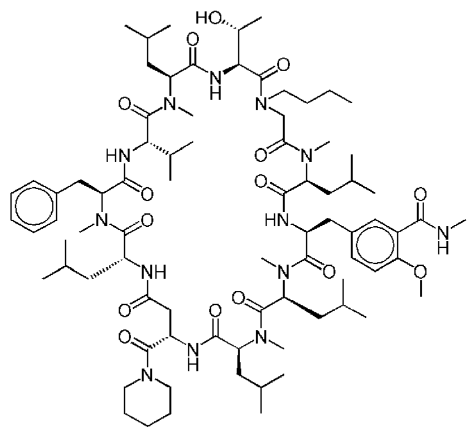 |

(continued)

| ID | Structural Formula | ID | Structural Formula |
|---|---|---|---|
| AP37 | 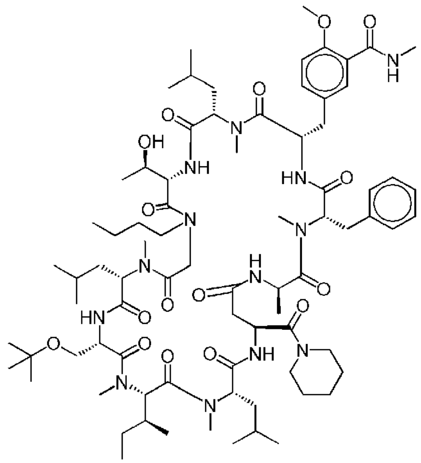 | AP38 | 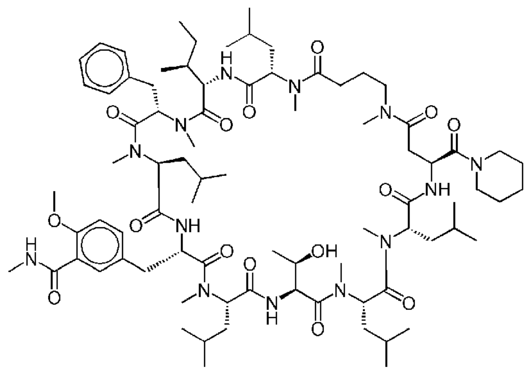 |
| AP39 | 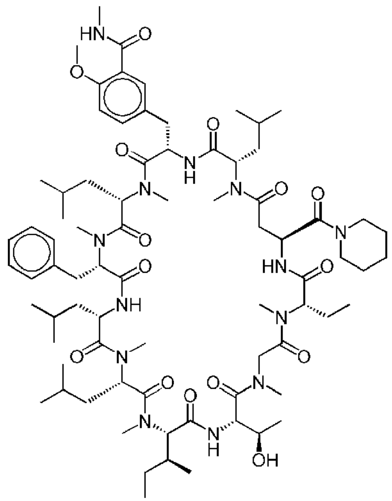 | AP40 | 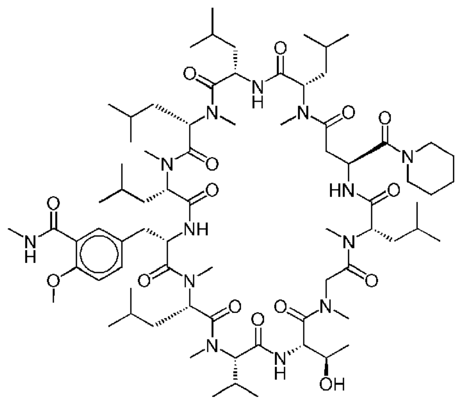 |

(continued)

| ID | Structural Formula | ID | Structural Formula |
|---|---|---|---|
| AP41 | 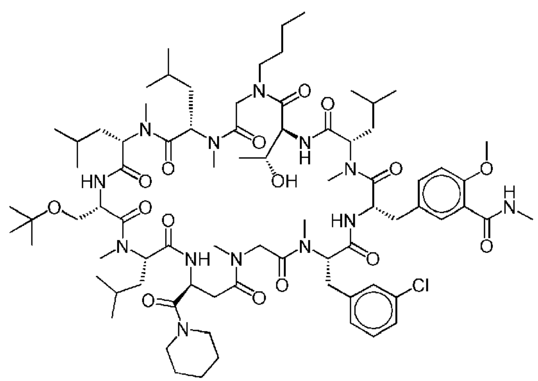 | AP42 | 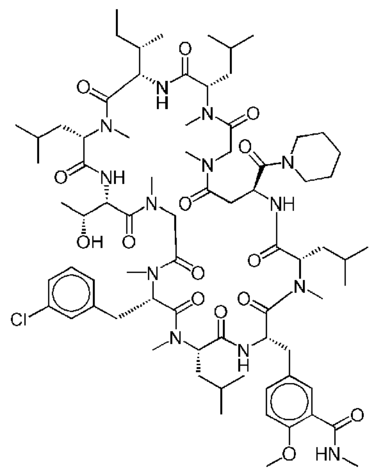 |
| AP43 | 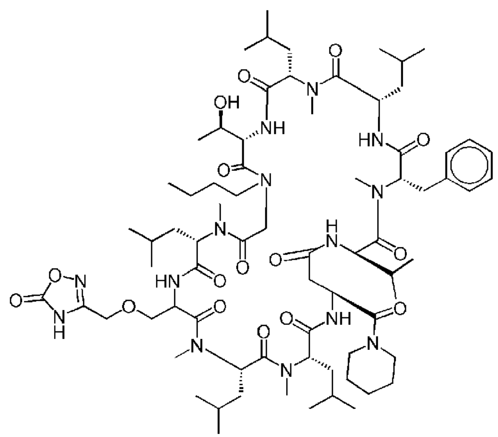 | AP44 | 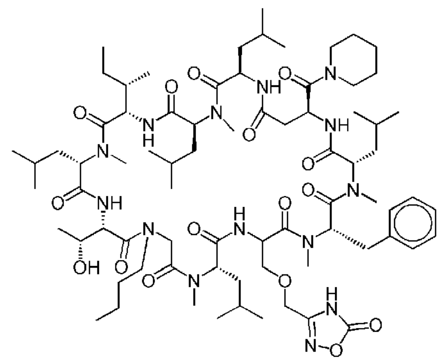 |

(continued)

| ID | Structural Formula | ID | Structural Formula |
|---|---|---|---|
| AP45 | 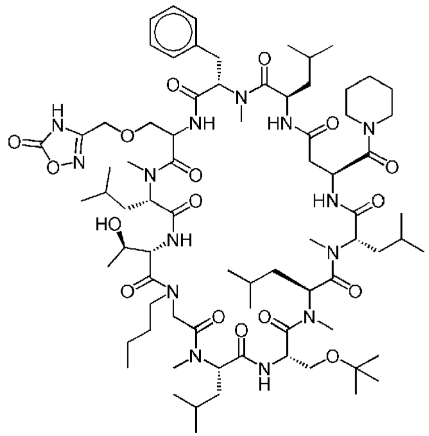 | AP46 | 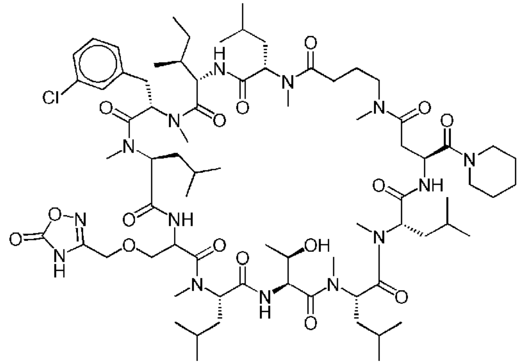 |
| AP47 | 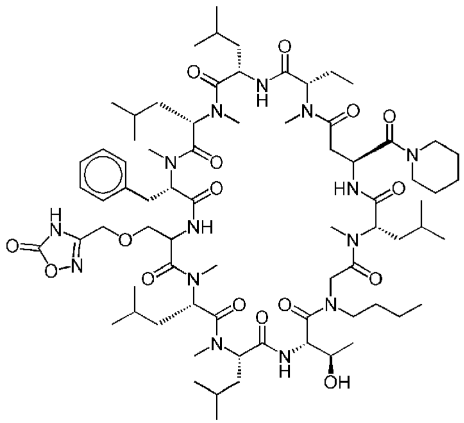 | AP48 | 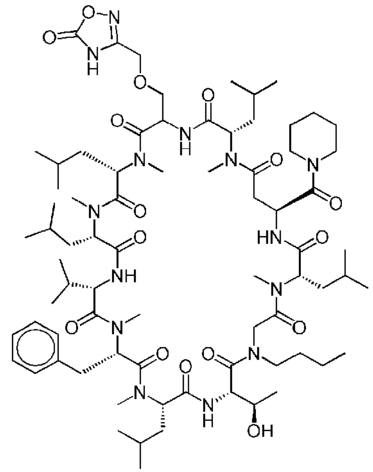 |

(continued)

| ID | Structural Formula | ID | Structural Formula |
|---|---|---|---|
| AP49 | 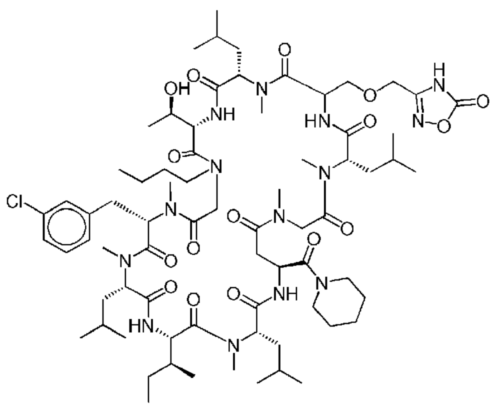 | AP50 | 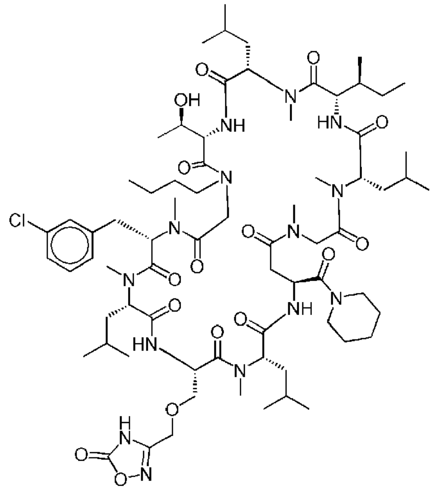 |
| AP51 | 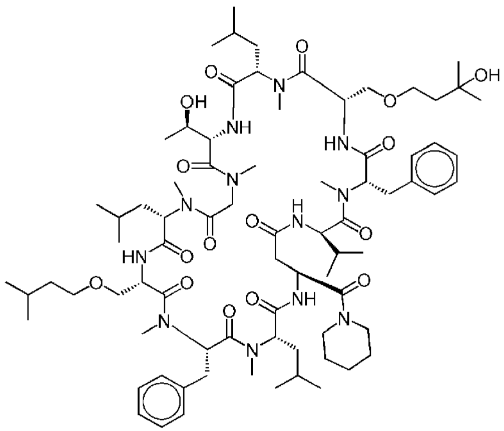 | AP52 | 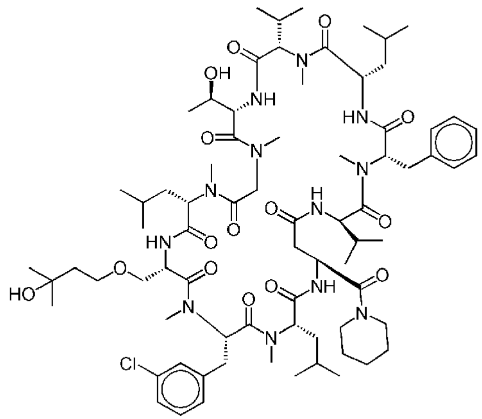 |

(continued)

| ID | Structural Formula | ID | Structural Formula |
|---|---|---|---|
| AP53 | 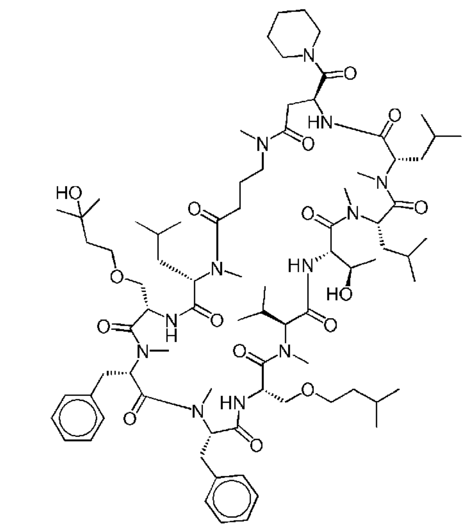 | AP54 | 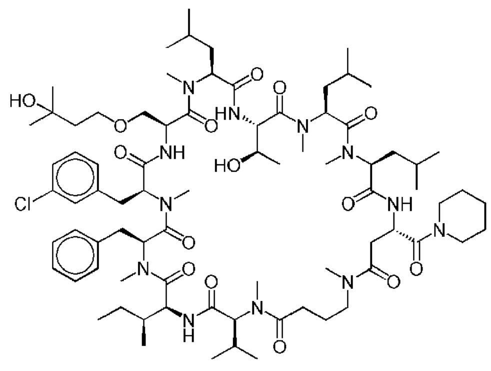 |
| AP55 | 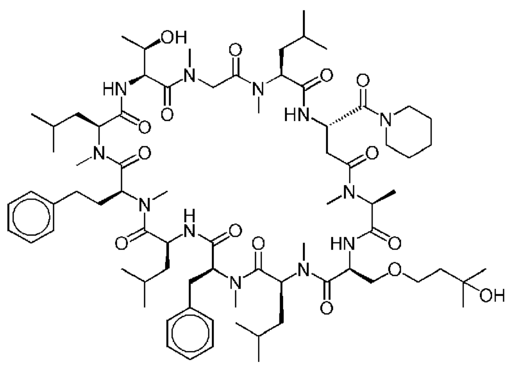 | AP56 | 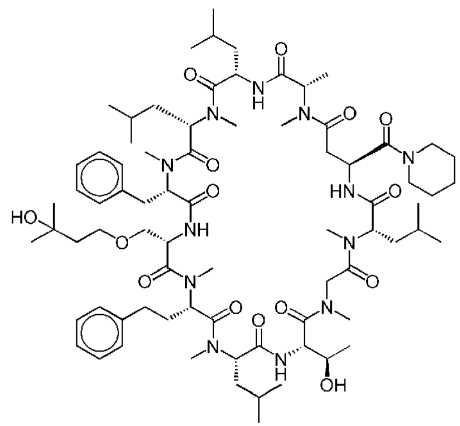 |

(continued)

| ID | Structural Formula | ID | Structural Formula |
|---|---|---|---|
| AP57 | 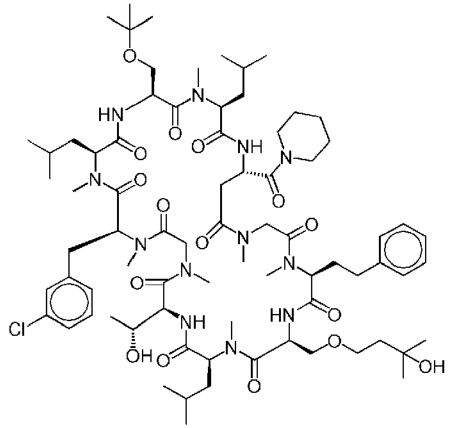 | AP58 | 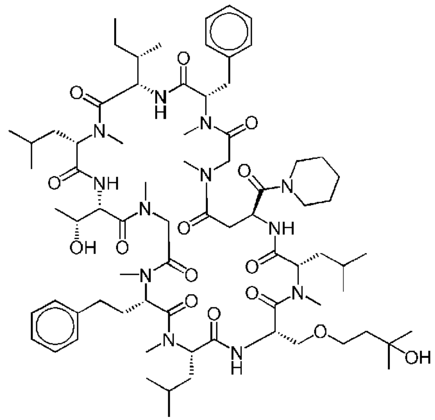 |
| AP59 | 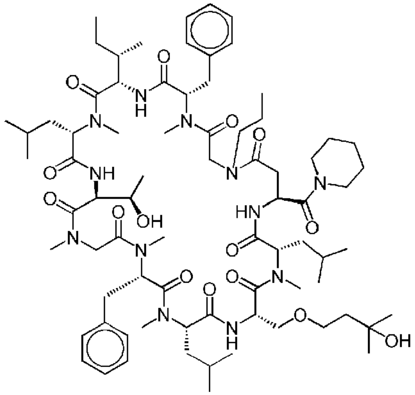 | AP60 | 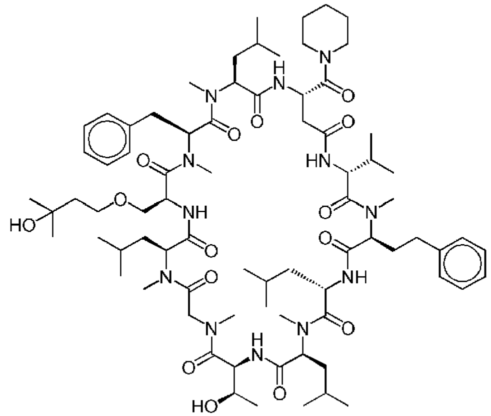 |

(continued)

| ID | Structural Formula | ID | Structural Formula |
|---|---|---|---|
| AP61 | 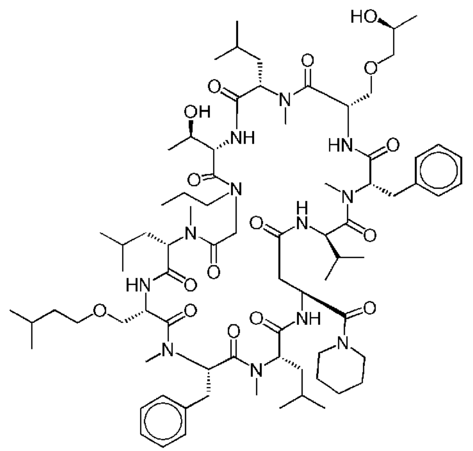 | AP62 | 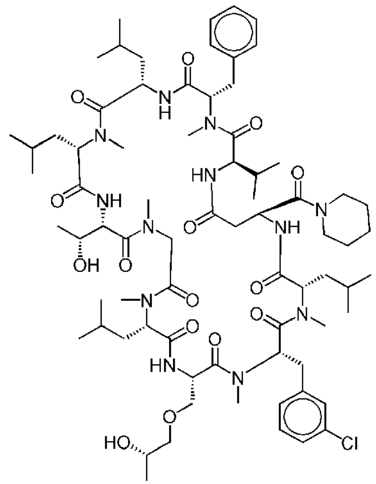 |
| AP63 | 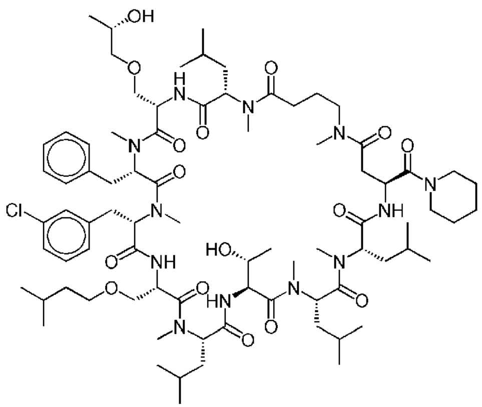 | AP64 | 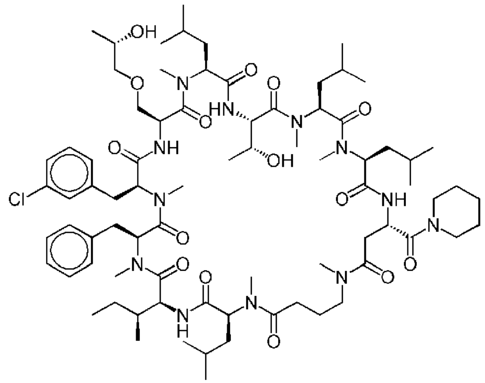 |

(continued)

| ID | Structural Formula | ID | Structural Formula |
|---|---|---|---|
| AP65 | 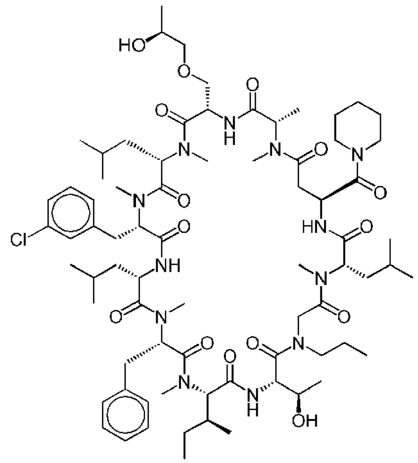 | AP66 | 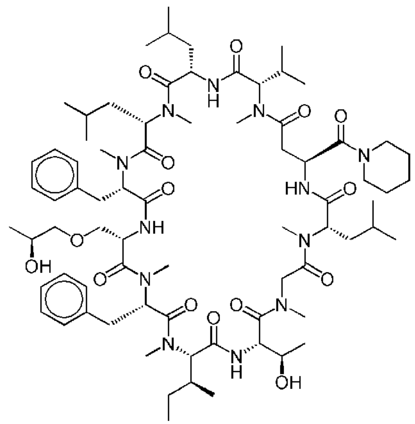 |
| AP67 | 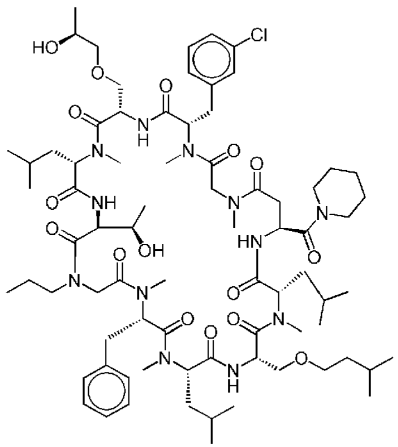 | AP68 | 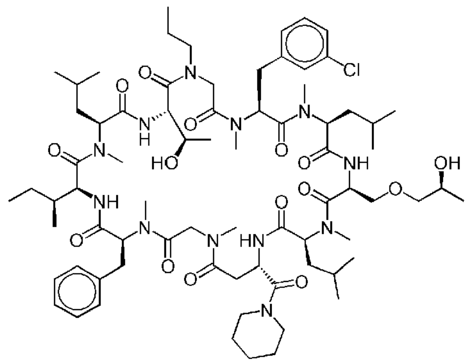 |

(continued)

| ID | Structural Formula | ID | Structural Formula |
|---|---|---|---|
| AP69 | 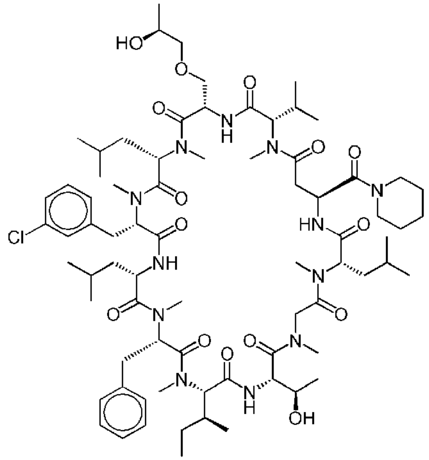 | AP70 | 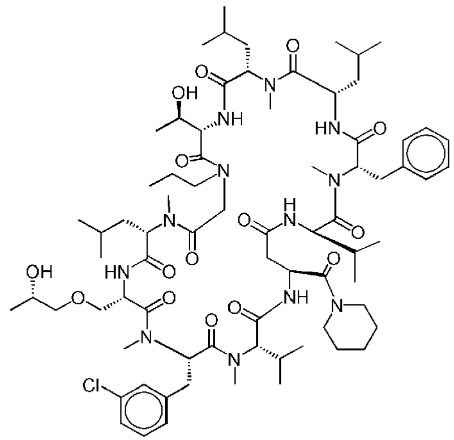 |
| AP71 | 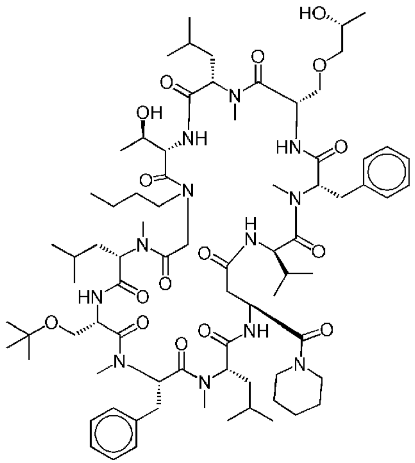 | AP72 | 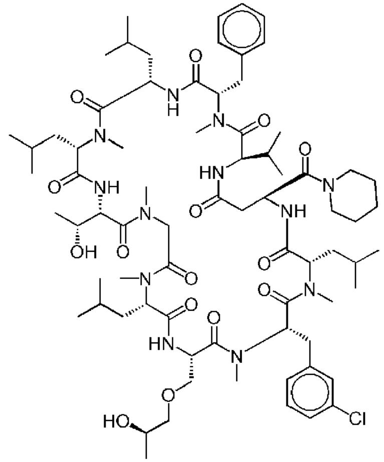 |

(continued)

| ID | Structural Formula | ID | Structural Formula |
| --- | --- | --- | --- |
| AP73 | 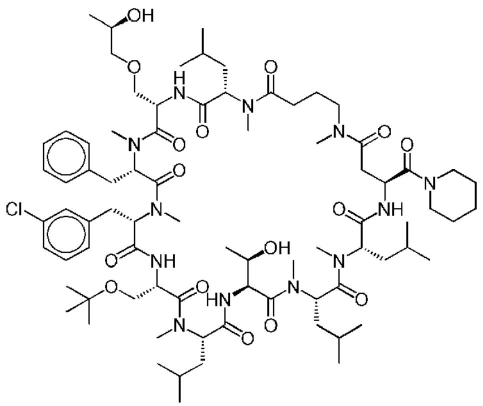 | AP74 | 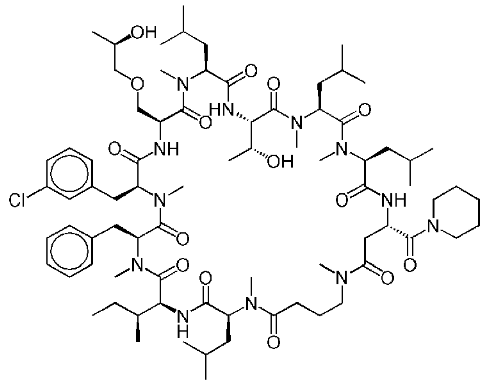 |
| AP75 | 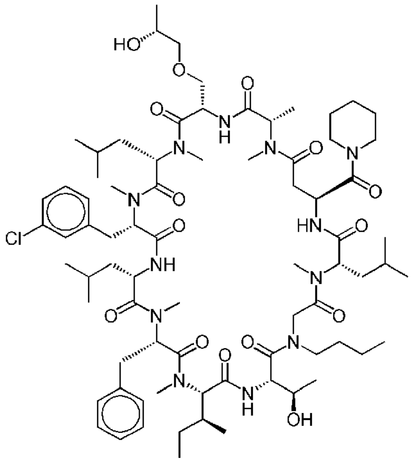 | AP76 | 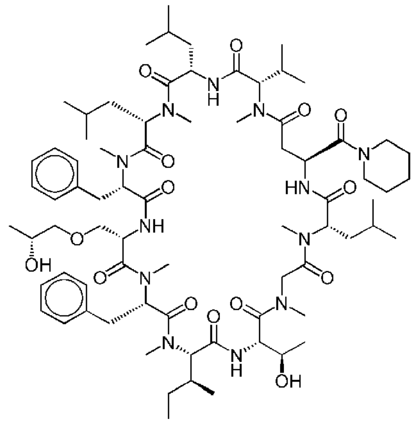 |

(continued)

| ID | Structural Formula | ID | Structural Formula |
|---|---|---|---|
| AP77 | 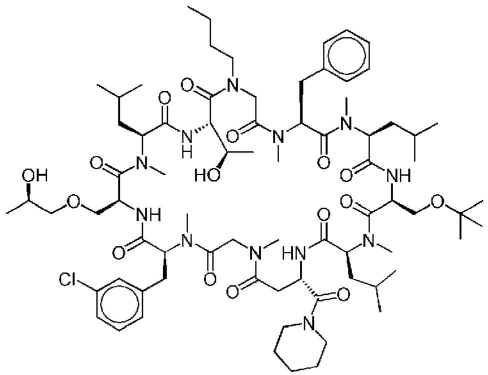 | AP78 | 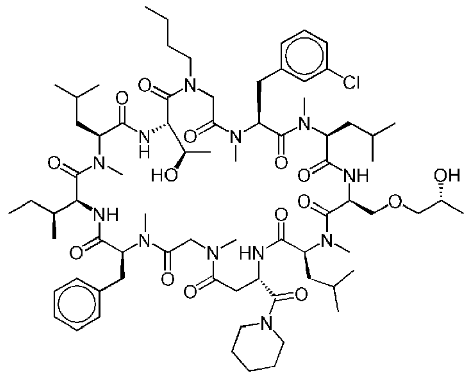 |
| AP79 | 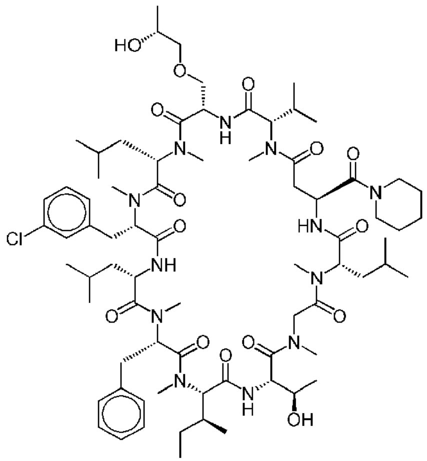 | AP80 | 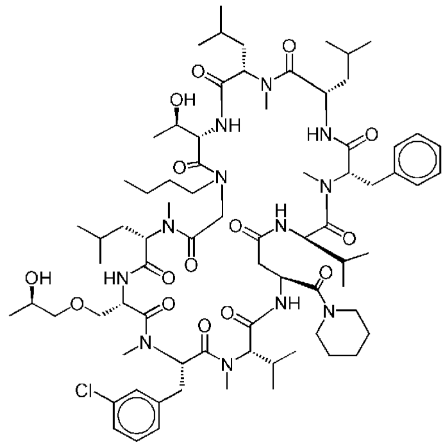 |

(continued)

| ID | Structural Formula | ID | Structural Formula |
|---|---|---|---|
| AP81 | 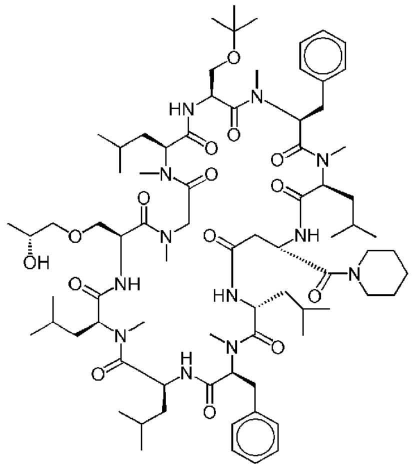 | AP82 | 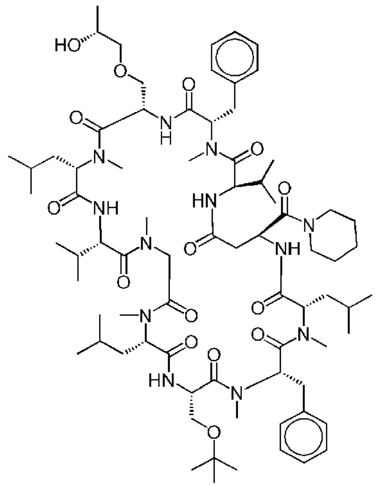 |
| AP83 | 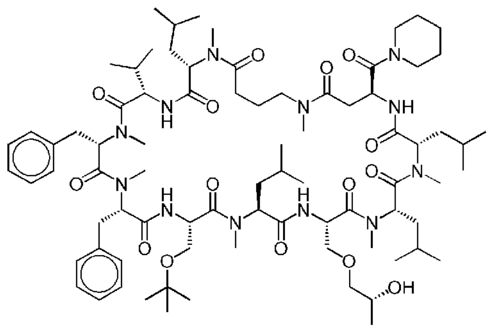 | AP84 | 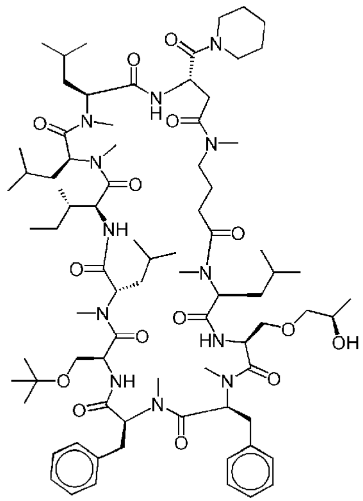 |

(continued)

| ID | Structural Formula | ID | Structural Formula |
|---|---|---|---|
| AP85 | 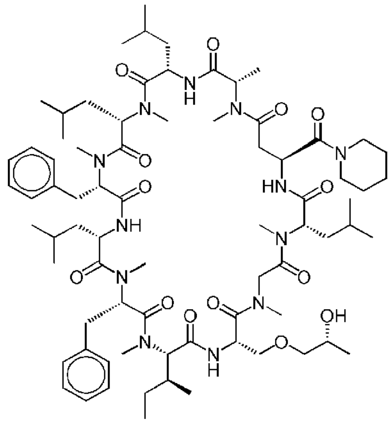 | AP86 | 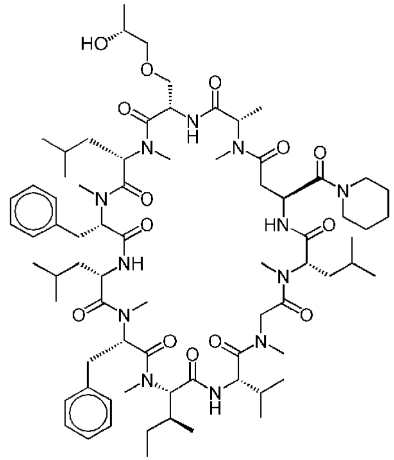 |
| AP87 | 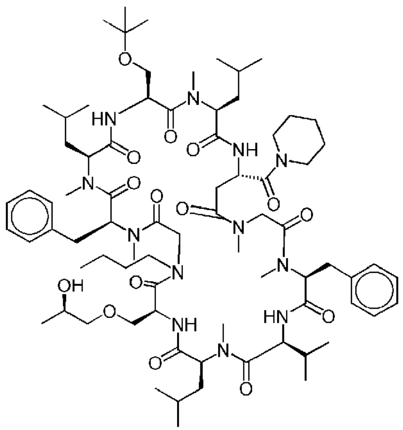 | AP88 | 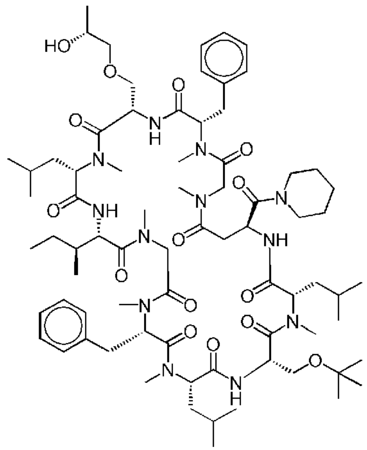 |

(continued)

| ID | Structural Formula | ID | Structural Formula |
|---|---|---|---|
| AP89 | 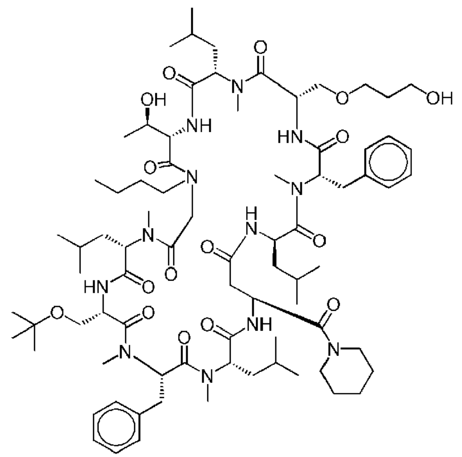 | AP90 | 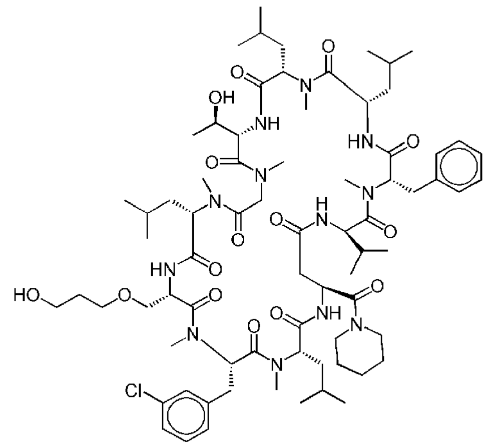 |
| AP91 | 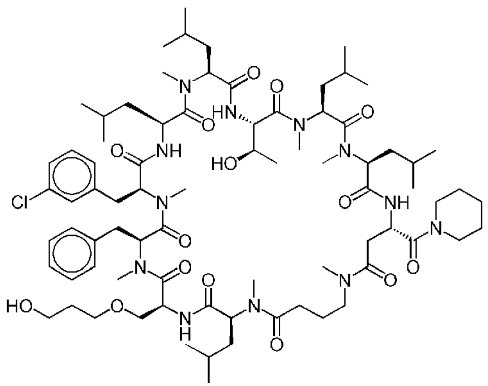 | AP92 | 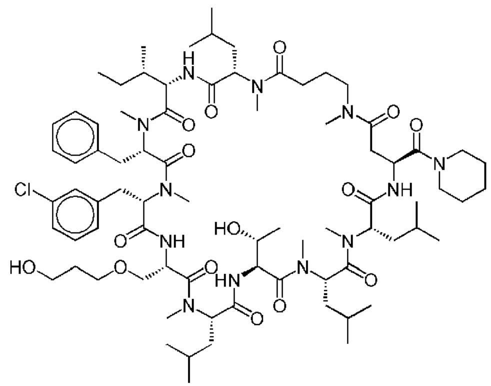 |

(continued)

| ID | Structural Formula | ID | Structural Formula |
|---|---|---|---|
| AP93 | 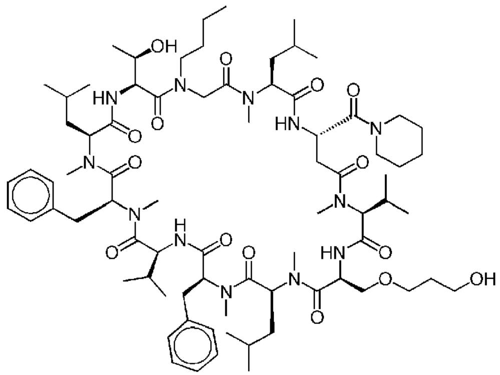 | AP94 | 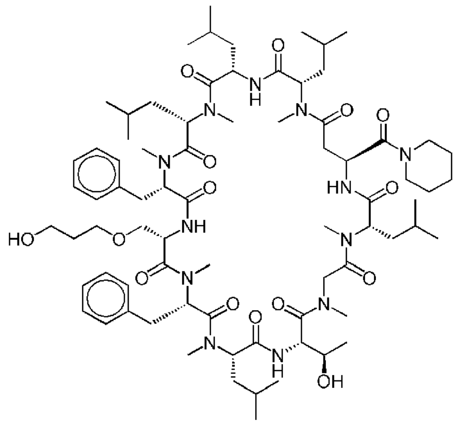 |
| AP95 | 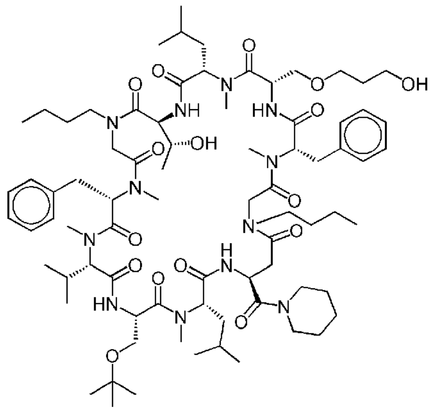 | AP96 | 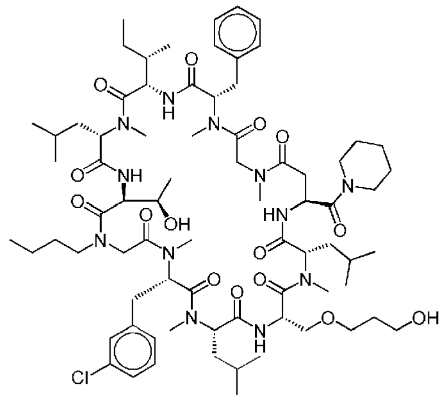 |

(continued)

| ID | Structural Formula | ID | Structural Formula |
|---|---|---|---|
| AP97 | 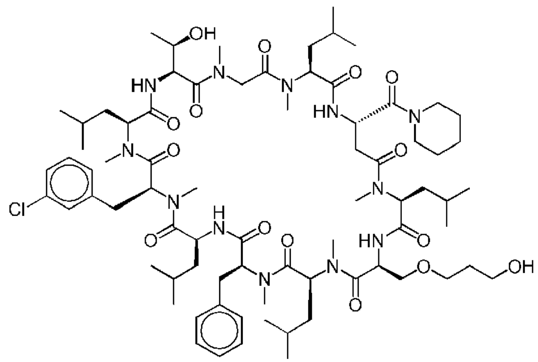 | AP98 | 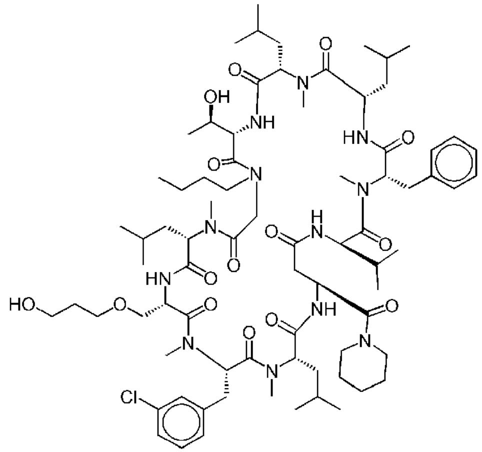 |
| AP99 | 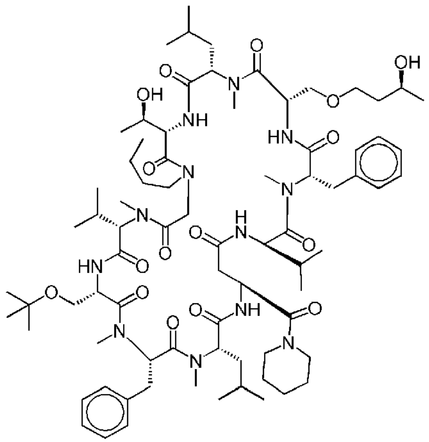 | AP100 | 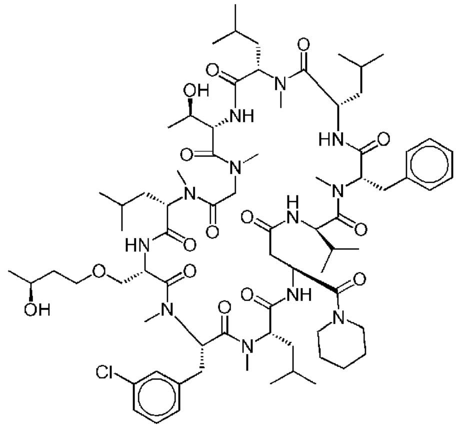 |

(continued)

| ID | Structural Formula | ID | Structural Formula |
|---|---|---|---|
| AP101 | 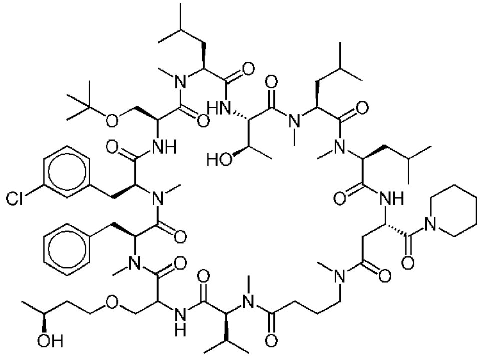 | AP102 | 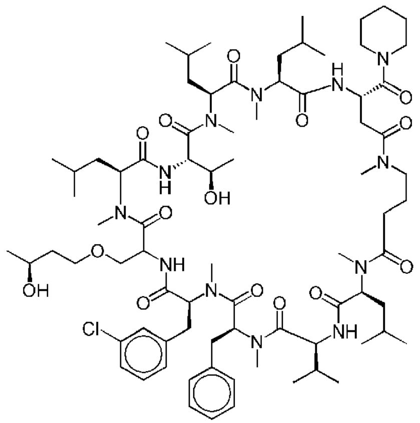 |
| AP103 | 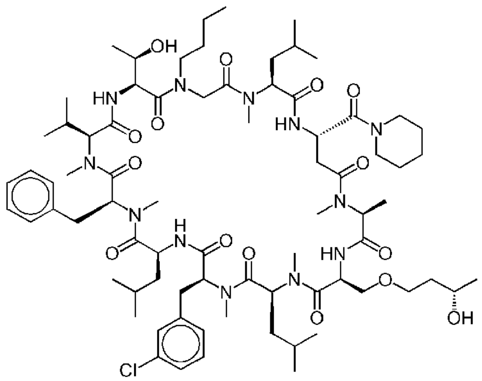 | AP104 | 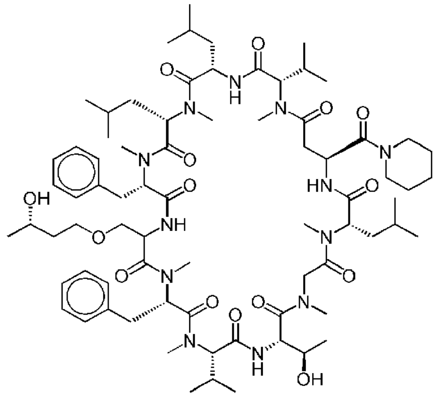 |

(continued)

| ID | Structural Formula | ID | Structural Formula |
|---|---|---|---|
| AP105 | 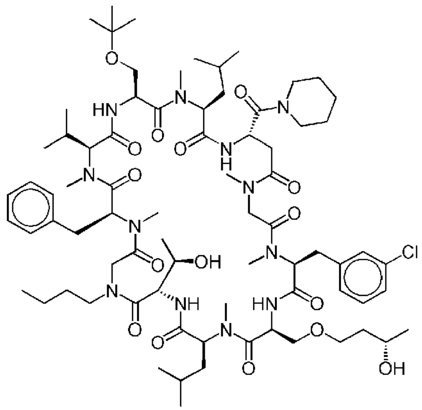 | AP106 | 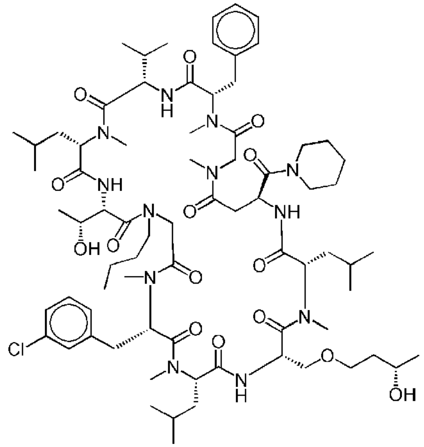 |
| AP107 | 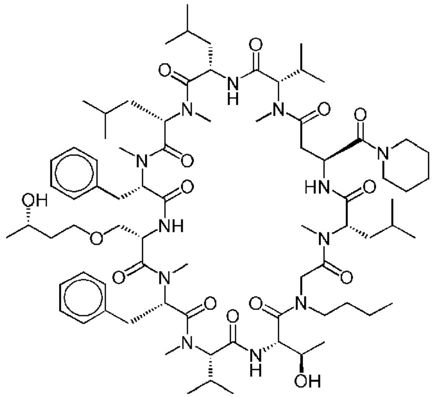 | AP108 | 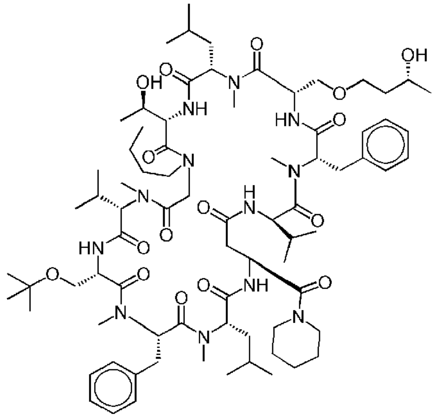 |

(continued)

| ID | Structural Formula | ID | Structural Formula |
|---|---|---|---|
| AP109 | 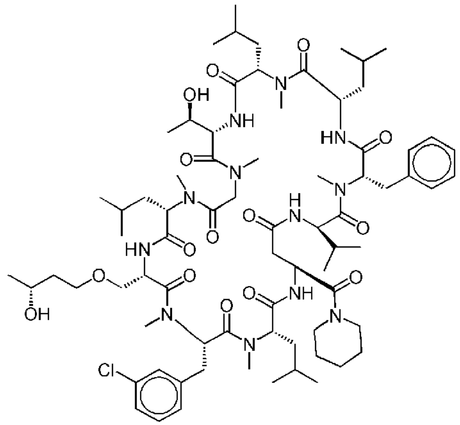 | AP110 | 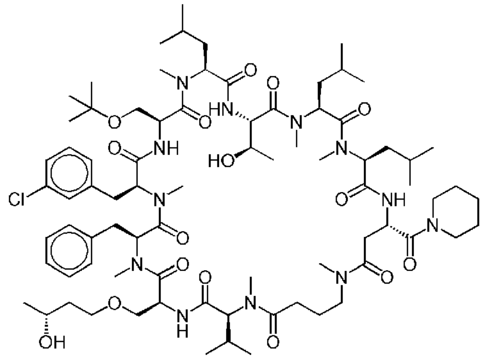 |
| AP111 | 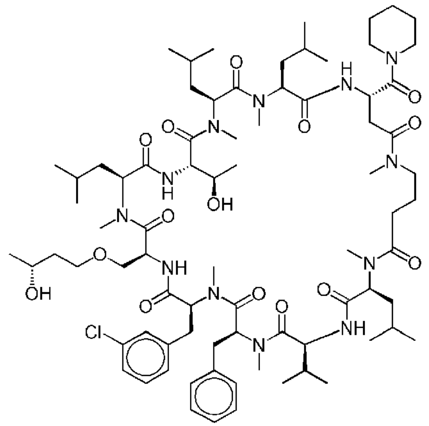 | AP112 | 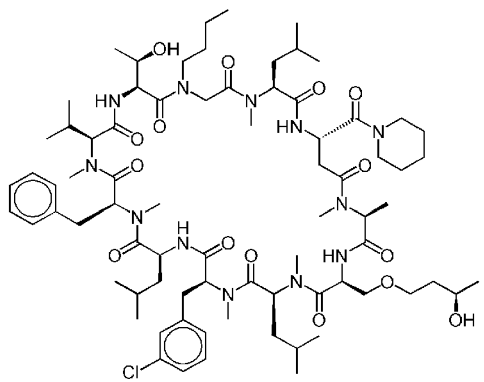 |

(continued)

| ID | Structural Formula | ID | Structural Formula |
|---|---|---|---|
| AP113 | 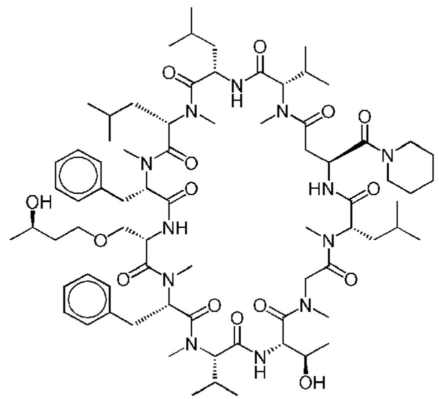 | AP114 | 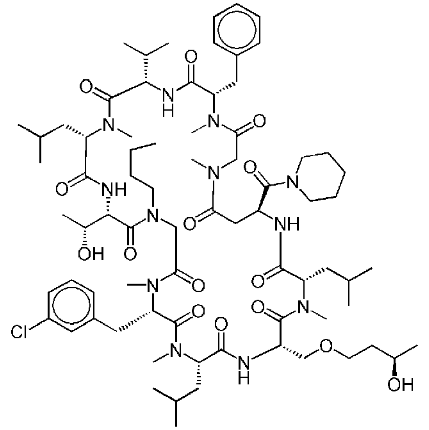 |
| AP115 | 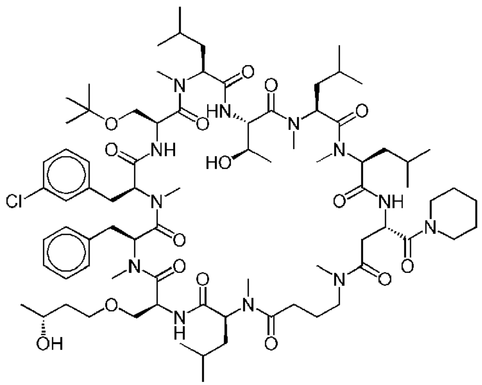 | AP116 | 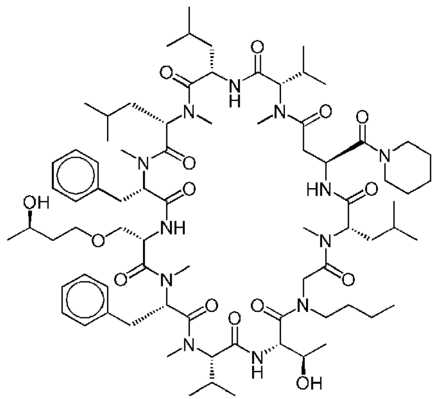 |

(continued)

| ID | Structural Formula | ID | Structural Formula |
|---|---|---|---|
| AP117 | 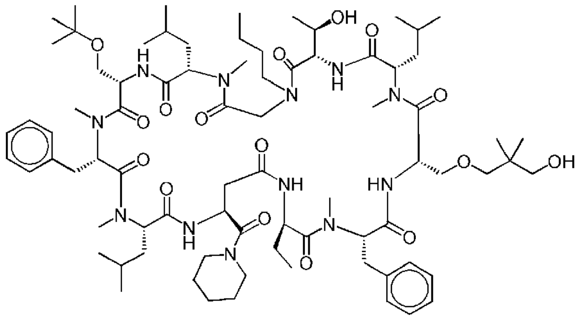 | AP118 | 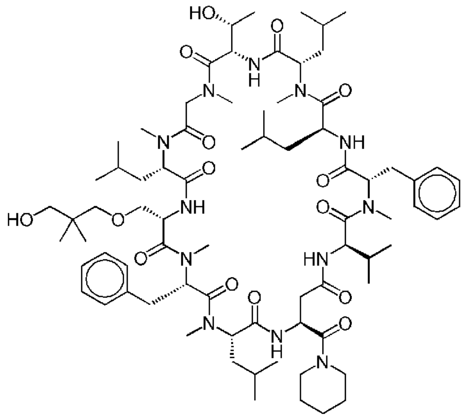 |
| AP119 | 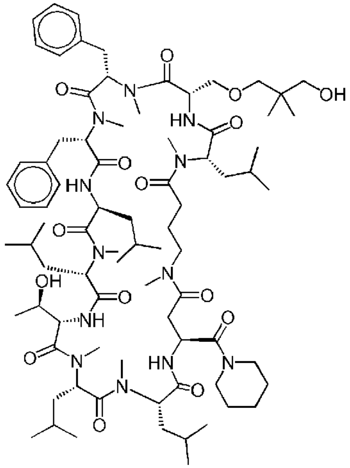 | AP120 | 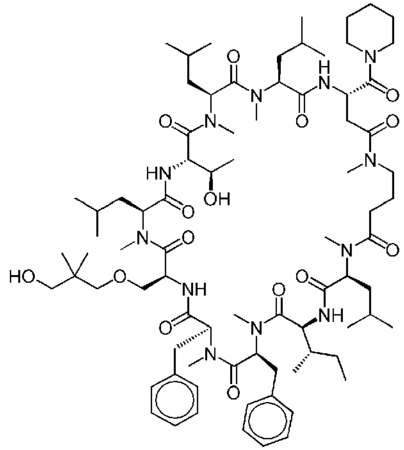 |

(continued)

| ID | Structural Formula | ID | Structural Formula |
|---|---|---|---|
| AP121 | 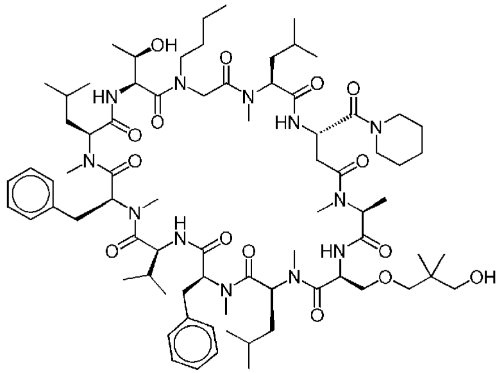 | AP122 | 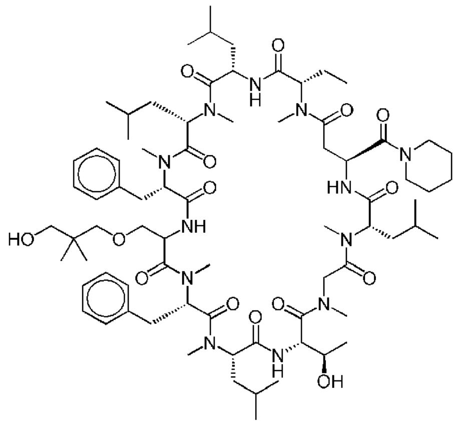 |
| AP123 | 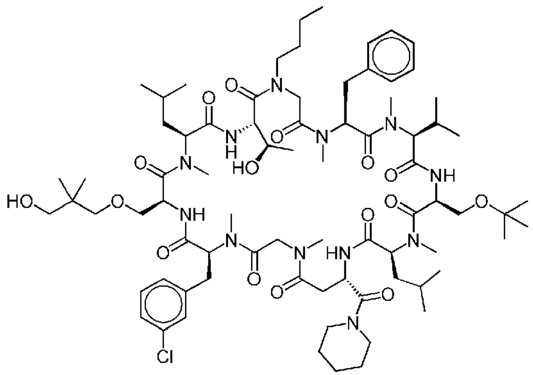 | AP124 | 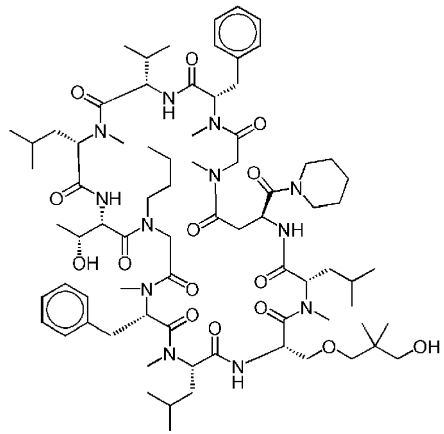 |

(continued)

| ID | Structural Formula | ID | Structural Formula |
|---|---|---|---|
| AP125 | 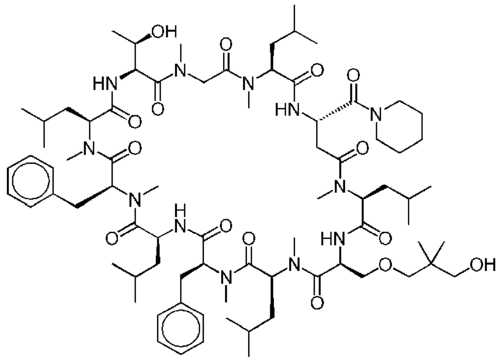 | AP126 | 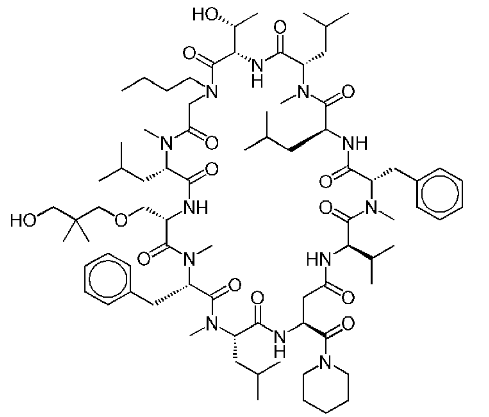 |
| AP127 | 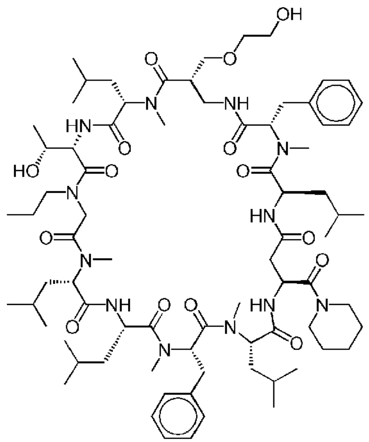 | AP128 | 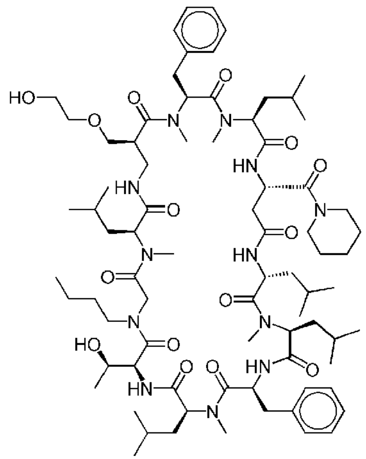 |

(continued)

| ID | Structural Formula | ID | Structural Formula |
|---|---|---|---|
| AP129 | 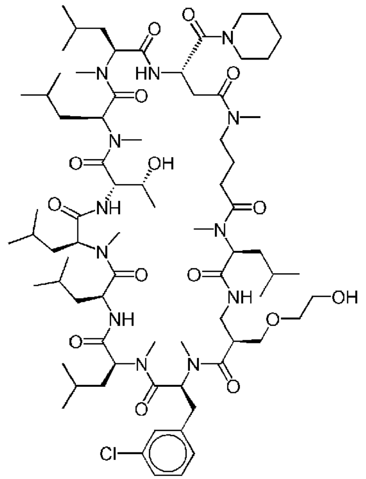 | AP130 | 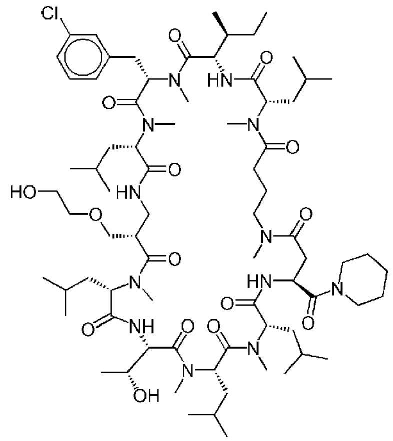 |
| AP131 | 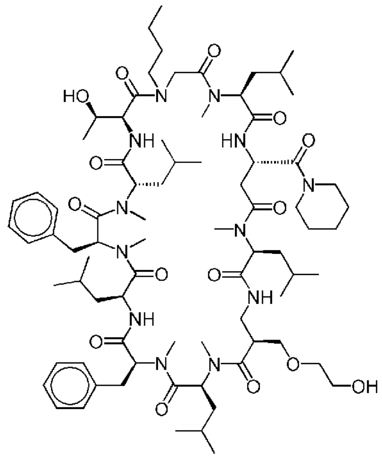 | AP132 | 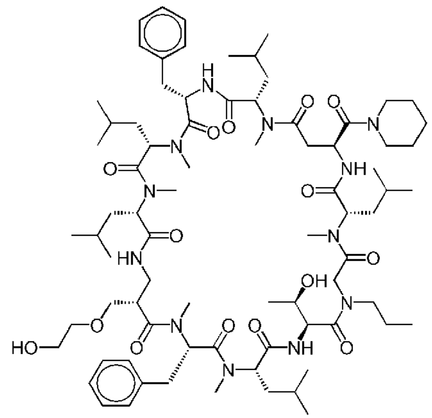 |

(continued)

| ID | Structural Formula | ID | Structural Formula |
|---|---|---|---|
| AP133 | 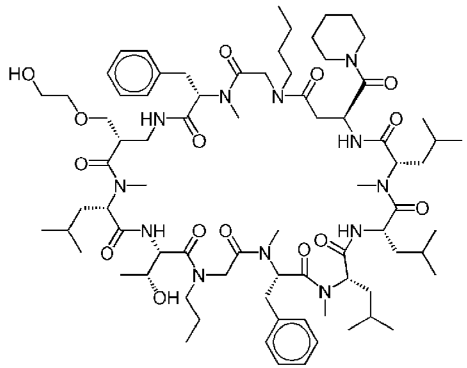 | AP134 | 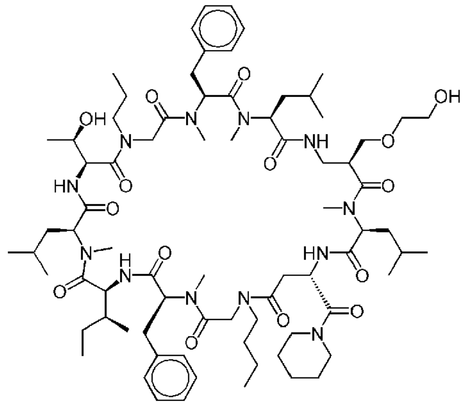 |
| AP135 | 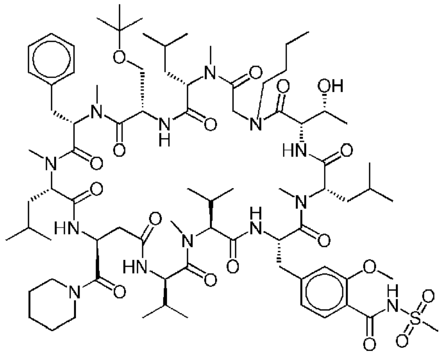 | AP136 | 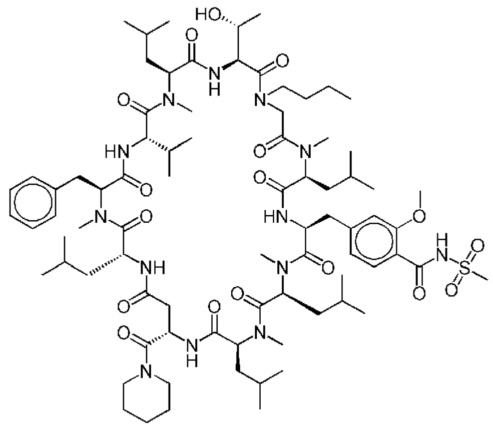 |

(continued)

| ID | Structural Formula | ID | Structural Formula |
|---|---|---|---|
| AP137 | 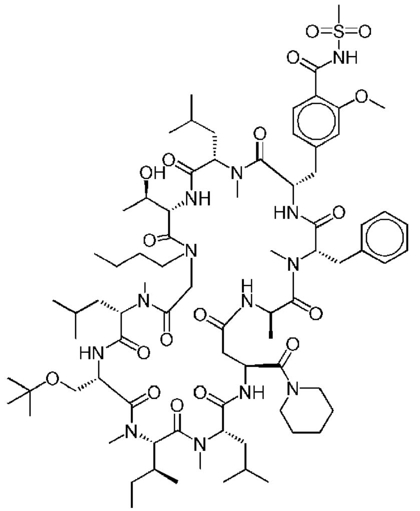 | AP138 | 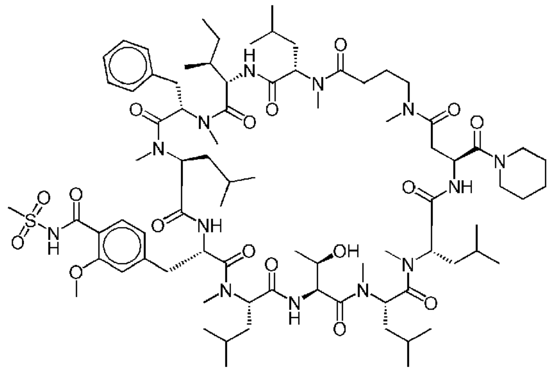 |
| AP139 | 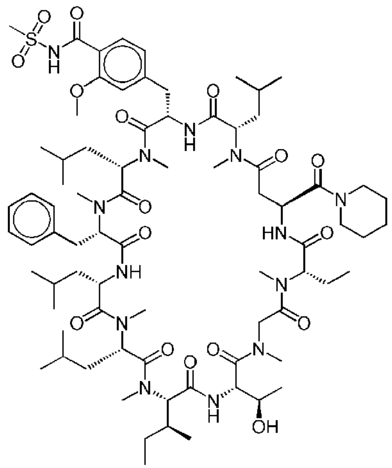 | AP140 | 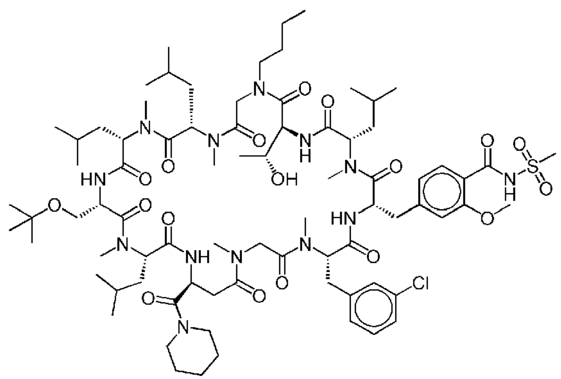 |

(continued)

| ID | Structural Formula | ID | Structural Formula |
|---|---|---|---|
| AP141 | 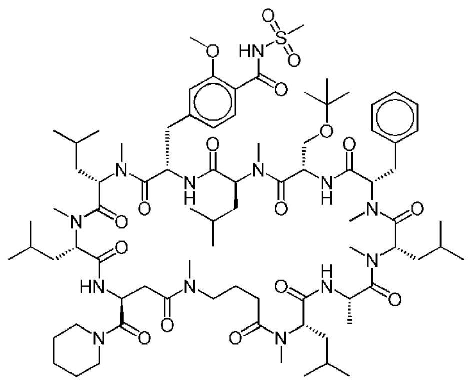 | AP142 | 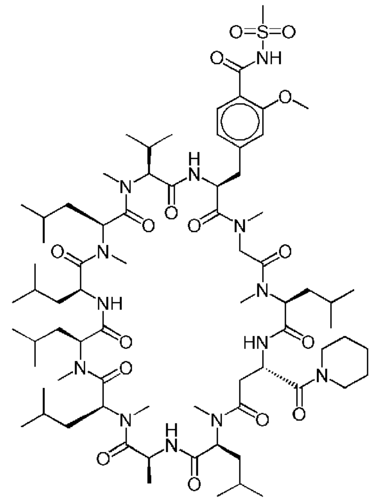 |
| AP143 | 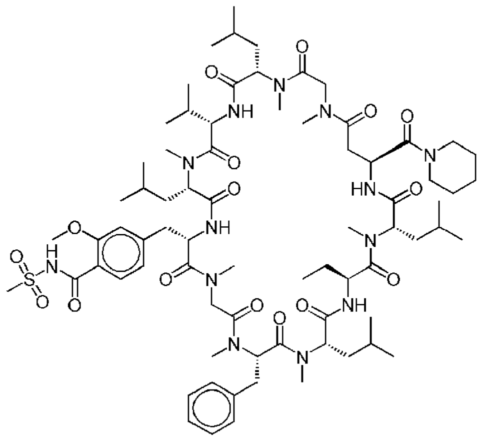 | | |

(continued)

| ID | Structural Formula | ID | Structural Formula |
|---|---|---|---|
| AP200 | 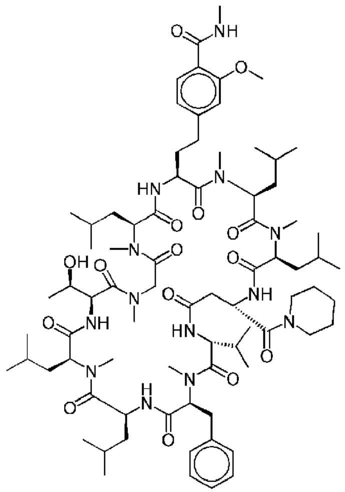 | AP201 | 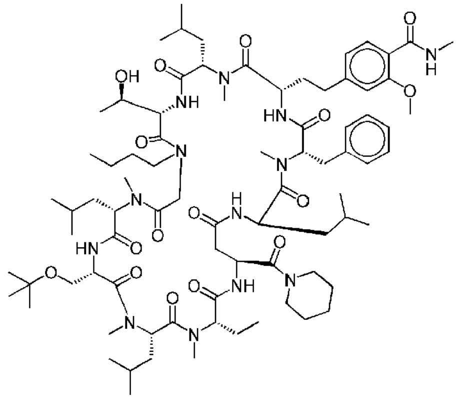 |
| AP202 | 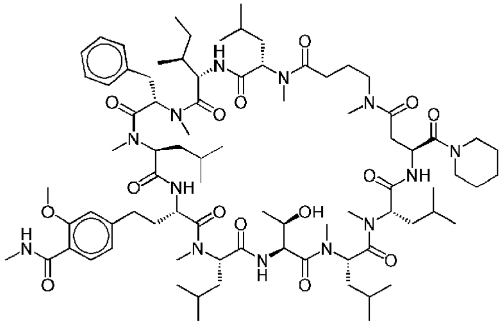 | AP203 | 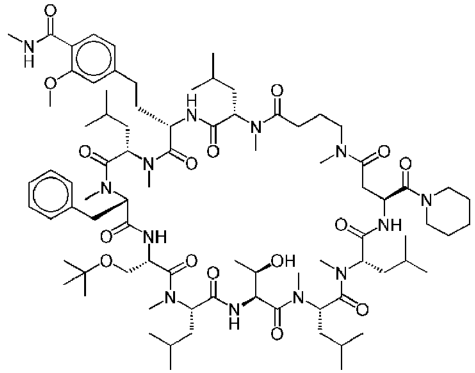 |

(continued)

| ID | Structural Formula | ID | Structural Formula |
|---|---|---|---|
| AP204 | 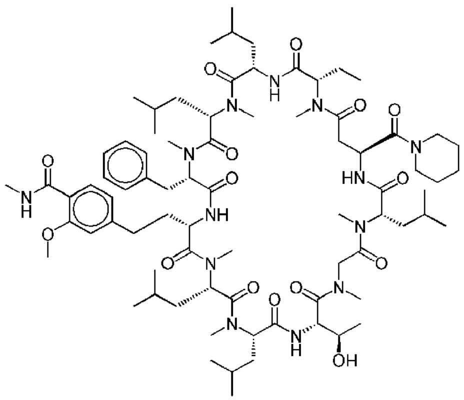 | AP205 | 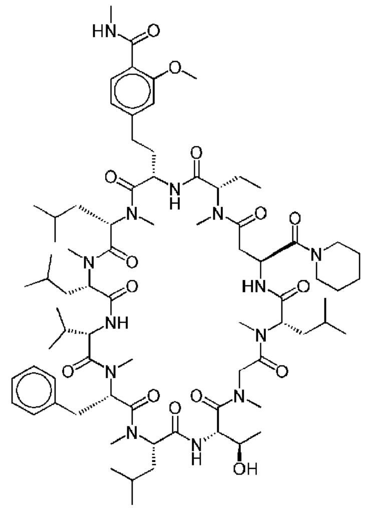 |
| AP206 | 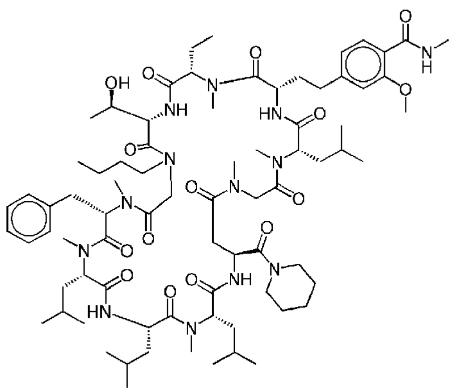 | AP207 | 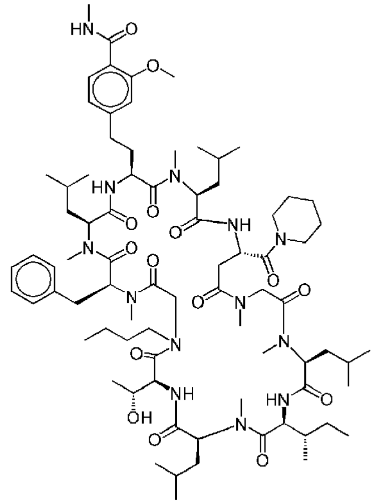 |

(continued)

| ID | Structural Formula | ID | Structural Formula |
|---|---|---|---|
| AP208 | 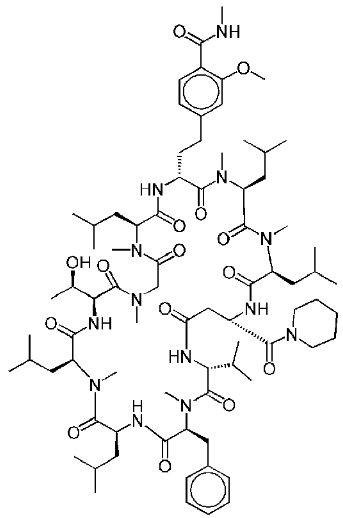 | AP209 | 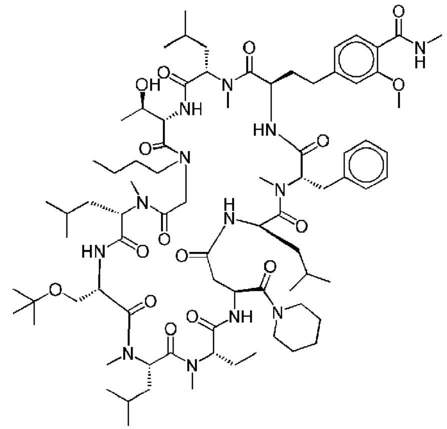 |
| AP210 | 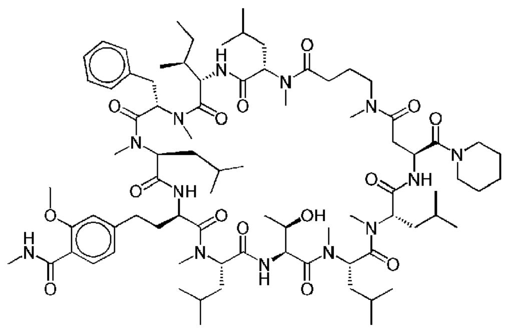 | AP211 | 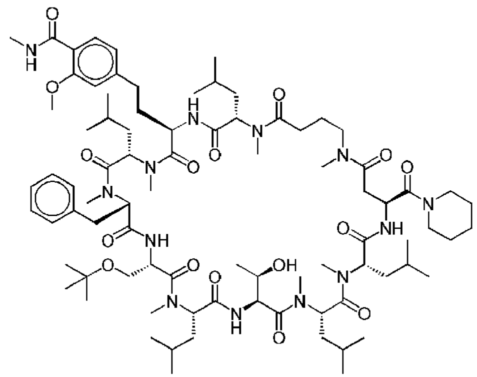 |

(continued)

| ID | Structural Formula | ID | Structural Formula |
|---|---|---|---|
| AP212 | 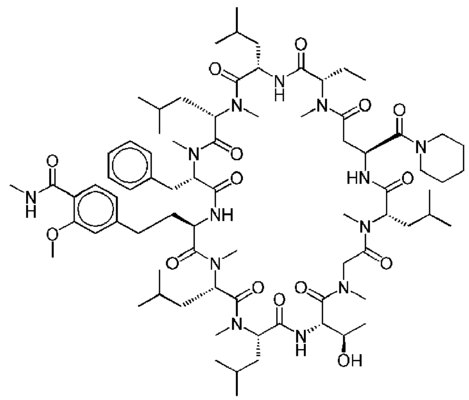 | AP213 | 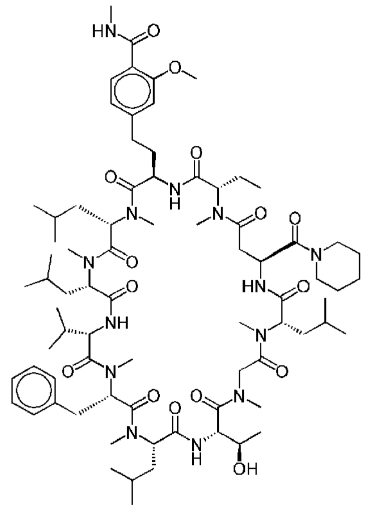 |
| AP214 | 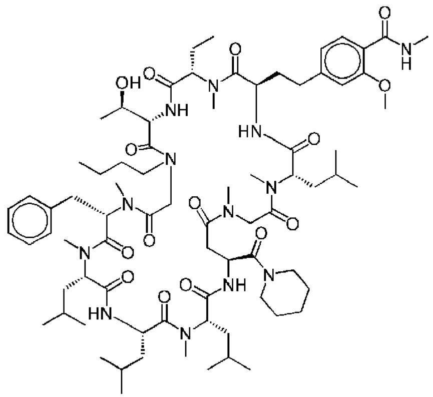 | AP215 | 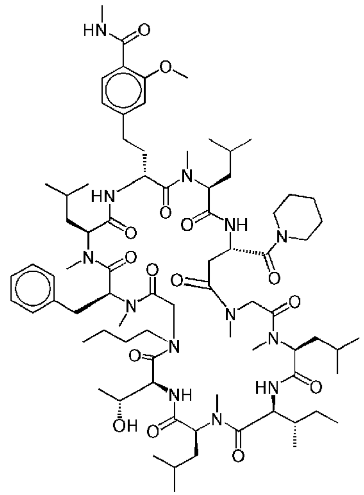 |

(continued)

| ID | Structural Formula | ID | Structural Formula |
|---|---|---|---|
| AP216 | 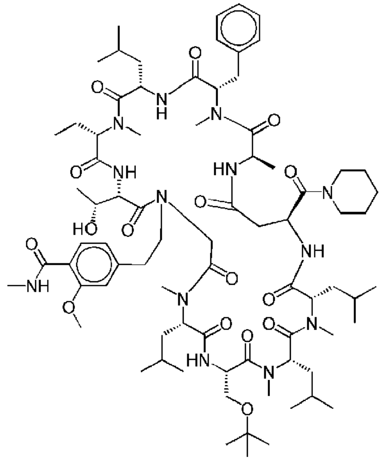 | AP217 | 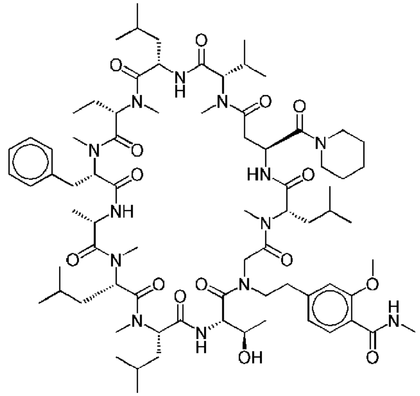 |
| AP218 | 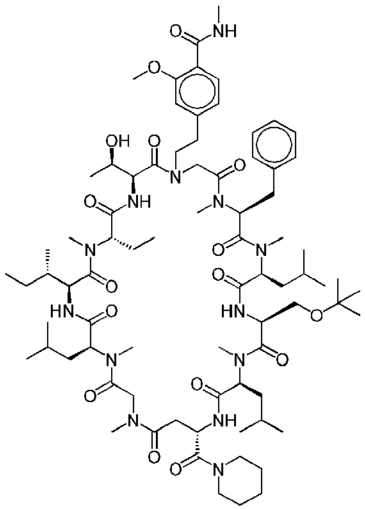 | AP219 | 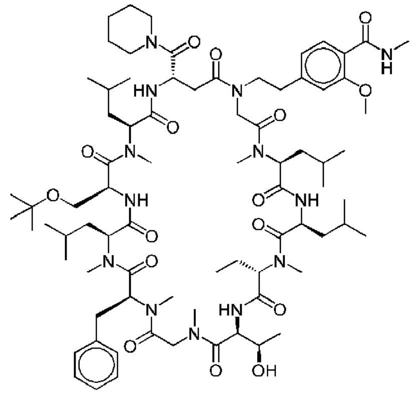 |

(continued)

| ID | Structural Formula | ID | Structural Formula |
|---|---|---|---|
| AP220 | 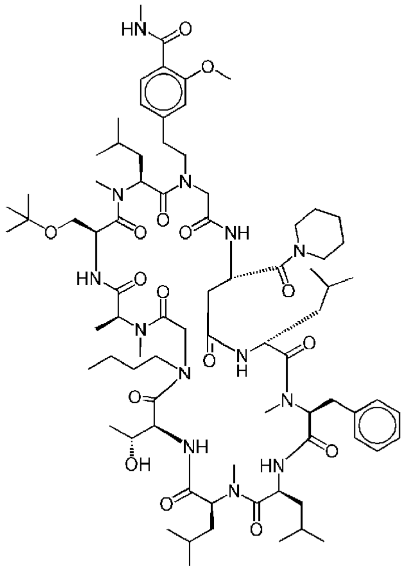 | AP221 | 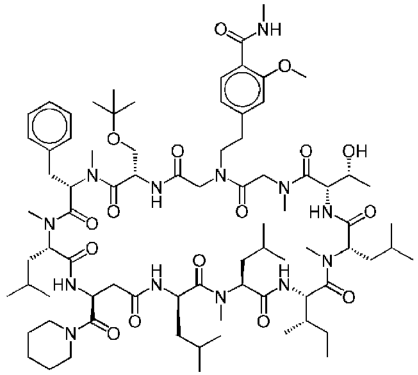 |
| AP222 | 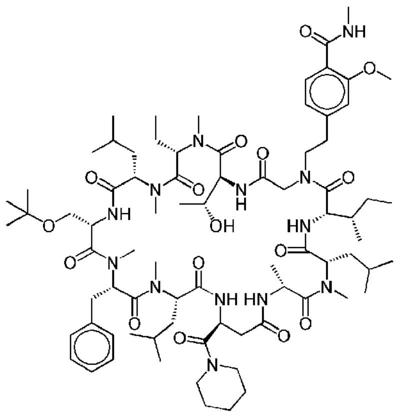 | AP223 | 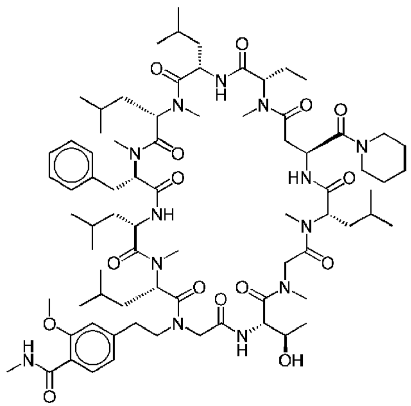 |

(continued)

| ID | Structural Formula | ID | Structural Formula |
|---|---|---|---|
| AP224 | 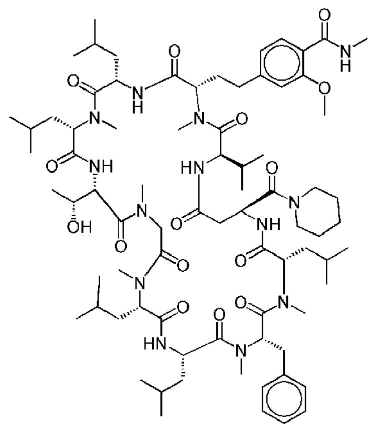 | AP225 | 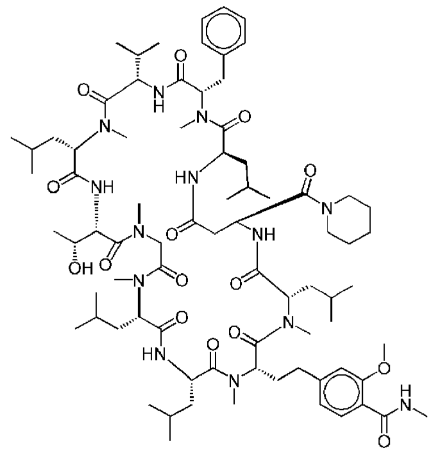 |
| AP226 | 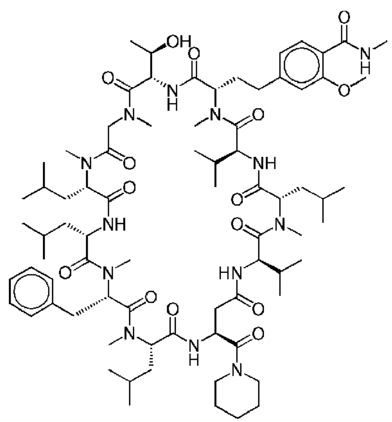 | AP227 | 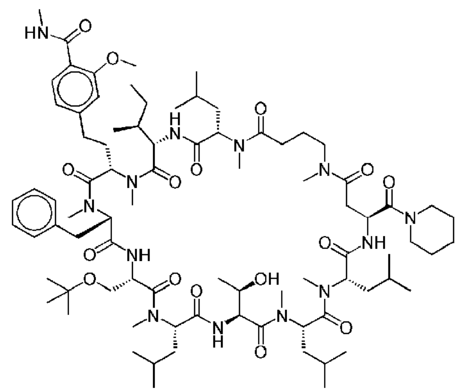 |

(continued)

| ID | Structural Formula | ID | Structural Formula |
|---|---|---|---|
| AP228 | 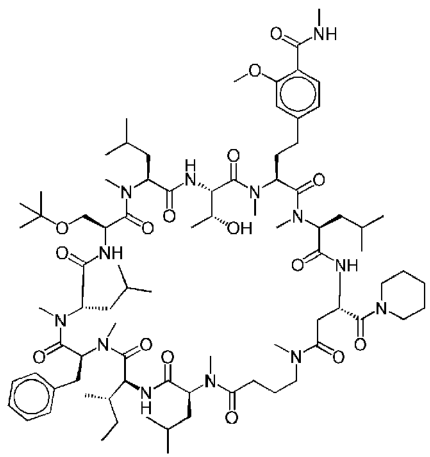 | AP229 | 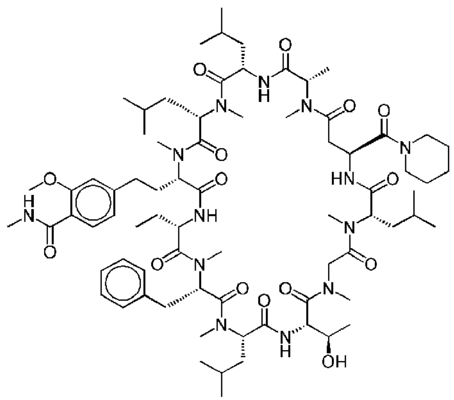 |
| AP230 | 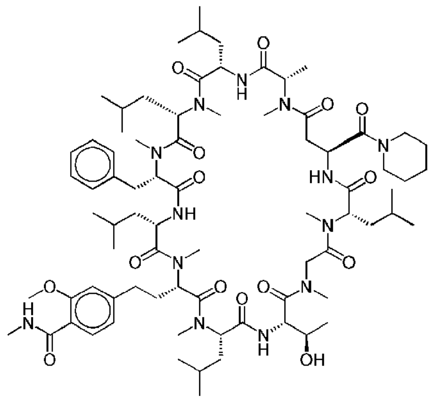 | AP231 | 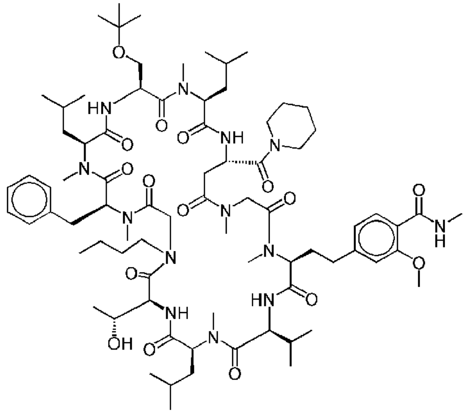 |

(continued)

| ID | Structural Formula | ID | Structural Formula |
|---|---|---|---|
| AP232 | 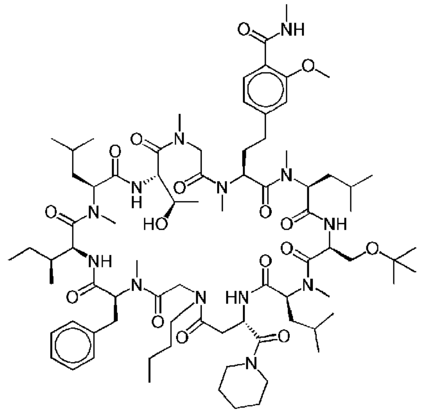 | AP233 | 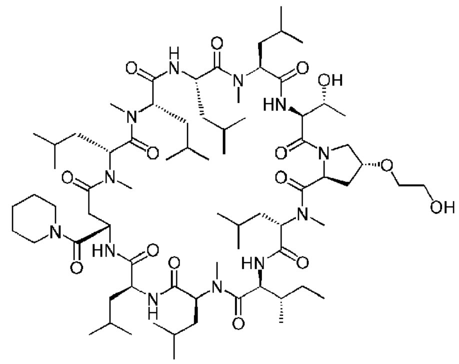 |
| AP234 | 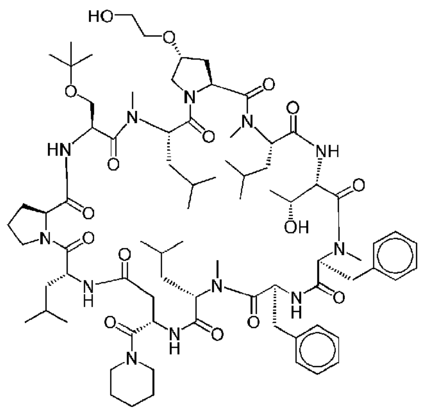 | AP235 | 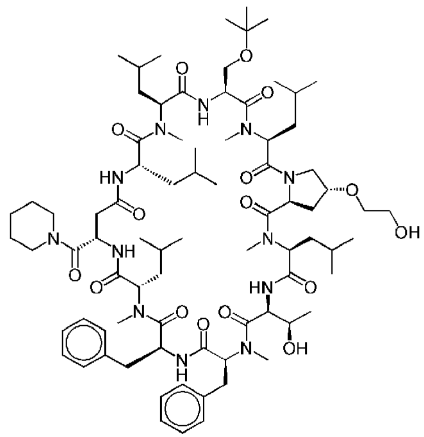 |

(continued)

| ID | Structural Formula | ID | Structural Formula |
|---|---|---|---|
| AP236 | 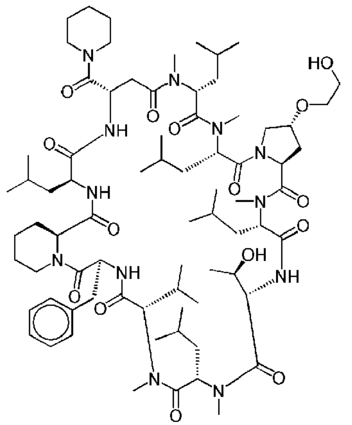 | AP237 | 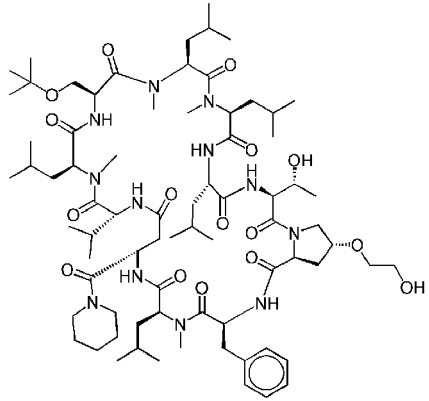 |
| AP238 | 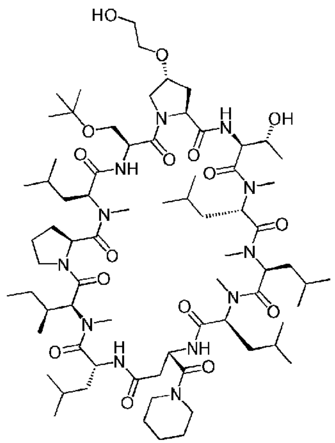 | AP239 | 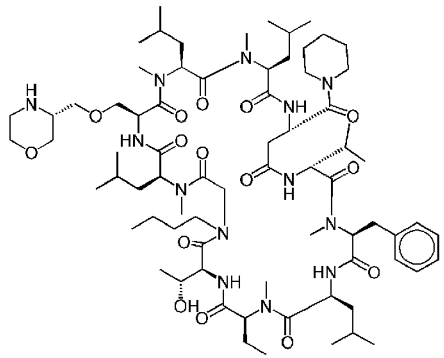 |

(continued)

| ID | Structural Formula | ID | Structural Formula |
|---|---|---|---|
| AP240 | 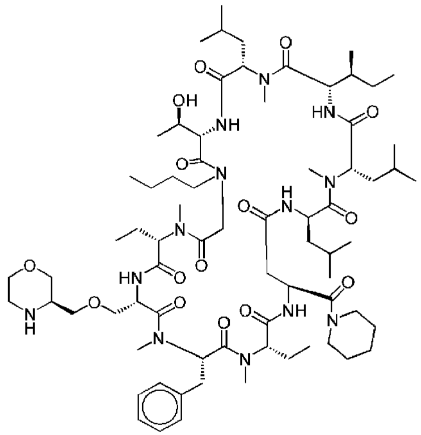 | AP241 | 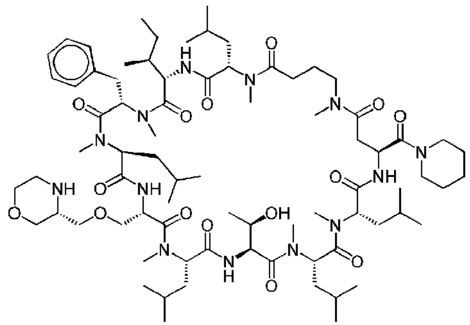 |
| AP242 | 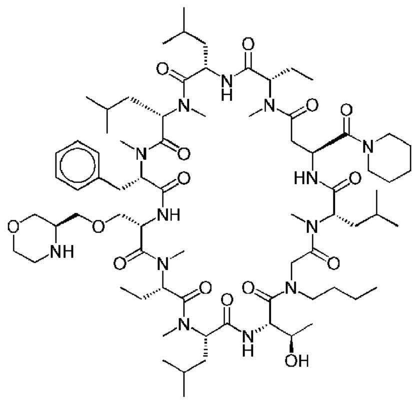 | AP243 | 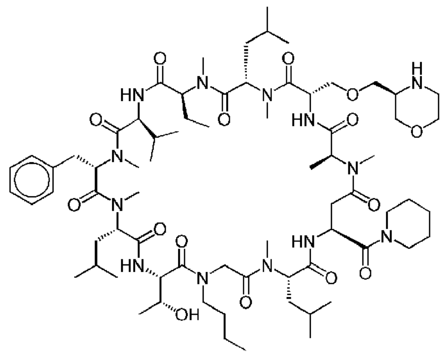 |

(continued)

| ID | Structural Formula | ID | Structural Formula |
|---|---|---|---|
| AP244 | 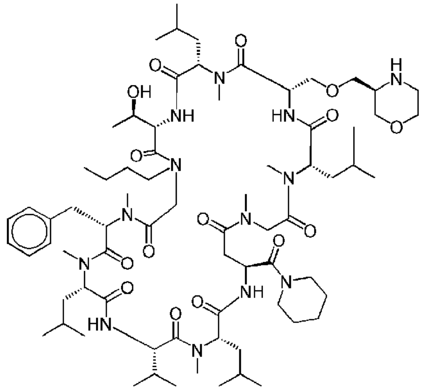 | AP245 | 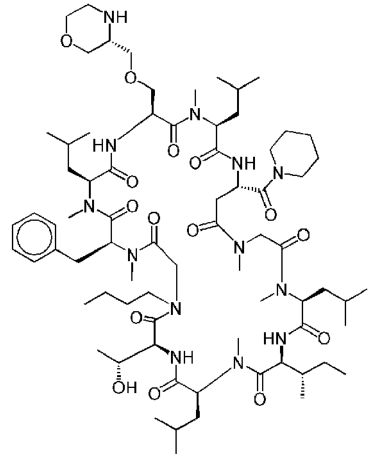 |
| AP246 | 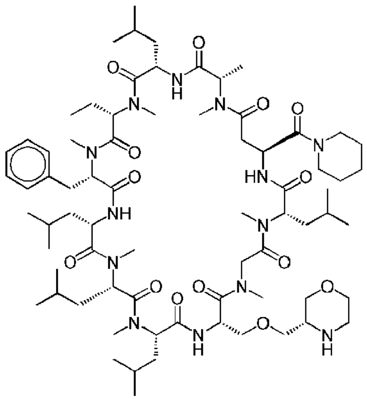 | AP247 | 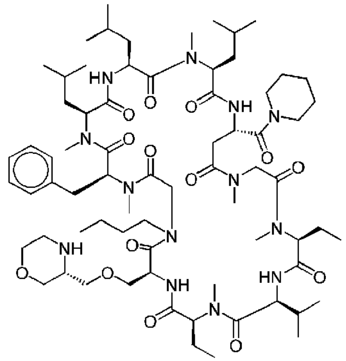 |

(continued)

| ID | Structural Formula | ID | Structural Formula |
|---|---|---|---|
| AP248 | 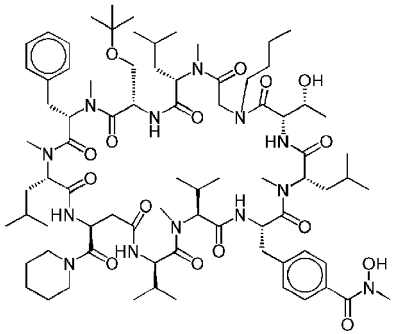 | AP249 | 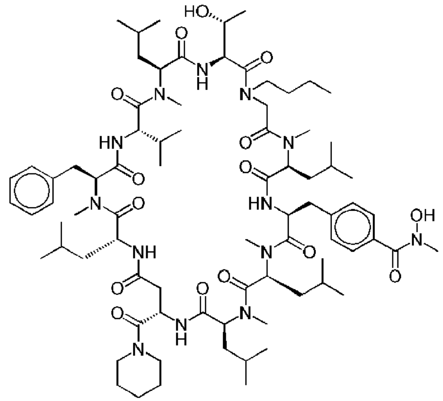 |
| AP250 | 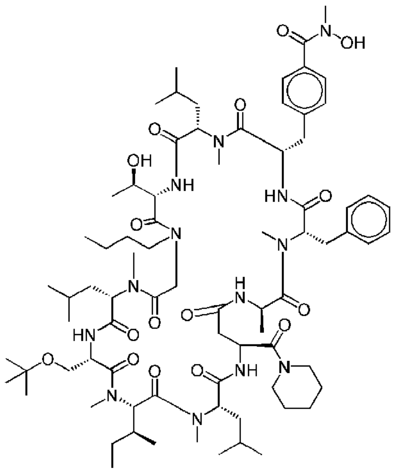 | AP251 | 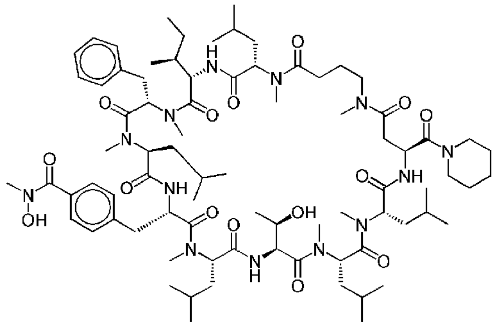 |

(continued)

| ID | Structural Formula | ID | Structural Formula |
|---|---|---|---|
| AP252 | 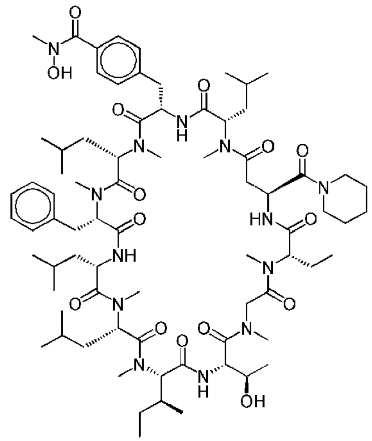 | AP253 | 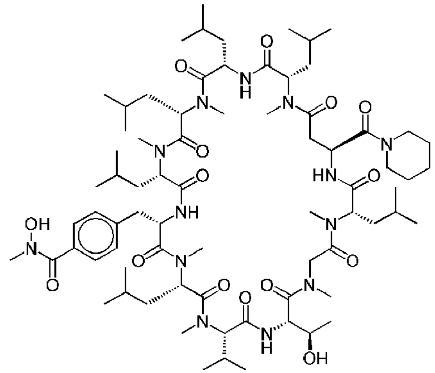 |
| AP254 | 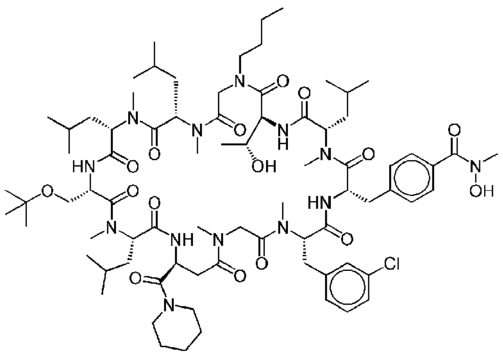 | AP255 | 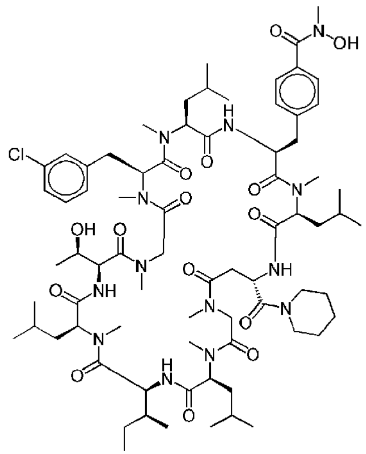 |

(continued)

| ID | Structural Formula | ID | Structural Formula |
|---|---|---|---|
| AP256 | 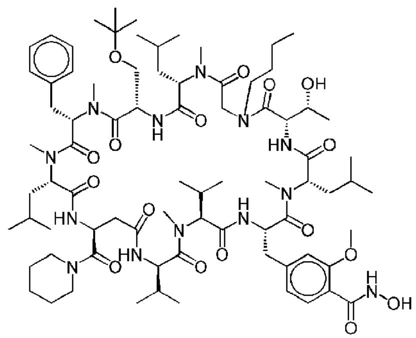 | AP257 | 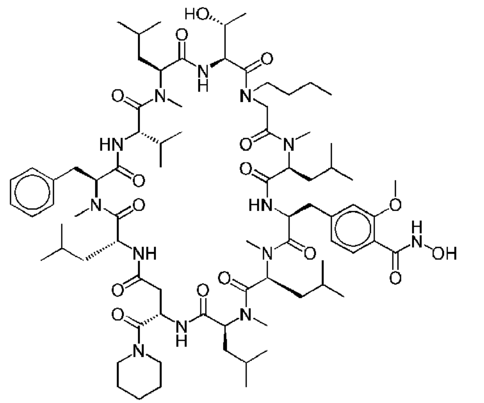 |
| AP258 | 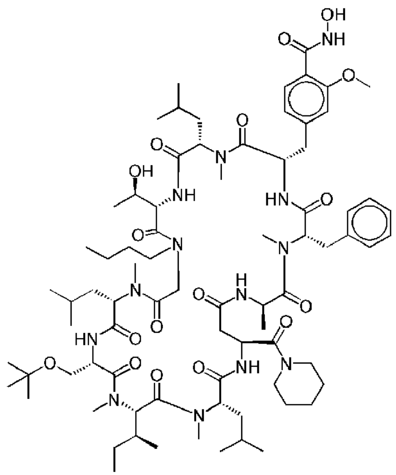 | AP259 | 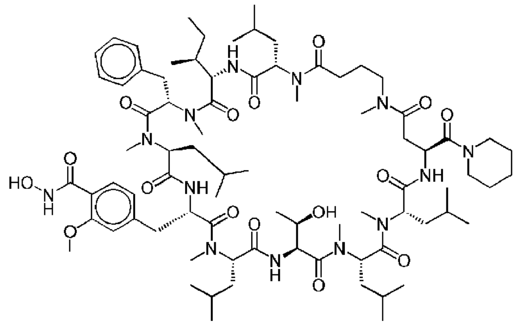 |

(continued)

| ID | Structural Formula | ID | Structural Formula |
|---|---|---|---|
| AP260 | 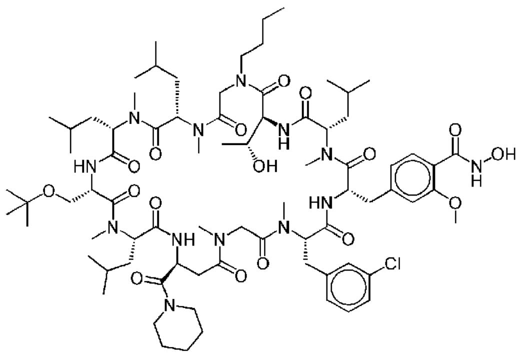 | AP261 | 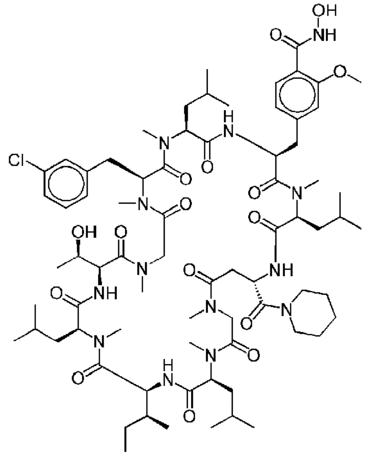 |
| AP262 | 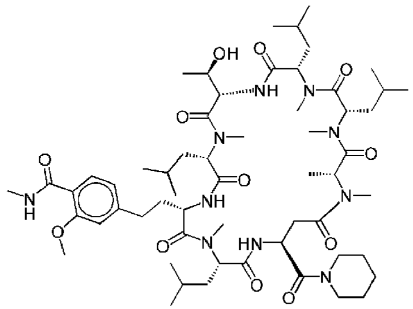 | AP263 | 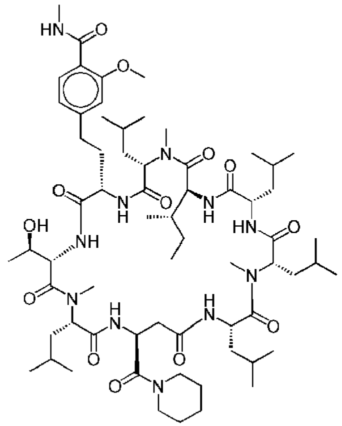 |

(continued)

| ID | Structural Formula | ID | Structural Formula |
|---|---|---|---|
| AP264 | 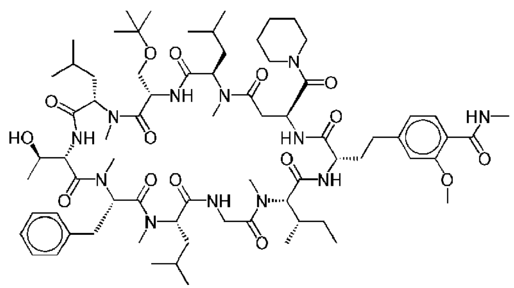 | AP265 | 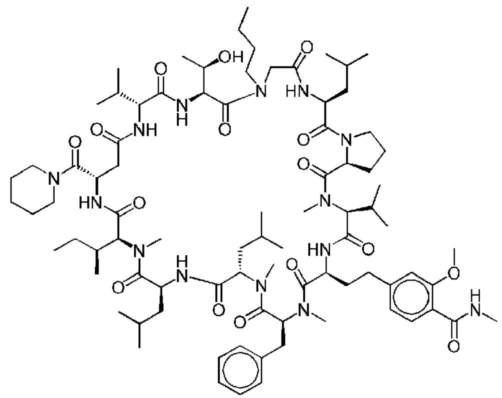 |
| AP266 | 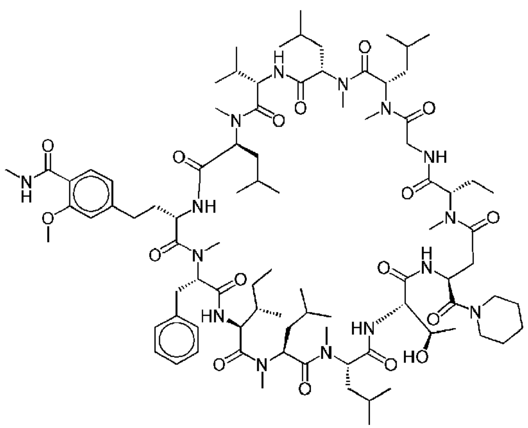 | | |

Analysis information of cyclic peptides, for which membrane permeability was evaluated in the above Example, is shown below.

[Table 16]

| Compound ID | Analytical Condition | LCMS(ESI) m/z | | Retention Time (min) |
|---|---|---|---|---|
| A01 | SQDFA40 | 1488 | (M+H)+ | 1.10 |
| A02 | SQDFA40 | 1450 | (M+H)+ | 0.90 |
| A03 | SQDFA40 | 1486 | (M+H)+ | 1.00 |
| A04 | SQDFA40 | 1486 | (M+H)+ | 0.98 |
| A05 | SQDFA40 | 1464 | (M+H)+ | 1.00 |
| A06 | SQDFA40 | 1430 | (M+H)+ | 0.88 |
| A07 | SQDFA40 | 1494 | (M+H)+ | 0.99 |
| A08 | SQDFA40 | 1450 | (M+H)+ | 0.91 |
| A09 | SQDFA40 | 1464 | (M+H)+ | 0.97 |
| A10 | SQDFA40 | 1464 | (M+H)+ | 0.99 |
| B01 | SQDFA05 | 1472 | (M+H)+ | 1.16 |
| B02 | SQDFA05 | 1434 | (M+H)+ | 1.02 |
| B03 | SQDFA05 | 1470 | (M+H)+ | 1.13 |
| B04 | SQDFA05 | 1470 | (M+H)+ | 1.11 |
| B05 | SQDFA05 | 1448 | (M+H)+ | 1.11 |
| B06 | SQDFA05 | 1414 | (M+H)+ | 1.01 |
| B07 | SQDFA05 | 1478 | (M+H)+ | 1.11 |
| B08 | SQDFA05 | 1434 | (M+H)+ | 1.03 |
| B09 | SQDFA05 | 1448 | (M+H)+ | 1.08 |
| B10 | SQDFA05 | 1448 | (M+H)+ | 1.08 |
| C01 | SQD2FA05 | 1542 | (M+H)+ | 1.23 |
| C02 | SQD2FA05 | 1590 | (M+H)+ | 1.17 |
| C03 | SQD2FA05 | 1526 | (M+H)+ | 1.12 |
| C04 | SQD2FA05 | 1498 | (M+H)+ | 1.14 |
| C05 | SQD2FA05 | 1484 | (M+H)+ | 1.02, 1.11 |
| C06 | SQD2FA05 | 1548 | (M+H)+ | 1.15 |
| C07 | SQD2FA05 | 1484 | (M+H)+ | 1.13 |
| C08 | SQD2FA05 | 1484 | (M+H)+ | 1.12 |
| C09 | SQD2FA05 | 1498 | (M+H)+ | 1.16 |
| C10 | SQD2FA05 | 1470 | (M+H)+ | 1.08 |
| C11 | SQD2FA05 | 1498 | (M+H)+ | 1.18 |
| C12 | SQD2FA05 | 1554 | (M+H)+ | 1.17 |
| C13 | SQD2FA05 | 1540 | (M+H)+ | 1.16 |
| C14 | SQD2FA05 | 1426 | (M+H)+ | 1.03, 1.08 |
| C15 | SQD2FA05 | 1454 | (M+H)+ | 1.10 |
| C16 | SQD2FA05 | 1484 | (M+H)+ | 1.15 |

(continued)

| Compound ID | Analytical Condition | LCMS(ESI) m/z | | Retention Time (min) |
|---|---|---|---|---|
| C17 | SQD2FA05 | 1484 | (M+H)+ | 1.10 |
| D01 | SQD2FA05 | 1540 | (M+H)+ | 1.20, 1.23 |
| D02 | SQD2FA05 | 1588 | (M+H)+ | 1.15, 1.19 |
| D03 | SQD2FA05 | 1524 | (M+H)+ | 1.06, 1.13 |
| D04 | SQD2FA05 | 1496 | (M+H)+ | 1.13, 1.15 |
| D05 | SQD2FA05 | 1482 | (M+H)+ | 1.03, 1.11 |
| D06 | SQD2FA05 | 1546 | (M+H)+ | 1.16, 1.23 |
| D07 | SQD2FA05 | 1482 | (M+H)+ | 1.06, 1.13 |
| D08 | SQD2FA05 | 1482 | (M+H)+ | 1.06, 1.12 |
| D09 | SQD2FA05 | 1496 | (M+H)+ | 1.17 |
| D10 | SQD2FA05 | 1468 | (M+H)+ | 1.09 |
| D11 | SQD2FA05 | 1496 | (M+H)+ | 1.17, 1.18 |
| D12 | SQD2FA05 | 1552 | (M+H)+ | 1.17 |
| D13 | SQD2FA05 | 1538 | (M+H)+ | 1.18 |
| D14 | SQD2FA05 | 1424 | (M+H)+ | 1.04, 1.07 |
| D15 | SQD2FA05 | 1452 | (M+H)+ | 1.10 |
| D16 | SQD2FA05 | 1482 | (M+H)+ | 1.12 |
| D17 | SQD2FA05 | 1482 | (M+H)+ | 1.11, 1.16 |
| E01 | SQD2FA05 | 1430 | (M+H)+ | 1.06 |
| E02 | SQD2FA05 | 1488 | (M+H)+ | 1.14 |
| E03 | SQD2AA50 | 1486 | (M+H)+ | 0.81 |
| E04 | SQD2FA05 | 1516 | (M+H)+ | 1.15 |
| E05 | SQD2FA50 | 1430 | (M+H)+ | 0.65 |
| E06 | SQD2AA50 | 1416 | (M+H)+ | 0.79 |
| E07 | SQD2AA50 | 1416 | (M+H)+ | 0.80 |
| E08 | SQD2AA50 | 1444 | (M+H)+ | 0.84 |
| F01 | SQD2FA05 | 1400 | (M+H)+ | 1.03 |
| F02 | SQD2FA50L | 1458 | (M+H)+ | 2.25 |
| F03 | SQD2FA50L | 1456 | (M+H)+ | 1.75 |
| F04 | SQD2FA05 | 1486 | (M+H)+ | 1.13 |
| F05 | SQD2FA50L | 1400 | (M+H)+ | 1.52 |
| F06 | SQD2FA05 | 1386 | (M+H)+ | 1.01 |
| F07 | SQD2FA05 | 1386 | (M+H)+ | 1.04 |
| F08 | SQD2FA05 | 1414 | (M+H)+ | 1.10 |
| G01 | SQDFA05 | 1457 | (M+H)+ | 0.88 |
| G02 | SQDFA05 | 1429 | (M+H)+ | 0.81 |
| G03 | SQDFA05 | 1499 | (M+H)+ | 0.88 |
| G04 | SQDFA05 | 1457 | (M+H)+ | 0.86 |

(continued)

| Compound ID | Analytical Condition | LCMS(ESI) m/z | | Retention Time (min) |
|---|---|---|---|---|
| G05 | SQDFA05 | 1429 | (M+H)+ | 0.82 |
| G06 | SQDFA05 | 1443 | (M+H)+ | 0.84 |
| G07 | SQDFA05 | 1457 | (M+H)+ | 0.88 |
| G08 | SQDFA05 | 1429 | (M+H)+ | 0.78 |
| G09 | SQDFA05 | 1499 | (M+H)+ | 0.85 |
| G10 | SQDFA05 | 1413 | (M+H)+ | 0.78 |
| G11 | SQDFA05 | 1399 | (M+H)+ | 0.79 |
| H01 | SQD2FA05 | 1439 | (M-H)- | 0.83 |
| H02 | SQD2FA05 | 1411 | (M-H)- | 0.78 |
| H03 | SQD2FA05 | 1483 | (M+H)+ | 0.86 |
| H04 | SQD2FA05 | 1441 | (M+H)+ | 0.81 |
| H05 | SQD2FA05 | 1413 | (M+H)+ | 0.78 |
| H06 | SQD2FA05 | 1427 | (M+H)+ | 0.80 |
| H07 | SQD2FA05 | 1441 | (M+H)+ | 0.84 |
| H08 | SQD2FA05 | 1413 | (M+H)+ | 0.75 |
| H09 | SQD2FA05 | 1483 | (M+H)+ | 0.83 |
| H10 | SQD2FA05 | 1397 | (M+H)+ | 0.76 |
| H11 | SQD2FA05 | 1383 | (M+H)+ | 0.75 |
| I01 | SQDFA40 | 1471 | (M+H)+ | 1.02 |
| 102 | SQDFA40 | 1499 | (M+H)+ | 1.09 |
| 103 | SQDFA40 | 1471 | (M+H)+ | 0.96 |
| 104 | SQDFA40 | 1499 | (M+H)+ | 1.07 |
| 105 | SQDFA40 | 1471 | (M+H)+ | 0.95 |
| 106 | SQDFA40 | 1471 | (M+H)+ | 1.00 |
| J01 | SQDFA50 | 1467 | (M-H)- | 0.86 |
| J02 | SQDFA50 | 1537 | (M+H)+ | 0.90 |
| J03 | SQDFA50 | 1469 | (M+H)+ | 0.83 |
| J04 | SQDFA50 | 1537 | (M+H)+ | 0.90 |
| J05 | SQDFA50 | 1469 | (M+H)+ | 0.84 |
| J06 | SQDFA50 | 1469 | (M+H)+ | 0.88 |
| AP01 | SQDFA50 | 1488 | (M+H)+ | 0.85 |
| AP02 | SQDFA50 | 1464 | (M+H)+ | 0.71 |
| AP03 | SQDFA50 | 1530 | (M+H)+ | 0.81 |
| AP04 | SQDFA50 | 1520 | (M+H)+ | 0.80 |
| AP05 | SQDFA50 | 1444 | (M+H)+ | 0.72 |
| AP06 | SQDFA50 | 1444 | (M+H)+ | 0.79 |
| AP07 | SQDFA50 | 1494 | (M+H)+ | 0.72 |
| AP08 | SQDFA50 | 1430 | (M+H)+ | 0.67 |

(continued)

| Compound ID | Analytical Condition | LCMS(ESI) m/z | | Retention Time (min) |
|---|---|---|---|---|
| AP09 | SQDFA50 | 1444 | (M+H)+ | 0.75 |
| AP10 | SQDFA50 | 1458 | (M+H)+ | 0.77 |
| AP11 | SQDFA40 | 1471 | (M+H)+ | 1.00 |
| AP12 | SQDFA40 | 1499 | (M+H)+ | 1.15 |
| AP13 | SQDFA40 | 1485 | (M+H)+ | 1.06 |
| AP14 | SQDFA40 | 1471 | (M+H)+ | 0.96 |
| AP15 | SQDFA40 | 1499 | (M+H)+ | 1.05 |
| AP16 | SQDFA40 | 1485 | (M+H)+ | 1.07 |
| AP17 | SQDFA40 | 1471 | (M+H)+ | 0.95 |
| AP18 | SQDFA40 | 1471 | (M+H)+ | 1.03 |
| AP19 | SQDFA40 | 1485 | (M+H)+ | 1.12 |
| AP20 | SQDFA05 | 1458 | (M+H)+ | 1.11 |
| AP21 | SQDFA05 | 1472 | (M+H)+ | 1.13 |
| AP22 | SQDFA05 | 1472 | (M+H)+ | 1.03 |
| AP23 | SQDFA05 | 1486 | (M+H)+ | 1.19 |
| AP24 | SQDFA05 | 1472 | (M+H)+ | 1.10 |
| AP25 | SQDFA05 | 1458 | (M+H)+ | 1.05 |
| AP26 | SQDFA05 | 1458 | (M+H)+ | 1.04 |
| AP27 | SQDFA05 | 1529 | (M+H)+ | 1.13 |
| AP28 | SQDFA05 | 1513 | (M+H)+ | 1.12 |
| AP29 | SQDFA05 | 1515 | (M+H)+ | 1.09 |
| AP30 | SQDFA05 | 1527 | (M+H)+ | 1.09 |
| AP31 | SQDFA05 | 1471 | (M+H)+ | 1.05 |
| AP32 | SQDFA05 | 1451 | (M+H)+ | 1.09 |
| AP33 | SQDFA05 | 1549 | (M+H)+ | 1.10 |
| AP34 | SQDFA05 | 1477 | (M+H)+ | 1.05 |
| AP35 | SQDFA05 | 1529 | (M+H)+ | 1.12 |
| AP36 | SQDFA05 | 1513 | (M+H)+ | 1.13 |
| AP37 | SQDFA05 | 1515 | (M+H)+ | 1.10 |
| AP38 | SQDFA05 | 1527 | (M+H)+ | 0.96 |
| AP39 | SQDFA05 | 1471 | (M+H)+ | 1.09 |
| AP40 | SQDFA05 | 1451 | (M+H)+ | 1.11 |
| AP41 | SQDFA05 | 1549 | (M+H)+ | 1.11 |
| AP42 | SQDFA05 | 1477 | (M+H)+ | 1.10 |
| AP43 | SQD2AA50 | 1464 | (M+H)+ | 0.78 |
| AP44 | SQD2AA50 | 1478 | (M+H)+ | 0.79 |
| AP45 | SQD2AA50 | 1508 | (M+H)+ | 0.81 |
| AP46 | SQD2AA50 | 1512 | (M+H)+ | 0.77 |

(continued)

| Compound ID | Analytical Condition | LCMS(ESI) m/z | | Retention Time (min) |
|---|---|---|---|---|
| AP47 | SQD2AA50 | 1464 | (M+H)+ | 0.75 |
| AP48 | SQD2AA50 | 1478 | (M+H)+ | 0.77 |
| AP49 | SQD2AA50 | 1470 | (M+H)+ | 0.78 |
| AP50 | SQD2AA50 | 1470 | (M+H)+ | 0.77 |
| AP51 | SQDFA50 | 1488 | (M+H)+ | 0.79 |
| AP52 | SQDFA50 | 1464 | (M+H)+ | 0.68 |
| AP53 | SQDFA50 | 1530 | (M+H)+ | 0.78 |
| AP54 | SQDFA50 | 1520 | (M+H)+ | 0.66, 0.77 |
| AP55 | SQDFA50 | 1444 | (M+H)+ | 0.63, 0.67 |
| AP56 | SQDFA50 | 1444 | (M+H)+ | 0.58, 0.64 |
| AP57 | SQDFA50 | 1494 | (M+H)+ | 0.67 |
| AP58 | SQDFA50 | 1430 | (M+H)+ | 0.60 |
| AP59 | SQDFA50 | 1444 | (M+H)+ | 0.69 |
| AP60 | SQDFA50 | 1458 | (M+H)+ | 0.70 |
| AP61 | SQDFA05 | 1486 | (M-H)- | 1.20 |
| AP62 | SQDFA05 | 1450 | (M+H)+ | 1.07 |
| AP63 | SQDFA05 | 1550 | (M+H)+ | 1.19 |
| AP64 | SQDFA05 | 1506 | (M+H)+ | 1.13 |
| AP65 | SQDFA05 | 1464 | (M+H)+ | 1.12 |
| AP66 | SQDFA05 | 1428 | (M-H)- | 1.07 |
| AP67 | SQDFA05 | 1492 | (M-H)- | 1.12 |
| AP68 | SQDFA05 | 1448 | (M-H)- | 1.07 |
| AP69 | SQDFA05 | 1462 | (M-H)- | 1.12 |
| AP70 | SQDFA05 | 1462 | (M-H)- | 1.12 |
| AP71 | SQD2FA05 | 1488 | (M+H)+ | 1.19 |
| AP72 | SQD2FA05 | 1450 | (M+H)+ | 1.07 |
| AP73 | SQD2FA05 | 1536 | (M+H)+ | 1.14 |
| AP74 | SQD2FA05 | 1506 | (M+H)+ | 1.13 |
| AP75 | SQD2FA05 | 1478 | (M+H)+ | 1.15 |
| AP76 | SQD2FA05 | 1430 | (M+H)+ | 1.06 |
| AP77 | SQD2FA05 | 1494 | (M+H)+ | 1.11 |
| AP78 | SQD2FA05 | 1464 | (M+H)+ | 1.10 |
| AP79 | SQD2FA05 | 1464 | (M+H)+ | 1.12 |
| AP80 | SQD2FA05 | 1478 | (M+H)+ | 1.16 |
| AP81 | SQD2FA05 | 1472 | (M+H)+ | 1.16 |
| AP82 | SQD2FA05 | 1444 | (M+H)+ | 1.14 |
| AP83 | SQD2FA05 | 1500 | (M+H)+ | 1.14 |
| AP84 | SQD2FA05 | 1514 | (M+H)+ | 1.18 |

(continued)

| Compound ID | Analytical Condition | LCMS(ESI) m/z | | Retention Time (min) |
|---|---|---|---|---|
| AP85 | SQD2FA05 | 1414 | (M+H)+ | 1.08 |
| AP86 | SQD2FA05 | 1400 | (M+H)+ | 1.06 |
| AP87 | SQD2FA05 | 1458 | (M+H)+ | 1.12 |
| AP88 | SQD2FA05 | 1430 | (M+H)+ | 1.06 |
| AP89 | SQD2FA05 | 1502 | (M+H)+ | 1.17 |
| AP90 | SQD2FA05 | 1450 | (M+H)+ | 1.04 |
| AP91 | SQD2FA05 | 1506 | (M+H)+ | 1.12 |
| AP92 | SQD2FA05 | 1506 | (M+H)+ | 1.11 |
| AP93 | SQD2FA05 | 1458 | (M+H)+ | 1.13 |
| AP94 | SQD2FA05 | 1444 | (M+H)+ | 1.08 |
| AP95 | SQD2FA05 | 1488 | (M+H)+ | 1.11 |
| AP96 | SQD2FA05 | 1464 | (M+H)+ | 1.08 |
| AP97 | SQD2FA05 | 1478 | (M+H)+ | 1.11 |
| AP98 | SQD2FA05 | 1492 | (M+H)+ | 1.15 |
| AP99 | SQD2FA05 | 1488 | (M+H)+ | 1.14 |
| AP100 | SQD2FA05 | 1464 | (M+H)+ | 1.06 |
| AP101 | SQD2FA05 | 1536 | (M+H)+ | 1.13 |
| AP102 | SQD2FA05 | 1506 | (M+H)+ | 1.09 |
| AP103 | SQD2FA05 | 1478 | (M+H)+ | 1.13 |
| AP104 | SQD2FA05 | 1430 | (M+H)+ | 1.03 |
| AP105 | SQD2FA05 | 1494 | (M+H)+ | 1.09 |
| AP106 | SQD2FA05 | 1464 | (M+H)+ | 1.07 |
| AP107 | SQD2FA05 | 1472 | (M+H)+ | 1.13 |
| AP108 | SQD2FA05 | 1488 | (M+H)+ | 1.15 |
| AP109 | SQD2FA05 | 1464 | (M+H)+ | 1.06 |
| AP110 | SQD2FA05 | 1536 | (M+H)+ | 1.13 |
| AP111 | SQD2FA05 | 1506 | (M+H)+ | 1.10 |
| AP112 | SQD2FA05 | 1478 | (M+H)+ | 1.12 |
| AP113 | SQD2FA05 | 1430 | (M+H)+ | 1.04 |
| AP114 | SQD2FA05 | 1464 | (M+H)+ | 1.07 |
| AP115 | SQD2FA05 | 1550 | (M+H)+ | 1.14 |
| AP116 | SQD2FA05 | 1472 | (M+H)+ | 1.13 |
| AP117 | SQD2FA05 | 1502 | (M+H)+ | 1.19 |
| AP118 | SQD2FA05 | 1444 | (M+H)+ | 1.08 |
| AP119 | SQD2FA05 | 1500 | (M+H)+ | 1.13 |
| AP120 | SQD2FA05 | 1500 | (M+H)+ | 1.13 |
| AP121 | SQD2FA05 | 1458 | (M+H)+ | 1.14 |
| AP122 | SQD2FA05 | 1444 | (M+H)+ | 1.01 |

(continued)

| Compound ID | Analytical Condition | LCMS(ESI) m/z | | Retention Time (min) |
|---|---|---|---|---|
| AP123 | SQD2FA05 | 1508 | (M+H)+ | 1.14 |
| AP124 | SQD2FA05 | 1444 | (M+H)+ | 1.08 |
| AP125 | SQD2FA05 | 1472 | (M+H)+ | 1.14 |
| AP126 | SQD2FA05 | 1486 | (M+H)+ | 1.19 |
| AP127 | SQD2FA05 | 1458 | (M+H)+ | 1.17 |
| AP128 | SQD2FA05 | 1472 | (M+H)+ | 1.12 |
| AP129 | SQD2FA05 | 1472 | (M+H)+ | 1.08 |
| AP130 | SQD2FA05 | 1472 | (M+H)+ | 1.01 |
| AP131 | SQD2FA05 | 1486 | (M+H)+ | 1.16 |
| AP132 | SQD2FA05 | 1472 | (M+H)+ | 1.05 |
| AP133 | SQD2FA05 | 1458 | (M+H)+ | 1.06 |
| AP134 | SQD2FA05 | 1458 | (M+H)+ | 1.04 |
| | | | | |
| AP135 | SQDFA05 | 1593 | (M+H)+ | 1.15 |
| AP136 | SQDFA05 | 1577 | (M+H)+ | 1.12 |
| AP137 | SQDFA05 | 1579 | (M+H)+ | 1.10 |
| AP138 | SQDFA05 | 1591 | (M+H)+ | 1.02 |
| AP139 | SQDFA05 | 1535 | (M+H)+ | 1.06 |
| AP140 | SQDFA05 | 1613 | (M+H)+ | 1.11 |
| AP141 | SQDFA05 | 1591 | (M+H)+ | 1.10 |
| AP142 | SQDFA05 | 1485 | (M+H)+ | 1.12 |
| AP143 | SQDFA05 | 1477 | (M+H)+ | 0.99 |
| AP200 | SQDAAs | 1483 | (M-H)- | 7.90 |
| AP201 | SQDAAs | 1543 | (M+H)+ | 8.33 |
| AP202 | SQDFAs | 1539 | (M-H)- | 6.36 |
| AP203 | SQDAAs | 1569 | (M-H)- | 8.27 |
| AP204 | SQDAAs | 1483 | (M-H)- | 7.74 |
| AP205 | SQDFAs | 1471 | (M+H)+ | 5.72 |
| AP206 | SQDFAs | 1469 | (M-H)- | 6.05 |
| AP207 | SQDFAs | 1497 | (M-H)- | 6.62 |
| AP208 | SQDAAs | 1483 | (M-H)- | 7.73 |
| AP209 | SQDFAs | 1543 | (M+H)+ | 6.74 |
| AP210 | SQDAAs | 1539 | (M-H)- | 7.84 |
| AP211 | SQDAAs | 1571 | (M+H)+ | 8.16 |
| AP212 | SQDAAs | 1485 | (M+H)+ | 7.52 |
| AP213 | SQDAAs | 1469 | (M-H)- | 7.48 |
| AP214 | SQDFAs | 1469 | (M-H)- | 6.38 |
| AP215 | SQDFAs | 1498 | (M-H)- | 5.90 |

(continued)

| Compound ID | Analytical Condition | LCMS(ESI) m/z | | Retention Time (min) |
|---|---|---|---|---|
| AP216 | SQDAAs | 1501 | (M+H)+ | 7.67 |
| AP217 | SQDAAs | 1469 | (M-H)- | 7.63 |
| AP218 | SQDFAs | 1499 | (M-H)- | 5.79 |
| AP219 | SQDAAs | 1501 | (M+H)+ | 7.66 |
| AP220 | SQDAAs | 1513 | (M-H)- | 7.87 |
| AP221 | SQDAAs | 1515 | (M+H)+ | 7.81 |
| AP222 | SQDAAs | 1501 | (M+H)+ | 7.62 |
| AP223 | SQDFAs | 1469 | (M-H)- | 5.59 |
| AP224 | SQDFAs | 1483 | (M-H)- | 5.90 |
| AP225 | SQDFAs | 1483 | (M-H)- | 5.83 |
| AP226 | SQDFAs | 1469 | (M-H)- | 5.72 |
| AP227 | SQDAAs | 1569 | (M-H)- | 7.88 |
| AP228 | SQDAAs | 1571 | (M+H)+ | 7.94 |
| AP229 | SQDAAs | 1441 | (M-H)- | 7.03 |
| AP230 | SQDAAs | 1471 | (M+H)+ | 7.47 |
| AP231 | SQDAAs | 1513 | (M-H)- | 7.82 |
| AP232 | SQDFAs | 1527 | (M-H)- | 5.78 |
| AP233 | SQDFAs | 1414 | (M-H)- | 6.24 |
| AP234 | SQDAAs | 1484 | (M+H)+ | 7.81 |
| AP235 | SQDFAs | 1514 | (M+H)+ | 6.68 |
| AP236 | SQDAAs | 1434 | (M+H)+ | 8.07 |
| AP237 | SQDAAs | 1452 | (M+H)+ | 8.09 |
| AP238 | SQDFAs | 1430 | (M+H)+ | 5.88 |
| AP239 | SQDFAs | 1437 | (M+H)+ | 4.50 |
| AP240 | SQDFAs | 1423 | (M+H)+ | 4.10 |
| AP241 | SQDAAs | 1479 | (M+H)+ | 7.90 |
| AP242 | SQDFAs | 1437 | (M+H)+ | 4.24 |
| AP243 | SQDFAs | 1409 | (M+H)+ | 3.91 |
| AP244 | SQDFAs | 1423 | (M+H)+ | 4.13 |
| AP245 | SQDFAs | 1437 | (M+H)+ | 4.48 |
| AP246 | SQDAAs | 1391 | (M-H)- | 7.32 |
| AP247 | SQDAAs | 1379 | (M+H)+ | 7.37 |
| AP248 | SQDAAs | 1515 | (M+H)+ | 8.25 |
| AP249 | SQDAAs | 1499 | (M+H)+ | 8.24 |
| AP250 | SQDAAs | 1501 | (M+H)+ | 8.00 |
| AP251 | SQDAAs | 1511 | (M-H)- | 7.94 |
| AP252 | SQDFAs | 1455 | (M-H)- | 5.75 |
| AP253 | SQDAAs | 1437 | (M+H)+ | 7.86 |

(continued)

| Compound ID | Analytical Condition | LCMS(ESI) m/z | | Retention Time (min) |
|---|---|---|---|---|
| AP254 | SQDAAs | 1535 | (M+H)+ | 8.15 |
| AP255 | SQDFAs | 1461 | (M-H)- | 5.81 |
| AP256 | SQDAAs | 1529 | (M-H)- | 8.05 |
| AP257 | SQDFAs | 1515 | (M+H)+ | 6.30 |
| AP258 | SQDFAs | 1517 | (M+H)+ | 6.08 |
| AP259 | SQDFAs | 1527 | (M-H)- | 5.62 |
| AP260 | SQDAAs | 1551 | (M+H)+ | 8.02 |
| AP261 | SQDFAs | 1479 | (M+H)+ | 5.57 |
| AP262 | SQDAAs | 1124 | (M-H)- | 6.54 |
| AP263 | SQDFAs | 1253 | (M+H)+ | 5.63 |
| AP264 | SQDAAs | 1400 | (M-H)- | 8.13 |
| AP265 | SQDAAs | 1594 | (M+H)+ | 8.07 |
| AP266 | SQDFAs | 1697 | (M+H)+ | 6.39 |

[Example 3] Conformational analysis of side chain partial structures

[Example 3-1] Calculation method for low energy conformation of side chain partial structure

The low energy conformation of a side chain partial structure was calculated using a partial structure obtained by cutting off the side chain portion of an amino acid from the side chain β position (carbon directly bonded to the main chain). The following scheme shows examples for Ser(EtOH), bAla(3R-MeOEtOH), bAla(2S-MeOEtOH), and Phe(3-OMe-4-CONMe).

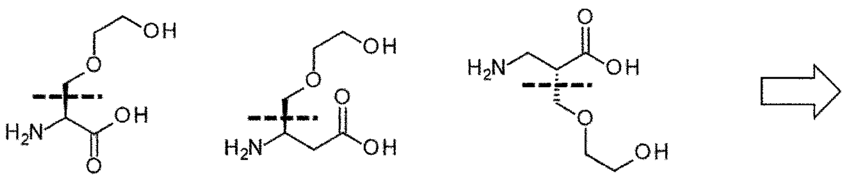

Partial structure

Ser(EtOH) bAla(3R-MeOEtOH) bAla(2S-MeOEtOH)

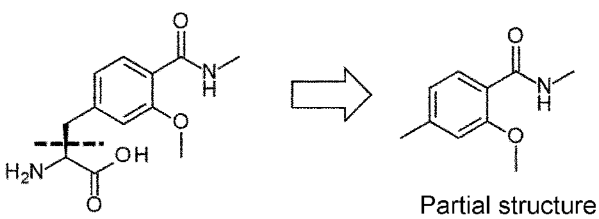

Partial structure

Phe(3-OMe-4-CONMe)

Concerning cyclic portions such as a partial structure in Hyp(2-EtOH) (a pyrrolidine ring in the case of Hyp(2-EtOH)), the low energy conformation was calculated using a partial structure obtained by cutting off the side chain portion of an amino acid from an atom directly bonded to the cyclic portion. The following scheme shows an example for Hyp(2-EtOH).

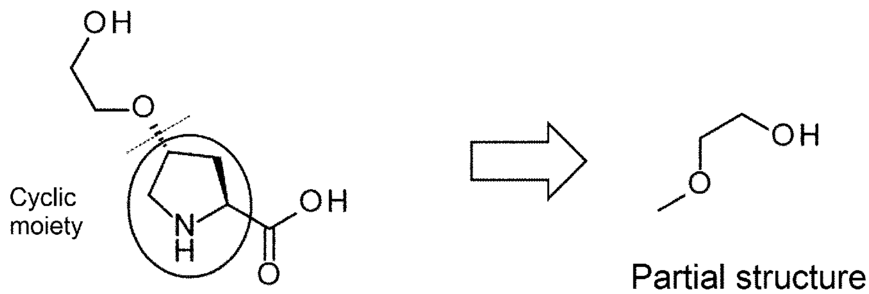


Partial structure

Hyp(2-EtOH)

For example, Ser(EtOH), bAla(3R-MeOEtOH), bAla(2S-MeOEtOH), and Hyp(2-EtOH) can be considered as having the same side chain partial structure.

Concerning a type of amino acids that have a substituent on the amino group of an amino acid such as (Ph(3-OMe-4-CONMe)Et)Gly, the low energy conformation was calculated using a partial structure obtained by cutting off the side chain portion of an amino acid from an atom directly bonded to an N atom.

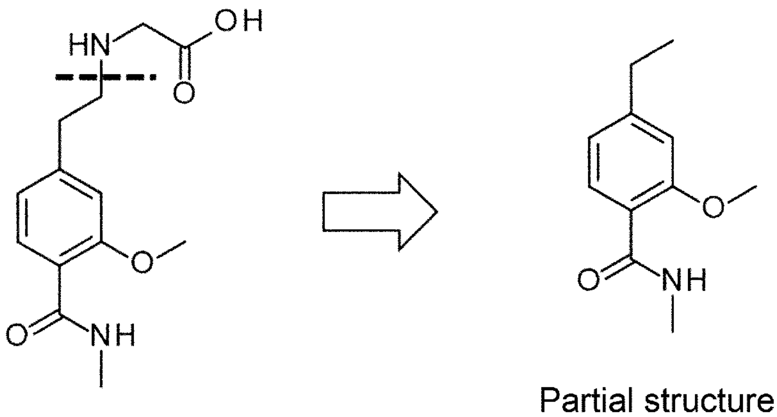

Partial structure

(Ph(3-OMe-4-CONMe)Et)Gly

The low energy conformation was calculated according to the following protocol.

(1) Initial structure generation

Concerning a rotatable single bond, initial dihedral angles of 0, 90, 180, and 270 degrees were given. Concerning an amide bond and a bond between C and OH, initial dihedral angles of 0 and 180 degrees were given. When calculating the side chain partial structure of Ser(EtOH), a total of 32 initial conformations were generated because 4 initial dihedral angles are considered for each of 2 single bonds, and 2 initial dihedral angles are considered for a bond between C and OH.

(2) Structural optimization

Structural optimization of all conformations generated in (1) was performed using Gaussian 09. A polarizable continuum model (PCM) was applied to the B3LYP/6-31G(d) level, and structural optimization was performed in simulated water.

(3) Selection of low energy conformation

Unique low energy conformations were selected from minimized conformations obtained in (2) in order of increasing energy. Conformational uniqueness is determined according to the distance (difference) between conformations at the dihedral angles considered during structure generation. A conformation, the distance of which between conformations in a dihedral angle space (see the defining equation below) is 60° or more with respect to all other low energy conformations, is defined as being unique.

[Expression 1]

$$\sqrt{\sum_{k=1}^{n}\left(\phi_k^i - \phi_k^j\right)^2}$$

$\phi_k^i$ : *k*-th dihedral angle of *i*-th conformation $\phi_k^j$ : *k*-th dihedral angle of *j*-th conformation

[Example 3-2] Potential energy surface calculation method

1) In the case of a potential energy surface in the following partial structure (e.g. the side chain partial structure of Ser(NMe-Aca)) (Fig. 3-1)

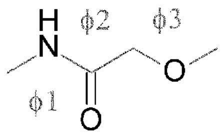

(1) Low energy conformations were calculated by the above-described protocol.
(2) Energy calculation for each conformation (grid point)

As initial values, φ1 was 180°, and φ2 and φ3 were 0°, 15°, and so on up to 345° at 15° intervals, and thus 24 × 24 = 576 initial conformations (grid points) were generated.
With φ2 and φ3 being fixed to the initial values, structural optimization of all generated conformations was performed using Gaussian 09. A polarizable continuum model (PCM) was applied to the B3LYP/6-31G(d) level, and structural optimization was performed in simulated water. As a result, energy (E(φ2, φ3)) of 576 conformations calculated using φ2 and φ3 at 15° intervals was obtained. Fig. 3-1 is a plot of ΔE(φ2, φ3) obtained from the following equation.

$$\Delta E(\varphi 2, \varphi 3) = E(\varphi 2, \varphi 3) - E_{lowest}$$

$E_{lowest}$: Energy of lowest energy conformation
2) In the case of a potential energy surface in the following partial structures (e.g. the side chain partial structure of Ser(EtOH), bAla(3R-MeOEtOH), bAla(2S-MeOEtOH), and Hyp(2-EtOH)) (Fig. 4-1)

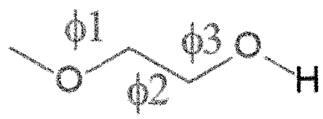

(1) Low energy conformations were calculated by the above-described protocol.
(2) Energy calculation for each conformation (grid point)

As initial values, φ1 was 0°, 90°, 180°, and 270°, φ2 was 0°, 15°, and so on up to 345° at 15° intervals, and φ3 was 0° and 180°, and thus 4 × 24 × 2 = 192 initial conformations (grid points) were generated. Meanwhile, when φ2 = 180°, 4 × 4 = 16 conformations were further generated with φ1 = 0°, 90°, 180°, and 270 °, and φ3 = 0°, 90°, 180°, and 270°. With φ2 being fixed to the initial values, structural optimization of all generated conformations was performed using Gaussian 09. A polarizable continuum model (PCM) was applied to the B3LYP/6-31G(d) level, and structural optimization was performed in simulated water. As a result, 4 × 2 = 8 E(φ2) values were obtained for each φ2. Among the E(φ2) values, an E(φ2) value of the lowest energy was defined as $E_{lowest}$ (φ2). Energies (ΔE(φ2)) of 24 conformations calculated with φ2 at 15° intervals were obtained by the following expression. Fig. 4-1 is a plot of ΔE(φ2).

$$\Delta E(\varphi 2) = E_{lowest}(\varphi 2) - E_{lowest}$$

$E_{lowest}$: Energy of lowest energy conformation

[Example 3-3] Investigation of conformational distribution of crystal structures using results of X-ray structural analysis registered in CSD (The Cambridge Structural Database) (Fig. 3-2, Fig. 4-2)

Using CSD (The Cambridge Structural Database) 5.39 + 2 updates, searches were conducted using the following queries.

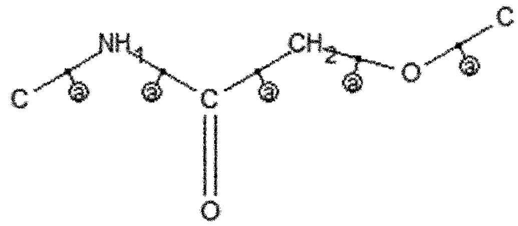

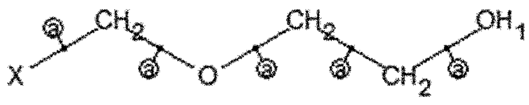

Searches were respectively conducted using the following search criteria: 3D coordinates determined, R factor ≤ 0.05, Not disordered, No errors, Not polymeric, No ions, No powder structures, and Only Organics. Figs. 3-2 and 4-2 are plots of the results. As a result of analysis, Fig. 3-2 and Fig. 4-2 did not show great discrepancy from Figs. 3-1 and 4-1 showing the result of calculation. More specifically, it was confirmed that these partial structures are structures that likely form an intramolecular hydrogen bond. Moreover, when two crystal structures (CSD codes: MAPPAV and MUVJOE) present in the vicinity of $\varphi 2$ = 180° in Fig. 3-2 were analyzed, it was confirmed that the donor-moiety amide NH group that forms an intramolecular hydrogen bond forms an intermolecular hydrogen bond with another molecule different from itself, and is thus used to interact with it. This confirms that the structures analyzed in Fig. 3-1 can take a conformation depending on the surrounding environment, such as a conformation forming an intramolecular hydrogen bond that is advantageous to membrane permeation when it passes through a membrane, and a conformation forming an intermolecular hydrogen bond that is advantageous in bonding when it interacts with its target.

[Example 3-4] Results of calculating low energy conformation of side chain partial structures

It was computationally shown that, in all amino acids shown below, the side chain partial structures are structures that likely take a conformation having an intramolecular hydrogen bond.

Ser(EtOH), bAla(3R-MeOEtOH), bAla(2S-MeOEtOH), and Hyp(2-EtOH): these three amino acids have the same side chain partial structure. According to the calculation result, the conformation having an intramolecular hydrogen bond was the most stable conformation.

Hph(3-OME-4-CONMe)-OH, D-Hph(3-OMe-4-CONMe)-OH, MeHph(3-OMe-4-CONMe)-OH, and (Ph(3-OMe-4-CONMe)Et)Gly: these four amino acids have the same side chain partial structure. According to the calculation result, the conformation having an intramolecular hydrogen bond was the most stable conformation.

Only the most stable conformations are depicted in the calculation result columns of Table 17.

[Table 17]

| Amino Acid | | | |
|---|---|---|---|
| Ser(EtOH) | bAla(3R-MeOEtOH) | bAla(2S-MeOEtOH) | Hyp(2-EtOH) |
| 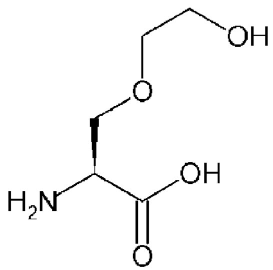 | 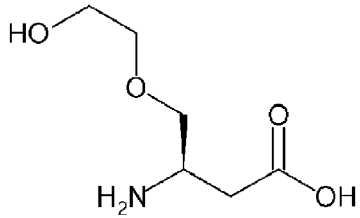 | 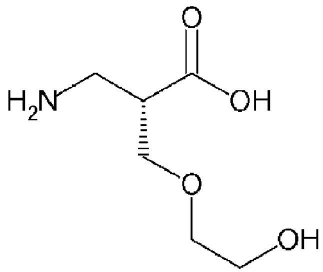 | 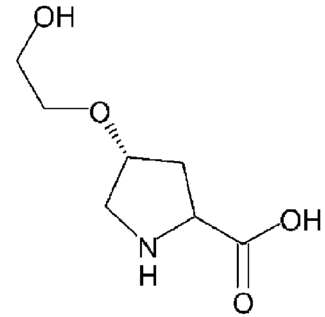 |
| | | | |
| Partial Structure | Calculation Result | | |
| 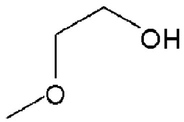 | 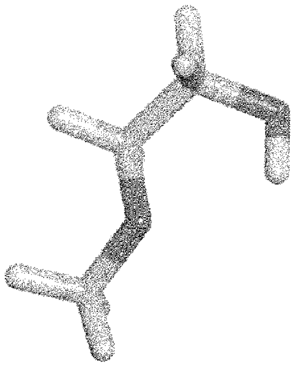 | | |
| Hph(3-OMe-4-CONMe) | D-Hph(3-OMe-4-CONMe) | MeHph(3-OMe-4-CONMe) | (Ph(3-OMe-4-CONMe)Et)Gly |

(continued)

| Amino Acid | | | |
|---|---|---|---|
| 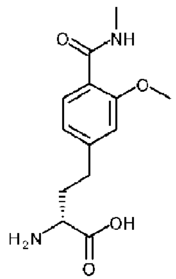 | 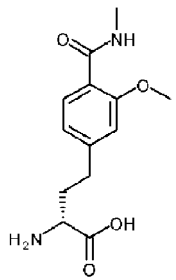 | 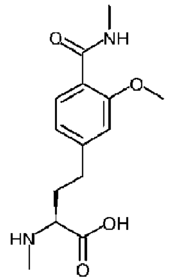 | 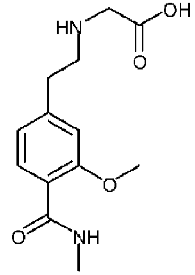 |
| | | | |
| Partial Structure | Calculation Result | | |
| 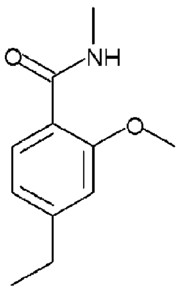 | 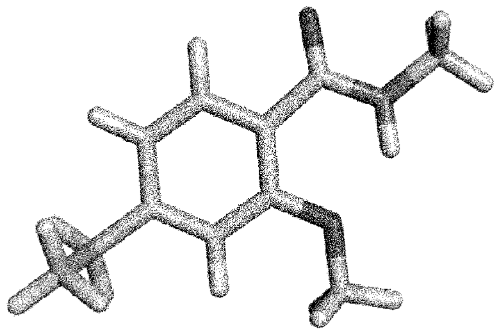 | | |

Ser(S-2-PrOH), Ser(R-2-PrOH), Ser(tBuOH), Ser(nPrOH), Ser(S-2-BuOH), Ser(R-2-BuOH), Ser(2-Me2-PrOH), Ser(2-Me-2-BuOH), Ser(NMe-Aca), Ser(NtBu-Aca), Phe(3-OMe-4-CONMe), Phe(4-OMe-3-CONMe), Tyr(3-OMe), Tyr(3-OMe), Phe(3-OMe-4-CONHMs), Phe(4-CONMeOH), and Phe(3-OMe-4-CONHOH): All calculation results showed that the conformation having an intramolecular hydrogen bond was the most stable conformation. Only the most stable conformations are depicted in the calculation result column of Table 18.

Ser(1-CF3-EtOH): Since this amino acid is racemic due to the carbon moiety to which the $CF_3$ group is attached, the stable conformation of each of the R-form Ser(R-1-CF3-EtOH) and the S-form Ser(S-1-CF3-EtOH) was calculated. Both calculation results showed that the conformation having an intramolecular hydrogen bond was the most stable conformation. Only the most stable conformations are depicted in the calculation result column of Table 18.

Ser(3-Me-5-Oxo-Odz): According to the calculation result, the conformation having an intramolecular hydrogen bond was 0.89 kJ/mol different from the most stable conformation. The calculation result column in the table depicts a conformation 0.89 kJ/mol different from the most stable conformation.

Ser(S-4-F2-Pyrro-Me) and Ser(S-Mor-3-Me): These amino acids have a secondary amino group in the side chain moiety, and therefore may have a charge. Calculations were made for both cases, *i.e.*, when not having a charge (neutral) and when having a charge (charged), and both calculation results showed that the conformation having an intramolecular hydrogen bond was the most stable conformation. Only the most stable conformations are depicted in the calculation result column of Table 18.

[Table 18]

| Amino Acid | | Partial Structure | Calculation Result |
|---|---|---|---|
| Abbreviation | Structure | | |
| Ser(S-PrOH) | 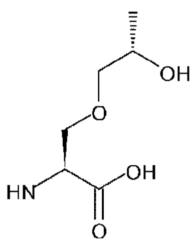 | 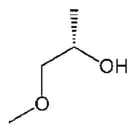 | 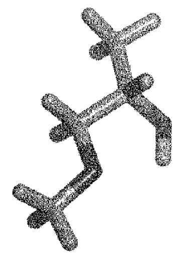 |
| Ser(R-PrOH) | 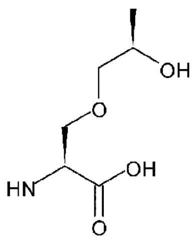 | 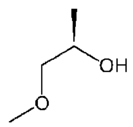 | 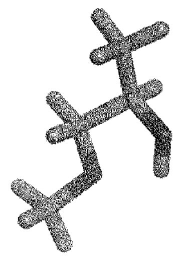 |
| Ser(R-1-CF3-EtOH) | 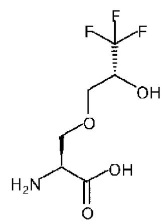 | 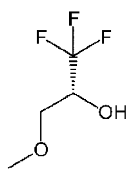 | 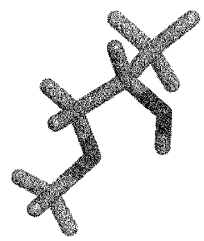 |
| Ser(S-1-CF3-EtOH) | 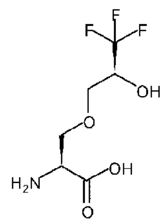 | 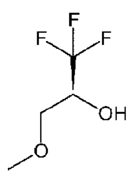 | 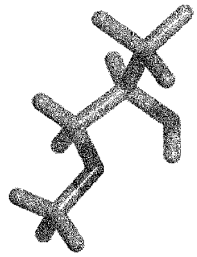 |

(continued)

| Amino Acid | | Partial Structure | Calculation Result |
|---|---|---|---|
| Abbreviation | Structure | | |
| Ser(tBuOH) | 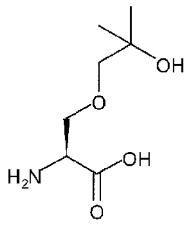 | 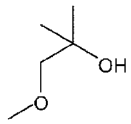 | 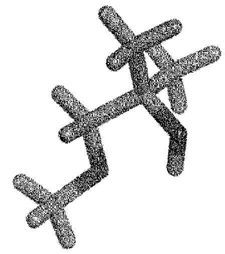 |
| Ser(nPrOH) | 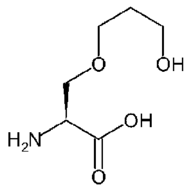 | 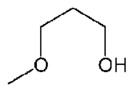 | 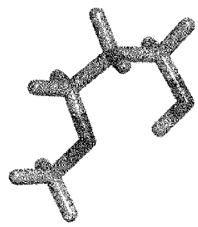 |
| Ser(S-2-BuOH) | 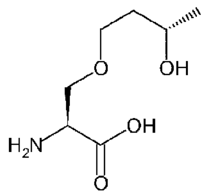 | 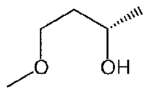 | 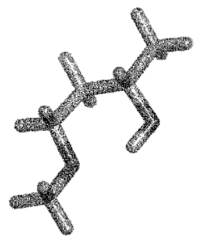 |
| Ser(R-2-BuOH) | 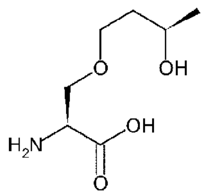 | 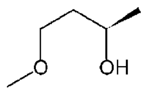 | 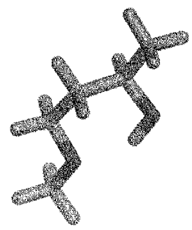 |
| Ser(2-Me2-PrOH) | 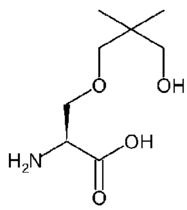 | 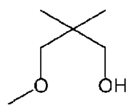 | 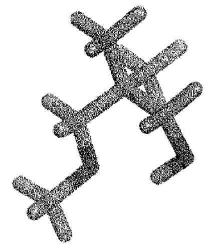 |
| Ser(2-Me-2-BuOH) | 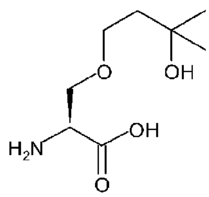 | 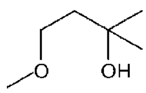 | 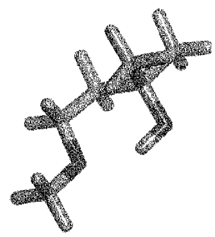 |

(continued)

| Amino Acid | | Partial Structure | Calculation Result |
|---|---|---|---|
| Abbreviation | Structure | | |
| Ser(NMe-Aca) | 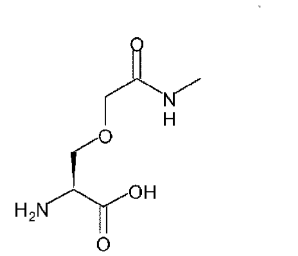 | 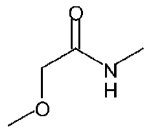 | 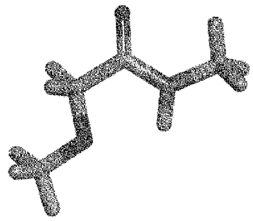 |
| Ser(tBu-Aca) | 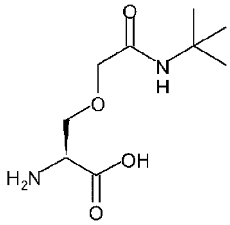 | 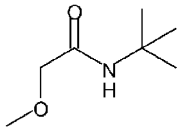 | 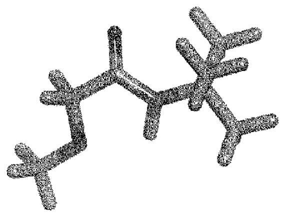 |
| Phe(3-OMe-4-CONMe) | 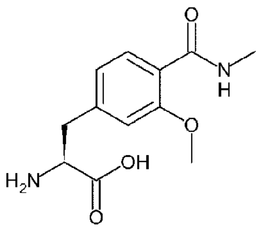 | 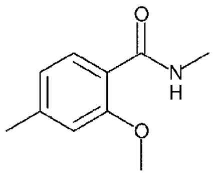 | 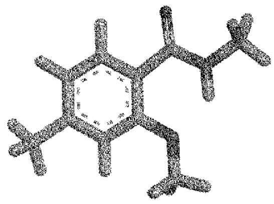 |
| Phe(4-OMe-3-CONMe) | 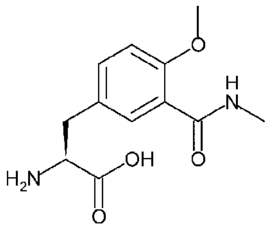 | 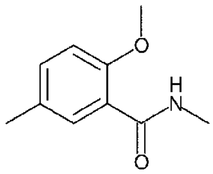 | 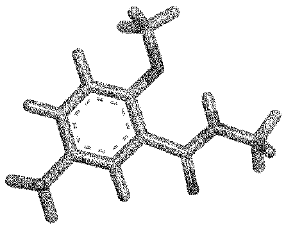 |
| Tyr(3-OMe) | 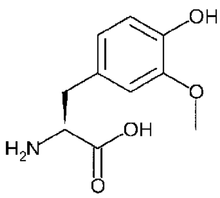 | 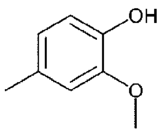 | 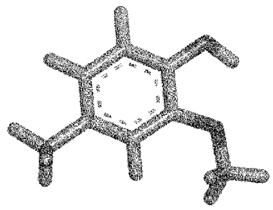 |
| Ser(3-Me-5-Oxo-Odz) | 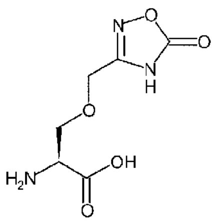 | 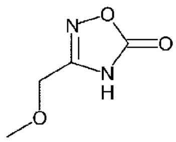 | 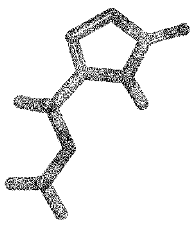 |

(continued)

| Amino Acid | | Partial Structure | Calculation Result |
|---|---|---|---|
| Abbreviation | Structure | | |
| Ser(S-4-F2-Pyrro-Me) (neutral) | 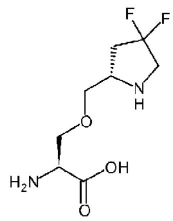 | 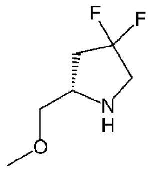 | 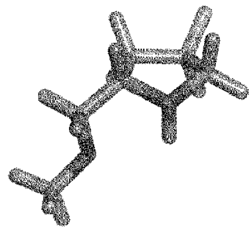 |
| Ser(S-4-F2-Pyrro-Me) (charged) | 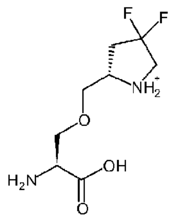 | 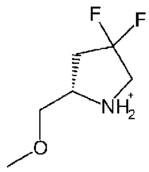 | 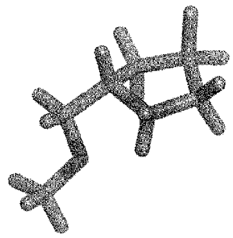 |
| Phe(3-OMe-4-CONHMs) | 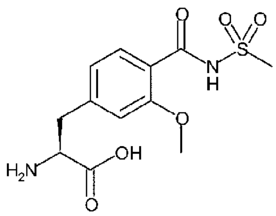 | 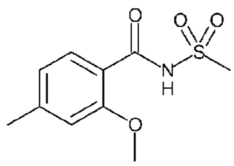 | 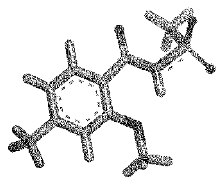 |
| Phe(4-CONMeOH) | 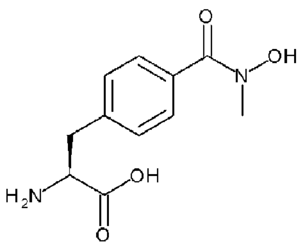 | 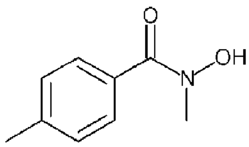 | 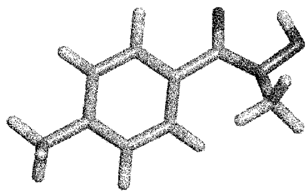 |
| Phe(3-OMe-4-CONHOH) | 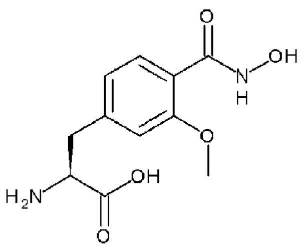 | 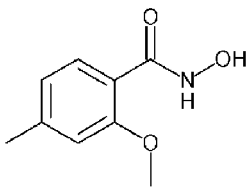 | 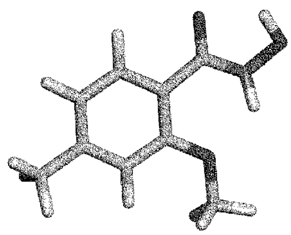 |
| Ser(S-M or-3-Me) (neutral) | 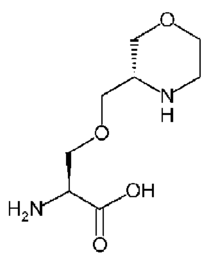 | 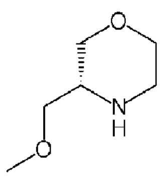 | 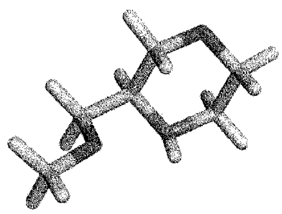 |

(continued)

| Amino Acid | | Partial Structure | Calculation Result |
|---|---|---|---|
| Abbreviation | Structure | | |
| Ser(S-M or-3-Me) (charged) | 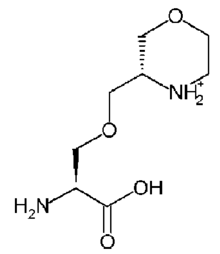 | 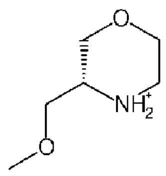 | 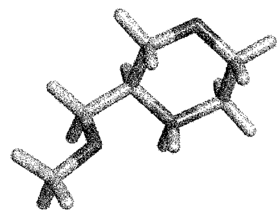 |

[Reference Example 1] Determination of the lengths of amino acid side chains

Amino acid side chains as used herein include chains attached to carbon atoms contained in amino acids (such as α-, β-, or γ-carbon atoms), and chains attached to nitrogen atoms. Herein, the length of an amino acid side chain can be determined by the following method. Specifically, the length can be determined by capping the N-terminus and the C-terminus of an amino acid unit with an acetyl group and a methylamino group, respectively, generating a conformation with Low Mode MD of molecular modeling software MOE (Chemical Computing Group), and measuring the distance from the atom to which the side chain moiety is attached (an α-carbon atom (Cα carbon) in the case of natural amino acids) to the most distal atom of the same side chain (excluding a hydrogen atom). A method of determining the length of the side chain of Phe or nBuGly is illustrated below as an example.

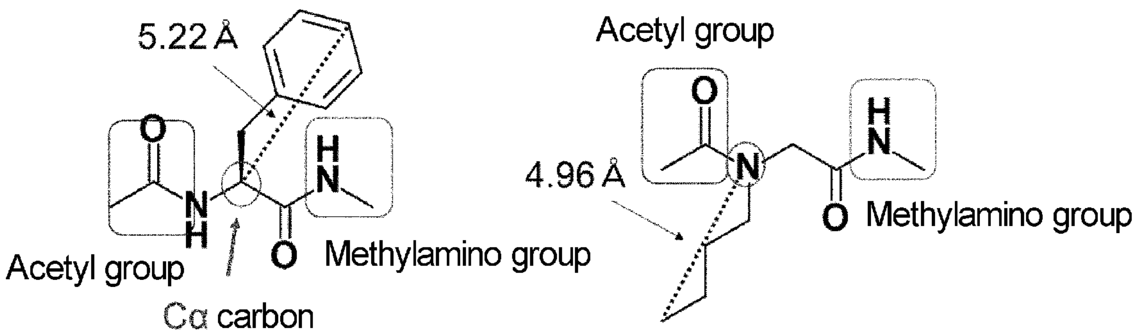


A method of determining the length of an amino acid side chain, Phe (left), nBuGly (right)

When an amino acid side chain forms a ring with part of the main chain structure, there are a plurality of atoms to which the side chain moiety is attached. In this case, the length of the side chain is the longest distance among the distances determined by calculating as described above for each atom to which the side chain moiety is attached and the most distal atom of the side chain (excluding a hydrogen atom). Taking Hyp(Et) as an example, the length of the Hyp(Et) side chain is 6.09 angstroms, because the length of the side chain determined from the nitrogen atom is 6.04 angstroms and the length of the side chain determined from the Cα carbon is 6.09 angstroms.

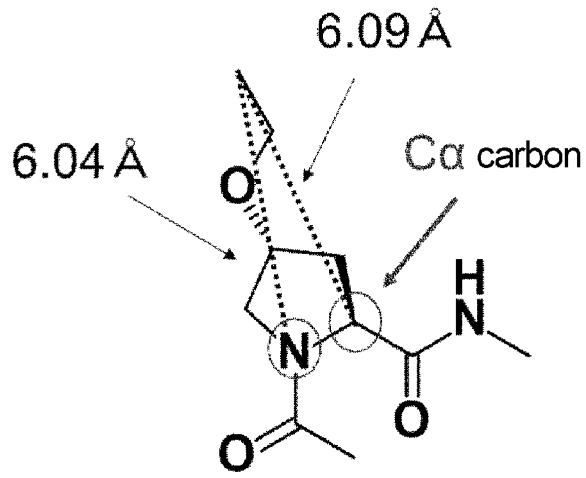


A method of determining the length of an amino acid side chain, Hyp (Et)

The lengths of the amino acid side chains calculated by the above method are shown below.

[Table 19]

| Amino Acid | Length of side chain (angstrom) |
|---|---|
| Ser (EtOH) | 6.00 |
| Phe (3-OMe-4-CONHMs) | 10.01 |

[Reference Example 2] Calculation of pKas

Herein, calculated pKas and calculated basic pKas of amino acid side chains or of side chains of the cyclic portion of a cyclic peptide compound can be determined using ADMET Predictor (Simulations Plus Inc., ver. 8.0). Calculated pKas and calculated basic pKas are calculated using a partial structure obtained by separating the side chain moiety starting from the position β of the side chain (the carbon directly attached to the main chain). The case of Lys is provided below as an example. The calculated basic pKa was calculated to be 10.5 using a partial structure including the position β of the side chain (the carbon directly attached to the main chain). When similarly calculating for acids, the side chain carboxy group of Asp had a calculated pKa of 4.3, the side chain phenolic hydroxyl group of Tyr had a calculated pKa of 9.9, the side chain phenolic hydroxyl group of 3-fluorotyrosine (Tyr(3-F)) had a calculated pKa of 8.7, and tetrazole had a calculated pKa of 3.7. On the other hand, for bases, the side chain guanidino group of Arg had a calculated basic pKa of 12.7, the imidazolyl group of His had a calculated basic pKa of 7.6, and pyridine had a calculated basic pKa of 5.4.

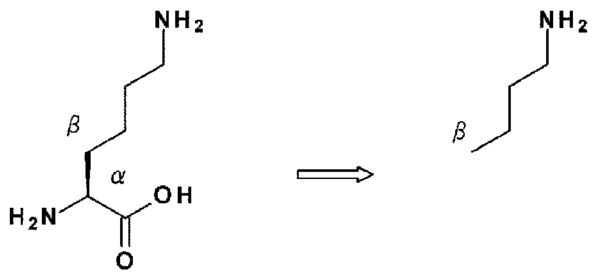

Lys

Partial Structure

A part of amino acids having basic side chains whose basic pKas were calculated by a method described herein are provided in the following table.

[Table 20]

| X | Ser(Et-2-NMe2) | MeAbu(pip-4-F2) | MeAbu(Mor) |
|---|---|---|---|
| Amino Acid Structure | 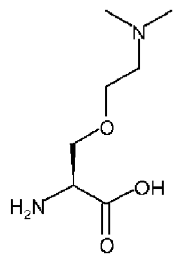 | 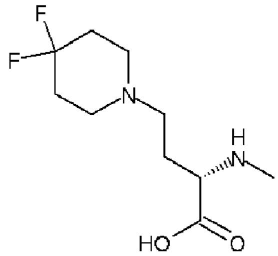 | 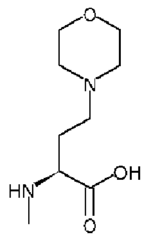 |
| basic pKa | 8.9 | 8.6 | 8.2 |
| | | | |
| X | Ser(Et-2-Mor) | MeAbu(pip-3-F2) | Ser(S-4-F2-Pyrro-Me) |
| Amino Acid Structure | 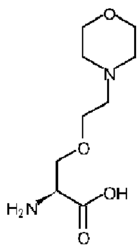 | 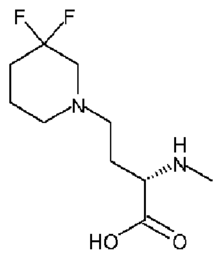 | 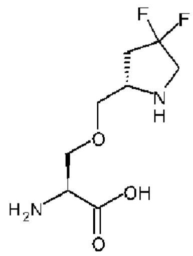 |
| basic pKa | 7.1 | 6.8 | 6.8 |

A part of amino acids having acidic side chains whose pKas were calculated by a method described herein are provided in the following table.

[Table 21]

| X | Abu(5-Oxo-Odz) | Gln(Ms) |
|---|---|---|
| Amino acid structure | 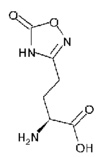 | 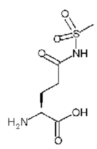 |
| pKa | 8.3 | 5.3 |

[Reference Example 3-1] Actual measurement of pKas of amino acid side chains

Herein, measurement of pKas of amino acid side chains was carried out according to the following procedure.
(Instrument used)

Sirius T3 (Sirius Analytical Instruments Ltd., Forest Row, East Sussex, RH18 5DW, UK)

[Table 22]

| | |
|---|---|
| pH Electrode: | Ag/AgCl, Double Junction Reference |
| Purge Gas: | Argon |
| pH Range: | 1.8-12.2 |

(continued)

| Measurement Condition | |
|---|---|
| Set Temperature | 25°C |
| Ion Concentration | 0.15M |
| Titration Solution | 0.5M KOH, 0.5M HCl |

(Implementation procedure)

0.15 M KCl was added to about 1 mg of a test substance as a powder or in about 100 μL of a 10 mM DMSO solution. When the test substance was a basic compound, 0.5 M HCl was added until pH 2 and the test substance was then titrated to pH 12 with 0.5 M KOH.

When the test substance was an acidic compound, 0.5 M KOH was added until pH 12 and the test substance was then titrated to pH 2 with 0.5 M HCl.

Titration operations were automatically performed by T3 manufactured by Sirius, and pKas were determined from the resulting titration curves using Sirius T3 Refinement software.

pKas were similarly determined using imipramine HCl, propranolol HCl, and warfarin as reference compounds, and were confirmed to be within the test results obtained in Sirius.

pKas of amino acid side chains were determined by synthesizing a sequence in which the amino acid to be evaluated is introduced into the second residue (the position X in the following table) of a peptide composed of three residues as shown below.

[Table 23]

| 3 | 2 | 1 | C-term |
|---|---|---|---|
| ZMeGly | X | MeGly | pip |

The results are provided below.

[Table 24]

| X | Ser(Et-2-NMe2) | Ser(Et-2-Mor) | MeAbu(Mor) | MeAbu(pip-4-F2) |
|---|---|---|---|---|
| Amino Acid Structure | 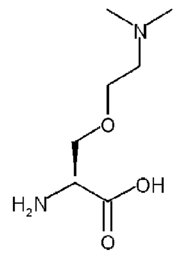 | 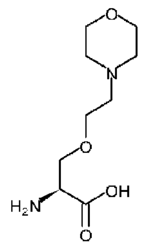 | 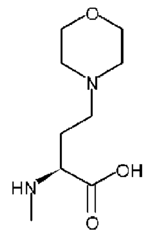 | 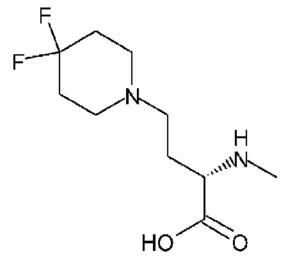 |
| basic pKa | 9.1 | 7.2 | 6.8 | 6.7 |
| | | | | |
| X | Ser(S-4-F2-Pyrro-Me) | MeAbu(pip-3-F2) | MeAbu (5-Oxo-Odz) | Gln(Ms) |
| Amino Acid Structure | 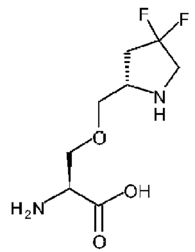 | 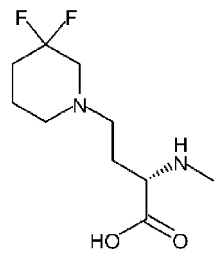 | 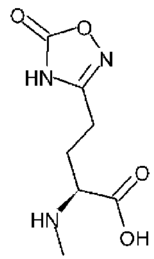 | 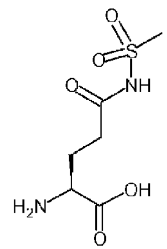 |
| pKa | 6.5 | 5.7 | 5.7 | 3.8 |

[Refernce Example 3-2] Synthesis of three-residue peptides used for pKa measurement

Peptides were elongated and cleaved from the resin by a similar procedure as in the chemical synthesis of peptide compounds described in Example 1, after which the C-terminal carboxylic acid was condensed with piperidine to synthesize pd30 to pd37. Fmoc-MeGly-Trt(2-Cl)-resin (Compound pd11) used for peptide synthesis was synthesized as follows. ZMeGly (Cbz-MeGly-OH, CAS #39608-31-6) was purchased from Tokyo Chemical Industry.

Synthesis of N-(((9H-fluoren-9-yl)methoxy)carbonyl)-N-methylglycine-2-chlorotrityl resin (Compound pd11, Fmoc-MeGly-Trt(2-Cl)-resin)

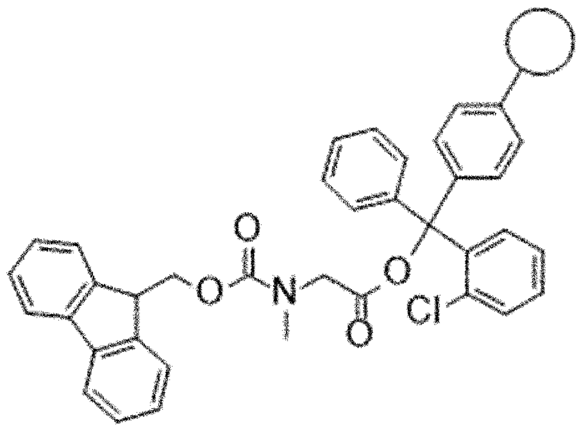

In a reaction vessel with a filter was placed 2-chlorotrityl chloride resin (1.58 mmol/g, 100-200 mesh, 1% DVB, purchased from Watanabe Chemical Industries, 10 g, 15.8 mmol) and dehydrated dichloromethane, and the vessel was shaken at room temperature for 1 h. The dichloromethane was removed by applying nitrogen pressure, after which a solution of commercially available N-(((9H-fluoren-9-yl)methoxy)carbonyl)-N-methylglycine (Fmoc-MeGly-OH) (3.54 g, 11.39 mmol) and diisopropylethylamine (DIPEA) (5.29 mL, 30.4 mmol) in dehydrated dichloromethane (130 mL) was added to the reaction vessel, and the vessel was shaken for 30 min. The reaction solution was removed by applying nitrogen pressure, after which dehydrated methanol (5.76 mL) and diisopropylethylamine (DIPEA) (5.29 mL, 30.4 mmol) were added to dehydrated dichloromethane (130 mL), the resulting mixture was added to the reaction vessel, and the vessel was shaken for 1 h. The reaction solution was removed by applying nitrogen pressure, after which dichloromethane was placed in the vessel, followed by shaking for 5 min. After removing the reaction solution by applying nitrogen pressure, dichloromethane was added, followed by shaking for 5 min. The reaction solution was removed by applying nitrogen pressure. The resulting resin was dried under reduced pressure overnight to afford N-(((9H-fluoren-9-yl)methoxy)carbonyl)-N-methylglycine-2-chlorotrityl resin (Compound pd11, Fmoc-MeGly-Trt(2-Cl)-resin) (13.2 g).

The loading amount of the resulting resin was calculated using the method described in the document (Letters in Peptie Science, 2002, 9, 203). N-(((9H-Fluoren-9-yl)methoxy)carbonyl)-N-methylglycine-2-chlorotrityl resin (Compound pd11, Fmoc-MeGly-Trt(2-Cl)-resin) (14.4 mg) was placed in a reaction vessel, DMF (2 mL) was added, and the vessel was shaken for 1 h. DBU (0.04 mL) was added to the reaction solution, the vessel was shaken for 30 min, DMF (10 mL) was then added, and 1 mL was taken out and further diluted with DMF so that the amount of the solution was 12.5 mL. The absorbance (294 nm) of the resulting solution was measured (using Shimadzu, UV-1600PC (cell length: 1.0 cm)), and the loading amount of N-(((9H-fluoren-9-yl)methoxy)carbonyl)-N-methylglycine-2-chlorotrityl resin (Compound pd11, Fmoc-MeGly-Trt(2-Cl)-resin) was calculated to be 0.789 mmol/g.

[Reference Example 3-3] Analytical information on peptide compounds measured for pKa

[Table 25]

| Compound ID | **3** | **2** | **1** | **C-term** | Analytical Condition | **LCMS(ESI) m/z** | | Retention Time (min) |
|---|---|---|---|---|---|---|---|---|
| pd30 | ZMeGly | Ser(Et-2-NMe2) | MeGly | pip | SQDFA05 | 520 | (M+H)+ | 0.45 |

(continued)

| Compound ID | 3 | 2 | 1 | C-term | Analytical Condition | LCMS(ESI) m/z | | Retention Time (min) |
|---|---|---|---|---|---|---|---|---|
| pd31 | ZMeGly | Ser(Et-2-Mor) | MeGly | pip | SQDFA05 | 562 | (M+H)+ | 0.45 |
| pd32 | ZMeGly | MeAbu(Mor) | MeGly | pip | SQDFA05 | 546 | (M+H)+ | 0.47 |
| pd33 | ZMeGly | MeAbu(pip-4-F2) | MeGly | pip | SQDFA05 | 580 | (M+H)+ | 0.50 |
| pd34 | ZMeGly | MeAbu(pip-3-F2) | MeGly | pip | SQDFA05 | 580 | (M+H)+ | 0.50 |
| pd35 | ZMeGly | MeAbu(5-Oxo-Odz) | MeGly | pip | SQDFA05 | 545 | (M+H)+ | 0.61 |
| pd36 | ZMeGly | Gln(Ms) | MeGly | pip | SQDFA05 | 568 | (M+H)+ | 0.58 |
| pd37 | ZMeGly | Ser(S-4-F2-Pyrro-Me) | MeGly | pip | SQDFA05 | 566 | (M-H)- | 0.48 |

[Reference Example 3-4] Structural information on peptide compounds measured for pKa

[Table 26]

| ID | Structural Formula |
|---|---|
| pd30 | 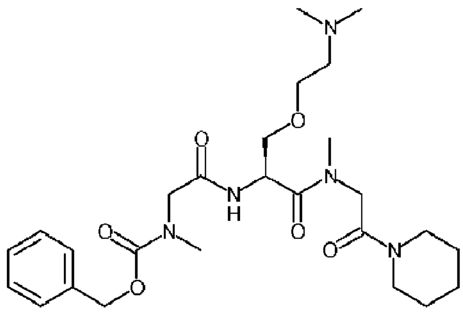 |
| pd31 | 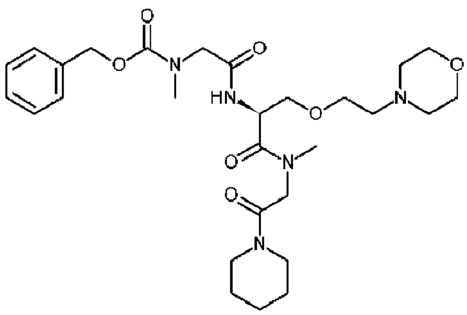 |
| pd32 | 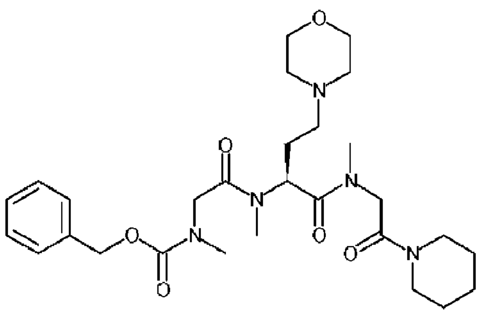 |

(continued)

| ID | Structural Formula |
|---|---|
| pd33 | 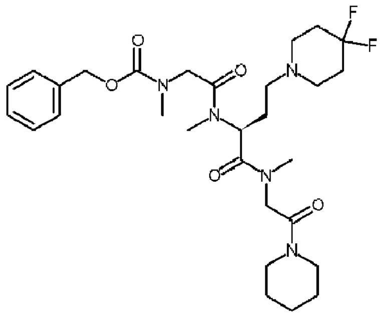 |
| pd34 | 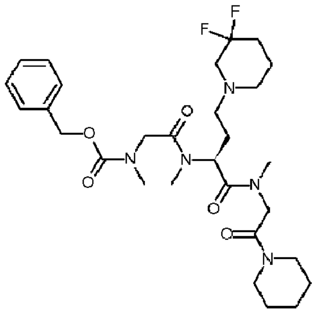 |
| pd35 | 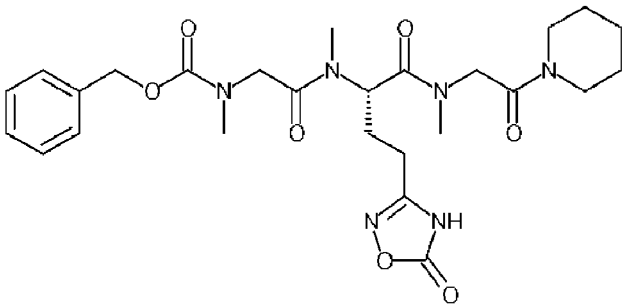 |
| pd36 | 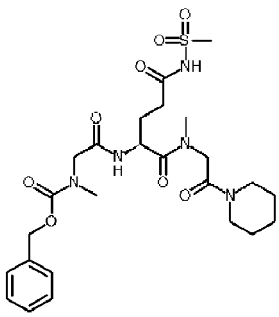 |

(continued)

| ID | Structural Formula |
|---|---|
| pd37 | 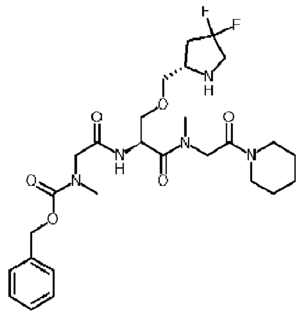 |

For sequences comprising Ser(Et-2-NMe2) that is an amino acid having a measured basic pKa of 9.1 and a calculated basic pKa of 8.9, membrane permeability was evaluated in the range of 1.21 ≤ ClogP/total aa ≤ 1.36, and for sequences comprising Gln(Ms) that is an amino acid having a measured pKa of 3.8 and a calculated pKa of 5.3, membrane permeability was evaluated in the range of 1.20 ≤ ClogP/total aa ≤ 1.33 (pd50 to pd70). On the other hand, examples of sequences that comprise MeAbu(pip-4-F2), MeAbu(Mor), Ser(Et-2-Mor), MeAbu(pip-3-F2), or Abu(5-Oxo-Odz) and that achieved a membrane permeability of $P_{app} \geq 1.0 \times 10^{-6}$ cm/sec are shown below (pd386 to pd395, pd452 to pd454, and pd482 to pd487).

[Reference Example 3-5] Membrane permeability of cyclic peptide compounds comprising Ser(Et-2-NMe2) or Gln(Ms)

[Table 27]

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd50 | D-Val | MePhe | Ser(Et-2-NMe2) | MeLeu | Thr | nPrGly | MeLeu | Ser(tBu) | MePhe | MeVal | Asp | pip | 1.21 | 1.5E-07 |
| pd51 | D-Leu | MePhe | Leu | MeLeu | Thr | nBuGly | MeLeu | Ser(Et-2-NMe2) | MePhe | MeVat | Asp | pip | 1.36 | 7.0E-08 |
| pd52 | D-Val | MePhe | Leu | MeLeu | Thr | nPrGly | MeLeu | Ser(Et-2-NMe2) | MePhe | MeVal | Asp | pip | 1.26 | 1.2E-07 |
| pd53 | D-Val | MePhe | Ser(Et-2-NMe2) | MeLeu | Thr | nBuGly | MeLeu | Ser(tBu) | MePhe | MeVal | Asp | pip | 1.26 | 1.4E-07 |
| pd54 | gMeAbu | MeLeu | Leu | MePhe | MePhe | Ser(Et-2-NMe2) | MeLeu | Thr | MeLeu | MeLeu | Asp | pip | 1.22 | 7.3E-08 |
| pd55 | gMeAbu | MeLeu | Ser(Et-2-NMe2) | MePhe | MePhe | Leu | MeLeu | Thr | MeLeu | MeLeu | Asp | pip | 1.22 | 1.0E-07 |
| pd56 | MeAla | Ser(Et-2-NMe2) | MeLeu | MePhe | Leu | MePhe | MeLeu | Thr | nBuGly | MeVal | Asp | pip | 1.28 | 1.2E-07 |
| pd57 | MeAla | Leu | MeLeu | MePhe | Ser(Et-2-NMe2) | MePhe | MeLeu | Thr | nPrGly | MeVal | Asp | pip | 1.23 | 1.2E-07 |
| pd58 | nBuGly | MePhe | Ser(Et-2-NMe2) | MeLeu | Thr | MeGly | MePhe | MeAbu | Ser (iPen) | MeLeu | Asp | pip | 1.22 | 2.1E-08 |
| pd59 | D-Leu | MePhe (3-Cl) | Gln(Ms) | MeLeu | Thr | nBuGly | MeLeu | Leu | MePhe | MeVal | Asp | pip | 1.23 | <8.8E-09 |
| pd60 | D-Leu | MePhe (3-Cl) | Leu | MeLeu | Thr | nBuGly | MeLeu | Gln(Ms) | MePhe (3-Cl) | MeVal | Asp | pip | 1.29 | 1.0E-08 |
| pd61 | D-Leu | MePhe (3-Cl) | Leu | MeAbu | Thr | nBuGly | MeLeu | Gln(Ms) | MePhe (3-Cl) | MeVal | Asp | pip | 1 21 | <9.4E-08 |
| pd62 | MeLeu | Gln(Ms) | MeLeu | MePhe | Leu | MePhe | MeLeu | Thr | nBuGly | MeVal | Asp | pip | 1.22 | <1.3E-07 |
| pd63 | MeAbu | Gln(Ms) | MeLeu | MePhe (3-Cl) | Leu | MePhe | MeLeu | Thr | nBuGly | MeVal | Asp | pip | 1.20 | <1.0E-07 |

(continued)

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd64 | MeLeu | Leu | MeAbu | MePhe | Gln(Ms) | MePhe (3-Cl) | MeLeu | Thr | nBuGly | MeLeu | Asp | pip | 1.25 | 1.5E-07 |
| pd65 | nBuGly | MePhe | Gln(Ms) | MeLeu | Thr | nBuGly | MePhe | MeLeu | Leu | MeLeu | Asp | pip | 1.24 | 6.0E-0B |
| pd66 | nBuGly | MePhe (3-Cl) | Gln(Ms) | MeLeu | Thr | nBuGly | MePhe | MeLeu | Leu | MeLeu | Asp | pip | 1.30 | 9.0E-08 |
| pd67 | D-Val | MePhe | Gln(Ms) | MeLeu | Leu | nBuGly | MeLeu | Ser(tBu) | MePhe | MeVal | Asp | pip | 1.26 | 6.0E-08 |
| pd68 | gMeAbu | MeLeu | Leu | MePhe | MePhe | Gln(Ms) | MeLeu | Leu | MeLeu | MeLeu | Asp | pip | 1.22 | 1.0E-07 |
| pd69 | MeAbu | Leu | MeLeu | MePhe | Gln(Ms) | MePhe | MeLeu | Leu | nBuGly | MeVal | Asp | pip | 1.33 | <1.4E-07 |
| pd70 | nBuGly | MePhe | Gln(Ms) | MeLeu | Leu | MeGly | MePhe | MeLeu | Leu | MeLeu | Asp | pip | 1.28 | 1.7E-07 |

Structures of cyclic peptides pd50 to pd70 are shown below. (In the structural formulae of the cyclic peptides, hydrogen atoms attached to a heteroatom may be omitted to clarify the structure.)

[Table 28]

| ID | Structural Formula | ID | Structural Formula |
|---|---|---|---|
| pd50 | 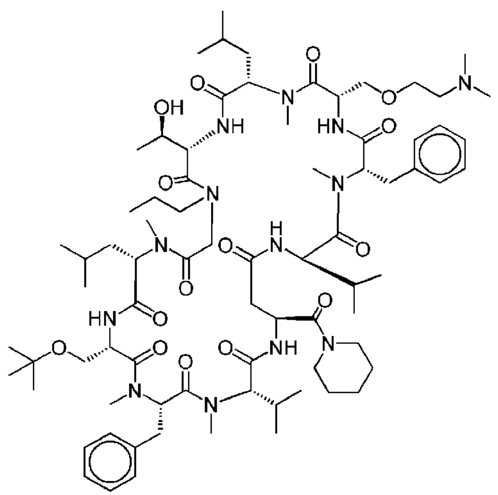 | pd51 | 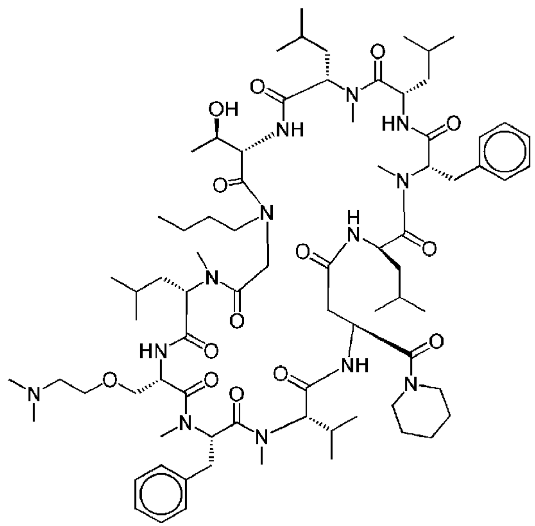 |
| pd52 | 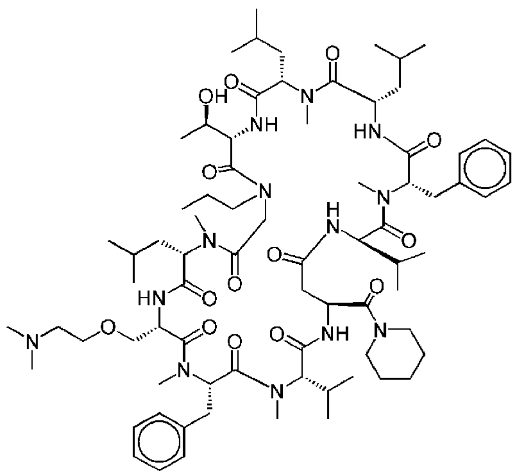 | pd53 | 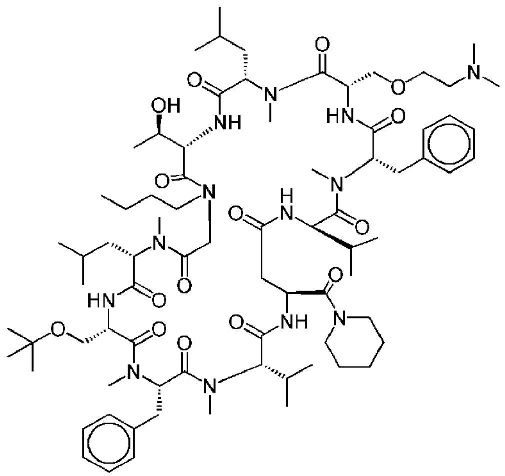 |

(continued)

| ID | Structural Formula | ID | Structural Formula |
|---|---|---|---|
| pd54 | 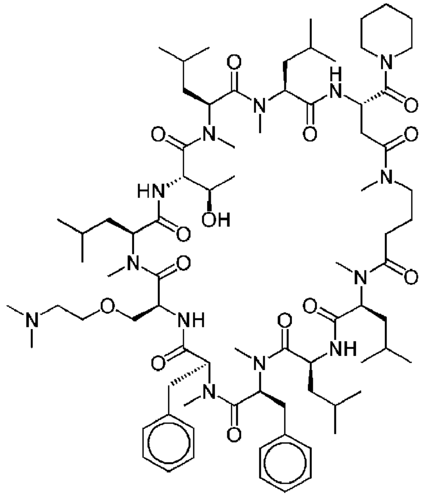 | pd55 | 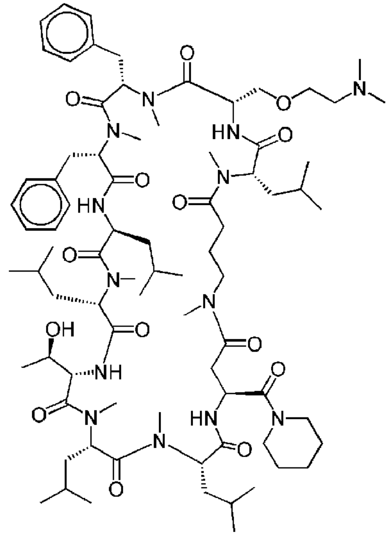 |
| pd56 | 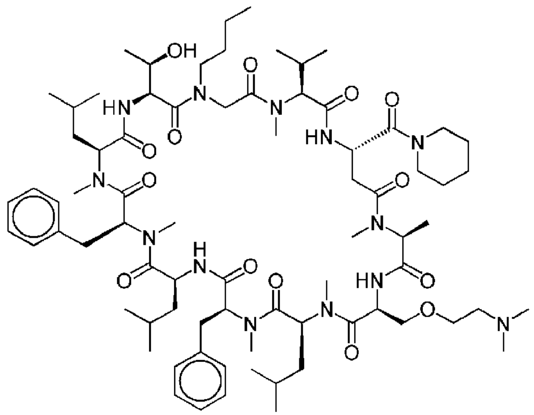 | pd57 | 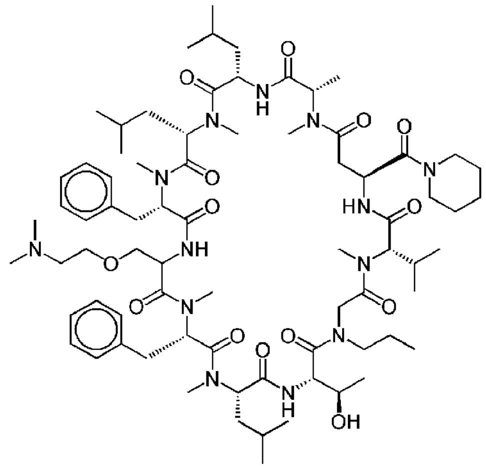 |

(continued)

| ID | Structural Formula | ID | Structural Formula |
|---|---|---|---|
| pd58 | 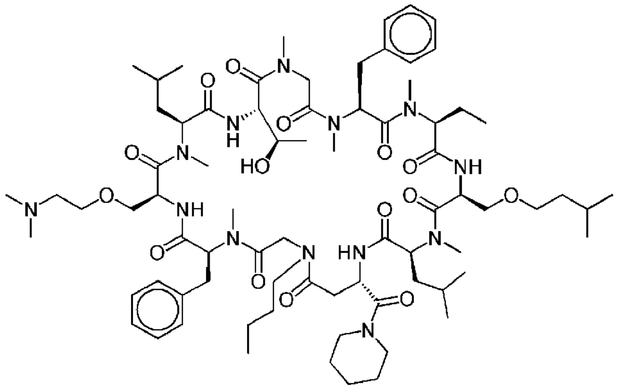 | pd59 | 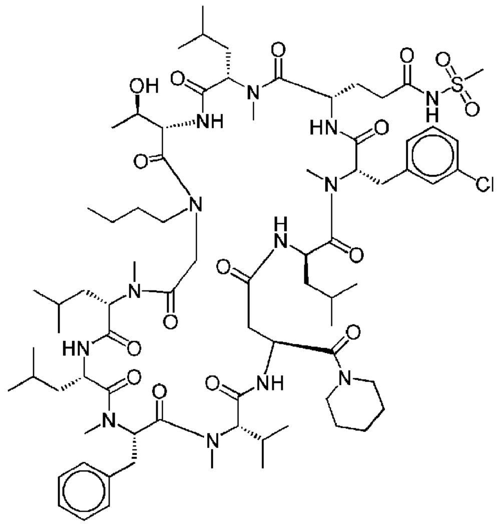 |
| pd60 | 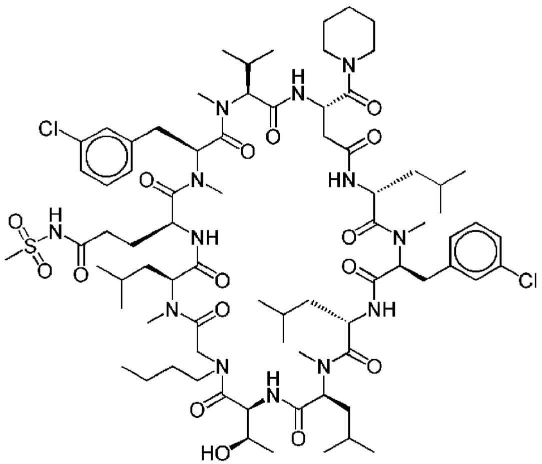 | pd61 | 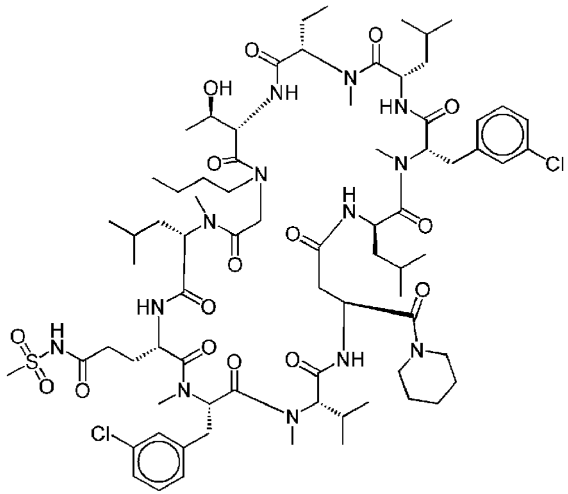 |

(continued)

| ID | Structural Formula | ID | Structural Formula |
|---|---|---|---|
| pd62 | 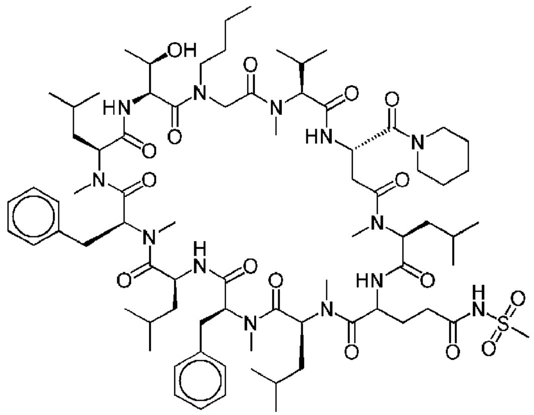 | pd63 | 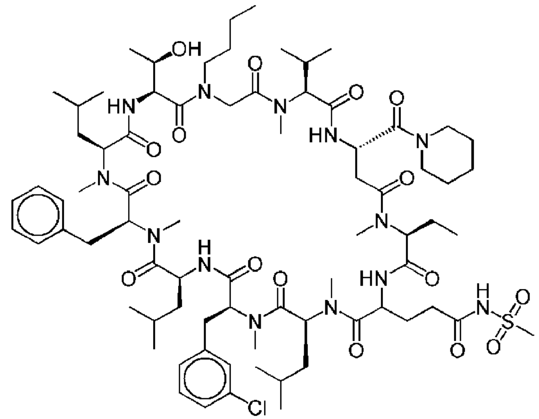 |
| pd64 | 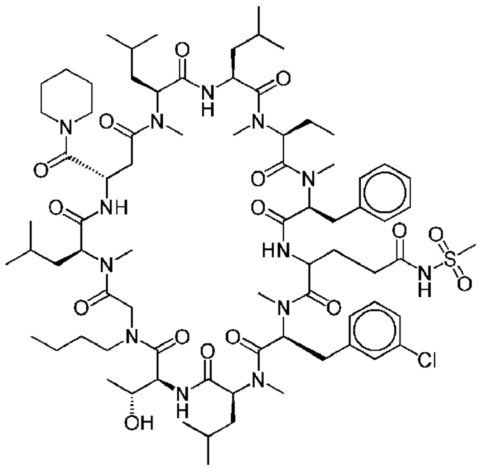 | pd65 | 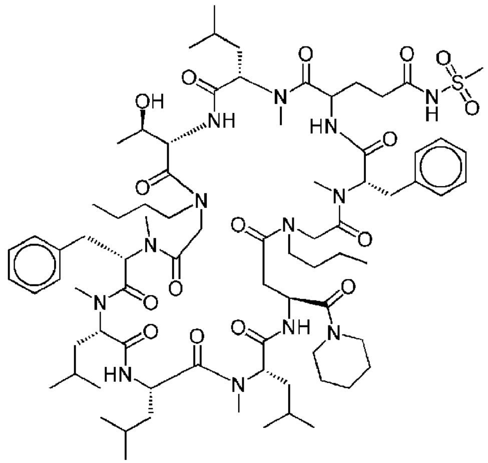 |

(continued)

| ID | Structural Formula | ID | Structural Formula |
|---|---|---|---|
| pd66 | 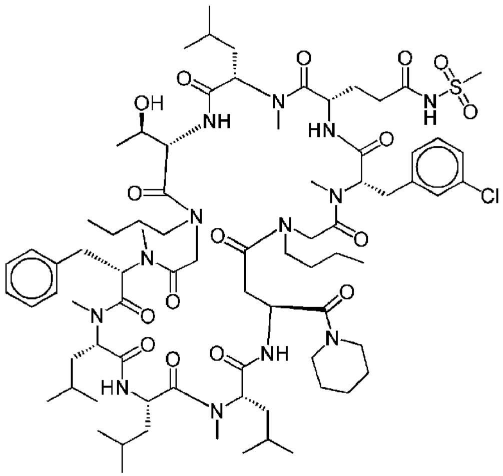 | pd67 | 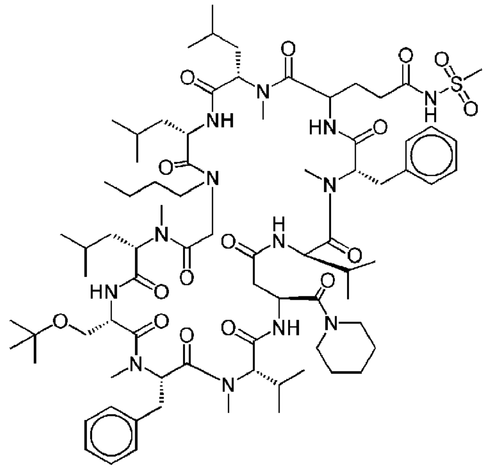 |
| pd68 | 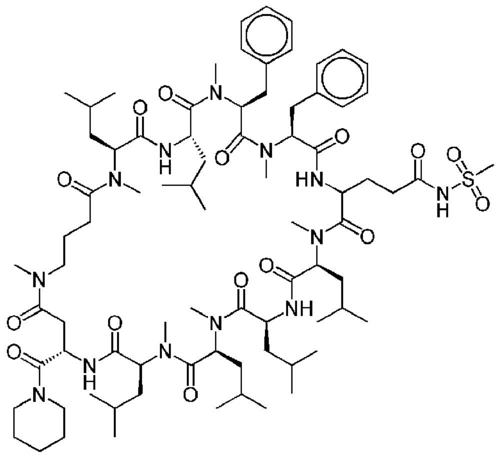 | pd69 | 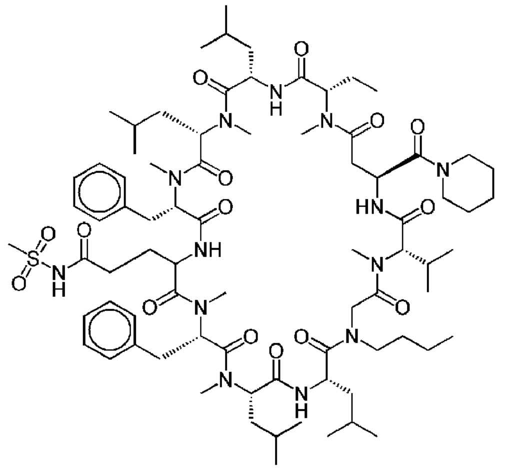 |

(continued)

| ID | Structural Formula | ID | Structural Formula |
|---|---|---|---|
| pd70 | 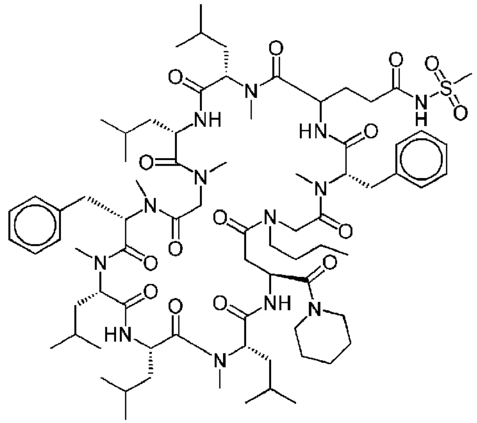 | | |

[Reference Example 3-6] Examples of cyclic peptide compounds comprising MeAbu(pip-4-F2), MeAbu(Mor), Ser(Et-2-Mor), MeAbu(pip-3-F2) or Abu(5-Oxo-Odz), which achieved $P_{app} \geqq 1.0 \times 10^{-6}$cm/sec

[Table 29]

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd386 | D-Ala | MePhe | Leu | MeAbu (pip-3-F2) | Thr | nBuGly | MeLeu | Ser(tBu) | MePhe | MeVal | Asp | pip | 1.26 | 2.3E-06 |
| pd387 | D-Val | MePhe | Leu | MeLeu | Thr | MeGly | MeAbu (pip-3-F2) | Ser(tBu) | MePhe | MeVal | Asp | pip | 1.19 | 3.5E-06 |
| pd388 | D-Ala | MePhe | Leu | MeLeu | Thr | nBuGly | MeVal | Leu | MePhe | MeAbu (pip-3-F2) | Asp | pip | 1.31 | 1.9E-06 |
| pd389 | D-Val | MeAbu (pip-3-F2) | Leu | MePhe | Thr | MeGly | MeLeu | Leu | MePhe (3-Cl) | MeAla | Asp | pip | 1.22 | 1.1E-06 |
| pd390 | g-MeAbu | MeLeu | Leu | MePhe | MePhe | Leu | MeLeu | Thr | MeAbu (pip-3-F2) | MeAla | Asp | pip | 1.17 | 2.0E-06 |
| pd391 | MeAla | Leu | MeLeu | MePhe | Leu | MePhe | MeAbu (pip-3-F2) | Thr | MeGly | MeVal | Asp | pip | 1.21 | 2.4E-06 |
| pd392 | MeAla | Leu | MeLeu | MePhe | Leu | MePhe | MeAla | Thr | nBuGly | MeAbu (pip-3-F2) | Asp | pip | 1.27 | 3.5E-06 |
| pd393 | MeGly | MePhe | Leu | MeLeu | Thr | MeGly | MePhe | MeAbu (pip-3-F2) | Ser (iPen) | MeLeu | Asp | pip | 1.22 | 2.8E-06 |
| pd394 | D-Val | MePhe | Leu | MeAbu (pip-4-F2) | Thr | nBuGly | MeLeu | Leu | MePhe | MeAla | Asp | pip | 1.22 | 1.8E-06 |

(continued)

| Compound ID | 11 | 10 | 9 | 8 | 7 | 6 | 5 | 4 | 3 | 2 | 1 | C-term | cLogP/ total AA | Caco-2 |
|---|---|---|---|---|---|---|---|---|---|---|---|---|---|---|
| pd395 | nBuGly | MePhe | Leu | MeLeu | Thr | MeGly | MePhe | MeAbu (pip-4-F2) | Leu | MeVal | Asp | pip | 1.24 | 1.7E-06 |
| pd452 | D-Leu | MePhe | Leu | MeAbu (Mor) | Thr | nBuGly | MeLeu | Ser(tBu) | MePhe | MeVal | Asp | pip | 1.25 | 1.3E-06 |
| pd453 | MeAla | Leu | MeLeu | MePhe | Leu | MePhe | MeAbu (Mor) | Thr | nBuGly | MeVal | Asp | pip | 1.22 | 2.3E-06 |
| pd454 | MeAla | Leu | MeLeu | MePhe | Leu | MePhe | MeLeu | Thr | nBuGly | MeAbu (Mor) | Asp | pip | 1.27 | 1.2E-06 |
| pd482 | MeAbu | Leu | MeLeu | MePhe | Ser(Et-2-Mor) | MeAbu | MeLeu | Thr | nBuGly | MeLeu | Asp | pip | 1.29 | 1.1E-06 |
| pd483 | MeAbu | Ser(Et-2-Mor) | MeLeu | MeAbu | Val | MePhe | MeLeu | Thr | nBuGly | MeLeu | Asp | pip | 1.24 | 1.9E-06 |
| pd484 | MeGly | MeLeu | Ser(Et-2-Mor) | MeLeu | Thr | nBuGly | MePhe | MeLeu | Val | MeLeu | Asp | pip | 1.23 | 1.8E-06 |
| pd485 | gMeAbu | MeLeu | Ile | MePhe | MeAbu | Ser (tBu) | MeLeu | Ser(Et-2-Mor) | MeLeu | MeLeu | Asp | pip | 1.28 | 1.2E-06 |
| pd486 | MeGly | MeAbu | Val | MeLeu | Ser(Et-2-Mor) | nBuGly | MePhe | MeLeu | Leu | MeLeu | Asp | pip | 1.34 | 1.1E-06 |
| pd487 | MeAbu | Leu | MeLeu | MePhe | Abu(5-Oxo-Odz) | MeLeu | MeLeu | Thr | nBuGly | MeLeu | Asp | pip | 1.26 | 2.2E-06 |

Structures of cyclic peptides pd386 to pd395, pd452 to pd454 and pd482 to 487 are shown below. (In the structural formulae of the cyclic peptides, hydrogen atoms attached to a heteroatom may be omitted to clarify th structure.)

| ID | Structural Formula | ID | Structural Formula |
|---|---|---|---|
| pd386 | 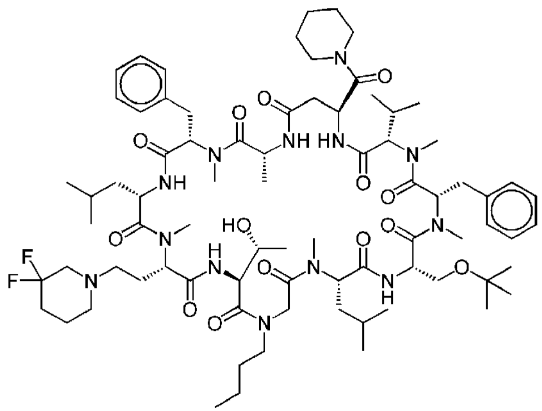 | pd387 | 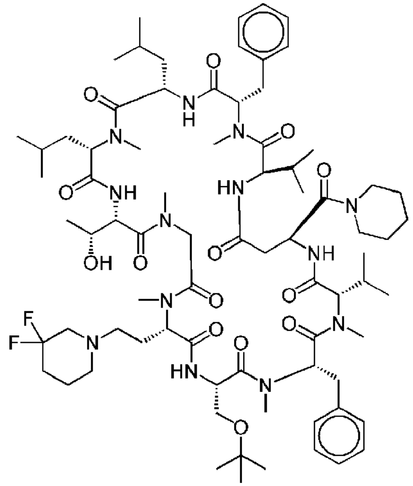 |
| pd388 | 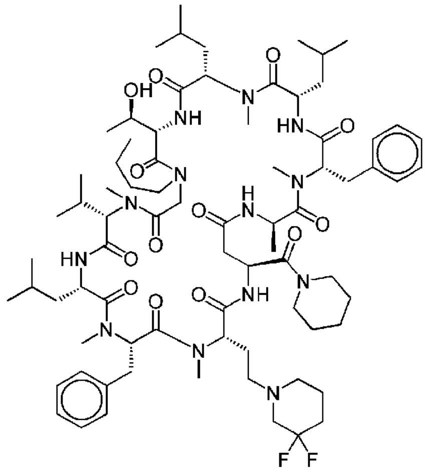 | pd389 | 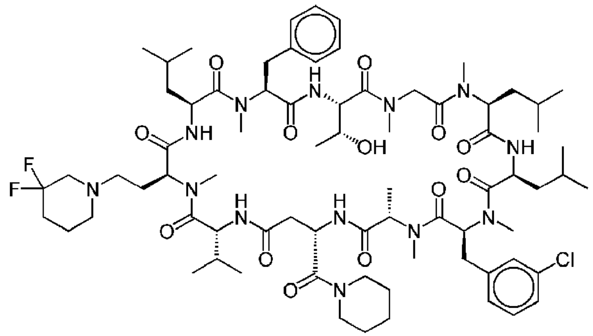 |

(continued)

| ID | Structural Formula | ID | Structural Formula |
|---|---|---|---|
| pd390 | 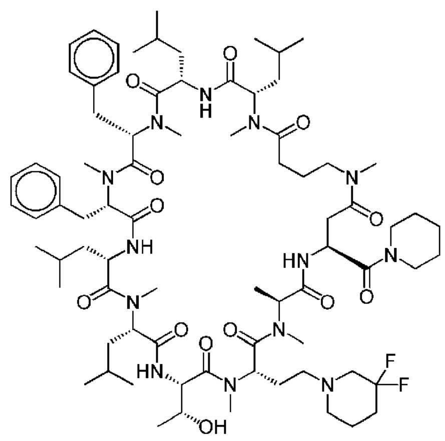 | pd391 | 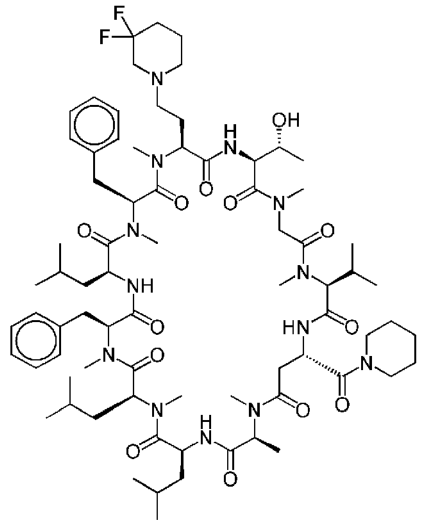 |
| pd392 | 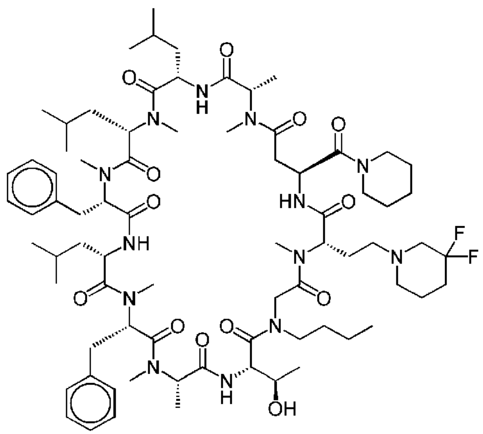 | pd393 | 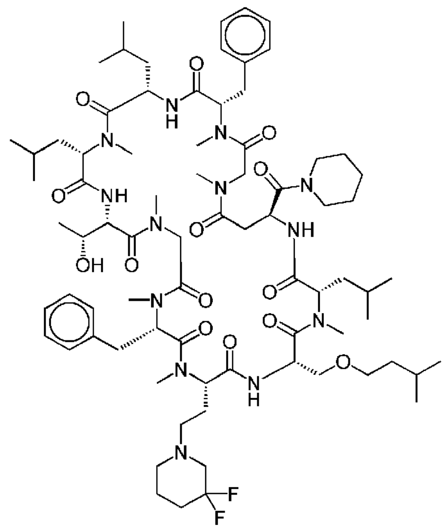 |

(continued)

| ID | Structural Formula | ID | Structural Formula |
|---|---|---|---|
| pd394 | 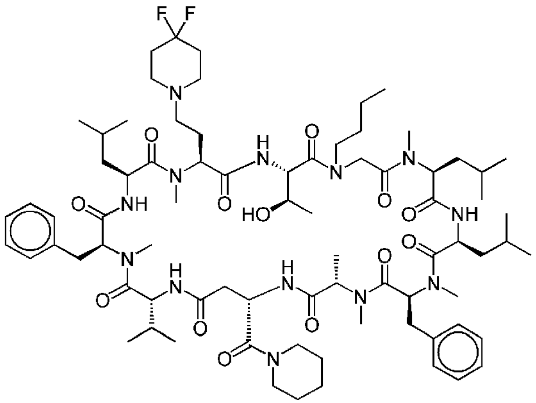 | pd395 | 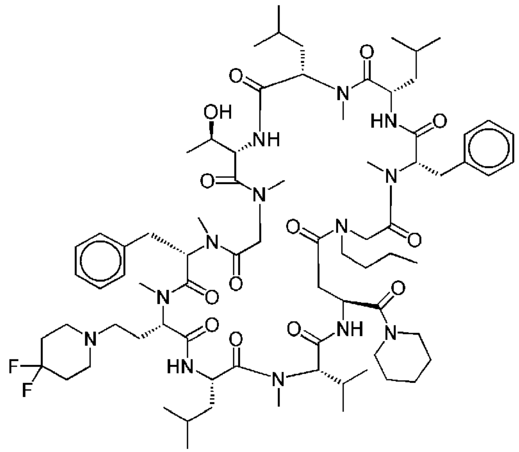 |
| pd452 | 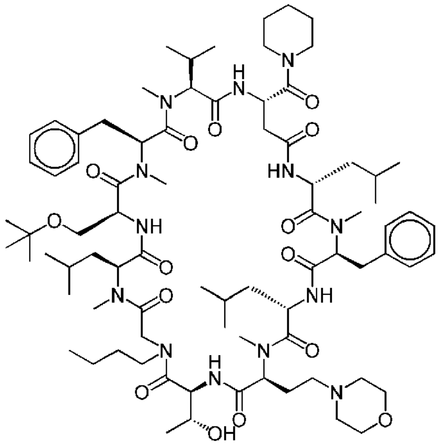 | pd453 | 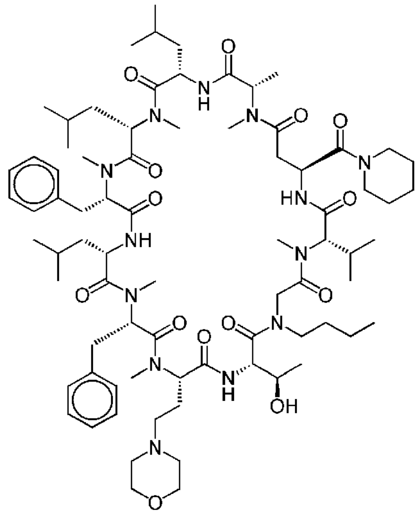 |

(continued)

| ID | Structural Formula | ID | Structural Formula |
|---|---|---|---|
| pd454 | 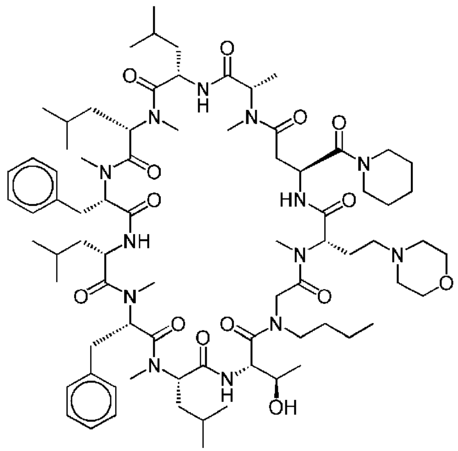 | pd482 | 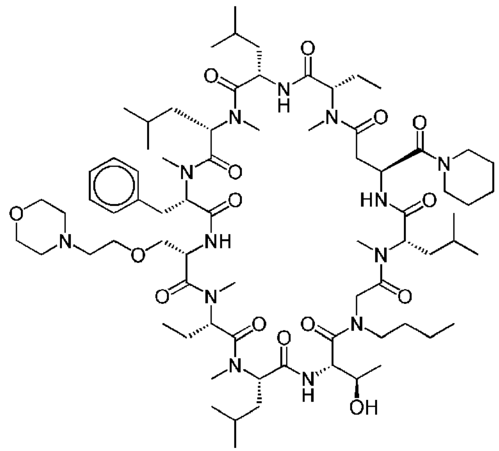 |
| pd483 | 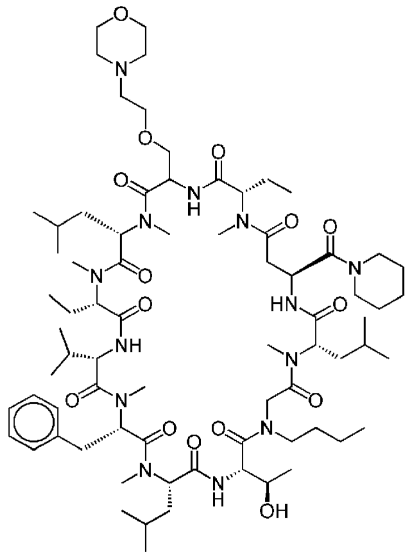 | pd484 | 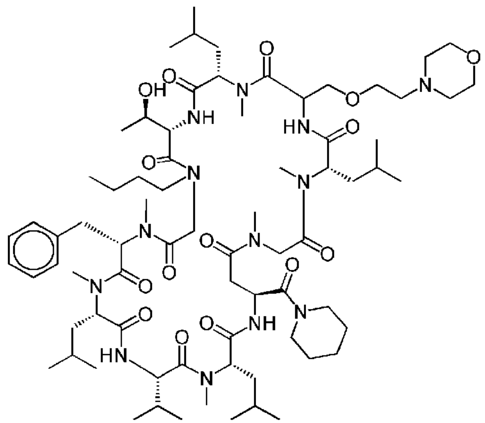 |

(continued)

| ID | Structural Formula | ID | Structural Formula |
| --- | --- | --- | --- |
| pd485 | 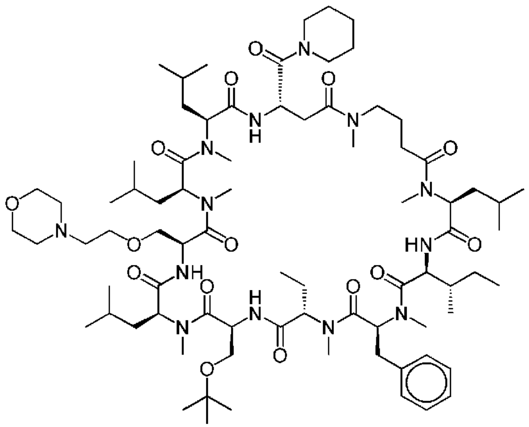 | pd486 | 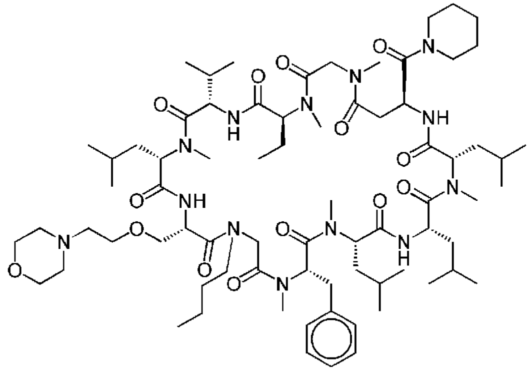 |
| pd487 | 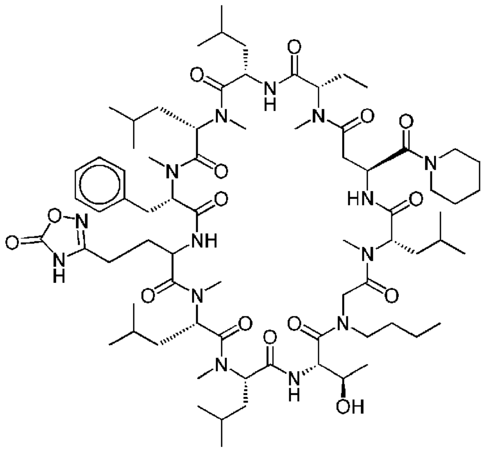 | | |

[Reference Example 3-7] Syntheses of Fmoc amino acids

Fmoc-Ser(Et-2-NMe2)-OH (Compound tm01), Fmoc-MeAbu(pip-4-F2)-OH (Compound tm04), Fmoc-MeAbu(pip-3-F2)-OH (Compound tm10), Fmoc-MeAbu(Mor)-OH (Compound tm19), Fmoc-Sert(Et-2-Mor)-OH (Compound tm20), Fmoc-Abu(5-Oxo-Odz)-OH (Compound tm21), and Fmoc-Gln(Ms)-OH (Compound tm22), which where used to synthesize three residue-peptides used to measure pKa and cyclic peptides whose membrane permeability was evaluated, were synthesized as follows.

Synthesis of N-(((9H-fluoren-9-yl)methoxy)carbonyl)-O-(2-(dimethylamino)ethyl)-L-serine (Compound tm01, Fmoc-Ser(Et-2-NMe2)-OH)

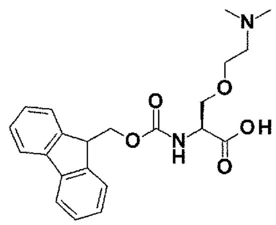

To commercially available trityl-L-serine (Trt-Ser-OH) triethylamine salt (5 g, 11.15 mmol) was added dimethylformamide (DMF) (40 mL) under a nitrogen atmosphere, after which sodium tert-pentoxide (7.36 g, 66.9 mmol) was added at room temperature and the mixture was stirred for 30 min. To the reaction solution was added 2-chloro-N,N-dimethylethan-1-amine hydrochloride (4.01 g, 27.9 mmol) at room temperature, and the mixture was stirred overnight. To the reaction solution was added formic acid (6.41 mL, 167 mmol), and purification by reverse phase column chromatography (10 mM aqueous ammonium acetate solution/methanol) gave O-(2-(dimethylamino)ethyl)-N-trityl-L-serine (Trt-Ser(Et-2-NMe2)-OH) (4.1 g).

To the resulting O-(2-(dimethylamino)ethyl)-N-trityl-L-serine (Trt-Ser(Et-2-NMe2)-OH) (4.1 g, 9.80 mmol) was added dichloromethane (10 mL), after which a 4N hydrochloric acid/1,4-dioxane solution (40 mL, 160 mmol) and water (4 mL) were added and the mixture was stirred at room temperature for 3 h. Water (80 mL) was added to the reaction solution, which was then washed with hexane twice. To the resulting aqueous layer were added sodium carbonate (26.0 g, 245 mmol), 1,4-dioxane (120 mL), and N-(9-fluorenylmethoxycarbonyloxy)succinimide (Fmoc-OSu) (3.97 g, 11.76 mmol) at 0°C, and the mixture was stirred at room temperature for 2 h. Formic acid (11.28 mL, 294 mmol) was added to the reaction solution, and the 1,4-dioxane was evaporated under reduced pressure. The resulting aqueous layer was purified by reverse phase column chromatography (0.1% aqueous formic acid solution/0.1% formic acid-acetonitrile solution) to afford N-(((9H-fluoren-9-yl)methoxy)carbonyl)-O-(2-(dimethylamino)ethyl)-L-serine (Compound tm01, Fmoc-Ser(Et-2-NMe2)-OH) formate (3.07 g, 69% over three steps). To the resulting N-(((9H-fluoren-9-yl)methoxy)carbonyl)-O-(2-(dimethylamino)ethyl)-L-serine (Compound tm01, Fmoc-Ser(Et-2-NMe2)-OH) formate (3.0 g, 7.53 mmol) were added dichloromethane (4 mL) and a 4N hydrochloric acid/1,4-dioxane solution (9.411 mL, 37.6 mmol), and the mixture was stirred at room temperature for 20 min. The reaction solution was concentrated under reduced pressure to afford N-(((9H-fluoren-9-yl)methoxy)carbonyl)-O-(2-(dimethylamino)ethyl)-L-serine (Compound tm01, Fmoc-Ser(Et-2-NMe2)-OH) hydrochloride (2.7 g, 82%).
LCMS (ESI) m/z = 399 (M+H)+

Retention time: 0.50 min (analytical condition SQDFA05)

Synthesis of tert-butyl (S)-2-((((9H-fluoren-9-yl)methoxy)carbonyl)amino)-4-(4,4-difluoropiperidin-1-yl)-4-oxobutanoate (Compound tm02, Fmoc-Asp(pip-4-F2)-OtBu)

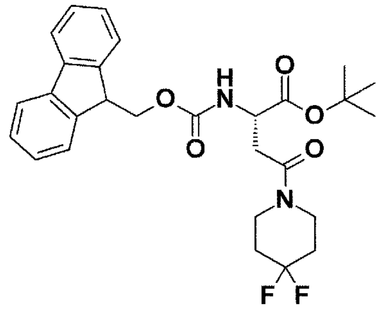

To a solution of 1-(3-dimethylaminopropyl)-3-ethylcarbodiimide hydrochloride (WSC·HCl) in N,N-dimethylformamide (12 mL) were added 1-hydroxybenzotriazole (HOBt) (723 mg, 5.35 mmol) and commercially purchased (S)-3-((((9H-fluoren-9-yl)methoxy)carbonyl)amino)-4-(tert-butoxy)-4-oxobutanoic acid (Fmoc-Asp-OtBu) (2 g, 4.86 mmol) at 0°C under a nitrogen atmosphere, and the mixture was stirred for 1 h. To the reaction solution was added a solution of 4,4-difluoropiperidine hydrochloride (843 mg, 5.35 mmol) and N-ethyl-N-isopropylpropan-2-amine (DIPEA) (931 μL, 5.35 mmol) in N,N-dimethylformamide (2 mL), and the mixture was stirred at 0°C for 3 h. Water was added to the reaction solution, and the mixture was extracted with ethyl acetate twice. The combined organic layers were washed with a 0.5 M aqueous hydrochloric acid solution, water, a 50% aqueous sodium bicarbonate solution, and 50% saline, and the resulting organic layers were concentrated under reduced pressure to afford tert-butyl (S)-2-((((9H-fluoren-9-yl)methoxy)carbonyl)amino)-4-(4,4-difluoropiperidin-1-yl)-4-oxobutanoate (Compound tm02, Fmoc-Asp(pip-4-F2)-OtBu) (2.635 g).
LCMS (ESI) m/z = 515 (M+H)+

Retention time: 0.97 min (analytical condition SQDFA05)

Synthesis of (S)-2-((((9H-fluoren-9-yl)methoxy)carbonyl)amino)-4-(4.4-difluoropiperidin-1-yl)butanoic acid (Compound tm03, Fmoc-Abu(pip-4-F2)-OH)

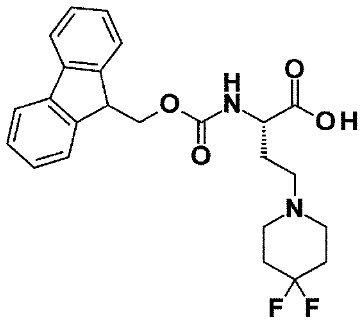

To a solution of triruthenium dodecacarbonyl (62 mg, 0.097 mmol) in tetrahydrofuran (5 mL) was added 1,1,3,3-tetramethyldisiloxane (2.76 mL, 15.55 mmol) under a nitrogen atmosphere, and the mixture was stirred at room temperature for 10 min. To the reaction solution was added tert-butyl (S)-2-((((9H-fluoren-9-yl)methoxy)carbonyl)amino)-4-(4,4-difluoropiperidin-1-yl)-4-oxobutanoate (Compound tm02, Fmoc-Asp(pip-4-F2)-OtBu) (1.0 g, 1.943 mmol) dissolved in tetrahydrofuran (7 mL). After stirring at 50°C for 3 h, the reaction solution was concentrated under reduced pressure. The resulting residue was dissolved in 2,2,2-trifluoroethanol (10 mL), trimethylchlorosilane (745 μL, 5.83 mmol) was added, and the mixture was stirred at room temperature for 1 h. After concentrating the reaction solution under reduced pressure, the resulting residue was dissolved in 1,4-dioxane (5 mL) and a 2N aqueous hydrochloric acid solution (10 mL) and the mixture was stirred at room temperature for 1 h. The reaction solution was extracted with tert-butyl methyl ether twice, and the organic layers were concentrated under reduced pressure. The resulting residue was purified by reverse phase column chromatography (0.1% aqueous formic acid solution/0.1% formic acid-acetonitrile solution) to afford (S)-2-((((9H-fluoren-9-yl)methoxy)carbonyl)amino)-4-(4,4-difluoropiperidin-1-yl)butanoic acid (Compound tm03, Fmoc-Abu(pip-4-F2)-OH) (540 mg, 63%).
LCMS (ESI) m/z = 445 (M+H)+

Retention time: 0.56 min (analytical condition SQDFA05)

Synthesis of (S)-2-((((9H-fluoren-9-yl)methoxy)carbonyl)(methyl)amino)-4-(4,4-difluoropiperidin-1-yl)butanoic acid (Compound tm04, Fmoc-MeAbu(pip-4-F2)-OH)

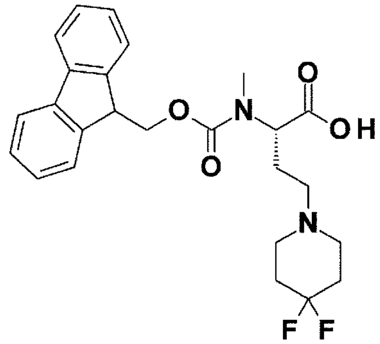

A solution of (S)-2-((((9H-fluoren-9-yl)methoxy)carbonyl)amino)-4-(4,4-difluoropiperidin-1-yl)butanoic acid (Compound tm03, Fmoc-Abu(pip-4-F2)-OH) (13 g, 9.25 mmol), paraformaldehyde (2.6 g, 28.86 mmol), and trifluoroacetic acid (30.59 g, 263.71 mmol) in toluene (60 mL) was stirred at room temperature for 16 h under a nitrogen atmosphere. The reaction solution was concentrated under reduced pressure, then dissolved in dichloromethane, and washed with a saturated aqueous sodium bicarbonate solution, water, and brine. The resulting organic layer was then dried over anhydrous sodium sulfate, filtered, and concentrated under reduced pressure. The resulting residue was purified by normal phase silica gel column chromatography (petroleum ether/ethyl acetate) to afford an intermediate (9H-fluoren-9-yl)methyl (S)-4-(2-(4,4-difluoropiperidin-1-yl)ethyl)-5-oxooxazolidine-3-carboxylate (10.9 g, 82%). The resulting intermediate (6 g, 13.14 mmol) was dissolved in trifluoroacetic acid (65 mL) and dichloroethane (65 mL), triethylsilane (13.5 g, 116.1 mmol) was added at room temperature, and the mixture was stirred at 70°C for 4 h. The reaction solution was brought back to room temperature and then concentrated under reduced pressure, and the resulting residue was dissolved in dichloromethane. The organic layer was washed with water, then dried over anhydrous sodium sulfate, filtered, and concentrated under reduced pressure. The resulting residue was purified by reverse phase column chromatography (0.5% aqueous hydrochloric acid solution/0.5% hydrochloric acid-acetonitrile solution) to afford (S)-2-((((9H-fluoren-9-yl)methoxy)carbonyl)(methyl)amino)-4-(4,4-difluoropiperidin-1-yl)butanoic acid (Compound tm04, Fmoc-MeAbu(pip-4-F2)-OH) (4.0 g, 66%).
LCMS (ESI) m/z = 459 (M+H)+

Retention time: 2.71 min (analytical condition SMD method 28)

Synthesis of 1-benzyl-3,3-difluoropiperidine (Compound tm05)

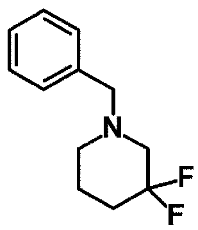

To a solution of 1-benzylpiperidin-3-one (60 g, 317.03 mmol) in dichloromethane was added dimethylaminosulfur trifluoride (DAST) (150 g, 4.04 mol) at 0°C under a nitrogen atmosphere, and the mixture was stirred for 2 h. To the reaction solution was then added 300 mL of a saturated aqueous sodium bicarbonate solution, the mixture was extracted with ethyl acetate three times, and the combined organic layers were washed with brine. The resulting organic layer was dried over anhydrous sodium sulfate, then filtered, and concentrated under reduced pressure to afford a crude product 1-benzyl-3,3-difluoropiperidine (Compound tm05) (31 g, 46%).

LCMS (ESI) m/z = 212 (M+H)+

Retention time: 0.94 min (analytical condition SMD method 31)

Synthesis of benzyl (S)-2-(((benzyloxy)carbonyl)(methyl)amino-4-(3,3-difluoropiperidin-1-yl)butanoate (Compound tm07, Cbz-MeAbu(pip-3-F2)-OBn)

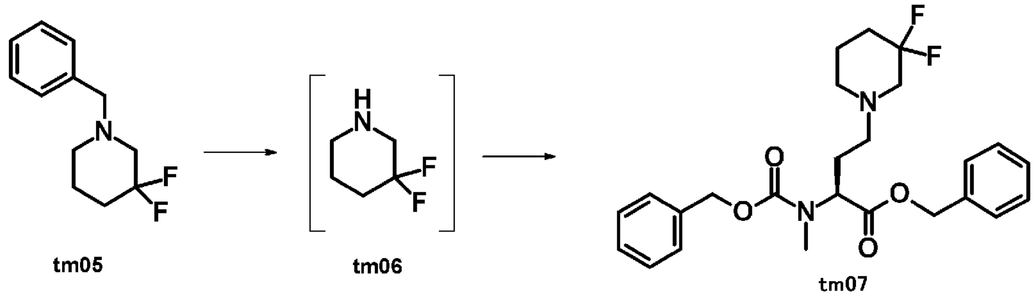

tm05 tm06 tm07

The resulting crude product 1-benzyl-3,3-difluoropiperidine (Compound tm05) was dissolved in 200 mL of methanol, 10% palladium/carbon (1 g) was added, and the mixture was stirred at room temperature for 48 h under a hydrogen atmosphere. The reaction solution was filtered and the resulting filtrate was concentrated under reduced pressure to afford 3,3-difluoropiperidine (Compound tm06) (19 g) as a crude product.

To a solution of separately synthesized benzyl (S)-2-(((benzyloxy)carbonyl)(methyl)amino)-4-(ethylthio)-4-oxobutanoate (Compound tm08, Cbz-MeAsp(SEt)-OBn) (20 g, 48.13 mmol), 3,3-difluoropiperidine (Compound tm06) (8.26 g, 68.19 mmol), triethylsilane (40 mL), and 10% palladium/carbon (2.6 g) in N,N-dimethylformamide (20 mL) was added sodium triacetoxyborohydride ($NaBH(OAc)_3$) (20.4 g, 204 mmol) at 0°C, and the mixture was stirred at room temperature for 30 min. The reaction solution was filtered and concentrated under reduced pressure, and the resulting crude product was purified by normal phase silica gel chromatography (hexane/ethyl acetate) to afford benzyl (S)-2-(((benzyloxy)carbonyl)(methyl)amino)-4-(3,3-difluoropiperidin-1-yl)butanoate (Compound tm07, Cbz-MeAbu(pip-3-F2)-OBn) (11 g, 50%).
LCMS (ESI) m/z = 461 (M+H)+

Retention time: 1.42 min (analytical condition SMD method 32)

Synthesis of (S)-2-((((9H-fluoren-9-yl)methoxy)carbonyl)(methyl)amino)-4-(3,3-difluoropiperidin-1-yl)butanoic acid (Compound tm10, Fmoc-MeAbu(pip-3-F2)-OH)

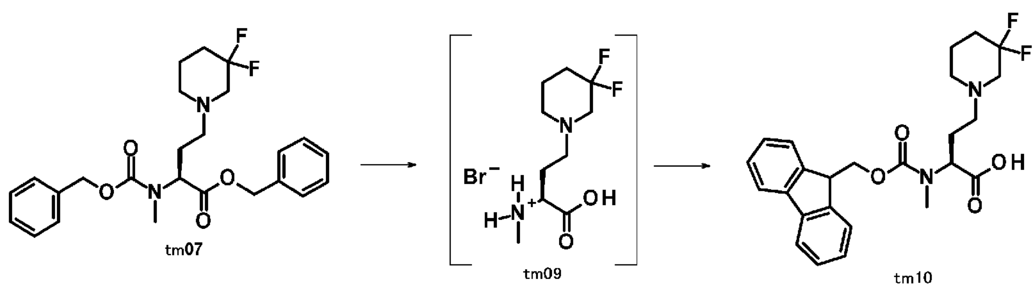

tm07 tm09 tm10

Benzyl (S)-2-(((benzyloxy)carbonyl)(methyl)amino)-4-(3,3-difluoropiperidin-1-yl)butanoate (Compound tm07, Cbz-MeAbu(pip-3-F2)-OBn) (20 g, 43.43 mmol) was dissolved in a 33% hydrogen bromide-acetic acid solution, and the reaction solution was stirred at room temperature for 2 h and then stirred at 50°C for a further 1 h. The reaction solution was concentrated under reduced pressure, and the resulting residue was precipitated by diethyl ether to afford (2S)-4-(3,3-difluoropiperidin-1-yl)-2-(methylamino)butanoic acid bromide (Compound tm09) (29 g) as a crude product.

The above crude product (2S)-4-(3,3-difluoropiperidin-1-yl)-2-(methylamino)butanoic acid bromide (Compound tm09) (7.08 g, 29.97 mmol) and N-(9-fluorenylmethoxycarbonyloxy)succinimide (Fmoc-OSu) (16 g, 47.48 mmol) were

dissolved in a solution of potassium carbonate in water/1,4-dioxane = 1/1 (300 mL), and the reaction solution was stirred at room temperature for 4 h. The reaction solution was then washed with diethyl ether three times, and the aqueous layer was adjusted to pH 3 with hydrochloric acid and extracted with ethyl acetate twice. The resulting organic layers were combined, dried over anhydrous sodium sulfate, filtered, and concentrated under reduced pressure, and the resulting residue was purified by reverse phase column chromatography (10 mmol aqueous ammonium bicarbonate solution/acetonitrile) to afford (S)-2-((((9H-fluoren-9-yl)methoxy)carbonyl)(methyl)amino)-4-(3,3-difluoropiperidin-1-yl)butanoic acid (Compound tm10, Fmoc-MeAbu(pip-3-F2)-OH) (7.2 g, 71%).
LCMS (ESI) m/z = 459 (M+H)+

Retention time: 1.61 min (analytical condition SMD method 5)

<u>Synthesis of (S)-2-(((benzyloxy)carbonyl)(methyl)amino)-4-(ethylthio)-4-oxobutanoic acid (Compound tm13, Cbz-MeAsp(SEt)-OH)</u>

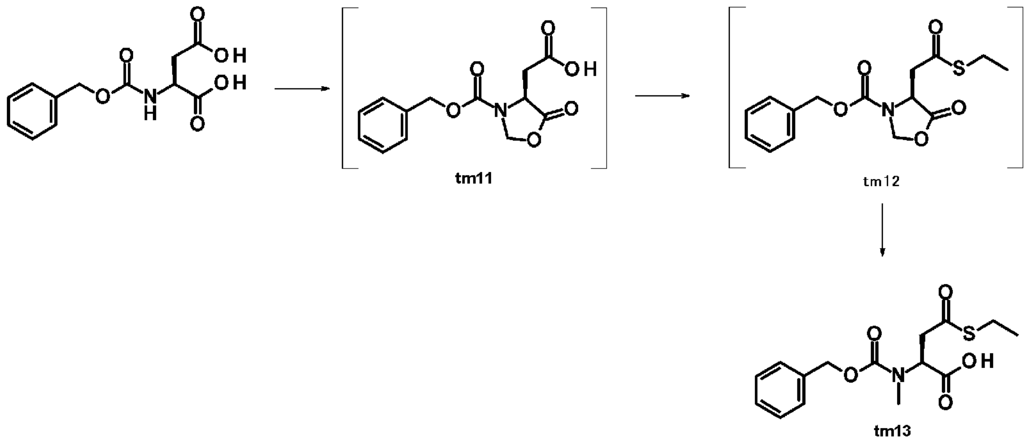

tm11

tm12

tm13

A solution of ((benzyloxy)carbonyl)-L-aspartic acid (Cbz-Asp-OH) (200 g, 748.41 mmol), paraformaldehyde (67.5 g, 749.35 mmol), and p-toluenesulfonic acid (7.74 g, 44.95 mmol) in toluene (2000 mL) was warmed to 110°C under a nitrogen atmosphere and stirred for three days. The reaction solution was brought to room temperature, water was then added, and the mixture was extracted with ethyl acetate three times. The combined organic layers were washed with brine, and the resulting organic layer was dried over anhydrous sodium sulfate, then filtered, and concentrated under reduced pressure to afford (S)-2-(3-((benzyloxy)carbonyl)-5-oxooxazolidin-4-yl)acetic acid (Compound tm11) as a crude product (200 g).

To a solution of a crude product of (S)-2-(3-((benzyloxy)carbonyl)-5-oxooxazolidin-4-yl)acetic acid (Compound tm11) synthesized by the same method as described above (265 g, 948.99 mmol), ethanethiol (88.3 g, 1.42 mol), and 4-dimethylaminopyridine (DMAP) (11.59 g, 95 mmol) in dichloromethane was added N,N'-dicyclohexylcarbodiimide (DCC) (214 g, 1.04 mol) at 0°C, and the mixture was stirred at room temperature for 5 h. The reaction solution was filtered, the resulting filtrate was washed with brine, and the resulting organic layer was dried over anhydrous sodium sulfate, then filtered, and concentrated under reduced pressure. The resulting crude product was purified by normal phase silica gel chromatography (petroleum ether/ethyl acetate) to afford a mixture of benzyl (S)-4-(2-(ethylthio)-2-oxoethyl)-5-oxooxazolidine-3-carboxylate (Compound tm12) (167 g).

To a solution of the obtained benzyl (S)-4-(2-(ethylthio)-2-oxoethyl)-5-oxooxazolidine-3-carboxylate (Compound tm12) (139 g, 1.20 mol) in dichloromethane (DCM) and trifluoroacetic acid (1500 mL/1500 mL) was added triethylsilane (139 g, 1.2 mol), and the mixture was stirred at room temperature for three days. The reaction solution was concentrated under reduced pressure, an aqueous potassium carbonate solution was added to the resulting residue, and the mixture was washed with diethyl ether three times. The resulting aqueous layer was adjusted to pH 3 with a 2N aqueous hydrochloric acid solution and extracted with ethyl acetate twice. The combined organic layers were washed with brine, dried over anhydrous sodium sulfate, then filtered, and concentrated under reduced pressure to afford a crude product of (S)-2-(((benzyloxy)carbonyl)(methyl)amino)-4-(ethylthio)-4-oxobutanoic acid (Compound tml3, Cbz-MeAsp(SEt)-OH) (75 g).
LCMS (ESI) m/z = 326 (M+H)+

Retention time: 1.00 min (analytical condition SMD method 16)

Synthesis of benzyl (S)-2-(((benzyloxy)carbonyl)(methyl)amino)-4-(ethylthio)-4-oxobutanoate (Compound tm08, Cbz-MeAsp(SEt)-OBn)

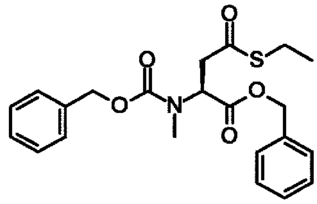

To a solution of the crude product of (S)-2-(((benzyloxy)carbonyl)(methyl)amino)-4-(ethylthio)-4-oxobutanoic acid (Compound tm13, Cbz-MeAsp(SEt)-OH) (3 g, 9.22 mmol) and potassium carbonate (1.9 g, 13.65 mmol) in N,N-dimethylformamide (46 mL) was added benzyl bromide (1.73 g, 10.11 mmol) at room temperature, and the mixture was stirred for 16 h and then filtered. The resulting filtrate was concentrated under reduced pressure, and the resulting residue was purified by normal phase silica gel chromatography (petroleum ether/ethyl acetate) to afford benzyl (S)-2-(((benzyloxy)carbonyl)(methyl)amino)-4-(ethylthio)-4-oxobutanoate (Compound tm08, Cbz-MeAsp(SEt)-OBn) (1.5 g). $^1$H NMR (300 MHz, $CDCl_3$): δ 7.36-7.31 (m, 10H), 5.19-4.89 (m, 5H), 3.34-2.85 (m, 7H), 1.30-1.21 (m, 3H)

(S)-2-((((9H-Fluoren-9-yl)methoxy)carbonyl)amino)-4-morpholinobutanoic acid (Compound tml7, Fmoc-Abu(Mor)-OH) was synthesized according to the following scheme.

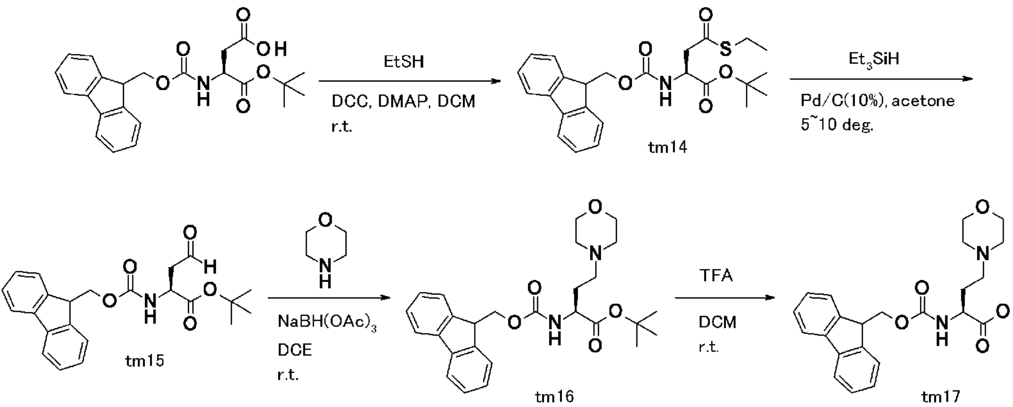


Synthesis of tert-butyl (S)-2-((((9H-fluoren-9-yl)methoxy)carbonyl)amino)-4-(ethylthio)-4-oxobutanoate (Compound tm14, Fmoc-Asp(SEt)-OtBu)

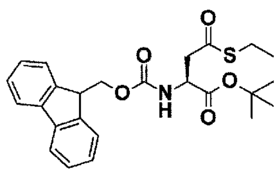

(S)-3-((((9H-Fluoren-9-yl)methoxy)carbonyl)amino)-4-(tert-butoxy)-4-oxobutanoic acid (Fmoc-Asp-OtBu) (5 g, 12.15 mmol), 4-dimethylaminopyridine (DMAP) (148 mg, 1.22 mmol), and ethanethiol (EtSH) (1.13 g, 18.19 mmol) were dissolved in dichloromethane (DCM) (50 mL), and N,N'-dicyclohexylcarbodiimide (DCC) (2.756 g, 1.337 mmol) was added under ice-cooling. After stirring at room temperature for 5 h, the solid was removed by filtration. The filtrate was diluted with dichloromethane (DCM), then washed with brine, dried over anhydrous sodium sulfate, then filtered, and concentrated under reduced pressure. The resulting residue was purified by normal phase column chromatography (0 to 40% ethyl acetate/petroleum ether) to afford tert-butyl (S)-2-((((9H-fluoren-9-yl)methoxy)carbonyl)amino)-4-(ethylthio)-4-oxobutanoate (Compound tml4, Fmoc-Asp(SEt)-OtBu) (3.9 g, 70%).
LCMS (ESI) m/z = 478 (M+Na)+

Retention time: 3.13 min (analytical condition SMD method 14)

Synthesis of tert-butyl (S)-2-((((9H-fluoren-9-yl)methoxy)carbonyl)amino)-4-oxobutanoate (Compound tm15)

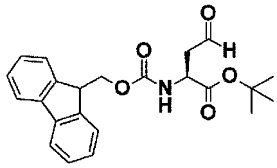

tert-Butyl (S)-2-((((9H-fluoren-9-yl)methoxy)carbonyl)amino)-4-(ethylthio)-4-oxobutanoate (Compound tm14, Fmoc-Asp(SEt)-OtBu) (1 g, 2.20 mmol) was dissolved in acetone (4.4 mL), and Pd/C (wet, 10%, 44 mg) was added. Triethylsilane (1.276 g, 10.97 mmol) was added under ice-cooling, and the mixture was stirred for 1 h. Pd/C was then removed by filtration and the filtrate was concentrated under reduced pressure. The resulting residue was purified by normal phase column chromatography (0 to 60% ethyl acetate/petroleum ether) to afford tert-butyl (S)-2-((((9H-fluoren-9-yl)methoxy)carbonyl)amino)-4-oxobutanoate (Compound tm15) (680 mg, 78%).
LCMS (ESI) m/z = 418 (M+Na)+

Retention time: 2.07 min (analytical condition SMD method 40)

Synthesis of tert-butyl (S)-2-((((9H-fluoren-9-yl)methoxy)carbonyl)amino)-4-morpholinobutanoate (Compound tm16, Fmoc-Abu(Mor)-OtBu)

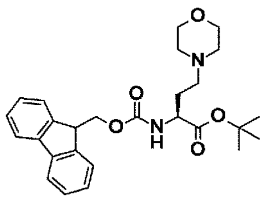

tert-Butyl (S)-2-((((9H-fluoren-9-yl)methoxy)carbonyl)amino)-4-oxobutanoate (Compound tml5) (1.6 g, 4.05 mmol) and morpholine (387 mg, 4.44 mmol) were dissolved in dichloroethane (DCE) (8 mL), and the reaction solution was stirred at room temperature for 1 h. A solution of sodium triacetoxyborohydride ($NaBH(OAc)_3$) (1.717 g, 8.10 mmol) in dichloroethane (DCE) (8 mL) was then added under ice-cooling, and the mixture was stirred for 4 h. The reaction solution was then diluted with dichloromethane and washed with a saturated aqueous sodium bicarbonate solution and brine, after which the organic layer was dried over anhydrous sodium sulfate, filtered, and then concentrated under reduced pressure. The resulting residue was purified by normal phase column chromatography (petroleum ether/ethyl acetate) to afford tert-butyl (S)-2-((((9H-fluoren-9-yl)methoxy)carbonyl)amino)-4-morpholinobutanoate (Compound tml6, Fmoc-Abu(Mor)-OtBu) (1.7 g, 98%).
LCMS (ESI) m/z = 467 (M+H)+

Retention time: 2.02 min (analytical condition SMD method 41)

Synthesis of (S)-2-((((9H-Fluoren-9-yl)methoxy)carbonyl)amino)-4-morpholinobutanoic acid (Compound tm17, Fmoc-Abu(Mor)-OH)

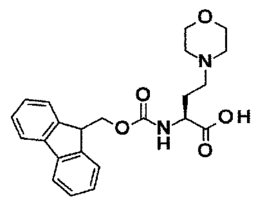

tert-Butyl (S)-2-((((9H-fluoren-9-yl)methoxy)carbonyl)amino)-4-morpholinobutanoate (Compound tm16, Fmoc-Abu(Mor)-OtBu) (9 g, 19.29 mmol) was dissolved in trifluoroacetic acid (TFA)/dichloroethane (DCE) (25 mL/25 mL), and the reaction solution was stirred at 40°C for 2 h. After cooling to room temperature, the reaction solution was diluted with dichloromethane and washed with water. The organic layer was dried over anhydrous sodium sulfate, then filtered, and concentrated under reduced pressure to afford (S)-2-((((9H-fluoren-9-yl)methoxy)carbonyl)amino)-4-morpholinobutanoic acid (Compound tm17, Fmoc-Abu(Mor)-OH) (7 g, 90%).
LCMS (ESI) m/z = 411 (M+H)+

Retention time: 1.20 min (analytical condition SMD method 42)

(S)-2-((((9H-Fluoren-9-yl)methoxy)carbonyl)(methyl)amino)-4-morpholinobutanoic acid (Compound tml9, Fmoc-MeAbu(Mor)-OH) was synthesized according to the following scheme.

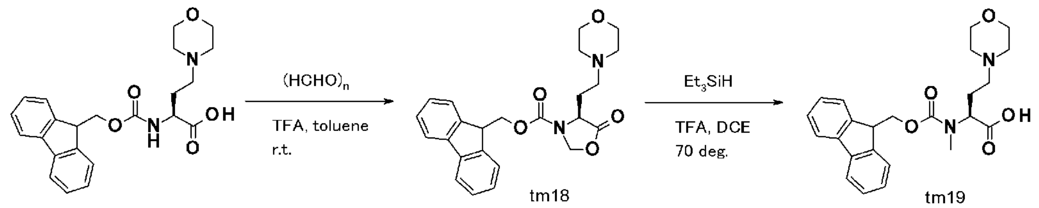


<u>Synthesis of (9H-fluoren-9-yl)methyl (S)-4-(2-morpholinoethyl)-5-oxooxazolidine-3-carboxylate (Compound tml8)</u>

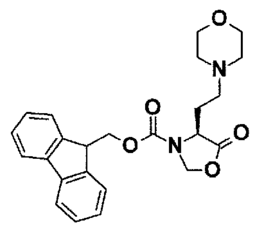

(S)-2-((((9H-Fluoren-9-yl)methoxy)carbonyl)amino)-4-morpholinobutanoic acid (Compound tml7, Fmoc-Abu(Mor)-OH) (600 mg, 1.46 mmol) and paraformaldehyde (131.4 mg, 1.46 mmol) were suspended in toluene (3 mL) and trifluoroacetic acid (TFA) (1.527 g, 13.16 mmol), and the suspension was stirred at room temperature for 16 h. The reaction solution was then concentrated under reduced pressure, and the resulting residue was diluted with dichloromethane and washed with a saturated aqueous sodium bicarbonate solution, water, and brine. The organic layer was dried over anhydrous sodium sulfate and then filtered, the filtrate was concentrated under reduced pressure, and the resulting residue was purified by normal phase column chromatography (petroleum ether/ethyl acetate) to afford (9H-fluoren-9-yl)methyl (S)-4-(2-morpholinoethyl)-5-oxooxazolidine-3-carboxylate (Compound tm18) (486 mg, 79%).
LCMS (ESI) m/z = 423 (M+H)+

Retention time: 1.14 min (analytical condition SMD method 40)

<u>Synthesis of (S)-2-((((9H-fluoren-9-yl)methoxy)carbonyl)(methyl)amino)-4-morpholinobutanoic acid (Compound tm19, Fmoc-MeAbu(Mor)-OH)</u>

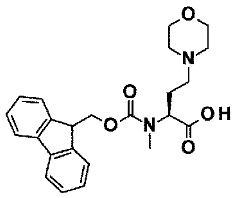

(9H-Fluoren-9-yl)methyl (S)-4-(2-morpholinoethyl)-5-oxooxazolidine-3-carboxylate (Compound tml8) (486 mg, 1.15 mmol) and triethylsilane (1.5 mL, 10.35 mmol) were dissolved in trifluoroacetic acid/dichloroethane (1/1, 12 mL), and the reaction solution was stirred at 70°C for 4 h. After cooling to room temperature, the reaction solution was concentrated under reduced pressure, the resulting residue was diluted with dichloromethane and washed with water, the organic layer was then dried over anhydrous sodium sulfate and filtered, and the filtrate was then concentrated under reduced pressure. The resulting residue was purified by reverse phase column chromatography (5 to 70% 0.5% aqueous hydrochloric acid solution/0.5% hydrochloric acid-acetonitrile solution) to afford (S)-2-((((9H-fluoren-9-yl)methoxy)carbonyl)(methyl)amino)-4-morpholinobutanoic acid (Compound tm19, Fmoc-MeAbu(Mor)-OH) (400 mg, 82%).
LCMS (ESI) m/z = 425 (M+H)+

Retention time: 1.40 min (analytical condition SMD method 34)

Synthesis of N-(((9H-fluoren-9-yl)methoxy)carbonyl)-O-(2-morpholinoethyl)-L-serine (Compound tm20, Fmoc-Ser(Et-2-Mor)-OH)

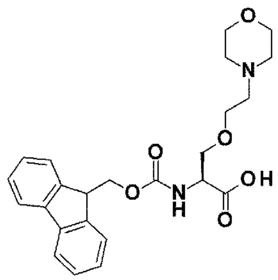

To a solution of commercially available (tert-butoxycarbonyl)-L-serine (Boc-Ser-OH) (20 g, 97.46 mmol) in dimethylformamide (DMF) (100 mL) was added sodium hydride (7.718 g, 321.62 mmol, 60% oil dispersion) at room temperature under a nitrogen atmosphere, and the mixture was stirred for 1 h. The reaction solution was cooled to 0°C, 4-(2-chloroethyl)morpholine (16.040 g, 107.21 mmol) was added dropwise, and the mixture was stirred at room temperature for 16 h. To the reaction solution was added a 50% aqueous formic acid solution at 0°C, the mixture was filtered, and the resulting filtrate was then purified by reverse phase column chromatography (water/acetonitrile) to afford N-(tert-butoxycarbonyl)-O-(2-morpholinoethyl)-L-serine (Boc-Ser(Et-2-Mor)-OH) (5.6 g, 18%).

To N-(tert-butoxycarbonyl)-O-(2-morpholinoethyl)-L-serine (Boc-Ser(Et-2-Mor)-OH) (5.6 g, 17.59 mmol) was added a 4N hydrochloric acid/1,4-dioxane solution (28.0 mL, 921.53 mmol) at room temperature, and the mixture was stirred for 1 h. The solvent was evaporated under reduced pressure, the resulting residue was washed with dichloromethane, and water (10 mL) was then added to prepare a solution. The resulting solution was adjusted to pH 7 with potassium carbonate, and water (100 mL) and 1,4-dioxane (150 mL) were added, after which potassium carbonate (4.465 g, 32.07 mmol) and N-(9-fluorenylmethoxycarbonyloxy)succinimide (Fmoc-OSu) (5.68 g, 16.84 mmol) were added at room temperature and the mixture was stirred for 16 h. The reaction solution was washed with diethyl ether, and the aqueous layer was adjusted to pH 1 with concentrated hydrochloric acid and extracted with dichloromethane three times. The resulting organic layers were washed with water, dried over anhydrous sodium sulfate, and filtered. The filtrate was concentrated under reduced pressure, and the resulting residue was purified by reverse phase column chromatography (0.1% aqueous hydrochloric acid solution/0.1% hydrochloric acid-acetonitrile solution) to afford N-(((9H-fluoren-9-yl)methoxy)carbonyl)-O-(2-morpholinoethyl)-L-serine (Compound tm20, Fmoc-Ser(Et-2-Mor)-OH) hydrochloride (3.2 g, 48% over two steps).
LCMS (ESI) m/z = 441 (M+H)+

Retention time: 0.50 min (analytical condition SQDFA05)

Synthesis of (S)-2-((((9H-fluoren-9-yl)methoxy)carbonyl)amino)-4-(5-oxo-4,5-dihydro-1,2,4-oxadiazol-3-yl)butanoic acid (Compound tm21, Fmoc-Abu(5-Oxo-Odz)-OH)

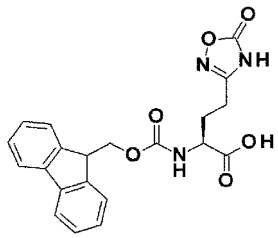

To a solution of commercially available (tert-butoxycarbonyl)-L-glutamine (Boc-Gln-OH) (50 g, 203.03 mmol) in pyridine (350 mL) was added N,N'-dicyclohexylcarbodiimide (DCC) (46.1 g, 223.43 mmol) at room temperature under a nitrogen atmosphere, and the mixture was stirred for 2 h. The reaction solution was filtered and the filtrate was concentrated. To the resulting residue was added dichloromethane and concentrated hydrochloric acid while adjusting it to pH = 3, and the mixture was extracted with dichloromethane twice. The resulting organic layers were dried over anhydrous sodium sulfate and filtered, and the solvent was evaporated under reduced pressure. The resulting residue was purified by normal phase column chromatography (petroleum ether/ethyl acetate) to afford (S)-2-((tert-butoxycarbonyl)amino)-4-cyanobutanoic acid (45.5 g, 98%).

To a solution of (S)-2-((tert-butoxycarbonyl)amino)-4-cyanobutanoic acid obtained as described above (50 g, 219.06 mmol) in ethanol (500 mL) were added hydroxylamine hydrochloride (32 g, 460.50 mmol) and triethylamine (83 mL) at room temperature, and the mixture was stirred at 80°C for 2 h. The reaction solution was concentrated under reduced pressure to afford (S)-2-((tert-butoxycarbonyl)amino)-5-(hydroxyamino)-5-iminopentanoic acid (107 g) as a crude product.

To a solution of the above crude product (S)-2-((tert-butoxycarbonyl)amino)-5-(hydroxyamino)-5-iminopentanoic acid (44 g, 168.40 mmol) in 1,4-dioxane (500 mL) were added 1,1'-carbonyldiimidazole (CDI) (39 g, 240.52 mmol) and 1,8-diazabicyclo[5.4.0]-7-undecene (DBU) (55 g, 361.27 mmol) at room temperature under a nitrogen atmosphere, and the mixture was stirred at 110°C for 4 h. The reaction solution was cooled at room temperature, then adjusted to pH = 2 with concentrated hydrochloric acid, and extracted with dichloromethane twice. The resulting organic layers were dried over anhydrous sodium sulfate and filtered, and the solvent was evaporated under reduced pressure to afford (S)-2-((tert-butoxycarbonyl)amino)-4-(5-oxo-4,5-dihydro-1,2,4-oxadiazol-3-yl)butanoic acid (Boc-Abu(5-Oxo-Odz)-OH) (11 g) as a crude product.

To a solution of (S)-2-((tert-butoxycarbonyl)amino)-4-(5-oxo-4,5-dihydro-1,2,4-oxadiazol-3-yl)butanoic acid (Boc-Abu(5-Oxo-Odz)-OH) obtained as described above (13.5 g, 46.99 mmol) in 1,4-dioxane (10 mL) was added a 4N hydrochloric acid/1,4-dioxane solution (140 mL) at room temperature under a nitrogen atmosphere, and the mixture was stirred for 16 h. The reaction solution was concentrated under reduced pressure to afford (S)-2-amino-4-(5-oxo-4,5-dihydro-1,2,4-oxadiazol-3-yl)butanoic acid (H-Abu(5-Oxo-Odz)-OH) (8.8 g) as a crude product.

To a solution of the resulting crude product (S)-2-amino-4-(5-oxo-4,5-dihydro-1,2,4-oxadiazol-3-yl)butanoic acid (H-Abu(5-Oxo-Odz)-OH) (8.8 g, 47.02 mmol) and potassium carbonate (13 g, 94.06 mmol) in water/1,4-dioxane (100 mL/100 mL) was added N-(9-fluorenylmethoxycarbonyloxy)succinimide (Fmoc-OSu) (14.3 g, 42.4 mmol) at room temperature under a nitrogen atmosphere, and the mixture was stirred for 3 h. The reaction solution was washed with t-butyl methyl ether/hexane (1/3), and the aqueous layer was adjusted to pH 2 with concentrated hydrochloric acid and extracted with dichloromethane twice. The resulting organic layers were dried over anhydrous sodium sulfate and filtered. The filtrate was concentrated under reduced pressure, and the resulting residue was purified by reverse phase column chromatography (water/acetonitrile) to afford (S)-2-((((9H-fluoren-9-yl)methoxy)carbonyl)amino)-4-(5-oxo-4,5-dihydro-1,2,4-oxadiazol-3-yl)butanoic acid (Compound tm21, Fmoc-Abu(5-Oxo-Odz)-OH) (2.67 g).
LCMS (ESI) m/z = 410 (M+H)+

Retention time: 0.66 min (analytical condition SQDFA05)

Synthesis of N2-(((9H-fluoren-9-yl)methoxy)carbonyl)-N5-(methylsulfonyl)-L-glutamine (Compound tm22, Fmoc-Gln(Ms)-OH)

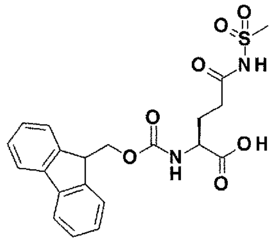

To a solution of commercially available (S)-4-((((9H-fluoren-9-yl)methoxy)carbonyl)amino)-5-(tert-butoxy)-5-oxopentanoic acid (Fmoc-Glu-OtBu) (20 g, 47 mmol), methanesulfonamide (20 g, 210 mmol), and 4-dimethylaminopyridine (DMAP) (1.3 g, 10.6 mmol) in dichloromethane (360 mL) was added 1-(3-dimethylaminopropyl)-3-ethylcarbodiimide hydrochloride (WSC·HCl) (9.64 g, 50.3 mmol) at room temperature under a nitrogen atmosphere, and the mixture was stirred for 16 h. The reaction solution was concentrated under reduced pressure, ethyl acetate was added, and the reaction solution was washed with a 0.1% aqueous hydrochloric acid solution three times and with water once, then dried over anhydrous sodium sulfate, and filtered. The resulting solution was concentrated under reduced pressure, and the residue was purified by normal phase column chromatography (petroleum ether/ethyl acetate) to afford tert-butyl N2-(((9H-fluoren-9-yl)methoxy)carbonyl)-N5-(methylsulfonyl)-L-glutaminate (Fmoc-Gln(Ms)-OtBu) (9.3 g, 39%).

To a solution of tert-butyl N2-(((9H-fluoren-9-yl)methoxy)carbonyl)-N5-(methylsulfonyl)-L-glutaminate (Fmoc-Gln(Ms)-OtBu) obtained as described above (5.5 g, 10.9 mmol) in 2,2,2-trifluoroethanol (TFE) (100 mL) was added chlorotrimethylsilane (TMSCl) (3.57 g, 32.861 mmol) at 0°C under a nitrogen atmosphere, and the mixture was stirred for 1 h. The reaction solution was concentrated under reduced pressure, t-butyl methyl ether was added to the resulting residue, and the mixture was concentrated again. This operation was further repeated twice, and recrystallization from acetonitrile/dichloromethane gave N2-(((9H-fluoren-9-yl)methoxy)carbonyl)-N5-(methylsulfonyl)-L-glutamine (Compound tm22, Fmoc-Gln(Ms)-OH) (3.8178 g, 77%) as a white solid. LCMS (ESI) m/z = 447 (M+H)+

Retention time: 0.65 min (analytical condition SQDFA05)

[Reference Example 4] Influence of ClogP on membrane permeability

Cyclic peptide compounds having a Trp side chain and cyclic peptide compounds not having the side chain were synthesized, and their membrane permeability was tested by the modified method. Since MeTrp contains a Trp side chain, cyclic peptide compounds containing MeTrp as a constituent amino acid are also encompassed within the cyclic peptide compounds having a Trp side chain. As a result, cyclic peptide compounds not having a Trp side chain were capable of achieving $P_{app} \geq 1.0 \times 10^{-6}$ cm/sec by increasing ClogP (Fig. 1-1). On the other hand, when one or more Trp side chains were contained, it was difficult to increase membrane permeability to $P_{app} \geq 1.0 \times 10^{-6}$ cm/sec even when ClogP was increased (Fig. 1-2). For example, Table 31 shows the sequences of cyclic peptide compounds that have a different Trp side chain position and different *N*-alkyl pattern and that were in the range of 1.06 ≤ ClogP/total aa ≤ 1.43, and it was difficult to achieve membrane permeability of $P_{app} \geq 1.0 \times 10^{-6}$ cm/sec with these cyclic peptide compounds.

In sum, when the membrane permeability of cyclic peptide compounds having no Trp side chain were evaluated, it was confirmed that the membrane permeability improves by increasing ClogP (Fig. 1-1), and thus the difference in ClogP/total AA values of two peptides to be compared were regulated so as to be between ±0.03.

[Table 31]

Membrane Permeability of Cyclic Peptide Compound Having Trp Side Chain

| Compound ID | 11 | 10 | 9 | 8 | 7 | 6 | 5 | 4 | 3 | 2 | 1 | C-term | cLogP/ total AA | Caco-2 (cm/sec) |
|---|---|---|---|---|---|---|---|---|---|---|---|---|---|---|
| pd100 | D-Val | MeAla | MeLeu | Leu | MePhe | Trp | MeLeu | MeGly | MeAla | Thr | Asp | pip | 1.06 | 1.1E-07 |
| pd101 | MeAla | Leu | MeLeu | MeLeu | Ala | MeLeu | MeIle | Trp | MeGly | MeAla | Asp | pip | 1.23 | 1.3E-07 |
| pd102 | MeAla | Leu | MeLeu | MeTrp | Val | MeLeu | MeIle | Thr | MeGly | MeLeu | Asp | pip | 1.25 | 1.1E-06 |
| pd103 | MeGly | MeLeu | Ile | MeLeu | Trp | MeGly | MeLeu | MeLeu | Ser(tBu) | MeAla | Asp | pip | 1.26 | 9.3E-08 |
| pd104 | MeAla | MeLeu | MeLeu | Ala | MeLeu | MeTrp | MePhe | Thr | MeAla | MeLeu | Asp | pip | 1.27 | 2.5E-08 |
| pd105 | MeAla | MeLeu | MeLeu | Ala | MeLeu | MeLeu | MeTrp | Thr | MeAla | MeLeu | Asp | pip | 1.27 | 2.8E-07 |
| pd106 | D-Val | MeTrp | Leu | MeLeu | Thr | MeGly | MeLeu | Ser(tBu) | MeLeu | MeLeu | Asp | pip | 1.28 | 3.6E-07 |
| pd107 | g-MeAbu | MeLeu | Ile | MePhe | MeTrp | Ser(tBu) | MeLeu | Thr | MeLeu | MeLeu | Asp | pip | 1.30 | 1.7E-07 |
| pd108 | D-Ala | MeLeu | Ile | MeLeu | Trp | MeGly | MeLeu | Ser(tBu) | MeLeu | MeVal | Asp | pip | 1.34 | 8.7E-07 |
| pd109 | MeAla | MeLeu | MeLeu | Val | MeLeu | MeTrp | MePhe | Thr | MeAla | MeLeu | Asp | pip | 1.36 | 9.2E-09 |
| pd110 | g-MeAbu | MeLeu | Ile | MeLeu | MeLeu | Ser(tBu) | MeLeu | Trp | MeAla | MeLeu | Asp | pip | 1.36 | 2.9E-07 |
| pd111 | MeLeu | Leu | MeLeu | MeTrp | Leu | MePhe | MeIle | Thr | MeGly | MeLeu | Asp | pip | 1.43 | 1.6E-07 |

Structures of cyclic peptides pd100 to pd111 are shown below. (In the structural formulae of the cyclic peptides, hydrogen atoms attached to a heteroatom may be omitted to clarify the structure.)

[Table 32]

| ID | Structural Formula | ID | Structural Formula |
|---|---|---|---|
| pd100 | 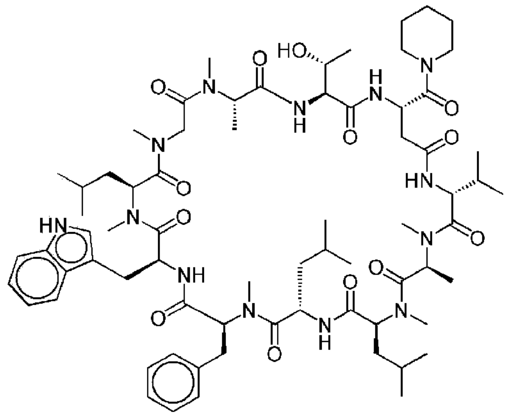 | pd101 | 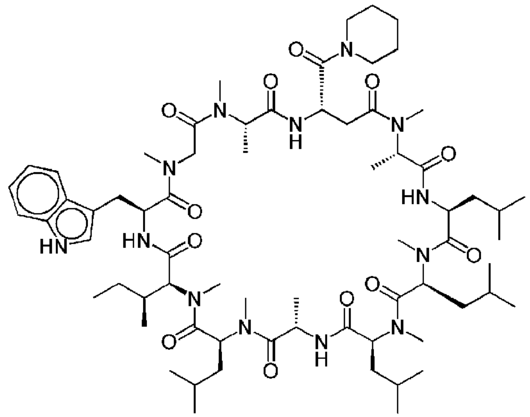 |
| pd102 | 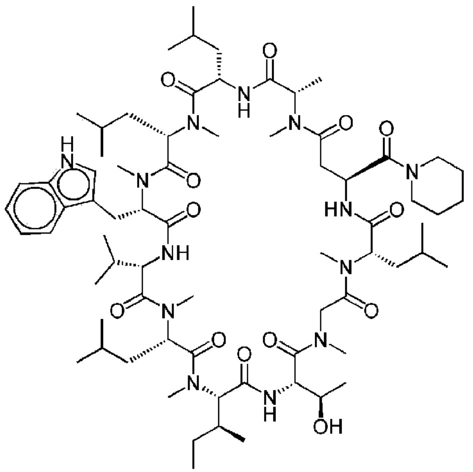 | pd103 | 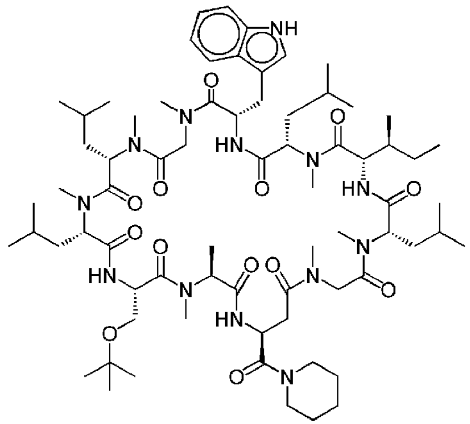 |

(continued)

| ID | Structural Formula | ID | Structural Formula |
|---|---|---|---|
| pd104 | 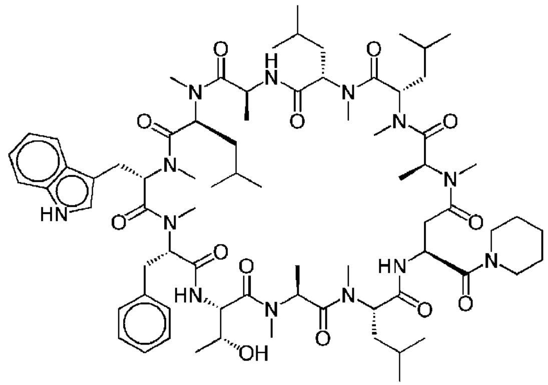 | pd105 | 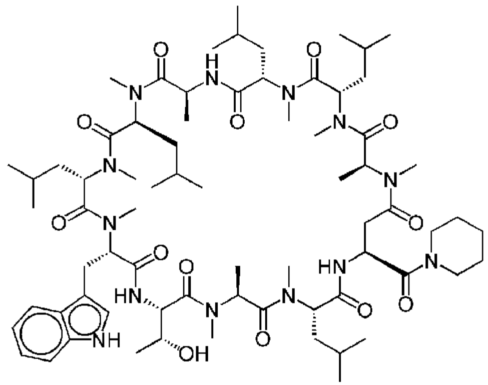 |
| pd106 | 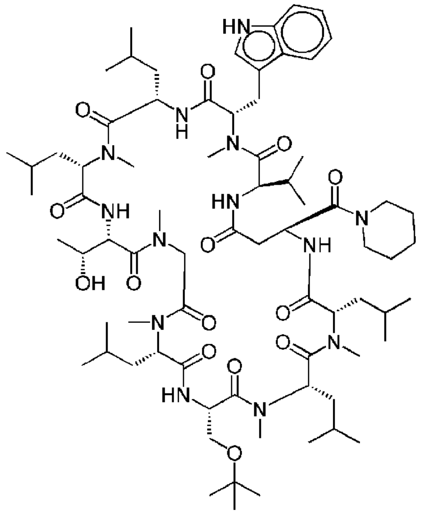 | pd107 | 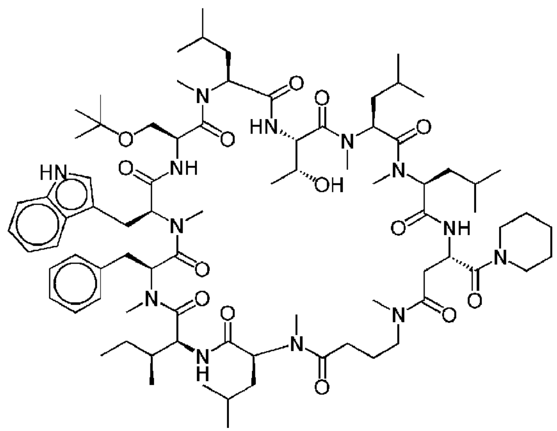 |

(continued)

| ID | Structural Formula | ID | Structural Formula |
|---|---|---|---|
| pd108 | 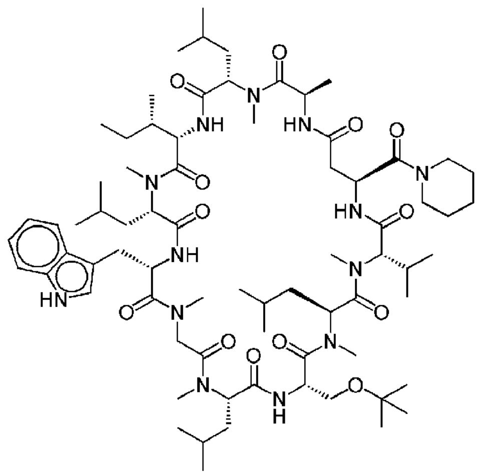 | pd109 | 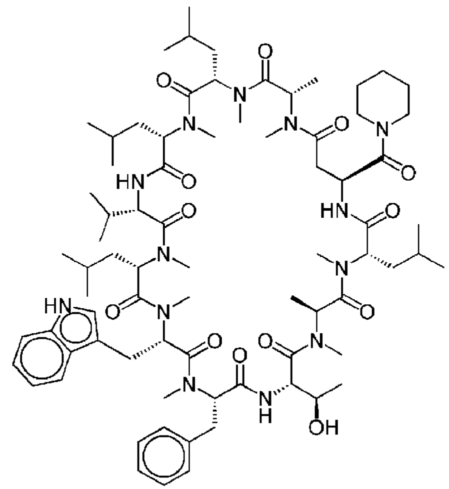 |
| pd110 | 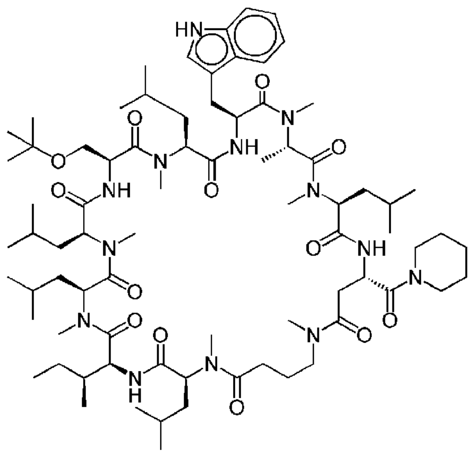 | pd111 | 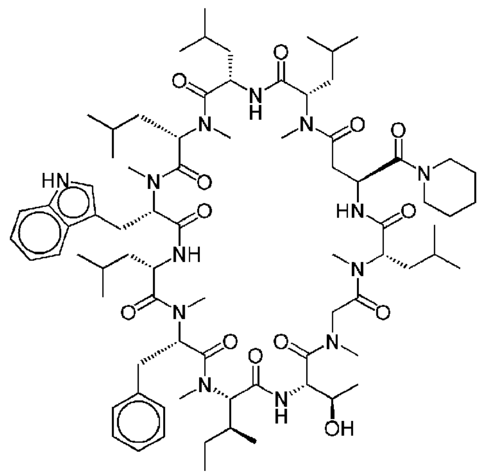 |

[Reference Example 5] Membrane permeability test of cyclic peptide compounds having Tyr(3-F) or Tyr side chain

Cyclic peptide compounds having a Tyr(3-F) or Tyr side chain were synthesized, and their membrane permeability was tested by the modified method. As a result, cyclic peptide compounds having a Tyr(3-F) or Tyr side chain also had the same tendency as cyclic peptide compounds having a Trp side chain. The sequences of cyclic peptide compounds in the range 1.06 ≤ ClogP/total aa ≤ 1.29 are shown in Table 33, and as in the case of Trp, it was difficult to achieve $P_{app} \geq 1.0 \times 10^{-6}$ cm/sec with these cyclic peptide compounds.

[Table 33]

Membrane Permeability of Cyclic Peptide Compound Having Tyr(3-F) or Tyr Side Chain

| Compound ID | 11 | 10 | 9 | 8 | 7 | 6 | 5 | 4 | 3 | 2 | 1 | C-term | cLogP/ totalAA | Caco-2 (cm/sec) |
|---|---|---|---|---|---|---|---|---|---|---|---|---|---|---|
| pd112 | D-Ala | MePhe | Ile | MeLeu | Tyr | MeGly | MeAla | Ser(tBu) | MeAla | MeLeu | Asp | pip | 1.06 | 1.1E-07 |
| pd113 | D-Val | MeAla | MeLeu | Leu | MePhe | Tyr(3-F) | MeLeu | MeAla | MeAla | Thr | Asp | pip | 1.07 | 2.9E-08 |
| pd114 | g-MeAbu | MeLeu | Tyr(3-F) | MeLeu | MePhe | Ser(tBu) | MeLeu | Thr | MeGly | MeLeu | Asp | pip | 1.08 | 1.7E-07 |
| pd115 | MeGly | MePhe | Val | MeLeu | Tyr | MeGly | MeAla | MeLeu | Ser(tBu) | MeVal | Asp | pip | 1.10 | 2.2E-07 |
| pd116 | MeAla | Leu | MeAla | MePhe | Val | MeAla | MeIle | Tyr | MeGly | MeLeu | Asp | pip | 1.12 | 1.4E-07 |
| pd117 | D-Ala | MeLeu | Ile | MeAla | Tyr | MeGly | MeLeu | Ser(tBu) | MePhe | MeVal | Asp | pip | 1.15 | 5.6E-07 |
| pd118 | D-Leu | MePhe | Tyr(3-F) | MeLeu | Thr | MeGly | MeLeu | Ser(tBu) | MeLeu | MeAla | Asp | pip | 1.15 | 2.0E-07 |
| pd119 | g-MeAbu | MeLeu | Ile | MePhe | MeLeu | Tyr(3-F) | MeLeu | Thr | MeAla | MeLeu | Asp | pip | 1.17 | 1.6E-07 |
| pd120 | MeAla | Leu | MeLeu | MePhe | Ala | MeAla | MeLeu | Tyr(3-F) | MeGly | MeLeu | Asp | pip | 1.18 | 2.3E-07 |
| pd121 | D-Val | MeAla | Val | MeLeu | Tyr | MeGly | MeLeu | Ser(tBu) | MePhe | MeVal | Asp | pip | 1.18 | 1.0E-06 |
| pd122 | MeAla | Tyr(3-F) | MeLeu | MeLeu | Val | MePhe | MeLeu | Thr | MeGly | MeLeu | Asp | pip | 1.21 | 4.6E-07 |
| pd123 | MeGly | MeLeu | Tyr(3-F) | MeLeu | Thr | MeGly | MePhe | MeLeu | Leu | MeLeu | Asp | pip | 1.21 | 4.4E-07 |
| pd124 | D-Ala | MeLeu | Ile | MeLeu | Tyr(3-F) | MeGly | MeLeu | Ser(tBu) | MePhe | MeAla | Asp | pip | 1.22 | 3.2E-07 |
| pd125 | g-MeAbu | MeVal | Ile | MeLeu | MePhe | Ser(tBu) | MeLeu | Tyr | MeAla | MeLeu | Asp | pip | 1.25 | 4.5E-07 |
| pd126 | MeAla | Val | MeLeu | MeLeu | Ala | MePhe | MeIle | Tyr | MeGly | MeLeu | Asp | pip | 1.25 | 4.2E-07 |
| pd127 | D-Val | MePhe | Leu | MeLeu | Tyr | MeGly | MeLeu | Ser(tBu) | MeLeu | MeAla | Asp | pip | 1.28 | 1.3E-07 |
| pd128 | D-Val | MePhe | Leu | MeLeu | Thr | MeGly | MeLeu | Tyr(3-F) | MeLeu | MeLeu | Asp | pip | 1.29 | 1.7E-07 |
| pd129 | g-MeAbu | MeLeu | Ile | MePhe | MeLeu | Ser(tBu) | MeLeu | Tyr | MeAla | MeLeu | Asp | pip | 1.29 | 1.6E-07 |

Structures of cyclic peptides pd112 to pd129 are shown below. (In the structural formulae of the cyclic peptides, hydrogen atoms attached to a heteroatom may be omitted to clarify the structure.)

[Table 34]

| ID | Structural Formula | ID | Structural Formula |
|---|---|---|---|
| pd112 | 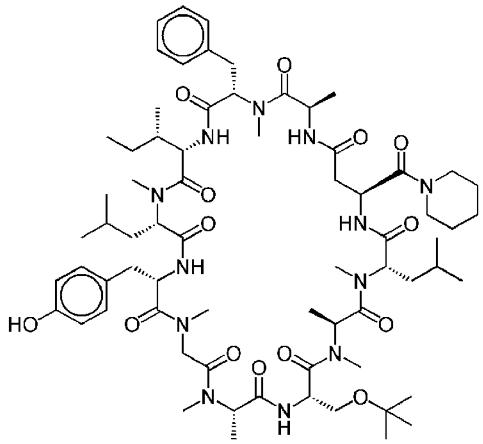 | pd113 | 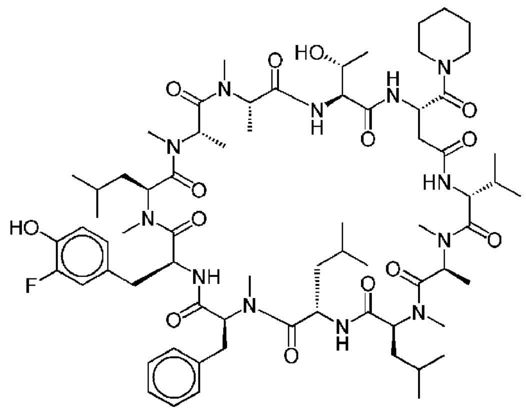 |
| pd114 | 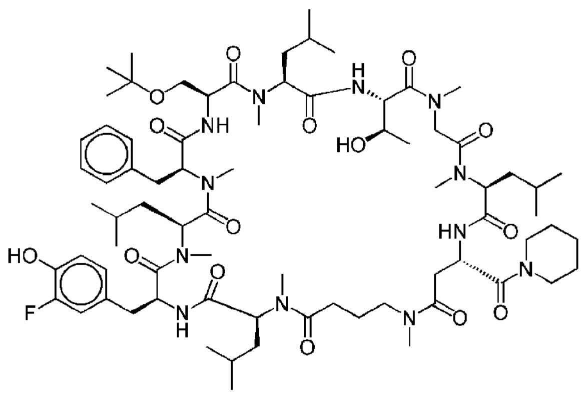 | pd115 | 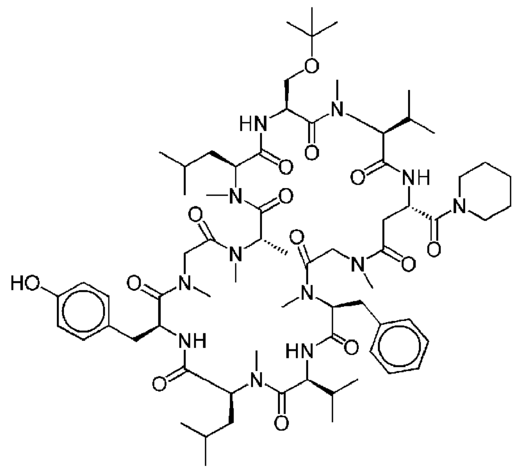 |

(continued)

| ID | Structural Formula | ID | Structural Formula |
|---|---|---|---|
| pd116 | 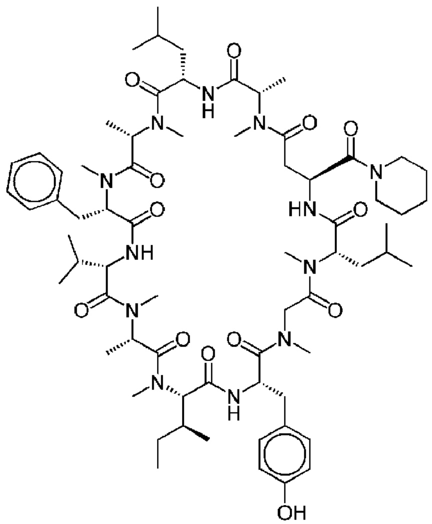 | pd117 | 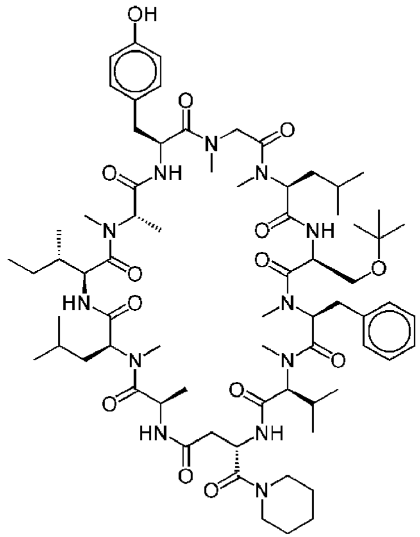 |
| pd118 | 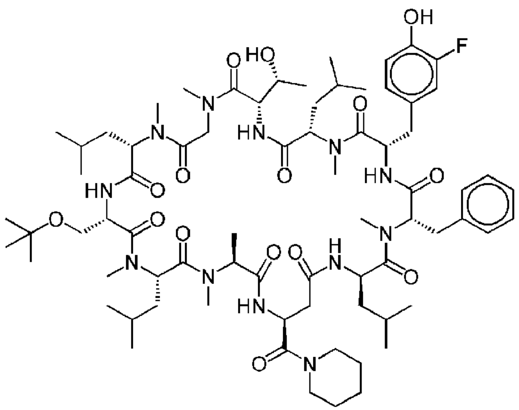 | pd119 | 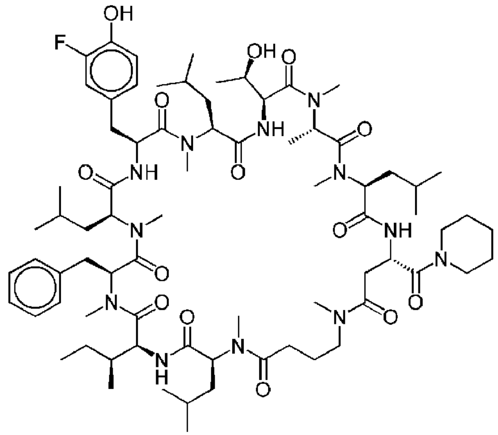 |

(continued)

| ID | Structural Formula | ID | Structural Formula |
|---|---|---|---|
| pd120 | 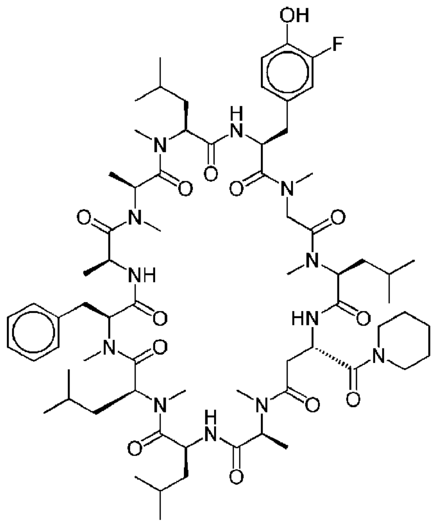 | pd121 | 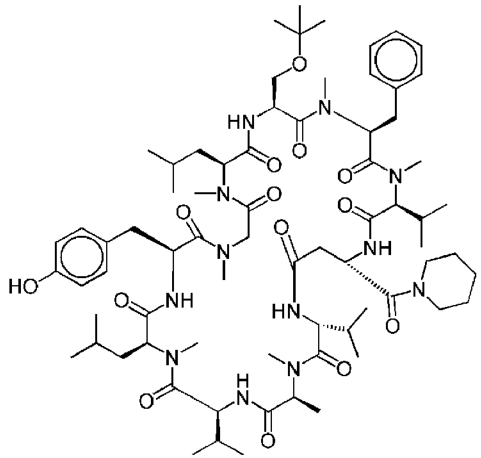 |
| pd122 | 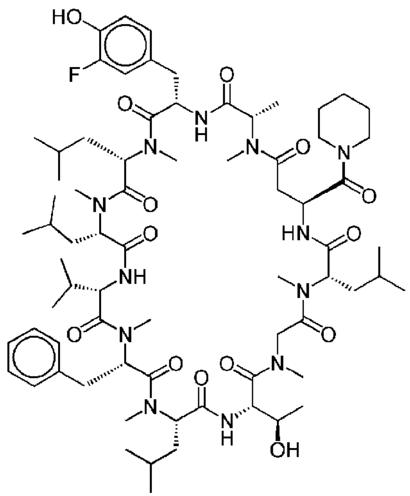 | pd123 | 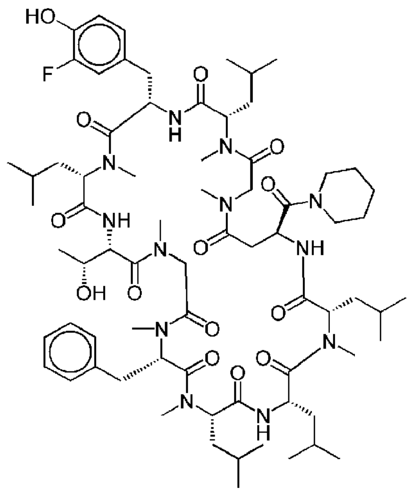 |

(continued)

| ID | Structural Formula | ID | Structural Formula |
|---|---|---|---|
| pd124 | 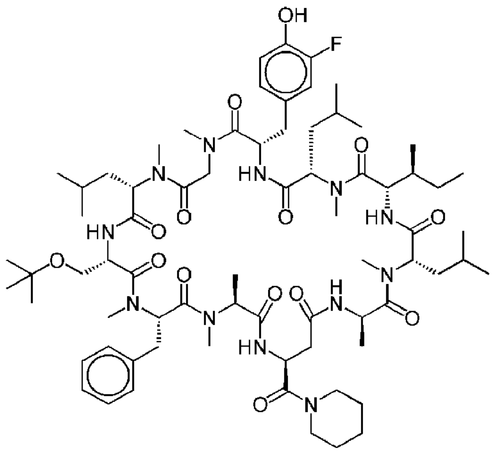 | pd125 | 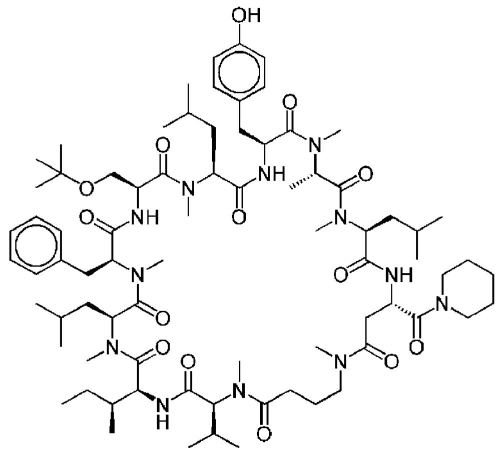 |
| pd126 | 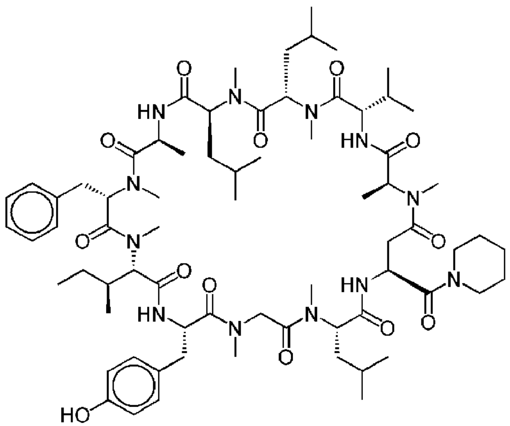 | pd127 | 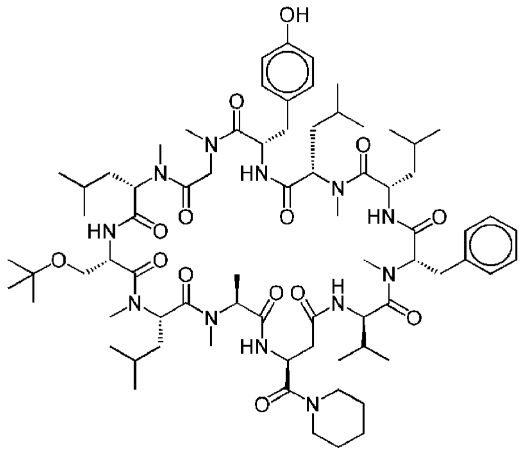 |

(continued)

| ID | Structural Formula | ID | Structural Formula |
|---|---|---|---|
| pd128 | 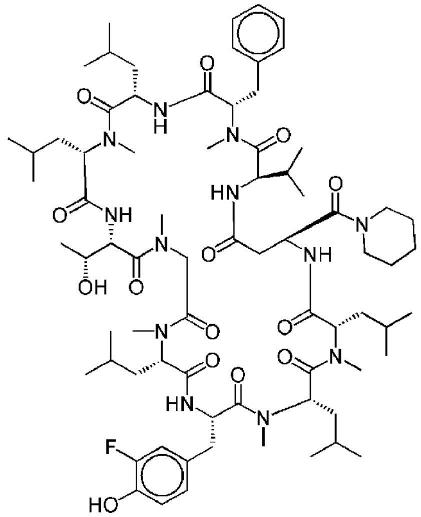 | pd129 | 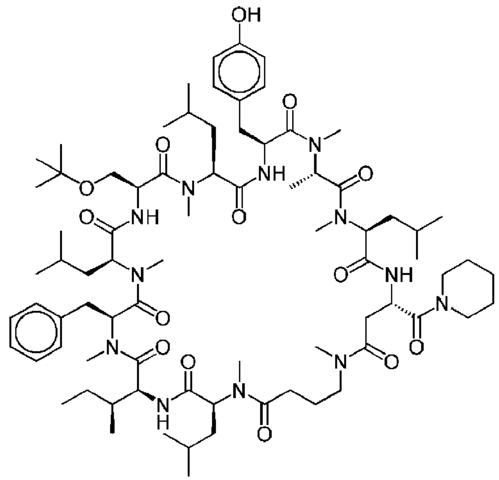 |

[Reference Example 6] ,Influence of aromatic ring count (ARC) in cyclic peptides on membrane permeability

It was confirmed that $P_{app}$, *i.e.,* membrane permeability, of cyclic peptide compounds tended to decrease associated with the increase in ARC (Fig. 2). Accordingly, two peptides to be compared were regulated so as to have the same aromatic ring count (ARC).

In this analysis, cyclic peptide compounds having a fused-ring structure in the side chains of the cyclic portion and cyclic peptide compounds having a substituted or unsubstituted hydroxyphenyl group were excluded from analysis targets. When ARC = 3, a $P_{app}$ of 1.0 x $10^{-6}$ or more was observed in sequences having a ClogP/total AA of 0.88 or more. On the other hand, almost no compounds where ARC = 4 had a $P_{app}$ of 1.0 x $10^{-6}$ or more even when such compounds had a ClogP/total AA (0.88 or more) comparable to those of the above compounds where ARC = 3. This demonstrated that when sequences where ARC = 3 and sequences where ARC = 4 are compared, the sequences where ARC = 3 had higher membrane permeability than the sequences where ARC = 4, although the sequences where ARC = 3 were as lipophilic as the sequences where ARC = 4 (Tables 35, 36, and 37).

The columns of Tables 35, 36, and 37 are described taking as an example pd198. pd198 is a cyclic peptide consisting of twelve residues, and forms a ring with 10 amino acid residues. Cyclized portion is defined as the first amino acid, and in pd198, the first amino acid is Asp. The amino acid adjacent to Asp and constituting the cyclic portion is defined as the second amino acid (which is Phe in pd198), and subsequent amino acids ranging from the third and fourth amino acids to the N-terminal amino acid are defined in the same manner (in pd198, the third amino acid is MeVal, the fourth amino acid is MeLeu, and the N-terminal amino acid is the 10th amino acid g-MeAbu because the cyclic portion is composed of 10 residues). The amino acids of the cyclized portion are represented by H-1, H-2, and H-3 sequentially from the C terminus. In pd198, since H-1 is MePhe and H-2 is Ala, it represents that MePhe is bonded to the C-terminus of Asp and Ala is then bonded to MePhe. The C-term column shows a functional group condensed with the carboxylic acid site of the C-terminal amino acid, where pip represents piperidine and pyrro represents pyrroridine (in pd198, the C-terminal amino acid is Ala of H-2, the carboxylic acid site of which is condensed with pip (piperidine)). Tables 35, 36, and 37 show that it is a peptide in which the N-terminal amino group and side chain carboxylic acid of amino acid at a cyclized portion are cyclized by an amide bond. For example, in pd 198, an amino group of the N-terminal g-MeAbu and side chain carboxylic acid of Asp at cyclized portion are cyclized by an amide bond. Cyclic peptides described in the text are all expressed in the same manner unless otherwise specifically stated.

[Table 35]

Sequences having $P_{app} \geqq 1.0 \times 10^{-6}$ at ARC = 3

| Compound ID | 11 | 10 | 9 | 8 | 7 | 6 | 5 | 4 | 3 | 2 | 1 | H-1 | H-2 | H-3 | C-term | cLogP/ total AA | Caco-2 (cm/sec) |
|---|---|---|---|---|---|---|---|---|---|---|---|---|---|---|---|---|---|
| pd162 | Pro | Phe | Ser(tBu) | MeLeu | Thr | MeGly | MeVal | Phe | MePhe(3-Cl) | MeLeu | MeAsp | | | | pip | 1.25 | 1.2E-06 |
| pd163 | D-Pro | Phe | Ser(tBu) | MeLeu | Thr | MeGly | MeVal | Phe | MePhe(3-Cl) | MeLeu | MeAsp | | | | pip | 1.25 | 1.7E-06 |
| pd167 | D-MeAla | Phe | Ser(tBu) | MeLeu | Thr | MeGly | MeVal | Phe | MePhe(3-Cl) | MeLeu | MeAsp | | | | pip | 1.27 | 1.7E-06 |
| pd177 | MeGly | MePhe | Ser(Bn) | MeLeu | Thr | MeGly | MePhe | MeLeu | Ser(tBu) | MeLeu | Asp | | | | pip | 1.21 | 3.0E-06 |
| pd178 | MeGly | MePhe | Ile | MeLeu | Thr | MeGly | MePhe | MeLeu | Ser(Bn) | MeIle | Asp | | | | pip | 1.26 | 2.5E-06 |
| pd179 | MeAla | Leu | MeLeu | MePhe | Ser(Bn) | MePhe | MeAla | Thr | MeGly | MeLeu | Asp | | | | pip | 1.18 | 2.0E-06 |
| pd180 | D-Val | MePhe | Ser(3-F-5-Me-Pyr) | MeLeu | Thr | MeGly | MeLeu | Ser(tBu) | MePhe | MeVal | Asp | | | | pip | 1.13 | 2.4E-06 |
| pd181 | MeVal | Ser(3-F-5-Me-Pyr) | MeLeu | MePhe | Leu | MePhe | MeIle | Thr | MeGly | MeLeu | Asp | | | | pip | 1.28 | 1.1E-06 |
| pd182 | MeVal | Ala | MePhe | MeLeu | MeGly | Thr | MeVal | Hph | MeLeu | MePhe | Asp | | | | pip | 1.25 | 2.6E-06 |
| pd183 | MeAla | MePhe | Hph | MeLeu | Thr | MeGly | MeLeu | MePhe | Ser(tBu) | MeAla | Asp | | | | pip | 1.16 | 2.1E-06 |
| pd184 | MeAla | MePhe | Hph | MeLeu | Thr | MeGly | MeLeu | MeHph | Ser(tBu) | MeAla | Asp | | | | pip | 1.21 | 1.2E-06 |
| pd185 | D-Ala | MePhe | Ser(Ph-3-Cl) | MeVal | Thr | MeGly | MeLeu | Ser(tBu) | MePhe | MeVal | Asp | | | | pip | 1.19 | 2.2E-06 |
| pd186 | MeAla | Val | MeLeu | MePhe | Ser(Ph-3-Cl) | MePhe | MeIle | Thr | MeGly | MeAla | Asp | | | | pip | 1.21 | 1.8E-06 |
| pd187 | D-Val | MePhe | Ser(Ph-3-Cl) | MeAla | Thr | MeGly | MeLeu | Ser(tBu) | MePhe | MeVal | Asp | | | | pip | 1.19 | 1.9E-06 |
| pd188 | MeAla | Ser(Ph-3-Cl) | MeLeu | MePhe | Ala | MePhe | MeIle | Thr | MeGly | MeLeu | Asp | | | | pip | 1.26 | 1.3E-06 |
| pd189 | MeGly | MePhe | Phe{#(CH2)2} | MeLeu | Thr | MeGly | MePhe | MeLeu | Ser(tBu) | MeIle | Asp | | | | pip | 1.29 | 1.8E-06 |
| pd190 | MeGly | MePhe | Ile | MeLeu | Thr | MeGly | MePhe | MeLeu | Phe{#(CH2)2} | MeAla | Asp | | | | pip | 1.21 | 1.5E-06 |
| pd191 | MeAla | Phe{#(CH2)2} | MeLeu | MePhe | Ala | MePhe | MeVal | Thr | MeGly | MeLeu | Asp | | | | pip | 1.21 | 1.5E-06 |
| pd192 | D-Val | MePhe | Phe{#(CH2)2} | MeLeu | Thr | MeGly | MeLeu | Ser(tBu) | MePhe | MeAla | Asp | | | | pip | 1.24 | 1.1E-06 |
| pd193 | D-Ala | MePhe | Val | MeLeu | Thr | MeGly | MeLeu | Phe{#(CH2)2} | MePhe | MeVal | Asp | | | | pip | 1.24 | 1.0E-06 |
| pd194 | D-Ala | MePhe | Val | MeLeu | Thr | nPrGly | MeLeu | Ser(tBu) | MePhe | MePhe | Asp | | | | pip | 1.25 | 5.3E-06 |
| pd195 | nPrGly | MePhe | Ile | MePhe | Thr | MeGly | MePhe | MeLeu | Ser(tBu) | MeVal | Asp | | | | pip | 1.26 | 5.0E-06 |
| pd196 | Pro | MePhe | Leu | MeLeu | Thr | MeGly | MeVal | Ser(tBu) | MePhe | MePhe | MeAsp | | | | pip | 1.24 | 4.0E-06 |
| pd197 | MeAla | MePhe | Leu | MeLeu | Ala(3-Pyr) | MeGly | MeLeu | Ser(tBu) | MePhe | MeVal | Asp | | | | pip | 1.25 | 3.0E-06 |
| pd198 | | g-MeAbu | Val | MeLeu | Thr | MePhe | Gly | MeLeu | MeVal | Phe | Asp | MePhe | Ala | | pip | 0.88 | 2.7E-06 |
| pd199 | D-MeAla | MePhe | Leu | MeLeu | Thr | MeGly | MeVal | Ser(tBu) | MePhe | MePhe | MeAsp | | | | pip | 1.26 | 2.6E-06 |
| pd200 | D-Pro | MePhe | Leu | MeLeu | Thr | MeGly | MeVal | Ser(tBu) | MePhe | MePhe | MeAsp | | | | pip | 1.24 | 2.6E-06 |

[Table 36]

| Compound ID | 11 | 10 | 9 | 8 | 7 | 6 | 5 | 4 | 3 | 2 | 1 | H-1 | H-2 | H-3 | C-term | cLogP/ total AA | Caco-2 (cm/sec) |
|---|---|---|---|---|---|---|---|---|---|---|---|---|---|---|---|---|---|
| pd201 | | D-MeAla | MeAla | MePhe | Thr | MeAla | MeLeu | Ile | MeLeu | MeVal | Asp | MePhe | MePhe | Ala | pip | 1.12 | 2.3E-06 |
| pd202 | MeAla | MePhe | Leu | MeLeu | Thr | MeGly | MeVal | Ser(tBu) | MePhe | MePhe | MeAsp | | | | pip | 1.26 | 2.1E-06 |
| pd203 | Pro | MePhe | Leu | MeLeu | Thr | MeGly | MeLeu | Ser(tBu) | MePhe | MePhe | Asp | | | | pip | 1.23 | 2.1E-06 |
| pd204 | MeAla | Leu | MeLeu | MePhe | Leu | MePhe | MePhe | Thr | MeGly | MeLeu | Asp | | | | pip | 1.29 | 2.0E-06 |
| pd205 | MeGly | MePhe | Ile | MePhe | Thr | MeGly | MePhe | MeLeu | Ser(tBu) | MeVal | Asp | | | | pip | 1.15 | 1.8E-06 |
| pd206 | D-Pro | MePhe | Leu | MeLeu | Thr | MeGly | MeLeu | Ser(tBu) | MePhe | MePhe | Asp | | | | pip | 1.23 | 1.8E-06 |
| pd207 | | | b-MeAla | MeLeu | MePhe | Leu | MePhe | Thr | MePhe | MeLeu | Asp | | | | pip | 1.28 | 1.7E-06 |
| pd208 | D-Ala | MeLeu | MeAla | MePhe | Ser(tBu) | MePhe | Leu | MeVal | Thr | MePhe | MeAsp | | | | pip | 1.26 | 1.7E-06 |
| pd209 | Ala | MePhe | Leu | MeLeu | Thr | MeGly | MeLeu | Ser(tBu) | MePhe | MePhe | MeAsp | | | | pip | 1.26 | 1.7E-06 |
| pd210 | D-Val | MePhe | Val | MePhe | Thr | MeGly | MeLeu | Ser(tBu) | MePhe | MeLeu | Asp | | | | pip | 1.23 | 1.6E-06 |
| pd211 | | | MeAla | MeLeu | Leu | MePhe | Thr | MePhe | MePhe | MeLeu | Asp | | | | pip | 1.39 | 1.4E-06 |
| pd212 | | | MeAla | Thr | MeAla | MeLeu | Val | MePhe | Phe | MeLeu | Asp | MePhe | Ala | | pip | 1.03 | 1.4E-06 |
| pd213 | MeAla | MeLeu | MeAla | MePhe | Ser(tBu) | MePhe | Leu | MeVal | Thr | MePhe | MeAsp | | | | pip | 1.31 | 1.3E-06 |
| pd214 | Pro | MeLeu | MeAla | MePhe | Ser(tBu) | MePhe | Leu | MeLeu | Thr | MePhe | Asp | | | | pip | 1.28 | 1.2E-06 |
| pd215 | D-Val | MePhe | Leu | MeLeu | Thr | MeGly | MeLeu | Ser(tBu) | MePhe | MePhe | Asp | | | | pip | 1.27 | 1.2E-06 |
| pd216 | g-MeAbu | MeLeu | Val | MePhe | MePhe | Ser(tBu) | MeLeu | Thr | MePhe | MeLeu | Asp | | | | pip | 1.25 | 1.2E-06 |
| pd217 | Ala | MePhe | Leu | MeLeu | Thr | MeGly | MeLeu | Ser(tBu) | MePhe | MePhe | Asp | | | | pip | 1.19 | 1.1E-06 |
| pd218 | | | MeAla | MeLeu | Leu | MeLeu | MePhe | Phe | MeLeu | Thr | Asp | MePhe | Ala | | pip | 1.21 | 1.1E-06 |
| pd219 | D-Pro | MeLeu | MeAla | MePhe | Ser(tBu) | MePhe | Leu | MeVal | Thr | MePhe | MeAsp | | | | pip | 1.29 | 1.1E-06 |
| pd220 | D-Ala | MeLeu | MeAla | MePhe | Ser(tBu) | MePhe | Leu | MeLeu | Thr | MePhe | Asp | | | | pip | 1.24 | 1.1E-06 |
| pd221 | D-Val | MePhe | Leu | MeLeu | Thr | MeGly | MeLeu | Val | MePhe | MePhe | Asp | | | | pip | 1.28 | 1.1E-06 |
| pd222 | D-MeAla | Ile | MeLeu | MeLeu | Phe | MeVal | MeAla | Thr | MeGly | MePhe | Asp | MePhe | Ala | | pip | 1.06 | 1.1E-06 |
| pd223 | | | D-MeAla | MeLeu | MePhe | Leu | MePhe | Thr | MePhe | MeLeu | Asp | | | | pip | 1.39 | 1.1E-06 |
| pd224 | D-Ala | MePhe | Leu | MeLeu | Thr | MeGly | MeLeu | Ser(tBu) | MePhe | MePhe | MeAsp | | | | pip | 1.26 | 1.0E-06 |
| pd225 | D-Leu | MePhe | Leu | MePhe | Thr | MeGly | MeLeu | Ile | MePhe | MeLeu | Asp | | | | pip | 1.37 | 1.0E-06 |

[Table 37]

Sequences having $P_{app} < 1.0 \times 10^{-6}$ at ARC = 4

| Compound ID | 11 | 10 | 9 | 8 | 7 | 6 | 5 | 4 | 3 | 2 | 1 | H-1 | H-2 | H-3 | C-term | cLogP/ total AA | Caco-2 (cm/sec) |
|---|---|---|---|---|---|---|---|---|---|---|---|---|---|---|---|---|---|
| pd226 | MeAla | MePhe | Leu | MeLeu | Thr | MeAla | MeAla(4-Thz) | Phe | MePhe | MeLeu | Asp | | | | pip | 1.19 | 6.8E-07 |
| pd227 | MePhe | Leu | MeLeu | MePhe | Leu | MePhe | MePhe | Thr | MeGly | MeLeu | Asp | | | | pip | 1.42 | 6.1E-07 |
| pd228 | MeLeu | Leu | MeLeu | MePhe | Phe | MePhe | MeAla(4-Thz) | Thr | MeGly | MeLeu | Asp | | | | pip | 1.27 | 5.8E-07 |
| pd229 | MeVal | MePhe | Leu | MeLeu | MeAla | MeGly | MePhe | Phe | MePhe | Thr | Asp | | | | pip | 1.24 | 4.1E-07 |
| pd230 | MePhe | Phe | Thr | MePhe | Leu | MePhe | MeLeu | MeGly | MeLeu | MeAla | Asp | | | | pip | 1.29 | 2.5E-07 |
| pd231 | MeAla | MePhe | Leu | MeLeu | Phe | MeGly | MePhe | Thr | MePhe | Leu | Asp | | | | pip | 1.24 | 2.1E-07 |
| pd232 | MeAla | MeLeu | MePhe | Ala | MeLeu | MePhe | MePhe | Thr | MeAla | MePhe | Asp | | | | pip | 1.26 | 1.9E-07 |
| pd233 | MeAla | MePhe | MeLeu | Val | MeLeu | MePhe | MePhe | Thr | MeAla | MePhe | Asp | | | | pip | 1.35 | 1.7E-07 |
| pd234 | D-Ala | MeLeu | Leu | MePhe | Ser(tBu) | MePhe | Thr | MeAla | MePhe | MePhe | Asp | | | | pip | 1.24 | 1.5E-07 |
| pd235 | | Ala | Phe | MeLeu | Ala(4-Thz) | MePhe | Gly | MeLeu | MeLeu | Leu | Asp | MePhe | Ala | | pip | 1.06 | 1.0E-07 |
| pd236 | MeAla | Leu | MeLeu | Phe | Phe | MePhe | MeAla(4-Thz) | Thr | MeGly | MeLeu | Asp | | | | pip | 1.08 | 9.6E-08 |
| pd237 | Ala | MePhe | MeAla | MePhe | MeLeu | Phe | MeLeu | Ser(tBu) | Thr | MePhe | Asp | | | | pip | 1.23 | 8.9E-08 |
| pd238 | MeAla | MePhe | Leu | Thr | MeLeu | MeAla | MeAla(4-Thz) | Phe | MePhe | MeLeu | Asp | | | | pip | 1.19 | 8.8E-08 |
| pd239 | | b-MeAla | MePhe | MeLeu | MeAla | MePhe | Ile | MeLeu | MeLeu | Thr | Asp | MePhe | MePhe | Ala | pip | 1.18 | 6.5E-08 |
| pd240 | | MeAla | MePhe | Ile | MeAla | MeLeu | MeLeu | Thr | MePhe | MeLeu | Asp | MePhe | MePhe | Ala | pip | 1.27 | 6.0E-08 |
| pd241 | | | D-Val | Ala | Thr | MeAla | Phe | MeLeu | MePhe | MeLeu | Asp | MePhe | Phe | | pip | 1.11 | 5.8E-08 |
| pd242 | | D-Ala | Thr | MePhe | Ile | MeAla | MeLeu | MePhe | MeLeu | MeLeu | Asp | MePhe | MePhe | Ala | pip | 1.22 | 5.8E-08 |
| pd243 | D-Leu | MeLeu | MePhe | MePhe | Ser(tBu) | MePhe | Leu | MeLeu | Thr | MePhe | Asp | | | | pip | 1.50 | 5.7E-08 |
| pd244 | Ala | Phe | MeLeu | MePhe | Leu | Thr | MeLeu | Phe | MeGly | MePhe | Asp | | | | pip | 1.18 | 4.5E-08 |
| pd245 | MeLeu | Thr | Leu | MePhe | MePhe | MeGly | Phe | MeAla | Phe | Ser(tBu) | Asp | | | | pip | 1.11 | 1.9E-08 |
| pd246 | D-Ala | MePhe | MeGly | Leu | Thr | Phe | MeLeu | Ser(tBu) | MePhe | MePhe | Asp | | | | pip | 1.13 | 1.4E-08 |
| pd247 | | | Ala | Thr | MeAla | MePhe | Val | MePhe | MeLeu | Gly | Asp | MePhe | MePhe | Ala | pip | 0.88 | 8.7E-08 |

Structures of the above-described cyclic peptides are shown below. (In the structural formulae of the cyclic peptides, hydrogen atoms attached to a heteroatom may be omitted to clarify the structure.)

[Table 38]

| ID | Structural Formula | ID | Structural Formula |
|---|---|---|---|
| pd162 | 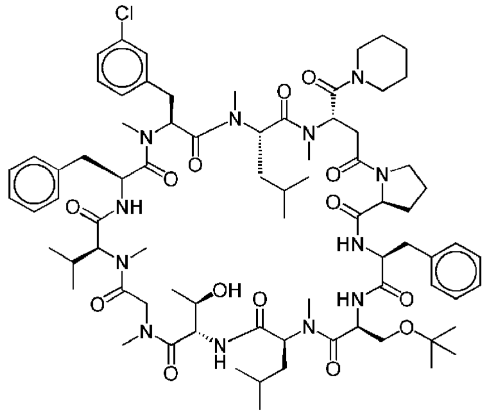 | pd163 | 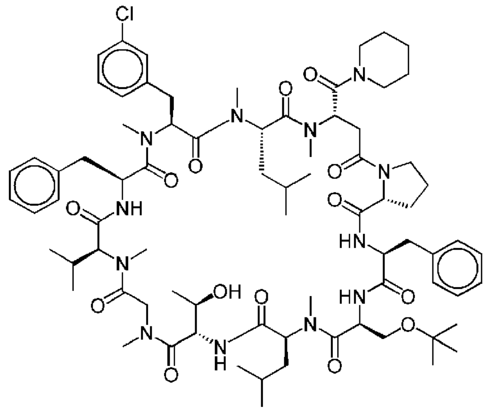 |
| pd167 | 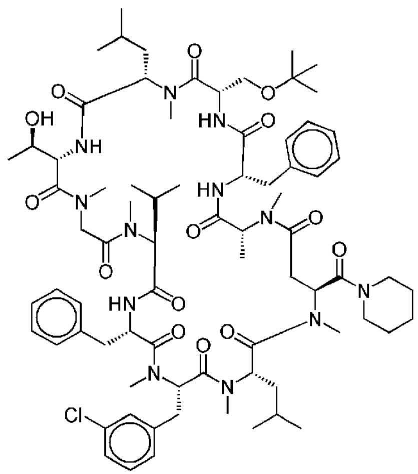 | pd177 | 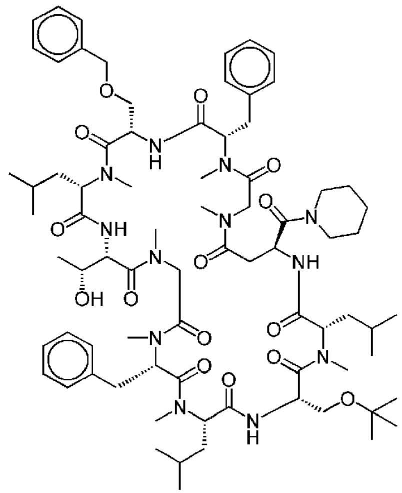 |

(continued)

| ID | Structural Formula | ID | Structural Formula |
|---|---|---|---|
| pd178 | 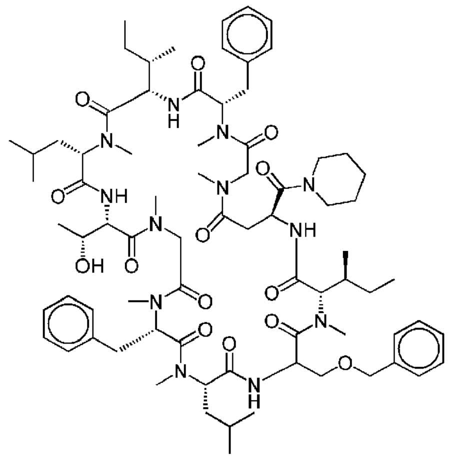 | pd179 | 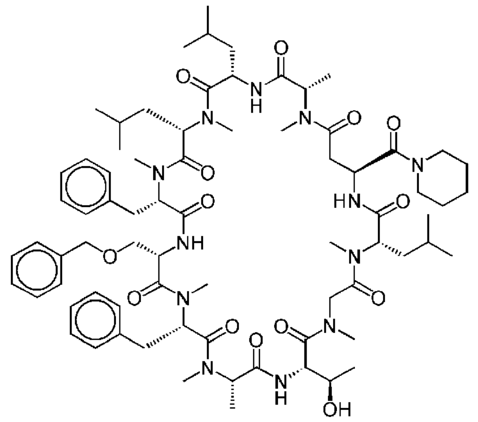 |
| pd180 | 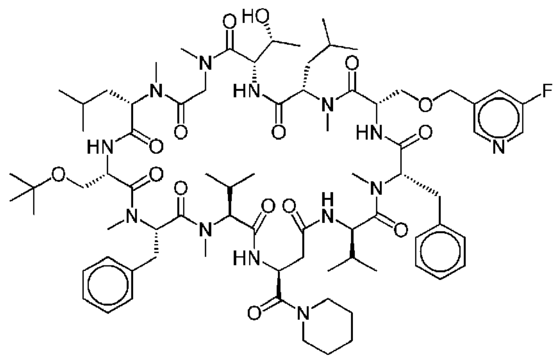 | pd181 | 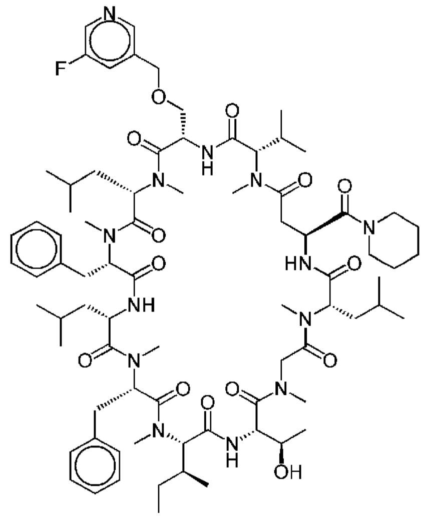 |

(continued)

| ID | Structural Formula | ID | Structural Formula |
|---|---|---|---|
| pd182 | 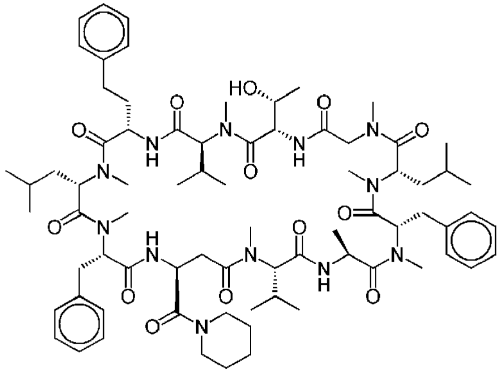 | pd183 | 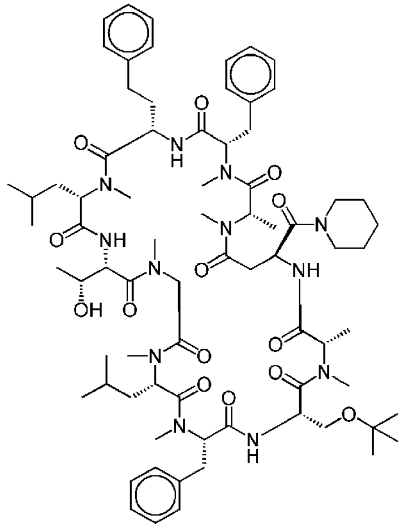 |
| pd184 | 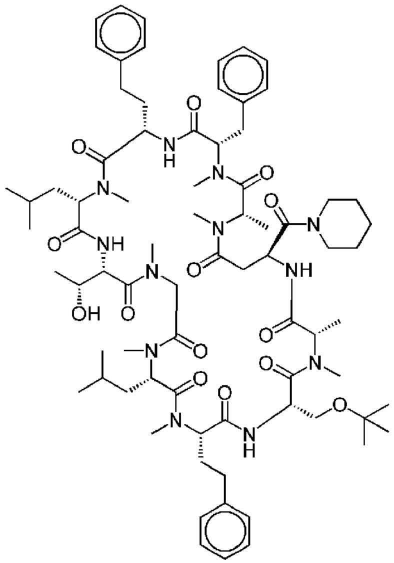 | pd185 | 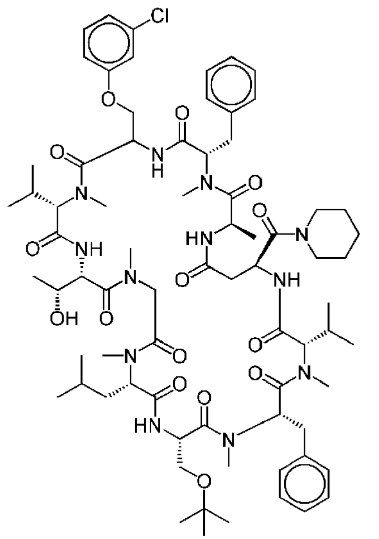 |

(continued)

| ID | Structural Formula | ID | Structural Formula |
|---|---|---|---|
| pd186 | 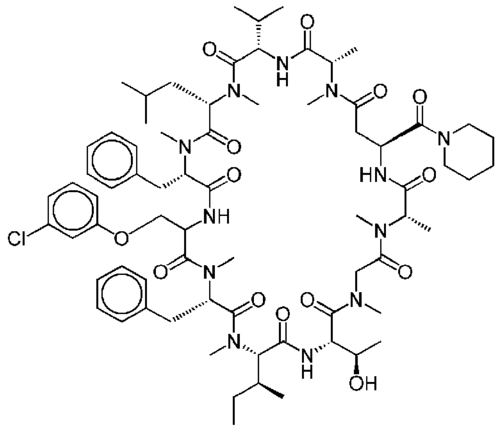 | pd187 | 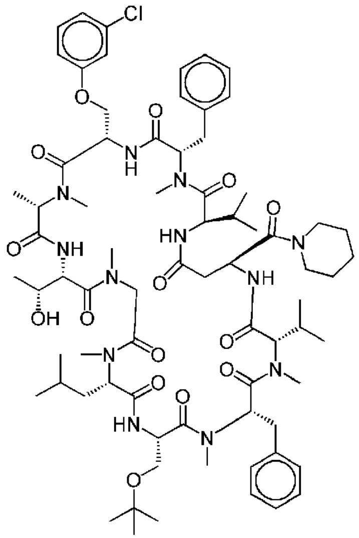 |
| pd188 | 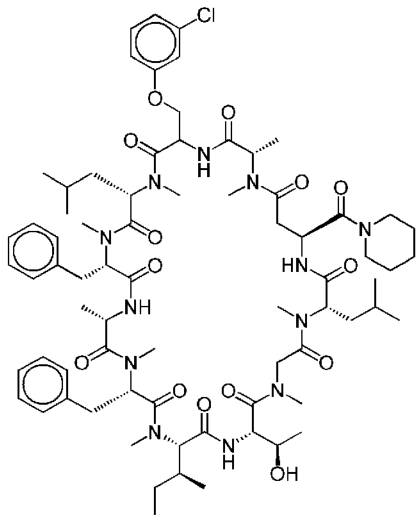 | pd189 | 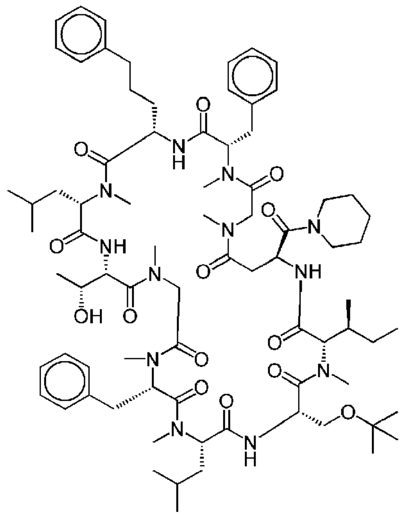 |

(continued)

| ID | Structural Formula | ID | Structural Formula |
|---|---|---|---|
| pd190 | 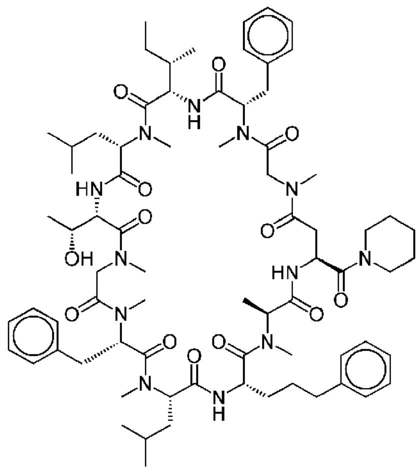 | pd191 | 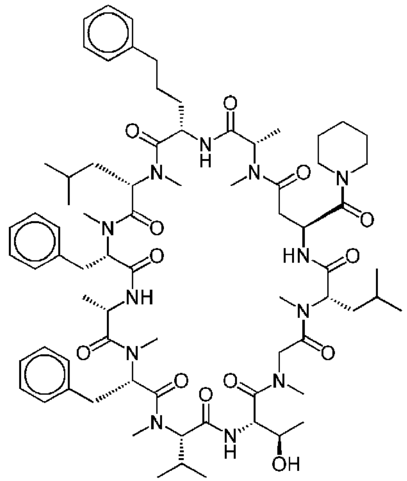 |
| pd192 | 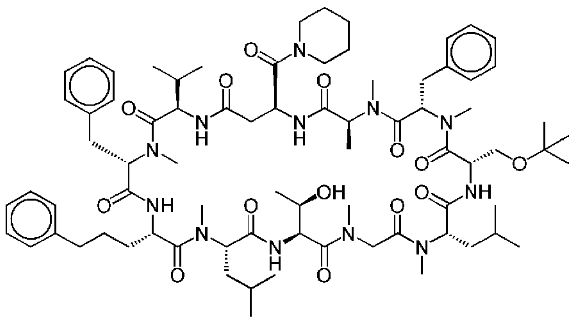 | pd193 | 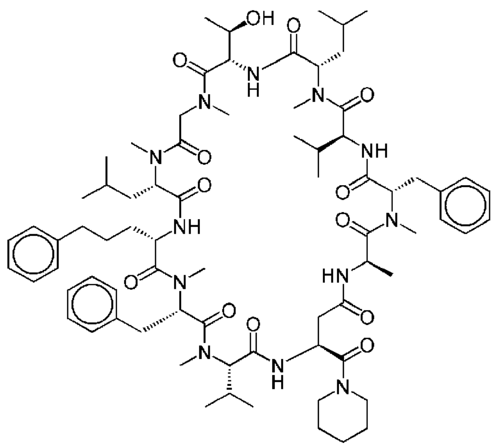 |

(continued)

| ID | Structural Formula | ID | Structural Formula |
|---|---|---|---|
| pd194 | 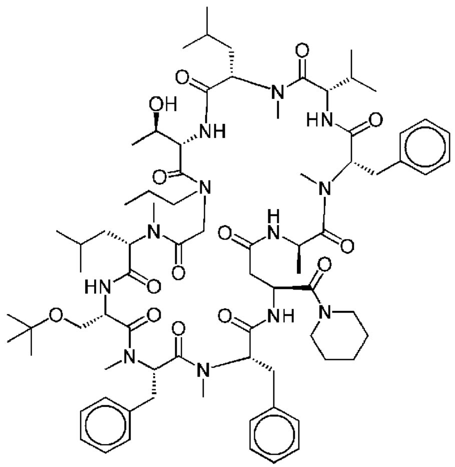 | pd195 | 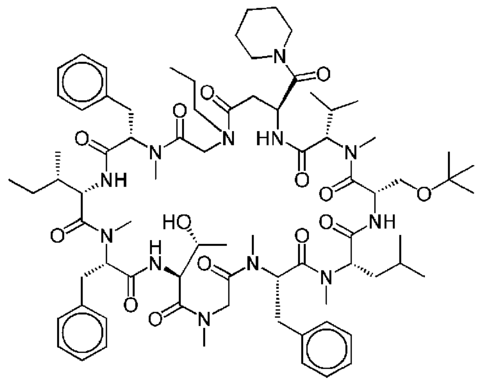 |
| pd196 | 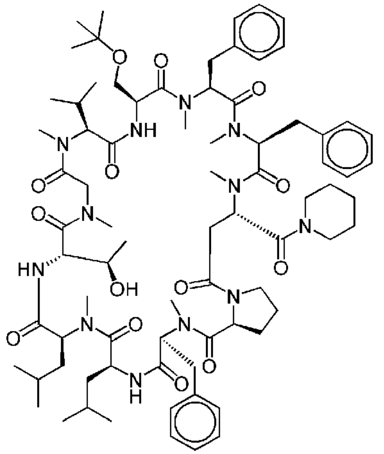 | pd197 | 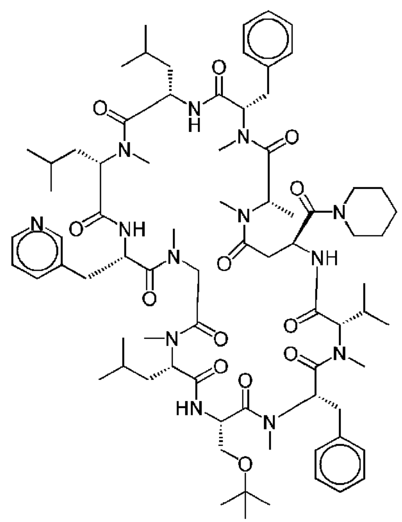 |

(continued)

| ID | Structural Formula | ID | Structural Formula |
|---|---|---|---|
| pd198 | 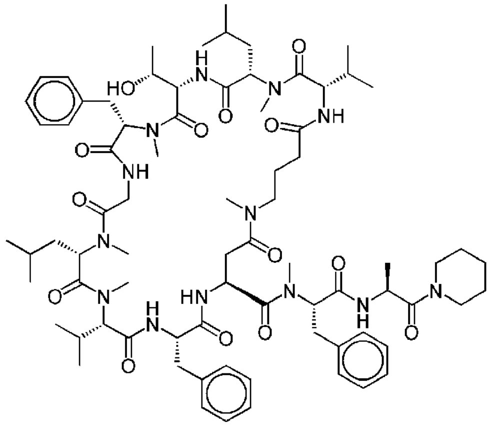 | pd199 | 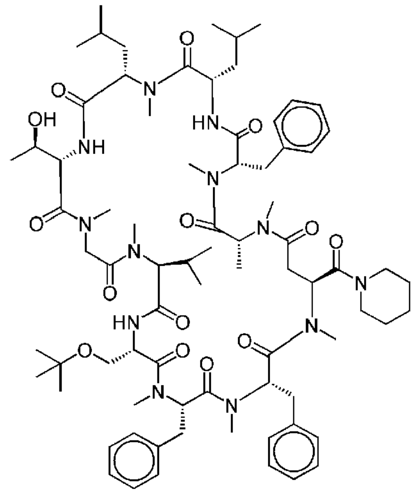 |
| pd200 | 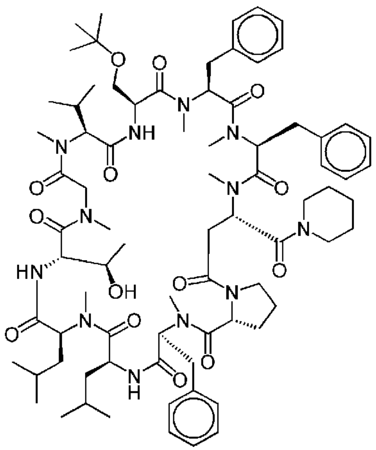 | pd201 | 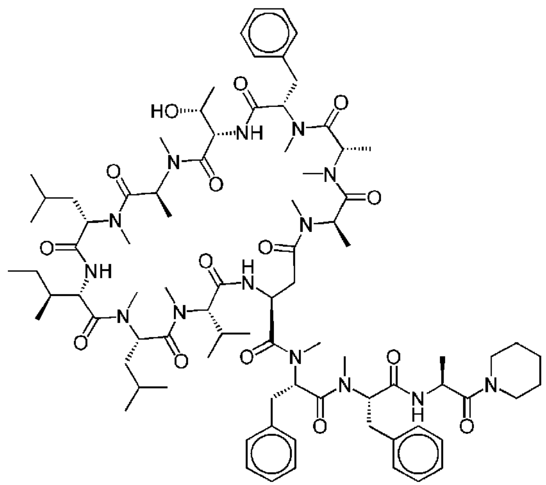 |

(continued)

| ID | Structural Formula | ID | Structural Formula |
|---|---|---|---|
| pd202 | 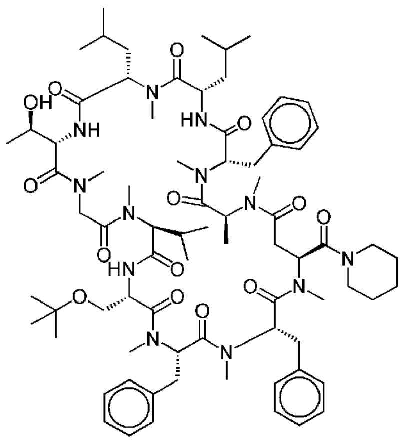 | pd203 | 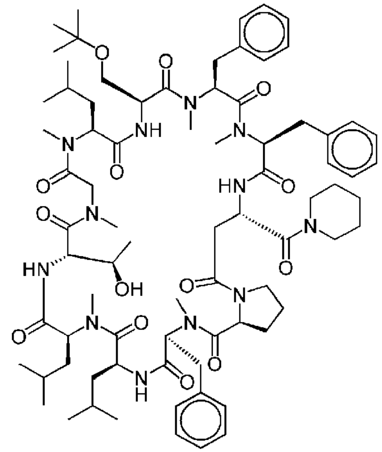 |
| pd204 | 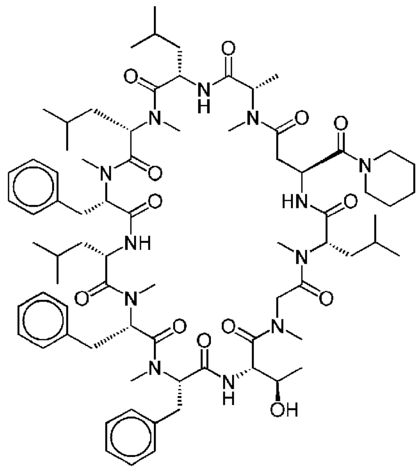 | pd205 | 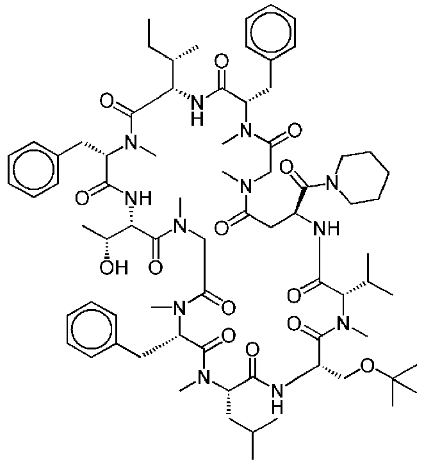 |

(continued)

| ID | Structural Formula | ID | Structural Formula |
|---|---|---|---|
| pd206 | 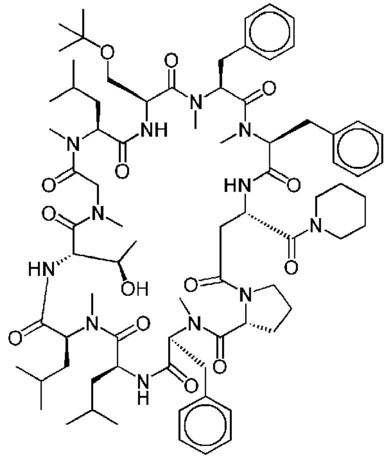 | pd207 | 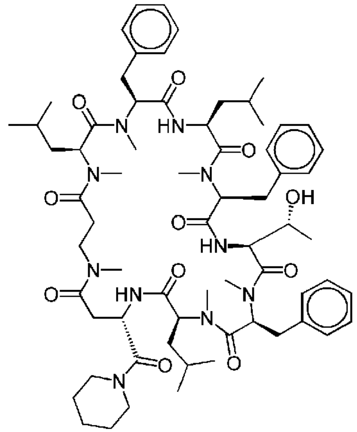 |
| pd208 | 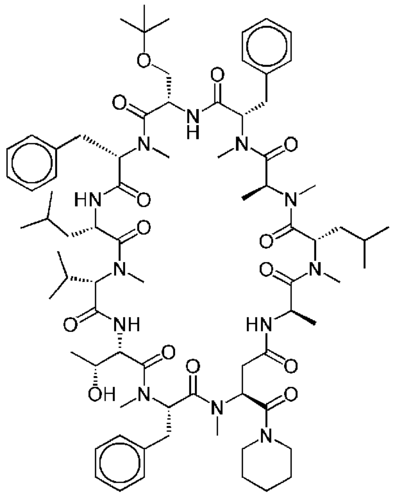 | pd209 | 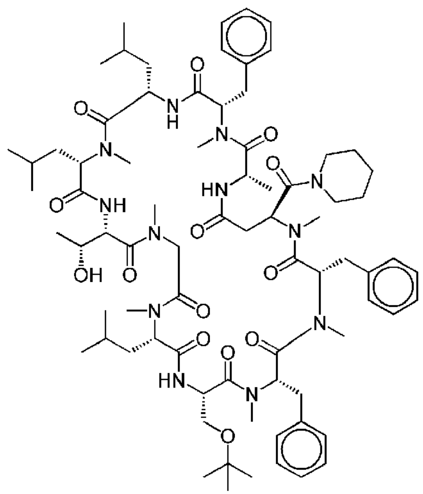 |

(continued)

| ID | Structural Formula | ID | Structural Formula |
|---|---|---|---|
| pd210 | 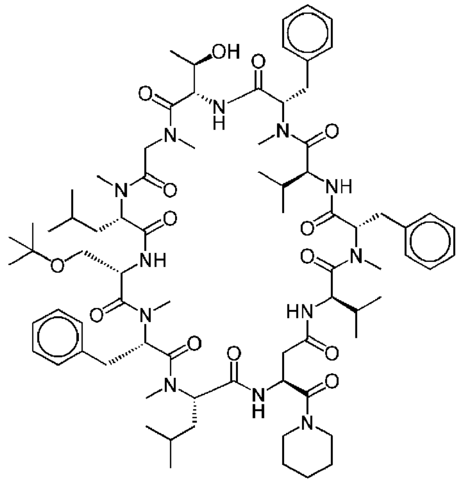 | pd211 | 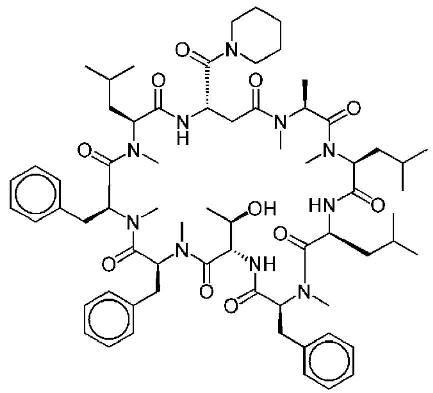 |
| pd212 | 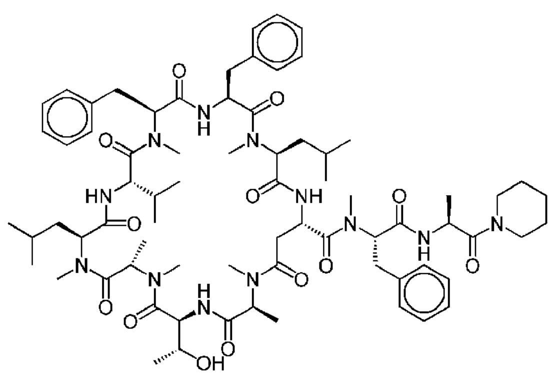 | pd213 | 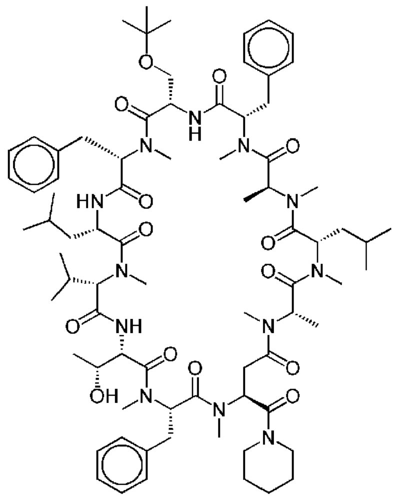 |

(continued)

| ID | Structural Formula | ID | Structural Formula |
|---|---|---|---|
| pd214 | 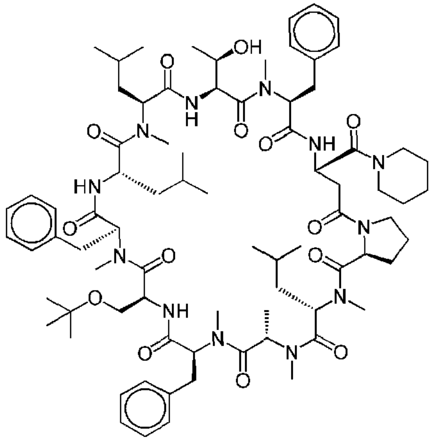 | pd215 | 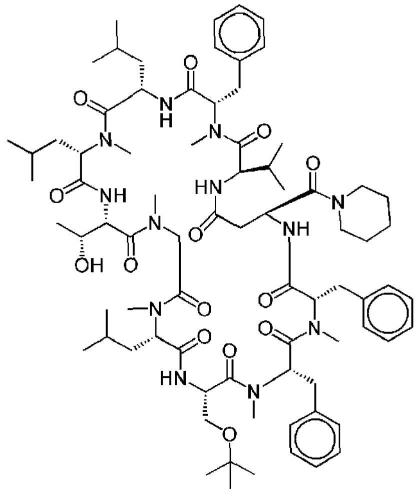 |
| pd216 | 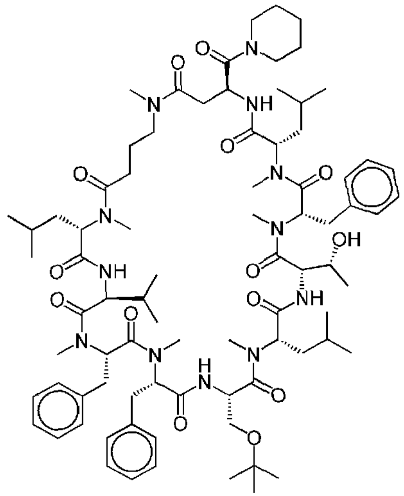 | pd217 | 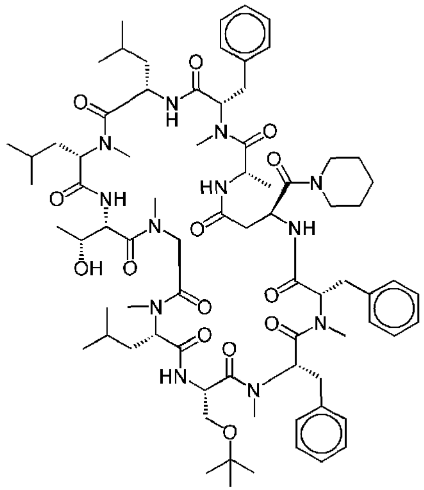 |

(continued)

| ID | Structural Formula | ID | Structural Formula |
|---|---|---|---|
| pd218 | 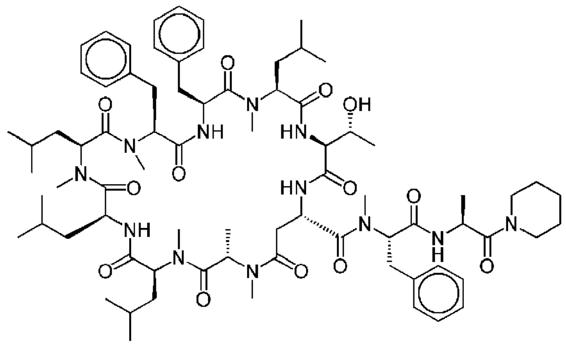 | pd219 | 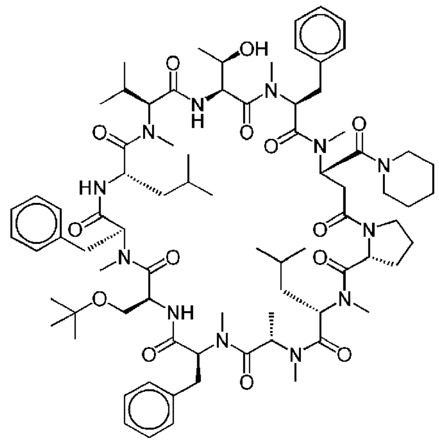 |
| pd220 | 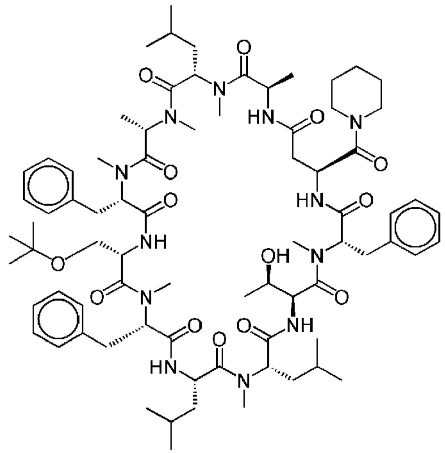 | pd221 | 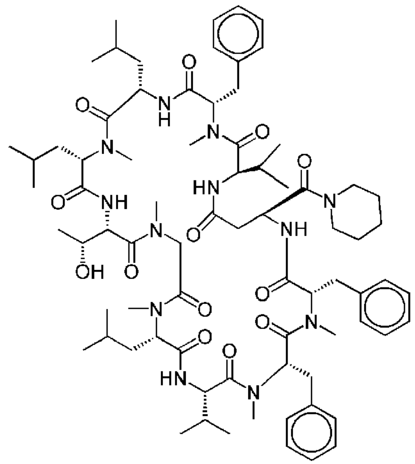 |

(continued)

| ID | Structural Formula | ID | Structural Formula |
|---|---|---|---|
| pd222 | 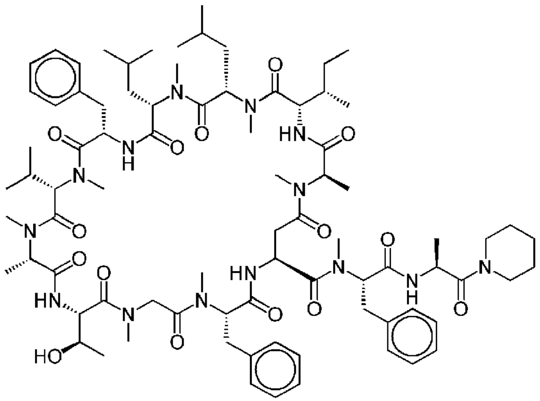 | pd223 | 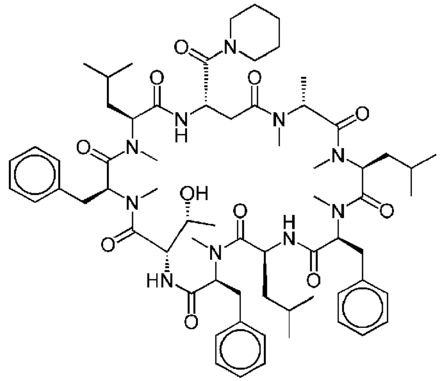 |
| pd224 | 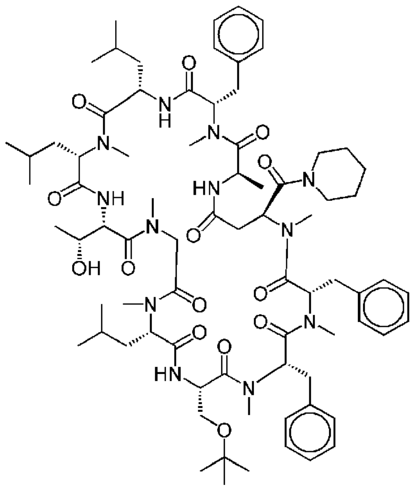 | pd225 | 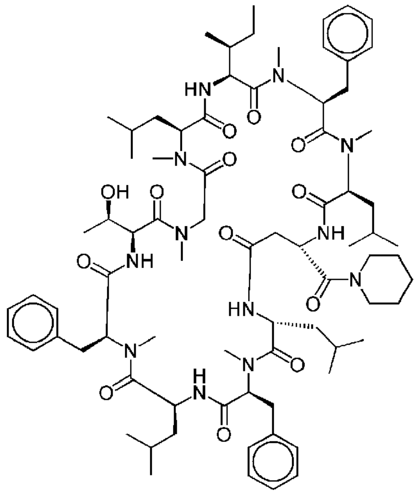 |

(continued)

| ID | Structural Formula | ID | Structural Formula |
|---|---|---|---|
| pd226 | 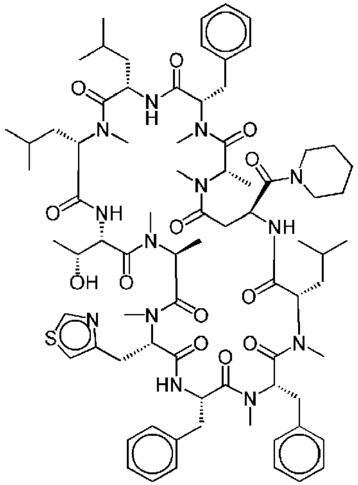 | pd227 | 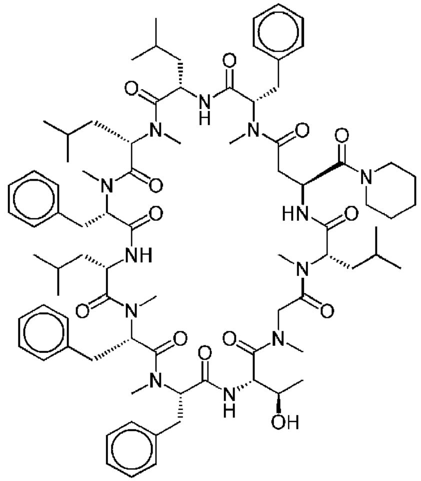 |
| pd228 | 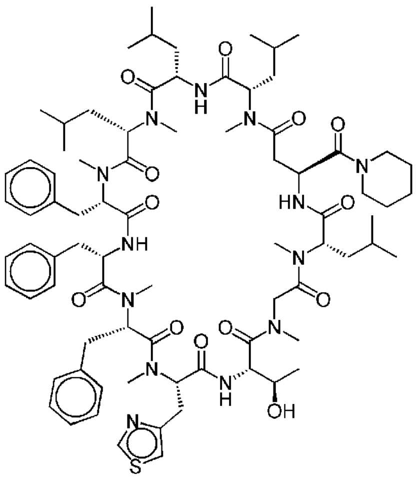 | pd229 | 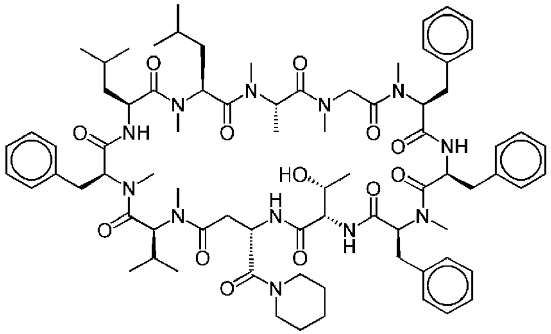 |

(continued)

| ID | Structural Formula | ID | Structural Formula |
|---|---|---|---|
| pd230 | 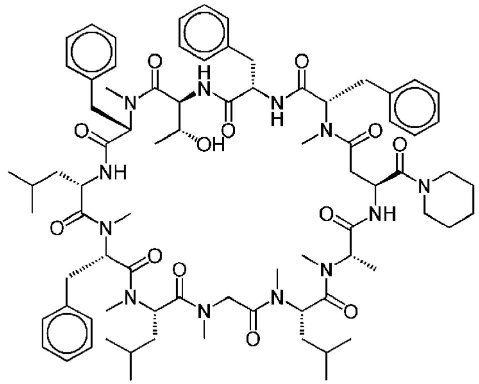 | pd231 | 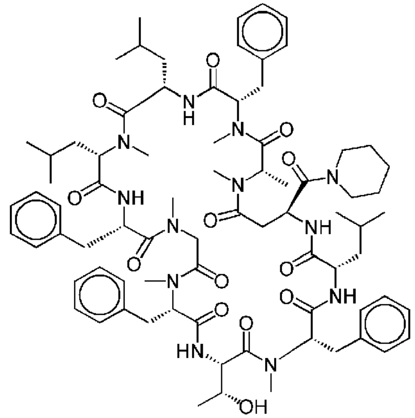 |
| pd232 | 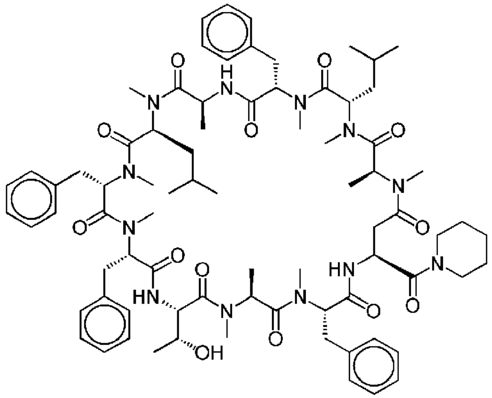 | pd233 | 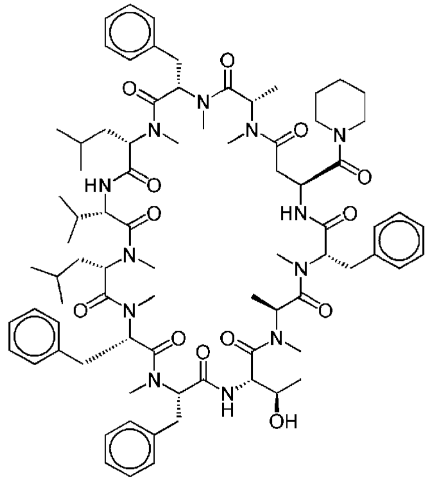 |

(continued)

| ID | Structural Formula | ID | Structural Formula |
|---|---|---|---|
| pd234 | 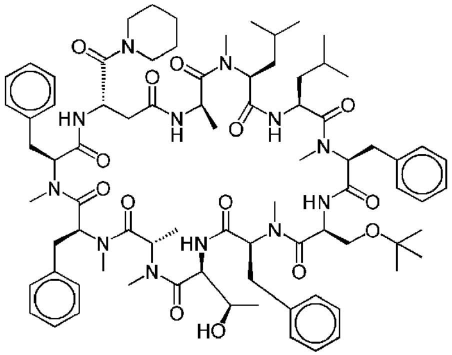 | pd235 | 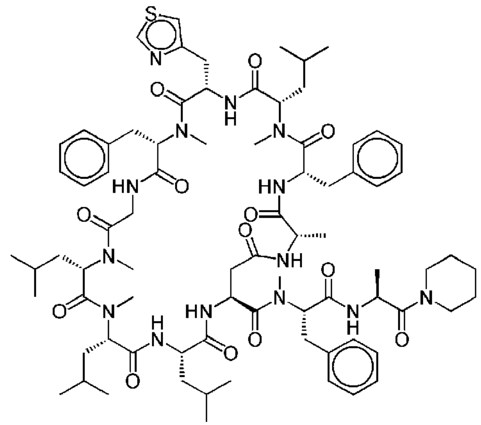 |
| pd236 | 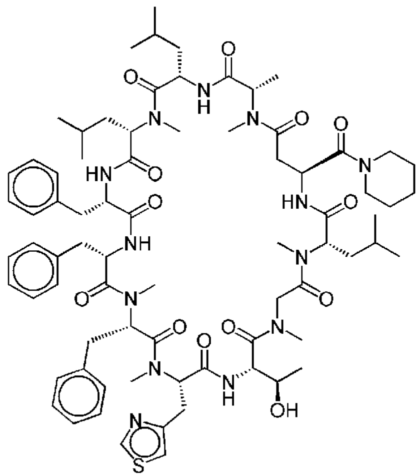 | pd237 | 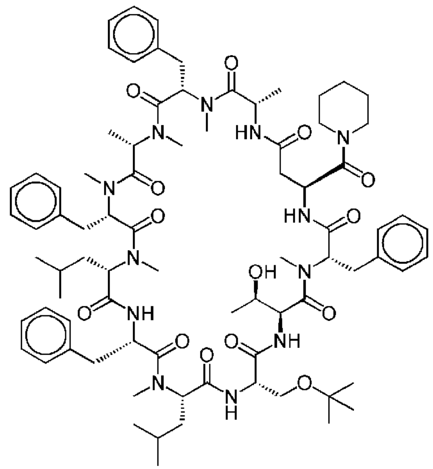 |

(continued)

| ID | Structural Formula | ID | Structural Formula |
|---|---|---|---|
| pd238 | 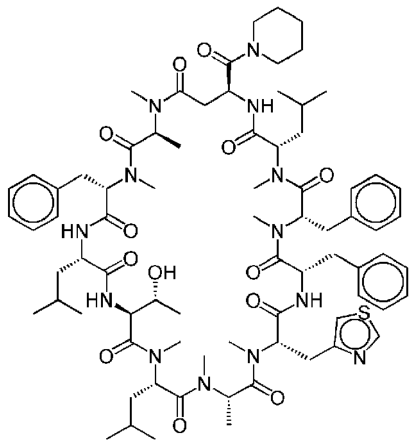 | pd239 | 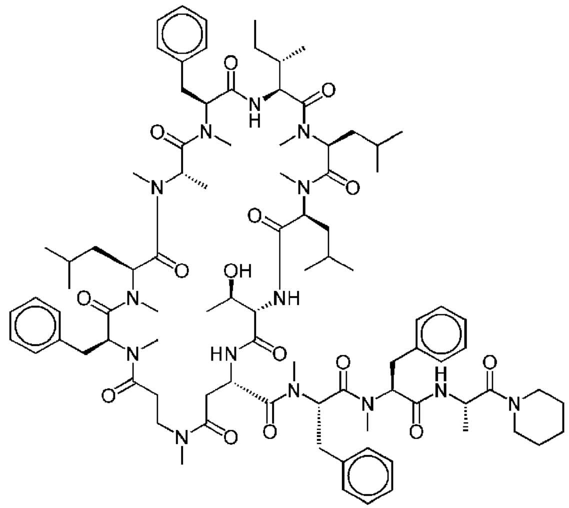 |
| pd240 | 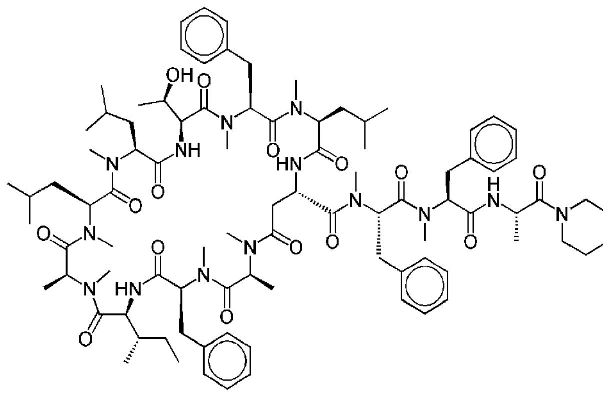 | pd241 | 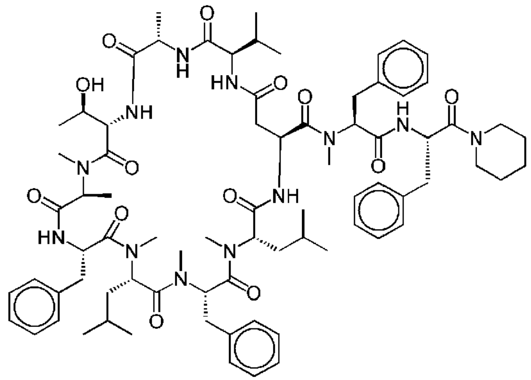 |

(continued)

| ID | Structural Formula | ID | Structural Formula |
|---|---|---|---|
| pd242 | 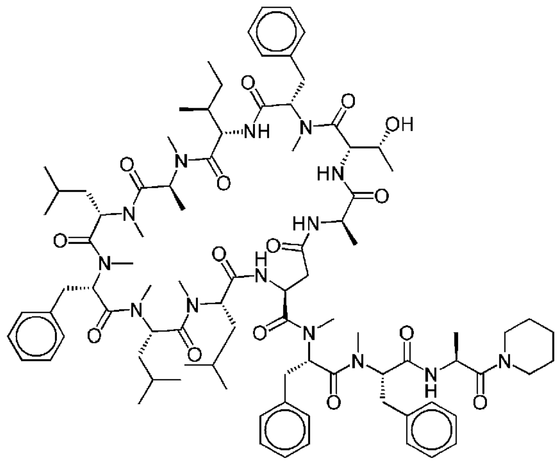 | pd243 | 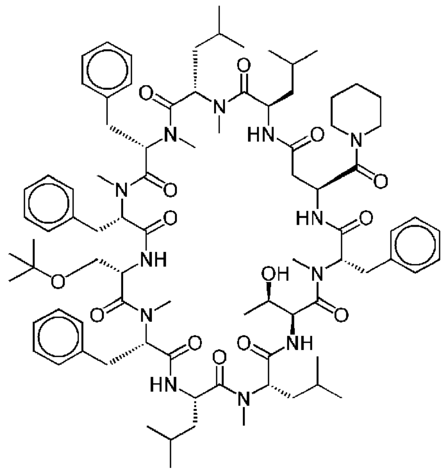 |
| pd244 | 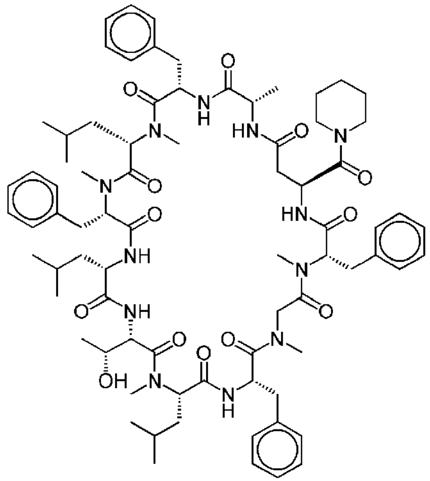 | pd245 | 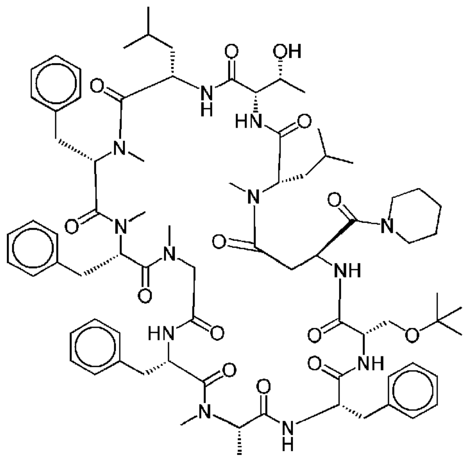 |

(continued)

| ID | Structural Formula | ID | Structural Formula |
|---|---|---|---|
| pd246 | 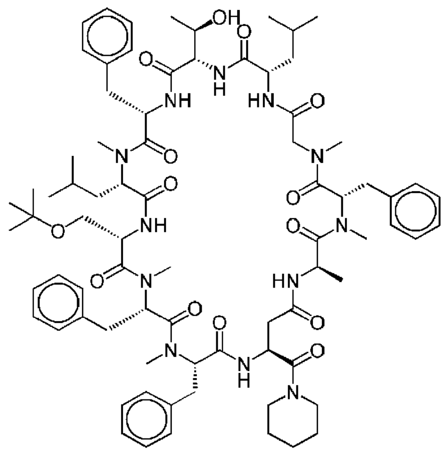 | pd247 | 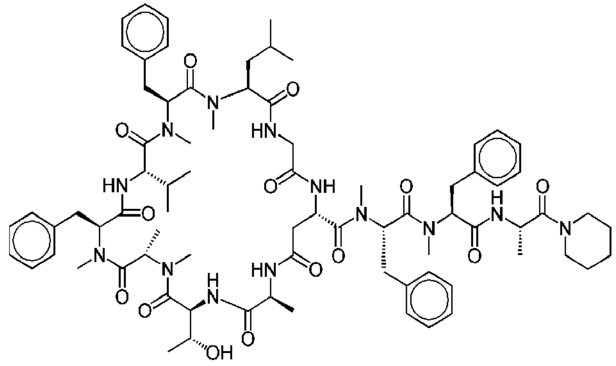 |

Analysis information on cyclic peptides for which membrame permeability was evaluated in Reference Examples is shown below.

[Table 39]

| Compound ID | Analytical Condition | LCMS(ESI) m/z | | Retention Time (min) |
|---|---|---|---|---|
| pd50 | SQDFA05 | 1473 | (M+H)+ | 0.84 |
| pd51 | SQDFA05 | 1471 | (M+H)+ | 0.84 |
| pd52 | SQDFA05 | 1443 | (M+H)+ | 0.79 |
| pd53 | SQDFA05 | 1487 | (M+H)+ | 0.85 |
| pd54 | SQDFA05 | 1485 | (M+H)+ | 0.74, 0.82 |
| pd55 | SQDFA05 | 1485 | (M+H)+ | 0.79, 0.85 |
| pd56 | SQDFA05 | 1443 | (M+H)+ | 0.77, 0.82 |
| pd57 | SQDFA05 | 1429 | (M+H)+ | 0.71 |
| pd58 | SQDFA05 | 1459 | (M+H)+ | 0.81 |
| pd59 | SQDAA50 | 1553 | (M+H)+ | 0.80 |
| pd60 | SQDAA50 | 1587 | (M+H)+ | 0.79 |
| pd61 | SQDAA50 | 1559 | (M+H)+ | 0.77 |
| pd62 | SQDAA50 | 1533 | (M+H)+ | 0.76 |
| pd63 | SQDAA50 | 1539 | (M+H)+ | 0.76 |
| pd64 | SQDAA50 | 1553 | (M+H)+ | 0.75 |
| pd65 | SQDAA50 | 1533 | (M+H)+ | 0.76 |
| pd66 | SQDAA50 | 1567 | (M+H)+ | 0.77 |
| pd67 | SQDAA50 | 1547 | (M+H)+ | 0.80 |
| pd68 | SQDAA50 | 1545 | (M+H)+ | 0.73 |
| pd69 | SQDAA50 | 1517 | (M+H)+ | 0.75 |
| pd70 | SQDAA50 | 1503 | (M+H)+ | 0.74 |
| pd100 | SQDFA05 | 1339.5 | (M+H)+ | 0.91 |
| pd 101 | SQDFA05 | 1303 | (M+H)+ | 1.02 |
| pd102 | SQDFA05 | 1361 | (M+H)+ | 1.00 |
| pd103 | SQDFA05 | 1361 | (M+H)+ | 1.12 |
| pd104 | SQDFA05 | 1395 | (M+H)+ | 1.03 |
| pd105 | SQDFA05 | 1361 | (M+H)+ | 1.01 |
| pd106 | SQDFA05 | 1419 | (M+H)+ | 1.11 |
| pd107 | SQDFA05 | 1509 | (M+H)+ | 1.12 |
| pd108 | SQDFA05 | 1389 | (M+H)+ | 1.11 |
| pd109 | SQDFA05 | 1423 | (M+H)+ | 1.06 |
| pd110 | SQDFA05 | 1445 | (M+H)+ | 1.17 |
| pd111 | SQDFA05 | 1451 | (M+H)+ | 1.13 |
| pd112 | SQDFA05 | 1330 | (M+H)+ | 0.89 |
| pd113 | SQDFA05 | 1348 | (M+H)+ | 0.84 |
| pd114 | SQDFA05 | 1448 | (M+H)+ | 1.02 |

(continued)

| Compound ID | Analytical Condition | LCMS(ESI) m/z | | Retention Time (min) |
|---|---|---|---|---|
| pd115 | SQDFA05 | 1344 | (M+H)+ | 0.89 |
| pd116 | SQDFA05 | 1300 | (M+H)+ | 0.90 |
| pd117 | SQDFA05 | 1358 | (M+H)+ | 0.96 |
| pd118 | SQDFA05 | 1420 | (M+H)+ | 1.00 |
| pd119 | SQDFA05 | 1432 | (M+H)+ | 0.98 |
| pd120 | SQDFA05 | 1332 | (M+H)+ | 0.94 |
| pd121 | SQDFA05 | 1372 | (M+H)+ | 1.00 |
| pd122 | SQDFA05 | 1390 | (M+H)+ | 1.01 |
| pd123 | SQDFA05 | 1390 | (M+H)+ | 0.97 |
| pd124 | SQDFA05 | 1390 | (M+H)+ | 0.98 |
| pd125 | SQDFA05 | 1442 | (M+H)+ | 1.07 |
| pd126 | SQDFA05 | 1342 | (M+H)+ | 1.01 |
| pd127 | SQDFA05 | 1400 | (M+H)+ | 1.06 |
| pd128 | SQDFA05 | 1418 | (M+H)+ | 1.05 |
| pd129 | SQDFA05 | 1456 | (M+H)+ | 1.06 |
| pd162 | SQDFA05 | 1466 | (M+H)+ | 1.11 |
| pd163 | SQDFA05 | 1466 | (M+H)+ | 1.13 |
| pd167 | SQDFA05 | 1454 | (M+H)+ | 1.12 |
| pd177 | SQDFA05 | 1450 | (M+H)+ | 1.09 |
| pd178 | SQDFA05 | 1420 | (M+H)+ | 1.09 |
| pd179 | SQDFA05 | 1392 | (M+H)+ | 1.05 |
| pd180 | SQDFA05 | 1483 | (M+H)+ | 1.13 |
| pd181 | SQDFA05 | 1481 | (M+H)+ | 1.17 |
| pd182 | SQDFA05 | 1390 | (M+H)+ | 1.08 |
| pd183 | SQDFA05 | 1406 | (M+H)+ | 1.09 |
| pd184 | SQDFA05 | 1420 | (M+H)+ | 1.10 |
| pd185 | SQDFA05 | 1442 | (M+H)+ | 1.12 |
| pd186 | SQDFA05 | 1398 | (M+H)+ | 1.06 |
| pd187 | SQDFA05 | 1442 | (M+H)+ | 1.12 |
| pd188 | SQDFA05 | 1412 | (M+H)+ | 1.09 |
| pd189 | SQDFA05 | 1448 | (M+H)+ | 1.13 |
| pd190 | SQDFA05 | 1376 | (M+H)+ | 1.05 |
| pd191 | SQDFA05 | 1376 | (M+H)+ | 1.05 |
| pd192 | SQDFA05 | 1434 | (M+H)+ | 1.13 |
| pd193 | SQDFA05 | 1390 | (M+H)+ | 1.08 |
| pd194 | SQDFA50 | 1434 | (M+H)+ | 0.82 |
| pd195 | SQDFA50 | 1434 | (M+H)+ | 0.78 |
| pd196 | SQDFA05 | 1446 | (M+H)+ | 1.15 |

(continued)

| Compound ID | Analytical Condition | LCMS(ESI) m/z | | Retention Time (min) |
|---|---|---|---|---|
| pd197 | SQDFA05 | 1433 | (M+H)+ | 0.93 |
| pd198 | SQDFA05 | 1447 | (M+H)+ | 1.03 |
| pd199 | SQDFA05 | 1434 | (M+H)+ | 1.13 |
| pd200 | SQDFA05 | 1446 | (M+H)+ | 1.15 |
| pd201 | SQDAA50 | 1574 | (M+H)+ | 0.90 |
| pd202 | SQDFA05 | 1434 | (M+H)+ | 1.12 |
| pd203 | SQDFA05 | 1446 | (M+H)+ | 1.13 |
| pd204 | SQDFA05 | 1404 | (M+H)+ | 1.10 |
| pd205 | SQDFA05 | 1406 | (M+H)+ | 1.03 |
| pd206 | SQDFA05 | 1446 | (M+H)+ | 1.12 |
| pd207 | SQDFA05 | 1220 | (M+H)+ | 0.82 |
| pd208 | SQDFA05 | 1434 | (M+H)+ | 1.12 |
| pd209 | SQDFA05 | 1434 | (M+H)+ | 1.16 |
| pd210 | SQDFA05 | 1434 | (M+H)+ | 1.13 |
| pd211 | SQDFA05 | 1220 | (M+H)+ | 1.05 |
| pd212 | SQDFA05 | 1348 | (M+H)+ | 1.04 |
| pd213 | SQDFA05 | 1448 | (M+H)+ | 1.12 |
| pd214 | SQDFA05 | 1460 | (M+H)+ | 1.13 |
| pd215 | SQDFA05 | 1448 | (M+H)+ | 1.15 |
| pd216 | SQDFA05 | 1490 | (M+H)+ | 1.16 |
| pd217 | SQDFA05 | 1420 | (M+H)+ | 1.09 |
| pd218 | SQDFA05 | 1404 | (M+H)+ | 1.14 |
| pd219 | SQDFA05 | 1460 | (M+H)+ | 1.06 |
| pd220 | SQDFA05 | 1434 | (M+H)+ | 1.15 |
| pd221 | SQDFA05 | 1404 | (M+H)+ | 1.10 |
| pd222 | SQDFA05 | 1544 | (M-H)- | 1.06 |
| pd223 | SQDFA05 | 1220 | (M+H)+ | 1.02 |
| pd224 | SQDFA05 | 1434 | (M+H)+ | 1.16 |
| pd225 | SQDFA05 | 1432 | (M+H)+ | 1.16 |
| pd226 | SQDFA05 | 1459 | (M+H)+ | 1.06 |
| pd227 | SQDFA05 | 1480 | (M+H)+ | 1.19 |
| pd228 | SQDFA05 | 1487 | (M+H)+ | 1.14 |
| pd229 | SQDFA05 | 1424 | (M+H)+ | 1.10 |
| pd230 | SQDFA05 | 1438 | (M+H)+ | 1.13 |
| pd231 | SQDFA05 | 1424 | (M+H)+ | 1.08 |
| pd232 | SQDFA05 | 1424 | (M+H)+ | 1.07 |
| pd233 | SQDFA05 | 1452 | (M+H)+ | 1.13 |
| pd234 | SQDFA05 | 1468 | (M+H)+ | 1.11 |

(continued)

| Compound ID | Analytical Condition | LCMS(ESI) m/z | | Retention Time (min) |
|---|---|---|---|---|
| pd235 | SQDFA05 | 1500 | (M+H)+ | 1.11 |
| pd236 | SQDFA05 | 1431 | (M+H)+ | 0.99 |
| pd237 | SQDFA05 | 1468 | (M+H)+ | 1.14 |
| pd238 | SQDFA05 | 1459 | (M+H)+ | 1.02 |
| pd239 | SQDFA05 | 1664 | (M+H)+ | 1.11 |
| pd240 | SQDAA50 | 1664 | (M+H)+ | 0.95 |
| pd241 | SQDFA50 | 1410 | (M+H)+ | 0.76 |
| pd242 | SQDAA50 | 1650 | (M+H)+ | 0.94 |
| pd243 | SQDAA50 | 1552 | (M+H)+ | 0.94 |
| pd244 | SQDFA05 | 1410 | (M+H)+ | 1.03 |
| pd245 | SQDFA05 | 1440 | (M+H)+ | 0.99 |
| pd246 | SQDFA05 | 1440 | (M+H)+ | 1.05 |
| pd247 | SQDFA05 | 1439 | (M+H)+ | 0.93 |
| pd386 | SQDFA05 | 1505 | (M+H)+ | 0.84 |
| pd387 | SQDFA05 | 1491 | (M+H)+ | 0.84 |
| pd388 | SQDFA05 | 1475 | (M+H)+ | 0.87 |
| pd389 | SQDFA05 | 1467 | (M+H)+ | 0.80 |
| pd390 | SQDFA05 | 1489 | (M+H)+ | 0.82 |
| pd391 | SQDFA05 | 1447 | (M+H)+ | 0.81 |
| pd392 | SQDFA05 | 1461 | (M+H)+ | 0.85 |
| pd393 | SQDFA05 | 1491 | (M+H)+ | 0.84 |
| pd394 | SQDFA05 | 1475 | (M+H)+ | 0.80 |
| pd395 | SQDFA05 | 1475 | (M+H)+ | 0.79 |
| pd452 | SQDFA05 | 1513 | (M+H)+ | 0.83 |
| pd453 | SQDFA05 | 1455 | (M+H)+ | 0.76 |
| pd454 | SQDFA05 | 1469 | (M+H)+ | 0.78 |
| pd482 | SQDAA50 | 1451 | (M+H)+ | 0.83 |
| pd483 | SQDAA50 | 1437 | (M+H)+ | 0.84 |
| pd484 | SQDAA50 | 1437 | (M+H)+ | 0.86 |
| pd485 | SQDAA50 | 1507 | (M+H)+ | 0.89 |
| pd486 | SQDAA50 | 1421 | (M+H)+ | 0.85 |
| pd487 | SQDAA50 | 1448 | (M+H)+ | 0.79 |

[Industrial Applicability]

The present invention can provide amino acids that improve membrane permeability of a peptide, and peptide compounds that include the amino acids.

## Claims

1. A peptide compound with two or more amino acids connected, wherein at least one of the amino acids is capable of forming a hydrogen bond in a side chain thereof.

2. The peptide compound of claim 1, wherein the amino acid capable of forming a hydrogen bond in a side chain thereof is capable of forming a pseudo 4- to 7-membered ring in the side chain.

3. The peptide compound of claim 1 or 2, wherein the amino acid capable of forming a hydrogen bond in a side chain thereof is represented by Formula A below:

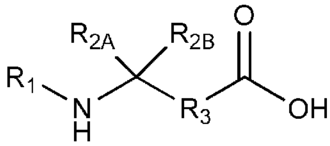 (A) ,

wherein

$R_1$ is hydrogen, $C_1$-$C_6$ alkyl, or a group represented by Formula 1 or Formula 2, and, in this case, as for $R_{2A}$ and $R_{2B}$, $R_{2A}$ is hydrogen, $C_1$-$C_6$ alkyl, or a group represented by Formula 1 or Formula 2, and $R_{2B}$ is hydrogen or $C_1$-$C_6$ alkyl, or $R_{2A}$ and $R_{2B}$ form a 4- to 6-membered ring together with the carbon atom to which they are bonded, or
$R_1$ forms a 4- to 6-membered hetero ring together with the nitrogen atom to which $R_1$ is bonded, $R_{2A}$ and the carbon atom to which $R_{2A}$ is bonded, and the hetero ring optionally has one or more substituents selected from the group consisting of a group represented by Formula 1 or Formula 2, -OH, and an alkoxy group, and, in this case, $R_{2B}$ is hydrogen or $C_1$-$C_6$ alkyl;
$R_3$ is a single bond or $-CHR_4-$;
$R_4$ is hydrogen, $C_1$-$C_4$ alkyl, or a group represented by Formula 1 or Formula 2;
Formula 1 and Formula 2 are respectively represented by the following formulae:

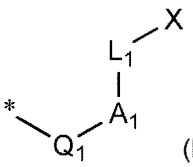 (Formla 1) 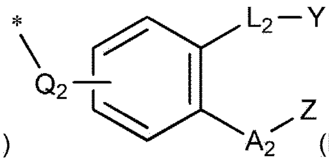 (Formla 2) ,

wherein

* indicates a point of bonding;
$Q_1$ and $Q_2$ are independently a single bond, $C_1$-$C_4$ alkylene, or $C_2$-$C_4$ heteroalkylene containing one oxygen atom;
$A_1$ is -O- or -S-;
$L_1$ is linear $C_1$-$C_3$ alkylene optionally substituted with one or more substituents selected from the group consisting of fluorine, $C_1$-$C_2$ alkyl, $C_1$-$C_2$ fluoroalkyl, and oxo (=O);
$A_2$ is a single bond, -O-, or -S-;
$L_2$ is a single bond or linear $C_1$-$C_3$ alkylene optionally substituted with one or more substituents selected from the group consisting of fluorine, $C_1$-$C_2$ alkyl, $C_1$-$C_2$ fluoroalkyl, and oxo (=O);
X is -OH, $-NR_{Z1}R_{Z2}$, $-CONR_{Z1}R_{Z2}$, or 5- to 6-membered saturated or unsaturated heterocyclyl containing 1 to 3 heteroatoms and optionally substituted with oxo or one or more halogens;
$R_{Z1}$ and $R_{Z2}$ are independently selected from the group consisting of hydrogen, -OH, $C_1$-$C_4$ alkyl, and $C_1$-$C_4$ alkylsulfonyl;
Y is -OH, $C_1$-$C_4$ alkylsulfonylamino, $-NR_{Z3}R_{Z4}$, $-CONR_{Z3}R_{Z4}$, or 5- to 6-membered saturated or unsaturated heterocyclyl containing 1 to 3 heteroatoms and optionally substituted with oxo or halogen;
$R_{Z3}$ and $R_{Z4}$ are independently selected from the group consisting of hydrogen, -OH, $C_1$-$C_4$ alkyl, and $C_1$-$C_4$ alkylsulfonyl; and
Z is hydrogen or $C_1$-$C_4$ alkyl,

provided that when $A_2$ is -O- or -S-, Z is not hydrogen, and the amino acid represented by Formula A contains at least one group represented by either Formula 1 or Formula 2.

**4.** The peptide compound of claim 3, wherein the amino acid represented by Formula A is selected from the group consisting of:

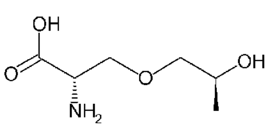 、 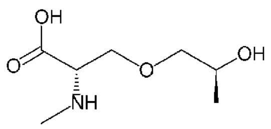 、 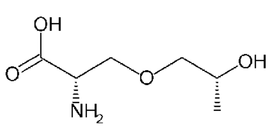 、

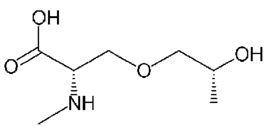 、 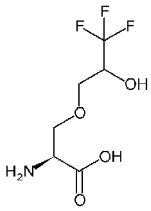 、 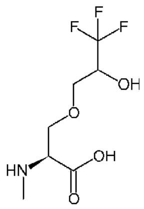 、 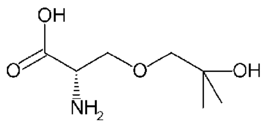 、

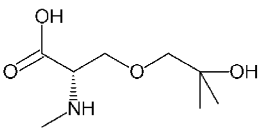 、 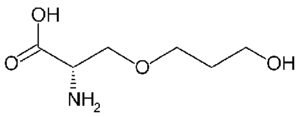 、 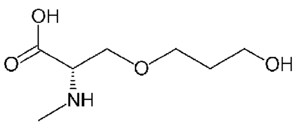 、

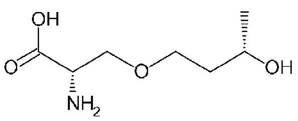 、 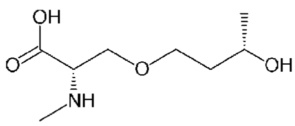 、 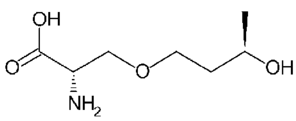 、

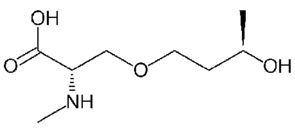 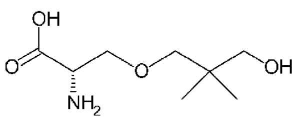 、 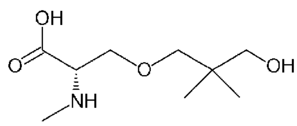 、

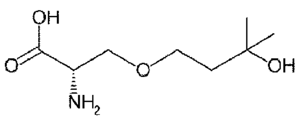 、 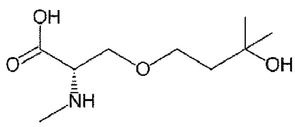 、 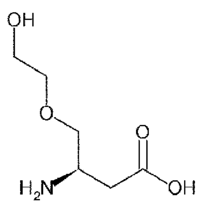 、

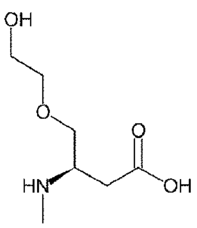 、 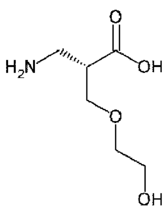 、 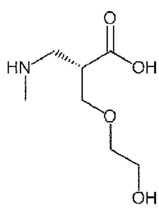 、 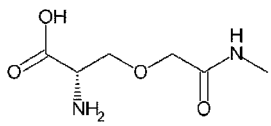 、

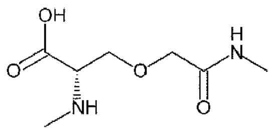, 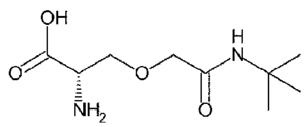, 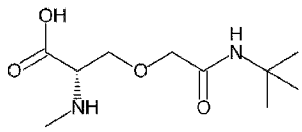,

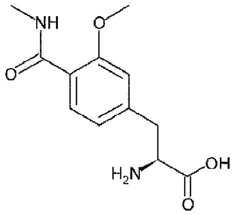, 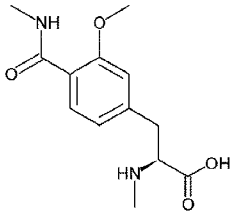, 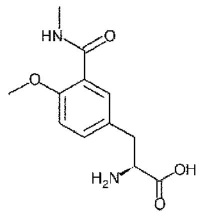, 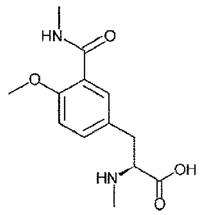,

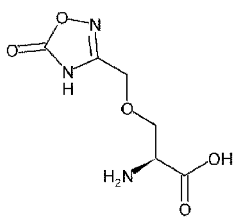, 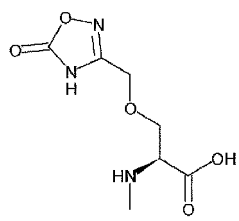, 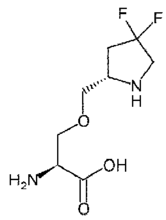, 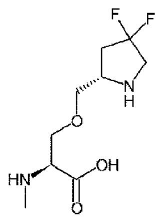,

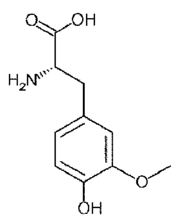, 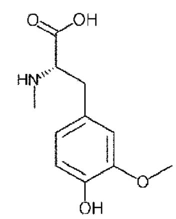, 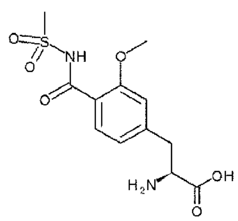, 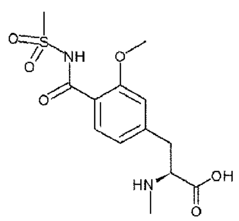,

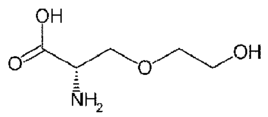, 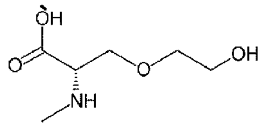,

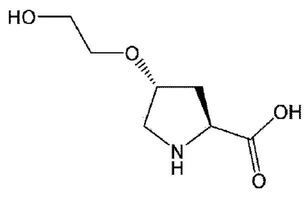, 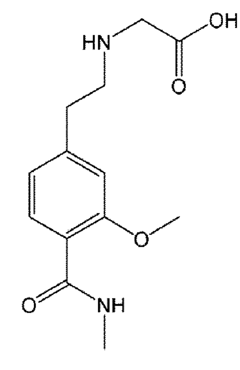, 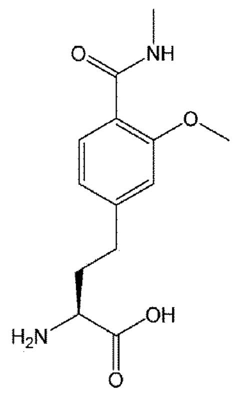, 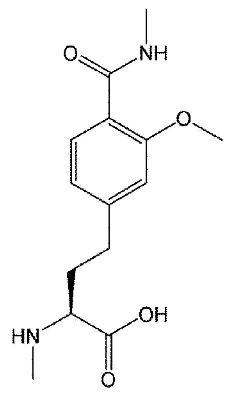.

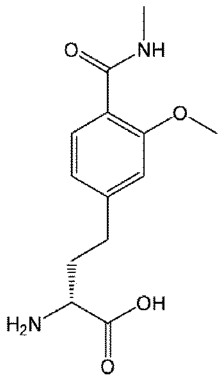 , 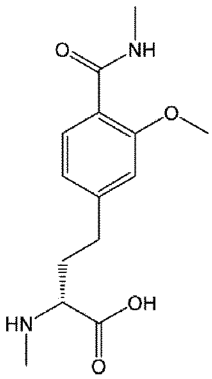 , 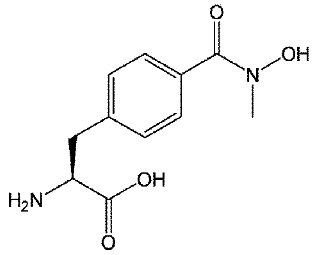 , 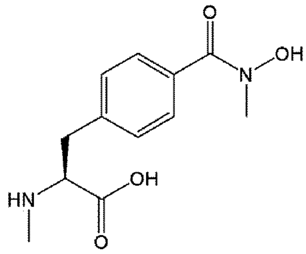 ,

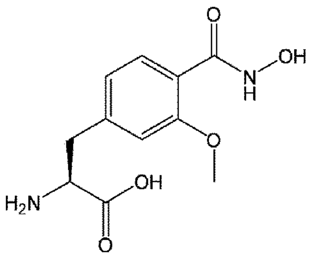 , 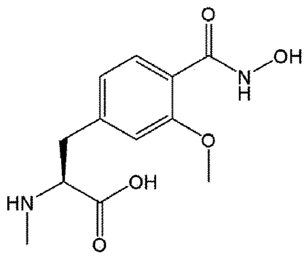 , 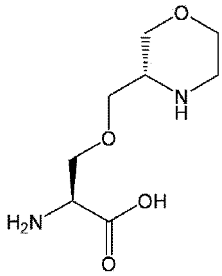 ,

and

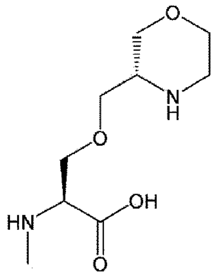

5. The peptide compound of any one of claims 1 to 4, which is composed of 5 to 30 amino acids.

6. The peptide compound of any one of claims 1 to 5, which is cyclic.

7. The peptide compound of claim 6, comprising a cyclic portion composed of 2 to 15 amino acids.

8. The peptide compound of any one of claim 1 to 7, comprising 2 to 30 *N*-substituted amino acids.

9. The peptide compound of any one of claims 1 to 8, wherein a proportion of the number of *N*-substituted amino acids to the total number of amino acids is 30% or higher.

10. The peptide compound of any one of claims 1 to 9, which has a ClogP of 4.0 to 18.

11. The peptide compound of any one of claims 1 to 10, which has a $P_{app}$ of $1.0 \times 10^{-7}$ cm/sec or higher.

12. A library comprising the peptide of any one of claims 1 to 11 and/or a nucleic acid encoding the peptide.

13. An amino acid represented by Formula A below:

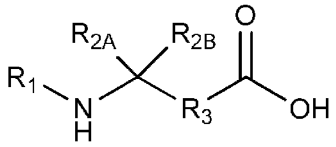 (A)

,

wherein

$R_1$ is hydrogen, $C_1$-$C_6$ alkyl, or a group represented by Formula 1 or Formula 2, and, in this case, as for $R_{2A}$ and $R_{2B}$, $R_{2A}$ is hydrogen, $C_1$-$C_6$ alkyl, or a group represented by Formula 1 or Formula 2, and $R_{2B}$ is hydrogen or $C_1$-$C_6$ alkyl, or $R_{2A}$ and $R_{2B}$ form a 4- to 6-membered ring together with the carbon atom to which they are bonded, or
$R_1$ forms a 4- to 6-membered hetero ring together with the nitrogen atom to which $R_1$ is bonded, $R_{2A}$ and the carbon atom to which $R_{2A}$ is bonded, and the hetero ring optionally has one or more substituents selected from the group consisting of a group represented by Formula 1 or Formula 2, -OH, and an alkoxy group, and, in this case, $R_{2B}$ is hydrogen or $C_1$-$C_6$ alkyl;
$R_3$ is a single bond or $-CHR_4-$;
$R_4$ is hydrogen, $C_1$-$C_4$ alkyl, or a group represented by Formula 1 or Formula 2;
Formula 1 and Formula 2 are respectively represented by the following formulae:

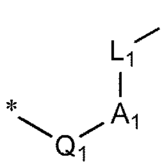 (Formla 1) 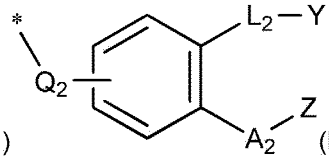 (Formula 2) ,

wherein

* indicates a point of bonding;
$Q_1$ and $Q_2$ are independently a single bond, $C_1$-$C_4$ alkylene, or $C_2$-$C_4$ heteroalkylene containing one oxygen atom;
$A_1$ is -O- or -S-;
$L_1$ is linear $C_1$-$C_3$ alkylene optionally substituted with one or more substituents selected from the group consisting of fluorine, $C_1$-$C_2$ alkyl, $C_1$-$C_2$ fluoroalkyl, and oxo (=O);
$A_2$ is a single bond, -O-, or -S-;
$L_2$ is a single bond or linear $C_1$-$C_3$ alkylene optionally substituted with one or more substituents selected from the group consisting of fluorine, $C_1$-$C_2$ alkyl, $C_1$-$C_2$ fluoroalkyl, and oxo (=O);
X is -OH, $-NR_{Z1}R_{Z2}$, $-CONR_{Z1}R_{Z2}$, or 5- to 6-membered saturated or unsaturated heterocyclyl containing 1 to 3 heteroatoms and optionally substituted with oxo or one or more halogens;
$R_{Z1}$ and $R_{Z2}$ are independently selected from the group consisting of hydrogen, -OH, $C_1$-$C_4$ alkyl, and $C_1$-$C_4$ alkylsulfonyl;
Y is -OH, $C_1$-$C_4$ alkylsulfonylamino, $-NR_{Z3}R_{Z4}$, $-CONR_{Z3}R_{Z4}$, or 5- to 6-membered saturated or unsaturated heterocyclyl containing 1 to 3 heteroatoms and optionally substituted with oxo or halogen;
$R_{Z3}$ and $R_{Z4}$ are independently selected from the group consisting of hydrogen, -OH, $C_1$-$C_4$ alkyl, and $C_1$-$C_4$ alkylsulfonyl; and
Z is hydrogen or $C_1$-$C_4$ alkyl,
provided that when $A_2$ is -O- or -S-, Z is not hydrogen, and
the amino acid represented by Formula A contains at least one group represented by either Formula 1 or Formula 2.

**14.** The amino acid of claim 13, which is selected from the group consisting of:

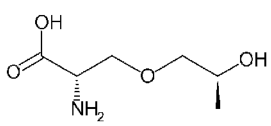、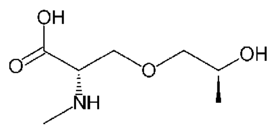、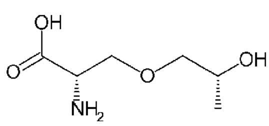、

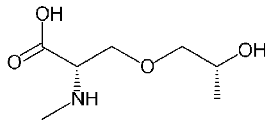、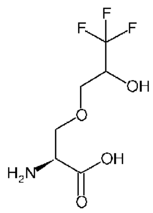、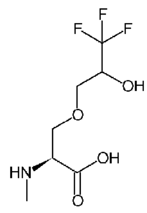、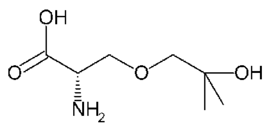、

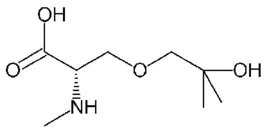、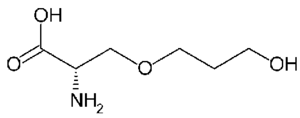、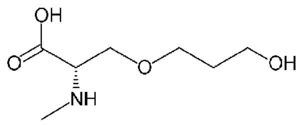、

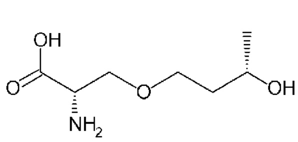、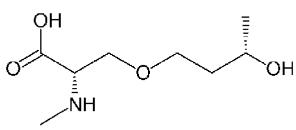、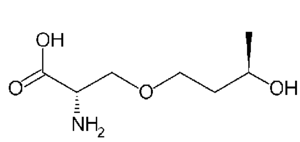、

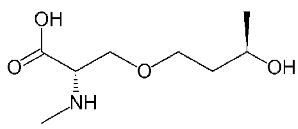、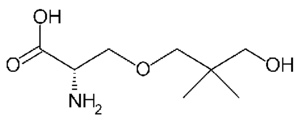、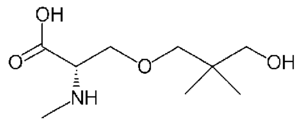、

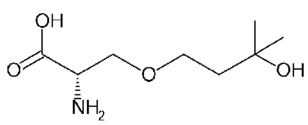、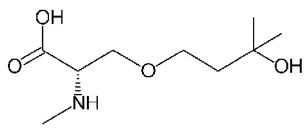、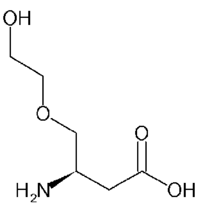、

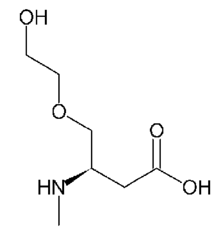、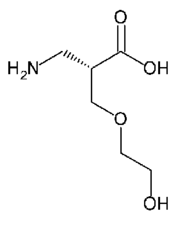、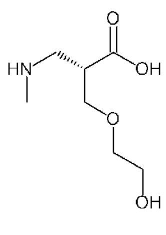、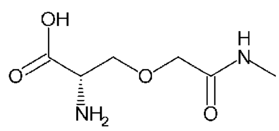、

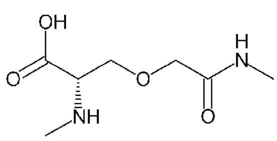、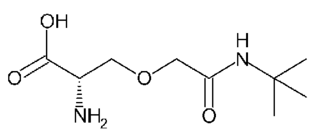、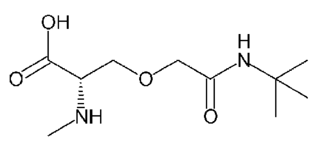、

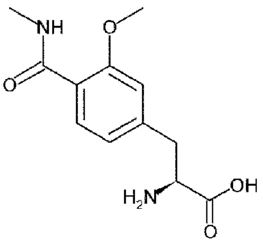,
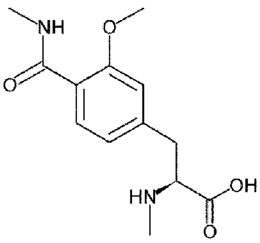,
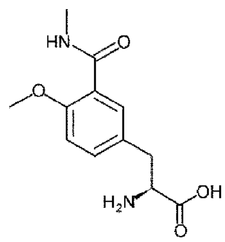,
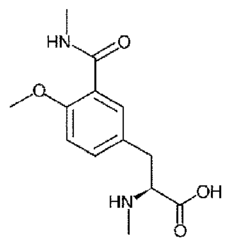,

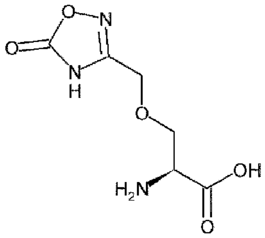,
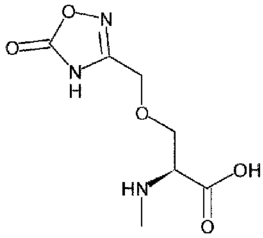,
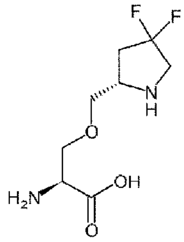,
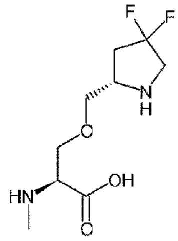,

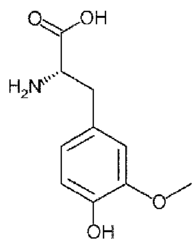,
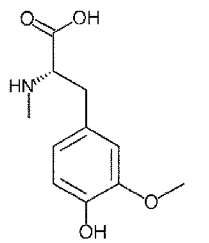,
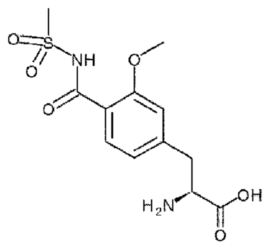,
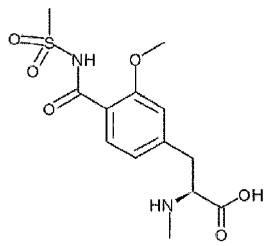

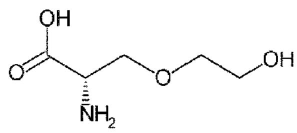,
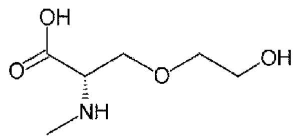,

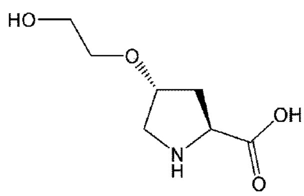,
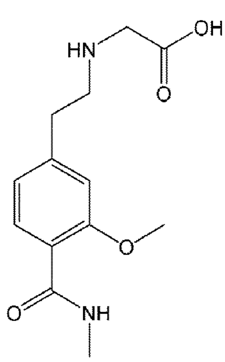,
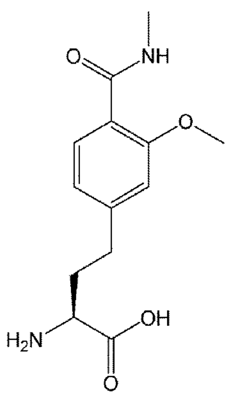,
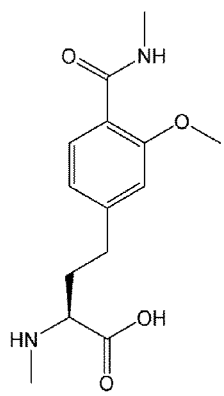,

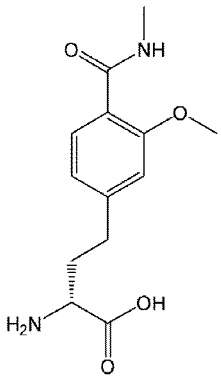
、
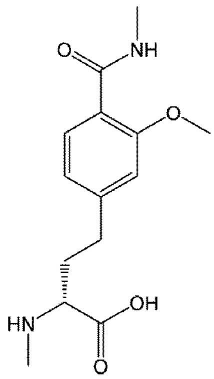
、
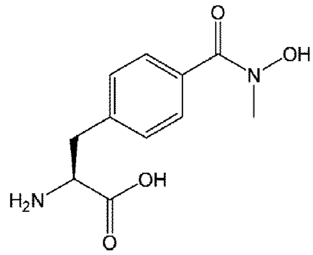
、
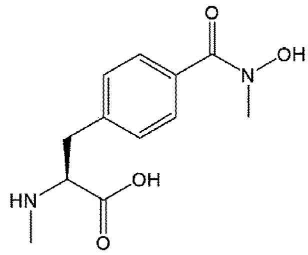
、

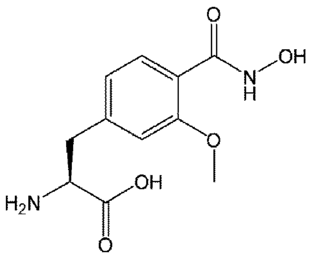
、
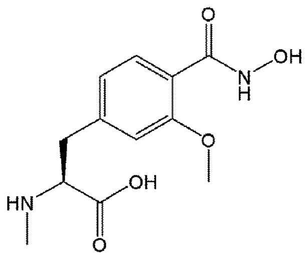
、
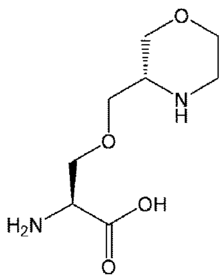
、

and

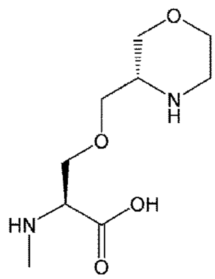

**15.** A protected amino acid, wherein an amino group and/or a carboxyl group contained in the amino acid of claim 13 or 14 is protected by a protecting group.

**16.** The protected amino acid of claim 15, wherein

the protecting group for the amino group is selected from the group consisting of an Fmoc group, a Boc group, a Cbz group, an Alloc group, a nosyl group, a dinitronosyl group, a t-Bu group, a trityl group, and a cumyl group, and/or
the protecting group for the carboxyl group is selected from the group consisting of a methyl group, an allyl group, a t-Bu group, a trityl group, a cumyl group, a methoxytrityl group, and a benzyl group.

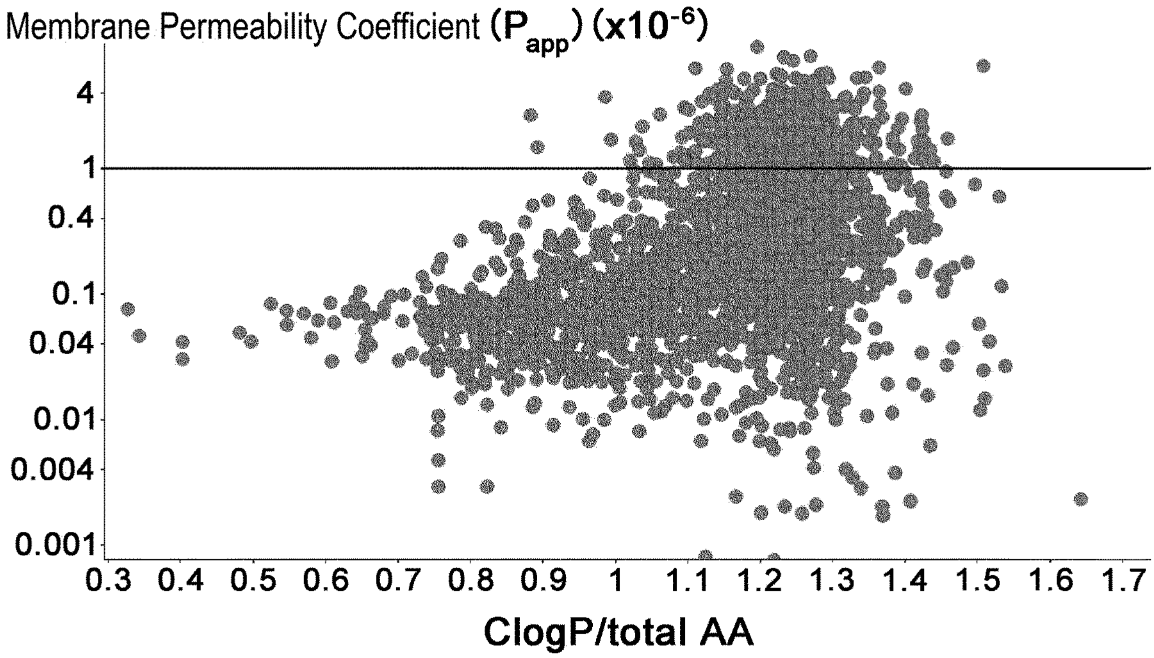
Membrane Permeability Coefficient (Papp) (x10-6)
4
1
0.4
0.1
0.04
0.01
0.004
0.001
0.3 0.4 0.5 0.6 0.7 0.8 0.9 1 1.1 1.2 1.3 1.4 1.5 1.6 1.7
ClogP/total AA

FIG. 1-1

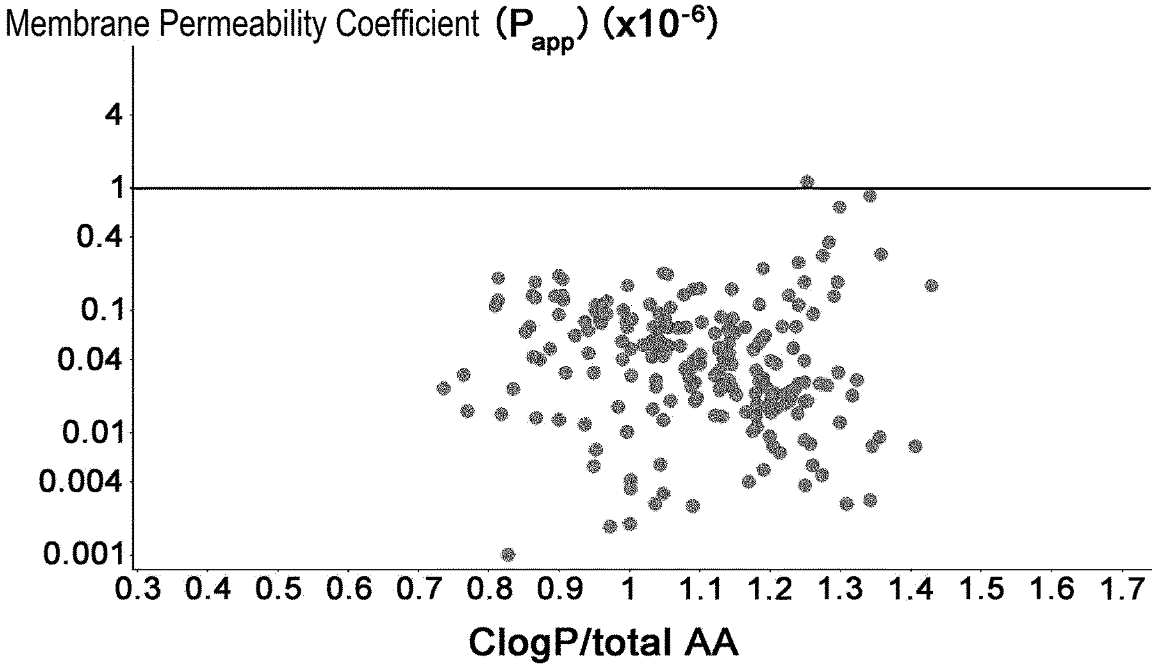
Membrane Permeability Coefficient ($P_{app}$) ($x10^{-6}$)
4
1
0.4
0.1
0.04
0.01
0.004
0.001
0.3 0.4 0.5 0.6 0.7 0.8 0.9 1 1.1 1.2 1.3 1.4 1.5 1.6 1.7
ClogP/total AA

FIG. 1-2

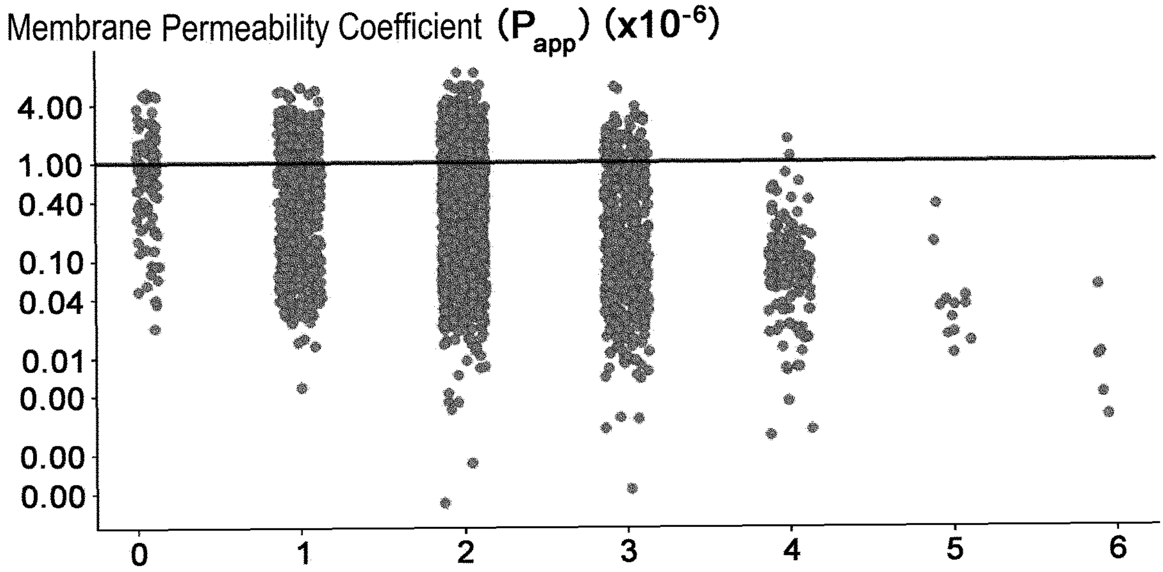

Membrane Permeability Coefficient ($P_{app}$) ($x10^{-6}$)
4.00
1.00
0.40
0.10
0.04
0.01
0.00
0.00
0.00
0
1
2
3
4
5
6


FIG. 2

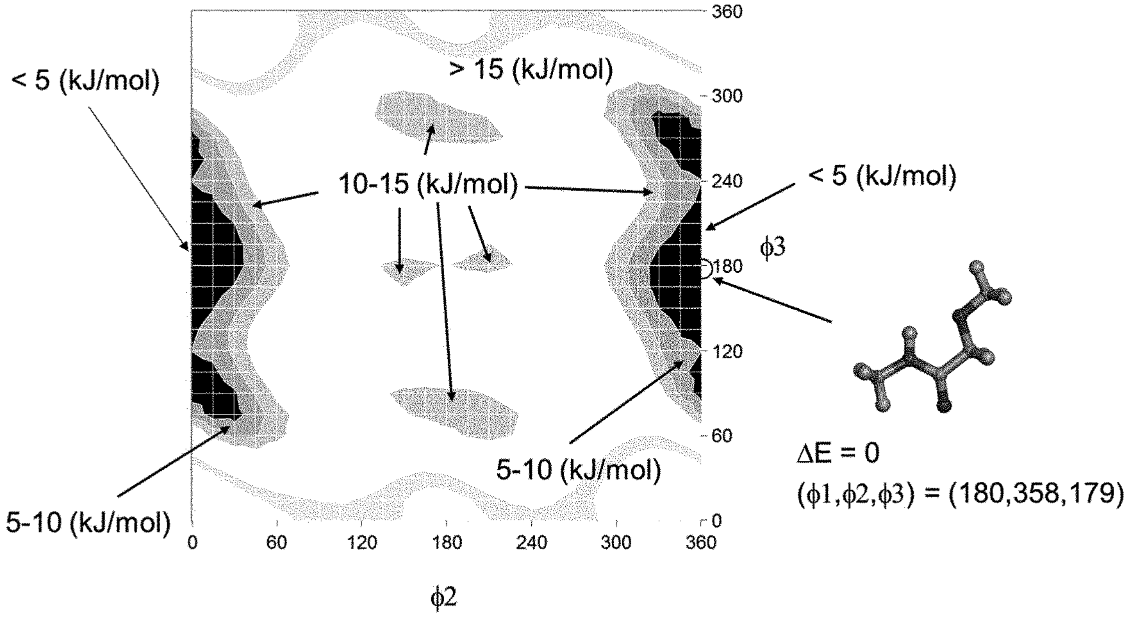
< 5 (kJ/mol)
> 15 (kJ/mol)
10-15 (kJ/mol)
< 5 (kJ/mol)
5-10 (kJ/mol)
5-10 (kJ/mol)
ϕ3
ϕ2
0
60
120
180
240
300
360
ΔE = 0
(ϕ1,ϕ2,ϕ3) = (180,358,179)

FIG. 3-1

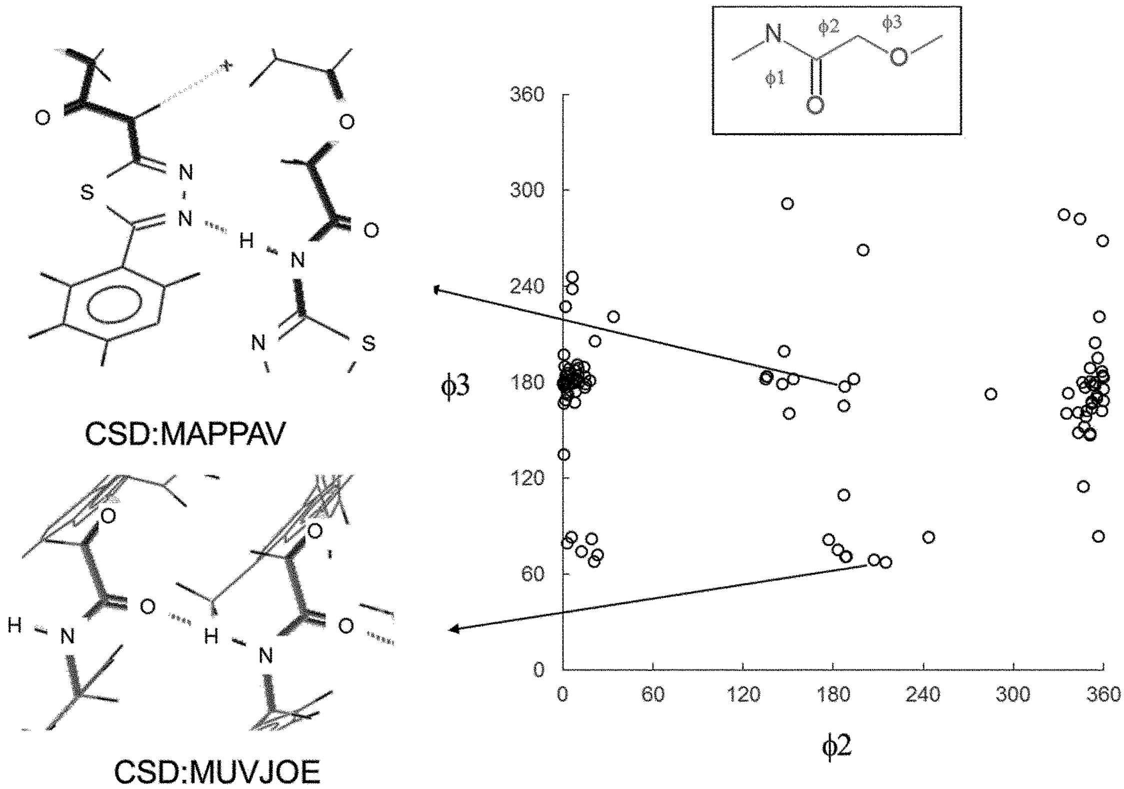


CSD:MAPPAV

CSD:MUVJOE

FIG. 3-2

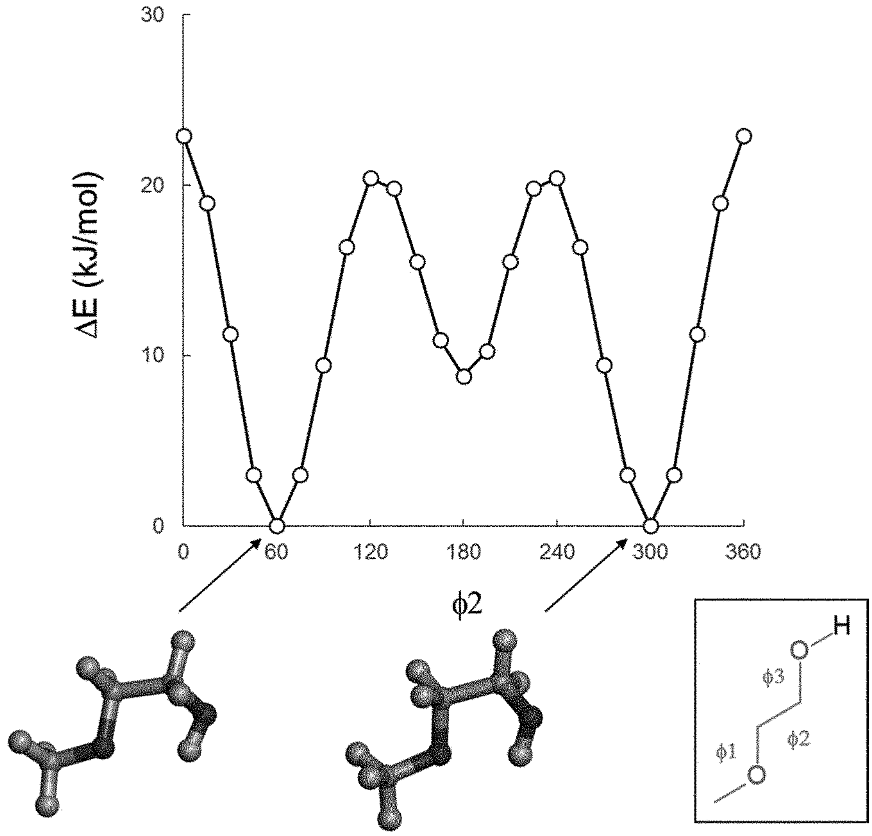


FIG. 4-1

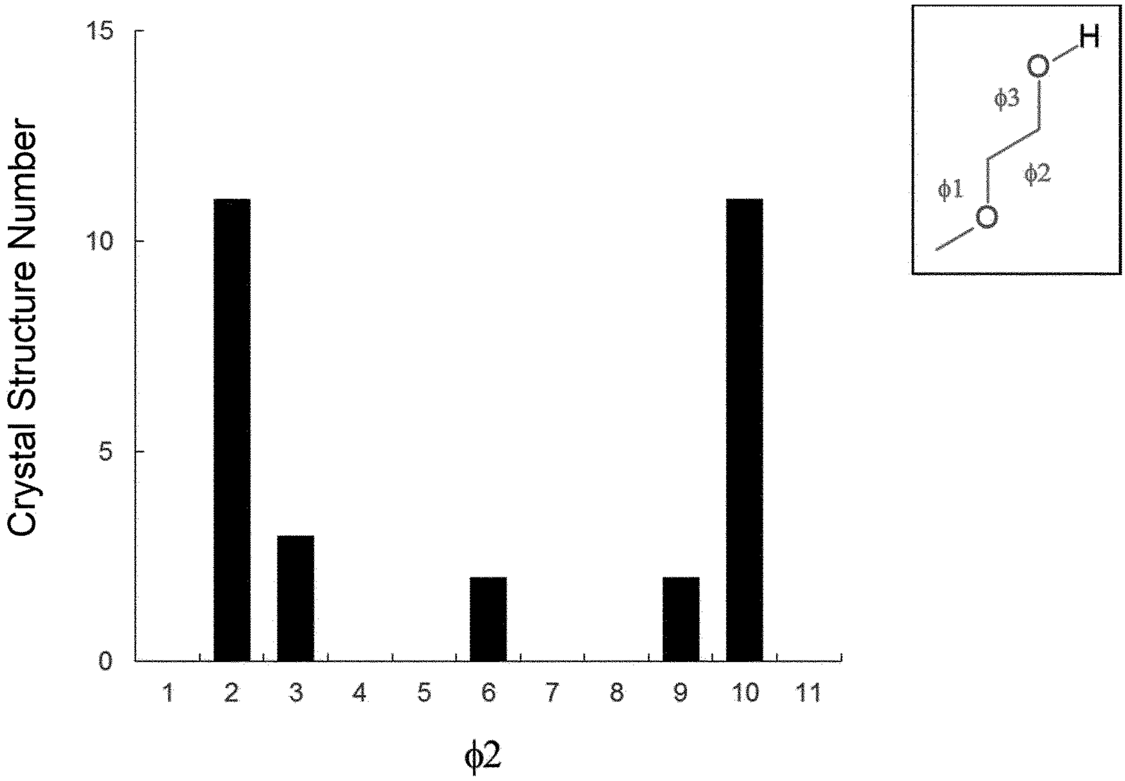


FIG. 4-2

**INTERNATIONAL SEARCH REPORT**

International application No.
PCT/JP2019/048720

A. CLASSIFICATION OF SUBJECT MATTER
C07C 229/22(2006.01)i; C07C 235/06(2006.01)i; C07C 271/22(2006.01)i; C07K 7/00(2006.01)i; C07K 11/02(2006.01)i; C07K 14/00(2006.01)i; C40B 40/08(2006.01)i; C40B 40/10(2006.01)i
FI: C07K7/00; C07K14/00; C40B40/10; C07K11/02; C40B40/08; C07C229/22; C07C271 /22; C07C235/06
According to International Patent Classification (IPC) or to both national classification and IPC

B. FIELDS SEARCHED
Minimum documentation searched (classification system followed by classification symbols)
C07C229/22; C07C235/06; C07C271/22; C07K7/00; C07K11/02; C07K14/00; C40B40/08; C40B40/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | RAFI, Salma B. et al., "Predicting and improving the Membrane Permeability of Peptidic Small Molecules", Journal of Medicinal Chemistry, 2012, vol. 55, pp. 3163-3169, abstract, introduction, tables 1, 2 abstract, introduction, tables 1, 2<br>abstract, introduction, tables 1, 2 | 1, 2, 5-12<br>3, 4, 13-17 |
| Y | KUHN, Bernd et al., "Intramolecular Hydrogen Bonding in Medicinal Chemistry", Journal of Medicinal Chemistry, 2010, vol. 53, pp. 2601-2611, abstract, fig. 5 abstract, fig. 5 | 3, 4, 13-17 |
| A | WO 2018/143145 A1 (CHUGAI PHARMACEUTICAL CO., LTD.) 09.08.2018 (2018-08-09) examples | 1-17 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 February 2020 (25.02.2020) | 10 March 2020 (10.03.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/048720

C (Continuation). DOCUMENTS CONSIDERED TO BE RELEVANT

| Category* | Citation of document, with indication, where appropriate, of the relevant passages | Relevant to claim No. |
|---|---|---|
| P, X | WO 2018/225864 A1 (CHUGAI PHARMACEUTICAL CO., LTD.) 13.12.2018 (2018-12-13) example 1 | 1-17 |
| A | ALEX, Alexander et al., "Intramolecular hydrogen bonding to improve membrane permeability and absorption in beyond rule of five chemical space", Med Chem Comm, 2011, vol. 2, pp. 669-674, abstract, fig. 1, 3 | 1-17 |

Form PCT/ISA/210 (continuation of second sheet) (January 2015)

**INTERNATIONAL SEARCH REPORT**
Information on patent family members

International application No.
PCT/JP2019/048720

| Patent Documents referred in the Report | Publication Date | Patent Family | Publication Date |
|---|---|---|---|
| WO 2018/143145 A1 | 09 Aug. 2018 | (Family: none) | |
| WO 2018/225864 A1 | 13 Dec. 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013100132 A
- WO 2012033154 A
- WO 2018124162 A
- WO 2016148044 A
- WO 2015179823 A
- WO 2013100132 A1


### Non-patent literature cited in the description


- *Future Med. Chem.,* 2009, vol. 1, 1289-1310
- *Angew. Chem. Int. Ed.,* 2018, vol. 57, 14414-14438
- *Saisin Soyaku Kagaku (The Practice of Medicinal Chemistry),* vol. 1, 87
- Thermal Measurements and Equilibrium. Experimental Chemistry Lecture. Maruzen Co., Ltd, vol. 5, 460
- Greene's "Protective Groups in Organic Synthesis. John Wiley & Sons, 2014
- *Journal of Organic Chemistry,* 2010, vol. 75, 245
- *J. Am. Chem. Soc.,* 1999, vol. 121, 6519
- *Strategic Applications of Named Reactions in Organic Synthesis,* 18
- Solid-phase Synthesis Handbook. Merck Co, 01 May 2002
- **GREENE.** Protective Groups in Organic Synthesis. John Wiley & Sons, 2014
- **SHIMIZU, Y ; INOUE, A. ; TOMARI, Y ; SUZUKI, T. ; YOKOGAWA, T. ; NISHIKAWA, K. ; UEDA, T.** Cell-free translation reconstituted with purified components. *Nat. Biotechnol.,* 2001, vol. 19, 751-5
- **SHIMIZU, Y ; UEDA, T.** Methods Mol. Biol. PURE technology, 2010, vol. 607, 11-21
- **JOSEPHSON, K. ; HARTMAN, MC. ; SZOSTAK, JW.** Ribosomal synthesis of unnatural peptides. *J. Am. Chem. Soc.,* 2005, vol. 127, 11727-35
- **KWON, I. et al.** Breaking the degeneracy of the genetic code. *J. Am. Chem. Soc.,* 2003, vol. 125, 7512-3
- **KAWAKAMI, T. et al.** Ribosomal synthesis of polypeptoids and peptoid-peptide hybrids. *J. Am. Chem. Soc.,* 2008, vol. 130, 16861-3
- **KAWAKAMI, T. et al.** Diverse backbone-cyclized peptides via codon reprogramming. *Nat. Chem. Biol.,* 2009, vol. 5, 888-90
- **DEDKOVA LM et al.** Construction of modified ribosomes for incorporation of D-amino acids into proteins. *Biochemistry,* 2006, vol. 45, 15541-51
- **DOI Y et al.** Elongation factor Tu mutants expand amino acid tolerance of protein biosynthesis system. *J Am Chem Soc.,* 2007, vol. 129, 14458-62
- **PARK HS et al.** Expanding the genetic code of Escherichia coli with phosphoserine. *Science,* 2011, vol. 333, 1151-4
- **ANDERSON, JC. ; SCHULTZ, PG.** Adaptation of an orthogonal archaeal leucyl-tRNA and synthetase pair for four-base, amber, and opal suppression. *Biochemistry,* 2003, vol. 42, 9598-608
- **MURAKAMI, H. ; KOUROUKLIS, D. ; SUGA, H.** Using a solid-phase ribozyme aminoacylation system to reprogram the genetic code. *Chem. Biol.,* 2003, vol. 10, 1077-84
- **KIGA, D. ; SAKAMOTO, K. ; KODAMA, K. ; KIGAWA, T. ; MATSUDA, T. ; YABUKI, T. ; SHIROUZU, M. ; HARADA, Y ; NAKAYAMA, H. ; TAKIO, K.** An engineered Escherichia coli tyrosyl-tRNA synthetase for site-specific incorporation of an unnatural amino acid into proteins in eukaryotic translation and its application in a wheat germ cell-free system. *Proc. Natl. Acad. Sci. USA.,* 2002, vol. 99, 9715-20
- **CHIN, JW. ; CROPP, TA. ; ANDERSON, JC. ; MUKHERJI, M. ; ZHANG, Z. ; SCHULTZ, PG. ; CHIN, JW.** An expanded eukaryotic genetic code. *Science,* 2003, vol. 301, 964-7
- **HARTMAN, MC ; JOSEPHSON, K. ; SZOSTAK, JW.** Enzymatic aminoacylation of tRNA with unnatural amino acids. *Proc. Natl. Acad. Sci. USA.,* 2006, vol. 103, 4356-61
- **SUBTELNY, AO. ; HARTMAN, MC. ; SZOSTAK, JW.** Ribosomal synthesis of N-methyl peptides. *J. Am. Chem. Soc.,* 2008, vol. 130, 6131-6
- **HECKLER, TG. ; CHANG, LH. ; ZAMA, Y. ; NAKA, T. ; CHORGHADE, MS. ; HECHT, SM.** T4 RNA ligase mediated preparation of novel "chemically misacylated" tRNAPheS. *Biochemistry,* 1984, vol. 23, 1468-73

- **MURAKAMI, H. ; BONZAGNI, NJ. ; SUGA, H.** Aminoacyl-tRNA synthesis by a resin-immobilized ribozyme. *J. Am. Chem. Soc.,* 2002, vol. 124, 6834-5
- **HASHIMOTO, N. ; NINOMIYA, K. ; ENDO, T. ; SISIDO, M.** Simple and quick chemical aminoacylation of tRNA in cationic micellar solution under ultrasonic agitation. *Chem. Commun. (Camb),* 2005, vol. 34, 4321-3
- **NINOMIYA, K. ; MINOHATA, T. ; NISHIMURA, M. ; SISIDO, M.** In situ chemical aminoacylation with amino acid thioesters linked to a peptide nucleic acid. *J. Am. Chem. Soc.,* 2004, vol. 126, 15984-9
- **ROBERTS RW ; SZOSTAK JW.** RNA-peptide fusions for the in vitro selection of peptides and proteins. *Proc Natl Acad Sci USA.,* 1997, vol. 94, 12297-302
- **NEMOTO N ; MIYAMOTO-SATO E ; HUSIMI Y ; YANAGAWA H.** In vitro virus: bonding of mRNA bearing puromycin at the 3'-terminal end to the C-terminal end of its encoded protein on the ribosome in vitro. *FEBS Lett.,* 1997, vol. 414, 405-8
- **YAMAGUCHI J ; NAIMUDDIN M ; BIYANI M ; SASAKI T ; MACHIDA M ; KUBO T ; FUNATSU T ; HUSIMI Y ; NEMOTO N.** cDNA display: a novel screening method for functional disulfide-rich peptides by solid-phase synthesis and stabilization of mRNA-protein fusions. *Nucleic Acids Res.,* 2009, vol. 37 (16), e108
- **MATTHEAKIS LC ; BHATT RR ; DOWER WJ.** An in vitro polysome display system for identifying ligands from very large peptide libraries. *Proc Natl Acad Sci USA.,* 1994, vol. 91, 9022-6
- **REIERSEN H ; LOBERSLI I ; LOSET GA ; HVATTUM E ; SIMONSEN B ; STACY JE ; MCGREGOR D ; FITZGERALD K ; WELSCHOF M ; BREKKE OH.** Covalent antibody display--an in vitro antibody-DNA library selection system. *Nucleic AcidsRes.,* 2005, vol. 33, e10
- **ODEGRIP R ; COOMBER D ; ELDRIDGE B ; HEDERERR ; KUHLMAN PA ; ULLMAN C ; FITZGERALD K ; MCGREGOR D.** CIS display: In vitro selection of peptides from librariesof protein-DNA complexes. *Proc Natl Acad Sci U S A.,* 2004, vol. 101, 2806-10
- **TAWFIK DS ; GRIFFITHS AD.** Man-made cell-like compartments for molecular evolution. *Nat Biotechnol.,* 1998, vol. 16, 652-6
- *CHEMICAL ABSTRACTS,* 87413-09-0
- *CHEMICAL ABSTRACTS,* 24621-61-2
- *CHEMICAL ABSTRACTS,* 6290-03-5
- *CHEMICAL ABSTRACTS,* 18325-45-6
- *Beilstein Journal of Organic Chemistry,* 2017, vol. 13, 2428-2441
- *CHEMICAL ABSTRACTS,* 156046-05-8
- *CHEMICAL ABSTRACTS,* 146549-21-5
- *Journal of Medicinal Chemistry,* 1991, vol. 34 (4), 1297-1301
- *Journal of Organic Chemistry,* 2014, vol. 79 (20), 9699-9703
- *CHEMICAL ABSTRACTS,* 741288-31-3
- *CHEMICAL ABSTRACTS,* 63148-57-2
- *CHEMICAL ABSTRACTS,* 3277-26-7
- *CHEMICAL ABSTRACTS,* 39608-31-6
- *Letters in Peptie Science,* 2002, vol. 9, 203